# EUROPEAN PATENT APPLICATION

(11) **EP 4 534 534 A1**
(43) Date of publication of application: **09.04.2025**
(21) Application number: 23815307.6
(22) Date of filing: 02.06.2023
(51) Int. Cl.: C07D 401/14, C07D 495/04, A61P 29/00, A61P 35/00

(54) **HETEROCYCLIC COMPOUND CCR4 INHIBITOR AND USER THEREOF**

(30) Priority: 02.06.2022 CN 202210625356; 21.06.2022 CN 202210703210; 11.07.2022 CN 202210811314; 20.07.2022 CN 202210856086; 17.08.2022 CN 202210989136; 11.11.2022 CN 202211410995; 12.01.2023 CN 202310040148; 29.03.2023 CN 202310320791
(71) Applicant: Xizang Haisco Pharmaceutical Co., Ltd., Lhoka, Tibet 856099 (CN)
(72) Inventor: LI, Yao, Lhoka, Tibet 856099 (CN); ZHANG, Guobiao, Lhoka, Tibet 856099 (CN); CHEN, Lei, Lhoka, Tibet 856099 (CN); ZHANG, Xiaobo, Lhoka, Tibet 856099 (CN); HU, Gang, Lhoka, Tibet 856099 (CN); GENG, Pengxin, Lhoka, Tibet 856099 (CN); LI, Wenfei, Lhoka, Tibet 856099 (CN); YAO, Hao, Lhoka, Tibet 856099 (CN); HUANG, Shilin, Lhoka, Tibet 856099 (CN); ZHANG, Yaming, Lhoka, Tibet 856099 (CN); YAN, Linjie, Lhoka, Tibet 856099 (CN); TANG, Pingming, Lhoka, Tibet 856099 (CN); TANG, Yingde, Lhoka, Tibet 856099 (CN); ZHANG, Chen, Lhoka, Tibet 856099 (CN); YAN, Pangke, Lhoka, Tibet 856099 (CN)
(74) Representative: IP TRUST SERVICES
(86) International application number: PCT/CN2023/097926
(87) International publication number: WO 2023/232130

(57) **Abstract**

Disclosed in the present invention are a heterocyclic compound as shown in formula (I) or a stereoisomer, deuterated compound, solvate, pharmaceutically acceptable salt, or co-crystal thereof and a pharmaceutical composition thereof, and a use thereof in the preparation of a drug for treating/preventing CCR4-mediated diseases. Groups in formula (I) are as defined in the description.

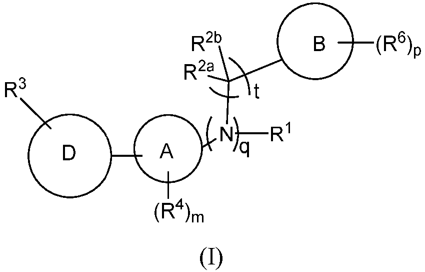

## Description

### Technical Field

The present invention relates to a CCR4 inhibitor, or a stereoisomer, pharmaceutically acceptable salt, solvate, co-crystal or deuterated compound thereof, and the use thereof in the preparation of a drug for treating related diseases mediated by CCR4.

### Background Art

C-C chemokine receptor type 4 (CCR4), also known as CD194, consists of a polypeptide chain containing a seven-transmembrane domain and belongs to the G protein-coupled receptor family. It contains 360 amino acids and has a molecular weight of about 41 kD. It is mainly expressed in various lymphocytes and tissues, and its ligands include a variety of chemokines. As an important chemokine receptor, CCR4 binds to other chemokine ligands, performs its functions, and participates in the regulation of human autoimmune diseases, mainly including atopic dermatitis, asthma, and cutaneous T-cell lymphoma. Studies have shown that CCR4 is expressed by Th2 cells, regulatory T cells (Tregs), mast cells and skin-homing lymphocyte Ag-positive T cells. It selectively inhibits the migration of Th2 cells to inflammatory tissues by blocking the highly expressed CCR4 receptor on Th2 cells, thus playing a role in the upstream pathway of the pathogenesis of inflammation such as asthma and atopic dermatitis. Therefore, it plays an important role in inflammatory diseases that are often accompanied by massive infiltration of Th2-type CD4+ T cells, such as atopic dermatitis, asthma, and allergic airway inflammation.

### Summary of the Invention

The present invention provides a compound of formula (I), (1-1), (I-1a), (I-1b), (I-1c), (I-1d), (I-1e), (I-1f), (I-1g), (I-1h), (I-2), (I-2a), (I-2b), (I-2c), (I-2d), (I-3), (I-3a), or (1-4), and a stereoisomer, a deuterated compound, a solvate, or a pharmaceutically acceptable salt or a co-crystal thereof, wherein the compound has the excellent effects of good activity, excellent physicochemical properties, ease of formulation, excellent pharmacokinetic properties, high bioavailability, and low toxic and side effects.

The compound of formula (I), (I-1), (I-1a), (I-1b), (I-1c), (I-1d), (I-1e), (I-1f), (I-1g), (I-1h), (I-2), (I-2a), (I-2b), (I-2c), (I-2d), (I-3), (I-3a), or (I-4), a stereoisomer, a deuterated compound, a solvate, or a pharmaceutically acceptable salt or a co-crystal thereof, 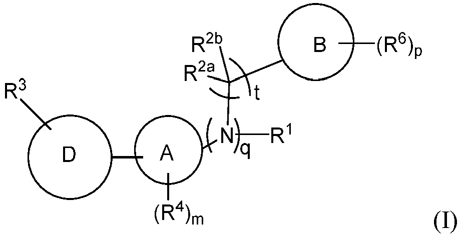 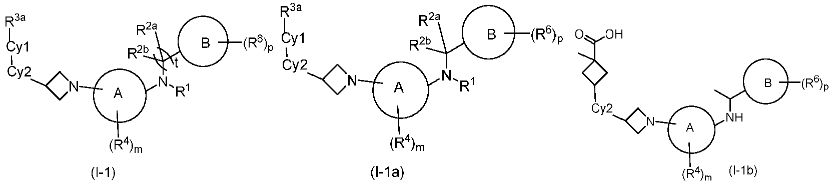 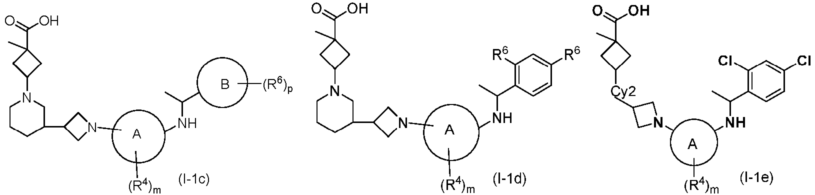 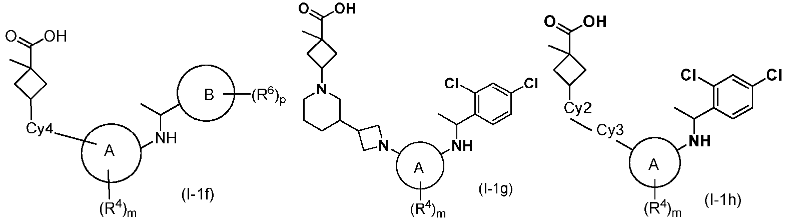 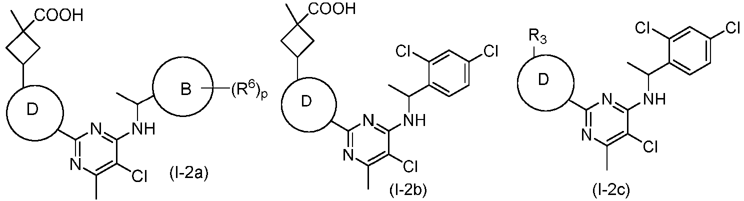 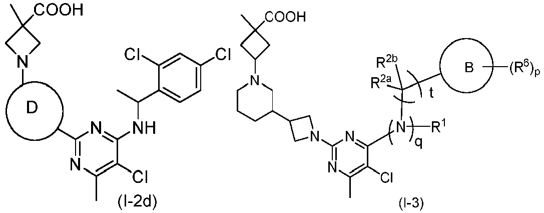 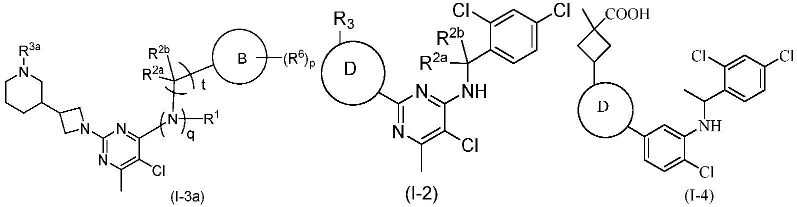 wherein
ring A is selected from 6-membered heteroaryl, 9- or 10-membered bicyclic heteroaryl, aryl, 9- or 10-membered bicyclic heterocyclyl;
in some embodiments, ring A is selected from 6-membered heteroaryl, 9- or 10-membered bicyclic heteroaryl, 6- or 10-membered aryl, 9- or 10-membered bicyclic heterocycloalkyl;
in some embodiments, ring A is selected from 6-membered heteroaryl, 9- or 10-membered bicyclic heteroaryl, or aryl;
ring B is selected from phenyl, 8- to 10-membered aryl, 5- or 6-membered heteroaryl, or 8- to 10-membered heteroaryl;
ring D is selected from -Cy2-Cy3-#, -L₁-Cy2-Cy3-#, -Cy2-L₁-#, -L₁-Cy2-#, - L₁-Cy3-#, Cy4, or -L₁-Cy4-#, wherein # represents the site where the ring D is attached to the ring A;
Cy2 is selected from 4- to 7-membered monoheterocycloalkyl, 7- to 10-membered bridged heterocycloalkyl, 7- to 10-membered spiroheterocycloalkyl, 8-to 10-membered fused heterocycloalkyl, 6- or 7-membered cycloalkyl, or phenyl, and the Cy2 is optionally substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
in some embodiments, Cy2 is selected from 4- to 7-membered monoheterocycloalkyl, 7- to 10-membered bridged heterocycloalkyl, 7- to 10-membered spiroheterocycloalkyl, 8- to 10-membered fused heterocycloalkyl, or 6- or 7-membered cycloalkyl, and the Cy2 is optionally substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy, and NH₂;
Cy3 is selected from 4- to 7-membered monoheterocycloalkyl, 7- to 10-membered spiroheterocycloalkyl, 5- or 6-membered heteroaryl, 6- to 10-membered fused heterocycloalkyl or phenyl, and the Cy3 is optionally substituted with 1 to 3 groups selected from =O, halogen, deuterium, CN, OH, C₁₋₄ alkyl and NH₂;
in some embodiments, Cy3 is selected from 4- to 7-membered monoheterocycloalkyl, 7- to 10-membered spiroheterocycloalkyl, 5- or 6-membered heteroaryl, or 6- to 10-membered fused heterocycloalkyl or phenyl;
Cy4 is selected from 7- to 10-membered bridged heterocycloalkyl, 7- to 10-membered spiroheterocycloalkyl, 8- to 10-membered fused heterocycloalkyl;
L₁ is selected from a bond, C₁₋₆ alkylene, C₂₋₆ alkenylene, C₂₋₆ alkynylene, or - C(=O)-;
in some embodiments, L₁ is selected from C₁₋₆ alkyl, C₂₋₆ alkenyl, C₂₋₆ alkynyl, or -C(=O)-;
each of m, p, q, and t is independently selected from 0, 1, 2, 3, 4, or 5;
each of R¹, R^{2a}, and R^{2b} is independently selected from H, deuterium, C₁₋₄ alkyl, haloC₁₋₄ alkyl, or deuterated C₁₋₄ alkyl;
R³ is selected from Cy1-R^{3a}, or R^{3a};
Cy1 is selected from 3- to 10-membered cycloalkyl or 4- to 10-membered heterocycloalkyl, and the cycloalkyl and heterocycloalkyl are optionally further substituted with 1 to 3 groups selected from C₁₋₆ alkyl, halogen, deuterium, cyano, nitro, OH, haloC₁₋₆ alkyl, or deuterated C₁₋₆ alkyl;
R^{3a} is selected from -COOH, COOC₁₋₆ alkyl, -P(O)(OH)OH, -P(O)(OH)H, hydroxyC₁₋₆ alkyl, -C₁₋₆ alkyl-COOH, 5- or 6-membered heteroaryl, or 5- or 6-membered heterocycloalkyl, and the heteroaryl and heterocycloalkyl are optionally further substituted with 1 to 3 groups selected from =O, C₁₋₆ alkyl, halogen, deuterium, cyano, nitro, OH, haloC₁₋₆ alkyl, or deuterated C₁₋₆ alkyl;
in some embodiments, R^{3a} is selected from -COOH, -COOC₁₋₆ alkyl, - P(O)(OH)OH, -P(O)(OH)H, hydroxyC₁₋₆ alkyl, -C₁₋₆ alkyl-COOH, -C₁₋₆ alkyl-OH, 5- or 6-membered heteroaryl, or 5- or 6-membered heterocycloalkyl, and the heteroaryl and heterocycloalkyl are optionally further substituted with 1 to 3 groups selected from =O, methyl, ethyl, halogen, deuterium, cyano, nitro, OH, and haloC₁₋₆ alkyl;
in some embodiments, R^{3a} is selected from -COOH, -COOC₁₋₆ alkyl, - P(O)(OH)OH, -P(O)(OH)H, hydroxyC₁₋₆ alkyl, -C₁₋₆ alkyl-COOH, -C₁₋₆ alkyl-OH, or 5- or 6-membered heteroaryl;
R⁴ is selected from deuterium, halogen, cyano, nitro, OH, amino, SF₅, N₃, C₁₋₆ alkyl, haloC₁₋₆ alkyl, deuterated C₁₋₆ alkyl, C₂₋₆ alkenyl, C₂₋₆ alkynyl, C₁₋₆ alkoxy, haloC₁₋₆ alkoxy, deuterated C₁₋₆ alkoxy, or -C(=O)R^{4a};
R^{4a} is selected from deuterium, halogen, OH, amino, or C₁₋₆ alkyl;
each of R⁶ and R^{6a} is independently selected from deuterium, halogen, cyano, nitro, OH, amino, SF₅, N₃, C₁₋₆ alkyl, haloC₁₋₆ alkyl, deuterated C₁₋₆ alkyl, C₂₋₆ alkenyl, C₂₋₆ alkynyl, C₁₋₆ alkoxy, haloC₁₋₆ alkoxy, or deuterated C₁₋₆ alkoxy;
alternatively, two R⁶ together with the atoms to which they are attached form 5- or 6-membered cycloalkenyl; the cycloalkenyl is optionally substituted with 1 to 5 selected from R^{6a};
alternatively, R⁴ and R¹ together with the atoms to which they are attached form 5- or 6-membered heteroaryl or 5- or 6-membered heterocycloalkyl;
alternatively, R¹ and R⁶ together with the atoms to which they are attached form 5- or 6-membered heterocycloalkyl;
alternatively, R^{2a} and R⁶ together with the atoms to which they are attached form 5- or 6-membered cycloalkyl;
alternatively, R¹ and R^{2a} together with the atoms to which they are attached form 4- to 6-membered heterocycloalkyl;
alternatively, R^{2a} and R^{2b} on the same carbon atom or different carbon atoms together with the atoms to which they are attached form 3-membered cycloalkyl, or 4- to 6-membered cycloalkyl;
alternatively, two R⁴ on adjacent ring atoms together with the atoms to which they are attached form C₄₋₆ cycloalkyl or 4- to 7-membered heterocycloalkyl, and the cycloalkyl and heterocycloalkyl are optionally further substituted with 1 to 3 groups selected from deuterium, halogen, C₁₋₆ alkyl, cyano, OH, amino, SF₅, N₃, haloC₁₋₆ alkyl, deuterated C₁₋₆ alkyl, C₂₋₆ alkenyl, C₂₋₆ alkynyl, C₁₋₆ alkoxy, haloC₁₋₆ alkoxy, deuterated C₁₋₆ alkoxy and COC₁₋₆ alkyl.

Examples of the C₄₋₆ cycloalkyl include, but are not limited to, cyclobutenyl, cyclopentenyl, and cyclohexenyl; examples of the 4- to 7-membered heterocycloalkyl include, but are not limited to, oxetenly, azetinyl, oxolyl, azacyclopentyl, oxacyclohexenyl, and azacyclohexenyl.

Further, The compound of formula (I), (I-1a), (I-1b), (I-1c), (I-1d), (I-1e), (I-1f), (I-1g), (I-2a), (I-2b), (I-2c), (I-2d), (I-3), (I-3a), or (I-2), a stereoisomer, a deuterated compound, a solvate, or a pharmaceutically acceptable salt or a co-crystal, 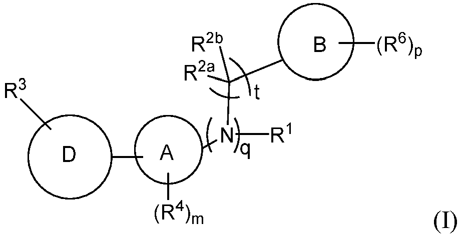 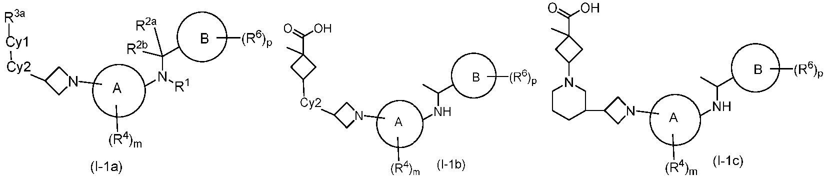 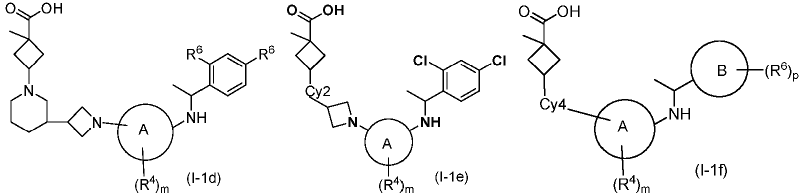 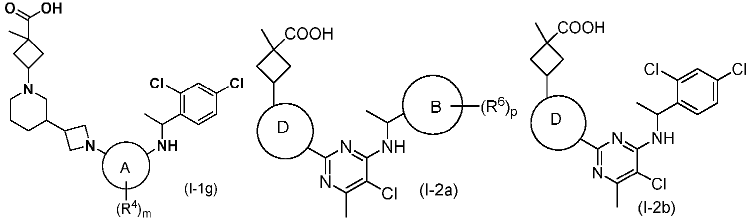 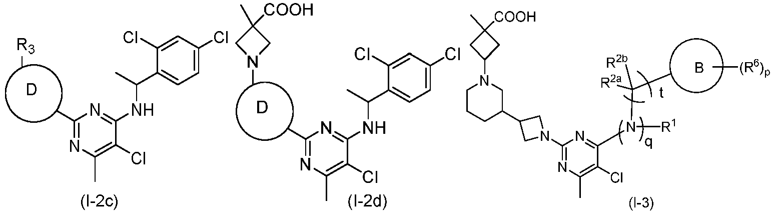 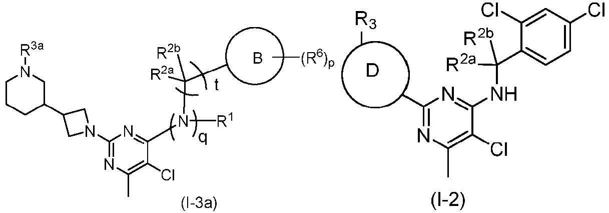 wherein
ring A is selected from 6-membered heteroaryl, or 9-10-membered bicyclic heteroaryl;
ring B is selected from phenyl, 8- to 10-membered aryl, 5- or 6-membered heteroaryl, or 8- to 10-membered heteroaryl;
ring D is selected from -Cy2-Cy3-#, -L₁-Cy2-Cy3-#, -Cy2-L₁-#, -L₁-Cy2-#, - L₁-Cy3-#, Cy4, or -L₁-Cy4-#, wherein # represents the site where the ring D is attached to the ring A;
Cy2 is selected from 4- to 7-membered monoheterocycloalkyl, 7- to 10-membered bridged heterocycloalkyl, 7- to 10-membered spiroheterocycloalkyl, 8-to 10-membered fused heterocycloalkyl, or 6- or 7-membered cycloalkyl, and the Cy2 is optionally substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
Cy3 is selected from 4- to 7-membered monoheterocycloalkyl, or 5- or 6-membered heteroaryl;
Cy4 is selected from 7- to 10-membered bridged heterocycloalkyl, 7- to 10-membered spiroheterocycloalkyl, 8- to 10-membered fused heterocycloalkyl;
L₁ is selected from C₁₋₆ alkyl, C₂₋₆ alkenyl, or C₂₋₆ alkynyl;
each of m, p, q, and t is independently selected from 0, 1, 2, 3, 4, or 5;
each of R¹, R^{2a}, and R^{2b} is independently selected from H, deuterium, C₁₋₄ alkyl, haloC₁₋₄ alkyl, or deuterated C₁₋₄ alkyl;
R³ is selected from Cy1-R^{3a}, or R^{3a};
Cy1 is selected from 3- to 10-membered cycloalkyl or 4- to 10-membered heterocycloalkyl, and the cycloalkyl and heterocycloalkyl are optionally further substituted with 1 to 3 groups selected from C₁₋₆ alkyl, halogen, deuterium, cyano, nitro, OH, haloC₁₋₆ alkyl, or deuterated C₁₋₆ alkyl;
R^{3a} is selected from -COOH, -COOC₁₋₆ alkyl, -P(O)(OH)OH, -P(O)(OH)H, or hydroxyC₁₋₆ alkyl;
R⁴ is selected from deuterium, halogen, cyano, nitro, OH, amino, SF₅, N₃, C₁₋₆ alkyl, haloC₁₋₆ alkyl, deuterated C₁₋₆ alkyl, C₂₋₆ alkenyl, C₂₋₆ alkynyl, C₁₋₆ alkoxy, haloC₁₋₆ alkoxy, deuterated C₁₋₆ alkoxy, or -C(=O)R^{4a};
R^{4a} is selected from deuterium, halogen, OH, amino, or C₁₋₆ alkyl;
R⁶ is selected from deuterium, halogen, cyano, nitro, OH, amino, SF₅, N₃, C₁₋₆ alkyl, haloC₁₋₆ alkyl, deuterated C₁₋₆ alkyl, C₂₋₆ alkenyl, C₂₋₆ alkynyl, C₁₋₆ alkoxy, haloC₁₋₆ alkoxy, or deuterated C₁₋₆ alkoxy;
alternatively, R⁴ and R¹ together with the atoms to which they are attached form 5- or 6-membered heteroaryl or 5- or 6-membered heterocycloalkyl;
alternatively, R¹ and R⁶ together with the atoms to which they are attached form 5- or 6-membered heterocycloalkyl;
alternatively, R^{2a} and R⁶ together with the atoms to which they are attached form 5- or 6-membered cycloalkyl;
alternatively, R¹ and R^{2a} together with the atoms to which they are attached form 4- to 6-membered heterocycloalkyl;
alternatively, R^{2a} and R^{2b} on the same carbon atom or different carbon atoms together with the atoms to which they are attached form a 4- to 6-membered cycloalkyl;
alternatively, two R⁴ on adjacent ring atoms together with the atoms to which they are attached form C₄₋₆ cycloalkyl or 4- to 7-membered heterocycloalkyl, and the cycloalkyl and heterocycloalkyl are optionally further substituted with 1 to 3 groups selected from deuterium, halogen, C₁₋₆ alkyl, cyano, OH, amino, SF₅, N₃, haloC₁₋₆ alkyl, deuterated C₁₋₆ alkyl, C₂₋₆ alkenyl, C₂₋₆ alkynyl, C₁₋₆ alkoxy, haloC₁₋₆ alkoxy, deuterated C₁₋₆ alkoxy and COC₁₋₆ alkyl.

Examples of the C₄₋₆ cycloalkyl include, but are not limited to, cyclobutenyl, cyclopentenyl, and cyclohexenyl; examples of the 4- to 7-membered heterocycloalkyl include, but are not limited to, oxetenly, azetinyl, oxolyl, azacyclopentyl, oxacyclohexenyl, and azacyclohexenyl.

The compound of formula (I) of the present invention, or a stereoisomer, a deuterated compound, a solvate, or a pharmaceutically acceptable salt or a co-crystal thereof, has the structure as shown in formula (1-1), (I-1a), (I-1b), (I-1c), (I-1d), (I-1e), (I-11), or (I-1g): 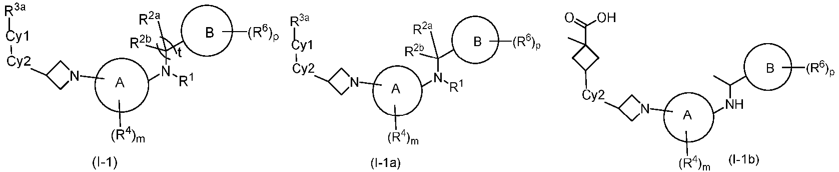 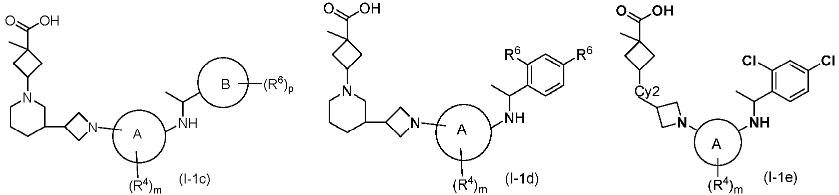 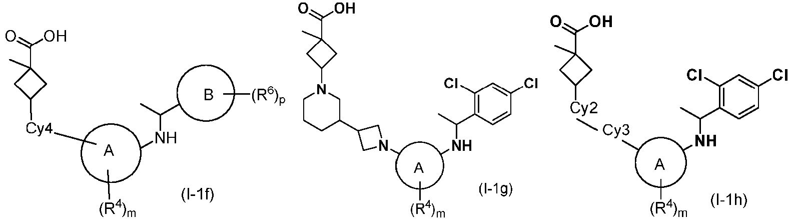 provided that the ring 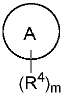 is not 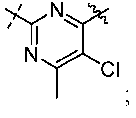 where 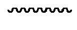 represents an attachment to the right side, and ------- represents an attachment to the left side;
the remaining groups are consistent with the above.

The compound of formula (I) of the present invention, or a stereoisomer, a deuterated compound, a solvate, or a pharmaceutically acceptable salt or a co-crystal thereof, has the structure as shown in formula (I-1a), (I-1b), (I-1c), (I-1d), (I-1e), (I-1f),or (I-1g): 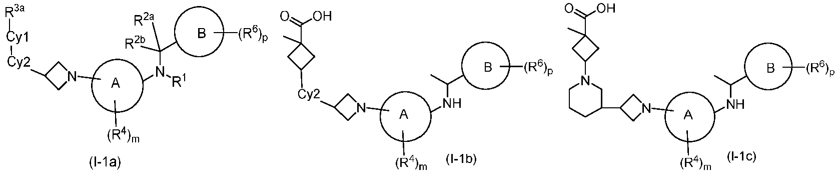 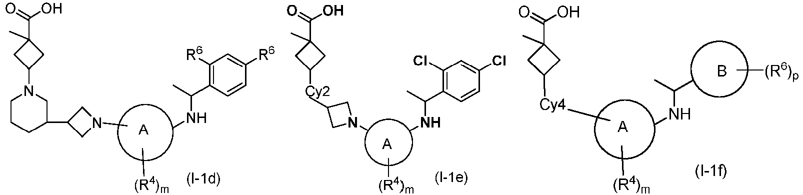 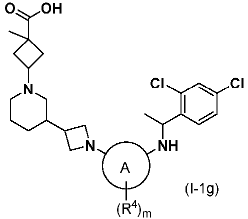 provided that the ring 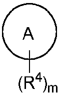 is not 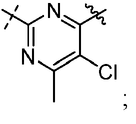 where 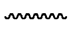 represents an attachment to the right side, and ------- represents an attachment to the left side;
the remaining groups are consistent with the above.

The compound of formula (I) of the present invention, or a stereoisomer, a deuterated compound, a solvate, or a pharmaceutically acceptable salt or a co-crystal thereof, has the structure as shown in formula (I-1a), (I-1b), (I-1c), (I-1d), (I-1e), (I-1f), (I-1g), or (I-1h): 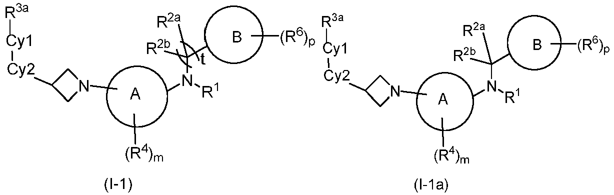 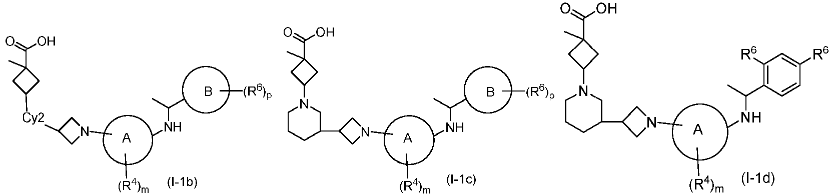 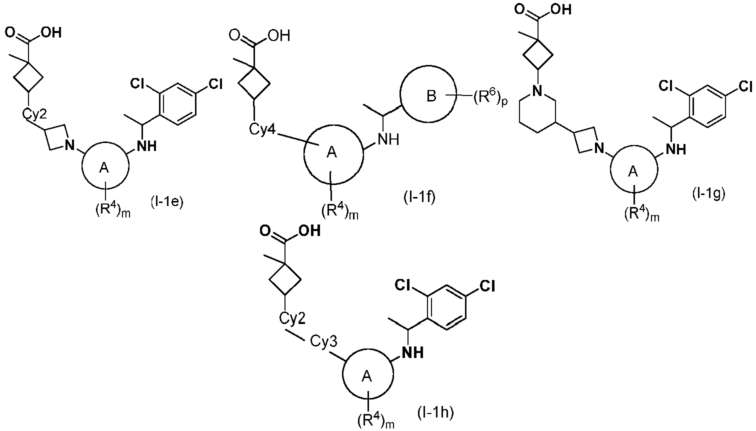 provided that:
(1) ring 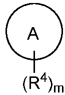 is not 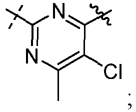
(2) in formula (I-1h), ring 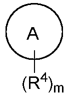 is not 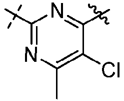 and Cy2 is not 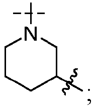
(3) in formula (I-1d), ring 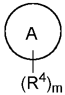 is not 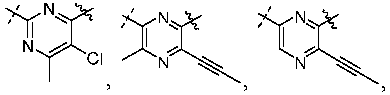 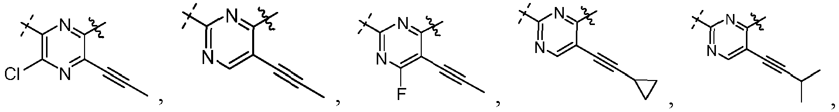 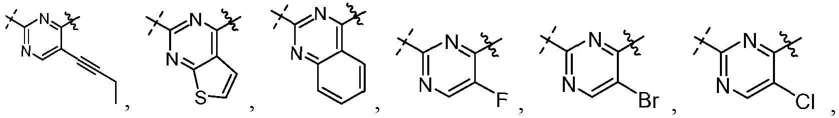 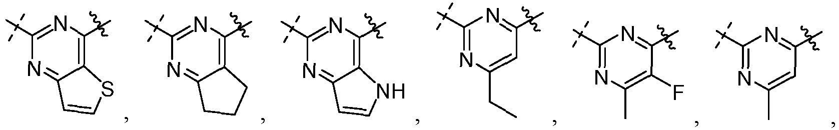 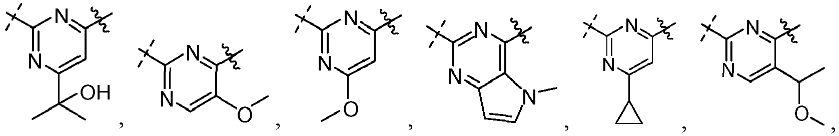 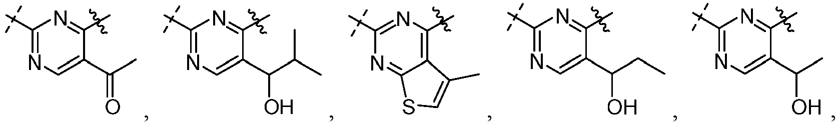 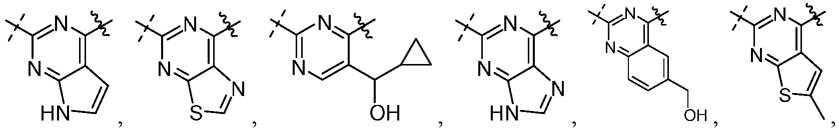 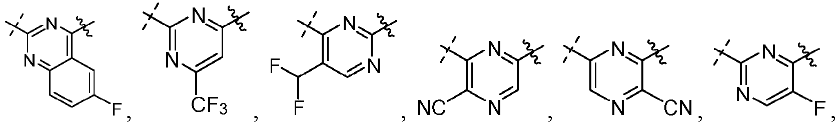 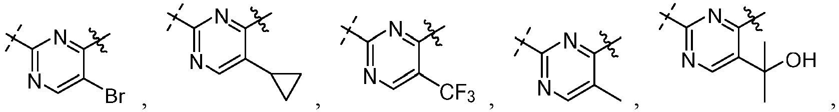 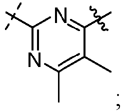
   where 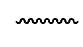 represents an attachment to the right side, andrepresents an attachment to the left side;
   in some embodiments, each R⁶ is independently selected from deuterium, halogen, cyano, OH, amino, SF₅, N₃, C₁₋₄ alkyl, haloC₁₋₄ alkyl, deuterated C₁₋₄ alkyl, C₂₋₄ alkenyl, C₂₋₄ alkynyl, or C₁₋₄ alkoxy; alternatively, two R⁶ together with the atoms to which they are attached form 5- or 6-membered cycloalkenyl; the cycloalkenyl is optionally substituted with 1 to 5 selected from R^{6a};
   in some embodiments, each R⁶ is independently selected from deuterium, halogen, cyano, OH, amino, C₁₋₂ alkyl, haloC₁₋₂ alkyl, deuterated C₁₋₂ alkyl, C₂₋₃ alkenyl, C₂₋₃ alkynyl, or C₁₋₂ alkoxy; alternatively, two R⁶ together with the atoms to which they are attached form 5- or 6-membered cycloalkenyl;
   in some embodiments, each R⁶ is independently selected from F, Cl, Br, OH, amino, methyl, ethyl, vinyl, ethynyl, methoxy, ethoxy, halomethyl, or haloethyl;
   in some embodiments, R⁴ is selected from deuterium, halogen, cyano, OH, amino, C₁₋₄ alkyl, haloC₁₋₄ alkyl, deuterated C₁₋₄ alkyl, C₂₋₄ alkenyl, C₂₋₄ alkynyl, C₁₋₄ alkoxy, or deuterated C₁₋₄ alkoxy; alternatively, two R⁴ on adjacent ring atoms together with the atoms to which they are attached form C₄₋₆ cycloalkyl or 4- to 7-membered heterocycloalkyl, and the cycloalkyl or heterocycloalkyl is optionally further substituted with 1 to 3 groups selected from deuterium, halogen, C₁₋₄ alkyl, cyano, OH, amino, haloC₁₋₄ alkyl, deuterated C₁₋₄ alkyl, C₂₋₄ alkenyl, C₂₋₄ alkynyl, C₁₋₄ alkoxy, or deuterated C₁₋₄ alkoxy;
   in some embodiments, R⁴ is selected from deuterium, F, Cl, Br, cyano, OH, amino, C₁₋₂ alkyl, haloC₁₋₂ alkyl, deuterated C₁₋₂ alkyl, C₂₋₄ alkenyl, C₂₋₄ alkynyl, C₁₋₂ alkoxy, or deuterated C₁₋₂ alkoxy; alternatively, two R⁴ on adjacent ring atoms together with the atoms to which they are attached form C₄₋₆ cycloalkyl or 4- to 7-membered heterocycloalkyl, and the cycloalkyl or heterocycloalkyl is optionally further substituted with 1 to 3 groups selected from deuterium, F, Cl, Br, C₁₋₂ alkyl, cyano, OH, amino, haloC₁₋₂ alkyl, deuterated C₁₋₂ alkyl, C₂₋₄ alkenyl, C₂₋₄ alkynyl, C₁₋₂ alkoxy, or deuterated C₁₋₂ alkoxy;
   in some embodiments, R⁴ is selected from deuterium, F, Cl, Br, cyano, OH, amino, methyl, ethyl, fluoromethyl, fluoroethyl, ethenyl, propenyl, ethynyl, propynyl, methoxy, or ethoxy; alternatively, two R⁴ on adjacent ring atoms together with the atoms to which they are attached form C₄₋₆ cycloalkyl or 4- to 7-membered heterocycloalkyl, and the cycloalkyl or heterocycloalkyl is optionally further substituted with 1 to 3 groups selected from deuterium, F, Cl, Br, cyano, OH, amino, methyl, ethyl, fluoromethyl, fluoroethyl, ethenyl, propenyl, ethynyl, propynyl, methoxy, or ethoxy;
   the remaining groups are consistent with the above.

The compound of formula (I) of the present invention, or a stereoisomer, a deuterated compound, a solvate, or a pharmaceutically acceptable salt or a co-crystal thereof, has the structure as shown in formula (I-1a), (I-1b), (I-1c), (I-1d), (I-1e), (I-1f),(I-1g), or (I-1h): 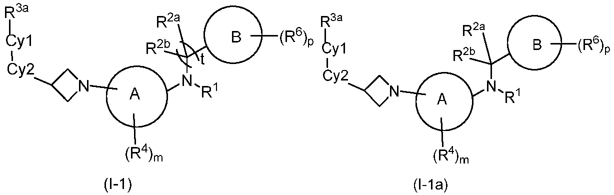 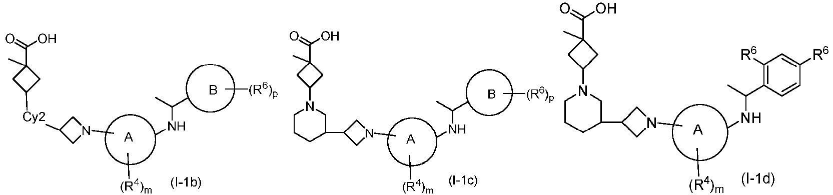 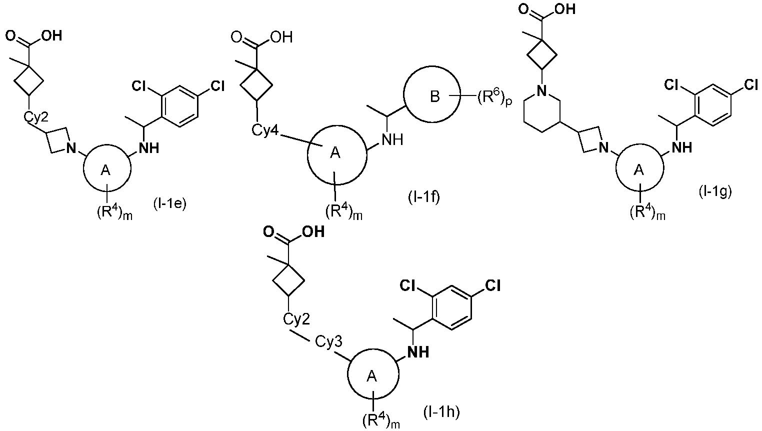 provided that:
(1) ring 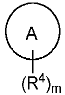 is not 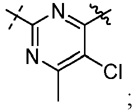
(2) in formula (I-1h), ring 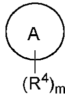 is not 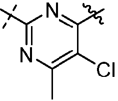 and Cy2 is not 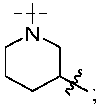
(3) in formula (I-1d), ring 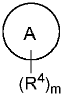 is selected from: 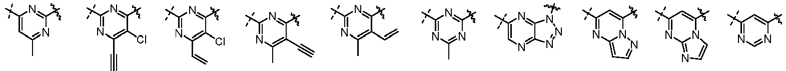 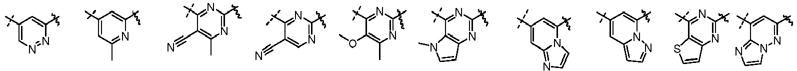 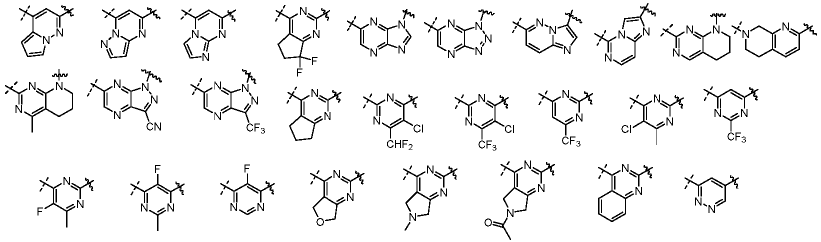 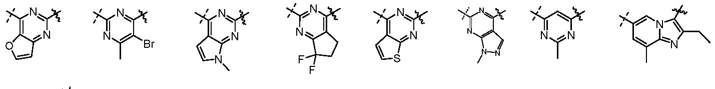 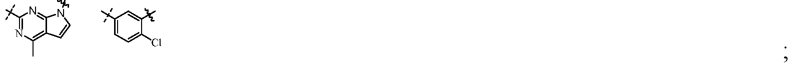
   where 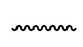 represents an attachment to the right side, andrepresents an attachment to the left side.
   the remaining groups are consistent with the above.

The compound of formula (I) of the present invention, or a stereoisomer, a deuterated compound, a solvate, or a pharmaceutically acceptable salt or a co-crystal thereof, has the structure as shown in formula (I-1d): 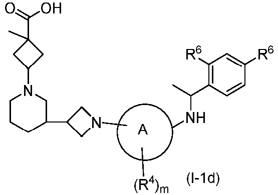 ring 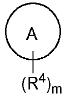 is selected from: 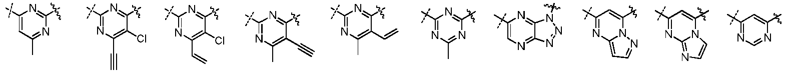 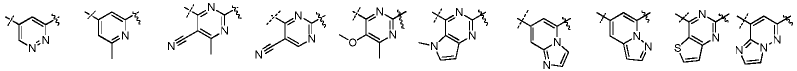 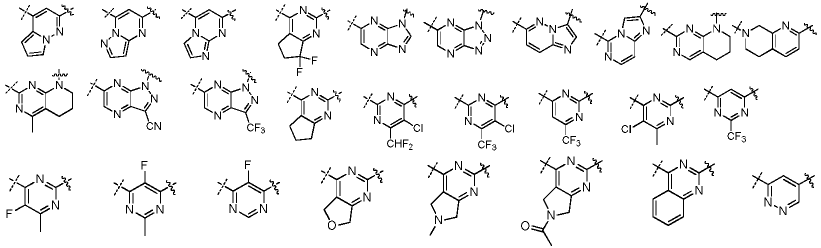 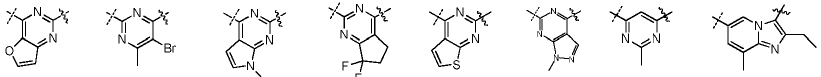 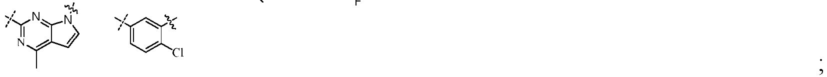
each R⁶ is independently selected from deuterium, halogen, cyano, OH, amino, SF₅, N₃, C₁₋₄ alkyl, haloC₁₋₄ alkyl, deuterated C₁₋₄ alkyl, C₂₋₄ alkenyl, C₂₋₄ alkynyl, or C₁₋₄ alkoxy; alternatively, two R⁶ together with the atoms to which they are attached form 5- or 6-membered cycloalkenyl; the cycloalkenyl is optionally substituted with 1 to 5 selected from R^{6a};
in some embodiments, each R6 is independently selected from F, Cl, Br, OH, amino, methyl, ethyl, vinyl, ethynyl, methoxy, ethoxy, fluoromethyl, or fluoroethyl;
the remaining groups are consistent with the above.

The compound of formula (I) of the present invention, or a stereoisomer, a deuterated compound, a solvate, or a pharmaceutically acceptable salt or a co-crystal thereof, has the structure as shown in formula (I-1h): 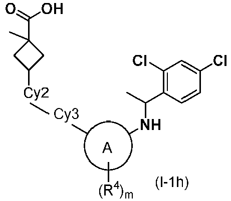
ring A is selected from 6-membered heteroaryl, 9- or 10-membered bicyclic heteroaryl, 6- to 10-membered aryl, 9- or 10-membered bicyclic heterocycloalkyl;
R⁴ is selected from deuterium, halogen, cyano, nitro, OH, amino, SF₅, N₃, C₁₋₆ alkyl, haloC₁₋₆ alkyl, deuterated C₁₋₆ alkyl, C₂₋₆ alkenyl, C₂₋₆ alkynyl, C₁₋₆ alkoxy, haloC₁₋₆ alkoxy, deuterated C₁₋₆ alkoxy, or -C(=O)R^{4a};
in some embodiments, R⁴ is selected from deuterium, halogen, cyano, nitro, OH, amino, C₁₋₄ alkyl, haloC₁₋₄ alkyl, deuterated C₁₋₄ alkyl, C₂₋₄ alkenyl, C₂₋₄ alkynyl, C₁₋₄ alkoxy, haloC₁₋₄ alkoxy, or deuterated C₁₋₄ alkoxy;
in some embodiments, the ring 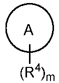 is selected from 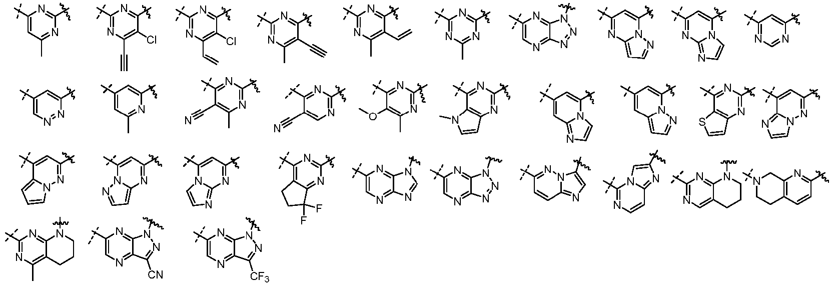 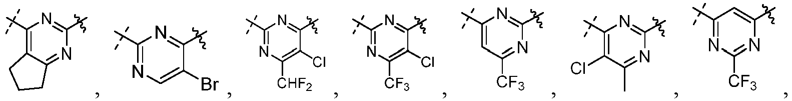 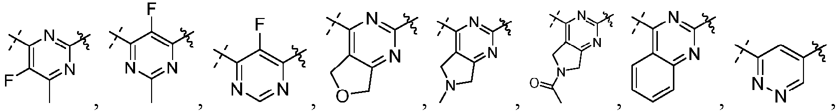 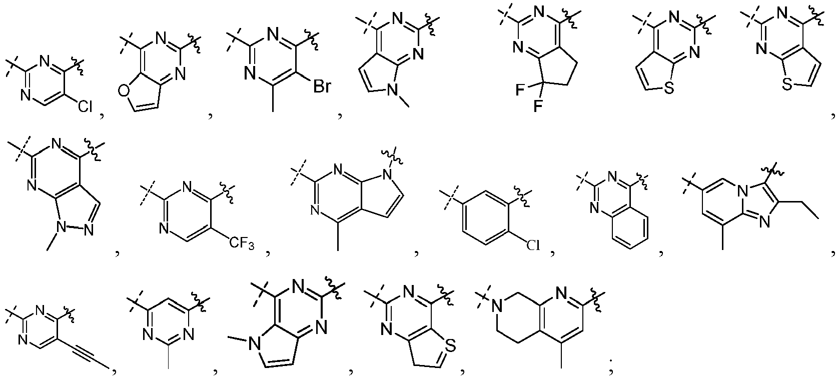
Cy2 is selected from 4- to 7-membered monoheterocycloalkyl, 7- to 10-membered bridged heterocycloalkyl, 7- to 10-membered spiroheterocycloalkyl, 8-to 10-membered fused heterocycloalkyl, 6- or 7-membered cycloalkyl, or phenyl, and the Cy2 is optionally substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy and NH₂;
in some embodiments, Cy2 is selected from 4- to 7-membered monoheterocycloalkyl, 7- to 9-membered bridged heterocycloalkyl, 7- to 9-membered spiroheterocycloalkyl, 8- to 10-membered fused heterocycloalkyl, 6-membered cycloalkyl, or phenyl, and the Cy2 is optionally substituted with 1 to 3 groups selected from =O, F, Cl, Br, deuterium, CN, OH, C₁₋₂ alkyl, haloC₁₋₂ alkyl, deuterated C₁₋₂ alkyl, C₁₋₂ alkoxy, and NH₂;
Cy3 is selected from 4- to 7-membered monoheterocycloalkyl, 5- or 6-membered heteroaryl, 6- to 10-membered fused heterocycloalkyl, or phenyl, and the Cy3 is optionally substituted with 1 to 3 groups selected from =O, halogen, deuterium, CN, OH, C₁₋₂ alkyl and NH₂;
in some embodiments, Cy3 is selected from 4- to 7-membered monoheterocycloalkyl, 5- or 6-membered heteroaryl, 6- to 8-membered fused heterocycloalkyl, or phenyl, and the Cy3 is optionally substituted with 1 to 3 groups selected from =O, F, Cl, Br, deuterium, CN, OH, methyl, ethyl and NH₂;
provided that: 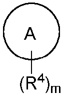 the ring is not 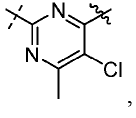 and Cy2 is not 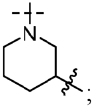
the remaining groups are consistent with the above.

The compound of formula (I) of the present invention, or a stereoisomer, a deuterated compound, a solvate, or a pharmaceutically acceptable salt or a co-crystal thereof, has the structure as shown in formula (I-2a), (I-2b), (I-2c), (I-2d), or (I-2): 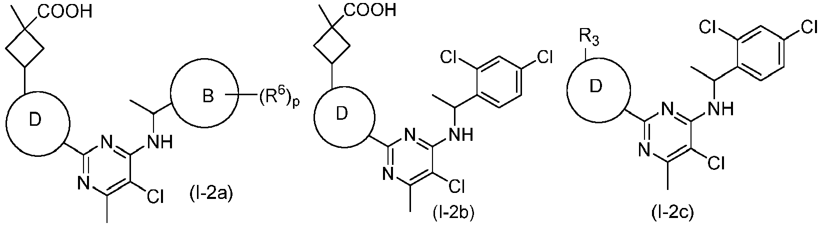 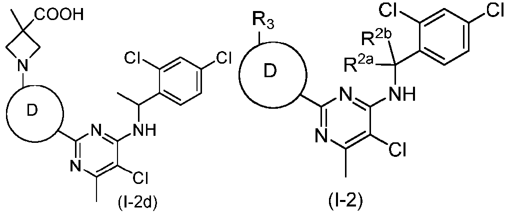
in some embodiments, ring D is selected from -Cy2-Cy3-#, -Cy2-L₁-#, -L₁-Cy2-#, or Cy4, wherein # represents the site where the ring D is attached to the ring A;
in some embodiments, ring D is selected from -Cy2-Cy3-#, wherein # represents the site where the ring D is attached to the ring A;
provided that the ring D is not selected from 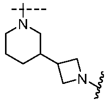 where 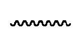 represents an attachment to the right side, and ------- represents an attachment to the left side;
the definitions of other groups are consistent with those mentioned above.

Further, the compound of formula (I) of the present invention, or a stereoisomer, a deuterated compound, a solvate, or a pharmaceutically acceptable salt or a co-crystal thereof, has the structure as shown in formula (I-2a), (I-2b), (I-2c) or (I-2d): 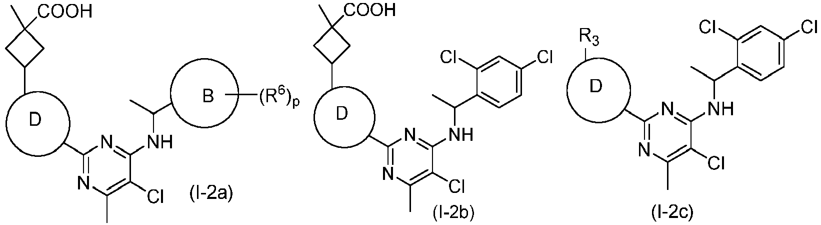 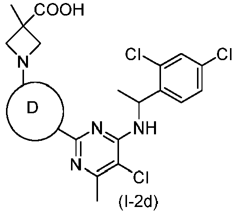
provided that the ring D is not selected from 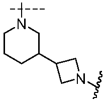 where 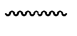 represents an attachment to the right side, and ------- represents an attachment to the left side;
the definitions of other groups are consistent with those mentioned above.

The compound of formula (I) of the present invention, or a stereoisomer, a deuterated compound, a solvate, or a pharmaceutically acceptable salt or a co-crystal thereof, has the structure as shown in formula (I-3): 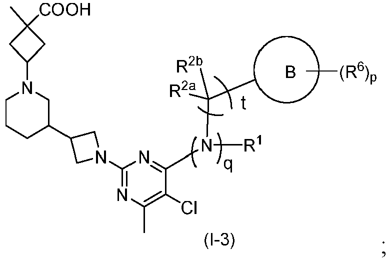
provided that, 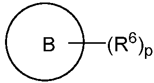 is not selected from 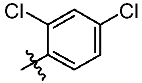
the definitions of other groups are consistent with those mentioned above.

The compound of formula (I) of the present invention, or a stereoisomer, a deuterated compound, a solvate, or a pharmaceutically acceptable salt or a co-crystal thereof, has the structure as shown in formula (I-3) or (I-3a): 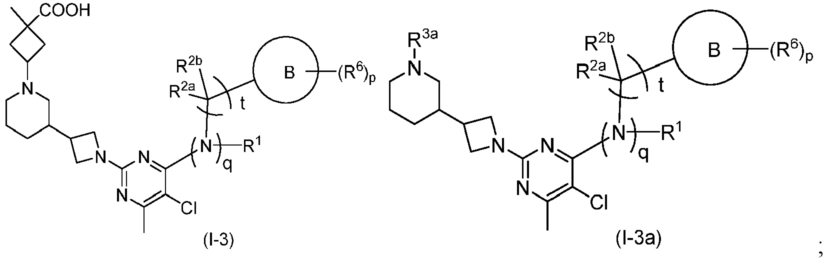
provided that, 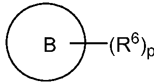 is not selected from 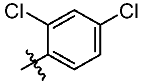
the definitions of other groups are consistent with those mentioned above.

The compound of formula (I) of the present invention, or a stereoisomer, a deuterated compound, a solvate, or a pharmaceutically acceptable salt or a co-crystal thereof, the compound has the structure as shown in formula (1-4): 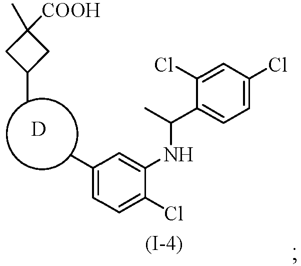
in some embodiments, ring D is selected from -Cy2-Cy3-#, -Cy2-L₁-#, or -L₁-Cy2-#, wherein # represents the site where the ring D is attached to the ring A;
in some embodiments, ring D is selected from -Cy2-Cy3-#, wherein # represents the site where the ring D is attached to the ring A;
the definitions of other groups are consistent with those mentioned above.

The compound of formula (I) of the present invention, or a stereoisomer, a deuterated compound, a solvate, or a pharmaceutically acceptable salt or a co-crystal thereof, wherein
ring 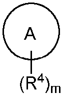 is selected from 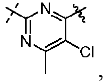 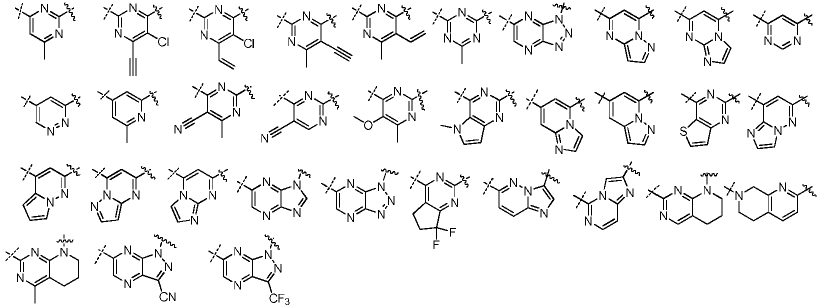 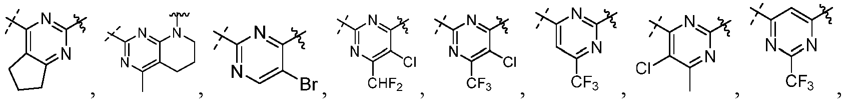 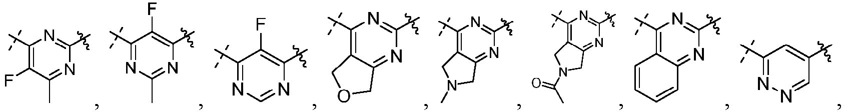 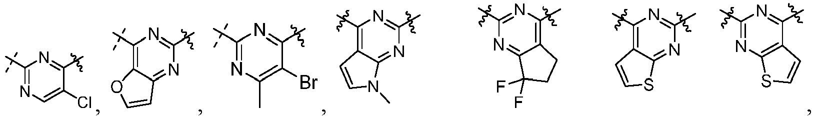
alternatively, ring 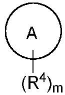 is selected from 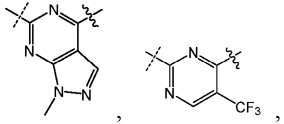 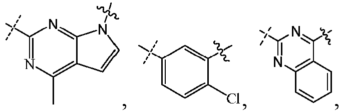
alternatively, ring 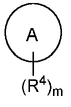 is selected from 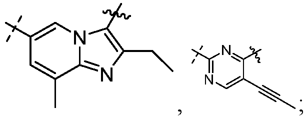
alternatively, ring 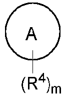 is selected from 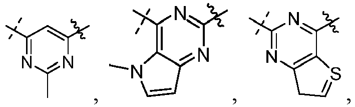 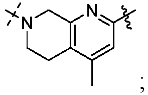
where 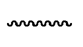 represents an attachment to the right side, andrepresents an attachment to the left side;
alternatively, R⁴, R¹ and the atoms to which they are attached together with the ring A form 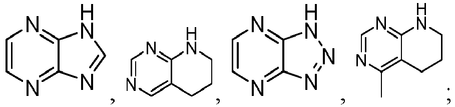
the definitions of other groups are consistent with those mentioned above.

Further, The compound of formula (I) of the present invention, or a stereoisomer, a deuterated compound, a solvate, or a pharmaceutically acceptable salt or a co-crystal thereof, wherein
ring 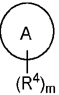 is selected from 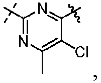 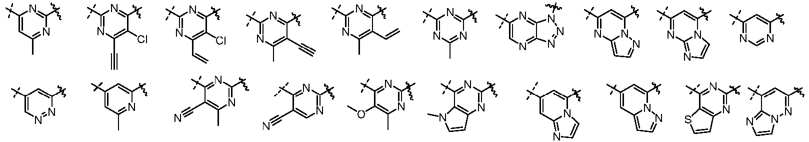 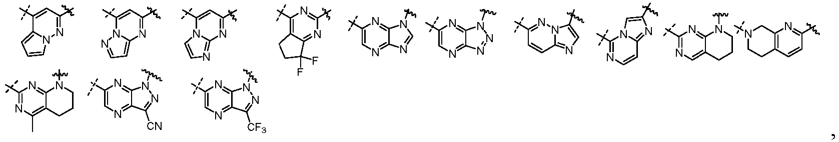 where 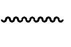 represents an attachment to the right side and ------- represents an attachment to the left side;
alternatively, R⁴, R¹ and the atoms to which they are attached together with the ring A form 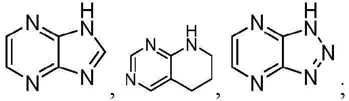
the definitions of other groups are consistent with those mentioned above.

The compound of formula (I) of the present invention, or a stereoisomer, a deuterated compound, a solvate, or a pharmaceutically acceptable salt or a co-crystal thereof,
Ring 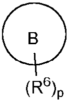 is selected from 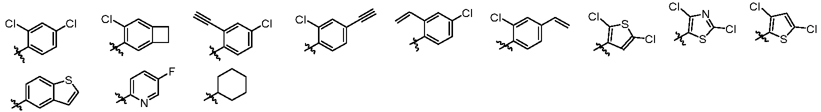 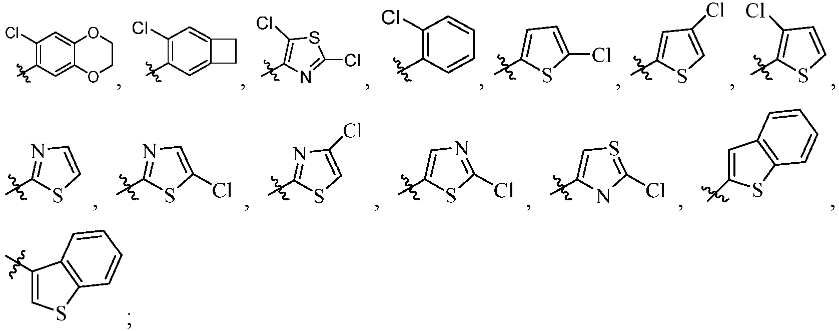
alternatively, R¹ and R⁶ and the atoms to which they are attached together with ring B form 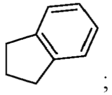
alternatively, R^{2a} and R⁶ and the atoms to which they are attached together with ring B form

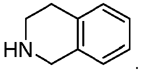

The compound of formula (I) of the present invention, or a stereoisomer, a deuterated compound, a solvate, or a pharmaceutically acceptable salt or a co-crystal thereof,
ring 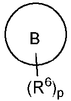 is selected from 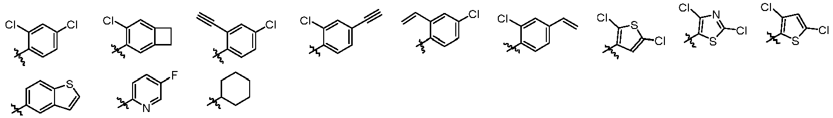 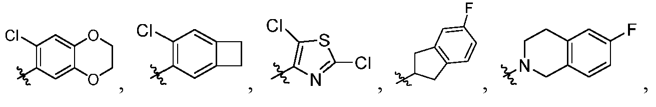 or ring 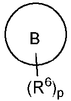 is selected from 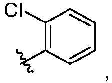 or selected from 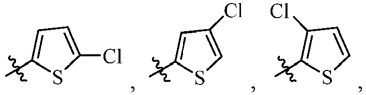 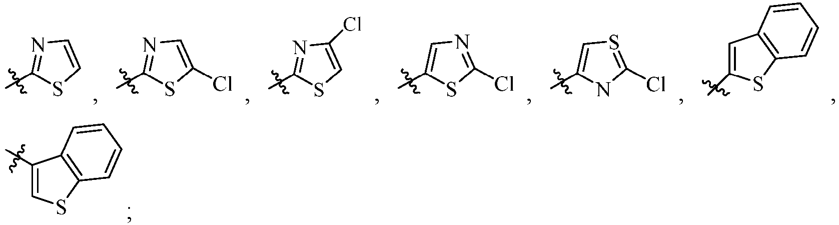
the definitions of other groups are consistent with those mentioned above.

Further, The compound of formula (I) of the present invention, or a stereoisomer, a deuterated compound, a solvate, or a pharmaceutically acceptable salt or a co-crystal thereof,
ring 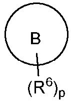 is selected from 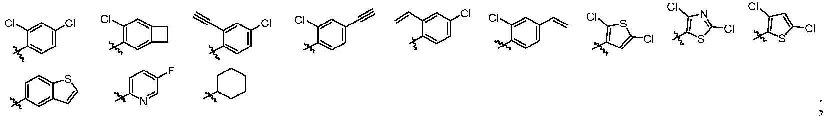
the definitions of other groups are consistent with those mentioned above.

The compound of formula (I) of the present invention, or a stereoisomer, a deuterated compound, a solvate, or a pharmaceutically acceptable salt or a co-crystal thereof, wherein
ring Cy2 is selected from: 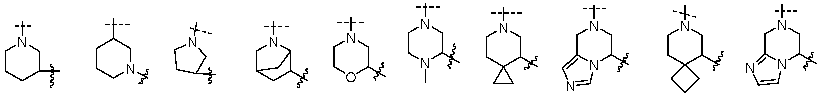 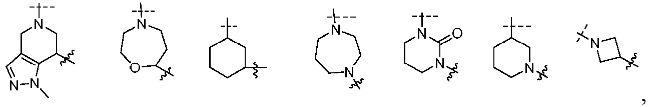
alternatively, ring Cy2 is selected from 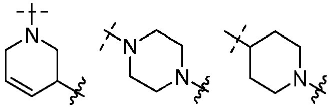 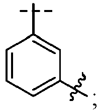
ring Cy3 is selected from 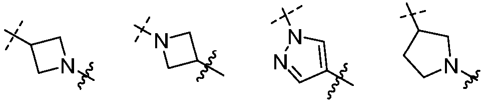 or ring Cy3 is selected from 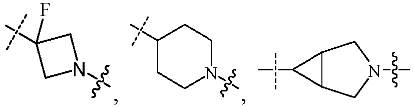 or ring Cy3 is selected from 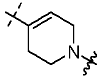 or ring Cy3 is selected from 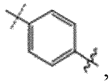 or ring Cy3 is selected from 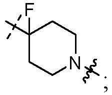
ring Cy4 is selected from 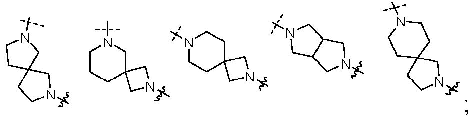
L₁ is selected from methylene, alkynylene, or -C(=O)-; further, L₁ is selected from methylene, or alkynylene;
where 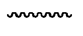 represents an attachment to the right side, andrepresents an attachment to the left side;
the definitions of other groups are consistent with those mentioned above.

The compound of formula (I) of the present invention, or a stereoisomer, a deuterated compound, a solvate, or a pharmaceutically acceptable salt or a co-crystal thereof, wherein
the ring D is selected from -Cy2-Cy3-#, -Cy2-L₁-#, -L₁-Cy2-#, or Cy4, wherein # represents the site where the ring D is attached to the ring A;
in some embodiments, ring D is selected from -Cy2-Cy3-#, -Cy2-L₁-#, or -L₁-Cy2-#, wherein # represents the site where the ring D is attached to the ring A;
in some embodiments, ring D is selected from -Cy2-Cy3-#, wherein # represents the site where the ring D is attached to the ring A;
Cy2 is selected from 4- to 7-membered monoheterocycloalkyl, 7- to 10-membered bridged heterocycloalkyl, 7- to 10-membered spiroheterocycloalkyl, 8-to 10-membered fused heterocycloalkyl, 6- or 7-membered cycloalkyl, or phenyl, and the Cy2 is optionally substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy and NH₂;
in some embodiments, Cy2 is selected from 4- to 7-membered monoheterocycloalkyl, 7- to 9-membered bridged heterocycloalkyl, 7- to 9-membered spiroheterocycloalkyl, 8- to 10-membered fused heterocycloalkyl, 6-membered cycloalkyl, or phenyl, and the Cy2 is optionally substituted with 1 to 3 groups selected from =O, F, Cl, Br, deuterium, CN, OH, C₁₋₂ alkyl, haloC₁₋₂ alkyl, deuterated C₁₋₂ alkyl, C₁₋₂ alkoxy, and NH₂;
Cy3 is selected from 4- to 7-membered monoheterocycloalkyl, 5- or 6-membered heteroaryl, 6- to 10-membered fused heterocycloalkyl, or phenyl, and the Cy3 is optionally substituted with 1 to 3 groups selected from =O, halogen, deuterium, CN, OH, C₁₋₂ alkyl and NH₂;
in some embodiments, Cy3 is selected from 4- to 7-membered monoheterocycloalkyl, 5- or 6-membered heteroaryl, 6- to 8-membered fused heterocycloalkyl, or phenyl, and the Cy3 is optionally substituted with 1 to 3 groups selected from =O, F, Cl, Br, deuterium, CN, OH, methyl, ethyl and NH₂;
L₁ is selected from methylene, ethylene, vinylene, ethynylene, or -C(=O)-;
The compound of formula (I) of the present invention, or a stereoisomer, a deuterated compound, a solvate, or a pharmaceutically acceptable salt or a co-crystal thereof, wherein
the ring D is selected from 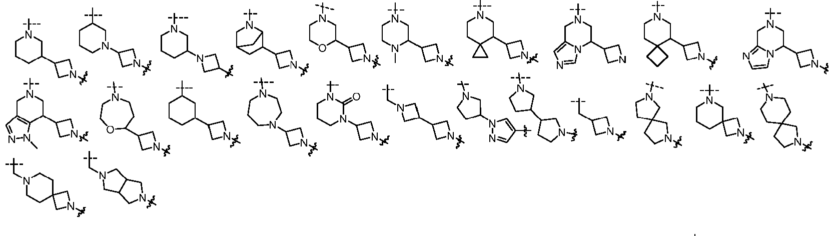 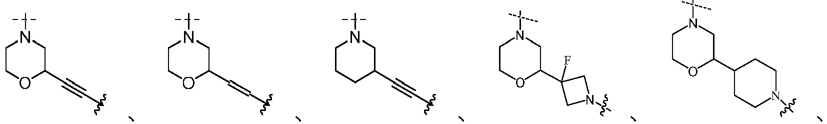 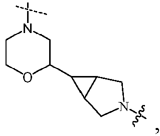 or ring D is selected from 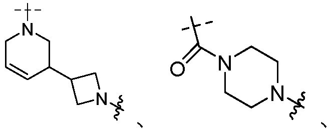 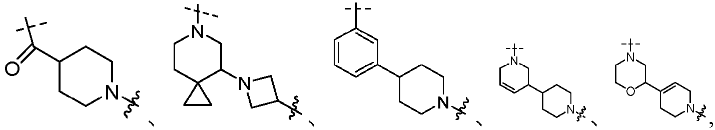 or ring D is selected from 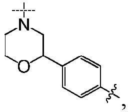 or ring D is selected from 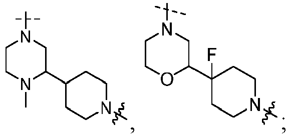
where 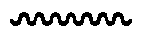 represents an attachment to the right side, andrepresents an attachment to the left side;
other groups are consistent with those mentioned above.

The compound of formula (I) of the present invention, or a stereoisomer, a deuterated compound, a solvate, or a pharmaceutically acceptable salt or a co-crystal thereof, wherein
the ring D is selected from 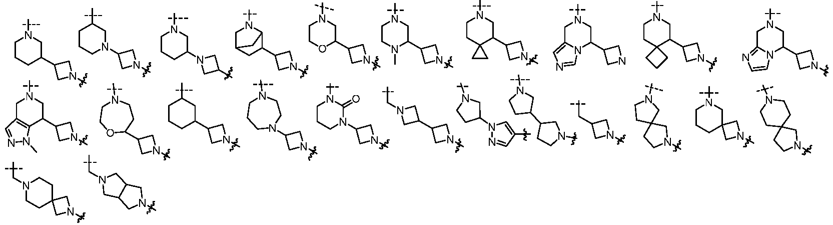 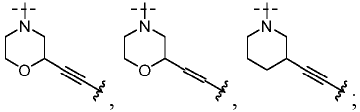 where 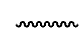 represents an attachment to the right side, andrepresents an attachment to the left side;
other groups are consistent with those mentioned above.

The compound of formula (I) of the present invention, or a stereoisomer, a deuterated compound, a solvate, or a pharmaceutically acceptable salt or a co-crystal thereof, wherein
the ring D is selected from 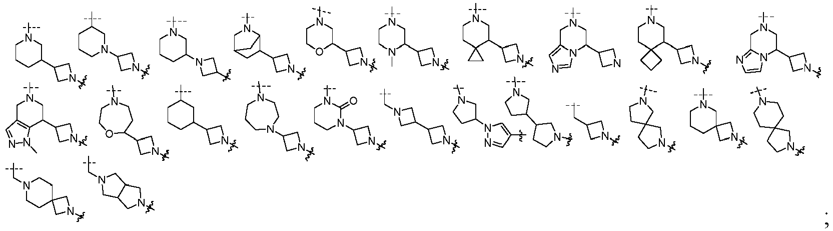
where 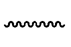 represents an attachment to the right side, andrepresents an attachment to the left side;
other groups are consistent with those mentioned above.

The compound of formula (I) of the present invention, or a stereoisomer, a deuterated compound, a solvate, or a pharmaceutically acceptable salt or a co-crystal thereof, wherein
Cy1 is selected from 3- to 6-membered monocyclic cycloalkyl, 7- to 10-membered bicyclic cycloalkyl, 4- to 6-membered monoheterocycloalkyl, or 7- to 10-membered bicyclic heterocycloalkyl, and the cycloalkyl and heterocycloalkyl are optionally further substituted with 1 to 3 groups selected from C₁₋₄ alkyl, halogen, deuterium, cyano, nitro, OH, haloC₁₋₄ alkyl, and deuterated C₁₋₄ alkyl;
the definitions of other groups are consistent with those mentioned above.
The compound of formula (I) of the present invention, or a stereoisomer, a deuterated compound, a solvate, or a pharmaceutically acceptable salt or a co-crystal thereof, wherein
R³ is selected from 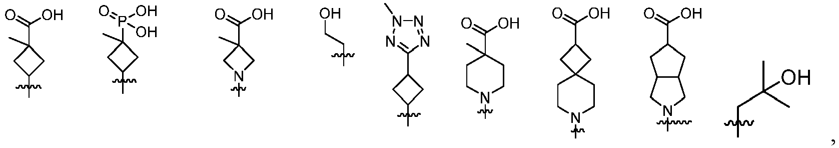 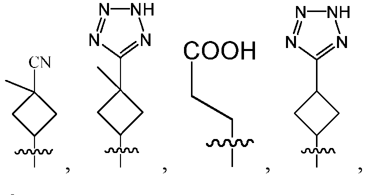 or R³ is selected from 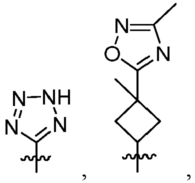 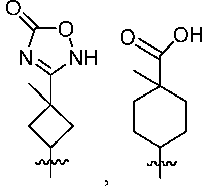 , or R³ is selected from 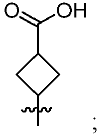
the definitions of other groups are consistent with those mentioned above.

The compound of formula (I) of the present invention, or a stereoisomer, a deuterated compound, a solvate, or a pharmaceutically acceptable salt or a co-crystal thereof, wherein
R³ is selected from 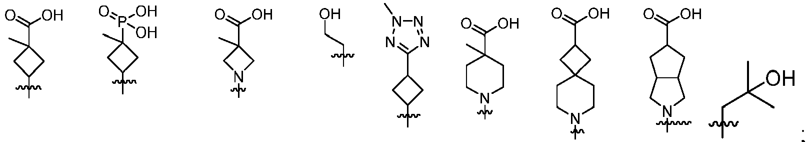
the definitions of other groups are consistent with those mentioned above.

The compound of formula (I) of the present invention, or a stereoisomer, a deuterated compound, a solvate, or a pharmaceutically acceptable salt or a co-crystal thereof, wherein
R³ is selected from 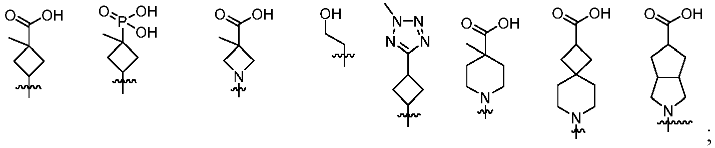
the definitions of other groups are consistent with those mentioned above.

The compound of the present invention, or a stereoisomer, a deuterated compound, a solvate, or a pharmaceutically acceptable salt or a co-crystal thereof, the compound is selected from one of the structures in Table 1 below:

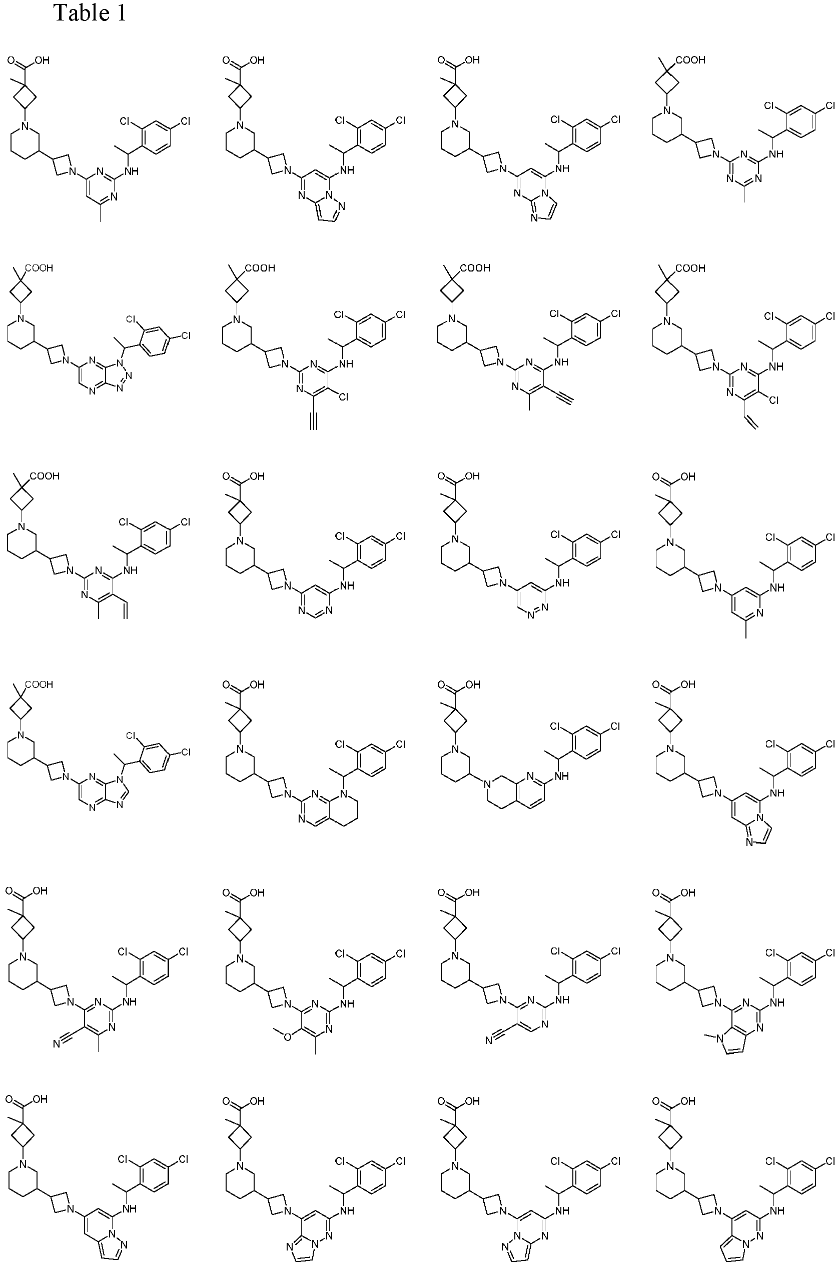

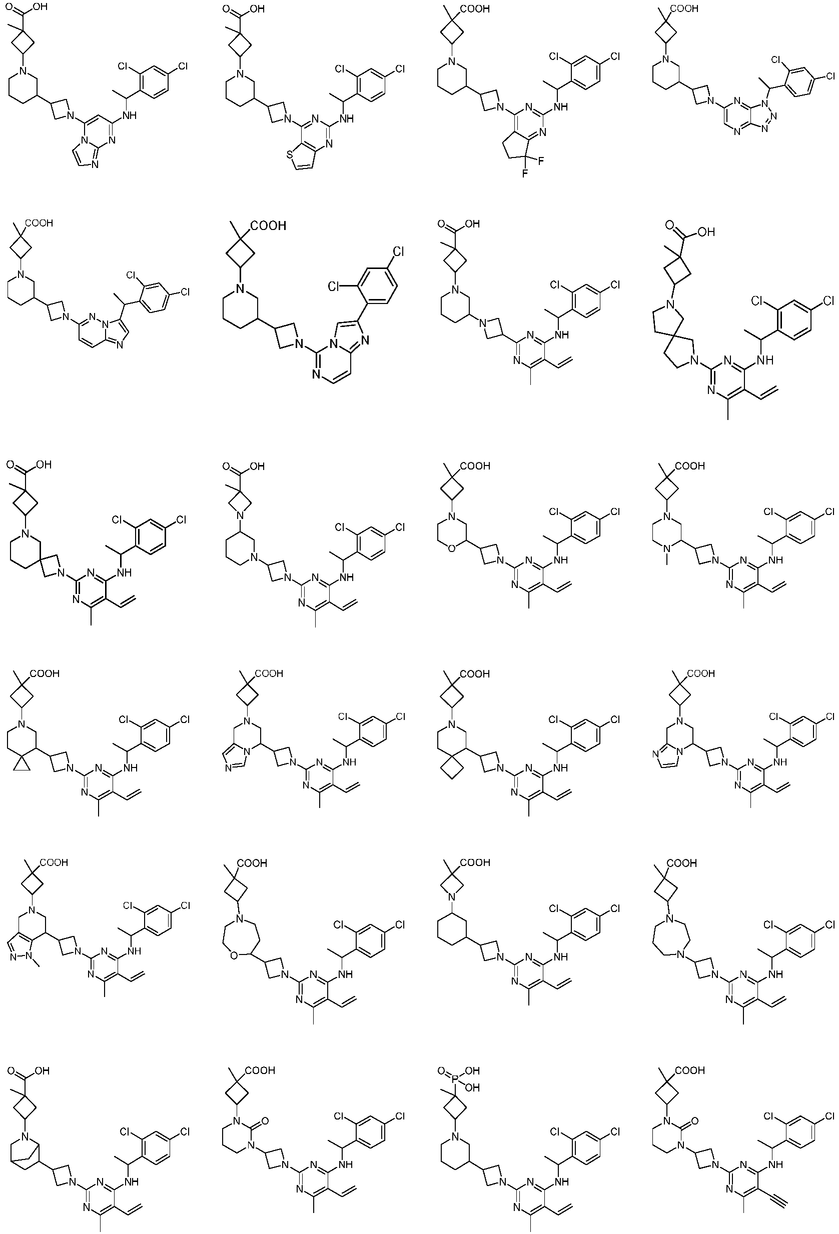

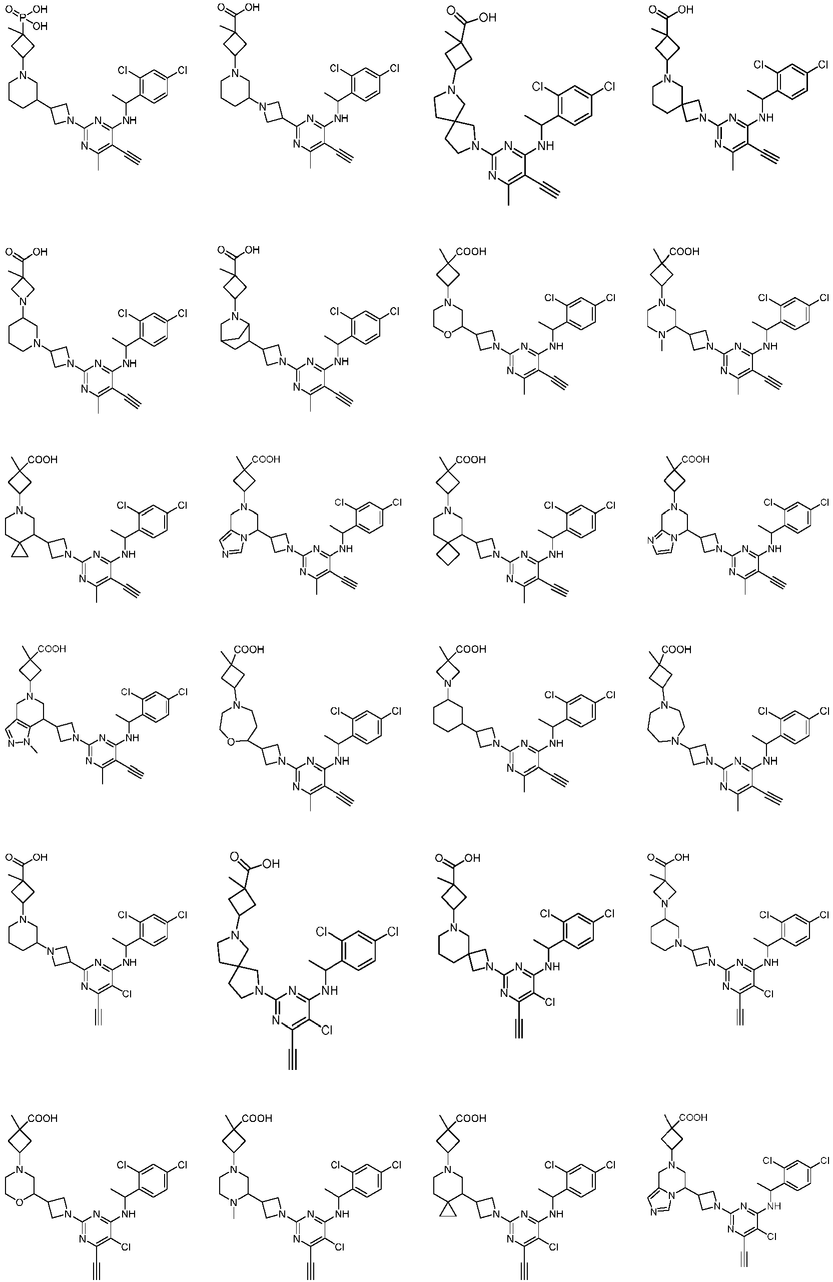

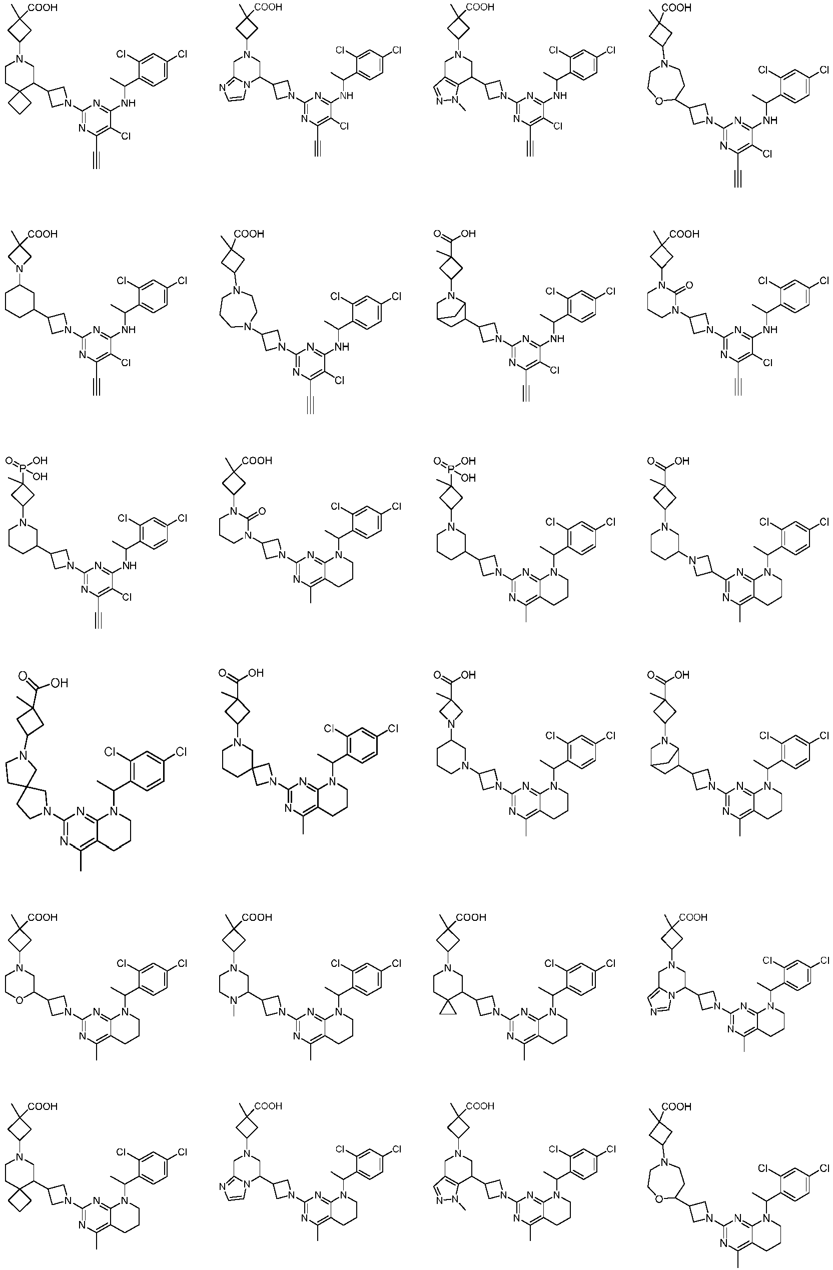

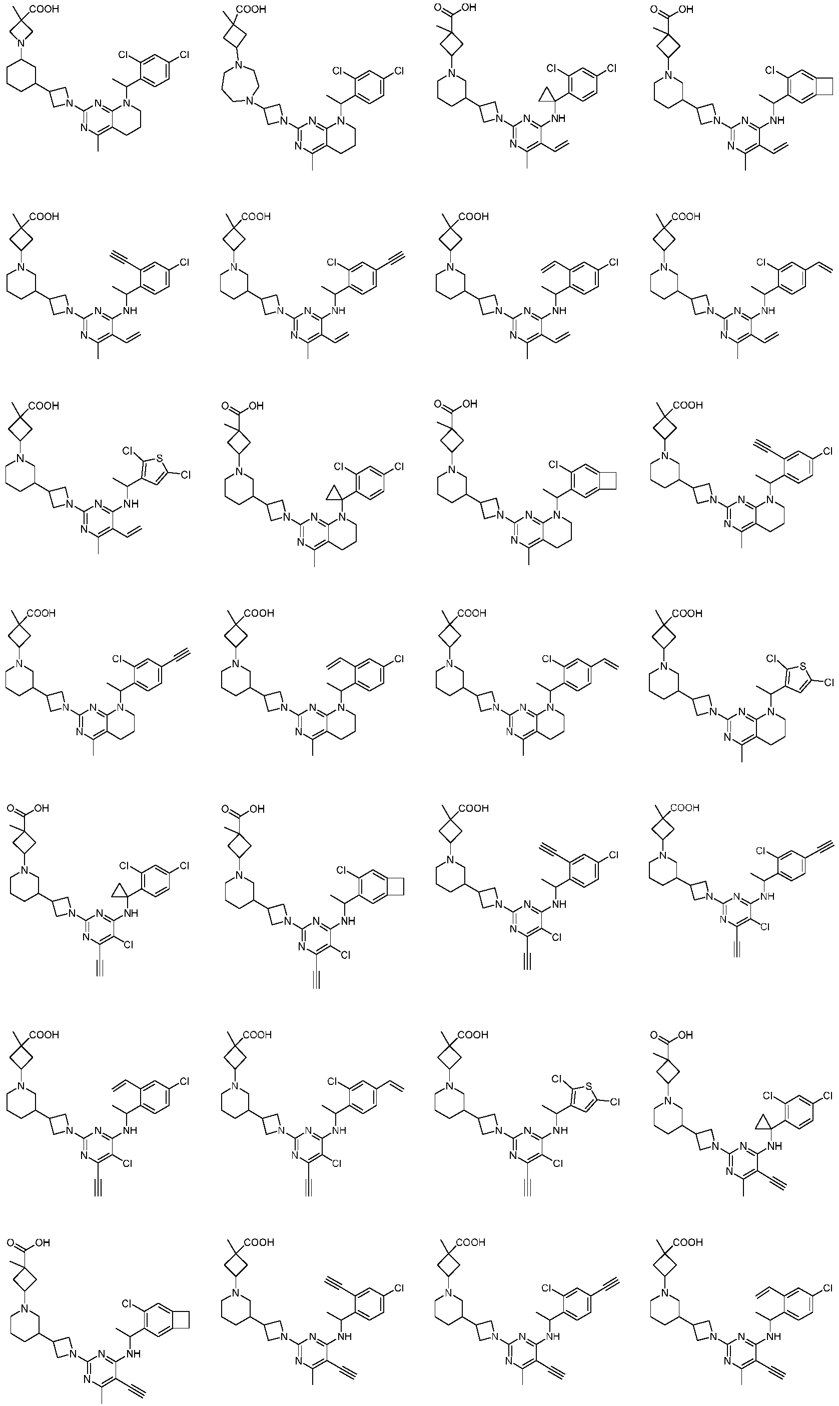

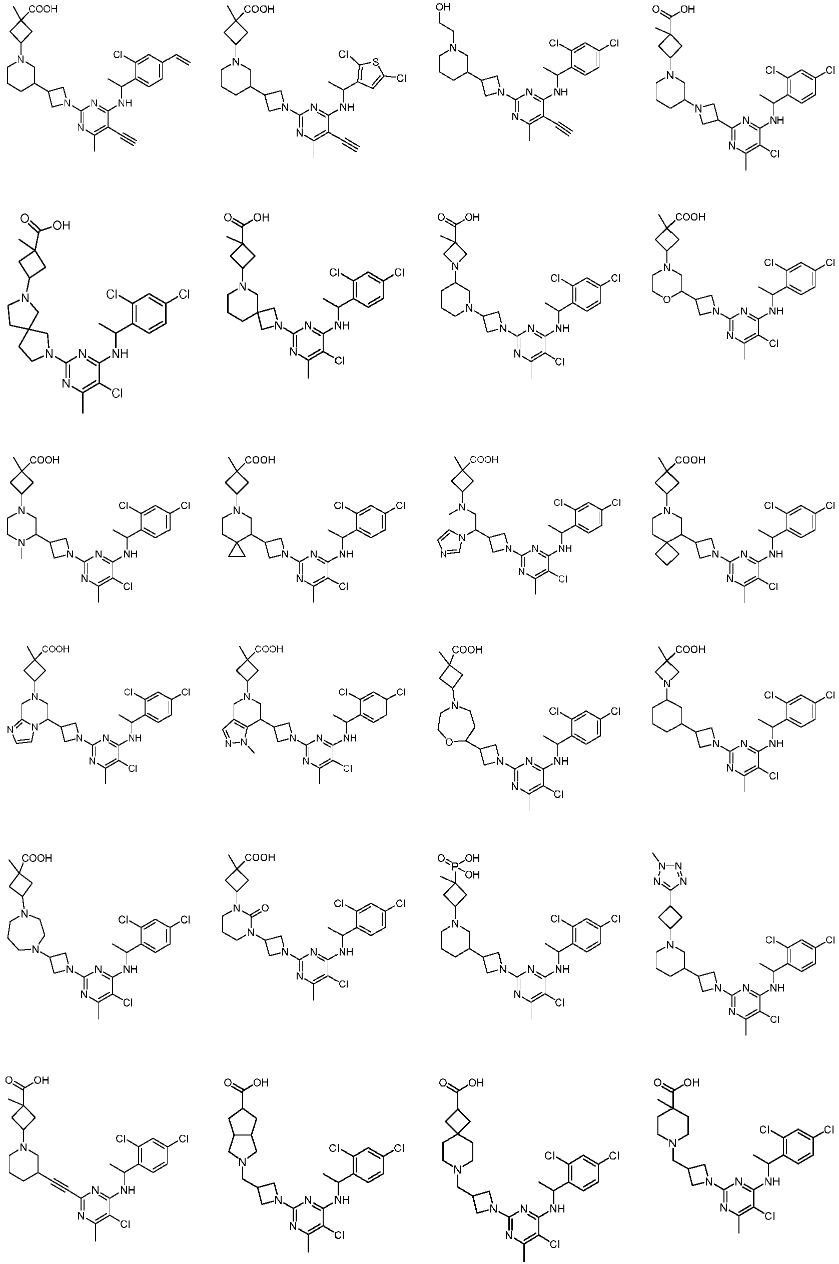

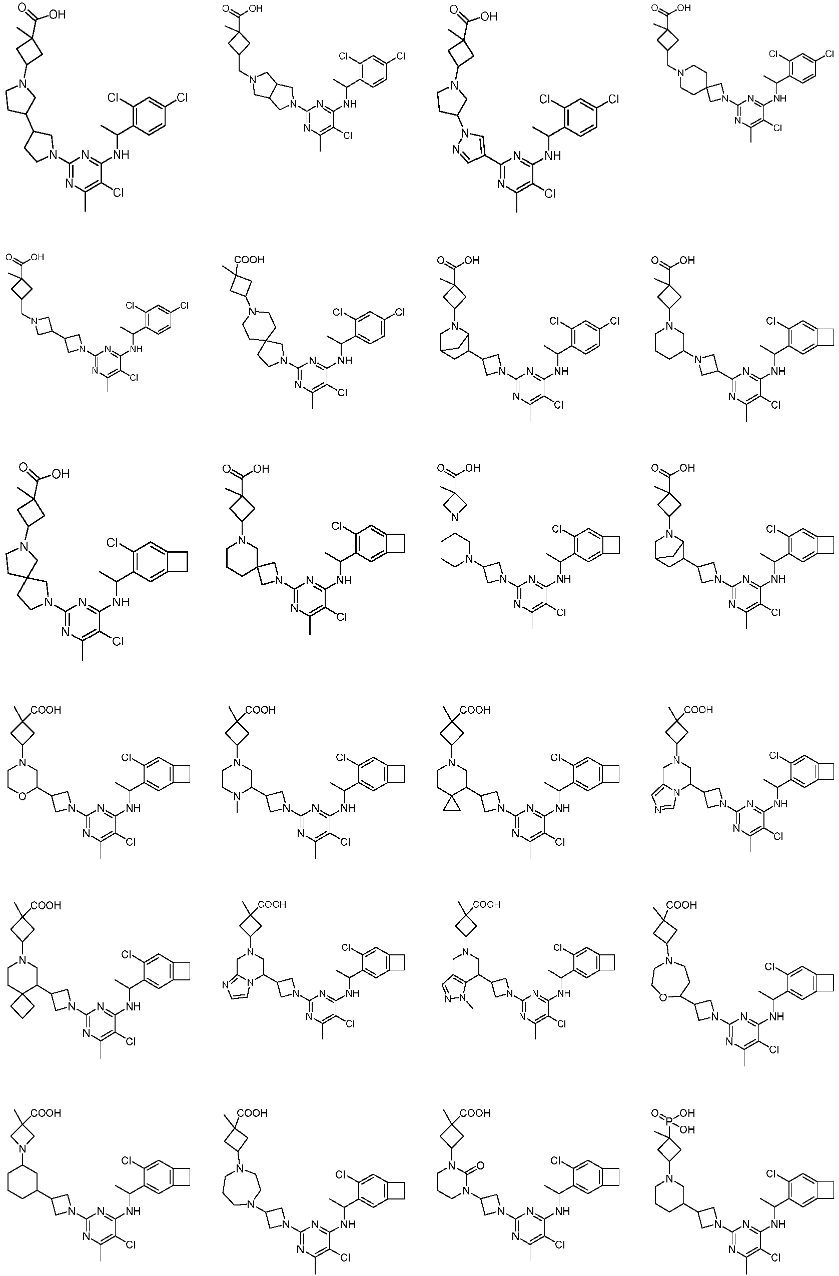

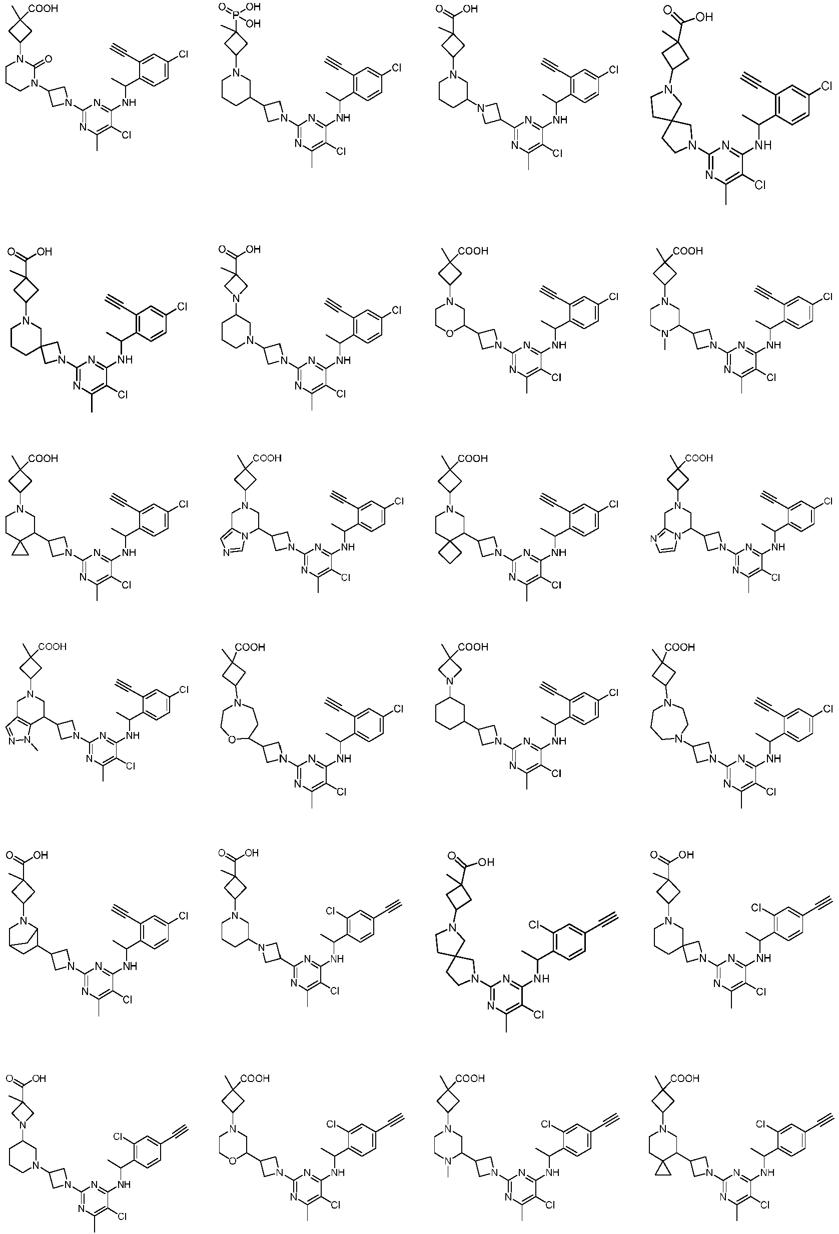

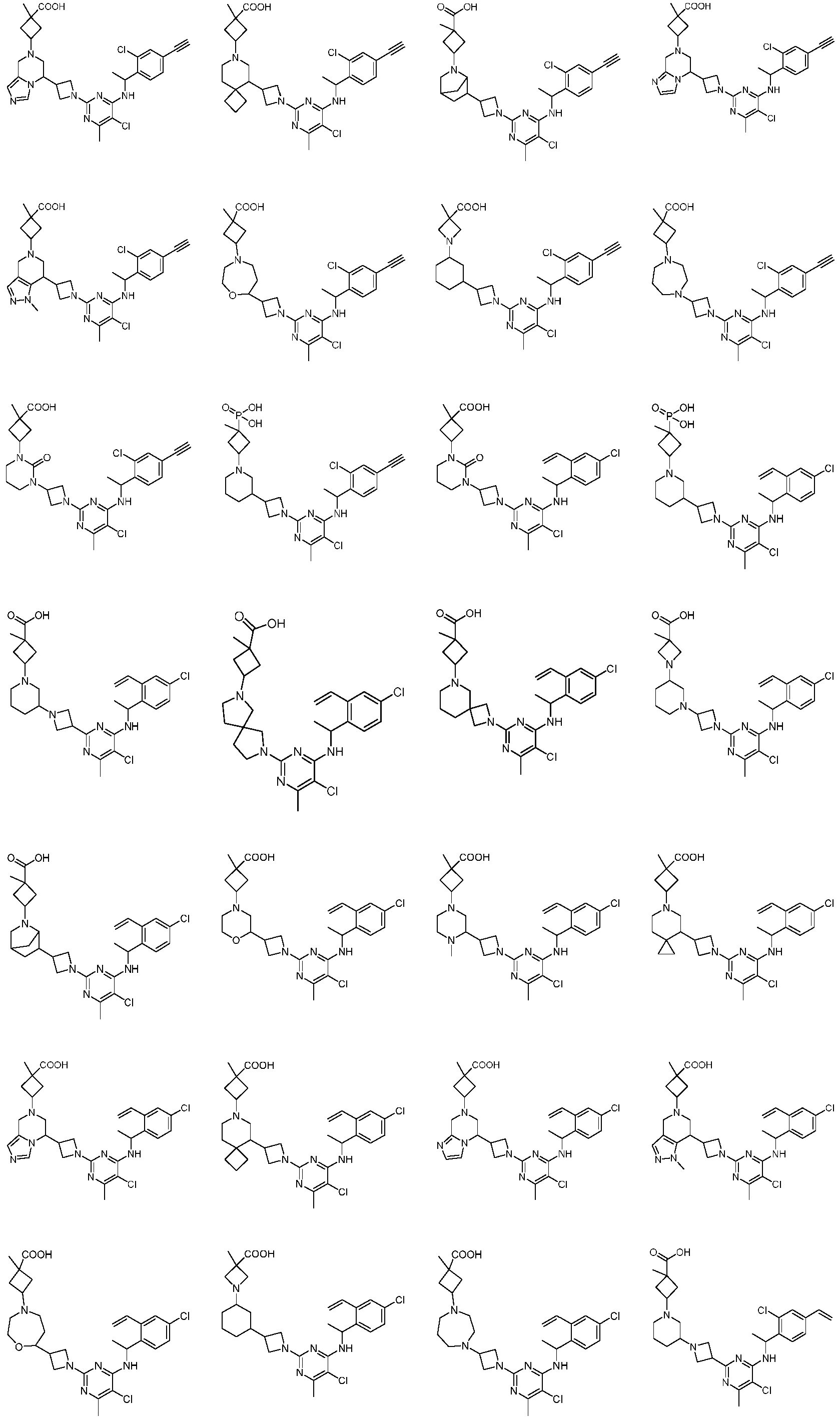

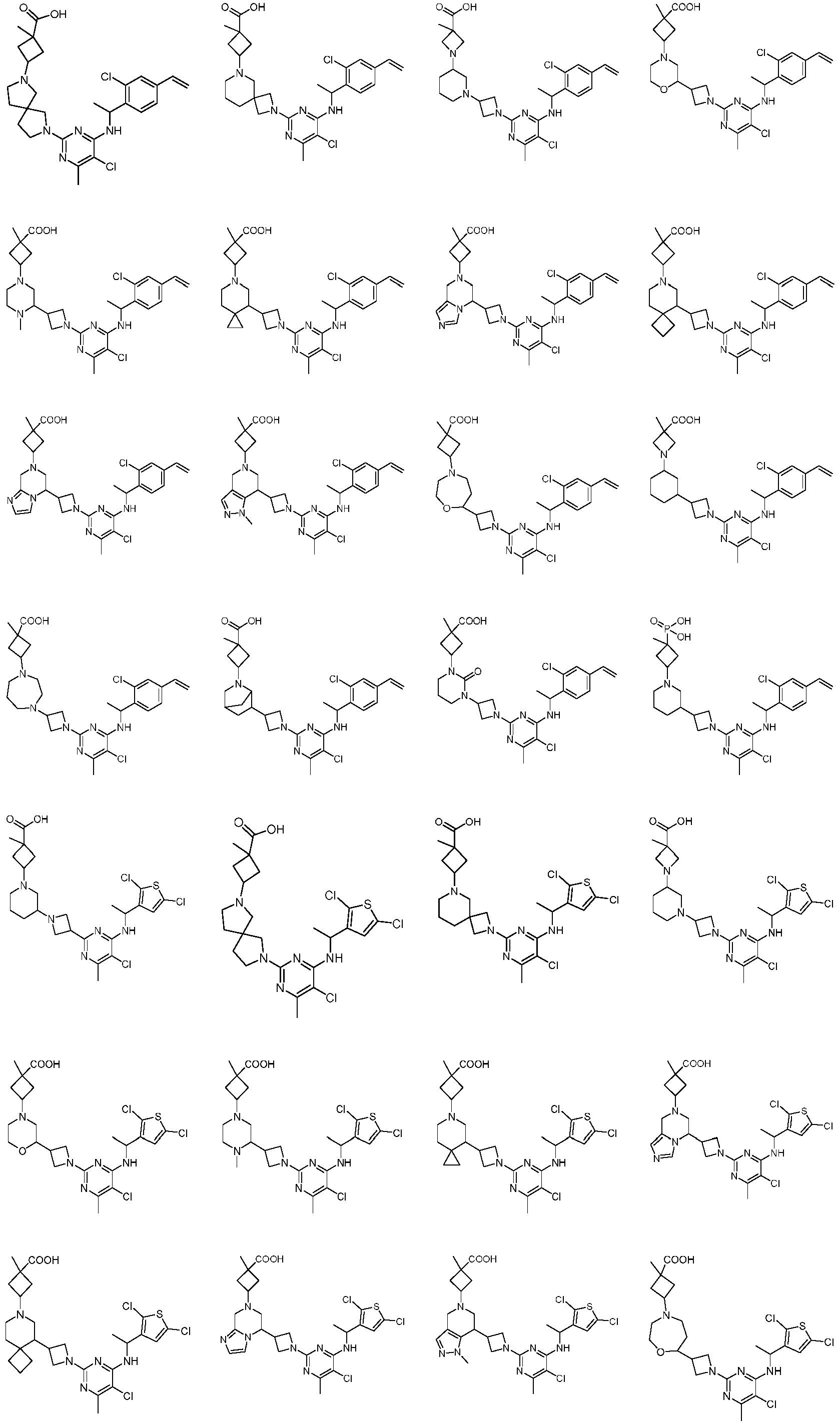

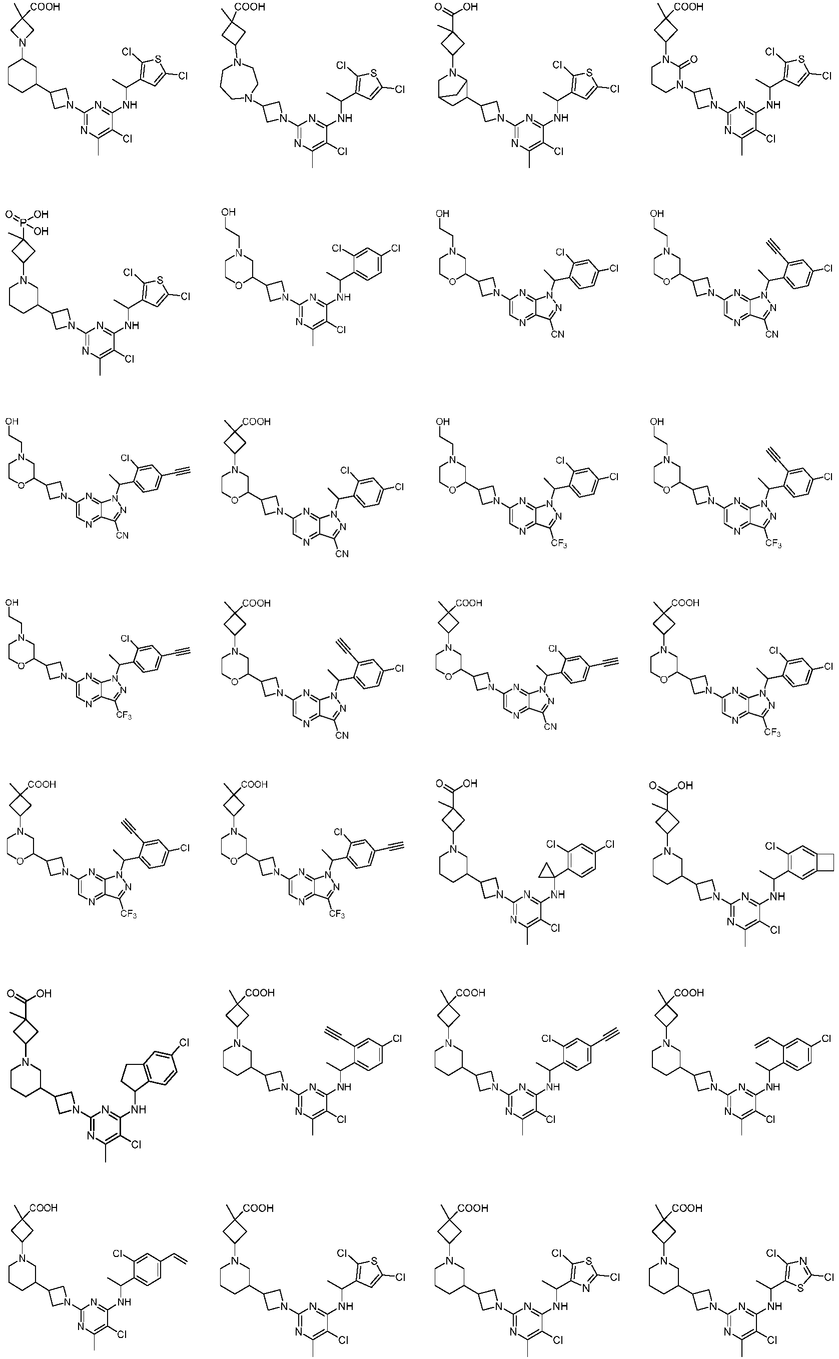

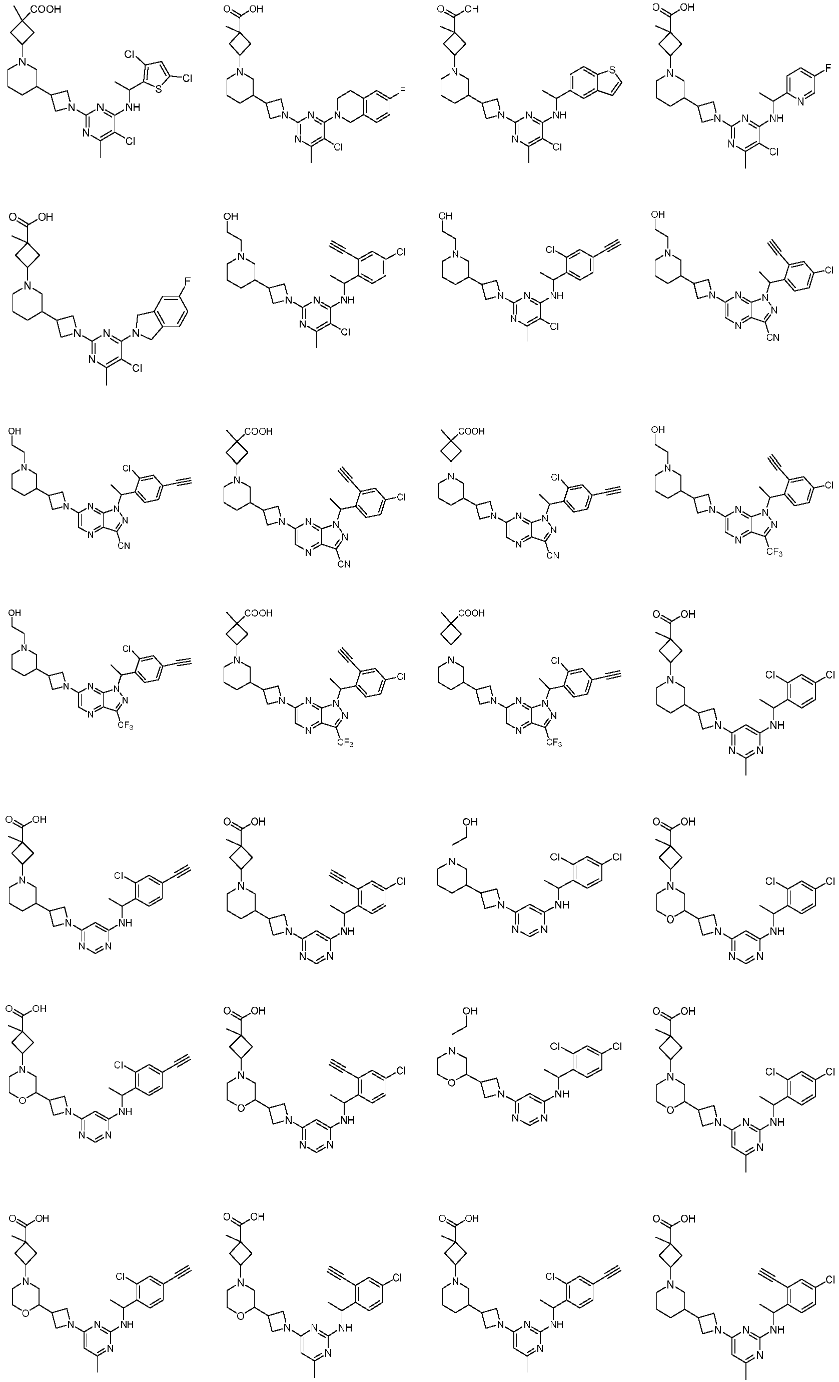

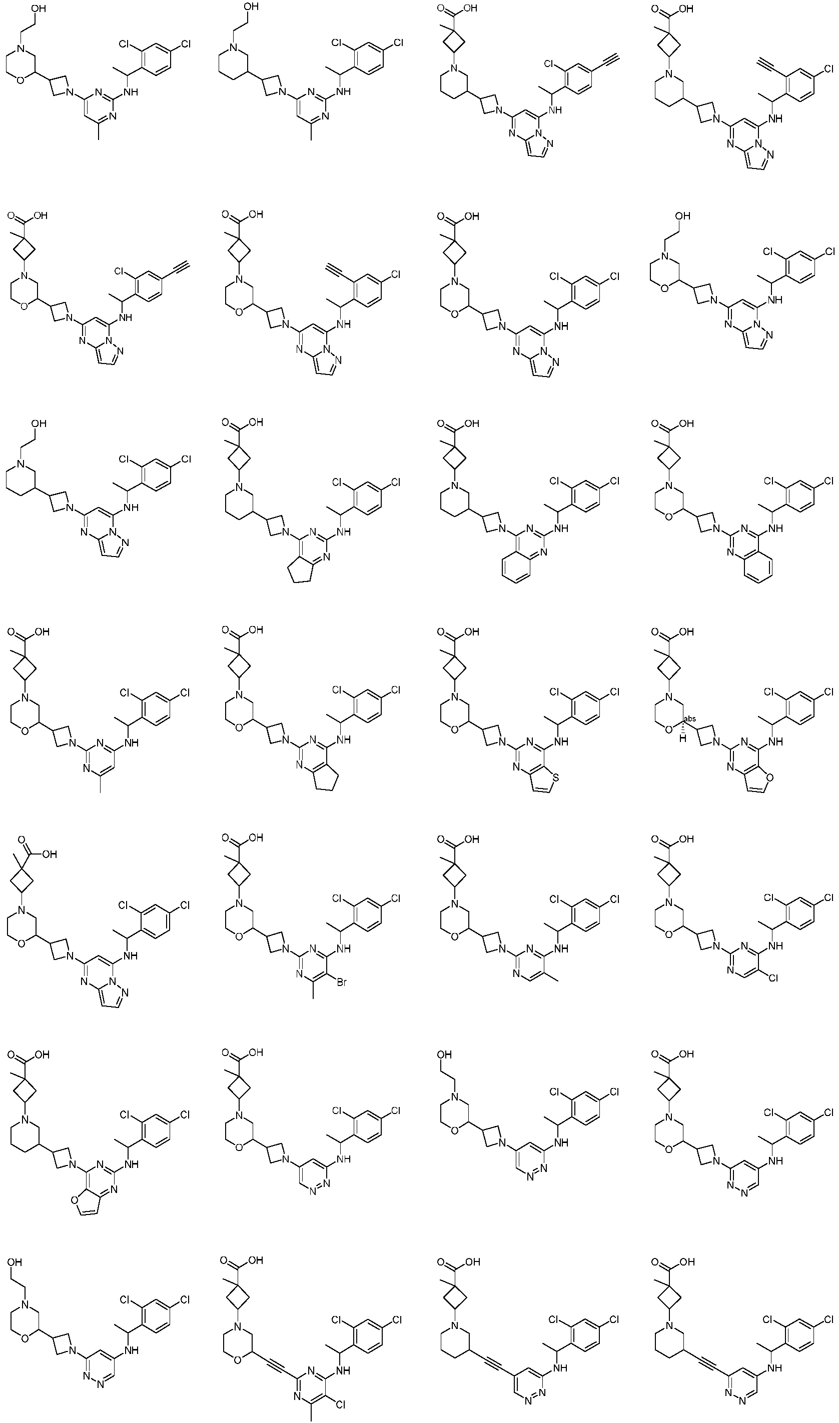

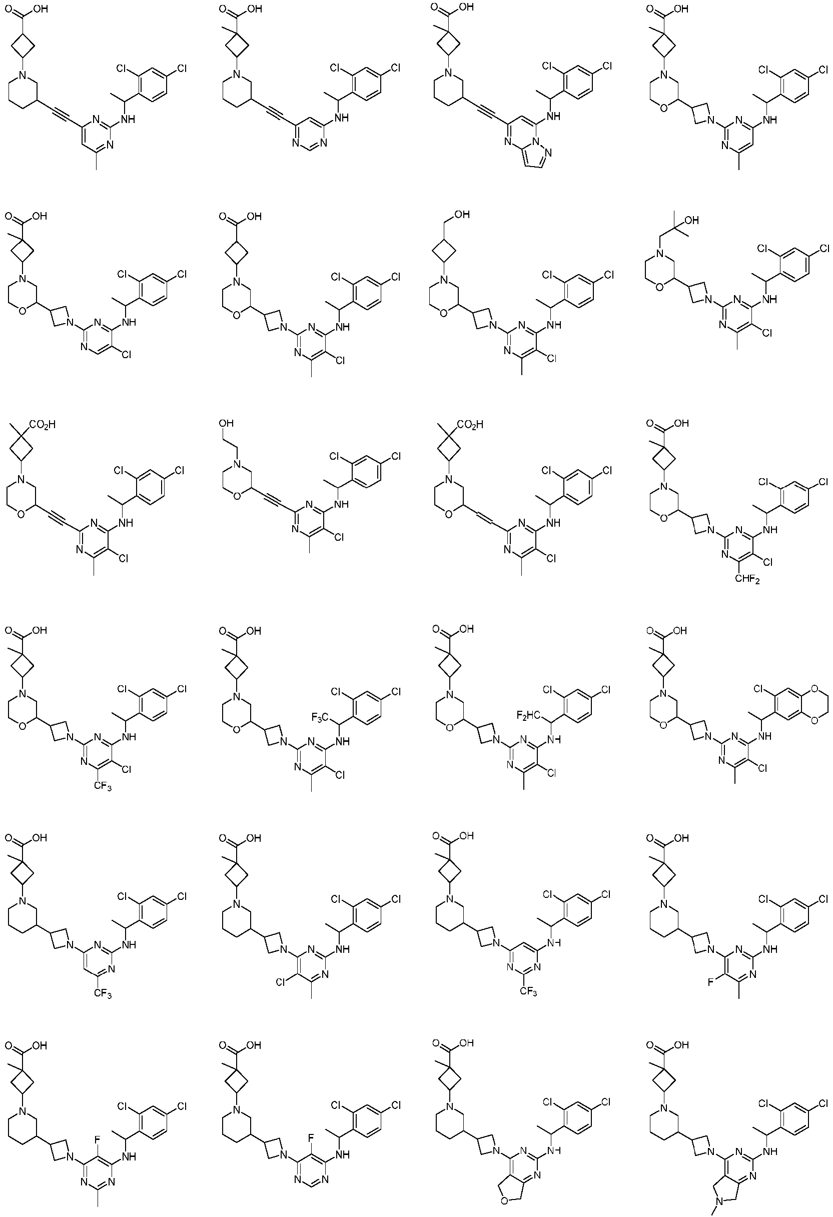

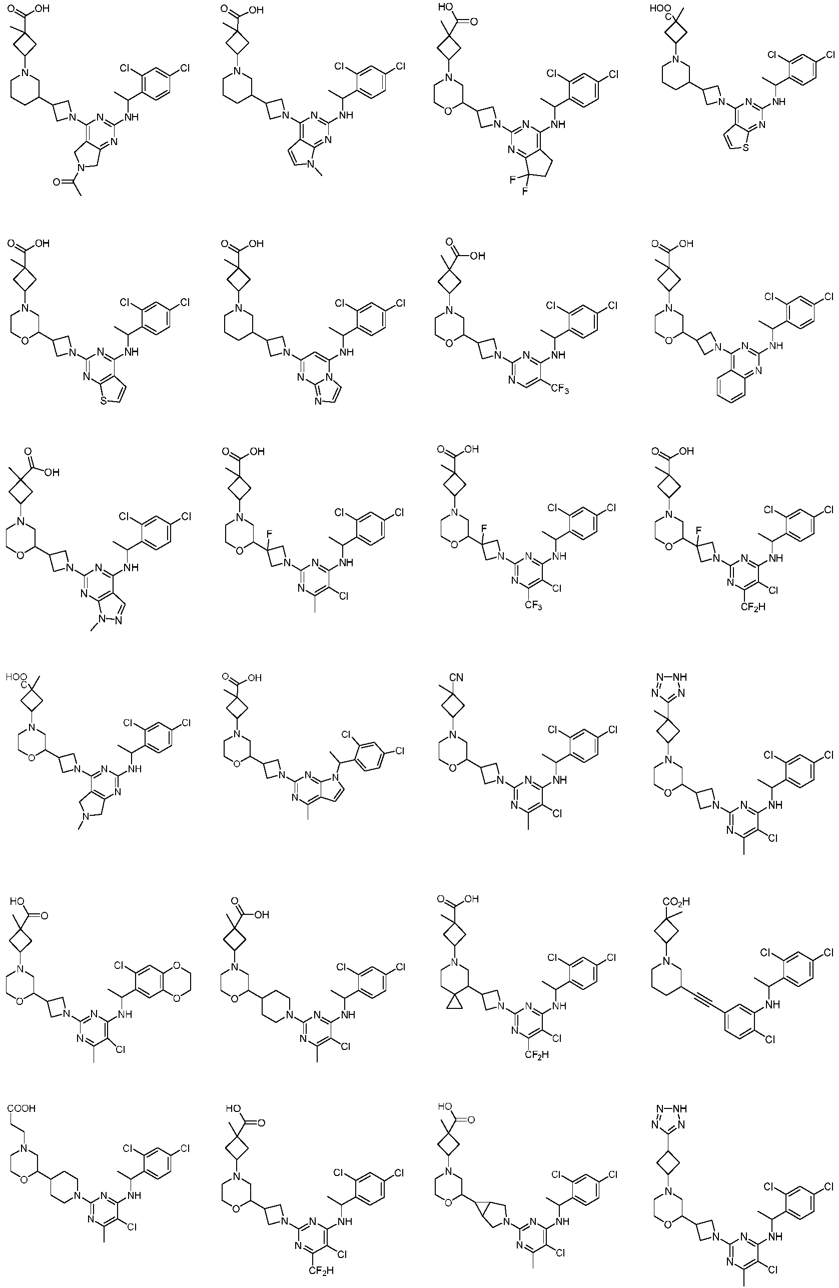

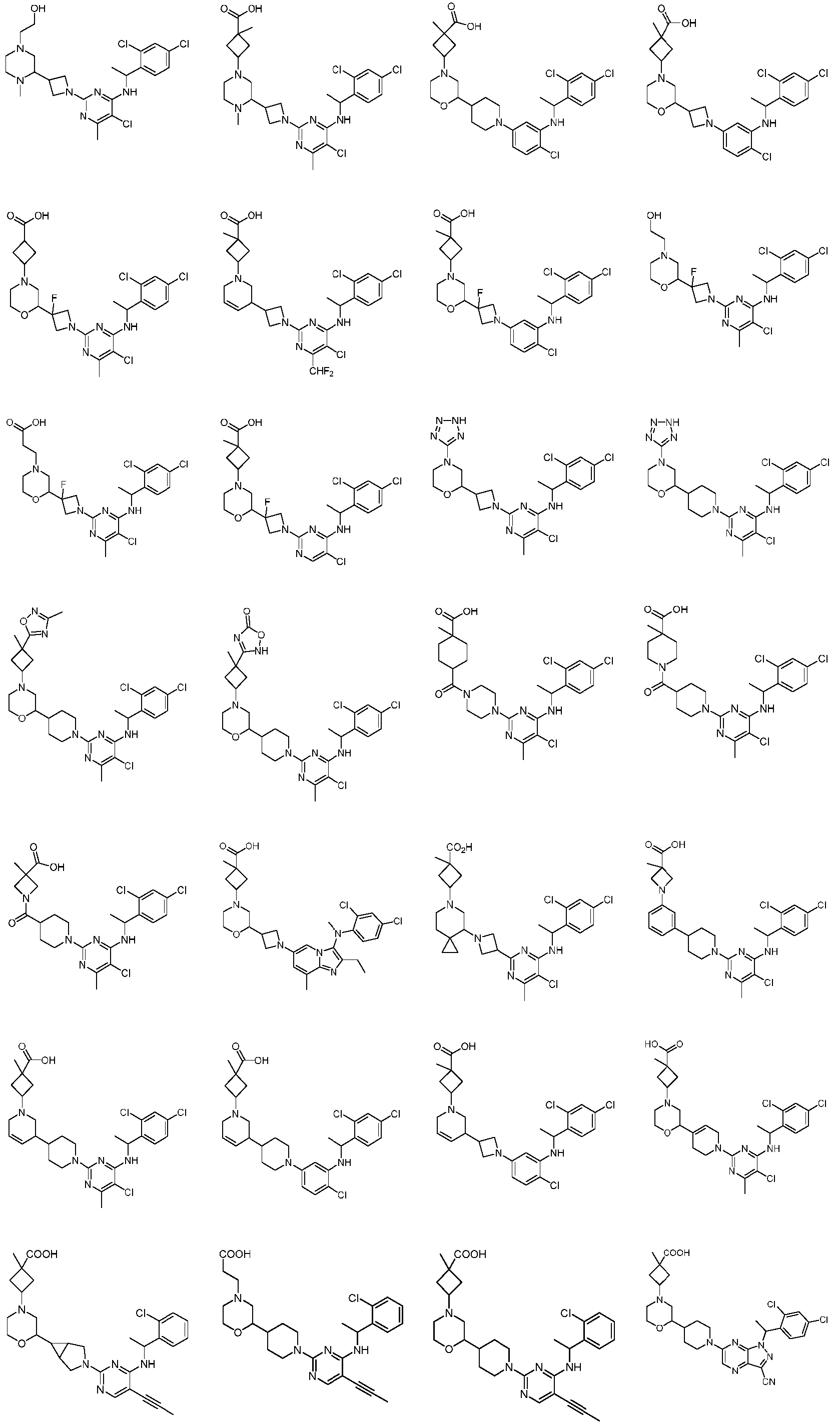

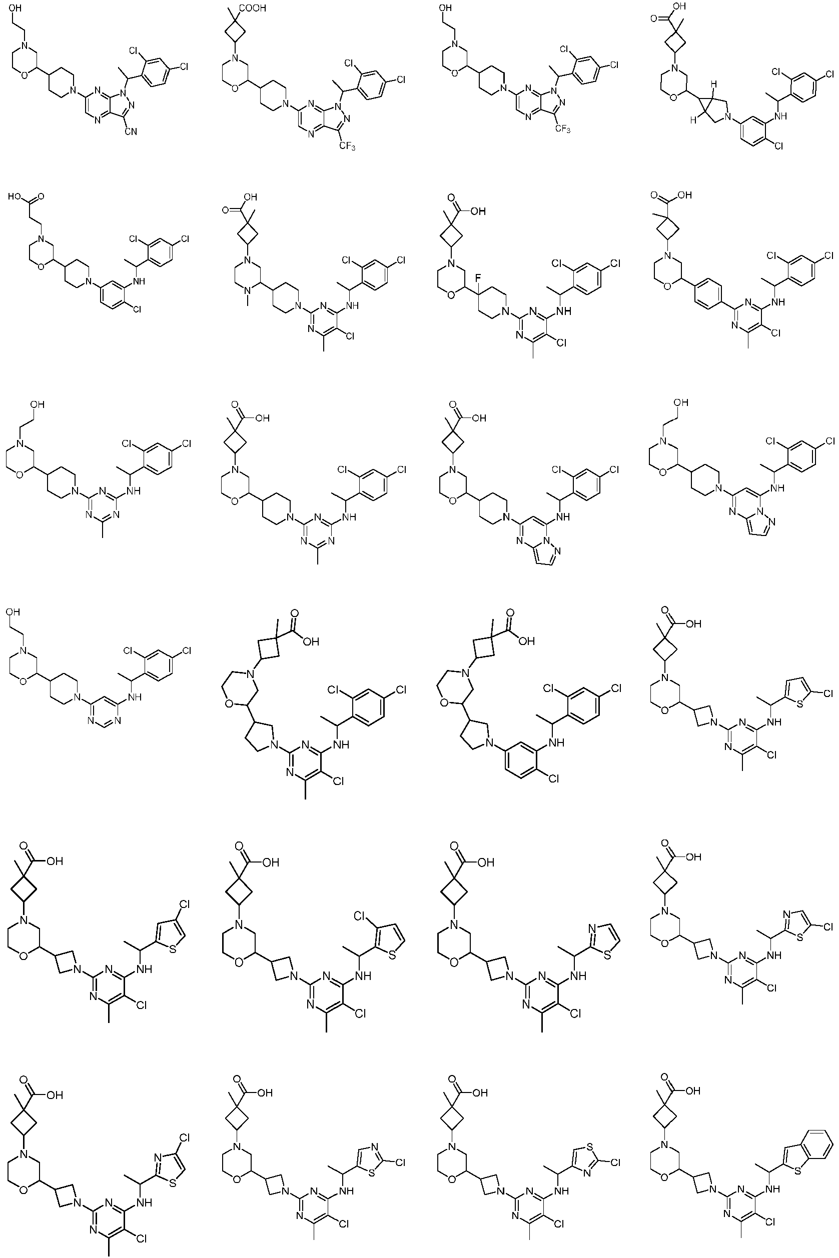

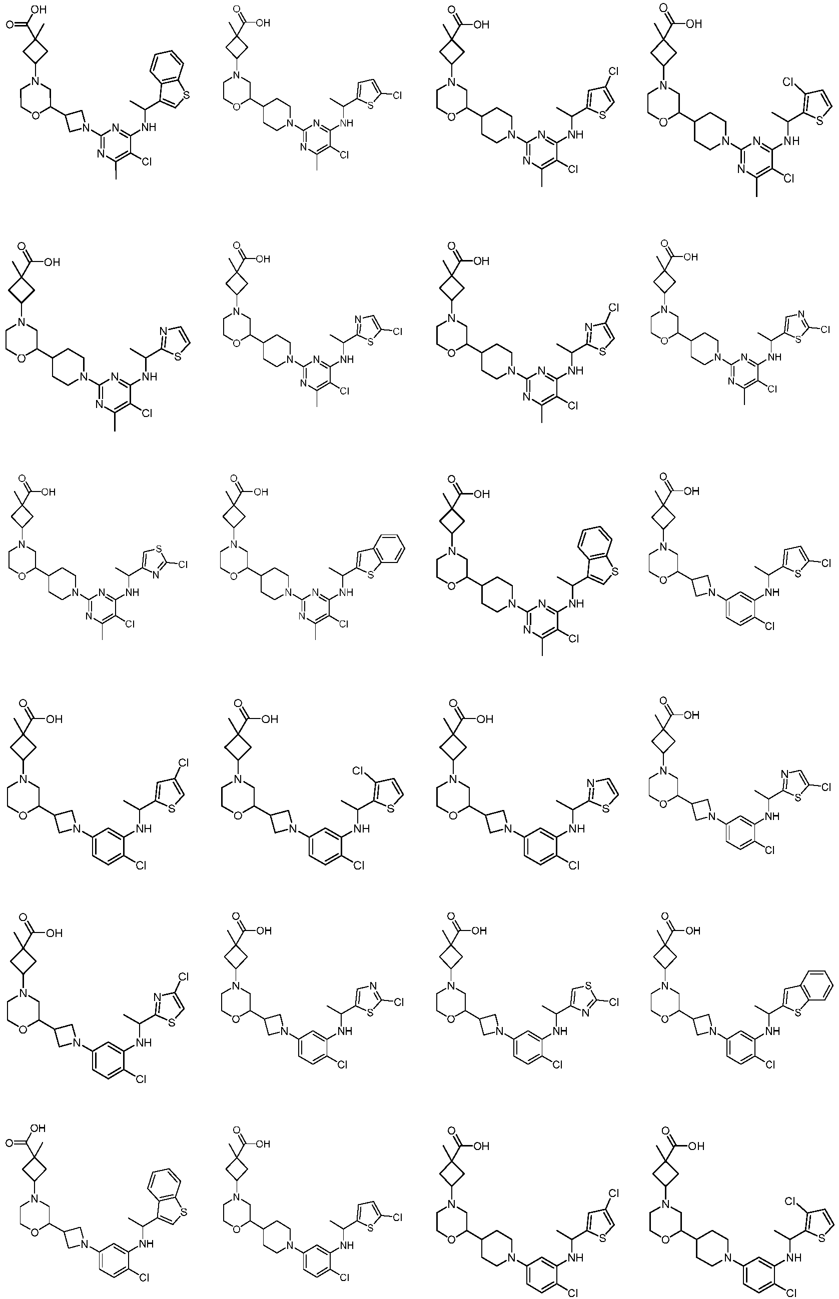

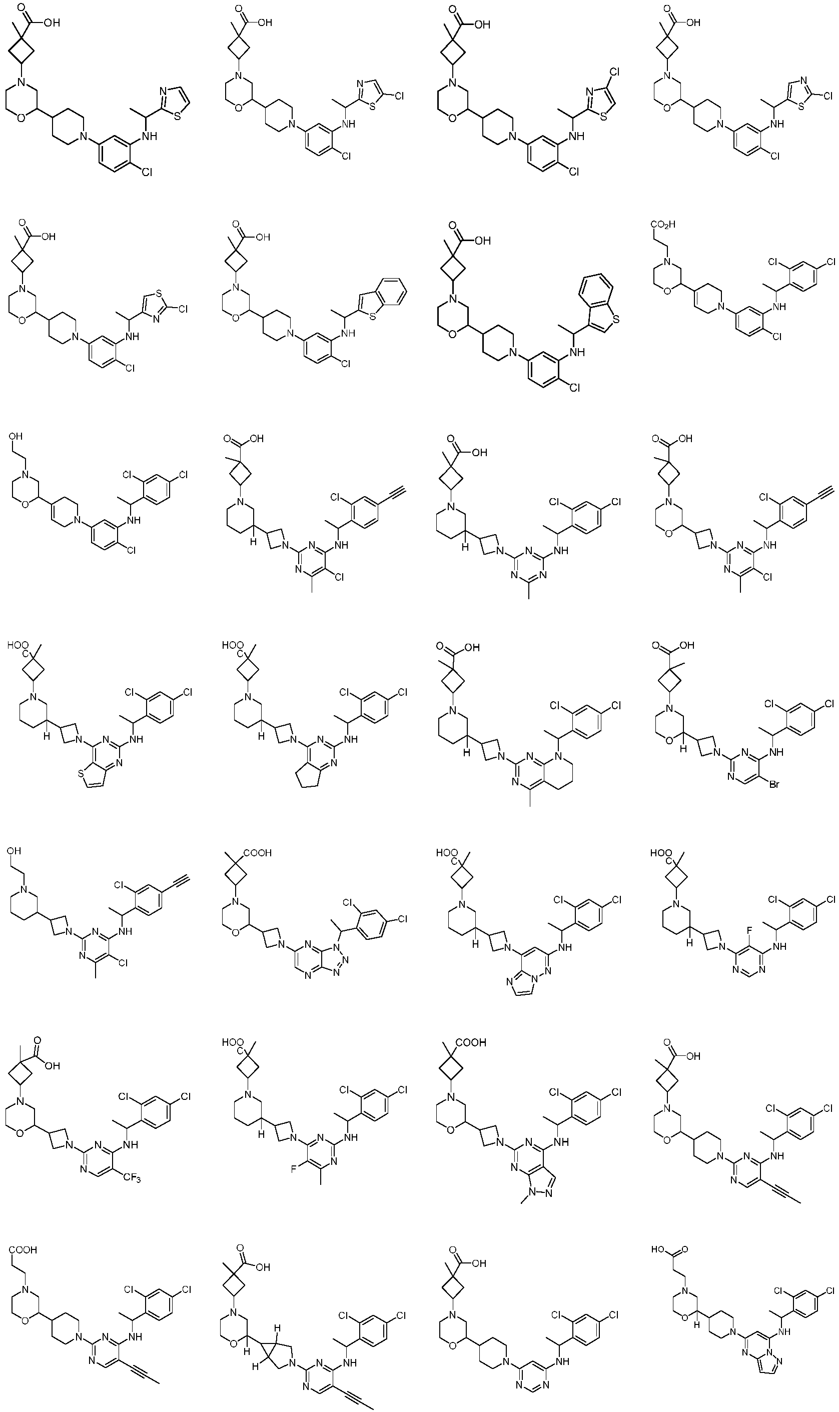

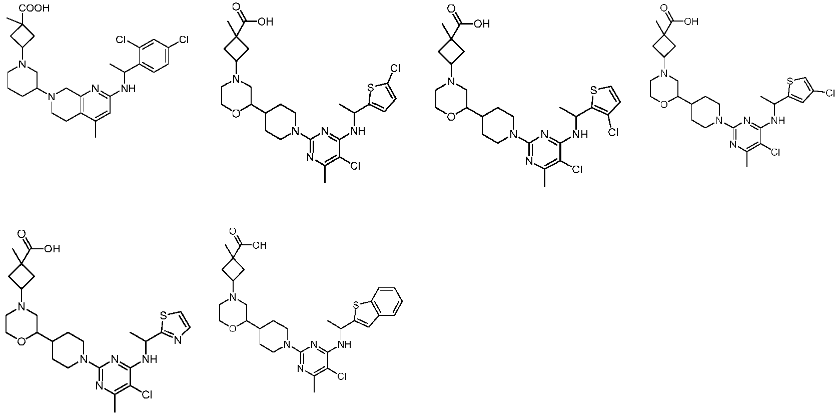

The compound of the present invention, or a stereoisomer, a deuterated compound, a solvate, or a pharmaceutically acceptable salt or a co-crystal thereof, the compound is selected from one of the structures in Table 2 below.

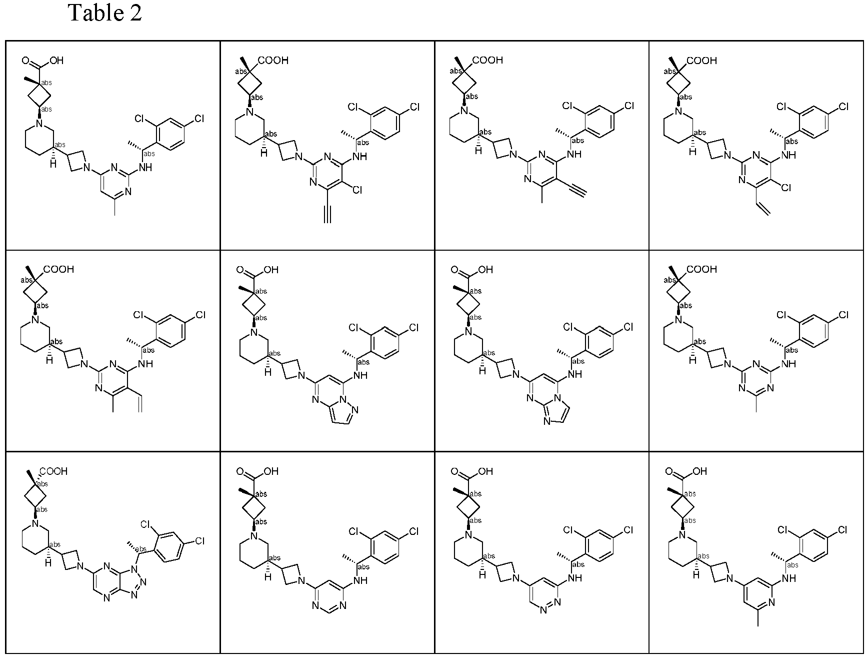

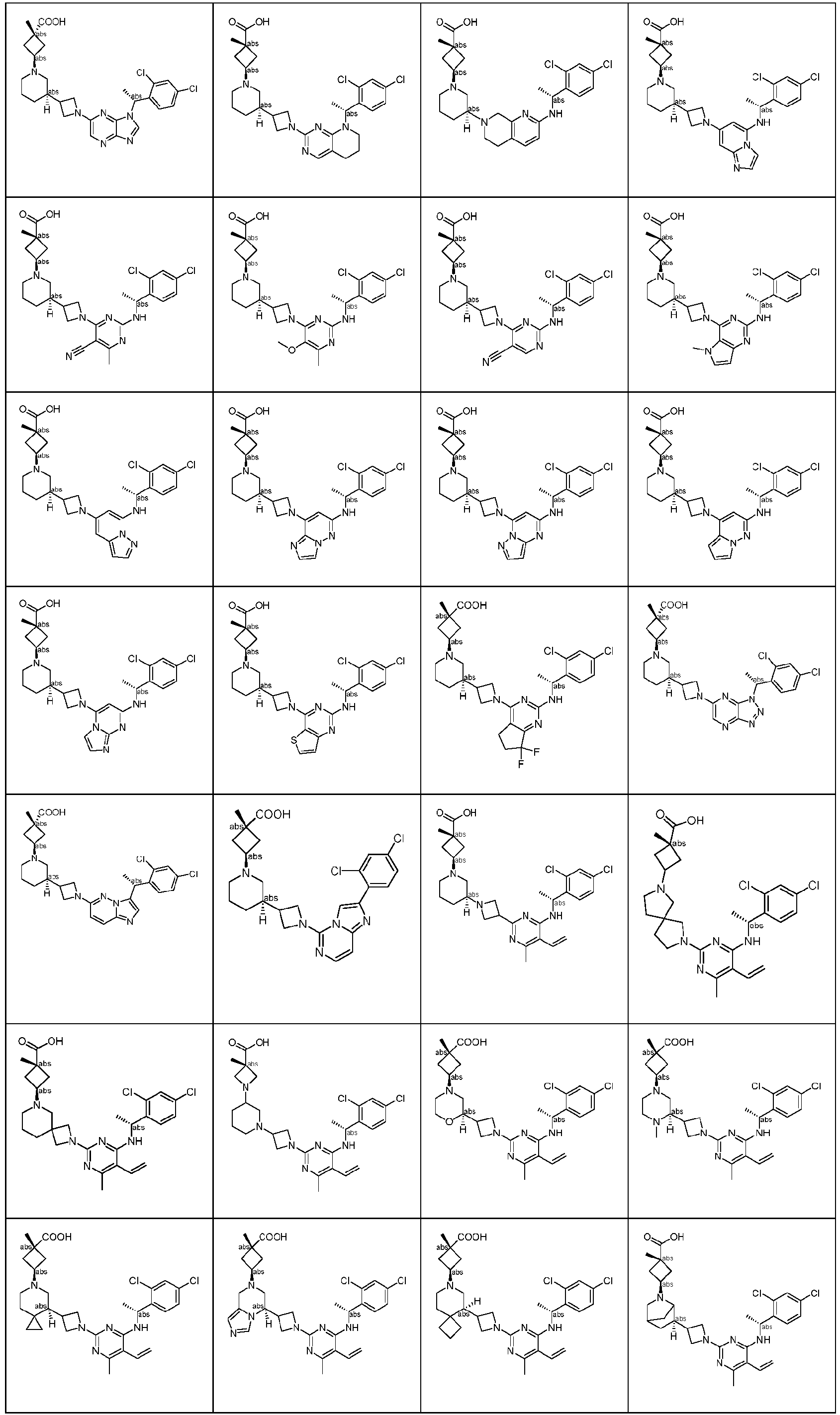

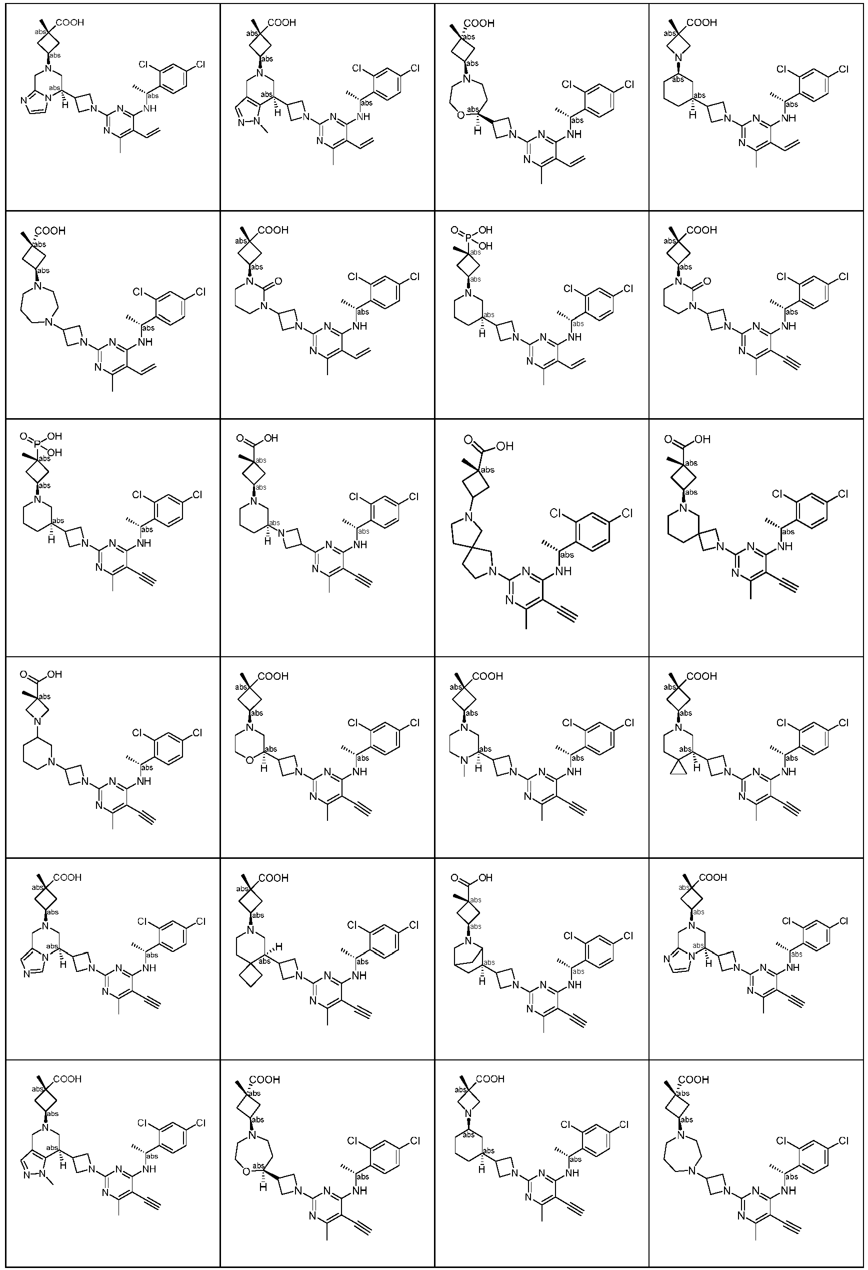

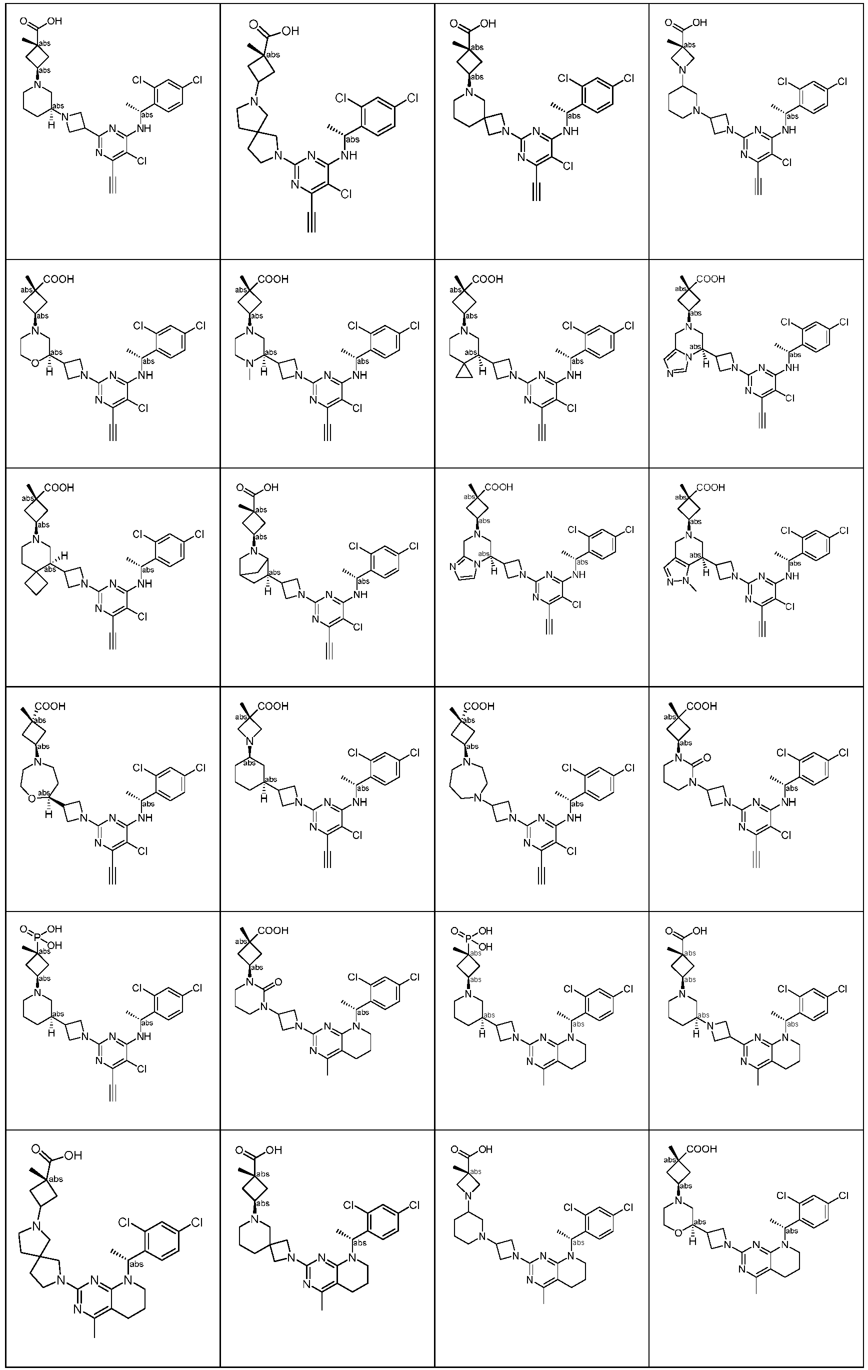

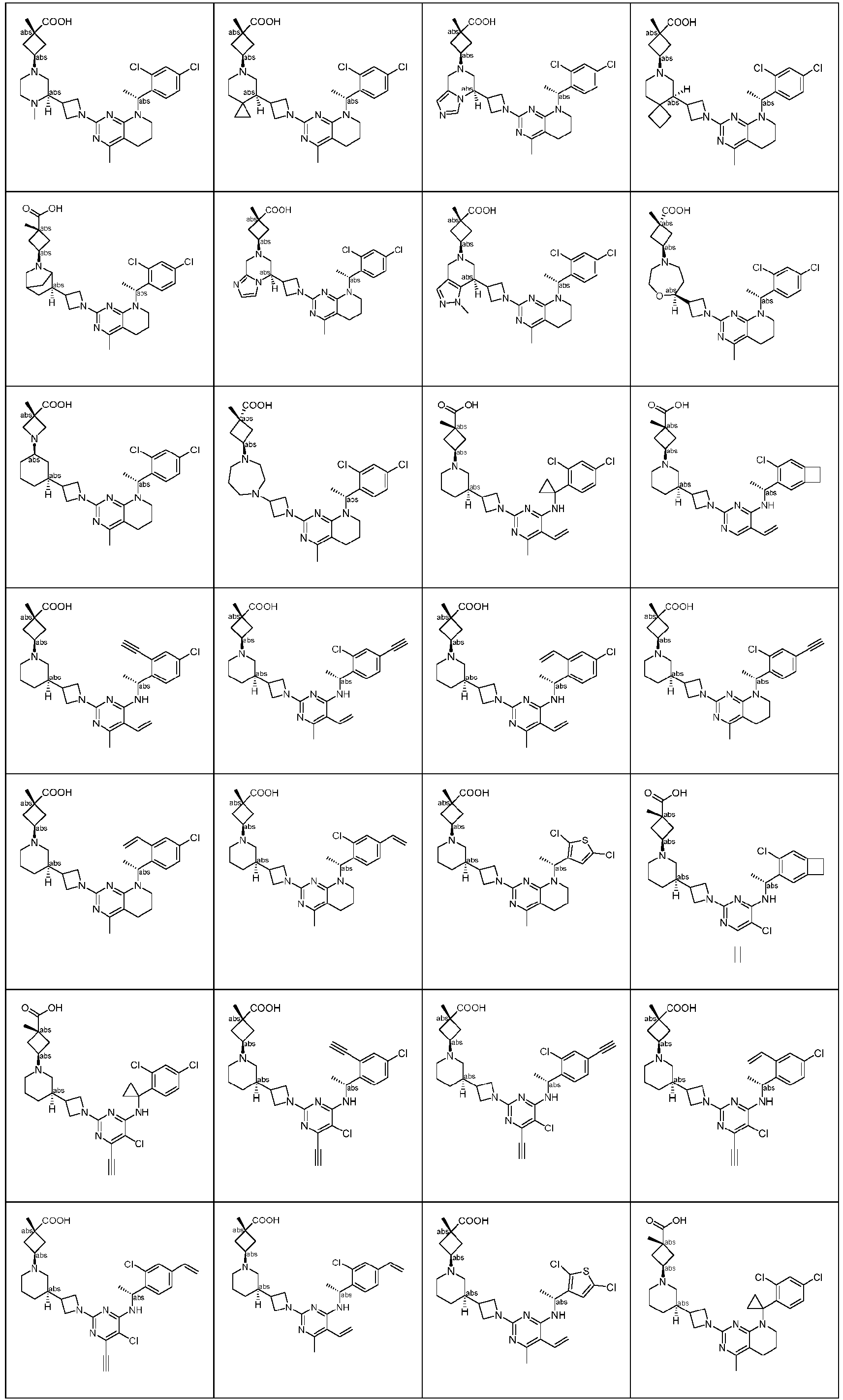

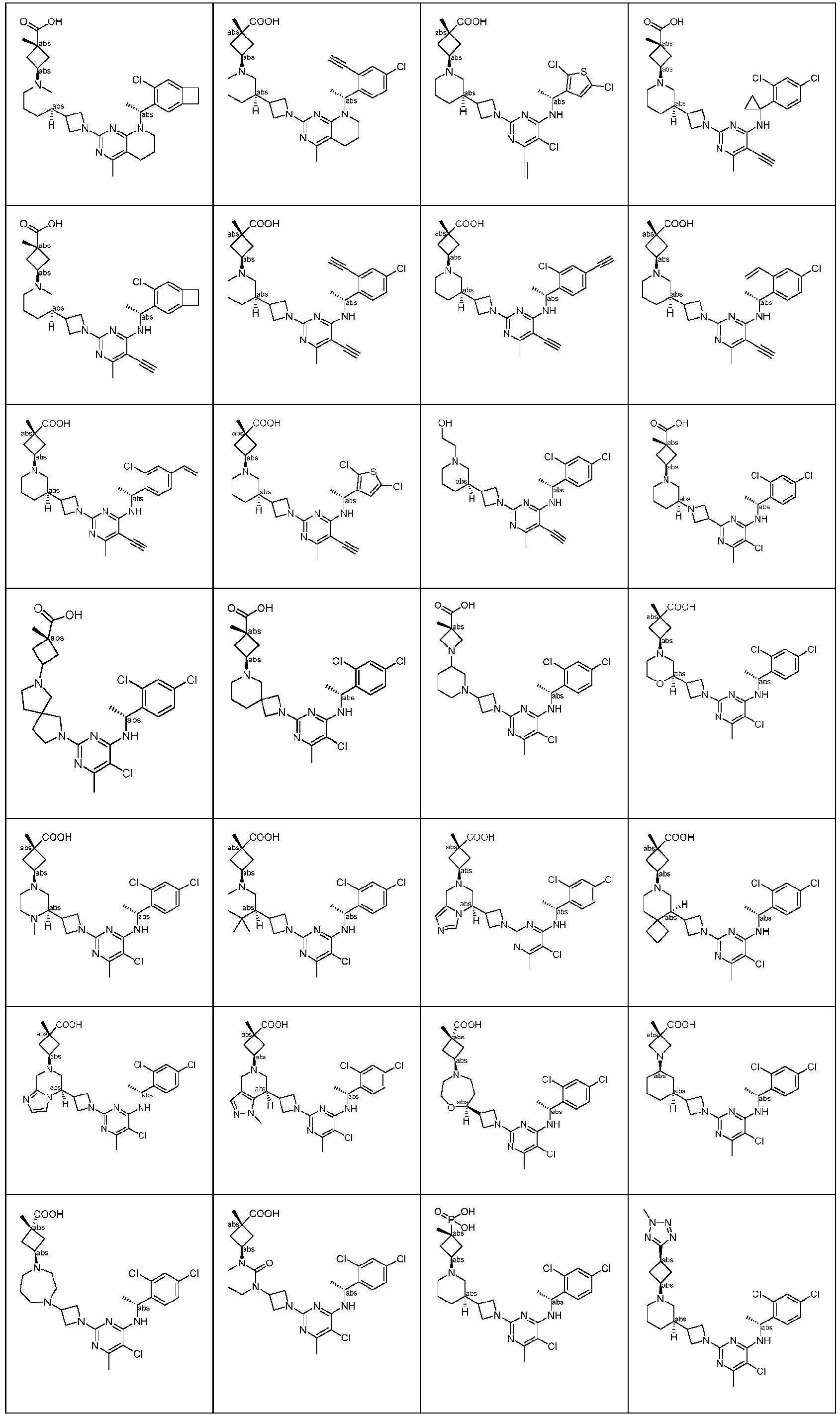

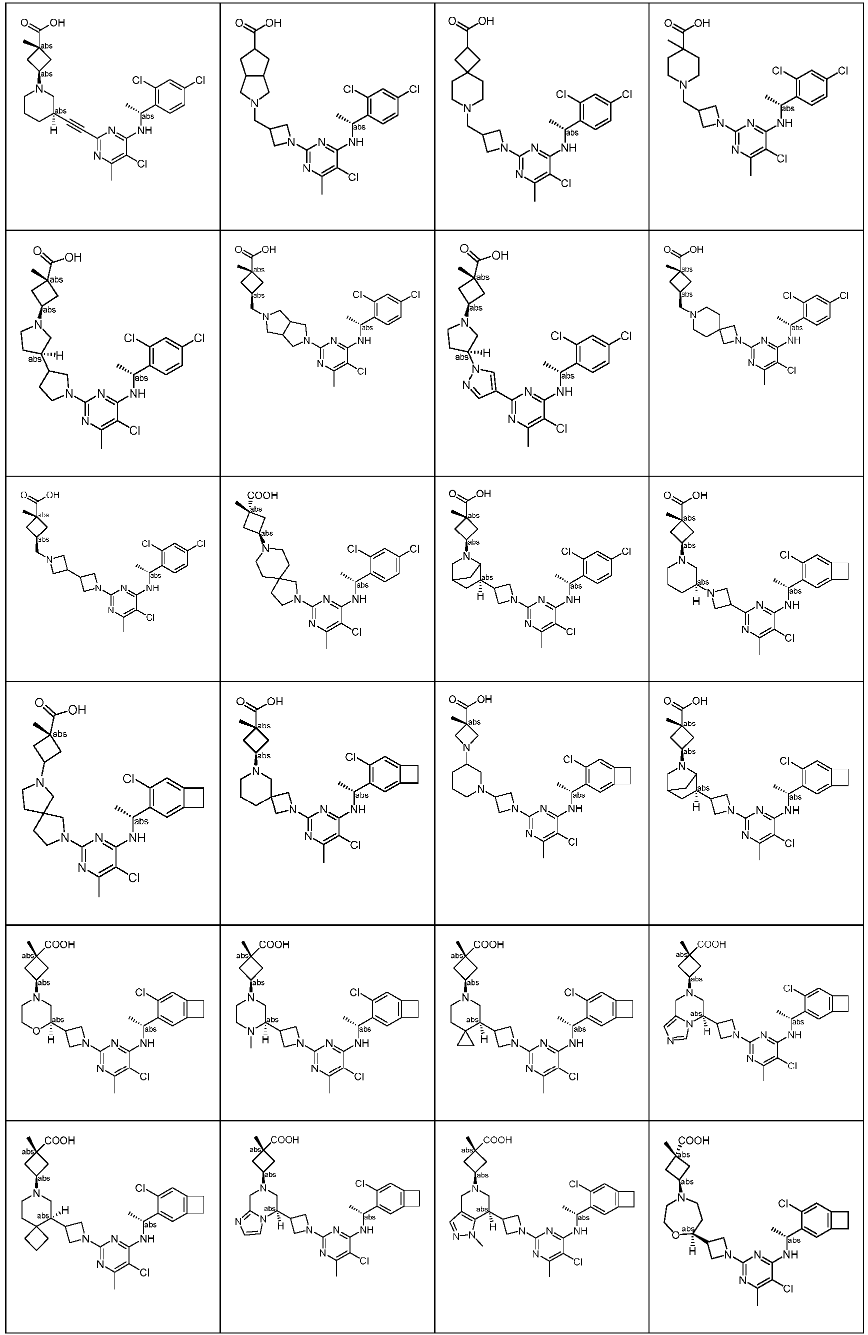

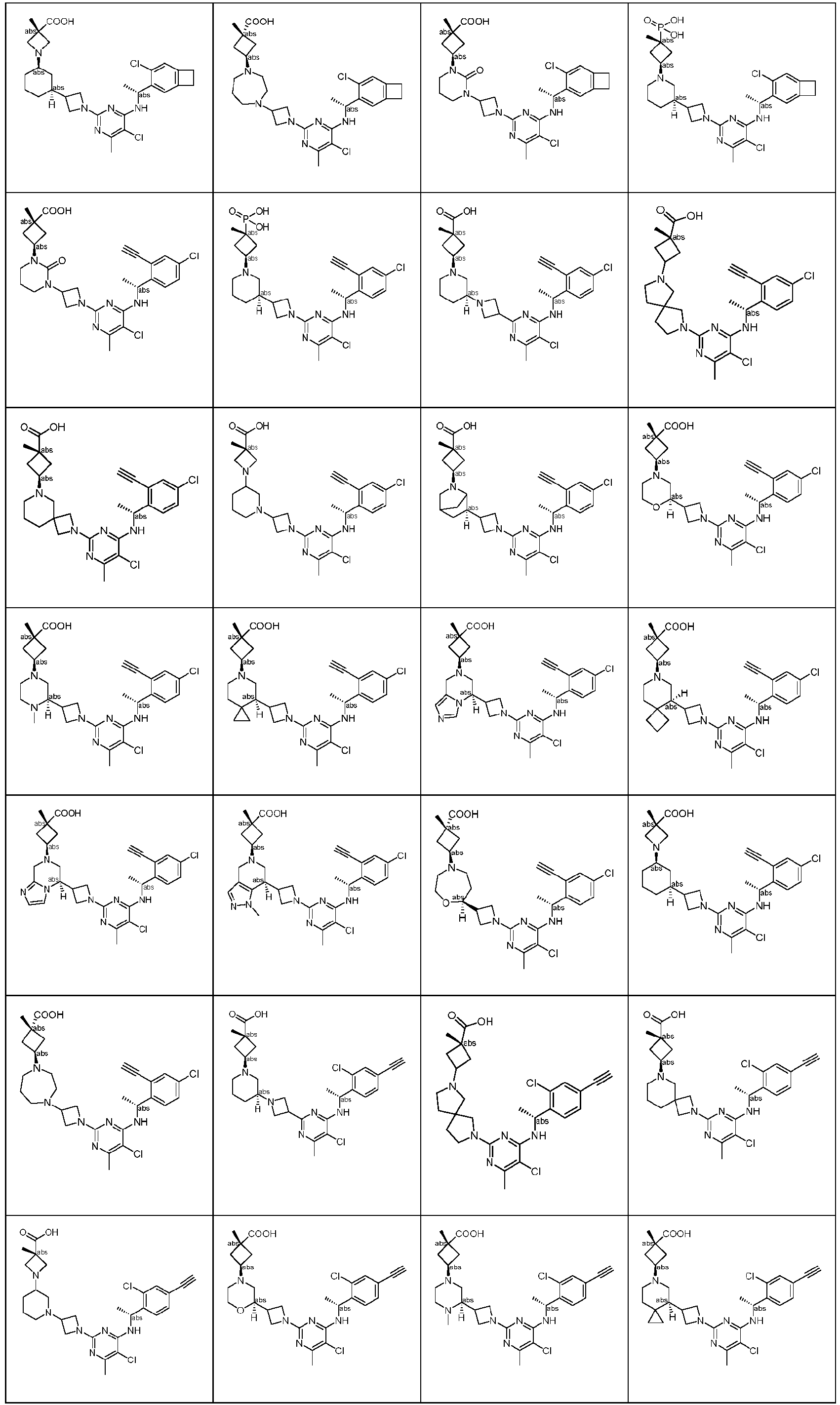

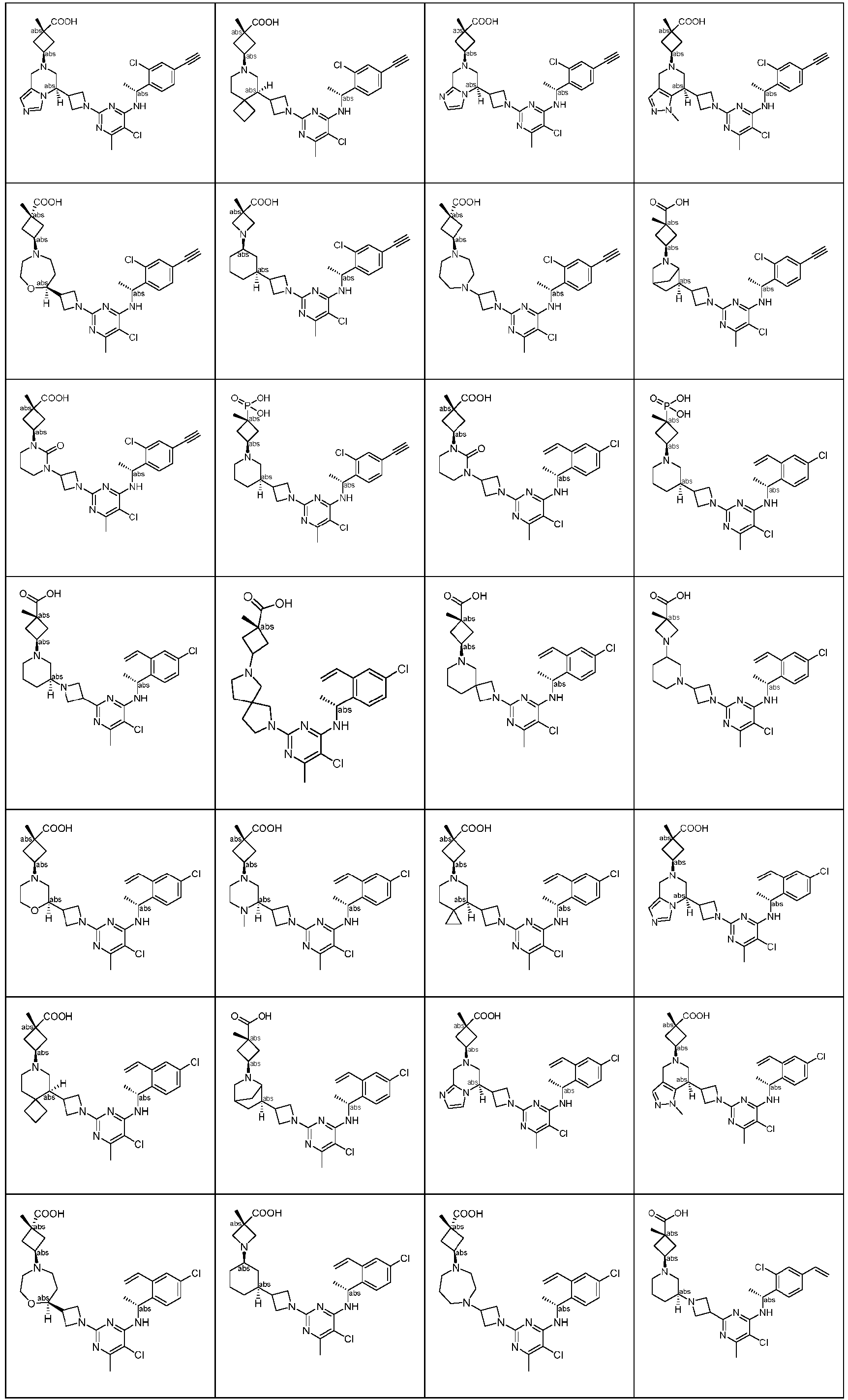

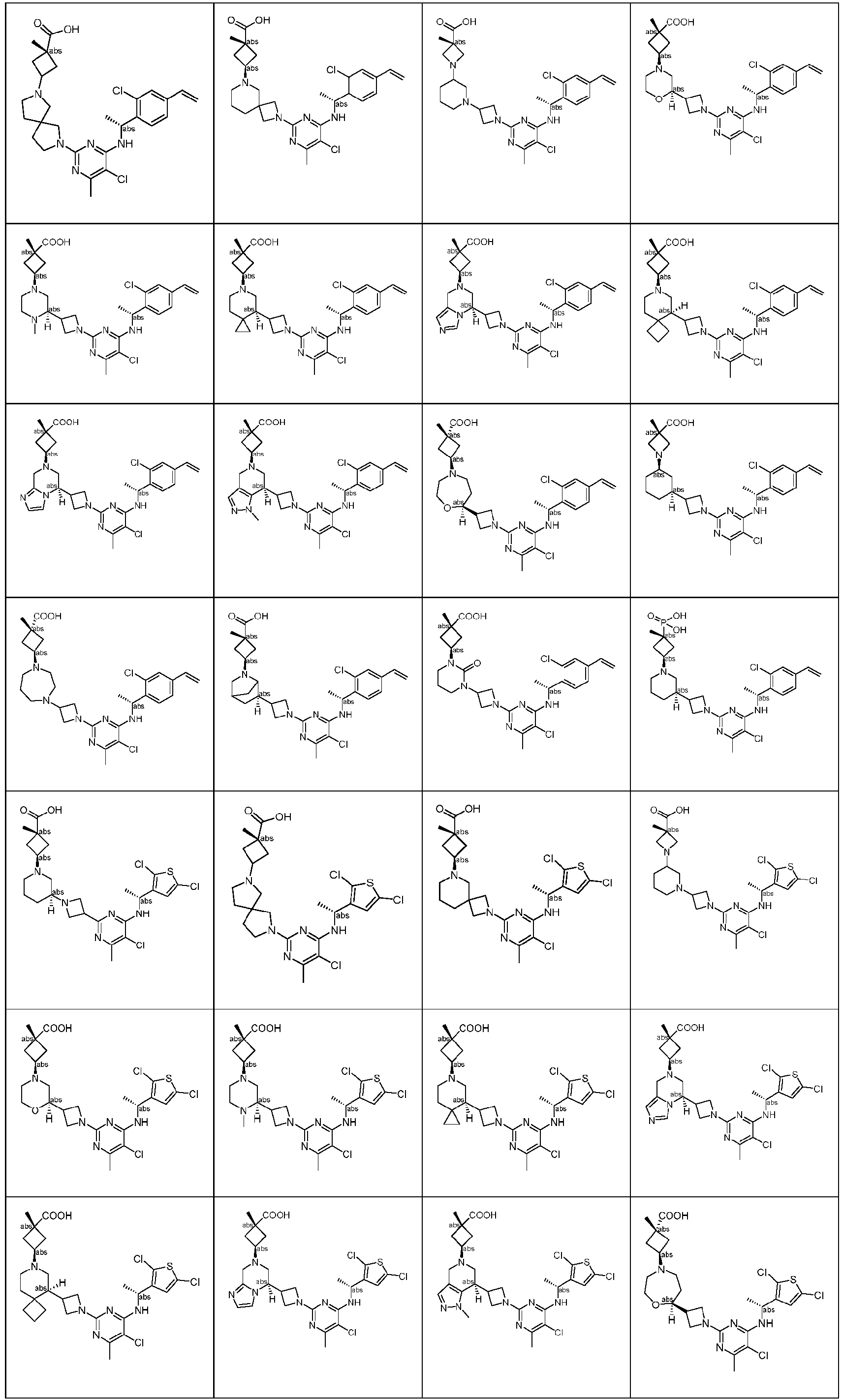

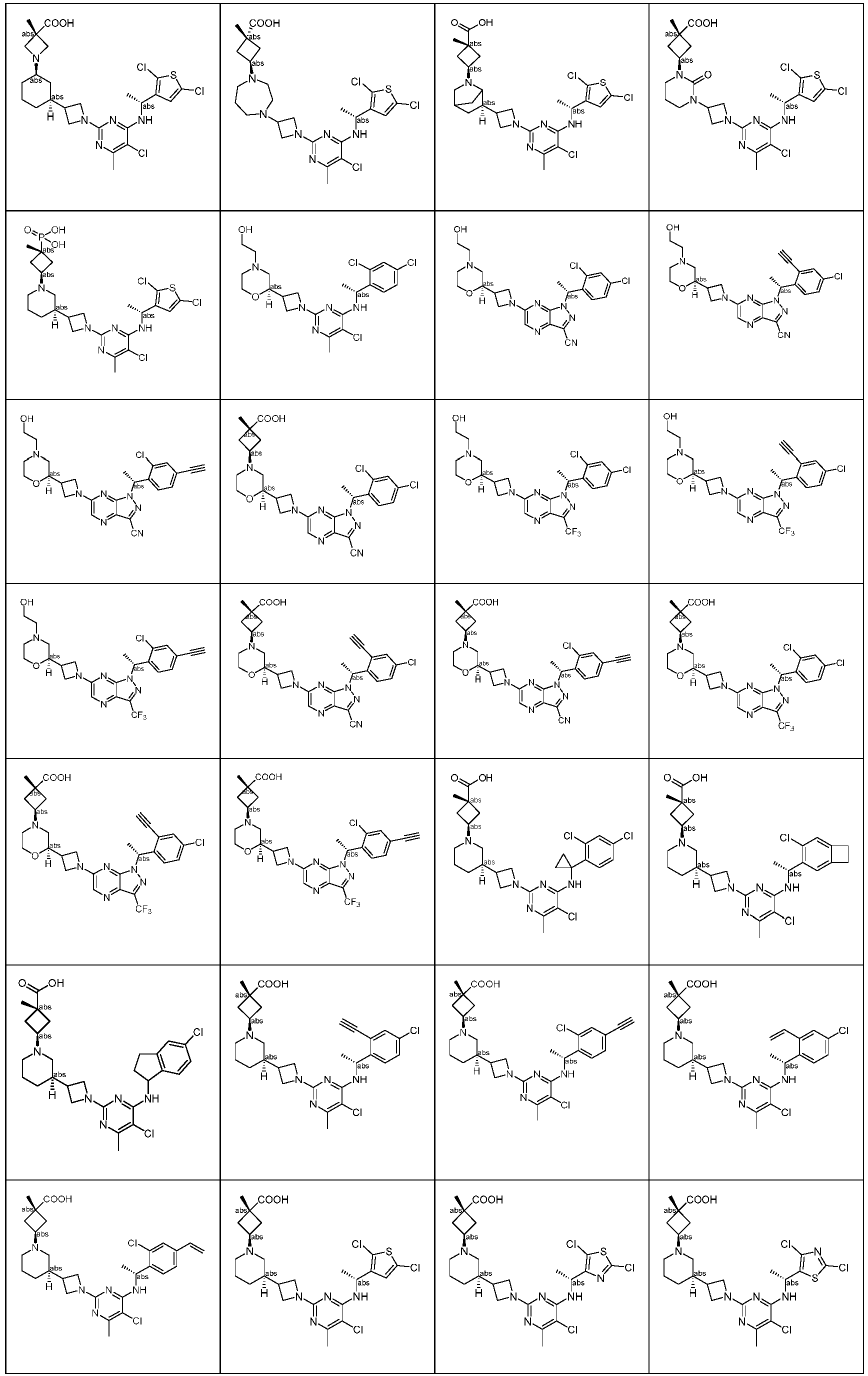

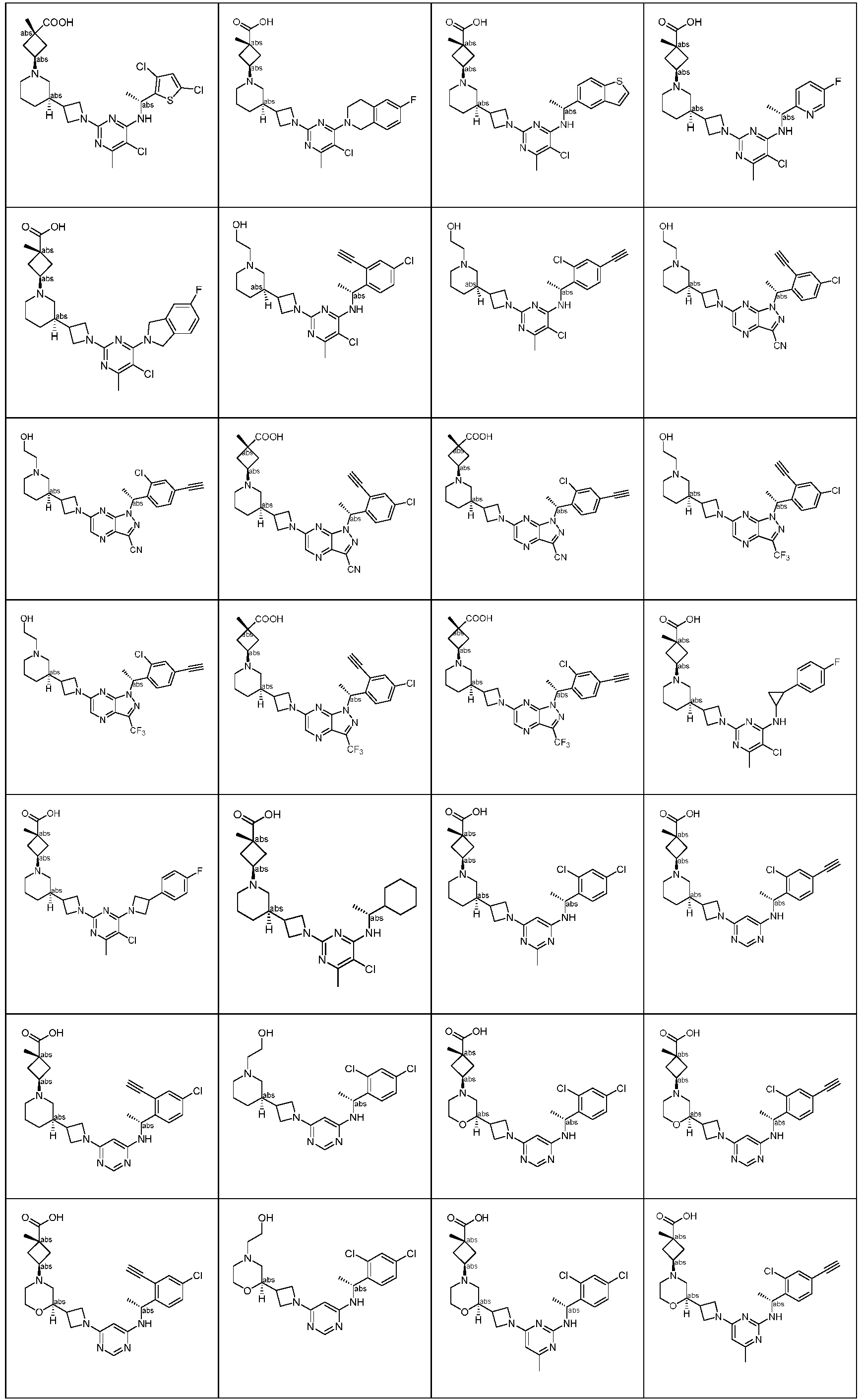

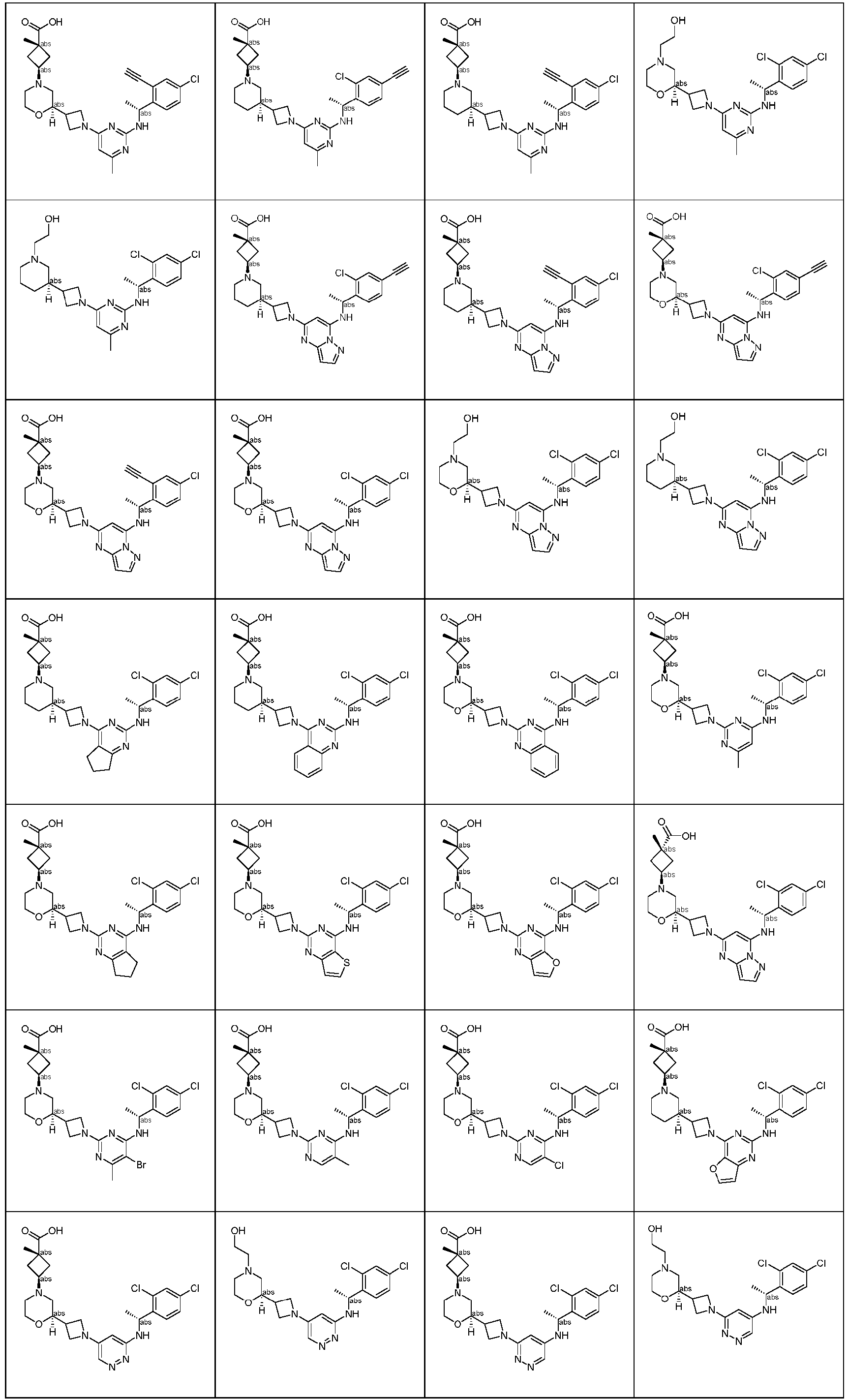

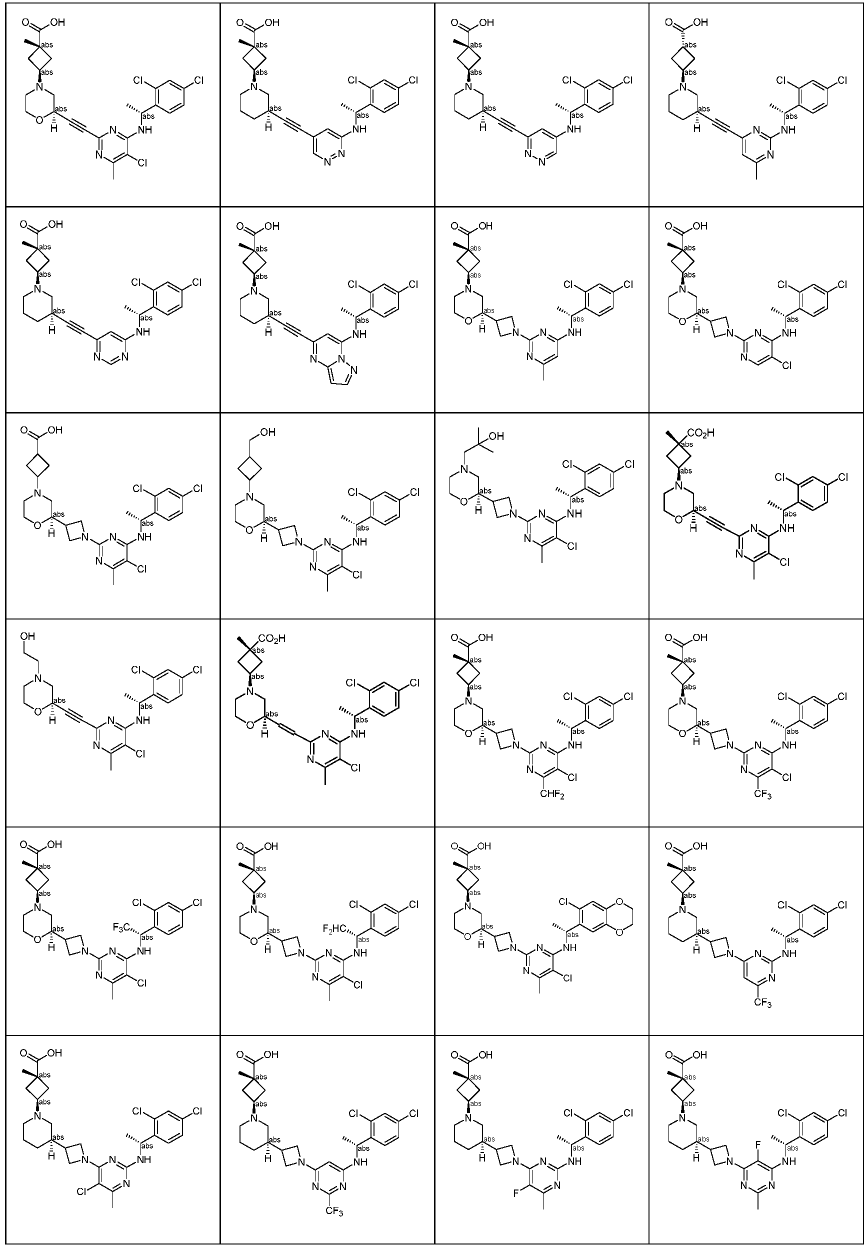

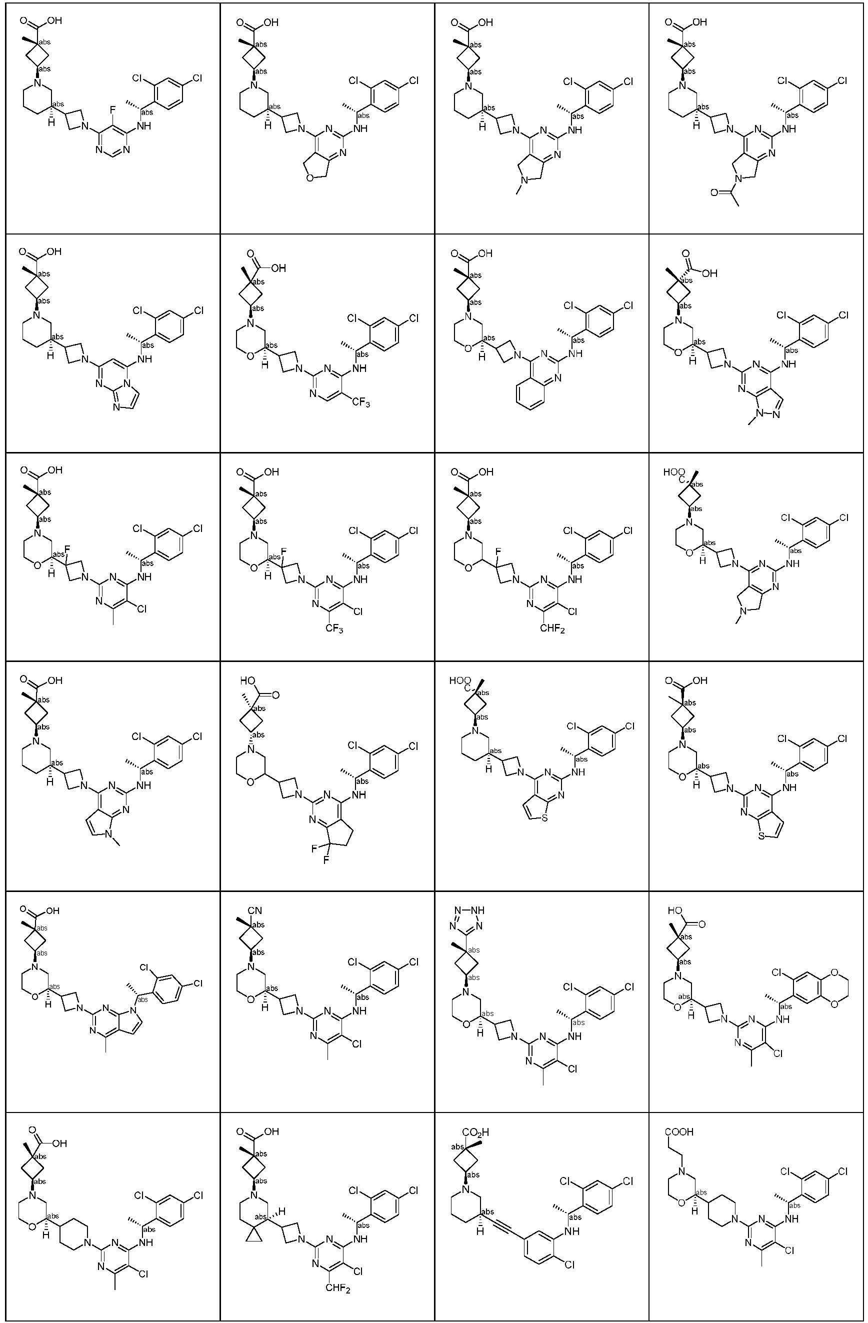

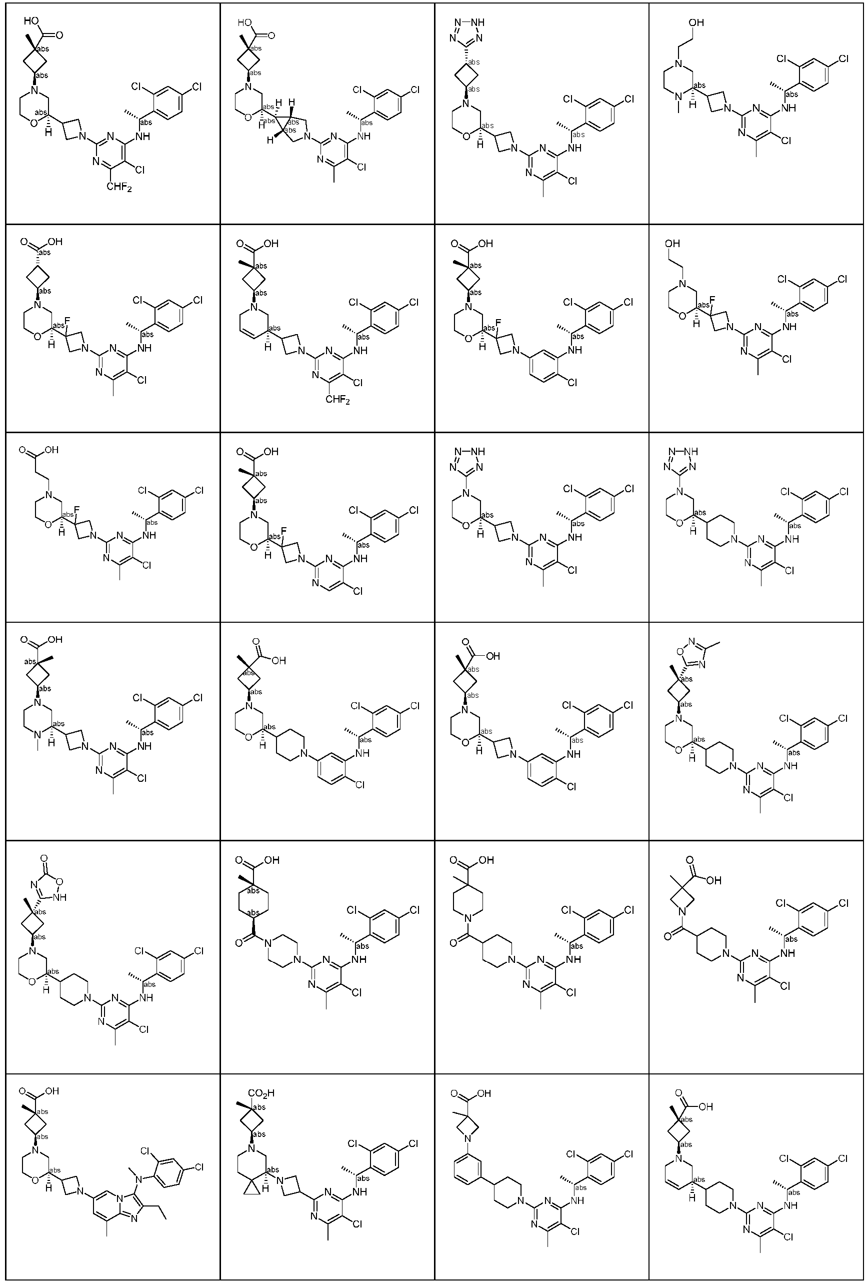

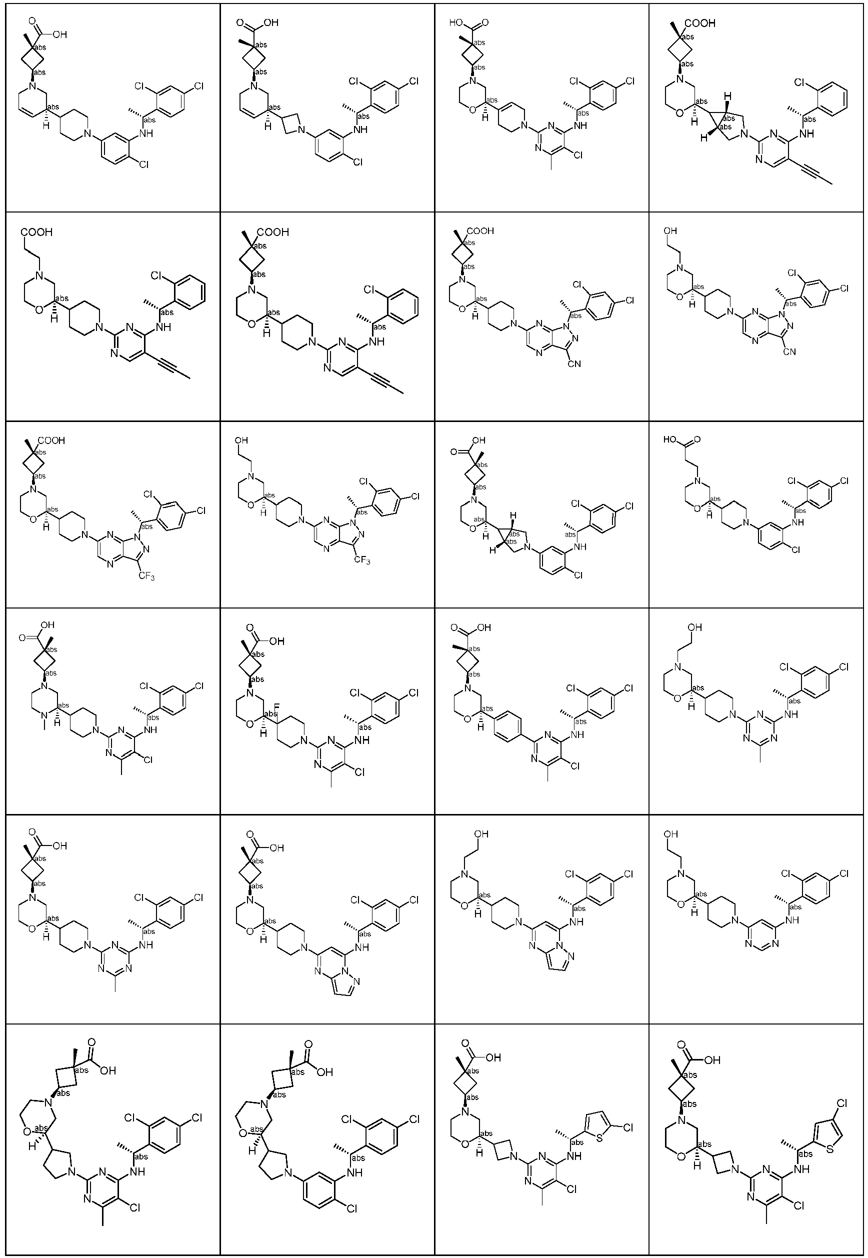

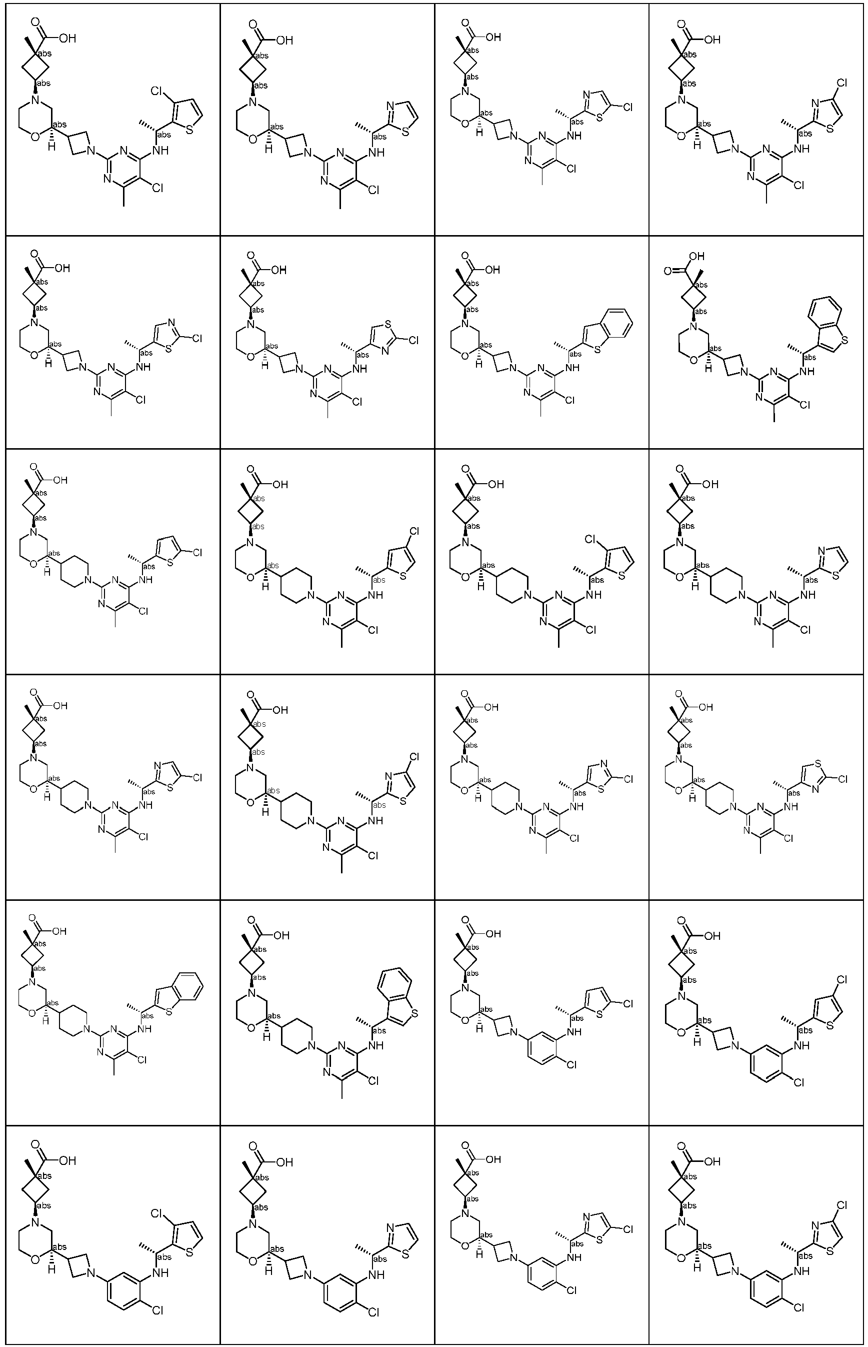

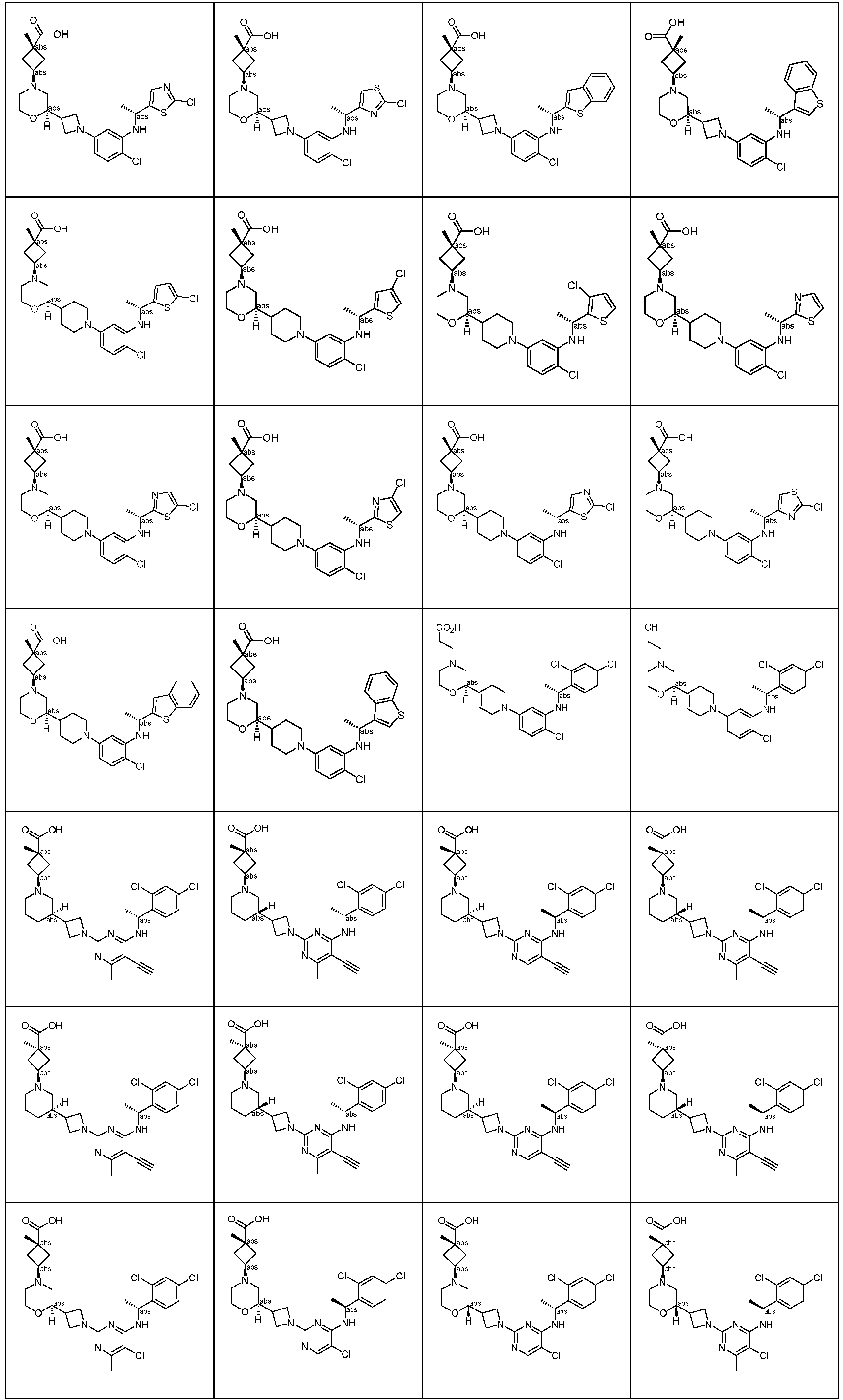

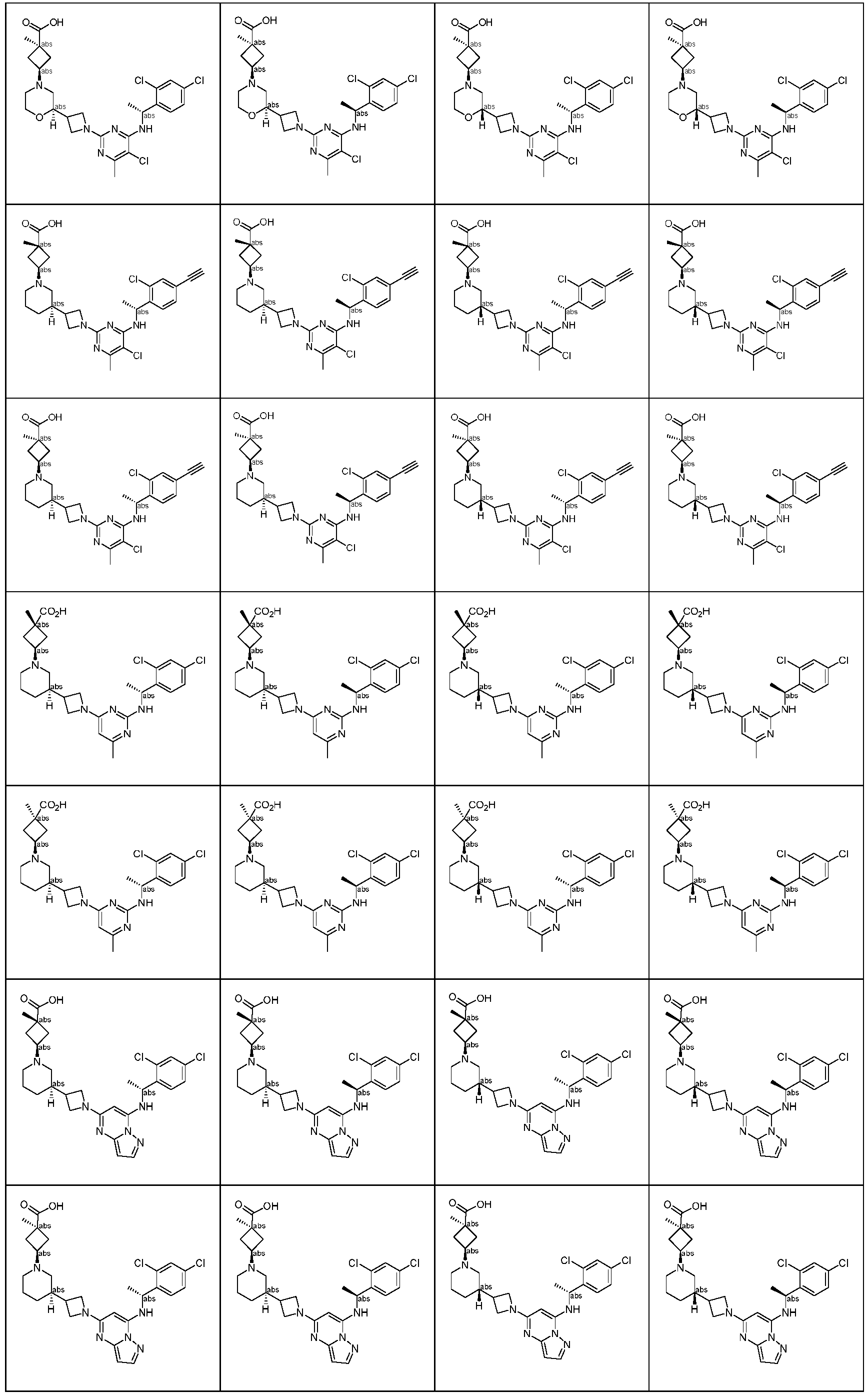

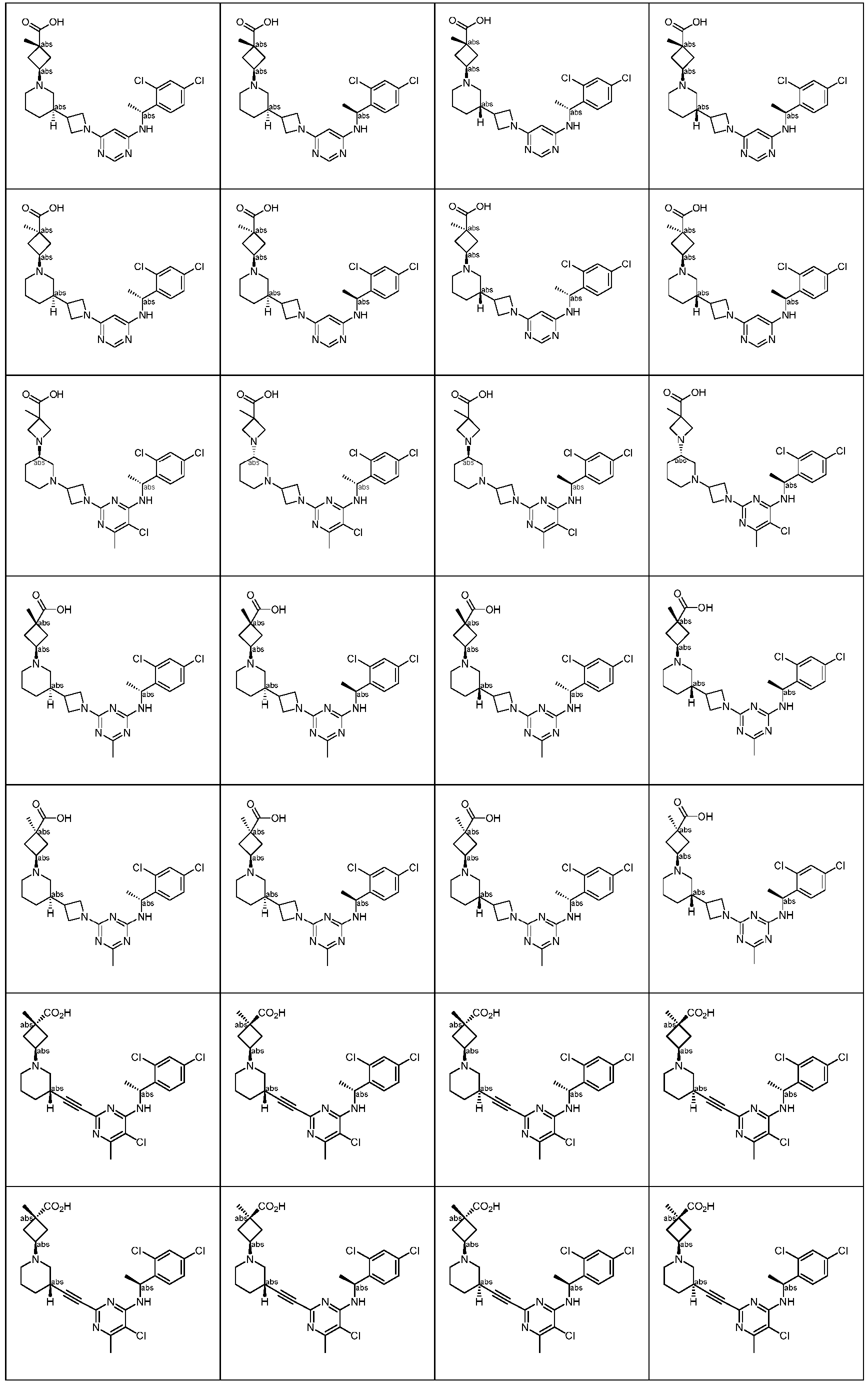

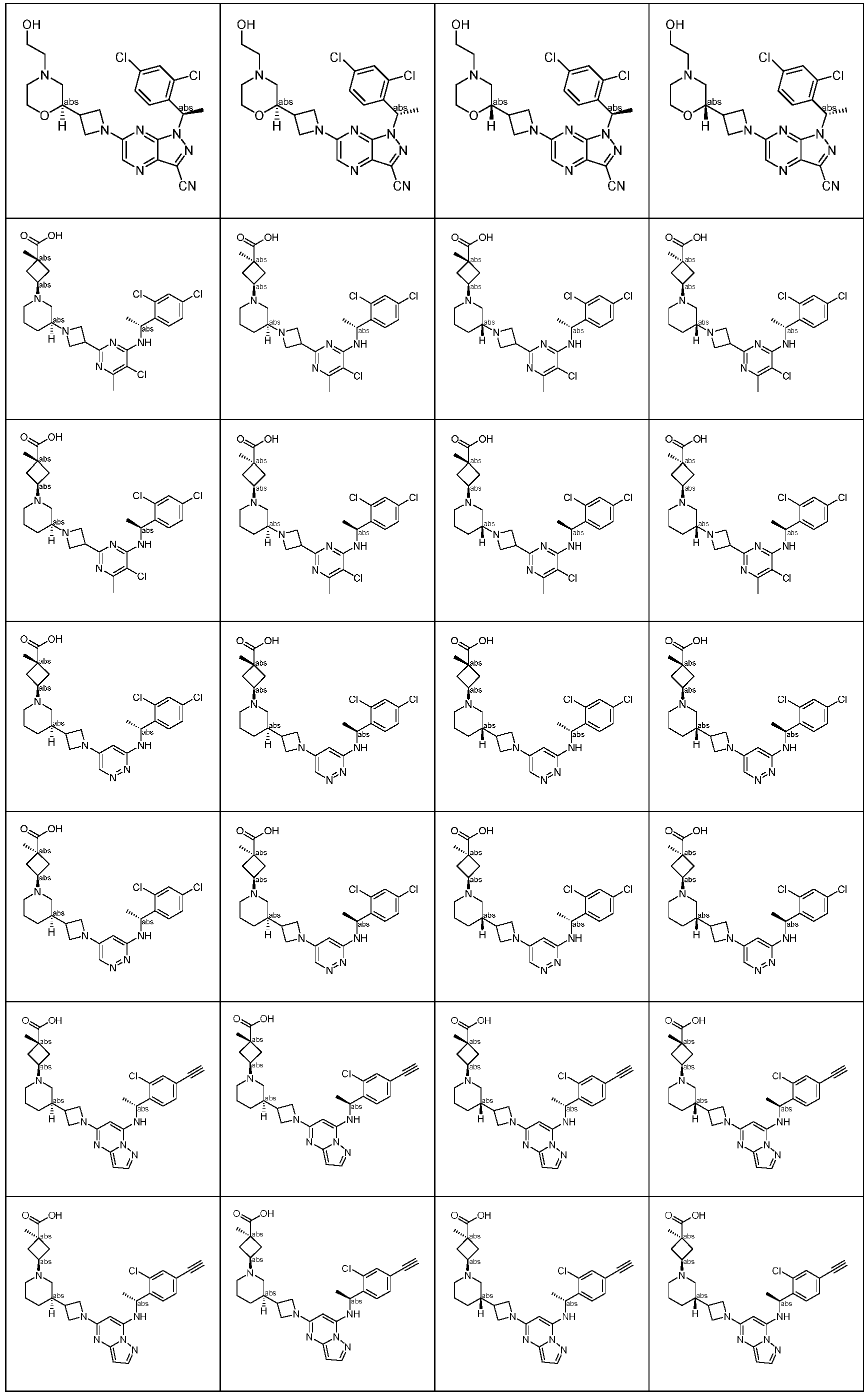

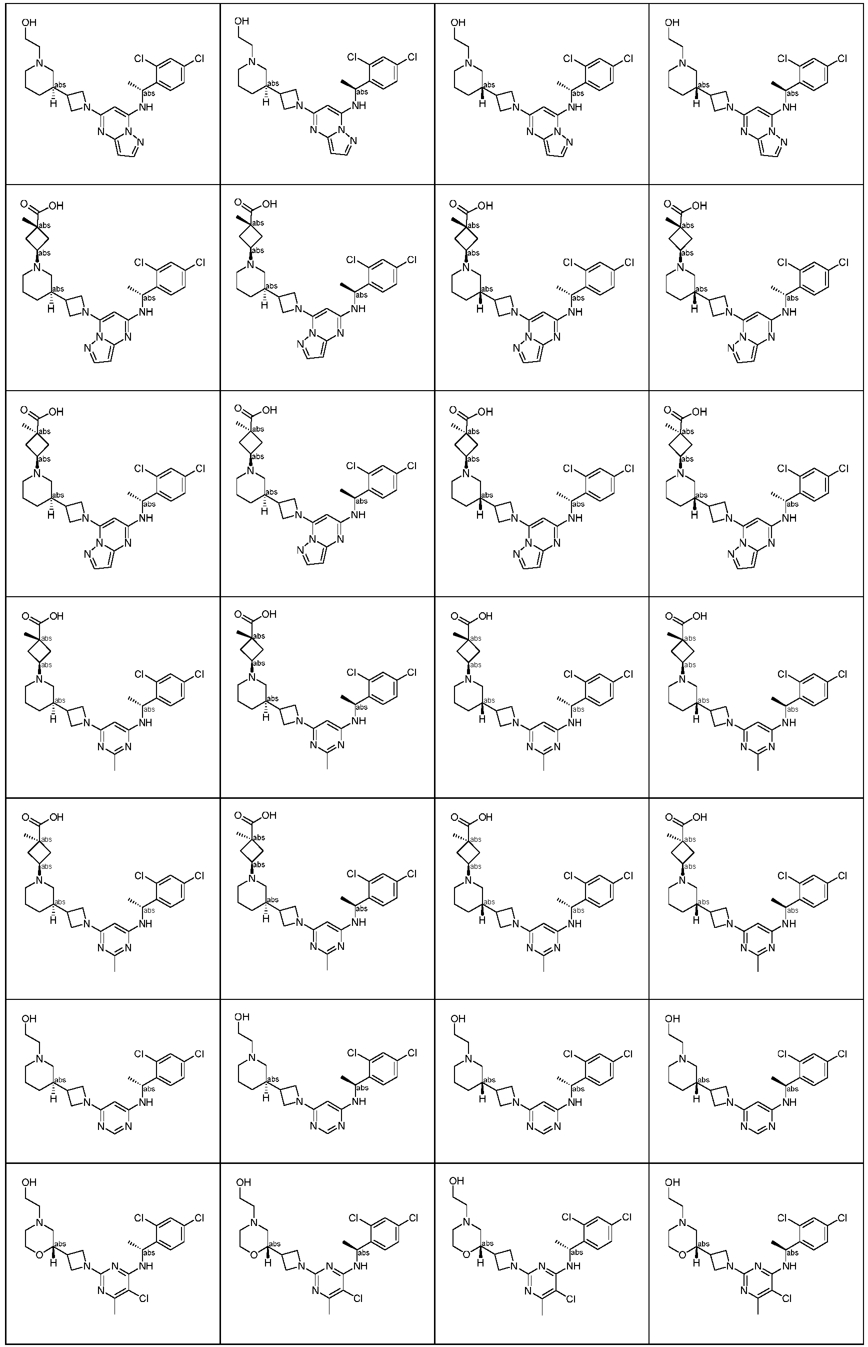

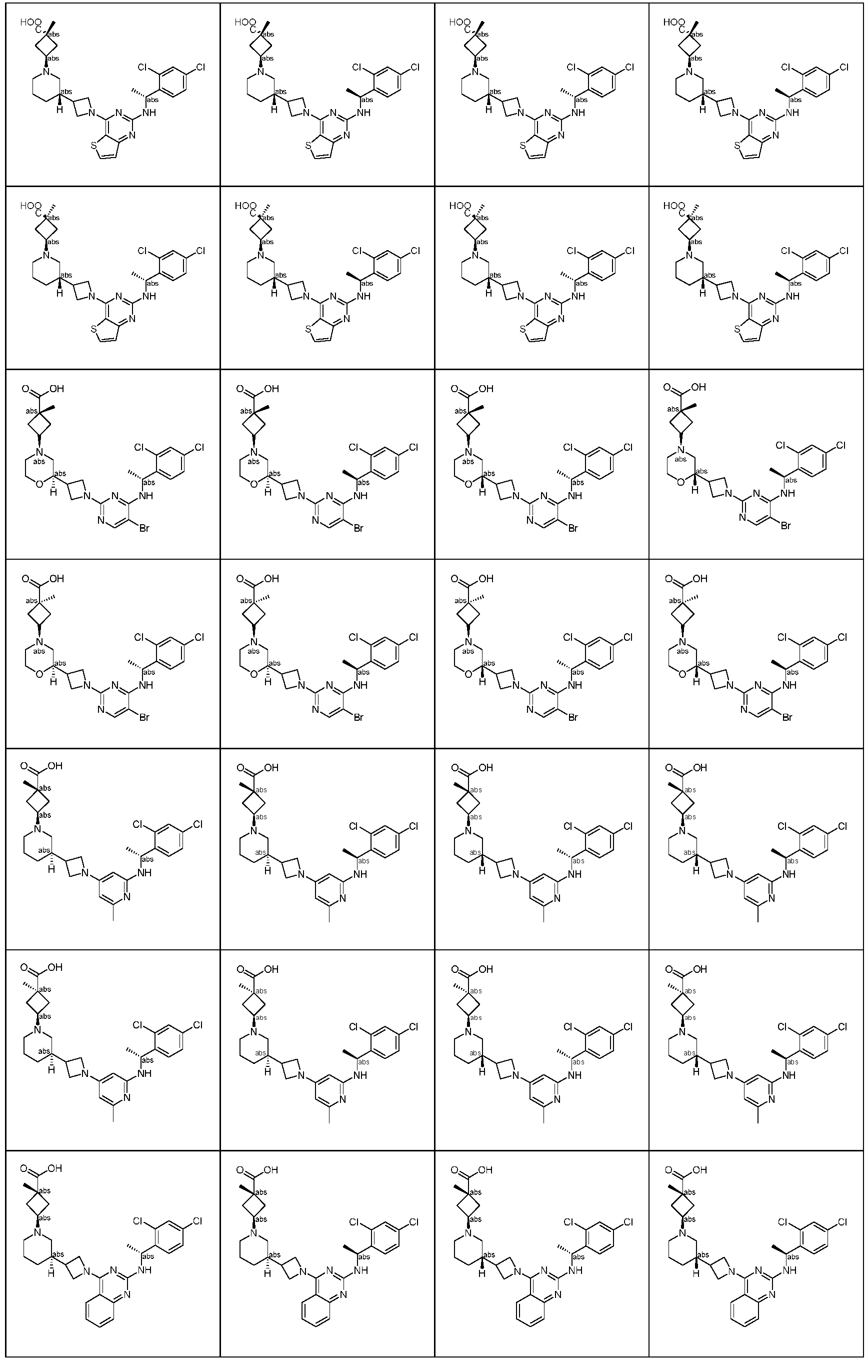

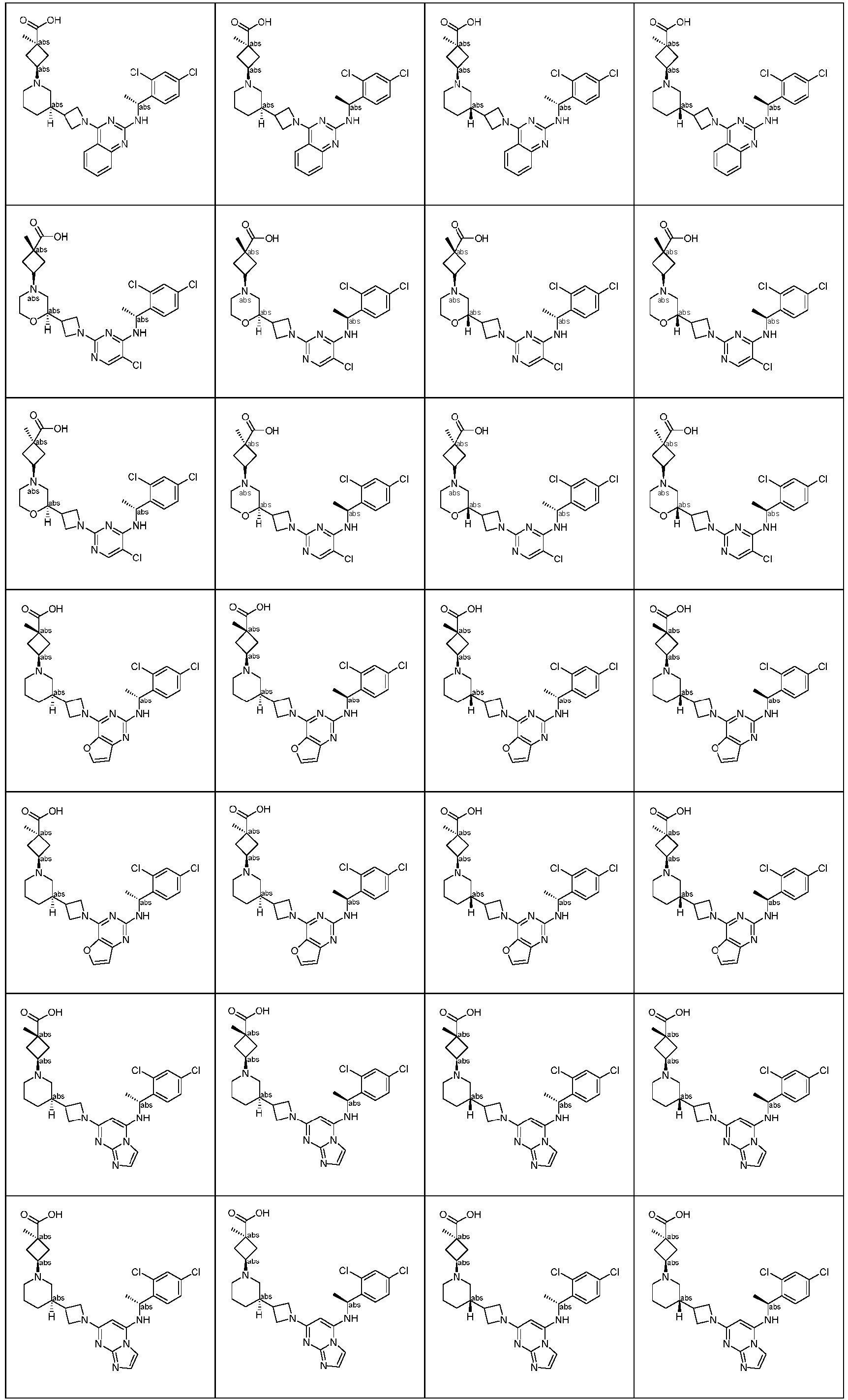

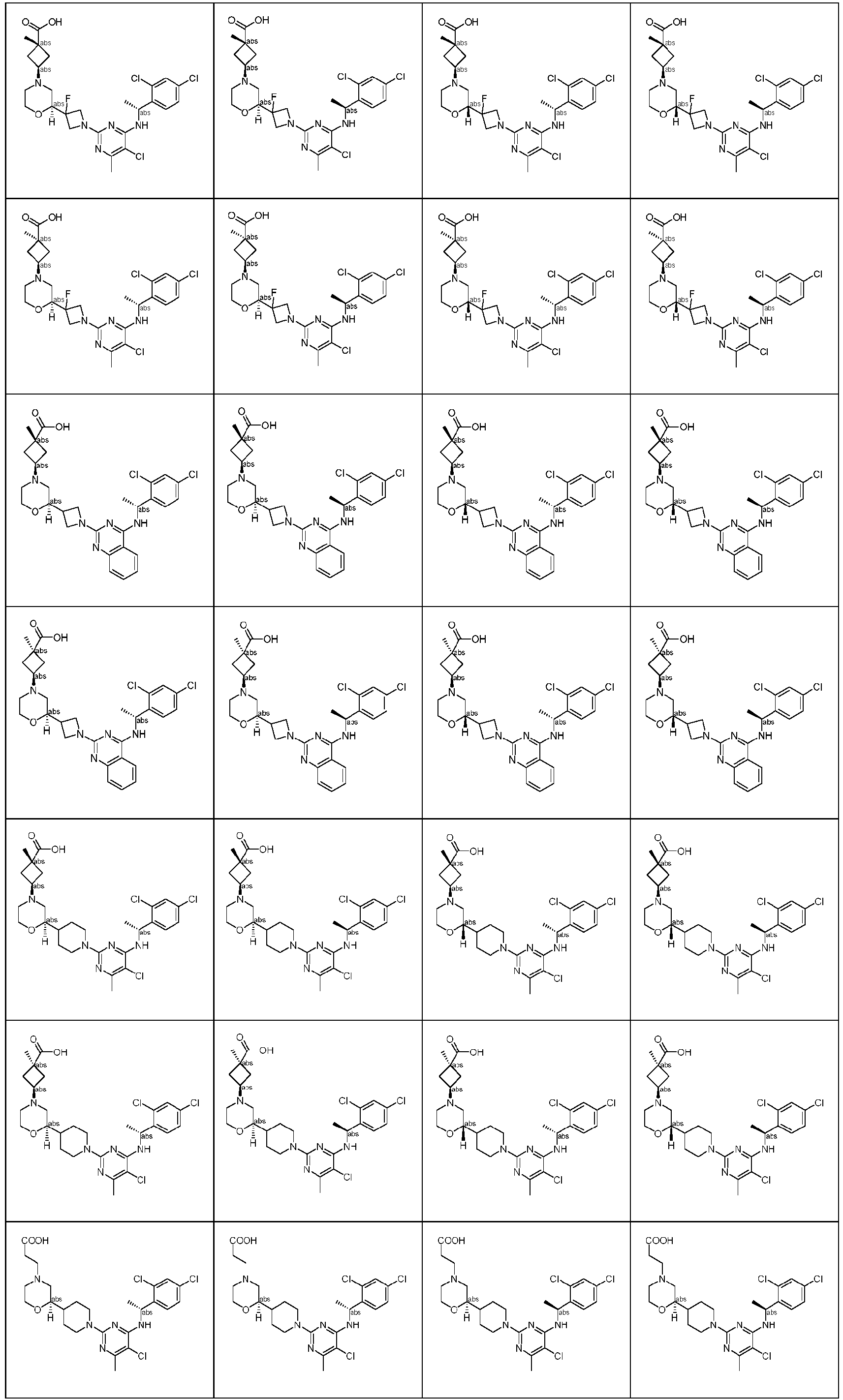

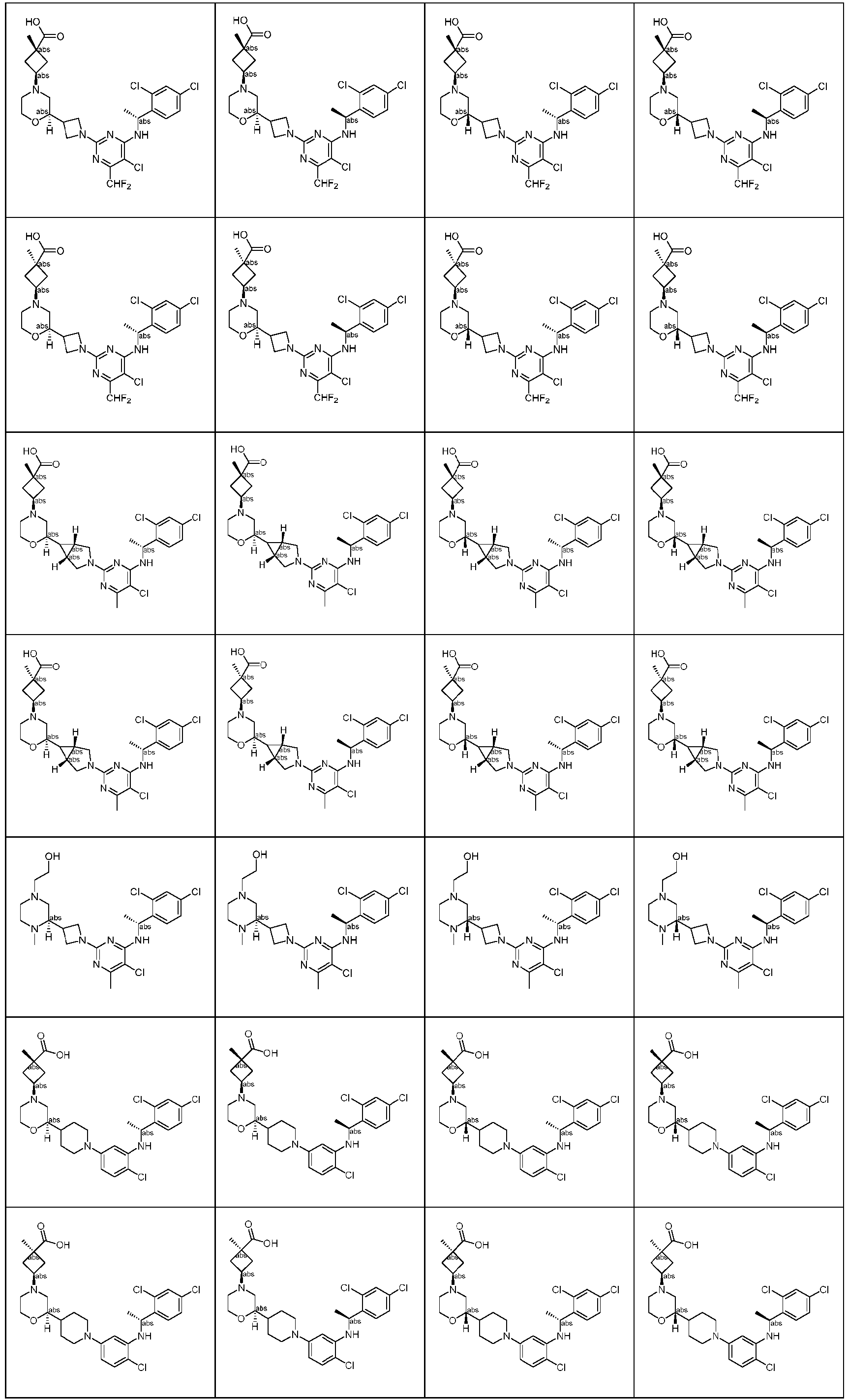

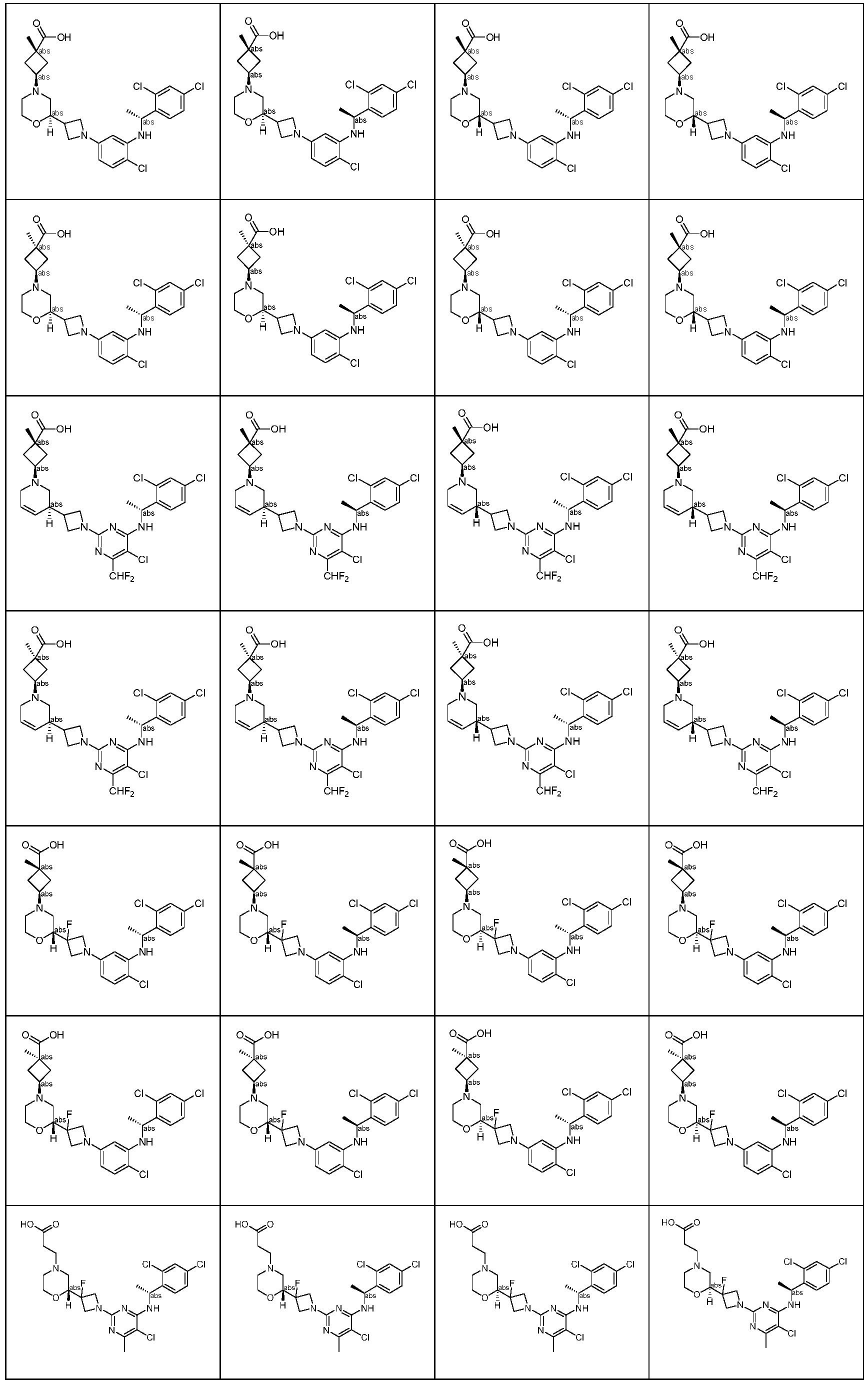

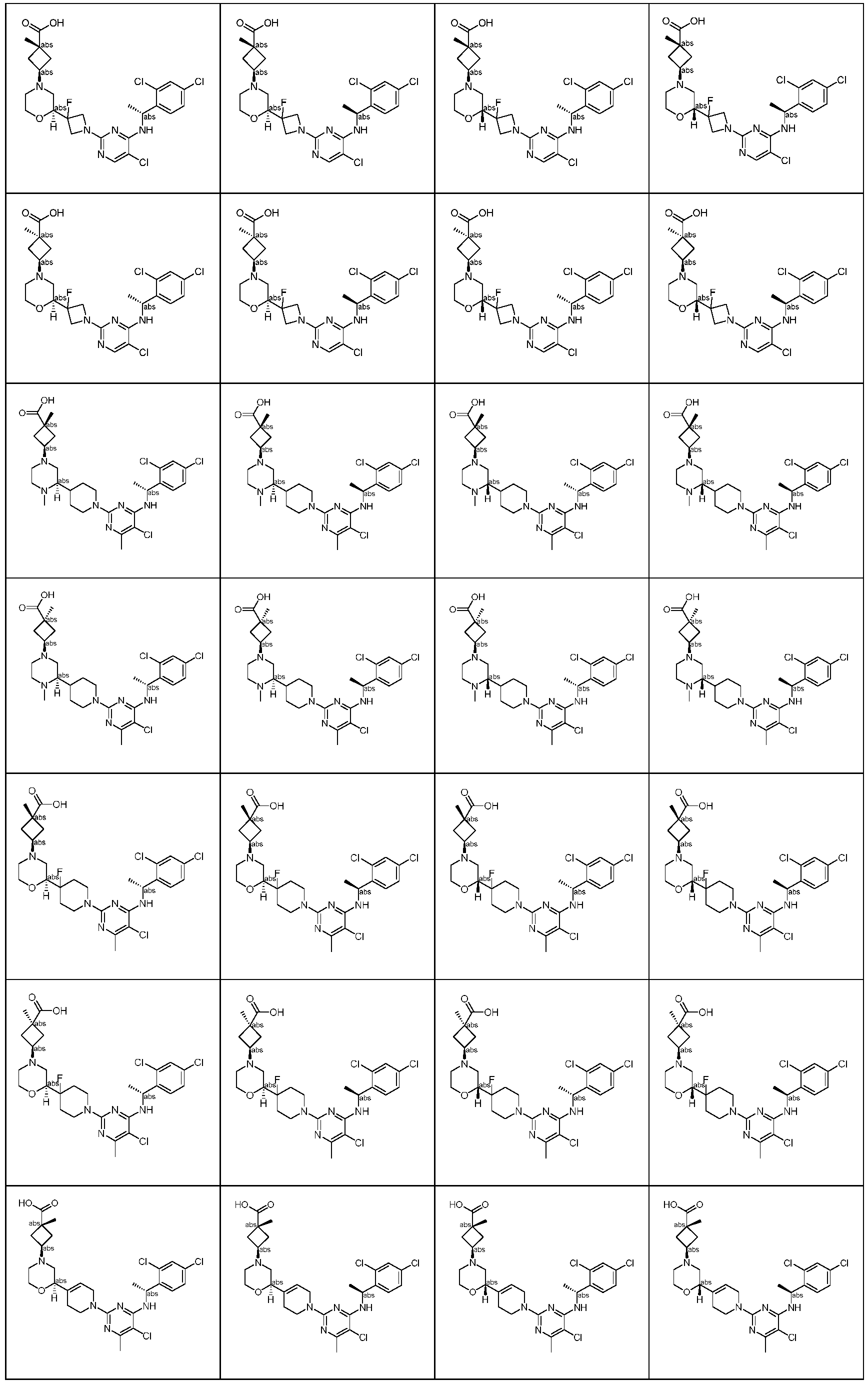

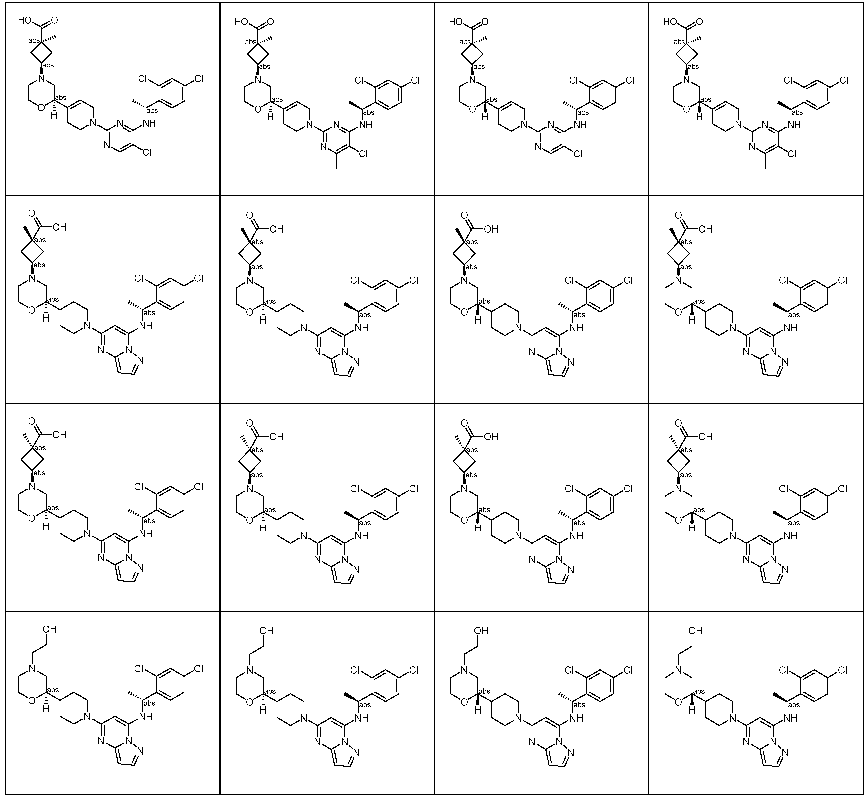

Note: abs denotes absolute configuration.

Secondly, the present invention also provides a pharmaceutical composition, comprising the compound, or the stereoisomer, solvate, deuterated compound, or pharmaceutically acceptable salt thereof according to any one of the preceding technical solutions, and a pharmaceutically acceptable carrier and/or excipient.

Further, the pharmaceutical composition or pharmaceutical preparation comprises 1-1500 mg of the compound, or the stereoisomer, deuterated compound, solvate, pharmaceutically acceptable salt or co-crystal thereof according to any one of the preceding technical solutions, and a pharmaceutically acceptable carrier and/or excipient.

Further, the present invention also provides the use of the compound, or the stereoisomer, solvate, deuterated compound, or pharmaceutically acceptable salt thereof, or the pharmaceutical composition according to any one of the preceding embodiments in the preparation of a drug for treating/preventing a CCR4-mediated disease. Further, the CCR4-mediated disease includes, but is not limited to a tumor or inflammation.

The present invention further provides a method for treating a disease in a mammal or human, the method comprises administering to a subject a therapeutically effective amount of the compound, or the stereoisomer, deuterated compound, solvate, pharmaceutically acceptable salt or co-crystal thereof according to any one of the preceding technical solutions, and a pharmaceutically acceptable carrier and/or excipient, wherein the therapeutically effective amount is preferably 1-1500 mg; the disease is preferably a tumor or inflammation.

The present invention further provides a method for treating a disease in a mammal or human, the method comprises administering to the mammal or human a therapeutically effective amount of the compound, or the stereoisomer, deuterated compound, solvate, pharmaceutically acceptable salt or co-crystal thereof or the pharmaceutical composition according to the present invention. In some embodiments, the mammal mentioned in the present invention does not include human.

The "effective amount" or "therapeutically effective amount" as described in the present application refers to administration of a sufficient amount of the compound disclosed in the present application that will alleviate to some extent one or more symptoms of the diseases or conditions being treated. In some embodiments, the outcome is the reduction and/or remission of signs, symptoms or causes of the disease, or any other desired change in the biological system. For example, an "effective amount" in terms of the therapeutic use is an amount of the composition comprising the compound disclosed in the present application that is required to provide clinically significant reduction of the symptoms of the disease. Examples of the therapeutically effective amount include, but are not limited to 1-1500 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1500 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1500 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, 10-20 mg; 20-1500 mg, 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, 20-30 mg; 50-1500 mg, 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg, 50-100 mg; 100-1500 mg, 100-1000 mg, 100-900 mg, 100-800 mg, 100-700 mg, 100-600 mg, 100-500 mg, 100-400 mg, 100-300 mg, 100-250 mg, or 100-200 mg.

The present invention relates to a pharmaceutical composition or pharmaceutical preparation comprising a therapeutically effective amount of the compound, or the stereoisomer, deuterated compound, solvate, pharmaceutically acceptable salt or co-crystal thereof according to the present invention, and a carrier and/or an excipient. The pharmaceutical composition can be in a unit preparation form (the amount of the active drug in the unit preparation is also referred to as the "preparation specification"). In some embodiments, the pharmaceutical composition comprises the compound, or the stereoisomer, deuterated compound, solvate, pharmaceutically acceptable salt, or co-crystal thereof according to the present invention in an amount including but not limited to 1-1500 mg, 5-1000 mg, 10-800 mg, 20-600 mg, 25-500 mg, 40-200 mg, 50-100 mg, 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg, and 1500 mg.

The present invention further provides a method for treating a disease in a mammal or human, the method comprising administering to a subject a therapeutically effective amount of the compound, or the stereoisomer, deuterated compound, solvate, pharmaceutically acceptable salt or co-crystal thereof according to the present invention, and a pharmaceutically acceptable carrier and/or excipient, wherein the therapeutically effective amount is preferably 1-1500 mg; the disease is preferably a tumor or inflammation.

The present invention also provides a method for treating a disease in a mammal or human, comprising administering to a subject a drug, i.e., the compound, or the stereoisomer, deuterated compound, solvate, pharmaceutically acceptable salt or co-crystal thereof according to the present invention, and a pharmaceutically acceptable carrier and/or excipient at a daily dose of 1-1500 mg/day, wherein the daily dose can be a single dose or divided doses; in some embodiments, the daily dose includes, but is not limited to 10-1500 mg/day, 20-1500 mg/day, 25-1500 mg/day, 50-1500 mg/day, 75-1500 mg/day, 100-1500 mg/day, 200-1500 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day, or 200-400 mg/day; in some embodiments, the daily dose includes, but is not limited to 1 mg/day, 5 mg/day, 10 mg/day, 20 mg/day, 25 mg/day, 50 mg/day, 75 mg/day, 100 mg/day, 125 mg/day, 150 mg/day, 200 mg/day, 400 mg/day, 600 mg/day, 800 mg/day, 1000 mg/day, 1200 mg/day, 1400 mg/day, or 1500 mg/day.

The present invention relates to a kit, wherein the kit may comprise a composition in the form of a single dose or multiple doses and comprises the compound, or the stereoisomer, deuterated compound, solvate, pharmaceutically acceptable salt or co-crystal thereof according to the present invention, and the amount of the compound, or the stereoisomer, deuterated compound, solvate, pharmaceutically acceptable salt or co-crystal thereof according to the present invention is identical to the amount of same in the above-mentioned pharmaceutical composition.

In the present invention, the amount of the compound, or the stereoisomer, deuterated compound, solvate, pharmaceutically acceptable salt or co-crystal thereof according to the present invention is calculated in the form of a free base in each case.

The term "preparation specification" refers to the weight of the active drug contained in each vial, tablet or other unit preparation.

### Synthetic route

Those skilled in the art would have been able to prepare the compounds of the present invention according to known organic synthesis techniques, and the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

Specific and similar reactants can be selectively identified by the indexes of known chemicals prepared by the Chemical Abstracts Service of the American Chemical Society, wherein the indexes are available in most public libraries and university libraries and online. Chemicals that are known but not commercially available in the catalog are optionally prepared by custom chemical synthesis plants, wherein many of standard chemical supply plants (such as those listed above) provide custom synthesis services.

### Term

Unless otherwise specified, the terms of the present invention have the following meanings.

The carbon, hydrogen, oxygen, sulfur, nitrogen and halogen involved in the groups and compounds of the present invention all include isotopes thereof, and are optionally further replaced by one or more of the corresponding isotopes thereof, wherein the isotopes of carbon include ¹²C, ¹³C and ¹⁴C; the isotopes of hydrogen include protium (H), deuterium (D, also known as heavy hydrogen) and tritium (T, also known as superheavy hydrogen); the isotopes of oxygen include ¹⁶O, ¹⁷O and ¹⁸O; the isotopes of sulfur include ³²S, ³³S, ³⁴S and ³⁶S; the isotopes of nitrogen include ¹⁴N and ¹⁵N; the isotope of fluorine includes ¹⁹F; the isotopes of chlorine include ³⁵Cl and ¹⁷Cl ; and the isotopes of bromine include ⁷⁹Br and ⁸¹Br.

The term "halogen" herein refers to F, Cl, Br, I, or isotopes thereof.

The term "halo" or "substituted with halogen" refers to being substituted with one or more groups selected from F, Cl, Br, I, or isotopes thereof, wherein the upper limit of the number of halogen substituents is equal to the sum of the number of hydrogens that can be substituted in the group to be substituted. Without particular limitation, the number of halogen substituents is any integer between 1 and the upper limit, and when the number of halogen substituents is greater than 1, the group to be substituted can be substituted with the same or different halogen. Generally, the circumstances of being substituted with 1-5 halogen, 1-3 halogen, 1-2 halogen, and 1 halogen are included.

The term "deuterium" refers to the isotope deuterium of hydrogen (H), which is synonymous with "D".

The term "deuterated" or "deuterated compound" refers to the case where a hydrogen atom on a group, such as alkyl, cycloalkyl, alkylene, aryl, heteroaryl, mercapto, heterocycloalkyl, alkenyl and alkynyl is substituted with at least one deuterium atom, wherein the upper limit of the number of deuterium substituents is equal to the sum of the number of hydrogens that can be substituted in the group to be substituted. Without particular limitation, the number of deuterium substituents is any integer between 1 and the upper limit, for example, 1-20 deuterium atoms, 1-10 deuterium atoms, 1-6 deuterium atoms, 1-3 deuterium atoms, 1-2 deuterium atoms or 1 deuterium atom.

Group "C_{x-y}" refers to a group comprising x to y carbon atoms, for example, "C₁₋₆ alkyl" refers to alkyl comprising 1-6 carbon atoms.

The term "alkyl" refers to a monovalent straight or branched saturated aliphatic hydrocarbon group, usually an alkyl group with 1 to 20 carbon atoms, or an alkyl group with 1 to 8 carbon atoms, or an alkyl group with 1 to 6 carbon atoms, or an alkyl group with 1 to 4 carbon atoms. Non-limiting examples of alkyl include methyl, ethyl, n-propyl, isopropyl, n-butyl, sec-butyl, neobutyl, tert-butyl, n-pentyl, isopentyl, neopentyl, n-hexyl, etc., and alkyl may be further substituted with a substituent.

The term "alkylene" refers to a divalent straight or branched saturated alkyl group. Examples of alkylene include, but are not limited to methylene, ethylidene, *etc.*

The term "haloalkyl" refers to an alkyl group in which one or more hydrogens are replaced by one or more halogen atoms (e.g., fluorine, chlorine, bromine, iodine, or isotopes thereof), wherein the upper limit of the number of halogen substituents is equal to the sum of the number of hydrogens that can be substituted in the alkyl group. Without particular limitation, the number of halogen substituents is any integer between 1 and the upper limit. Generally, the alkyl group is substituted with 1-5 halogen, 1-3 halogen, 1-2 halogen or 1 halogen; and when the number of halogen substituents is greater than 1, the group to be substituted can be substituted with the same or different halogen. Specific examples include, but are not limited to -CF₃, -CH₂Cl, -CH₂CF₃, -CCl₂, CF₃, etc.

The term "alkoxy" or "alkyloxy" refers to -O-alkyl, such as -O-C₁₋₈ alkyl, -O-C₁₋₆ alkyl, -O-C₁₋₄ alkyl or -O-C₁₋₂ alkyl. Non-limiting and specific examples of alkoxy or alkyloxy include methoxy, ethoxy, n-propoxy, isopropoxy, n-butoxy, sec-butoxy, tert-butoxy, n-pentoxy, n-hexyloxy, cyclopropoxy, cyclobutoxy, etc. The alkoxy may be optionally substituted with a substituent.

The term "haloalkoxy" refers to -O-haloalkyl, such as -O-halo C₁₋₈ alkyl, -O-halo C₁₋₆ alkyl, -O-halo C₁₋₄ alkyl or -O-halo C₁₋₂ alkyl; the upper limit of the number of halogen substituents is equal to the sum of the number of hydrogens that can be substituted in the group to be substituted. Without particular limitation, the number of halogen substituents is any integer between 1 and the upper limit, preferably 1-5 halogen, 1-3 halogen, 1-2 halogen, and 1 halogen; and when the number of halogen substituents is greater than 1, the group to be substituted can be substituted with the same or different halogen. Non-limiting examples of haloalkoxy include monofluoromethoxy, difluoromethoxy, trifluoromethoxy, difluoroethyloxy, *etc.*

The "hydroxy C₁₋₆ alkyl" refers to a hydroxy-substituted alkyl having 1 to 6 carbon atoms.

The term "alkenyl" refers to a straight or branched hydrocarbon group comprising at least one carbon-carbon double bond (C=C) and generally comprises 2 to 18 carbon atoms, such as 2 to 8 carbon atoms, further such as 2 to 6 carbon atoms, and still further such as 2 to 4 carbon atoms. Examples of alkenyl include, but are not limited to ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc.; and the alkenyl may further be optionally substituted with a substituent.

The term "alkenylene" refers to a linear or branched divalent unsaturated hydrocarbon group comprising at least one carbon-carbon double bond (C=C) and generally comprises 2 to 18 carbon atoms, such as 2 to 8 carbon atoms, further such as 2 to 6 carbon atoms, and still further such as 2 to 4 carbon atoms. Non-limiting examples of alkenylene include ethynylene and the alkenylene may be optionally substituted with a substituent.

The term "alkynyl" refers to a straight or branched hydrocarbon group comprising at least one carbon-carbon triple bond (C≡C) and generally comprises 2 to 18 carbon atoms, further comprises 2 to 8 carbon atoms, further comprises 2 to 6 carbon atoms, and still further comprises 2 to 4 carbon atoms. Examples of alkynyl include, but are not limited to ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl, 4-decynyl, etc.; and the alkynyl may be optionally substituted with a substituent.

The term "alkynylene" refers to a linear or branched divalent unsaturated hydrocarbon group containing a carbon-carbon triple bond (C≡C) and generally comprises 2 to 18 carbon atoms, further comprises 2 to 8 carbon atoms, further comprises 2 to 6 carbon atoms, and further comprises 2 to 4 carbon atoms. Non-limiting examples of alkynylene include ethynylene, propynylene and butynylene; and the alkynylene may be optionally substituted with a substituent.

The term "cycloalkyl" refers to a saturated or partially unsaturated, non-aromatic carbocyclic hydrocarbon group containing no ring heteroatoms. The cycloalkyl may be monocyclic, bicyclic or polycyclic, the bicyclic or polycyclic cycloalkyl may be in the form of a fused ring, a spiro ring, a bridged ring or a combination thereof, and may comprise one or more aromatic rings, but the ring system is non-aromatic as a whole, and the attachment site may be on an aromatic ring or a non-aromatic ring. Generally, the cycloalkyl contains 3 to 20 carbon atoms, further contains 3-8 carbon atoms, and still further contains 3-6 carbon atoms; when the cycloalkyl is monocyclic cycloalkyl, the cycloalkyl contains 3-15 carbon atoms, or 3-10 carbon atoms, or 3-8 carbon atoms, or 3-6 carbon atoms; when the cycloalkyl is bicyclic or polycyclic cycloalkyl, the cycloalkyl contains 5-12 carbon atoms, or 5-11 carbon atoms, or 6-10 carbon atoms. Non-limiting examples of cycloalkyl include cyclopropyl, cyclobutyl, cyclopentyl, cyclohexyl, cycloheptyl, butenyl, cyclopentenyl, cyclohexenyl, 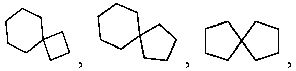 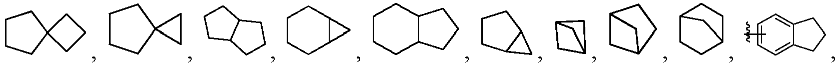 etc., and the cycloalkyl may be optionally substituted with a substituent.

The term "cycloalkylene" refers to a divalent group of cycloalkyl.

The term "aryl" refers to an aromatic carbocycle that does not contain heteroatoms, including monocyclic aryl and fused aryl. Generally, the aryl contains 6 to 14 carbon atoms, and further contains 6 to 10 carbon atoms. Non-limiting examples of aryl include phenyl, naphthyl, anthryl, phenanthryl, aryl and 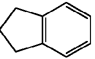 which may be optionally substituted with a substituent.

"Carbocycle" or "carbocyclyl" refers to a saturated, partially unsaturated, or aromatic carbocycle, and its meaning includes aryl and cycloalkyl. The carbocycle may be monocyclic, bicyclic or polycyclic, and the bicyclic or polycyclic carbocycle may be in the form of a bridged ring, a fused ring, a spiro ring and a combination thereof. Generally, the carbocycle contains 3-12 carbon atoms, or 3-10 carbon atoms, or 3-6 carbon atoms. Non-limiting examples of the monocyclic carbocycle include cyclopropyl, cyclobutyl, cyclopentyl, cyclohexyl, cycloheptyl, phenyl, *etc.* A bicyclic bridged ring includes 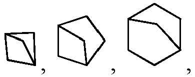 *etc.,* a bicyclic fused ring includes 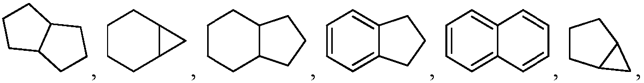 *etc.,* and a bicyclic spiro ring includes 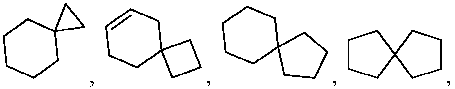 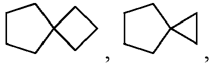 *etc.* The carbocycle may be optionally substituted with a substituent.

"Heterocycloalkyl" refers to a saturated or partially unsaturated non-aromatic carbocycle containing 1, 2, 3, or 4 heteroatoms selected from N, S or O. The heterocycloalkyl may be monocyclic, bicyclic or polycyclic, the bicyclic or polycyclic heterocycloalkyl may be in the form of a bridged ring, a fused ring, a spiro ring or a combination thereof, and may comprise one or more aromatic rings or heteroaromatic rings, but the ring system is non-aromatic as a whole, and the attachment site may be on an aromatic ring or a non-aromatic ring. Generally, the heterocycloalkyl is a 3- to 20-membered ring. When the heterocycloalkyl is monocyclic heterocycloalkyl, the heterocycloalkyl is usually a 3- to 15-membered ring, or a 3- to 10-membered ring, or a 3- to 8-membered ring, or a 3- to 6-membered ring; when the heterocycloalkyl is bicyclic or polycyclic heterocycloalkyl, the heterocycloalkyl is usually a 5- to 12-membered ring, or a 5-to 11-membered ring, or a 6- to 9-membered ring. The heteroatoms N and S include their oxidation states. Non-limiting examples of heterocycloalkyl include azetidinyl, morpholinyl, piperazinyl, piperidyl, tetrahydropyranyl, oxetanyl, pyranyl, azacyclopentenyl, azacyclohexenyl, oxacyclopentenyl, oxacyclohexenyl, etc., and the heterocycloalkyl may be optionally substituted with a substituent.

"Heteroaromatic ring" or "heteroaryl", unless otherwise specified, refers to an aromatic ring containing 1 to 4 heteroatoms selected from N, O or S and their oxidation states, which may be monocyclic, bicyclic or polycyclic, wherein the bicyclic or polycyclic heteroaromatic ring or heteroaryl may be in the form of a bridged ring, a fused ring, a spiro ring and a combination thereof. The bicyclic or polycyclic heteroaromatic ring or heteroaryl can be formed by fusion of heteroaryl to aryl, or of heteroaryl to heteroaryl, wherein the heteroaryl or aryl may be the attachment site. Non-limiting examples of heteroaromatic ring or heteroaryl include furyl, thienyl, pyrrolyl, oxazolyl, thiazolyl, imidazolyl, pyrazolyl, pyridyl, pyrimidyl, pyridazinyl, pyrazinyl, indolyl, purinyl, 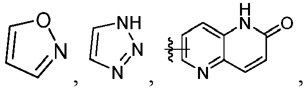 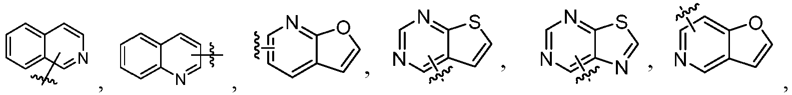 etc. The heteroaryl may be optionally substituted with a substituent.

The term "heterocycle" or "heterocyclyl" refers to a saturated or unsaturated, aromatic or non-aromatic ring containing 1 to 4 heteroatoms selected from N, O or S and their oxidation states, and its meaning includes heteroaryl and heterocycloalkyl. The heterocycle may be in the form of a monocyclic heterocycle, a bicyclic bridged heterocycle, a bicyclic fused heterocycle, a bicyclic spiro heterocycle or a combination thereof. The heterocycle is usually a 3- to 12-membered heterocycle, or a 5- to 12-membered heterocycle, or a 5- to 7-membered heterocycle. Heterocyclyl can be connected to a heteroatom or a carbon atom. Non-limiting examples of heterocyclyl include oxiranyl, aziridinyl, oxetanyl, azetidinyl, 1,3-dioxolanyl, 1,4-dioxolanyl, 1,3-dioxanyl, piperazinyl, azepanyl, pyridyl, furyl, thienyl, pyranyl, N-alkylpyrrolyl, pyrimidyl, pyrazinyl, pyrazolyl, pyridazinyl, imidazolyl, piperidyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzoimidazolyl, benzopyridyl, pyrrolopyridyl, benzodihydrofuryl, azabicyclo[3.2.1]octanyl, azabicyclo[5.2.0]nonanyl, oxatricyclo[5.3.1.1]dodecyl, azaadamantyl, oxaspiro[3.3]heptanyl, 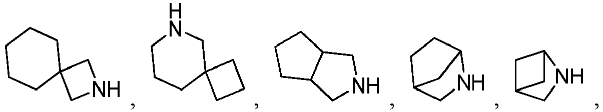 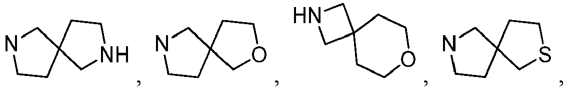 etc., and the heterocycle may be optionally substituted with a substituent.

The term "heterocyclene" refers to a substituted or unsubstituted, saturated or unsaturated, aromatic or non-aromatic, divalent heterocyclyl group. Non-limiting examples of heterocyclene include 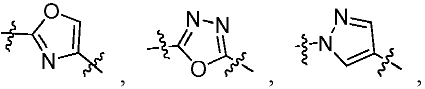 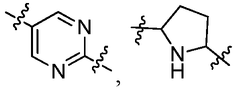 *, etc.*

The term "spiro ring" refers to a polycyclic group sharing one carbon atom (referred to as a spiro atom) between rings, which may contain 0 or at least 1 double or triple bond, and may contain 0 to 5 heteroatoms selected from N, O, S, P, Si and their oxidation states. Generally, a spiro ring is a 6- to 14-membered ring, or a 6- to 12-membered ring, or a 6- to 10-membered ring. Generally, a spiro ring is a spiro ring formed by a three-membered ring and a three-membered ring, a three-membered ring and a four-membered ring, a three-membered ring and a five-membered ring, a three-membered ring and a six-membered ring, a four-membered ring and a four-membered ring, a four-membered ring and a five-membered ring, a four-membered ring and a six-membered ring, a five-membered ring and a five-membered ring or a five-membered ring and a six-membered ring. Non-limiting examples of the spiro ring include: 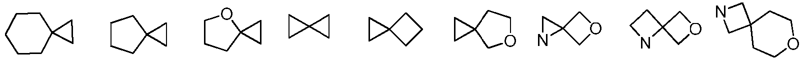 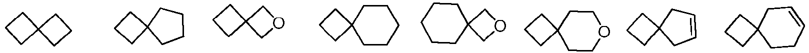 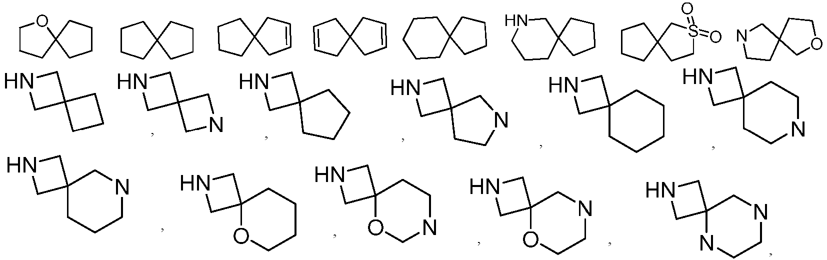 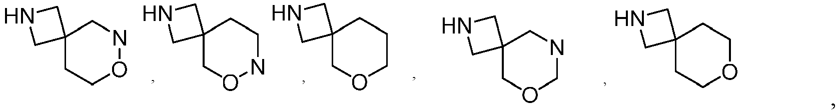 and the spiro ring may be optionally substituted with a substituent.

The term "fused ring ( 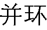 / 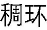 )" refers to a polycyclic group in which the rings share two adjacent ring atoms and one chemical bond. The fused ring may contain one or more double or triple bonds, and may contain 0 to 5 heteroatoms selected from N, S, O, P, Si and their oxidation states. Generally, a fused ring is a 5- to 20-membered ring, or a 5- to 14-membered ring, or a 5- to 12-membered ring or a 5- to 10-membered ring. Generally, a fused ring is in the form of a three-membered ring fused a four-membered ring (indicating a fused ring formed by a three-membered ring and a four-membered ring, and either the three-membered ring or the four-membered ring may be possibly used as the basic ring according to the IUPC nomenclature; similarly hereinafter), a three-membered ring fused a five-membered ring, a three-membered ring fused a six-membered ring, a four-membered ring fused a four-membered ring, a four-membered ring fused a five-membered ring, a four-membered ring fused a six-membered ring, a five-membered ring fused a five-membered ring, a five-membered ring fused a six-membered ring, and a six-membered ring fused a six-membered ring. Non-limiting examples of the fused ring include purine, quinoline, isoquinoline, benzopyran, benzofuran, benzothiophene, 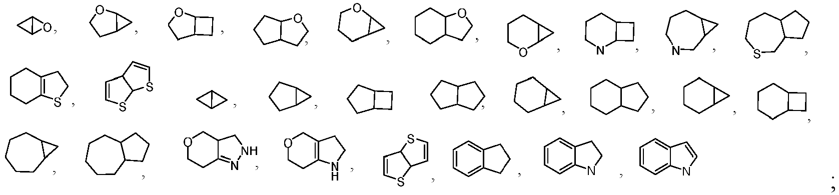 and the fused ring may be aromatic or non-aromatic and is optionally substituted with a substituent.

The term "bridged ring" refers to a ring system in which two non-adjacent ring atoms are shared between two rings, which may contain one or more double or triple bonds. The bridged ring may contain 0 to 5 heteroatoms selected from N, S, O, P, Si and their oxidation states. Generally, the bridged ring has 5 to 20, or 5 to 14, or 5 to 12, or 5 to 10 ring atoms. Non-limiting examples of the bridged ring include adamantane,

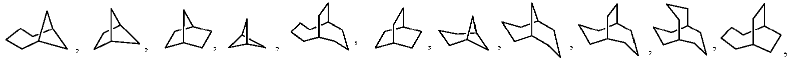

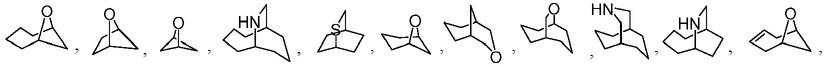

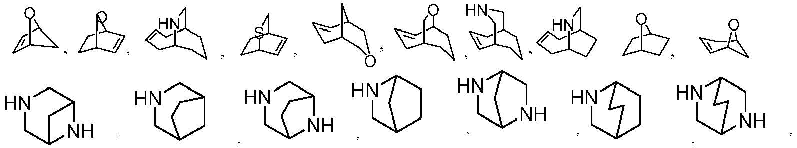

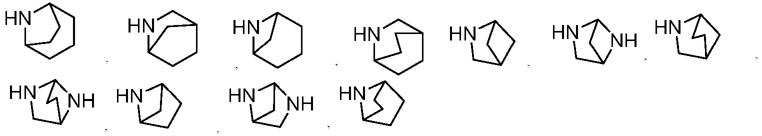

Unless otherwise specified, the term "substitution" or "substituent" refers to any substitution at a position allowed by chemical theory, and the number of substituents conforms to the rules of chemical bonding. Exemplary substituents include, but are not limited to: C₁₋₆ alkyl, C₂₋₆ alkenyl, C₂₋₆ alkynyl, C₃₋₈ heteroalkyl, C₅₋₁₂ aryl, 5- to 12-membered heteroaryl, hydroxyl, C₁₋₆ alkoxy, C₅₋₁₂ aryloxy, thiol, C₁₋₆ alkylthio, cyano, halogen, C₁₋₆ alkylthiocarbonyl, C₁₋₆ alkylcarbamoyl, N-carbamoyl, nitro, silyl, sulfinyl, sulfonyl, sulfoxide, halo C₁₋₆ alkyl, halo C₁₋₆ alkoxy, amino, phosphonic acid, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, -OC(=O)NH(C₁₋₆ alkyl), -NHC(=O)(C₁₋₆ alkyl), -N(C₁₋₆ alkyl)C(=O)(C₁₋₆ alkyl), -NHCO₂(C₁₋₆ alkyl), -NHC(=O)N(C₁₋₆ alkyl)₂, -HC(=O)NH(C₁₋₆ alkyl), -NHC(=O)NH₂, -NHSO₂(C₁₋₆ alkyl), -SO₂N(C₁₋₆ alkyl)₂, -SO₂NH(C₁₋₆ alkyl), -SO₂NH₂, -SO₂C₁₋₆ alkyl, etc.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds described herein or the stereoisomers, deuterated compounds, solvates, pharmaceutically acceptable salts or co-crystals thereof and other components comprising physiologically/pharmaceutically acceptable carriers and/or excipients.

The term "carrier" refers to: a system that does not cause significant irritation to the organism and does not eliminate the biological activity and characteristics of the administered compound and can change the way the drug enters the human body and the distribution of the drug in the body, control the release rate of the drug and delivery the drug to targeted organs. Non-limiting examples of the carrier include microcapsule, microsphere, nanoparticle, liposome, etc.

The term "excipient" refers to: a substance that is not a therapeutic agent per se, but used as a diluent, adjuvant, adhesive and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, an excipient can provide various functions and can be described as a wetting agent, a buffer, a suspending agent, a lubricant, an emulsifier, a disintegrating agent, an absorbent, a preservative, a surfactant, a colorant, a flavoring agent or a sweetening agent. Examples of excipients include, but are not limited to: (1) sugars, such as lactose, glucose and sucrose; (2) starch, such as corn starch and potato starch; (3) cellulose and its derivatives, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatin; (7) talc; (8) excipients, such as cocoa butter or suppository wax; (9) oils, such as peanut oil, cottonseed oil, safflower oil, sesame oil, olive oil, corn oil and soybean oil; (10) diols, such as propylene glycol; (11) polyols, such as glycerin, sorbitol, mannitol and polyethylene glycol; (12) esters, such as ethyl oleate and ethyl laurate; (13) agar; (14) buffers, such as magnesium hydroxide and aluminum hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and (22) other non-toxic compatible substances used in a pharmaceutical preparation.

The term "stereoisomer" refers to an isomer produced as a result of different spatial arrangement of atoms in molecules, including cis-trans isomers, enantiomers and conformational isomers.

The compounds of the present invention also include tautomers thereof, for example, when the present invention describes the left side compound in which the pyrimidine ring is substituted with OH, the right side tautomer compound is also included.

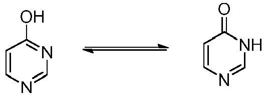

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

### Detailed Description of Embodiments

The technical solutions of the present invention will be described in detail below in conjunction with examples, but the protection scope of the present invention includes but is not limited thereto.

### Test method

The structure of the compound is determined by nuclear magnetic resonance (NMR) or (and) mass spectrometry (MS). The NMR shift (δ) is given in the unit of 10⁻⁶ (ppm). NMR is measured with (Bruker Avance III 400 and Bruker Avance 300) NMR instrument, and the solvent for determination is deuterated dimethyl sulfoxide (DMSO-*d*₆), deuterated chloroform (CDCl₃), and deuterated methanol (CD₃OD), and the internal standard is tetramethylsilane (TMS);
MS is determined with Agilent 6120B (ESI) and Agilent 6120B (APCI); and
HPLC is determined with Agilent 1260DAD high pressure liquid chromatograph (Zorbax SB-C₁₈ 100 × 4.6 mm,3.5 µM);
Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm;
and for the column chromatography, Yantai Huanghai silica gel of 200-300 mesh silica gel is generally used as a carrier.

### Example 1:

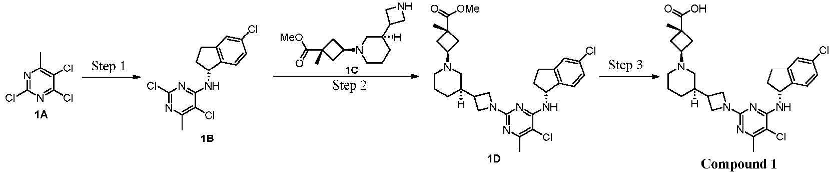

Step 1: **1A** (1.0 g, 5.10 mmol), (1R)-5-chloro-2,3-dihydro-1H-inden-1-amine (0.85 g, 5.10 mmol) was dissloved in acetonitrile (20 mL), triethylamine (1.04 g, 10.20 mmol) was added, and after the addition was completed, the reaction was performed at room temperature for 16 h. The obtained system was concentrated under reduced pressure, water was added (20 mL), and ethyl acetate was used for extraction (20 mL x 3). Organic phases were combined, washed with saturated aqueous sodium chloride solution (20 mL), dried over anhydrous sodium sulfate, and filtered, the filtrate was concentrated under reduced pressure, and the residue was separated by silica gel column chromatography (PE:EA (v/v) = 10:1) to obtain the compound **1B** (1.0 g, yield: 57%).

LC-MS (ESI): m/z =328.0 [M+H]⁺.

Step 2: **1B** (0.2 g, 0.61 mmol), **1C** (synthesized with reference to the method described in patent WO 2019147862) (0.16 g, 0.61 mmol), triethylamine (0.12 g, 1.22 mmol), and cesium fluoride (0.09 g, 0.61 mmol) were dissolved in dimethyl sulfoxide (20 mL) in sequence, and the reaction was performed at 100°C for 4 h. After the reaction was completed, the obtained system was cooled to room temperature, water (20 mL) was added, ethyl acetate was used for extraction (20 mL × 3), the organic phases were combined, washed with saturated aqueous sodium chloride solution (20 mL), dried over anhydrous sodium sulfate, and filtered, the filtrate was concentrated under reduced pressure, and the residue was separated by silica gel column chromatography (DCM:MeOH (v/v) = 10:1) to obtain the compound **1D** of interest (0.12 g, yield: 35%).

LC-MS (ESI): m/z =558.3 [M+H]⁺.

Step 3: Compound **1D** (0.12 g, 0.22 mmol) was dissolved in (THF:H₂O (v/v) = 1:1) solution (4 mL), lithium hydroxide (0.026 g, 1.1 mmol) was added, and the obtained system was reacted under stirring at room temperature for 4 h. After the reaction was completed, the reaction liquid was concentrated under reduced pressure, and the residue was prepared by HPLC to obtain **compound 1** (0.07 g, yield: 60%).

Preparation method: (Instrument name: Waters AutoP; Chromatographic column: Sunfire C18 (19 x 250 mm, 5 µm) Mobile phase: Phase A: Acetonitrile; Phase B: water (containing 0.1% TFA); Gradient: A: from 10% to 50%, 15 min.

¹H NMR (400 MHz, CD₃CN; trifluoroacetic acid salt) δ 7.33 (s, 1H), 7.29-7.22 (m, 2H), 6.95-6.93 (m, 1H), 4.25-4.22 (m, 2H), 4.02-3.99 (m, 2H), 3.58-3.57 (m, 1H), 3.44-3.40 (m, 1H), 3.35-3.33 (m, 1H), 3.13-3.06 (m, 2H), 2.96-2.90 (m, 3H), 2.65-2.54 (m, 6H), 2.42(s, 3H), 2.24-2.22 (m, 2H), 2.18-2.06 (m, 3H), 1.87-1.84 (m, 2H), 1.37 (s, 3H).

LC-MS (ESI): m/z = 544.2 [M+H]⁺.

### Example 2:

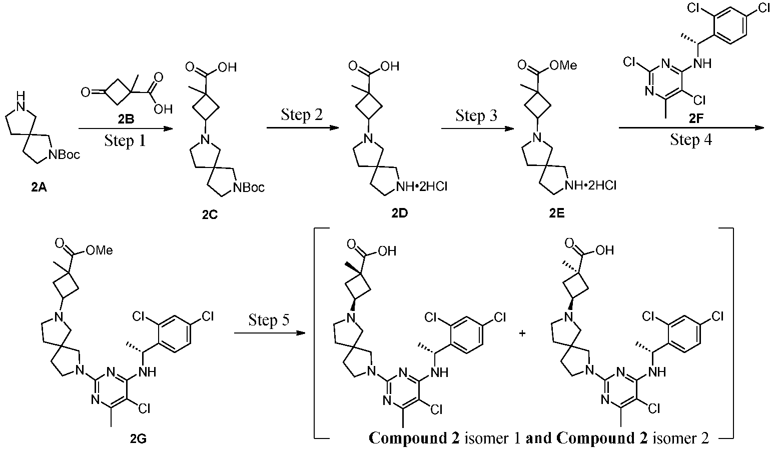

Step 1: Raw material **2A** (3 g, 13.27 mmol) was dissolved in dichloroethane (100 mL), **2B** (2.55 g, 19.91 mmol) was added, stirred and dissolved, sodium borohydride acetate (5.62 g, 26.54 mmol) was added, the obtained system was cooled to 0°C, and acetic acid (0.80 g, 13.27 mmol) was added dropwise. The obtained system was naturally warm to room temperature and reacted overnight. The crude product obtained after concentration under reduced pressure was purified using a medium-pressure preparation instrument Biotage Isolera One (80 g silica gel column, MeOH:DCM = 0% → 20%) to obtain the product **2C** (3.10 g, yield: 68.92%).

LC-MS (ESI): m/z =339.2 [M+H]⁺.

Step 2: Compound **2C** (1.4 g, 4.14 mmol) was dissolved in 1,4-dioxane (10 mL), and hydrogen chloride 1,4-dioxane solution (4M, 10 mL) was added dropwise. The obtained system was stirred at room temperature until the raw material disappeared, and the reaction liquid was concentrated to obtain the compound **2D,** which was directly used in the next reaction without further purification.

LC-MS (ESI): m/z =239.1[M+H]⁺.

Step 3: Compound **2D** was dissolved in methanol (20 mL), and thionyl chloride (0.92 g, 7.72 mmol) was slowly added dropwise at room temperature. After the dropwise addition was completed, the temperature was raised for reflux for 4 h. The reaction was cooled to room temperature, and then concentration was performed to obtain **2E** (1.21 g, two-step yield: 89.4%).

LC-MS (ESI): m/z =253.2[M+H]⁺.

Step 4: Compound **2E** was dissolved in DMSO (10 mL), compound **2F** (synthesized with reference to the method described in patent WO 2019147862) (1.08 g, 3.07 mmol) was added and the obtained system was stirred, and triethylamine (0.62 g, 6.14 mmol) and cesium fluoride (0.93 g, 6.14 mmol) were added in sequence. The obtained system was reacted under nitrogen atmosphere at 100°C for 4 h. The reaction was cooled to room temperature, then water (40 mL) was added, ethyl acetate was used for extraction (30 mL × 3), the combined organic phases were washed with saturated brine (40 mL × 2), dried over anhydrous sodium sulfate, and concentrated, and the crude product was purified using a medium-pressure preparation instrument Biotage Isolera One (24 g silica gel column, eluent: 0-30% EA/PE) to obtain the product **2G** (0.48 g, yield: 27.58%).

LC-MS (ESI): m/z =568.1 [M+H]⁺.

Step 5: Compound **2G** (0.48 g, 0.85 mmol) was dissolved in tetrahydrofuran (5 mL) and water (5 mL), lithium hydroxide monohydrate (0.18 g, 4.25 mmol) was added, and the obtained system was stirred at room temperature overnight. 6N hydrochloric acid was added dropwise to adjust the pH to 6-7, and the mixture was concentrated to obtain a crude product. The crude product was purified by preparative high performance liquid phase chromatography to obtain compound **2,** isomer 1 (0.13 g, yield: 19.58%) and compound **2,** isomer 2 (0.14 g, yield: 21.09%).

HPLC preparation method: instrument: waters 2767 preparative liquid chromatographic instrument; chromatographic column: SunFire @ Prep C18 (19 mm × 250 mm). The sample was dissolved in methanol and filtered through a 0.22 µm filter head to prepare a sample liquid. Preparative chromatography conditions: composition of mobile phases A and B: mobile phase A: acetonitrile, mobile phase B: water (containing 1% TFA), gradient elution, mobile phase A: 10% to 55%, flow rate: 15 mL/min. elution time: 20 min.

HPLC analytical method: instrument: Shimadzu LC-20AT, column: chromatographic column model: Xtimate C18 4.6 * 50mm, 3µm, mobile phase A: 0.05% TFA solution, mobile phase B: acetonitrile, gradient: A 95-5% B 5-95%, flow rate: 1 mL/min, column temperature: 35°C, wavelength: 210 nm/254 nm, acquisition time: 10 min.

Compound **2**, isomer 1 (retention time: 3.253 min): LC-MS (ESI): m/z =554.2 [M+H]⁺.

¹H NMR (400 MHz, CD₃CN, trifluoroacetate salt) δ 10.66 (s, 1H), 7.52 (d, 1H), 7.45-7.37 (m, 2H), 7.35-7.34 (m, 1H), 5.64-5.57(m, 1H), 4.05-3.82 (m, 2H), 3.76-3.40 (m, 4H), 3.35-3.12 (m, 2H), 2.87 -2.60 (m, 3H), 2.47 (s, 3H), 2.26-2.11 (m, 5H), 1.61 (d, 3H), 1.42 (d, 3H).

Compounds **2**, isomer 2 (retention time: 3.230 min): LC-MS (ESI): m/z =554.2 [M+H]⁺.

¹H NMR (400 MHz, CD₃CN, trifluoroacetate salt) δ 10.60 (s, 1H), 7.52 (s, 1H),7.45-7.34 (m, 3H), 5.62-5.59 (m, 1H), 4.07-3.63 (m, 5H), 3.59-3.35 (m, 2H), 2.88-2.75 (m, 3H), 2.50-2.34 (m, 4H), 2.26-2.09 (m, 4H), 1.61 (d, 3H), 1.41 (d, 3H).

### Example 3:

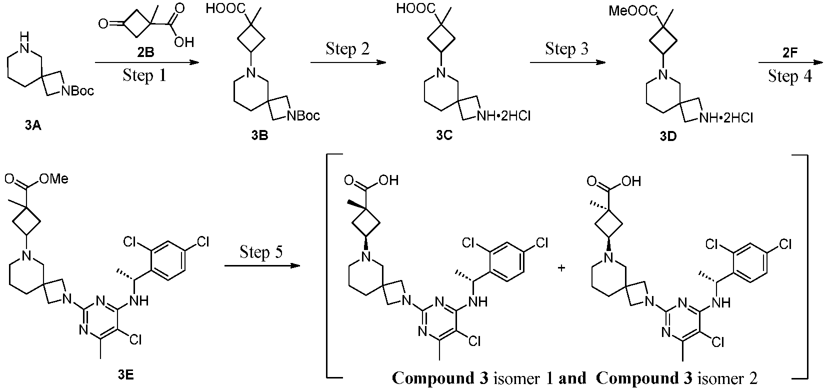

Using compounds **3A** as raw material, the step 1, 2, 3, 4 and 5 of example 2 were referenced to obtain the compound **3** isomer 1 (0.15 g), and compounds **3** isomer 2 (0.13 g).

HPLC analytical method: instrument: Shimadzu LC-20AT, column: chromatographic column model: Xtimate C18 4.6 * 50mm, 3µm, mobile phase A: 0.05% TFA solution, mobile phase B: acetonitrile, gradient: A 95-5% B 5-95%, flow rate: 1 mL/min, column temperature: 35°C, wavelength: 210 nm/254 nm, acquisition time: 10 min. Compounds **3** isomer 1 (retention time: 3.416 min): LC-MS (ESI): m/z =554.2 [M+H]⁺.

¹H NMR (400 MHz, CD₃CN) 7.45 (t, 1H), 7.38-7.37 (m, 1H), 7.28-7.27 (m, 1H), 5.45-5.41 (m, 1H), 3.83-3.44 (m, 6H), 2.50-2.45 (m, 2H), 2.25 (s, 3H), 2.25-2.17 (m, 3H), 1.70-1.66 (m, 5H), 1.49 (d, 3H), 1.32-1.30 (m, 4H).

Compounds **3,** isomer 2 (retention time: 3.443 min): LC-MS (ESI): m/z =554.2 [M+H]⁺.

¹H NMR (400 MHz, CD₃CN) 7.46 (t, 1H),7.39 (d, 1H), 7.28-7.27 (m, 1H), 5.48-5.43 (m, 1H), 3.69-3.43 (m, 4H), 2.95-2.87 (m, 1H), 2.57-2.52 (m, 1H), 2.45-2.35 (m, 4H), 2.26 (s, 3H), 1.95-1.90 (m, 2H), 1.65-1.60 (m, 4H), 1.51 (d, 3H), 1.37-1.29 (m, 4H).

### Example 4:

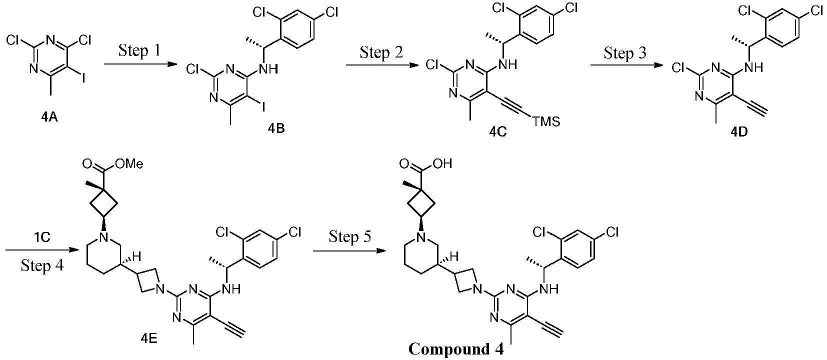

Step 1: **4A** (1.0 g, 3.50 mmol), (R)-1-(2,4-dichlorophenyl)ethan-1-amine (0.66 g, 3.50 mmol), and triethylamine (1.5 mL) were dissolved in methanol (10 mL) in sequence, and the obtained system was stirred at room temperature overnight. The obtained system was concentrated under reduced pressure, the residue was separated and purified by silica gel column chromatography (petroleum ether:ethyl acetate (v/v) = 30:1) to obtain the title compound **4B** (1.2 g, 77%).

LCMS m/z =443.9 [M+ H]⁺.

Step 2: Compound **4B** (0.87 g, 1.97 mmol), trimethylethynylsilane (0.39 g, 3.94 mmol), bis(triphenylphosphine)palladium(II) chloride (69.14 mg, 0.10 mmol), cuprous iodide (18.76 mg, 0.10 mmol) were added to triethylamine (8 mL) in sequence, and the obtained system was stirred at 50°C for 4 h under a nitrogen atmosphere. The reaction was cooled to room temperature, water (10 mL) was added, ethyl acetate was used for extraction (15 mL × 3), the organic phases were combined, washed with saturated aqueous sodium chloride solution (3 mL), dried over anhydrous sodium sulfate, filtered, and concentrated. The residue was separated and purified by silica gel column chromatography (petroleum ether:ethyl acetate (v/v) = 30:1) to obtain the title compound **4C** (310 mg, 38%).

LCMS m/z =340.1 [M-72+H]⁺.

Step 3: Compound **4C** (0.11 g, 0.27 mmol) and potassium carbonate (37.32 mg, 0.54 mmol) were added to methanol (4 mL) in sequence, and the obtained system was stirred at room temperature for 2 h. After the reaction was completed, water (5 mL) was added, the obtained system was extracted with ethyl acetate (10 mL × 3), washed with saturated aqueous sodium chloride solution (2 mL), dried over anhydrous sodium sulfate, filtered, and concentrated. The residue was separated and purified by silica gel column chromatography (dichloromethane:methanol (v/v) = 9:1) to obtain the title compound **4D** (50.0 mg, 54%).

Step 4: **4D** (50.0 mg, 0.15 mmol), **1C** (42.0 mg, 0.15 mmol), triethylamine (0.1 mL, 0.75 mmol), and cesium fluoride (4.6 mg, 0.03 mmol) were added to dimethyl sulfoxide (3 mL) in sequence, and the obtained system was stirred at 100°C for 2 h. The reaction was cooled to room temperature, water (10 mL) was added, the obtained system was extracted with ethyl acetate (10 mL × 3), washed with saturated aqueous sodium chloride solution (2 mL), dried over anhydrous sodium sulfate, filtered, and concentrated. The residue was separated and purified by silica gel column chromatography (dichloromethane:methanol (v/v) = 9:1) to obtain the title compound **4E** (47 mg, 54%).

Step 5: **4E** (20.0 mg, 0.035 mmol), lithium hydroxide (3.4 mg, 0.14mmol) were added to the mixed solvent of methanol (2 mL), tetrahydrofuran (1 mL) and water (1 mL) in sequence, and the obtained system was stirred at room temperature for 2h. The crude product was isolated and purified by preparative HPLC.

Separation method: instrument: waters 2767 preparative liquid chromatographic instrument; chromatographic column: SunFire @ Prep C18 (19 mm x 250 mm); the sample was filtered with a 0.45 µm filter head to prepare a sample liquid; Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min; retention time: 7.0 min, to obtain title **compound 4** (12 mg, 62%).

¹H NMR (400 MHz, DMSO-*d6*) δ 9.86 (s, 1H), 9.52 (s, 1H), 7.57 (d, 1H), 7.46-7.30 (m, 2H), 5.51 (s, 1H), 4.09 (s, 3H), 3.85 (s, 3H), 3.66-3.65 (m, 1H), 3.34 (d, 1H), 3.19 (s, 1H), 2.63 (d, 2H), 2.52 (s, 2H), 2.46 (s, 3H), 2.33 (s, 1H), 2.29-2.17 (m, 2H), 1.90 (d, 1H), 1.64 (s, 1H), 1.50 (d, 1H), 1.45 (d, 2H), 1.31 (s, 3H), 1.00 (s, 1H).

LCMS m/z =556.2 [M+H]⁺.

### Example 5:

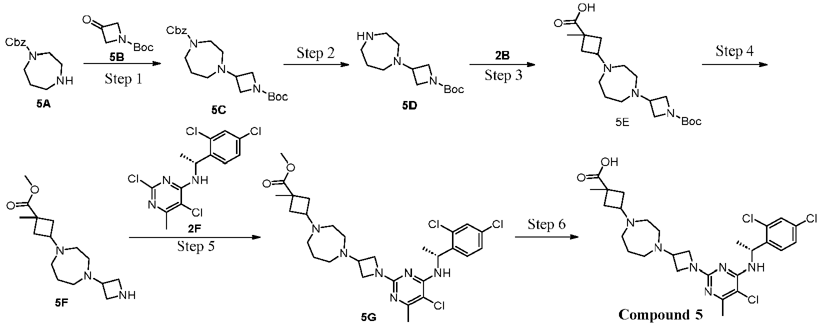

Step 1: Compound **5A** (3.00 g, 12.8 mmol) was dissolved in methanol (15 mL), and 1-Boc-3-azetidinone **5B** (3.29 g, 19.2 mmol) was added and a few drops of acetic acid were added, after the addition was completed, stirring was continued at room temperature for 2 h, and then sodium cyanoborohydride (1.62 g, 25.60 mmol) was added, and the obtained system was reacted under stirring for 18 h. The reaction was poured into water (30 mL), extraction was performed with dichloromethane (20 mL×3), the combined organic phases were dried over anhydrous sodium sulfate, filtered, and concentrated, and the residue was separated and purified by silica gel column chromatography (petroleum ether:ethyl acetate (v/v) = 20:1-10:1) to obtain the title compound **5C** (3.50 g, 70%).

LC-MS (ESI): m/z = 390.2 [M+H]⁺.

Step 2: Compound **5C** (3.50 g, 8.97 mmol) was dissolved in ethyl acetate (300 mL), palladium on carbon (951 mg, 8.97 mmol) was added, and the obtained system was stirred at room temperature for 5 h under a hydrogen atmosphere. The palladium on carbon was removed by filtration on celite and the filtrate was concentrated to obtain the title compound **5D** as a white oily liquid. The obtained compound could be directly used in the next reaction without further purification.

LC-MS (ESI): m/z =256.2 [M+H]⁺.

Step 3: Compound **5D** was dissolved in methanol (10 mL), and compound **2B** (1.21 g, 9.44 mmol) and a few drops of acetic acid were added, after the addition was completed, stirring was continued at room temperature for 5 h, and then sodium cyanoborohydride (793.8 mg, 12.6 mmol) was added, and the obtained system was reacted under stirring for 1 h. The reaction was poured into water (30 mL), the pH was adjusted to 4-5 with aqueous citric acid solution, extraction was performed with dichloromethane (20 mL×3), the combined organic phases were dried over anhydrous sodium sulfate, filtered, and concentrated, and the residue was separated and purified by silica gel column chromatography (dichloromethane:methanol (v/v) = 20:1-10:1) to obtain the title compound **5F** (924.0 mg, two-step yield: 28%).

LC-MS (ESI): m/z =368.3 [M+H]⁺.

Step 4: Compound **5F** (924.0 mg, 2.50 mmol) was dissolved in methanol (8 mL), dichlorosulfoxide (2 mL) was added under an ice bath, after the addition was completed, the obtained system was heated to 70°C, reacted for 1 h, and concentrated to obtain the hydrochloride of the title compound **5F.** The obtained compound could be directly used in the next reaction without further purification.

LC-MS (ESI): m/z =282.2 [M+H]⁺.

Step 5: Using the hydrochloride salt of the above-mentioned **5F,** and compound **2F** (876 mg, 2.52 mmol) as raw materials, the operation method of step 4 of example 2 was referenced to obtain the compound **5G** of interest (449.1 mg, two-step yield: 31%).

LC-MS (ESI): m/z =595.3 [M+H]⁺.

Step 6: Using compound **5G** (449.1 mg, 0.76 mmol) as the raw material, the step 5 of example 2 was referenced to obtain **compound 5** (0.265 g, 61%).

¹H NMR (400 MHz, Methanol-*d*₄) δ 7.48-7.46 (m, 1H), 7.41-7.37 (m, 1H), 7.34-7.29 (m, 1H), 5.62-5.50 (m, 1H), 4.34-3.98 (m, 3H), 3.94-3.76 (m, 2H), 3.75-3.61 (m, 1H), 3.33-3.04 (m, 3H), 2.94 -2.58 (m, 7H), 2.43 (s, 3H), 2.36-2.18 (m, 2H), 2.08 (s, 2H), 1.62-1.51 (m, 3H), 1.48-1.34 (m, 3H).

LC-MS (ESI): m/z = 581.2 [M+H]⁺.

### Example 6:

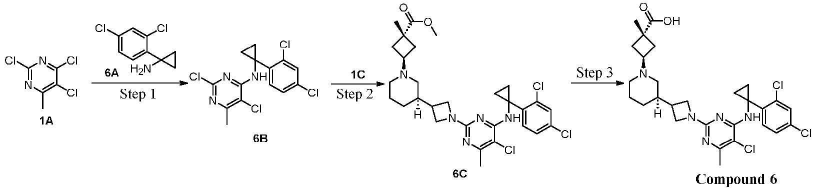

Using **1A** (100 mg, 0.51 mmol) and **6A** (103 mg, 0.51 mmol) as the raw materials, the operation method of steps 1, 2, and 3 of example 1 was referenced to obtain **compound 6** (117 mg).

¹H NMR (400 MHz, CDCl₃) δ 12.18 (s, 1H), 7.51 (d, 1H), 7.39 (d, 1H), 7.30 (d, 1H), 6.97 (s, 1H), 4.28-4.27 (m, 4H), 3.61-3.38 (m, 3H), 2.72 (s, 2H), 2.60 (s, 1H), 2.57-2.49 (m, 2H), 2.47 (s, 2H), 2.42 (s, 3H), 1.99 (s, 2H), 1.84 (s, 1H), 1.35-1.34 (m, 5H), 1.26 (s, 3H), 0.93-0.79 (m, 1H).

LCMS m/z=578.2[M+H]⁺.

### Example 7:

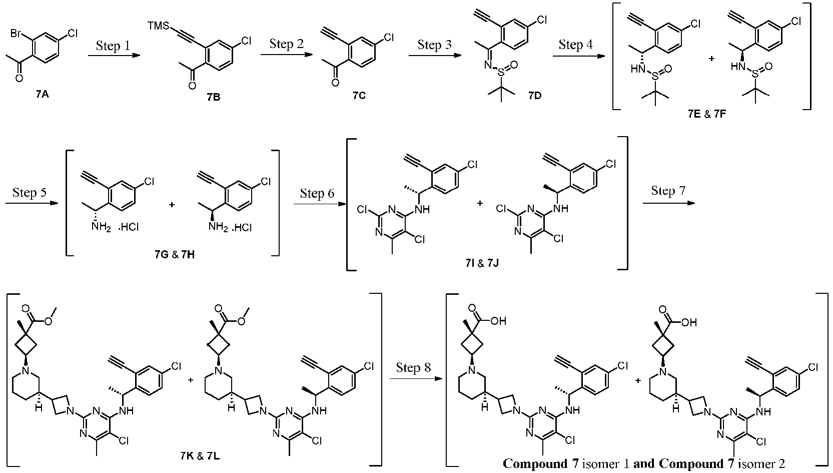

Step 1: 1-(2-bromo-4-chlorophenyl)ethane-1-one (12.0 g, 51.37 mmol), trimethylethynylsilane (7.57 g, 77.05 mmol), bis(triphenylphosphine)palladium(II) chloride (3.61 g, 5.14 mmol) and copper iodide (0.98 g, 5.14 mmol) were added to triethylamine (120 mL) in sequence, and the obtained system was subjected to nitrogen replacement three times, heated to 55°C and reacted for 1.5 h. After the reaction was completed, the obtained system was cooled to room temperature and concentrated, and the residue was separated and purified by column chromatography (petroleum ether:ethyl acetate (v/v) = 100:1-20:1)to obtain the product **7B** (11.0 g, 85.38%).

¹H NMR (400 MHz, DMSO-*d*₆) δ 7.75 (d, 1H), 7.62 (d, 1H), 7.58-7.57 (m, 1H), 2.62 (s, 3H), 0.24 (s, 9H).

Step 2: Potassium carbonate (1.65 g, 11.95 mmol) and methanol (10 mL) were added to a solution of **7B** (6.0 g, 23.92 mmol) in tetrahydrofuran (60 mL) in sequence, and the obtained system was stirred at room temperature for 4 h. After the reaction was completed, water (120 mL) and ethyl acetate (200 mL) were added, extraction and liquid separation were performed, and the aqueous phase was extracted with ethyl acetate (200 mL × 3), the combined organic phases was washed with saturated brine (300 mL), dried over anhydrous sodium sulfate, filtered, concentrated, separated and purified by column chromatography (petroleum ether:ethyl acetate (v/v) = 100:1-10:1) to obtain the product **7C** (4.0 g, 93.62%).

¹H NMR (400 MHz, DMSO-*d6*) δ 7.80 (d, 1H), 7.68 (d, 1H), 7.61-7.60 (m, 1H), 4.58 (s, 1H), 2.61 (s, 3H).

Step 3: **7C** (2 g, 11.20 mmol) was dissolved in tetrahydrofuran (20 mL), and tetraisopropyl titanate (6.37 g, 22.40 mmol) and R-(+)-tert-butylsulfinamide (1.36 g, 11.20 mmol) were added in sequence, the obtained system was heated to 60°C and reacted for 16 h. After the reaction was completed, the reaction was cooled to room temperature, brine (30 mL) was added, a solid precipitated, and filtered, the filter cake was washed with ethyl acetate (100 mL), the filtrate was subjected to extraction and liquid seperation and the organic phase was dried over anhydrous sodium sulfate, filtered, and concentrated, and subjected to column chromatography (petroleum ether:ethyl acetate (v/v) = 20:1-5:1) to obtain **7D** (1.4 g, 44.36%).

LCMS (ESI): m/z =282.0 [M+H]⁺.

Step 4: **7D** (1.40 g, 4.97 mmol) was dissolved in a mixed solvent (v:v=98:2) (15 mL) of tetrahydrofuran and water, and the obtained system was cooled to - 30°C and sodium borohydride (0.38 g, 9.94 mmol) was added in portions, and the reaction was performed for 2 h. After the reaction was completed, water (20 mL) and ethyl acetate (30 mL) were added, extraction was performed, the aqueous phase was extracted with ethyl acetate (30 mL), the combined organic phases were washed with saturated brine, dried over anhydrous sodium sulfate, filtered, and concentrated, and separated and purified by column chromatography (petroleum ether:ethyl acetate (v/v) = 10:1-3:1) to obtain **7E** (Rf = 0.4 (petroleum ether:ethyl acetate (v/v) = 3:1), 0.7 g, 49.62%) and isomers thereof **7F**(Rf = 0.3 (petroleum ether:ethyl acetate (v/v) = 3:1), 0.7 g, 49.62%).

Isomer **7E:** ¹H NMR (400 MHz, DMSO-*d6*) δ 7.56-7.45 (m, 3H), 5.47 (d, 1H), 4.89-4.78 (m, 1H), 4.58 (s, 1H), 1.44 (d, 3H), 1.10 (s, 9H).

Isomer **7F:** ¹H NMR (400 MHz, DMSO-*d6*) δ 7.62-7.57 (m, 1H), 7.53-7.47 (m, 2H), 5.87 (d, 1H), 4.81-4.80 (m, 1H), 4.58 (s, 1H), 1.35 (d, 3H), 1.10 (s, 9H).

Step 5: **7E** (0.70 g, 2.47 mmol) was added to hydrogen chloride 1,4-dioxane solution (4M, 10 mL), the obtained system was stirred at room temperature for 1 h, and concentrated, a mixed solvent of petroleum ether and ethyl acetate ((v/v) = 5:1, 10 mL) was added, stirred, and filtered, and the filter cake was collected to obtain **7G** (0.44 g, 75.32%).

LCMS (ESI): m/z =180.1 [M+H]⁺.

**7H** was obtained by using **7F** as the raw material according to the above synthesis method.

Step 6: Using 2,4,5-trichloro-6-methylpyrimidine (0.39 g, 1.95 mmol) and **7G** (0.35 g, 1.95 mmol) as raw materials, the operation method of step 1 of example 1 was referenced to obtain 7**I** (0.43 g, 65.34%).

LCMS (ESI): m/z =340.0 [M+H]⁺.

**7J** was obtained by using **7H** as the raw material according to the above synthesis method.

Step 7: with **1C** (0.533 g, 1.76 mmol) and 7**I** (0.3 g, 0.88 mmol) as the raw materials, and the operation method of step 2 of example 1 was referenced to obtain **7K** (0.15 g, 29.88%).

LCMS (ESI): m/z =570.4[M+H]⁺.

**7L** was obtained by using **7J** as the raw material according to the above synthesis method.

Step 8: Using **7K** (150 mg, 0.26 mmol) as raw material, the operation method of step 3 of example 1 was referenced to obtain **compound 7** isomer 1 (35 mg, 24.19%).

¹H NMR (400 MHz, DMSO-*d6*) δ 7.48-7.36 (m, 3H), 7.20 (d, 1H), 5.51-5.50 (m, 1H), 4.55 (s, 1H), 3.84 (t, 1H), 3.78 (s, 1H), 3.59-3.48 (m, 2H), 2.57 (m, 2H), 2.44-2.43 (m, 2H), 2.34-2.24 (m, 1H), 2.17 (s, 3H), 1.71-1.53 (m, 5H), 1.44 (d, 4H), 1.39-1.32 (m, 1H), 1.32-1.28 (m, 1H), 1.27 (s, 3H), 1.24 (m, 1H), 0.82-0.69 (m, 1H).

LCMS m/z =556.2 [M+H]⁺.

**Compound 7** isomer 2 was obtained by using **7L** as the raw material according to the above synthesis method.

¹H NMR (400 MHz, DMSO-*d6*) δ 7.53-7.33 (m, 3H), 7.19 (d, 1H), 5.50-5.49 (m, 1H), 4.54 (d, 1H), 3.84-3.83 (m, 1H), 3.76 (s, 1H), 3.54-3.53 (m, 2H), 2.65 (d, 2H), 2.48-2.24 (m, 3H), 2.17 (s, 3H), 1.97-1.17 (m, 15H), 0.89-0.72 (m, 1H).

LCMS m/z =556.4 [M+H]⁺.

### Example 8:

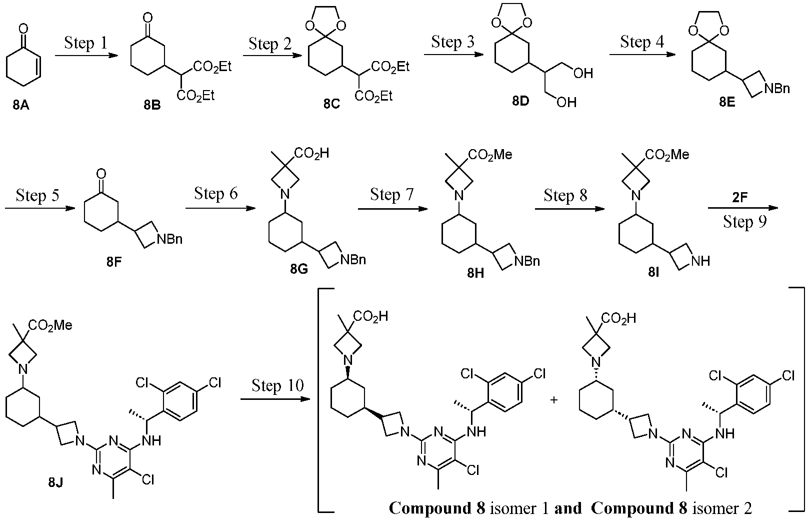

Step 1: Compound **8A** (31 g, 0.32 mol) was dissolved in tetrahydrofuran (1.5 L) at room temperature, diethyl malonate (51.6 g, 0.32 mol) was added dropwise, after the obtained system was stirred for 5 min, a solution of potassium tert-butoxide (39.8 g, 0.35 mol) in tetrahydrofuran (0.5 L) was added dropwise, after the dropwise addition was completed, the reaction was performed at room temperature for 1 h. TLC showed that the raw materials were reacted completely, and dilute hydrochloric acid (2N, 200 mL) was added dropwise to adjust the pH to 3, and the mixture was extracted with EA (200 mL×2). The combined organic phases were washed with saturated aqueous sodium bicarbonate solution (100 mL×2), washed with saturated brine (100 mL×2), dried over anhydrous sodium sulfate, filtered, and concentrated to obtain 70 g (0.27 mol, yield: 85%) of a crude product of compound **8B.**

Step 2: At room temperature, the crude product of compound **8B** (70 g, 0.27 mol), ethylene glycol (50.3 g, 0.81 mol), and p-toluenesulfonic acid (4.7 g, 27 mmol) were dissolved in toluene (700 mL) in sequence, a water separator and a condenser were installed, the temperature was raised to 120°C and the reaction was performed overnight. TLC showed that the reaction was completed. The mixture was cooled to room temperature, concentrated under reduced pressure, diluted with water (200 mL), extracted with EA (200 mL × 2), and the combined organic phases were washed with saturated aqueous sodium bicarbonate solution (100 mL × 2), washed with saturated brine (100 mL × 2), dried over anhydrous sodium sulfate, filtered and concentrated to obtain a crude product. The crude product was separated and purified by column chromatography (10% EA in PE) to obtain compound **8C** (53 g, yield: 65%).

Step 3: Under an ice bath, lithium aluminum tetrahydride (8.34 g, 0.22 mol) was added in portions to diethyl ether (300 mL), and a solution of compound **8C** (44 g, 0.15 mol) in diethyl ether (300 mL) was added dropwise. After the dropwise addition was completed, the mixture was heated to reflux for 1 h. TLC showed that the reaction was completed. The mixture was cooled to room temperature. Under an ice bath, water (8.4 mL), 15% NaOH aqueous solution (8.4 mL), and water (25 mL) were added dropwise in sequence to quench the reaction. Filtration was perfomed and the filtrate was concentrated under reduced pressure to obtain a crude product which was separated and purified by column chromatography (7% MeOH in CH₂Cl₂) to obtain compound **8D** (26 g, 0.12 mol).

Step 4: At -20° and under nitrogen atmosphere, compound **8D** (10.8 g, 50 mmol) and diisopropylethylamine (16.1 g, 125 mmol) were added to dry acetonitrile (250 mL), and trifluoromethanesulfonic anhydride (29.7 g, 105 mmol) was slowly added dropwise. After the obtained system was reacted under stirring for 1 h, diisopropylethylamine (16.1 g, 125 mmol) and benzylamine (8.1 g, 75 mmol) were added in sequence, and then the obtained system was heated to 90°C for 2 h. TLC showed the reaction was completed. The mixture was cooled to room temperature, diluted with water (100 mL) and EA (100 mL), and extracted, and the organic phase was washed with water (200 mL × 2), dried over anhydrous sodium sulfate, filtered, and concentrated to obtain a crude product which was purified by silica gel column chromatography (5% MeOH in CH₂Cl₂) to obtain compound **8E** (10.81 g, yield: 75%).

LC-MS (ESI): m/z = 288.3 [M+H]⁺.

Step 5: Compound **8E** (3.84 g, 13.4 mmol) was added to tetrahydrofuran (20 mL) at room temperature, the obtained system was cooled to 0°C, hydrogen chloride 1,4-dioxane solution (3M, 22.3 mL) was added dropwise, the reaction mixture was heated to room temperature and stirred for 1 h. TLC/LCMS showed that the reaction was completed. Water (20 mL) and EA (30 mL) were added for dilution and extraction was perfomed. The obtained aqueous phase was adjusted to pH 9 with saturated aqueous sodium bicarbonate solution and extracted with dichloromethane (30 mL × 2). The combined organic phases were dried over anhydrous sodium sulfate, filtered, and concentrated to obtain a crude product which was purified by silica gel column chromatography (3% MeOH in CH₂Cl₂) to obtain compound **8F** (2.56 g, yield: 79%).

LC-MS (ESI): m/z = 244.2 [M+H]⁺.

Step 6: Compound **8F** (600 mg, 2.47 mmol), 3-methyl-3-azetidinecarboxylic acid (285 mg, 2.47 mmol), and sodium triacetoxyborohydride (1.05 g, 4.94 mmol) were added to 1,2-dichloroethane (20 mL) in sequence at room temperature. The obtained system was cooled to 0°C, acetic acid (149 mg, 2.47 mmol) was added dropwise, and the obtained system was warmed to room temperature for reaction overnight. TLC/LCMS showed that the reaction was completed, and the crude product obtained after concentration under reduced pressure was separated and purified by silica gel column chromatography (70% MeOH in CH₂Cl₂) to obtain compound **8G** (650 mg, yield: 77%).

LC-MS (ESI): m/z = 343.2 [M+H]⁺.

Step 7: Compound **8G** (620 mg, 1.74 mmol) was added to methanol (50 mL) at room temperature, the temperature was lowered to 0°C, thionyl chloride (2.07 g, 17.4 mmol) was added dropwise, and the temperature was raised to room temperature for reaction for 1 h. TLC/LCMS showed that the reaction was completed, and after concentration under reduced pressure, the hydrochloride salt of compound **8H** was obtained, which was directly used in the next reaction without further purification.

LC-MS (ESI): m/z = 357.2 [M+H]⁺.

Step 8: The hydrochloride salt of compound **8H** was added to methanol (10 mL) at room temperature, the obtained system was subjected to hydrogen replacement three times, and then heated to 50°C and reacted overnight. TLC/LCMS showed that the reaction was completed, filtration was performed, and the filtrate was concentrated under reduced pressure to obtain the hydrochloride salt of compound **8I**, which was used directly in the next reaction without further purification.

LC-MS (ESI): m/z = 267.3 [M+H]⁺.

Step 9: Using the hydrochloride salt of **8I**, and compound **2F** (537 mg, 1.53 mmol) as raw materials, the operation method of step 2 of example 1 was referenced to obtain compound **8J** (240 mg, 0.413 mmol, three-step yield: 24%).

LC-MS (ESI): m/z = 580.2/582.2 [M+H]⁺.

Step 10: Using compound **8J** (240 mg, 0.413 mmol) as the raw material, the operation method of step 3 of example 1 was referenced to obtain **compound 8** isomer 1 (58.7 mg, 24.2%) and **compound 8** isomer 2 (52.3 mg, 21.8%).

HPLC analytical method: instrument: SHIMADZU LC-30AD sf, column: Chiralcel C2-3 50×4.6mm I.D., 3µm, mobile phase: A for CO₂, B for MeOH +ACN (0.05% DEA), gradient: B 50%, flow rate: 3mL/min, back pressure: 100 bar, column temperature: 35°C, wavelength: 220 nm.

**Compound 8** isomer 1 (retention time: 0.901 min): ¹H NMR (400 MHz, Chloroform-d) δ 7.36 (s, 1H), 7.23 (d, 1H), 7.18 (d, 1H), 5.52 (m, 1H), 5.41-5.43 (m, 1H), 3.85-3.98 (m, 2H), 3.63 (m, 1H), 3.23-3.42 (m, 2H), 2.81 (m, 2H), 2.51 (m, 2H), 2.30 (s, 3H), 2.01 (m, 2H), 1.75 (m, 2H), 1.51 (m, 3H), 1.46 (m, 3H), 1.26 (s, 3H), 1.23 (m, 2H), 0.86 (m, 1H).

LC-MS (ESI): m/z = 566.4/568.4 [M+H]⁺.

**Compound 8** isomer 2 (retention time: 1.243 min): ¹H NMR (400 MHz, Chloroform-d) δ 7.34 (s, 1H), 7.23 (d, 1H), 7.18 (d, 1H), 5.51 (m, 1H), 5.41-5.43 (m, 1H), 3.88-3.98 (m, 2H), 3.63 (m, 1H), 3.23-3.42 (m, 2H), 2.82 (m, 2H), 2.49 (m, 2H), 2.28 (s, 3H), 2.01-2.03 (m, 2H), 1.75 (m, 2H), 1.52 (m, 3H), 1.46 (m, 3H), 1.25 (s, 3H), 1.21-1.22 (m, 2H), 0.85 (m, 1H).

LC-MS (ESI): m/z = 566.4/568.4 [M+H]⁺.

### Example 9:

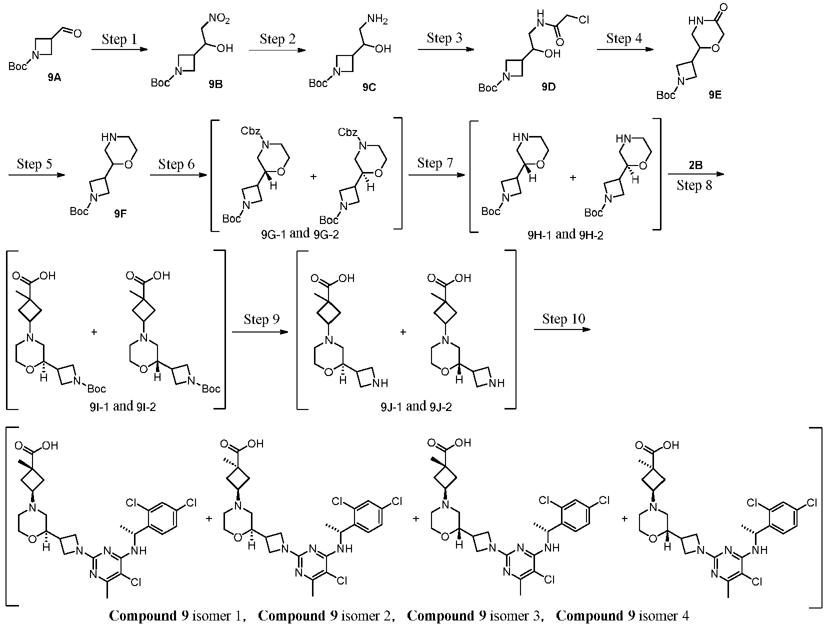

Step 1: Compound **9A** (20.0 g, 108.0 mmol) was dissolved in methanol (200 mL), and triethylamine (21.8 g, 218.0 mmol) and nitromethane (26.4 g, 432.0 mmol) were added in sequence. After the addition was completed, the mixture was stirred at room temperature for 17 h. The crude product of compound **9B** (26.5 g, 107.6 mmol) was obtained by concentration and used directly in the next step without further purification.

Step 2: Compound **9B** (26.5 g, 107.6 mmol) was dissolved in methanol (250 mL), and Pd/C (5.2 g, Pd content 10%) was added and reacted under hydrogen atmosphere for 15 h. Filtration was performed and the filtrate was concentrated to obtain the crude product of compound **9C** (21.1 g, 90.6%), which was used directly in the next step without further purification.

M/Z (ESI): m/z =217.1 [M+H]⁺.

Step 3: Compound **9C** (21.1 g, 91.6 mmol) was dissolved in a mixed solvent of tetrahydrofuran (200 mL) and water (100 mL), and sodium bicarbonate (24.6 g, 292.6 mmol) was added. The obtained system was cooled to 5°C, and chloroacetyl chloride (22.1 g, 195.1 mmol) was added dropwise. The temperature was controlled between 5°C and 10°C, and after the dropwise addition was completed, the obtained system was warmed to room temperature and reacted for 1 h. After the reaction was completed, water (100 mL) was added, and ethyl acetate was used for extraction (300 mL × 2). The combined organic phase was washed with saturated brine, dried over anhydrous sodium sulfate, filtered, and concentrated. The residue was separated and purified by silica gel column chromatography (petroleum ether:ethyl acetate (v/v) = 1:2) to obtain the title compound **9D** (17.2 g, 60.2%).

Step 4: Compound **9D** (17.2 g, 58.8 mmol) was dissolved in tert-butanol (170 mL), and potassium tert-butoxide (13.2 g, 117.5 mmol) was added in portions. After the addition was completed, the obtained system was heated to 50°C and reacted for 2 h. After the reaction was completed, the mixture was cooled to room temperature, saturated aqueous ammonium chloride solution (200 mL) was added to quench the reaction, and the mixture was extracted with ethyl acetate (150 mL × 2). The combined organic phases were washed with saturated brine, dried over anhydrous sodium sulfate, and filtered. The filtrate was concentrated and the residue was separated and purified by silica gel column chromatography (petroleum ether:ethyl acetate (v/v) = 1:2) to obtain the title compound **9E** (13.5 g, 89.6%).

M/Z (ESI): m/z =201.1 [M+H-tBu]⁺.

Step 5: Under nitrogen atmosphere, compound **9E** (13.5 g, 52.7 mmol) was dissolved in tetrahydrofuran (150 mL), the obtained system was cooled to 0°C, and 2-hydrobis(dimethoxyethoxy) sodium aluminate (45.1 mL, 3.5 mol/L solution in toluene) was slowly added dropwise. After the dropwise addition was completed, the reaction was continued at 0-5°C for 3 h. After the reaction was completed, 10% sodium hydroxide aqueous solution (45 mL) was slowly added dropwise to quench the reaction. After quenching, anhydrous magnesium sulfate was added and filtration was performed on celite. The filtrate was concentrated and the residue was separated and purified by silica gel column chromatography (dichloromethane:methanol (v/v) = 10:3) to obtain the title compound **9F** (7.6 g, 59.5%).

M/Z (ESI): m/z =187.1 [M+H-tBu]⁺.

Step 6: Compounds **9F** (7.6 g, 31.4 mmol) was dissolved in a mixed solvent of tetrahydrofuran (50 mL) and water (50 mL), and sodium bicarbonate (7.9 g, 94.1 mmol) was added. The obtained system was cooled to 0-5°C, and benzyl chloroformate (6.4 g, 37.6 mmol) was slowly added dropwise. After the dropwise addition was completed, the reaction was continued at 0-5°C for 1 h. After the reaction was completed, water (100 mL) was added, and ethyl acetate (150 mL × 2) was used for extraction. The combined organic phase was washed with saturated brine, dried over anhydrous sodium sulfate, and filtered, and the filtrate was concentrated. The residue was separated and purified by silica gel column chromatography (petroleum ether:ethyl acetate (v/v) = 3:1) to obtain 9.2 g of the racemate. The racemate was separated by chiral preparative HPLC to obtain two isomers, compound **9G-**1 (3.8 g, 32.2%) and compound **9G-**2 (3.4 g, 28.8%).

Chiral HPLC analytical method: 1. Instruments: SHIMADZU LC-30AD sf; 2. Chromatographic column: Chiralpak IC -3 50×4.6mm I.D., 3µm; 3. Mobile phase system: A for CO2 and B for MeOH (0.05%DEA); 4. Gradient: B 5%-40%; 5. Flow rate: 3mL/min. Compounds **9G** -1 (retention time 1.59 min) and compound **9G** -2 (retention time 2.02 min).

Preparative chromatography separation conditions: 1. Instruments: Waters 150 SFC; 2. Chromatographic column: Chiralpak IC -Column (250×30mm, I.D 30mm, 10um particle size); 3. Mobile phase system: A for CO₂ and B for MeOH (0.1%NH₃•H₂O); 4. Gradient: B 45%; 5. Flow rate: 180 mL/min.

Analytical method: 1. Instruments: SHIMADZU LC-30AD sf; 2. Chromatographic column: Chiralpak IC -3 50×4.6mm I.D., 3µm; 3. Mobile phase system: A for CO2 and B for MeOH (0.05%DEA); 4. Gradient: B 5%-40%; 5. Flow rate: 3mL/min. Step 7: Compounds **9G** -1 (3.8 g, 10.1 mmol) was dissolved in methanol (40 mL), Pd/C (0.8 g, Pd content 10%) was added, and the reaction was carried out for 3 h under hydrogen atmosphere. Filtration was performed and the filtrate was concentrated to obtain the title compound **9H**-1 (2.3 g, 94.1%).

Referring to the above operation, compound **9G**-2 (3.4 g, 9.0 mmol) was used as the raw material to obtain the title compound **9H**-2 (2.1 g, 96.0%).

Step 8: Compound **9H**-1 (1.1 g, 4.5 mmol) and 3-carbonyl-1-methyl-cyclobutanecarboxylic acid (0.64 g, 5.0mmol) were dissolved in 1,2-dichloroethane in sequence (15 mL), and glacial acetic acid (0.27 g, 4.5 mmol) was added. Sodium triacetoxyborohydride (1.4 g, 6.8 mmol) was added in portions, and the reaction was carried out for 15 h after the addition was completed. After the reaction was completed, water (30 mL) was added to quench the reaction, and dichloromethane (50 mL × 2) was used for extraction. The combined organic phases were washed with saturated brine, dried over anhydrous sodium sulfate, and filtered. The filtrate was concentrated and the residue was separated and purified by silica gel column chromatography (dichloromethane:methanol (v/v) = 10:1) to obtain the title compound **9I**-1 (1.0 g, 62.1%).

Referring to the above operation, compound **9H**-2 (1.0 g, 4.1 mmol) was used as the raw material to obtain the title compound **9I**-2 (0.86 g, 58.7%).

M/Z (ESI): m/z =299.2[M+H-tBu]⁺.

Step 9: Compound **9I**-1 (1.0 g, 2.8 mmol) was dissolved in dichloromethane (10 mL), trifluoroacetic acid (3 mL) was added, and the reaction was performed at room temperature for 30 min. After the reaction was complete, the obtained system was directly concentrated to obtain the trifluoroacetate salt of compound **9J**-1 (1.3 g, 95.6%).

Referring to the above operation, compound **9I**-2 (0.86 g, 2.4 mmol) was used as the raw material to obtain the trifluoroacetate salt of the title compound **9J**-2 (1.1 g, 93.8%).

Step 10: Compound **2F** (0.5 g, 1.4 mmol) and compound **9J**-1 (1.3 g, 2.7 mmol) were dissolved in dimethyl sulfoxide (5 mL) in sequence, and N,N-diisopropylethylamine (0.55 g, 4.3 mmol) and cesium fluoride (0.43 g, 2.8 mmol) were added, the temperature was raised to 100°C and the reaction was performed for 5 h. After the reaction was completed, the mixture was cooled to room temperature, and water (50 mL) was added, followed by extraction with ethyl acetate (50 mL ×3). The combined organic phases were washed with saturated brine, dried over anhydrous sodium sulfate, and filtered. The filtrate was concentrated and the residue was separated and purified by silica gel column chromatography (dichloromethane:methanol (v/v) = 10:1) to obtain the title **compound 9** isomer 1 (Rf = 0.6 (dichloromethane:methanol = 10:1), 160.0 mg, 19.8%) and the title **compound 9** isomer 2 (Rf = 0.4 (dichloromethane:methanol = 10:1), 80.0 mg, 9.9%).

**Compound 9** isomer 1: ¹H NMR (400 MHz, CDCl₃) δ 7.34 (d, 1H), 7.21 (d, 1H), 7.17-7.15 (m, 1H), 5.55 (d, 1H), 5.46-5.40 (m, 1H), 3.99-3.95 (m, 1H), 3.90-3.66 (m, 6H), 2.88-2.55 (m, 6H), 2.31 (s, 3H), 2.01-1.93 (m, 3H), 1.58-1.56 (m, 1H), 1.51 (d, 3H), 1.42 (s, 3H).

M/Z (ESI): m/z =568.2[M+H]⁺.

**Compound 9** isomer 2: ¹H NMR (400 MHz, CDCl₃) δ 7.35 (d, 1H), 7.22 (d, 1H), 7.19-7.16 (m, 1H), 5.55 (d, 1H), 5.47-5.41 (m, 1H), 4.02-3.75 (m, 7H), 3.03-2.96 (m, 2H), 2.88-2.85 (m, 1H), 2.62-2.56 (m, 3H), 2.32 (s, 3H), 2.16-2.06 (m, 3H), 1.76-1.70 (m, 1H), 1.51 (d, 3H), 1.42 (s, 3H).

M/Z (ESI): m/z =568.2[M+H]⁺.

Referring to the above operation, compound **9J**-2 (1.1 g, 2.4 mmol) was used as the raw material to obtain the title **compound 9** isomer 3 (Rf=0.6 (dichloromethane:methanol=10:1), 110.0 mg, 13.6%) and the title **compound 9** isomer 4 (Rf=0.4 (dichloromethane:methanol=10:1), 70.0 mg, 8.7%).

**Compound 9** isomer 3: ¹H NMR (400 MHz, CDCl₃) δ 7.34 (d, 1H), 7.22 (d, 1H), 7.18-7.16 (m, 1H), 5.54 (d, 1H), 5.46-5.40 (m, 1H), 4.00-3.96 (m, 1H), 3.90-3.60 (m,6H), 2.83-2.55 (m, 6H), 2.30 (s, 3H), 1.95-1.91 (m, 3H), 1.58-1.56 (m, 1H), 1.51 (d, 3H), 1.40 (s, 3H).

M/Z (ESI): m/z =568.2[M+H]⁺.

**Compound 9** isomer 4: ¹H NMR (400 MHz, CDCl₃) δ 7.35 (d, 1H), 7.23 (d, 1H), 7.18-7.16 (m, 1H), 5.52 (d, 1H), 5.46-5.40 (m, 1H), 3.96-3.75 (m, 7H), 3.00-2.96 (m, 2H), 2.88-2.85 (m, 1H), 2.62-2.53 (m, 3H), 2.31 (s, 3H), 2.17-2.05 (m, 3H), 1.73-1.68 (m, 1H), 1.51 (d, 3H), 1.41 (s, 3H).

M/Z (ESI): m/z =568.2[M+H]⁺.

### Example 10:

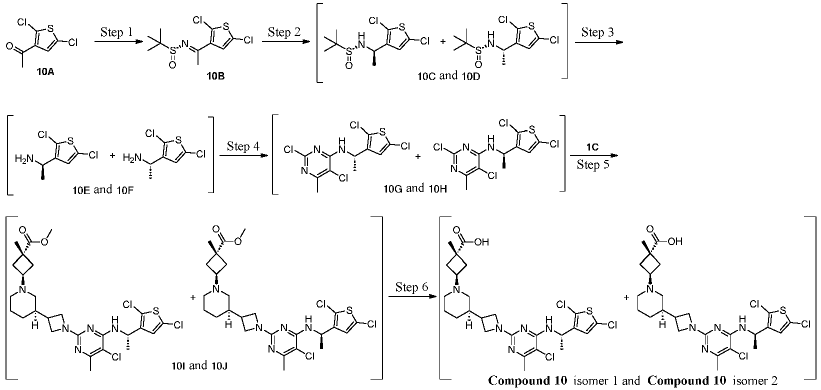

Using 2,5-dichloro-3-acetylthiophene (2 g, 10.26 mmol) as the raw material, the operation methods of steps 3 and 4 (compound **10C** (Rf=0.6 (petroleum ether:ethyl acetate=5:1)) and compound **10D** Rf=0.4 (petroleum ether:ethyl acetate=5:1)), 5, 6, 7, and 8 of example 7 were referenced to obtain **compound 10** isomer 1 (70 mg) and **compound 10** isomer 2 (67 mg).

**Compound 10** isomer 1: ¹H NMR (400 MHz, CDCl₃) δ 6.69 (s, 1H), 5.36 (d, 1H), 5.20-5.19 (m, 1H), 4.04-4.03 (m, 1H), 3.98-3.97 (m, 1H), 3.80-3.79 (m, 1H), 3.65 (s, 1H), 3.11 (s, 2H), 3.02 (d, 1H), 2.60-2.59 (m, 2H), 2.30 (s, 3H), 2.21 (d, 2H), 1.94 (s, 2H), 1.86-1.70 (m, 3H), 1.62 (s, 1H), 1.49 (d, 3H), 1.38 (s, 3H), 0.87 (d, 1H).

LCMS m/z = 572.1 [M+H]⁺.

**Compound 10** isomer 2: ¹H NMR (400 MHz, CDCl₃) δ 6.68 (s, 1H), 5.35 (d, 1H), 5.18-5.17 (m, 1H), 4.03-4.02 (m, 1H), 3.96-3.95 (m, 1H), 3.75-3.74 (m, 2H), 3.01 (d, 3H), 2.59 (d, 2H), 2.30 (s, 4H), 2.11 (s, 2H), 1.88 (s, 2H), 1.82-1.66 (m, 3H), 1.55 (s, 1H), 1.49 (d, 3H), 1.39 (s, 3H), 0.88 (s, 1H).

LCMS m/z = 572.1 [M+H]⁺.

### Compound 11:

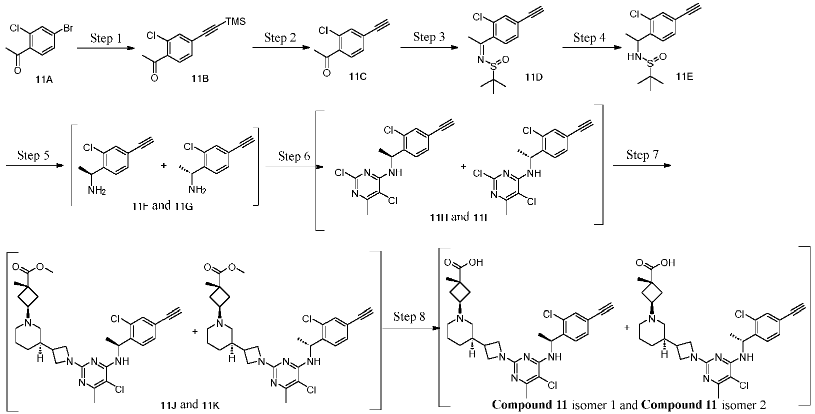

Using **11A** (15.0 g, 64.21 mmol) as the raw material, the operation method of steps 1 to 8 of example 7 was referenced to obtain **compound 11** isomer 1 (47 mg) and **compound 11** isomer 2 (45 mg), among which isomers **11F** (retention time 1.01 min) and **11G** (retention time 1.12 min) (Chiral HPLC analytical method: 1. Instruments: SHIMADZU LC-30AD sf; 2. Chromatographic column: Chiralpak IC -3 50×4.6mm I.D., 3µm; 3. Mobile phase system: A for CO2 and B for MeOH (0.05%DEA); 4. Gradient: B 5%-40%; 5. Flow rate: 3mL/min.).

**Compound 11** isomer 1: ¹H NMR (400 MHz, DMSO-*d6*) δ 7.48-7.44 (m, 2H), 7.38-7.37 (m, 1H), 7.28 (d, 1H), 5.48- 5.36 (m, 1H), 4.18 (s, 1H), 3.84-3.83 (m, 1H), 3.73 (s, 1H), 3.55 (s, 2H), 3.38 (s, 3H), 2.67-2.66 (m, 1H), 2.59 (s, 1H), 2.33 (s, 1H), 2.17 (s, 3H), 2.15-2.07 (m, 2H), 1.89-1.88 (m, 2H), 1.70-1.69 (m, 1H), 1.57 (s, 2H), 1.44 (d, 3H), 1.38 (d, 2H), 1.30 (s, 3H).

LCMS m/z =556.2 [M+H]⁺.

**Compound 11** isomer 2: ¹H NMR (400 MHz, DMSO-d6) δ 7.49-7.42 (m, 1H), 7.38 (d, 1H), 7.27 (d, 1H), 5.48-5.36 (m, 1H), 4.22 (s, 1H), 3.82-3.81 (m, 1H), 3.74 (s, 1H), 3.53 (s, 2H), 3.38 (s, 3H), 2.65 (d, 1H), 2.55 (d, 1H), 2.31 (d, 1H), 2.18 (s, 3H), 2.15-2.07 (m, 2H), 1.87 (d, 2H), 1.68 (s, 1H), 1.57 (s, 1H), 1.44 (d, 3H), 1.36 (d, 2H), 1.29 (s, 3H).

LCMS m/z =556.2 [M+H]⁺.

### Example 12:

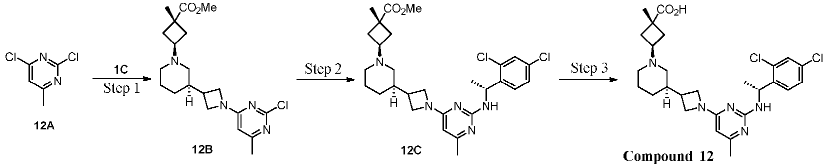

Step 1: Compound **1C** (636 mg, 1.87 mmol) and 2,4-dichloro-6-methylpyrimidine (457 mg, 2.81 mmol) were added to acetonitrile (10 mL) in sequence at room temperature. After mixing evenly, triethylamine (760 mg, 7.52 mmol) was added dropwise and stirred at room temperature overnight. TLC/LCMS showed the reaction was completed, and EA (50 mL) was added for dilution. The organic phase was washed with water (50 mL ×3), dried over anhydrous sodium sulfate, filtered and concentrated. The crude product was purified by silica gel column chromatography (5% MeOH in CH₂Cl₂) to obtain compound **12B** (225 mg, yield: 31%).

LC-MS (ESI): m/z = 393.2 [M+H]⁺.

Step 2: At room temperature, compound **12B** (205 mg, 0.52 mmol) was dissolved in ethylene glycol dimethyl ether (5 mL) and tert-butyl alcohol (5 mL), and (*R*)-1-(2,4-dichlorophenyl)ethylamine (119 mg, 0.62 mmol), potassium tert-butoxide (88 mg, 0.78 mmol), BINAP (130 mg, 0.21 mmol), and Pd₂(dba)₃ (95 mg, 0.1 mmol) were added in sequence. The mixture was stirred evenly, nitrogen was introduced, and the reaction was performed at 100°C for 1 h after the reaction tube was sealed. The mixture was cooled to room temperature, and dilute hydrochloric acid was added dropwise to adjust the pH to 5. The mixture was concentrated under reduced pressure to obtain a crude product of **12C** which was directly used in the next reaction without further purification.

Step 3: Methanol (5 mL), water (5 mL) and lithium hydroxide (35 mg, 1.47 mmol) were added to compound **12C** in sequence at room temperature, and the mixture was stirred at room temperature for 1 h. TLC/LCMS showed that the reaction was completed. The pH was adjusted to 5 with dilute hydrochloric acid and the mixture was concentrated under reduced pressure. The obtained crude product was purified by preparative high performance liquid phase chromatography to obtain **compound 12** (22 mg, 0.041 mmol).

Preparative HPLC separation methods: instrument: waters 2767 preparative liquid chromatographic instrument; chromatographic column: XSelect@ Prep C18 (19mm×250mm), preparative chromatographic conditions: the sample was dissolved in DMF and filteration was performed with a 0.45 µm filter head to prepare a sample liquid, the preparative chromatography conditions were: a. composition of mobile phases A and B: mobile phase A: acetonitrile, mobile phase B: water (containing 0.05% aqueous ammonia) b. gradient elution, mobile phase A: 5%-40%, flow rate: 15 mL/min, d. elution time: 18 min.

¹H NMR (400 MHz, DMSO-*d*₆) δ 7.49 (m, 2H), 7.36 (m, 2H), 5.43 (m, 1H), 5.29 (m, 1H), 3.85 (m, 2H), 3.54 (m, 3H), 2.58-2.60 (m, 4H), 2.43 (m, 2H), 2.02 (s, 3H), 1.61-1.69 (m, 5H), 1.33 (m, 2H), 1.26 (m, 3H), 1.24 (s, 3H), 1.51 (d, 3H), 1.46 (m, 3H), 1.26 (s, 3H), 1.23 (m, 2H), 0.84 (m, 1H).

LC-MS (ESI): m/z = 532.2 [M+H]⁺.

### Example 13:

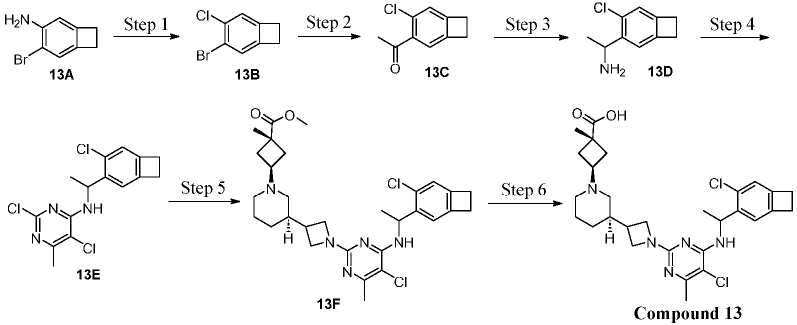

Step 1: Tert-butyl nitrite (2.31 g, 22.42 mmol) was added dropwise to a solution of copper chloride (2.51 g, 18.68 mmol) in MeCN (40 mL). After stirring for ten mins, 4-bromobicyclo[4.2.0]octa-1,3,5-trien-3-amine **13A** (3.7 g, 18.68 mmol) (prepared with reference WO 2019183145A1) was added to the system in portions. Stirring was continued for one hour. The reaction was quenched by dropwise addition of 1 N HCl solution (20 mL). After stirring for ten min, the mixture was extracted with ethyl acetate (40 mL). The organic phase was washed with saturated brine (30 mL), dried over anhydrous sodium sulfate, and filtered, and the filtrate was concentrated under reduced pressure and seperated by silica gel column chromatography (PE) to obtain compound **13B** of interest (2.5 g, yield: 61.54%).

¹H NMR (400 MHz, CDCl₃) δ 7.29 (s, 1H), 7.15 (s, 1H), 3.18-3.10 (m, 4H).

Step 2: Compound **13B** (2.5 g, 11.49 mmol), tributyl(1-ethoxyethylene)tin (4.98 g, 13.79 mmol), Pd(PPh₃)₂Cl₂(0.81 g, 1.15 mmol) and potassium carbonate (2.38 g, 17.23 mmol) were added to anhydrous 1,4-dioxane (40 mL) in sequence. The reaction was carried out at 85°C for 3 h under nitrogen protection. After cooling to room temperature, aqueous potassium fluoride solution (10%wt, 30 mL) was added to the system and stirring was continued for 30 min. Filtration was performed and the filter cake was washed twice with ethyl acetate. The filtrate was concentrated under reduced pressure, and hydrochloric acid (2N, 20 mL) and tetrahydrofuran (20 mL) were added and stirred for about 20 min. The mixture was extracted with dichloromethane (30 mL). The organic phase was washed with saturated brine, dried over anhydrous sodium sulfate, filtered, and the filtrate was concentrated under reduced pressure. The residue was separated and purified by silica gel column chromatography (PE/EA (v/v) = 15:1) to obtain the compound **13C** of interest (0.82 g, yield: 39%).

¹H NMR (400 MHz, CDCl₃) δ 7.16 (s, 1H), 7.09 (s, 1H), 3.22-3.15 (m, 4H), 2.61 (s, 3H).

Step 3: Compound **13C** (0.82 g, 4.54 mmol), ammonium acetate (4.20 g, 54.5 mmol) and sodium cyanoborohydride (1.71 g, 27.2 mmol) were added to methanol (30 mL) in sequence, the temperature was raised to 60°C and the reaction was performed for 16 h. The reaction was completed as detected by TLC. The mixture was cooled to room temperature, concentrated under reduced pressure, and water was added (30 mL). The mixture was extracted with dichloromethane (30 mL×5). The combined organic phase was dried over anhydrous sodium sulfate, filtered, concentrated, and separated by silica gel column chromatography (DCM:MeOH (v/v) = 10:1) to obtain the compound **13D** of interest (0.62 g, yield: 75%).

LC-MS (ESI): m/z =182.1 [M+H]⁺.

Step 4: Using 2,4,5-trichloro-6-methylpyrimidine (0.62 g, 3.12 mmol) and compound **13D** (0.57 g, 3.12 mmol) as the raw materials, the operation method of step 1 of example 1 was referenced to obtain compound **13E** (0.68 g, yield: 63.23%)

LC-MS (ESI): m/z =342.0 [M+H]⁺.

Step 5: Using **1C** (0.68 g, 2.55 mmol) and compound **13E** (0.88 g, 2.55 mmol) as the raw materials, the operation method of step 2 of example 1 was referenced to obtain the compound **13F** of interest (0.57 g, yield: 50%).

LC-MS (ESI): m/z =572.2 [M+H]⁺.

Step 6: Using compound **13F** (0.57 g, 1 mmol) as the raw material, the operation method of step 3 of example 1 was referenced to obtain the compound **13** of interest (0.49 g, yield: 86%).

¹H NMR (400 MHz, CD₃OD) δ 7.07 (d, 1H), 7.01 (s, 1H), 5.61-5.51 (m, 1H), 4.04-3.97 (m, 1H), 3.94-3.88 (m, 1H), 3.74-3.66 (m, 1H), 3.61-3.50 (m, 1H), 3.46-3.33 (m, 1H), 3.15-3.07 (m, 4H), 2.78 -2.66 (m, 2H), 2.52-2.40 (m, 1H), 2.40-2.30 (m, 1H), 2.26 (s, 3H), 2.23-2.12 (m, 1H), 2.02-1.87 (m, 5H), 1.86-1.75 (m, 2H), 1.74-1.59 (m, 1H), 1.46 (d, 3H), 1.37 (d, 3H), 1.12-0.98 (m, 1H).

LC-MS (ESI): m/z =558.2 [M+H]⁺.

### Example 14:

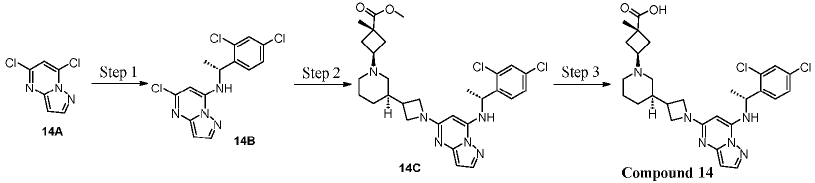

Step 1: 5,7-dichloropyrazolo[1,5-A]pyrimidine **14A** (0.72 g, 3.83 mmol) and compound (*R*)-2,4-dichloro-ALPHA-methyl-benzylamine (0.73 g, 3.83 mmol) were dissolved in isopropyl alcohol (20 mL), and then triethylamine (0.77 g, 7.66 mmol) was added dropwise, and after the reaction was performed at room temperature overnight, the obtained system was concentrated under reduced pressure, and the residue was purified by silica gel column chromatography (PE/EA (v/v) = 4:1) to obtain compound **14B** (1.12 g, yield: 86%).

LC-MS (ESI): m/z =341.0 [M+H]⁺.

Step 2: Compound **14B** (0.34 g, 1.00 mmol) and compound **1C** (0.27 g, 1.01 mmol) were dissolved in DMF(20 mL), and then DIPEA (0.26 g, 2.00 mmol) was added dropwise, the reaction was performed at 90°C for 40 h and then concentration was performed under reduced pressure, and the residue was purified by silica gel column chromatography (DCM/MeOH (v/v) = 12:1) to obtain compound **14C** (32 mg, yield: 5.6%).

LC-MS (ESI): m/z =571.2 [M+H]⁺.

Step 3: Compound **14C** (32 mg, 0.056 mmol) was dissolved in tetrahydrofuran (5 mL), methanol (2 mL) and water (2 mL) were added, and lithium hydroxide monohydrate (42 mg, 1 mmol) was added after stirring evenly. The mixture was reacted at room temperature for 3 h, and the pH was adjusted to 5 with hydrochloric acid (1N aqueous solution). The system was concentrated under reduced pressure and directly separated by silica gel column chromatography (DCM:MeOH (v/v) =10:1) to obtain a crude product of the compound **14** of interest. The compound was further subjected to preparative high performance liquid phase chromatography (preparation conditions 1. instruments: waters 2767 preparative liquid chromatographic instrument; chromatographic column: SunFire @ Prep C18 (19 mm x 250 mm). 2. The sample was dissolved in DMF and filtered with a 0.45 µm filter head to prepare a sample liquid. 3. Preparative chromatography conditions: a. composition of mobile phases A and B: mobile phase A: acetonitrile, mobile phase B: water (containing 1% TFA), b. gradient elution, mobile phase A: 10% to 80% c. flow rate: 15 ml/min. d. elution time: 18 min.) The trifluoroacetate salt of **compound 14** (19 mg, yield: 43%) was obtained.

¹H NMR (400 MHz, Methanol-*d*₄) δ 7.96 (d, 1H), 7.55 (d, 1H), 7.53 (d, 1H), 7.37 (dd, 1H), 6.17 (d, 1H), 5.25 (q, 1H), 4.77 (s, 1H), 4.31 (t, 1H), 4.25-4.13 (m, 1H), 4.10-4.03 (m, 1H), 4.02- 3.93 (m, 1H), 3.80-3.68 (m, 1H), 3.54-3.45 (m, 1H), 3.42-3.33 (m, 2H), 2.88-2.78 (m, 2H), 2.75-2.63 (m, 2H), 2.48 (t, 1H), 2.30-2.18 (m, 2H), 2.16-1.99 (m, 1H), 1.98-1.88 (m, 1H), 1.85-1.73 (m, 1H), 1.70 (d, 3H), 1.42 (s, 3H), 1.17 (q, 1H).

LC-MS (ESI): m/z =557.60 [M+H]⁺.

### Example 15:

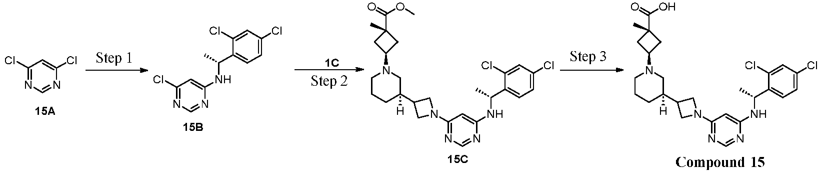

Using compound **15A** (1.5 g, 10.13 mmol) and (*R*)-1-(2,4-dichlorophenyl)ethylamine (1.91 g, 10.13 mmol) as the raw materials, the operation method of steps 1 to 3 of example 14 was referenced to obtain **compound 15** (15 mg).

¹H NMR (400 MHz, CD₃CN; trifluoroacetic acid salt) δ 8.06 (s, 1H), 7.54-7.53 (m, 1H), 7.47-7.45 (m, 1H), 7.39-7.37 (m, 1H), 5.03-5.00 (m, 1H), 4.89 (s, 1H), 4.21-4.14 (m, 1H), 3.90-3.87 (m, 1H), 3.61-3.57 (m, 1H), 3.43-3.32 (m, 3H), 2.74-2.69 (m, 4H), 2.52-2.46 (m, 4H), 2.21-2.15 (m, 3H), 1.85-1.79 (m, 3H), 1.56-1.54 (d, 3H), 1.37(s, 3H), 1.11-1.05 (m, 1H).

LC-MS (ESI): m/z = 518.2 [M+H]⁺.

### Example 16:

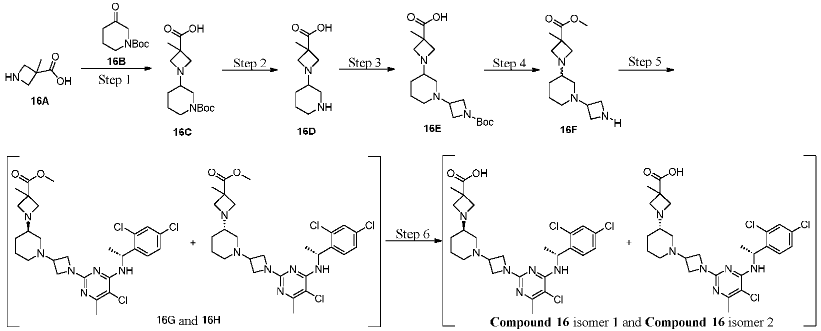

Step 1: 3-methylazetidine-3-carboxylic acid (2.00 g, 17.4 mmol) was dissolved in dry 1,2-dichloroethane (50 mL), and N-tert-butoxycarbonyl-3-piperidone (4.15 g, 20.8 mmol) and acetic acid (1.04 g, 17.4 mmol) were added in sequence, the obtained system was stirred at room temperature for 5 h, then sodium triacetoxyborohydride (7.37 g, 34.7 mmol) was added, and the obtained system was reacted under stirring at room temperature overnight. After the reaction was completed as monitored by TLC, water (2 mL) was added to quench the reaction, and the crude product obtained by concentration was separated and purified by silica gel column chromatography (dichloromethane:methanol (v/v) = 4:1) to obtain **16C** (4.51 g, 86.7%).

LC-MS (ESI): m/z =299.2 [M+H]⁺.

Step 2: Compound **16C** (4.51 g, 15.1 mmol) was dissolved in dichloromethane (90 mL), and trifluoroacetic acid (30 mL) was added at room temperature. The reaction was continued for 1 h after the addition was completed. After the reaction was completed as monitored by TLC, the mixture was concentrated to obtain the trifluoroacetate salt of **16D,** and the crude product was directly used in the next reaction without further purification.

LC-MS (ESI): m/z =199.3 [M+H]⁺;

Step 3: methanol (70 mL) was added to the crude trifluoroacetate salt of compound **16D** obtained in the previous step, and dissolved under stirring. N-tert-butyloxycarbonyl-3-azetidinone (8.29 g, 48.4 mmol) and acetic acid (1.45 g, 24.2 mmol) were added in sequence. After stirring at room temperature for 10 h, sodium cyanoborohydride (2.95 g, 46.9 mmol) was added and stirring was continued at room temperature for 6 h. After the reaction was completed as monitored by TLC, water (2 mL) was added to quench the reaction and the obtained system was concentrated. The crude product was separated and purified by silica gel column chromatography (dichloromethane:methanol (v/v) = 4:1) to obtain **16E** (3.69 g, two-step yield: 69.1%).

LC-MS (ESI): m/z =354.2 [M+H]⁺;

Step 4: Compound **16E** (1.50 g, 4.25 mmol) was dissolved in methanol (40 mL), and thionyl chloride (2.53 g, 21.25 mmol) was added dropwise at room temperature. After the addition was completed, the reaction was continued for 3 h. After the reaction was completed as monitored by TLC, water (1 mL) was added to quench the reaction. After concentration, the resulting crude product was redissolved in methanol (10 mL), ethyl acetate (50 mL) was added to precipitate a solid, which was filtered and washed with ethyl acetate. The filter cake was dried to obtain the hydrochloride salt of compound **16F** (1.28 g, 80.1%).

LC-MS (ESI): m/z =268.2 [M+H]⁺;

Step 5: **2F** (500 mg, 1.43 mmol), hydrochloride of compound **16F** (697 mg, 1.85 mmol), triethylamine (872 mg, 8.55 mmol) and cesium fluoride (433 mg, 2.85 mmol) were added to dimethyl sulfoxide (20 mL) in sequence and stirred and the obtained system was heated to 100°C and reacted for about 4 h. After the reaction was completed, the mixture was cooled to room temperature, and water (50 mL) and saturated aqueous sodium chloride solution (10 mL) were added. The mixture was extracted with ethyl acetate (30 mL×8). The combined organic phase was washed with saturated aqueous sodium chloride solution (50 mL) and water (50 mL) in sequence, dried over anhydrous sodium sulfate, filtered, and concentrated. The crude product was separated and purified by silica gel column chromatography (dichloromethane:methanol (v/v) = 9:1 to 5:1), and further separated and purified by chiral HPLC to obtain two isomers **16G** ( 159 mg, 19.2%) and **16H** (150 mg, 18.1%).

HPLC analytical method: 1. Instruments: SHIMADZU LC-30AD SFC; 2. Chromatographic column: Chiralcel OX-3 50×4.6mm I.D., 3µm; 3. Mobile phase system: A for CO₂, B for 0.05%DEA in MeOH; 4. Gradient: B 5%-40%; 5. Flow rate: 3 mL/min. isomer **16G,** retention time 1.16 min; **16H,** retention time 1.76 min.

Chiral preparative HPLC separation and purification method: instrument: Waters 150 MGM; chromatographic column: Chiralcel OJ Column (250 ×30mm, I.D 30mm, 10um particle size); mobile phase: A: carbon dioxide, and B: methanol (0.1% aqueous ammonia); isocratic elution: 35% mobile phase B; flow rate: 100 mL/min; back pressure: 100 bar; column temperature: 25°C; wavelength: 220 nm; elution time: 2.9 min.

LC-MS (ESI): m/z =581.2 [M+H]⁺ ;

Step 6: Compound **16G** (150 mg, 0.258 mmol) was dissolved in a mixed solvent of tetrahydrofuran (4 mL), methanol (2 mL) and water (2 mL), lithium hydroxide monohydrate (54.1 mg, 1.29 mmol) was added at room temperature, and then the obtained system was reacted under stirring overnight. After the disappearance of the raw materials as monitored by TLC, the mixture was concentrated and the residue was separated and purified by preparative HPLC to obtain **compound 16** isomer 1 (112 mg, 76.5%).

Preparative HPLC separation and purification method: 1. Instruments: waters 2767 preparative liquid chromatographic instrument; chromatographic column: SunFire @ Prep C18 (19 mm x 250 mm). 2. The sample was filtered with a 0.45 µm filter head to prepare a sample liquid. 3. Preparative chromatography conditions: a. composition of mobile phases A and B: mobile phase A: acetonitrile; mobile phase B: water (containing 0.5% ammonium acetate); b. gradient elution, mobile phase A: 5%-50%; c. flow rate: 12 mL/min; d. elution time: 10 min,

¹H NMR (400 MHz, CD₃OD): *δ* 7.43 (d, 1H), 7.37 (d, 1H), 7.25-7.24 (m, 1H), 5.49-5.48 (m, 1H), 4.32-4.31 (m, 2H), 4.04-3.97 (m, 1H), 3.87-3.86 (m, 3H), 3.79-3.78 (m, 1H), 3.50 (s, 1H), 3.37 -3.29 (m, 1H), 3.26-3.16 (m, 1H), 2.56 (d, 1H), 2.42-2.25 (m,3H), 2.27 (s, 3H), 1.91-1.81 (m, 1H), 1.74 (d, 1H), 1.68-1.55 (m, 2H), 1.55-1.46 (m, 6H).

LC-MS (ESI): m/z =567.2 [M+H]⁺ ;

Using **16H** as the raw material, **compound 16** isomer 2 was obtained by referring to the above synthesis method.

¹H NMR (400 MHz, CD₃OD): *δ* 7.42 (d, 1H), 7.37 (d, 1H), 7.25-7.24 (m, 1H), 5.48-5.47 (m, 1H), 4.31-4.30 (m, 2H), 3.98-3.97 (m, 1H), 3.94-3.81 (m, 3H), 3.69-3.68 (m, 1H), 3.61-3.52 (m, 1H), 3.39-3.32 (m, 1H), 3.24-3.13 (m, 1H), 2.52-2.38 (m, 3H), 2.27 (s, 4H), 1.86-1.74 (m, 1H), 1.73-1.56 (m, 3H), 1.56-1.46 (m, 6H).

LC-MS (ESI): m/z =567.2 [M+H]⁺.

### Example 17:

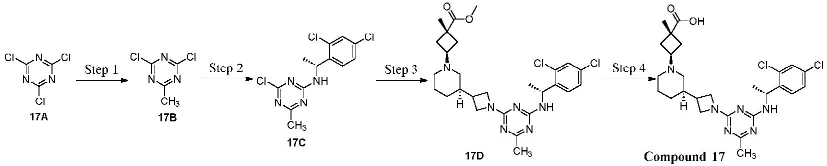

Step 1: **17A**(3.7 g, 19.99 mmol) was dissolved in DCM (20 mL), 1M methylmagnesium bromide (8.3 g, 69.10 mmol) was added dropwise at -20°C, after the dropwise addition was completed, the obtained system was naturally warmed to room temperature and stirred for 3h. After the reaction was completed, saturated ammonium chloride solution (20 mL) was added to quench the reaction, and ethyl acetate (50 mL ×3) was used for extraction. The combined organic phases were washed with saturated saline solution, dried over anhydrous sodium sulfate, and filtered, the filtrate was concentrated and the residue was separated by silica gel column chromatography (petroleum ether:ethyl acetate (v/v) = 50:1) to obtain **17B** (2.7 g, 82%).

Steps 2 to 4: Using **17B** (2.0 g, 12.18 mmol) as the raw material, the operation method of steps 1 to 3 of example 14 was referenced to obtain the title **compound 17** (67.0 mg).

¹H NMR (400 MHz, CDCl₃) δ 7.34 (d, 1H), 7.31 (d, 1H), 7.18 (d, 1H), 5.40 (s, 1H), 4.67 (s, 2H), 4.06 (s, 2H), 3.81 (s, 2H), 3.46 (s, 1H), 2.88 (s, 2H), 2.64 (s, 2H), 2.38 (s, 1H), 2.22 (s, 3H), 2.08 (s, 1H), 1.92 (s, 2H), 1.70 (s, 4H), 1.46 (d, 3H), 1.41 (s, 3H), 0.86 (s, 1H).

LCMS m/z =533.2 [M+H]⁺.

### Example 18:

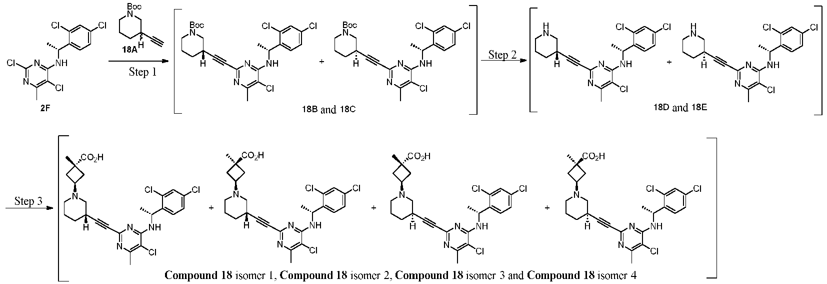

Step 1: Under nitrogen protection, compound **2F** (1.70 g, 4.84 mmol), compound **18A** (3.02 g, 14.5 mmol), bis(triphenylphosphine)palladium(II) chloride (340 mg, 0.484 mmol), copper iodide (462 mg, 2.42 mmol), and triphenylphosphine (254 mg, 0.969 mmol) were all added to a round bottom flask, and dry *N*,*N*-dimethylformamide (25 mL) and triethylamine (10 mL) were added, the temperature was raised to 100°C and the reaction was performed overnight. The reaction of the raw materials was completed as monitored by TLC. The mixture was cooled to room temperature and concentrated under reduced pressure. The residue was separated and purified by silica gel column chromatography (petroleum ether:ethyl acetate (v/v) = 4:1) to obtain a mixture of **18B** and **18C** (2.36 g, 92.9%).

LC-MS (ESI): m/z =523.2 [M+H]⁺.

The above mixture was separated by chiral HPLC to obtain compound **18B** (1.01 g, 39.8%) and compound **18C** (551 mg, 21.7%). Chiral preparation method: instrument: Waters 150 MGM; chromatographic column: Chiralpak Column; mobile phase: A: carbon dioxide, and B: methanol (0.1% aqueous ammonia); isocratic elution: 35% mobile phase B; flow rate: 100 mL/min; back pressure: 100 bar; column temperature: 25°C; wavelength: 220 nm.

HPLC analytical method: 1. Instruments: SHIMADZU LC-30AD SFC; 2. Chromatographic column: Chiralcel OX-3 50×4.6mm I.D., 3µm; 3. Mobile phase system: A for CO₂, B for 0.05%DEA in MeOH; 4. Gradient: B 5%-40%; 5. Flow rate: 3 mL/min. Compounds **18B** , retention time 1.09 min; Compounds **18C**, retention time 1.18 min.

Step 2: Compound **18B** (300 mg, 0.573 mmol) was dissolved in dichloromethane (9 mL), and trifluoroacetic acid (3 mL) was added at room temperature. The reaction was continued for 1 h after the addition was completed. After the reaction was completed as monitored by TLC, the mixture was concentrated to obtain the trifluoroacetate salt of **18D**, and the crude product was directly used in the next reaction without further purification.

LC-MS (ESI): m/z = 423.1 [M+H]⁺;

Using **18C** as the raw material, compound **18E** was obtained by referring to the above synthesis method.

Step 3: The crude trifluoroacetate salt of compound **18D** obtained in the previous step was dissolved in 1,2-dichloroethane (30 mL), and compound **2B** (157 mg, 1.23 mmol) and acetic acid (36.8 mg, 0.613 mmol) were added in sequence, the obtained system was stirred at 60°C for 1 h, then sodium triacetoxyborohydride (390 mg, 1.84 mmol) was added, and the obtained system was reacted under stirring at 60°C for 5 h. After the reaction was completed as monitored by TLC, water (1 mL) was added to quench the reaction, and the mixture was directly concentrated under reduced pressure. The residue was separated and purified by preparative HPLC, and the pH was adjusted to 7-8 with aqueous sodium bicarbonate solution to obtain **compound 18** isomer 1 (64 mg, two-step yield: 20.9%) and **compound 18** isomer 2 (78 mg, two-step yield: 25.4%).

HPLC analysis conditions: 1. Instruments: Shimadzu LC-20AT; 2. Chromatographic column: Xtimate C18 4.6*50mm, 3µm; 3. Mobile phase system: A for 0.05% TFA in H2O; B for ACN; 4. Gradient: B 5-95%; 5. Flow rate: 1.0 mL/min, run time: 10 min. retention time: **compound 18** isomer 1: tR = retention time: 4.114 min; **Compound 18** isomer 2: tR retention time: =4.162 min.

Preparative HPLC separation and purification method: 1. Instruments: waters 2767 preparative liquid chromatographic instrument; chromatographic column: SunFire @ Prep C18 (19 mm x 250 mm). 2. The sample was filtered with a 0.45 µm filter head to prepare a sample liquid. 3. Preparative chromatography conditions: a. composition of mobile phases A and B: mobile phase A: acetonitrile; mobile phase B: water (containing 0.1% trifluoroacetic acid); b. gradient elution, mobile phase A: 5%-50%; c. flow rate: 12 mL/min; d. elution time: 30 min.

**Compound 18** isomer 1: ¹H NMR (400 MHz, Methanol-*d*4) δ 7.46 - 7.38 (m, 2H), 7.27 (dd, 1H), 5.60 (q, 1H), 3.50-3.42 (m, 1H), 3.38 (q, 1H), 3.27 (d, 1H), 3.19-3.07 (m, 1H), 2.71-2.48 (m, 4H), 2.38 (s, 3H), 2.23-2.09 (m, 3H), 1.98-1.82 (m, 2H), 1.70-1.58 (m, 1H), 1.55 (d, 3H), 1.38 (s, 3H).

LC-MS (ESI): m/z =535.2 [M+H]⁺;

**Compound 18** isomer 2: ¹H NMR (400 MHz, Methanol-*d*4) δ 7.45-7.38 (m, 2H), 7.27 (dd, 1H), 5.60 (q, 1H), 3.27-3.11 (m, 2H), 3.02-2.85 (m, 2H), 2.73-2.62 (m, 2H), 2.52-2.32 (m, 5H), 2.08-1.83 (m, 4H), 1.77 - 1.58 (m, 2H), 1.55 (d, 3H), 1.38 (s, 3H).

LC-MS (ESI): m/z =535.2 [M+H]⁺;

Using **18E** as the raw material, **compound 18** isomer 3 (53 mg, two-step yield: 17.3%) and **compound 18** isomer 4 (111 mg, two-step yield: 36.2%) were obtained by referring to the above synthesis method.

HPLC analysis conditions: 1. Instruments: Shimadzu LC-20AT; 2. Chromatographic column: Xtimate C18 4.6*50mm, 3µm; 3. Mobile phase system: A for 0.05% TFA in H2O; B for ACN; 4. Gradient: B 5-95%; 5. Flow rate: 1.0 mL/min, run time: 10 min. retention time: compound 18 isomer 3 retention time: 4.122 min; compound 18 isomer 4 retention time: 4.147 min.

**Compound 18** isomer 3: ¹H NMR (400 MHz, Methanol-*d*4) δ 7.45-7.38 (m, 2H), 7.27 (dd, 1H), 5.60 (q, 1H), 3.46-3.34 (m, 2H), 3.23 (d, 1H), 3.09 (tt, 1H), 2.69-2.48 (m, 4H), 2.39 (s, 3H), 2.25-2.07 (m, 3H), 1.99-1.79 (m, 2H), 1.72-1.58 (m, 1H), 1.55 (d, 3H), 1.38 (s, 3H).

LC-MS (ESI): m/z =535.2 [M+H]⁺;

**Compound 18** isomer 4: ¹H NMR (400 MHz, Methanol-*d*4) δ 7.47-7.37 (m, 2H), 7.26 (dd, 1H), 5.60 (q, 1H), 3.26-3.11 (m, 2H), 3.03-2.81 (m, 2H), 2.78-2.61 (m, 2H), 2.51-2.32 (m, 5H), 2.11-1.81 (m, 4H), 1.76-1.56 (m, 2H), 1.55 (d, 3H), 1.37 (s, 3H).

LC-MS (ESI): m/z =535.2 [M+H]⁺.

### Example 19:

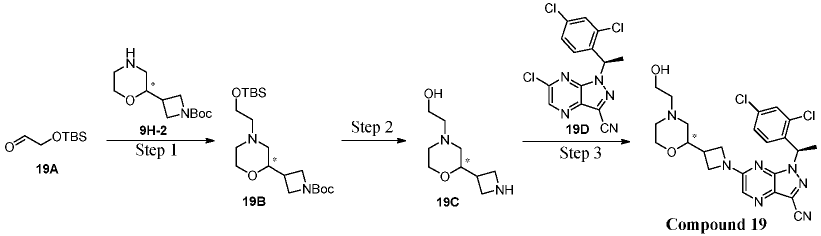

Step 1: Compound **19A** (140 mg, 0.83 mmol) was dissolved in 1,2-dichloroethane (3 mL) under nitrogen atmosphere at room temperature, **9H-2** (carbon* indicates a single configuration R or S) (200 mg, 0.83 mmol) was added, the obtained system was stirred for 5 min, then sodium triacetoxyborohydride (350 mg, 1.66 mmol) was added and the reaction was performed overnight. After the reaction was completed, methanol (2 mL) was added to quench the reaction, and the obtained system was concentrated, and purified by column chromatography (eluent ratio: MeOH/DCM=0%-10%) to obtain compound **19B** (298 mg, 90%).

LC-MS (ESI): m/z =401.6 [M+H]⁺.

Step 2: Compound **19B** (298 mg, 0.74 mmol) was dissolved in dichloromethane (3 mL) at room temperature, hydrogen chloride-1,4-dioxane solution (4M, 2 mL) was added, and the reaction was performed for 1 h. After the reaction was completed, the reaction liquid was concentrated to obtain the hydrochloride salt (200 mg) of compound **19C,** which was directly used in the next reaction without further purification.

LC-MS (ESI): m/z =187.1[M+H]⁺.

Step 3: Compounds **19C** (200 mg, 0.99 mmol) and **19D** (350 mg, 0.99 mmol) (synthesized according to the method described in reference patent WO 2018022992) were dissolved in N,N-dimethylformamide (5 mL), and N,N-diisopropylethylamine (260 mg, 2.01 mmol) was added at room temperature and the obtained system was reacted overnight. The reaction was completed as monitored by LCMS, and the crude product obtained by concentration under reduced pressure was separated by preparative HPLC to obtain **compound 19** (150 mg, 31%). Separation method: 1. Instruments: waters 2767 preparative liquid chromatographic instrument; chromatographic column: SunFire@ Prep C18 (19 mm × 250 mm). 2. The sample was filtered with a 0.45 µm filter head to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min.

¹H NMR (400 MHz, Chloroform-*d*) δ 7.83 (s, 1H), 7.40-7.35 (m, 2H), 7.20-7.19 (m, 1H), 6.47-6.46 (m, 1H), 4.25-4.16 (m, 3H), 4.10-4.09 (m, 1H), 3.97-3.92 (m, 1H), 3.79-3.66 (m, 4H), 2.91-2.81 (m, 3H), 2.64-2.63 (m, 2H), 2.33-2.31 (m, 1H), 2.01-2.00 (m, 1H), 1.91-1.90 (m, 3H).

LC-MS (ESI): m/z =502.5[M+H]⁺.

### Example 20:

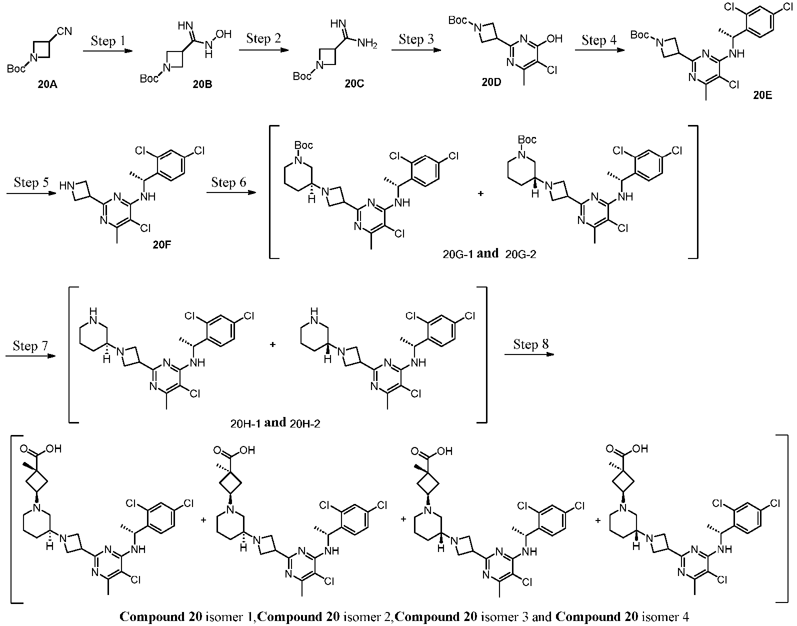

Step 1: Compound **20A** (10.0 g, 54.9 mmol) was dissolved in ethanol (25 mL), hydroxylamine hydrochloride (4.58 g, 65.9 mmol)and triethylamine (6.66 g, 65.9 mmol) were added, the temperature was raised to reflux and the reaction was performed for 24 h. After the reaction was completed, the mixture was cooled to room temperature and concentrated under reduced pressure. The residue was dissolved in dichloromethane (30 mL). The organic phase was washed with water (20 mL×3) and brine (20 mL×3) in sequence, dried over anhydrous sodium sulfate, filtered and concentrated to obtain the crude title compound **20B** (9.00 g, 75%). The compound was directly used for the next reaction without further purification.

LC-MS (ESI): m/z = 216.2 [M+H]⁺.

Step 2: Compound **20B** (9.00 g, 41.9 mmol) was dissolved in methanol (100 mL), raney nickel (4.47 g, 41.9 mmol) was added under an ice bath, and the obtained system was stirred for 3 h under hydrogen atmosphere. TLC and LC-MS showed that the reaction was completed and the reaction was stopped. The reaction liquid was filtered through celite and concentrated to obtain the title compound **20C** (7.11 g, 85%) as an oily liquid. The obtained compound could be directly used in the next reaction without further purification.

LC-MS (ESI): m/z =200.2 [M+H]⁺.

Step 3: Compound **20C** (5.0 g, 25.1 mmol) and ethyl 2-chloroacetoacetate (4.1 g, 25.1 mmol) were dissolved in anhydrous methanol (10 mL), and potassium tert-butoxide was added slowly under an ice bath, the obtained system was reacted under stirring for 1 h, and then warmed to reflux and reacted overnight. After the reaction was completed, the mixture was cooled to room temperature and concentrated. Water (30 mL) was added to the residue, and the pH was adjusted to 6-7 with aqueous citric acid solution. The mixture was extracted with dichloromethane (20 mL×3). The combined organic phases were dried over anhydrous sodium sulfate, filtered, and concentrated. The residue was separated and purified by silica gel column chromatography (dichloromethane:methanol (v/v) = 10:1-5:1) to obtain the title compound **20D** (3.8 g, 51%).

LC-MS (ESI): m/z =300.3 [M+H]⁺.

Step 4: Compound **20D** (3.8 g, 12.7 mmol) and (*R*)-1-(2,4-dichlorophenyl)ethan-1-amine *(2.9* g, 15.2 mmol) were dissolved in N,N-dimethylformamide (18 mL), and then BOP (9.0 g, 20.3 mmol) and DBU (5.8 g, 38.1 mmol) were added in sequence. The mixture was stirred at room temperature for 3 h under a nitrogen atmosphere. After the reaction was completed, water (15 mL) was slowly added to quench the reaction, and the mixture was extracted with ethyl acetate (20 mL×3). The combined organic phase was dried over anhydrous sodium sulfate, filtered, and concentrated. The residue was separated and purified by silica gel column chromatography (petroleum ether:ethyl acetate (v/v) = 3:1-2:1) to obtain the title compound **20E** (4.2 g, 70.2%).

LC-MS (ESI): m/z =471.2 [M+H]⁺.

Step 5: Compound **20E** (4.2 g, 8.9 mmol) was dissolved in dichloromethane (8 mL), and hydrogen chloride-1,4-dioxane solution (8 mL) was added and the obtained system was reacted at room temperature for 1 h. Concentration gave the hydrochloride salt of the title compound **20F** (3.0 g, 92%). The obtained compound could be directly used in the next reaction without further purification.

LC-MS (ESI): m/z =371.2 [M+H]⁺.

Step 6: Compound **20F** (3.0 g, 8.1 mmol) was dissolved in 1,2-dichloroethane (10 mL), N-tert-butoxycarbonyl-3-piperidone(2.4 g, 12.1 mmol) and a few drops of acetic acid were added, after the addition was completed, the obtained system was reacted under stirring for 3 h. Sodium cyanoborohydride (1.52 g, 24.2 mmol) was added and stirring was continued for 18 h. The reaction was poured into water (30 mL), aqueous sodium bicarbonate solution (5 mL) was added, and extraction was performed with ethyl acetate (20 mL×3), the combined organic phases were dried over anhydrous sodium sulfate, and concentrated, and the residue was separated and purified by silica gel column chromatography (dichloromethane:methanol (v/v) = 90:10) to obtain the compound **20G** (racemate) (3.0 g, 67%). Compound **20G** was separated by chiral preparative HPLC to obtain two isomers, compound **20G**-1 (0.35 g, retention time 1.52 min, 23.3%) and compound **20G**-2 (0.42 g, retention time 1.60 min, 28.0%).

HPLC analytical method: 1. Instruments: SHIMADZU LC-30AD sf; 2. Chromatographic column: Chiralcel OX-3 50×4.6mm I.D., 3µm; 3. Mobile phase system: A for CO2 and B for EtOH (0.05%DEA); 4. Gradient: B 5%-40%; 5. Flow rate: 3mL/min.

Preparative chromatography separation conditions: 1. Instruments: Waters 150 SFC; 2. Chromatographic column: Chiralpak IC -Column (250×30 mm, I.D 30 mm, 10 um particle size); 3. Mobile phase system: A for CO₂ and B for MeOH (0.1%NH₃•H₂O); 4. Gradient: B 45%; 5. Flow rate: 180 mL/min.

LC-MS (ESI): m/z =554.2 [M+H]⁺.

Step 7: Compound **20G**-1(0.21 g, 0.38 mmol) was dissolved in dichloromethane (6 mL), hydrogen chloride-1,4-dioxane solution (5 mL) was added, and after the addition was completed, the reaction was performed at room temperature for 1 h. Concentration gave the hydrochloride salt of the title compound **20H-1** (0.16 g, 95%). The obtained compound could be directly used in the next reaction without further purification.

LC-MS (ESI): m/z =454.2 [M+H]⁺.

Referring to the above operation, compound **20G**-2 (0.25 g, 0.45 mmol) was used as the raw material to obtain compound **20H**-2 (0.197 g, 96.0%).

LC-MS (ESI): m/z =454.2 [M+H]⁺.

Step 8: Compound **20H-1** (0.11 g, 0.23 mmol) was dissolved in methanol (10 mL), and 3-carbonyl-1-methyl-cyclobutanecarboxylic acid (0.10 g, 0.28 mmol) and sodium triacetoxyborohydride (58 mg, 0.9 mmol) were added, glacial acetic acid (0.11 g, 0.23 mmol) was added under an ice bath and a nitrogen atmosphere, the obtained system was stirred for 5 min, warmed to room temperature and stirred for 18 h. Concentration gave the crude product, which was purified by preparative high performance liquid phase chromatography to obtain **compound 20,** isomer 1 (0.15 g, retention time 3.32 min, yield: 28.56%) and **compound 20,** isomer 2 (0.13 g, retention time 3.30 min, yield: 24.75%).

HPLC analytical method: 1. Instrument: Shimadzu LC-20AT; 2. Chromatographic column: Xtimate C18 4.6*50mm, 3µm; 3. Mobile phase A: 0.05% TFA solution, mobile phase B: acetonitrile; 4. Gradient: A 95-5% B 5-95%; 5. Flow rate: 1 mL/min, column temperature: 35°C, wavelength: 210 nm/254 nm, acquisition time: 10 min.

Preparation method: instrument: waters 2767 preparative liquid chromatographic instrument; chromatographic column: SunFire @ Prep C18 (19 mm × 250 mm). The sample was dissolved in methanol and filtered through a 0.22 µm filter head to prepare a sample liquid. Preparative chromatography conditions: composition of mobile phases A and B: mobile phase A: acetonitrile, mobile phase B: water (containing 1% ammonium acetate), gradient elution, mobile phase A: 10%-40%, flow rate: 12 mL/min. Elution time: 25 min.

Compound **20**, isomer 1: LC-MS (ESI): m/z =566.2 [M+H]⁺.

¹H NMR (400 MHz, Methanol-*d*₄) δ 7.39 (d, 1H), 7.27 (d, 1H), 7.17-7.14 (m, 1H), 5.47-5.42 (m, 1H), 3.77 (d, 1H), 3.67-3.54 (m, 2H), 3.47-3.42 (m, 1H), 3.23 (d, 1H), 3.06-3.03 (m, 1H), 2.73-2.46 (m, 5H), 2.31 (s, 3H), 1.93-1.52 (m, 7H), 1.48 (d, 3H), 1.27-1.13 (m, 4H).

Compound **20**, isomer 2: LC-MS (ESI): m/z =566.2 [M+H]⁺.

¹H NMR (400 MHz, Methanol-*d*₄) δ 7.43-7.36 (m, 1H), 7.26 (d, 1H), 7.16-7.13 (m, 1H), 5.46-5.43 (m, 1H), 3.76 (s, 1H), 3.58 (d, 2H), 3.45-3.42 (m, 1H), 3.17 (s, 2H), 2.65-2.62 (m, 3H), 2.49-2.37 (m, 2H), 2.31 (s, 3H), 2.16-1.48 (m, 8H), 1.47 (d, 3H), 1.28 (s, 3H).

Referring to the above operation, compound **20H-2** (3.4 g, 9.0 mmol) was used as the raw material to obtain the title compound **20,** isomer 3 (0.15 g, retention time 3.34 min, yield: 28.56%), compound **20,** isomer 4 (0.13 g, retention time 3.31 min, yield: 24.75%).

HPLC analytical method: 1. Instrument: Shimadzu LC-20AT; 2. Chromatographic column: Xtimate C18 4.6*50mm, 3µm; 3. Mobile phase A: 0.05% TFA solution, mobile phase B: acetonitrile; 4. Gradient: A 95-5% B 5-95%; 5. Flow rate: 1 mL/min, column temperature: 35°C, wavelength: 210 nm/254 nm, acquisition time: 10 min.

Compound **20**, isomer 3: LC-MS (ESI): m/z =566.2 [M+H]⁺.

¹H NMR (400 MHz, Methanol-*d*₄) δ 7.39 (d, 1H), 7.27 (d, 1H), 7.17-7.14 (m, 1H), 5.45-5.42 (m, 1H), 3.72-3.68 (m, 2H), 3.60-3.57 (m, 1H), 3.46-3.42 (m, 1H), 3.19-2.88 (m, 2H), 2.67-2.41 (m, 5H), 2.31 (s, 3H), 1.96-1.53 (m, 8H), 1.48 (d, 3H), 1.26 (s, 3H).

Compound **20**, isomer 4: LC-MS (ESI): m/z =566.2 [M+H]⁺.

¹H NMR (400 MHz, Methanol-*d*₄) δ 7.40 (d, 1H), 7.27 (d, 1H), 7.17-7.08 (m, 1H), 5.44-5.41 (m, 1H), 3.71-3.68 (m, 2H), 3.59-3.55 (m, 1H), 3.42-3.38 (m, 1H), 3.15-3.08 (m, 2H), 2.79-2.36 (m, 5H), 2.30 (s, 3H), 2.10-1.57 (m, 8H), 1.47 (d, 3H), 1.27 (s, 3H).

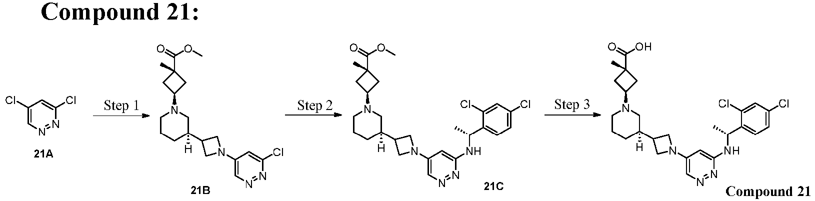

Using compound **21A** (0.5 g, 3.38 mmol) and compound **1C** (1.0 g, 3.38 mmol) as the raw materials, the operation method of steps 1 to 3 of example 14 was referenced to obtain the title **compound 21** (25 mg).

¹H NMR (400 MHz, Methanol-*d*₄) δ 7.70 (d, 1H), 7.45 (d, 1H), 7.36 (d, 1H), 7.28 (m, 1H), 5.42 (s, 1H), 5.01-4.98 (m, 1H), 4.35-3.78 (m, 4H), 3.64 (s, 1H), 3.47- 3.25 (m, 2H), 2.81-2.52 (m, 4H), 2.36 (s, 1H), 2.15 (s, 2H), 2.06-1.61 (m, 5H), 1.51 (d, 3H), 1.32 (s, 3H).

LC-MS (ESI): m/z = 518.2 [M+H]⁺.

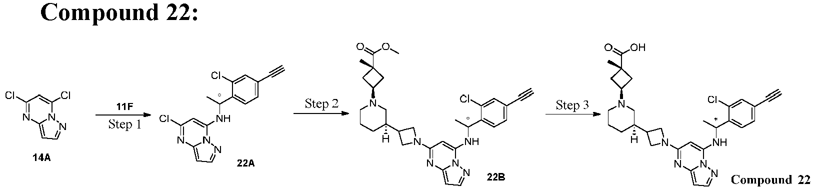

Using **11F** (526.0 mg, 2.94 mmol) and 5,7-dichloropyrazolo[1,5-A]pyrimidine (500.0 mg, 2.67 mmol) as the raw materials, the operation method of steps 1 to 3 of example 14 was referenced to obtain the title **compound 22** (20 mg).

¹H NMR (400 MHz, Methanol-*d*₄) δ 7.72 (d, 1H), 7.45 (d, 1H), 7.39 (d, 1H), 7.29-7.26 (m, 1H), 5.86 (d, 1H), 5.05-4.98 (m, 1H), 4.61 (s, 1H), 3.91-3.88 (m, 2H), 3.74- 3.53 (m, 2H), 3.46-3.32 (m, 1H), 3.29-3.22 (m, 1H), 3.11 (d, 1H), 2.68-2.56 (m, 2H), 2.41-2.38 (m, 2H), 2.16-2.13 (m, 1H), 1.99-1.69 (m, 8H), 1.56 (d, 3H), 1.28 (s, 3H).

LCMS m/z =556.2 [M+H]⁺.

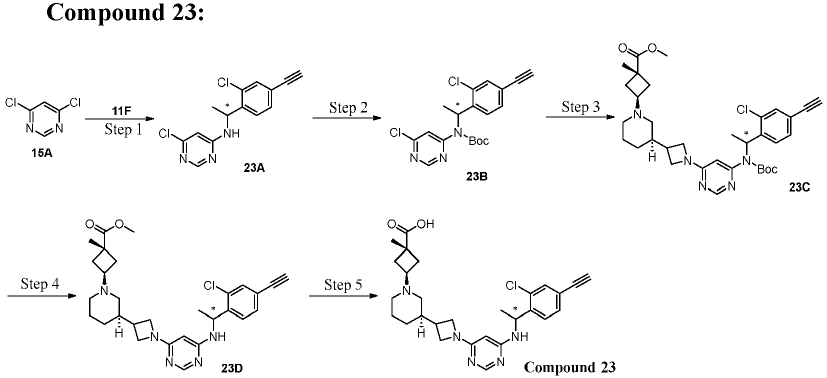

Step 1: Compound **15A** (0.5 g, 3.40 mmol) was dissolved in acetonitrile (15 mL), compound **11F** (0.73 g, 4.10 mmol) and triethylamine (1.0 g, 10.1 mmol) were added, the temperature was raised to 70°C and the reaction was performed for 16 h. After the reaction was completed, the obtained system was cooled to room temperature, and concentrated, the concentrate was dissolved in dichloromethane (30 mL), washed with water (20 mL×3) and brine (20 mL×3) in sequence, and the organic phases were dried over anhydrous sodium sulfate, filtered, and concentrated, the obtained crude product was separated and purified by silica gel column chromatography (petroleum ether:ethyl acetate (v/v) = 3:1-2:1) to obtain the title compound **23A** (0.61 g, 62%).

LC-MS (ESI): m/z = 291.2 [M+H]⁺.

Step 2: Compound **23A** (0.61 g, 2.1 mmol) was dissolved in tetrahydrofuran (10 mL), di-tert-butyl dicarbonate (0.92 g, 4.2 mmol) and DMAP (45 mg, 0.4 mmol) were added, and the obtained system was reacted under reflux for 12 h. After the reaction was completed, the mixture was cooled to room temperature and concentrated. The concentrate was dissolved in dichloromethane (30 mL), washed with water (20 mL×3) and brine (20 mL×3) in sequence, and the organic phase was dried over anhydrous sodium sulfate, filtered, and concentrated to obtain a crude product, which was separated and purified by silica gel column chromatography (petroleum ether:ethyl acetate (v/v) = 3:1-2:1) to obtain the title compound **23B** (0.65 g, 83%).

LC-MS (ESI): m/z = 391.2 [M+H]⁺.

Step 3: Compound **23B** (0.32 g, 0.83 mmol) was dissolved in dimethyl sulfoxide (5 mL), compound **1C** (0.22 g, 0.83 mmol) and triethylamine (0.25 g, 2.49 mmol) were added, the temperature was raised to 150°C, and the reaction was performed under microwave conditions for 1 h. After the reaction was completed, the mixture was cooled to room temperature, and the reaction liquid was dissolved in ethyl acetate (30 mL), the obtained system was washed with water (20 mL×3) and brine (20 mL×3) in sequence. The organic phase was dried over anhydrous sodium sulfate, filtered, and concentrated to obtain a crude product, which was separated and purified by silica gel column chromatography (petroleum ether:ethyl acetate (v/v) = 3:1-2:1) to obtain the title compound **23C** (0.32 g, 62%).

LC-MS (ESI): m/z = 622.2 [M+H]⁺.

Step 4: **23C** (0.32 g, 0.52 mmol) was dissolved in dichloromethane (8 mL), hydrogen chloride dioxane solution (8 mL) was added, and the reaction was performed at room temperature for 1 h after addition was completed. Concentration gave the hydrochloride salt of the title compound **23D** (0.24 g, 90%). The obtained compound could be directly used in the next reaction without further purification.

LC-MS (ESI): m/z =522.2 [M+H]⁺.

Step 5: Compound **23D** (240 mg, 0.47 mmol) was dissolved in tetrahydrofuran (3 mL) and water (3 mL), lithium hydroxide hydrate (98.0 mg, 2.34 mmol) was added, and the obtained system was stirred evenly and reacted at room temperature for 12 h. After the reaction was completed, the mixture was concentrated under reduced pressure and the residue was separated and purified by high performance preparative liquid chromatography to obtain the title **compound 23** (0.17 g, 71%).

Preparation method: 1. Instruments: waters 2767 preparative liquid chromatographic instrument; chromatographic column: SunFire @ Prep C18 (19 mm x 250 mm). 2. The sample was filtered with a 0.45 µm filter head to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min.

¹H NMR (400 MHz, Methanol-*d*₄) δ 7.82 (d, 1H), 7.39 (d, 1H), 7.34-7.18 (m, 2H), 4.97 (d, 2H), 3.88-3.85 (m, 2H), 3.71-3.52 (m, 3H), 3.40-3.27 (m, 1H), 3.13-2.98 (m, 1H), 2.79-2.55 (m, 2H), 2.53-2.29 (m, 2H), 2.11-2.07 (m, 1H), 1.94-1.47 (m, 8H), 1.39 (d, 3H), 1.27 (s, 3H).

LC-MS (ESI): m/z = 508.2 [M+H]⁺.

### Example 24:

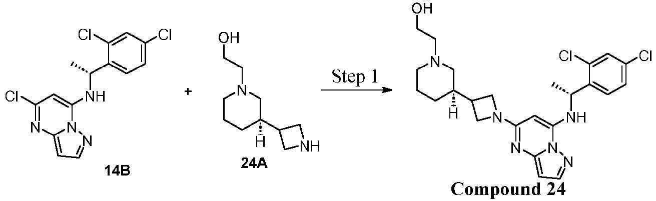

**14B** (0.34 g, 1.0 mmol), **24A** (0.22 g, 1.2 mmol) (prepared with reference to CN 111732572 A), Pd₂(dba)₃ (91 mg, 0.10 mmol), BINAP (0.12 g, 0.20 mmol) and cesium carbonate (0.65 g, 2 mmol) were added to toluene (20 mL) in sequence, the temperature was raised to 95°C and the reaction was performed for 6 h. After the reaction was completed, the mixture was cooled to room temperature, water (30 mL) was added, and the mixture was extracted with ethyl acetate (30 mL×2). The combined organic phase was washed with saturated brine (30 mL), dried over anhydrous sodium sulfate, and filtered, and the filtrate was concentrated under reduced pressure. The residue was separated by silica gel column chromatography (DCM:MeOH (v/v)=8:1) to obtain the crude product of the **compound 24** of interest. The crude product was further subjected to preparative high performance liquid phase chromatography to obtain the trifluoroacetate salt of **compound 24** (0.16 g, yield: 22%).

Preparation conditions: 1. Instruments: waters 2767 preparative liquid chromatographic instrument; chromatographic column: SunFire @ Prep C18 (19 mm x 250 mm). 2. The sample was dissolved in DMF and filtered with a 0.45 µm filter head to prepare a sample liquid. 3. Preparative chromatography conditions: a. composition of mobile phases A and B: mobile phase A: acetonitrile, mobile phase B: water (containing 0.1% TFA) b. gradient elution, mobile phase A 5%-50% c. flow rate: 12 mL/min. d. elution time: 18 min.

¹H NMR (400 MHz, Methanol-*d*₄) δ 7.97 (d, 1H), 7.59-7.52 (m, 2H), 7.39 (dd, 1H), 6.20 (d, 1H), 5.28 (q, 1H), 4.80 (s, 1H), 4.34 (t, 1H), 4.23 (s, 1H), 4.08-3.95 (m, 2H), 3.95-3.88 (m, 2H), 3.68 (d, 1H), 3.58 (d, 1H), 3.31-3.24 (m, 2H), 3.00-2.90 (m, 1H), 2.78-2.65 (m, 2H), 2.25-2.09 (m, 1H), 2.06 (d, 1H), 1.99-1.81 (m, 2H), 1.72 (d, 3H), 1.27-1.14 (m, 1H).

LC-MS (ESI): m/z =489.3 [M+H]⁺.

### Example 25:

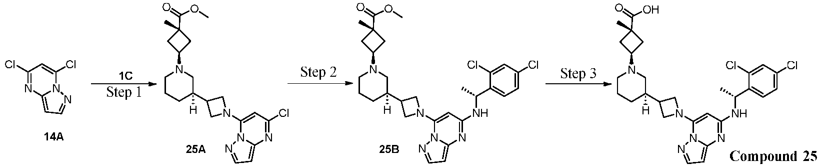

Using **14A** (0.21 g, 1.11 mmol) and **1C** (0.32 g, 1.20 mmol) as the raw materials, the operation method of steps 1 to 3 of example 12 was referenced to obtain the trifluoroacetate salt of **compound 25** (0.11 g).

¹H NMR (400 MHz, Methanol-*d*₄) δ 7.87 (d, 1H), 7.57 (d, 1H), 7.50 (d, 1H), 7.41 (dd, 1H), 6.15 (t, 1H), 5.19 (q, 1H), 4.69 (s, 3H), 4.45 (s, 2H), 3.82-3.75 (m, 1H), 3.53 (d, 1H), 3.42 (d, 1H), 2.90-2.80 (m, 2H), 2.80-2.65 (m, 2H), 2.55-2.45 (m, 1H), 2.27 (d, 2H), 2.17 (s, 1H), 2.07 (d, 1H), 1.98 (d, 1H), 1.87-1.72 (m, 1H), 1.64 (d, 3H), 1.44 (s, 3H), 1.26-1.12 (m, 1H).

LC-MS (ESI): m/z =557.3 [M+H]⁺.

### Example 26:

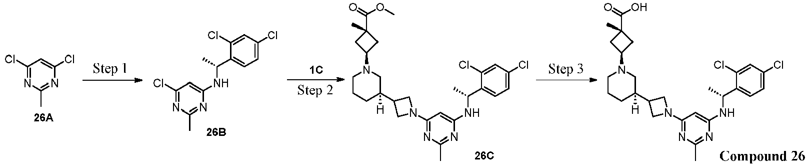

Using **26A** (2.0 g, 12.35 mmol) and (*R*)-1-(2,4-dichlorophenyl)ethylamine (2.33 g, 12.35 mmol) as raw materials, the operation method of steps 1 to 3 of example 14 was referenced to obtain **compound 26** (50 mg).

¹H NMR (400 MHz, CD₃CN; trifluoroacetic acid salt) 7.51-7.50 (m, 1H), 7.44-7.42 (m, 1H), 7.36-7.33 (m, 1H), 4.96 (s, 1H), 4.73 (s, 1H), 4.14-4.04 (m, 2H), 3.90-3.85 (m, 2H), 3.65-3.53 (m, 2H), 3.39-3.29 (m, 3H), 2.69-2.66 (m, 3H), 2.50-2.45 (m, 3H), 2.33 (s, 3H), 2.20-2.15 (m, 3H), 1.85-1.75 (m, 2H), 1.52-1.50 (d, 3H), 1.34 (s, 3H), 1.07-1.01 (m, 1H).

LC-MS (ESI): m/z = 532.2 [M+H]⁺.

### Example 27:

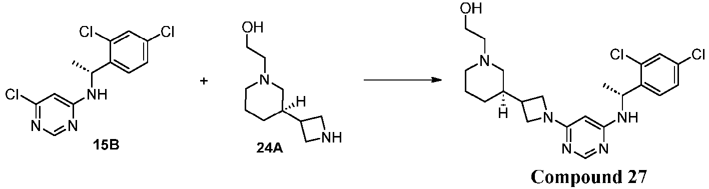

**15B** (0.4 g, 1.33 mmol), **24A** (0.25 g, 1.33 mmol), Pd₂(dba)₃ (0.25 g, 0.27 mmol), XantPhos (0.31 g, 0.54 mmol) and cesium carbonate (1.29 g, 3.99 mmol) were dissolved in 1,4-dioxane (20 mL) in sequence, the temperature was raised to 100°C and the reaction was performed for 4 h. After the reaction was completed, the mixture was cooled to room temperature, water (20 mL) was added, and the mixture was extracted with ethyl acetate (20 mL×3). The organic phases were combined, washed with saturated sodium chloride aqueous solution (20 mL), dried over anhydrous sodium sulfate, filtered, and the filtrate was concentrated under reduced pressure. The residue was separated by silica gel column chromatography (DCM:MeOH (v/v) = 5:1) to obtain the **compound 27** of interest (0.05 g, yield: 9%).

¹H NMR (400 MHz, CD₃CN) δ 8.03 (s, 1H), 7.51-7.50 (m, 1H), 7.45-7.41 (m, 1H), 7.36-7.34 (m, 1H), 5.00-4.97 (m, 1H), 4.85 (s, 1H), 4.19-4.11 (m, 1H), 3.85-3.81 (m, 4H), 3.59-3.47 (m, 3H), 3.18-3.13 (m, 2H), 2.79-2.75 (m, 2H), 2.50-2.47 (m, 3H), 2.19-2.16 (m, 1H), 1.89-1.82 (m, 3H), 1.56-1.54 (d, 3H), 1.07-1.04 (m, 1H).

LC-MS (ESI): m/z = 450.2 [M+H]⁺.

### Example 28:

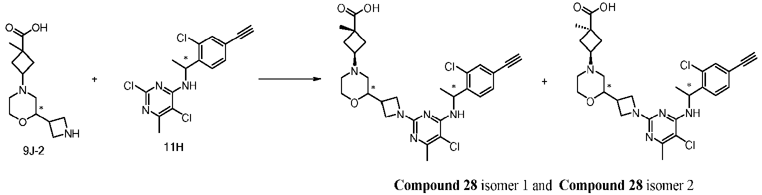

Compound **11H** (200.0 mg, 0.59 mmol), intermediate **9J-2** (300.1 mg, 1.18 mmol), cesium fluoride (227.0 mg, 1.49 mmol), N, N-diisopropylethylamine (0.3 mL) were added to dimethyl sulfoxide (5 mL) in sequence, the obtained system was stirred evenly, then the temperature was raised to 100°C and the reaction was performed for 16 h. After the reaction was completed, the mixture was cooled to room temperature, and water (30 mL) was added, followed by extraction with ethyl acetate (40 mL×2). The combined organic phases were washed with saturated brine (20 mL), dried over anhydrous sodium sulfate, and filtered, and the filtrate was concentrated under reduced pressure. The residue was separated by silica gel column chromatography (EA:MeOH(v/v)=7:1) to obtain **compound 28,** isomer 1 (Rf=0.5, (EA:MeOH(v/v)= (7:1))(30 mg, 10%) and isomer 2 (Rf=0.4, (EA:MeOH(v/v)=7:1)) (20 mg, 6%).

Compound **28,** isomer 1: LC-MS (ESI): m/z =558.2 [M+H]⁺.

1H NMR (400 MHz, DMSO-d6) δ 12.18 (s, 1H), 7.48 (d, 1H), 7.46 (d, 1H), 7.39 (dd, 1H), 7.27 (d, 1H), 5.47-5.37 (m, 1H), 4.22 (d, 1H), 3.75 (d, 4H), 3.50-3.35 (m, 4H), 2.77-2.61 (m, 1H), 2.57 (d, 3H), 2.41 (d, 1H), 2.18 (s, 3H), 2.13 (d, 1H), 1.92-1.65 (m, 3H), 1.44 (d, 4H), 1.32 (s, 1H), 1.27 (s, 2H).

Compound 28, isomer 2: LC-MS (ESI): m/z =558.2 [M+H]⁺.

1H NMR (400 MHz, DMSO-*d6*) δ 12.08 (s, 1H), 7.48 (s, 1H), 7.46 (d, 1H), 7.39 (d, 1H), 7.29 (d, 1H), 5.49-5.35 (m, 1H), 4.22 (s, 1H), 3.75 (d, 4H), 3.50-3.36 (m, 2H), 2.71-2.54 (m, 4H), 2.41 (d, 2H), 2.17 (s, 3H), 1.74 (d, 3H), 1.44 (d, 3H), 1.36 (d, 1H), 1.27 (s, 3H), 1.23 (s, 1H).

### Example 29:

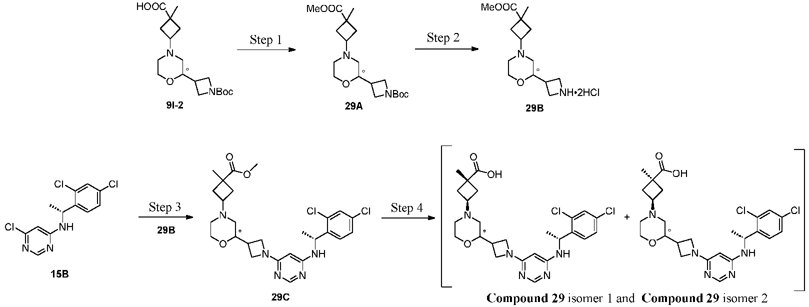

Step 1: Compound **9I-2** (8.4 g, 23.72 mmol) was dissolved in dichloromethane (500 mL) at room temperature, and (trimethylsilyl)diazomethane (23.72 mL, 2M) was added dropwise, after the dropwise addition was completed and the reaction was performed for 1 h. After concentration, the residue was separated and purified by silica gel column chromatography (dichloromethane:methanol (v/v) = 20:1) to obtain the title compound **29A** (8.4 g, 96.3%).

LC-MS (ESI): m/z =369.2 [M+H]⁺.

Step 2: Compound **29A** (8.4 g, 22.82 mmol) was dissolved in dichloromethane (500 mL), hydrogen chloride dioxane solution (200 mL, 4M) was added, and the reaction was performed at room temperature for 30 min. After the reaction was completed, the mixture was directly concentrated to obtain compound **29B** (7.5 g, 100%).

M/Z (ESI): m/z =269.2 [M+H]⁺.

Step 3: Compound **15B** (95.3 mg, 0.32 mmol), **29B** (170.0 mg, 0.63 mmol), cesium fluoride (63.0 mg, 0.63 mmol), and N,N-diisopropylethylamine (0.5 mL) were added to dimethyl sulfoxide (5 mL) in sequence and stirred at 130°C under microwave for 1 h. After the reaction was completed, the mixture was cooled to room temperature, and water (30 mL) was added. The mixture was extracted with ethyl acetate (40 mL×2). The combined organic phases were washed with saturated brine (15 mL), dried over anhydrous sodium sulfate, and filtered, and the filtrate was concentrated under reduced pressure. The residue was separated by silica gel column chromatography (dichloromethane:methanol (v/v) = 15:1) to obtain **29C** (100.0 mg, 30%).

Step 4: Compound **29C** (100.0 mg, 0.19 mmol) was dissolved in a mixed solvent (6 mL) (tetrahydrofuran: methanol: water (v/v/v)=1:1:1), and lithium hydroxide (18.0 mg, 0.76 mmol) was added, the obtained system was stirred at room temperature for 2h. After the reaction was completed, the obtained system was concentrated and the residue was separated by silica gel column chromatography (EA:MeOH(v/v)=4:1) to obtain **compound 29,** isomer 1 (Rf=0.6, (EA:MeOH(v/v)=4:1)) (30 mg, 30%), **Compound 29,** isomer 2 (Rf=0.5, (EA:MeOH (v/v)=4:1)) (22 mg, 22%).

Compound **29,** isomer 1: LC-MS (ESI): m/z =520.5 [M+H]⁺.

¹H NMR (400 MHz, DMSO-*d6*) δ 7.88 (s, 1H), 7.55 (d, 1H), 7.45-7.37 (m, 3H), 5.14 (s, 2H), 3.82 (d, 3H), 3.71-3.65 (m, 2H), 3.55-3.44 (m, 4H), 2.75-2.68 (m, 3H), 2.42 (d, 2H), 1.83-1.68 (m, 3H), 1.44 (t, 1H), 1.36 (d, 3H), 1.28 (s, 3H).

Compound **29,** isomer 2: LC-MS (ESI): m/z =520.5 [M+H]⁺.

¹H NMR (400 MHz, DMSO-d6) δ 7.88 (s, 1H), 7.55 (d, 1H), 7.46-7.37 (m, 3H), 5.14 (s, 2H), 3.82 (d, 3H), 3.72-3.65 (m, 2H), 3.53-3.44 (m, 4H), 2.73 (d, 2H), 2.60 (d, 2H), 2.18-2.13 (m, 1H), 1.93-1.76 (m, 3H), 1.47 (t, 1H), 1.36 (d, 3H), 1.31 (s, 3H).

### Example 30:

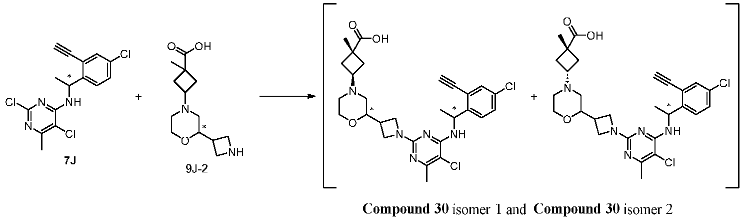

**7J** (0.4 g, 1.18 mmol) was dissolved in dimethyl sulfoxide (5 mL), and **9J-2** (0.3 g, 1.18 mmol), N,N-diisopropylethylamine (0.46 g, 3.54 mmol) and cesium fluoride (0.36 g, 2.36 mmol) were added in sequence, the temperature was raised to 100°C and the reaction was performed for 4 h. After the reaction was completed, the mixture was cooled to room temperature, and water (15 mL) and ethyl acetate (15 mL) were added for extraction. The aqueous phase was back-extracted with ethyl acetate (15 mL×2). The combined organic phases were washed with saturated brine, dried over anhydrous sodium sulfate, filtered, and concentrated. The residue was purified by silica gel column chromatography (ethyl acetate:methanol (v/v) = 10:1) to obtain **compound 30** isomer 1 (Rf = 0.4 (ethyl acetate:methanol = 10:1), 35.0 mg, 5.3%) and the title **compound 30** isomer 2 (Rf = 0.2 (ethyl acetate:methanol = 10:1)).

**Compound 30** isomer 1: ¹H NMR (400 MHz, DMSO-*d*₆) δ 12.20 (s, 1H), 7.49-7.37 (m, 3H), 7.18 (d, 1H), 5.58-5.40 (m, 1H), 4.55 (s, 1H), 3.87-3.62 (m, 4H), 3.49-3.37 (m, 2H), 2.68-2.54 (m, 4H), 2.46-2.37 (m, 2H), 2.17 (s, 3H), 1.96-1.86 (m, 1H), 1.81-1.66 (m, 3H), 1.44 (d, 3H), 1.41-1.33 (m, 1H), 1.27 (s, 3H).

M/Z (ESI): m/z =558.6[M+H]⁺.

### Example 31:

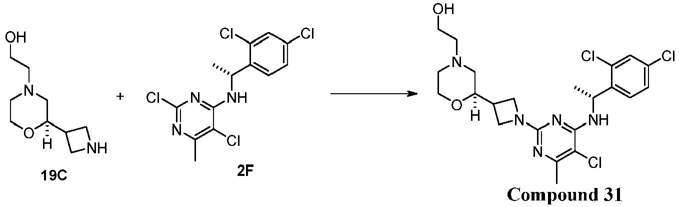

Compound **2F** (0.15 g, 0.43 mmol) and compound **19C** (0.26 g, 0.65 mmol) were dissolved in dimethyl sulfoxide (5 mL), triethylamine (0.13 g, 1.3 mmol) and cesium fluoride (0.13 g, 0.86 mmol) were added, and the temperature was raised to 100°C and the reaction liquid was stirred for 5 h. After the reaction was completed, the mixture was cooled to room temperature, water (20 mL) and ethyl acetate (30 mL×3) were added for extraction. The combined organic phases were washed with saturated brine, dried over anhydrous sodium sulfate, and filtered. The filtrate was concentrated and the residue was separated and purified by silica gel column chromatography (dichloromethane:methanol (v/v) = 10:1) to obtain the title **compound 31** (90.0 mg, 41.8%).

¹H NMR (400 MHz, CDCl₃) δ 7.35 (d, 1H), 7.23 (d, 1H), 7.18-7.16 (m, 1H), 5.50 (d, 1H), 5.46-5.41 (m, 1H), 3.99-3.96 (m, 1H), 3.91-3.59 (m,8H), 2.75 (d, 2H), 2.60-2.54 (m, 3H), 2.30 (s, 3H), 2.28-2.22 (m, 1H), 1.92-1.87 (m, 1H), 1.51 (s, 3H).

MS M/Z (ESI): m/z =500.2[M+H]⁺.

### Example 32:

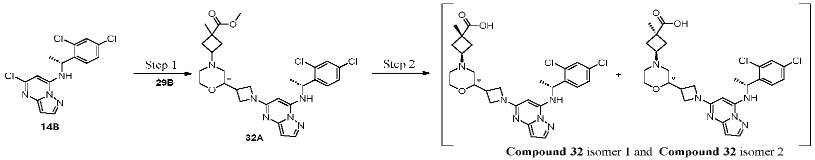

Using compound **14B** (160.6 mg, 0.47 mmol) and **29B** (120.0 mg, 0.47 mmol) as the raw materials, the operation method of steps 3 and 4 of example 29 was referenced to obtain **compound 32,** isomer 1 (Rf=0.6, (ethyl acetate:methanol (v/v)=5:1), 4 mg), and **compound 32,** isomer 2 (Rf=0.5, (EA:MeOH(v/v)=5:1), 5 mg).

Compound **32,** isomer 1: LC-MS (ESI): m/z =577.3 [M+H]⁺.

¹H NMR (400 MHz, DMSO-*d6*) δ 7.99 (d, 1H), 7.81 (d, 1H), 7.66-7.60 (m, 2H), 7.45-7.40 (m, 1H), 5.90 (d, 1H), 4.74 (s, 1H), 3.86-3.47 (m, 6H), 3.49 (d, 2H), 2.69- 2.59 (m, 5H), 2.12 (s, 1H), 2.00 (d, 1H), 1.81 (d, 3H), 1.58 (d, 3H), 1.24 (s, 3H).

Compound **32,** isomer 2: LC-MS (ESI): m/z =577.3 [M+H]⁺.

¹H NMR (400 MHz, DMSO-*d6*) δ 7.99 (d, 1H), 7.81 (d, 1H), 7.67-7.60 (m, 2H), 7.43 (dd, 1H), 5.90 (d, 1H), 4.73 (s, 1H), 3.90-3.67 (m, 6H), 3.48 (d, 2H), 2.71-2.57 (m, 5H), 2.44- 2.39 (m, 1H), 2.16 (s, 1H), 1.92-1.82 (m, 1H), 1.79 (d, 1H), 1.69 (d, 1H), 1.59 (d, 3H), 1.23 (s, 3H).

### Example 33:

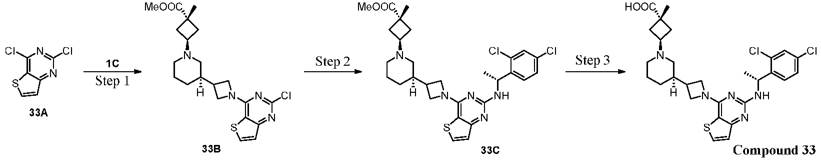

Using **33A** (0.4 g, 1.96 mmol) and **1C** (0.52 g, 1.96 mmol) as the raw materials, the operation method of steps 1 to 3 of example 12 was referenced to obtain **compound 33** (100 mg).

¹H NMR (400 MHz, CD₃CN; trifluoroacetic acid salt) δ 9.95 (s, 1H), 8.00-7.99 (m, 1H), 7.48-7.43 (m, 2H), 7.31-7.29 (m, 1H), 7.10-7.08 (m, 1H), 5.52-5.43 (m, 1H), 4.56-4.51 (m, 1H), 4.33-4.28 (m, 2H), 4.08-4.00 (m, 1H), 3.61-3.55 (m, 1H), 3.41-3.31 (m, 3H), 2.71-2.68 (m, 3H), 2.48-2.33 (m, 4H), 2.25-2.12 (m, 2H), 1.85-1.80 (m, 2H), 1.51-1.49 (d, 3H), 1.40-1.34 (m, 3H), 1.09-1.01 (m, 1H).

LC-MS (ESI): m/z = 574.2 [M+H]⁺.

### Example 34:

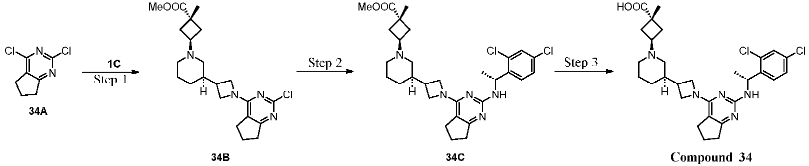

Using **34A** (0.2 g, 1.06 mmol) and **1C** (0.28 g, 1.06 mmol) as the raw materials, the operation method of steps 1 to 3 of example 12 was referenced to obtain **compound 34** (80 mg).

¹H NMR (400 MHz, CD₃OD) 7.42-7.40 (m, 2H), 7.27-7.24 (m, 1H), 5.37-5.32 (m, 1H), 4.19-4.09 (m, 2H), 3.92-3.88 (m, 1H), 3.71-3.66 (m, 1H), 3.34-3.32 (m, 2H), 3.27-3.24 (m, 1H), 3.11-3.06 (m, 1H), 2.80-2.64 (m, 6H), 2.48-2.42 (m, 2H), 2.13-1.92 (m, 7H), 1.83-1.80 (m, 2H), 1.71-1.68 (m, 1H), 1.47-1.45 (d, 3H), 1.39 (s, 3H), 1.09-1.00 (m, 1H).

LC-MS (ESI): m/z = 558.2 [M+H]⁺.

### Example 35:

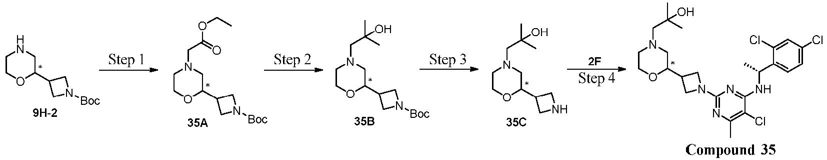

Step 1: Tert-butyl 3-(morpholin-2-yl)azetidine-1-carboxylate (1 g, 4.17 mmol) was dissolved in acetonitrile (10 mL), and ethyl 2-bromoacetate (1.04 g, 6.25 mmol) and diisopropylethylamine (1.61 g, 12.51 mmol) were added, and the obtained system was stirred at room temperature for 3 h. After the reaction was completed, the obtained system was concentrated to obtain the crude product of **35B** (1.31 g, 96%).

LC-MS (ESI): m/z = 329.5[M+H]⁺.

Step 2: Under nitrogen atmosphere, compound **35B** (300 mg, 0.91 mmol) was dissolved in dry tetrahydrofuran (5 mL), methylmagnesium bromide (2.73 mmol) was added dropwise at -78°C, after the dropwise addition was completed, the reaction was performed at room temperature for 2 h. After the reaction was completed, saturated aqueous ammonium chloride solution (10 mL) was added to quench the reaction, and ethyl acetate (15 mL×2) was used for extraction. The combined organic phases were washed with saturated brine, dried over anhydrous sodium sulfate, and filtered. The filtrate was concentrated and subjected to column chromatography (petroleum ether:ethyl acetate (v/v) = 0-30%) to obtain 35C (200 mg, 70%).

LC-MS (ESI): m/z=315.5 [M+H]⁺.

Step 3: Compound **35C** (200 mg, 0.64) was dissolved in hydrogen chloride 1,4-dioxane (2 mL), and stirred at room temperature for 1 h, and the obtained system was directly dried to obtain the hydrochloride compound of **35D** (150 mg).

LC-MS (ESI): m/z = 215.2[M+H]⁺.

Step 4: Compound **35D** (150 mg, 0.64 mmol) was dissolved in DMSO (5 mL), and (R)-2,5-dichloro-N-(1-(2,4-dichlorophenyl)ethyl)-6-methylpyrimidin-4-amine (225 mg, 0.64 mmol), DIEA (248 mg, 1.92 mmol), and cesium fluoride (97 mg, 0.64 mmol) were added in sequence. The obtained system was heated to 100°C and stirred for 2 h. After the reaction was completed, water (15 mL) and ethyl acetate (15 mL) were added for extraction and liquid separation. The aqueous phase was extracted with ethyl acetate (15 mL×2). The combined organic phases were washed with saturated brine, dried over anhydrous sodium sulfate, filtered, and concentrated. The residue was purified by column chromatography (ethyl acetate: petroleum ether (v/v) = 0-50%) to obtain **compound 35** (80 mg, 24%).

LC-MS (ESI): m/z = 528.6[M+H]⁺.

¹H NMR (400 MHz, CDCl₃) δ 7.35 (d, 1H), 7.23 (d, 1H), 7.18-7.17 (m, 1H), 5.50 (d, 1H), 5.44-5.43 (m, 1H), 3.98 (t, 1H), 3.89-3.80 (m, 2H), 3.74-3.73 (m, 2H), 3.65-3.64 (m, 2H), 2.73 (s, 2H), 2.58-2.52 (m, 1H), 2.46 (t, 1H), 2.33 (d, 2H), 2.30 (s, 3H), 2.09 (t, 1H), 1.51 (d, 3H), 1.17 (s, 6H).

### Example 36:

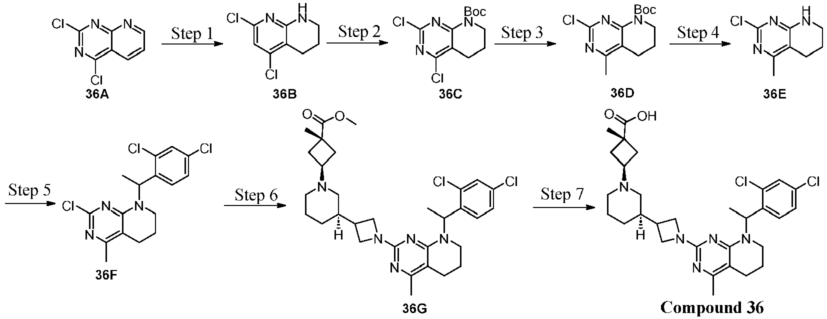

Step 1: Compound **36A** (2.8 g, 13.97 mmol) and platinum dioxide (0.16 g, 0.70 mmol) were mixed and dissolved in a solution of ethanol (20 mL) and ethyl acetate (30 mL), the reaction was performed at room temperature for 4 h under a hydrogen atmosphere. The obtained system was filtered through celite and the filter cake was washed with ethyl acetate (30 mL). The filtrate was concentrated under reduced pressure and separated by silica gel column chromatography (PE:EA (v/v)=3:1) to obtain the compound **36B** of interest (0.52 g, yield: 18%).

LC-MS M/Z (ESI)=204.30 [M+H]⁺.

Step 2: Compound **36B** (0.52 g, 2.55 mmol) and di-tert-butyl dicarbonate (1.11 g, 5.07 mmol) were mixed and dissolved in acetonitrile (15 mL), and DMAP (62 mg, 0.51 mmol) was added, the reaction was performed at room temperature for 4 h. After the reaction was completed, the obtained system was concentrated under reduced pressure and directly separated by silica gel column chromatography (PE:EA (v/v)=5:1) to obtain the compound **36C** of interest (0.58 g, yield: 75%).

LC-MS M/Z (ESI)=248.1 [M-^{t}Bu+H]⁺.

Step 3: Compound **36C** (0.58 g, 1.91 mmol) and iron acetylacetonate (67 mg, 0.19 mmol) were mixed and dissolved in THF (10 mL), the obtained system was cooled to 0°C, and methylmagnesium chloride (1.2 mL, 3M solution in THF) was added dropwise, after the dropwise addition was completed, the reaction was continued under the same condition for 1 h. The reaction was quenched with saturated ammonium chloride (30 mL), and extracted with ethyl acetate (30 mL×2), and the combined organic phase was dried over anhydrous sodium sulfate and filtered. The filtrate was concentrated under reduced pressure and separated by silica gel column chromatography (PE:EA(v/v)=4:1) to obtain the compound **36D** of interest (0.32 g, yield: 59%).

¹H NMR (400 MHz, Chloroform-d) δ 3.79-3.72 (m, 2H), 2.65 (t, 2H), 2.40 (s, 3H), 2.03 -1.94 (m, 2H), 1.56 (s, 9H).

Step 4: Compound **36D** (0.32 g, 1.13 mmol) was dissolved in DCM (10 mL), the obtained system was cooled to 0°C, trifluoroacetic acid (3 mL) was added dropwise, after the dropwise addition was completed, the reaction was continued at room temperature for 1 h After concentration under reduced pressure, ethyl acetate (30 mL) was added, and the mixture was washed with saturated sodium bicarbonate (30 mL). The aqueous phase was extracted again with ethyl acetate (30 mL). The organic phases were combined, dried over anhydrous sodium sulfate, and filtered. The filtrate was concentrated under reduced pressure to obtain the compound **36E** of interest (0.14 g, yield: 67%).

Step 5: Compound **36E** (0.14 g, 0.76 mmol) was dissolved in DMF (10 mL), the obtaines system was cooled to 0°C, sodium hydride (60 mg, 1.52 mmol, 60% wt) was added; after the reaction was continued at room temperature for 20 min, 1-(1-bromoethyl)-2,4-dichlorobenzene (0.39 g, 1.52 mmol) was added dropwise; after the dropwise addition was completed, the temperature was raised to room temperature and the reaction was performed for 30 min. The reaction was quenched with saturated aqueous sodium bicarbonate solution (30 mL), extracted with ethyl acetate (30 mL×2), and the combined organic phase was dried over anhydrous sodium sulfate and filtered. The filtrate was concentrated under reduced pressure and separated by silica gel column chromatography (PE:EA (v/v)=4:1) to obtain the compound **36F** of interest (0.19 g, yield: 70%).

LC-MS M/Z (ESI)=356.0 [M+H]⁺.

Step 6: **36F** (0.19 g, 0.53 mmol) was dissolved in DMSO (10 mL), **1C** (0.14 g, 0.53 mmol), triethylamine (0.27 g, 2.65 mmol) and cesium fluoride (0.16 g, 1.06 mmol) were added in sequence, after the addition was completed, the reaction was preformed at 100°C for 24 h. After the reaction was cooled to room temperature, water (20 mL) was added and the mixture was extracted with ethyl acetate (30 mL×3). The combined organic phase was washed with saturated brine (40 mL), dried over anhydrous sodium sulfate, and filtered, and the filtrate was concentrated under reduced pressure and purified by silica gel column chromatography (DCM:MeOH (v/v)=12:1) to obtain the compound **36G** of interest (36 mg, yield: 12%).

LC-MS M/Z (ESI)=586.60 [M+H]⁺.

Step 7: **36G** (0.036 g, 0.061 mmol) was dissolved in tetrahydrofuran (10 mL), methanol (3 mL) and water (3 mL) were added, the obtained system was stirred evenly, and lithium hydroxide (0.015 g, 0.63 mmol) was added, the reaction was performed at room temperature for 3 h. Hydrochloric acid (1N aqueous solution) was added dropwise to adjust the pH to 5. The system was concentrated under reduced pressure and directly separated by silica gel column chromatography (DCM:MeOH (v/v) =10:1) to obtain a crude product of the compound **36** of interest. The compound was further subjected to preparative high performance liquid phase chromatography (preparation conditions 1. Instruments: waters 2767 preparative liquid chromatographic instrument; chromatographic column: SunFire@ Prep C18 (19mm×250mm). 2. The sample was dissolved in DMF and filtered with a 0.45 µm filter head to prepare a sample liquid. 3. Preparative chromatography conditions: a. composition of mobile phases A and B: mobile phase A: acetonitrile, mobile phase B: water (containing 0.1% TFA) b. gradient elution, mobile phase A: 5% to 50%, c. flow rate: 15 ml/min. d. elution time: 18 min.) The trifluoroacetate salt of compound **36** (15 mg, yield: 30%) was obtained.

LC-MS M/Z (ESI)=572.2 [M+H]⁺.

¹H NMR (400 MHz, Methanol-*d*₄) δ 7.61-7.51 (m, 2H), 7.47-7.41 (d, 1H), 6.33- 6.21 (m, 1H), 4.38-4.25 (m, 2H), 4.12-3.97 (m, 2H), 3.82-3.69 (m, 1H), 3.53 (d, 1H), 3.42-3.35 (d, 2H), 3.02-2.90 (m, 1H), 2.89-2.78 (m, 2H), 2.79-2.59 (m, 3H), 2.59-2.42 (m, 2H), 2.30 (s, 5H), 2.17-2.02 (m, 2H), 1.99-1.75 (m, 4H), 1.62 (d, 3H), 1.44 (s, 3H), 1.27-1.12 (m, 1H).

### Example 37:

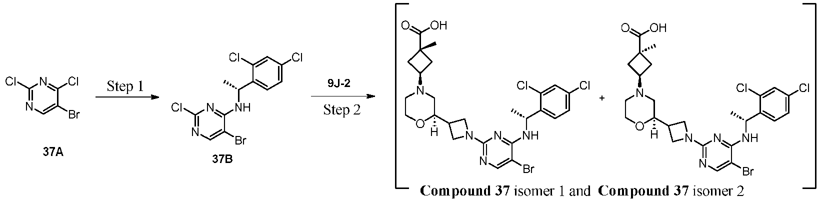

Step 1: Compound **37A** (0.5 g, 2.22 mmol) was dissolved in acetonitrile (10 mL), and (R)-1-(2,4-dichlorophenyl)ethan-1-amine (0.46 g, 2.44 mmol) and triethylamine (672 mg, 6.66 mmol) were added, the reaction was performed at room temperature for 16 h. After the reaction was completed, water (20 mL) and ethyl acetate (30 mL) were added for extraction and liquid separation. The aqueous phase was extracted with ethyl acetate (30 mL). The combined organic phases were washed with saturated brine, dried over anhydrous sodium sulfate, filtered, and concentrated. The crude product was separated and purified by silica gel column chromatography (petroleum ether:ethyl acetate (v/v) = 5:1) to obtain compound **37B** (0.5 g, 59%).

LCMS (ESI): m/z =380.1 [M+H]⁺.

Step 2: Compound **37B** (200 mg, 0.53 mmol), N,N-diisopropylethylamine (341 mg, 2.65 mmol) and cesium fluoride (8 mg, 0.05 mmol) were added to a solution of compound **9J-2** (135 mg, 0.53 mmol) in dimethyl sulfoxide (3 mL), the temperature was raised to 100°C and the reaction was performed for 16 h. After the reaction was completed, the mixture was cooled to room temperature, and water (15 mL) and ethyl acetate (15 mL) were added for extraction and liquid separation. The aqueous phase was extracted with ethyl acetate (15 mL). The combined organic phases were washed with saturated brine, dried over anhydrous sodium sulfate, filtered, and concentrated. After the filtrate was concentrated, the residue was separated and purified by silica gel column chromatography (ethyl acetate:methanol (v/v) = 5:1) to obtain the title **compound 37** isomer 1 (Rf = 0.6 (ethyl acetate: methanol = 5:1), 30.0 mg, 9%) and the title **compound 37** isomer 2 (Rf = 0.4 (ethyl acetate: methanol = 5:1), 20.0 mg, 6%).

Compound **37,** isomer 1: LC-MS (ESI): m/z =598.1 [M+H]⁺.

¹H NMR (400 MHz, CDCl₃) δ 7.98 (s, 1H), 7.36 (d, 1H), 7.22-7.17 (m, 2H), 5.55 -5.41 (m, 2H), 3.99-3.77 (m, 5H), 3.68-3.58 (m, 2H), 2.82-2.77 (m, 1H), 2.69-2.51 (m, 6H), 1.92-1.84 (m, 2H), 1.52 (d, 3H), 1.40 (s, 3H).

Compound **37,** isomer 2: LC-MS (ESI): m/z =598.1 [M+H]⁺.

¹H NMR (400 MHz, CDCl₃) δ7.90 (s, 1H), 7.36 (d, 1H), 7.24-7.16 (m, 2H), 5.49 -5.41 (m, 2H), 3.97-3.92 (m, 2H), 3.87-3.77 (m, 3H), 3.02-2.86 (m, 3H), 2.64-2.52 (m, 3H), 2.18-2.00 (m, 4H), 1.73-1.64 (m, 1H), 1.52 (d, 3H), 1.42 (s, 3H).

### Example 38:

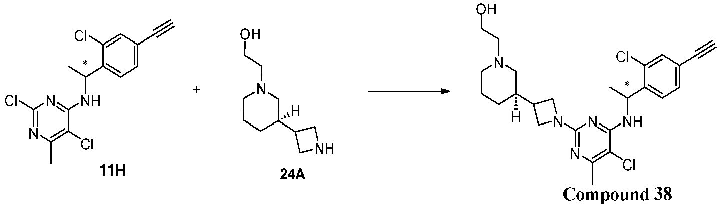

Using **24A** (170 mg, 0.92 mmol) and **11H** (313.4 mg, 0.92 mmol) as the raw materials, the operation method of example 24 was referenced to obtain **compound 38** (38 mg, 10%).

¹H NMR (400 MHz, DMSO-*d6*) δ 7.49 (d, 1H), 7.45 (d, 1H), 7.40-7.39 (m, 1H), 7.28 (d, 1H), 5.41 (d, 1H), 4.23 (s, 1H), 3.84 (t, 1H), 3.76 (s, 1H), 3.52 (d, 3H), 2.67 (s, 2H), 2.50 (s, 2H), 2.45-2.25 (m, 3H), 2.18 (s, 3H), 1.93 (s, 1H), 1.59 (s, 4H), 1.42 (t, 4H), 1.23 (s, 1H).

LC-MS (ESI): m/z =488.6 [M+H]⁺.

### Example 39:

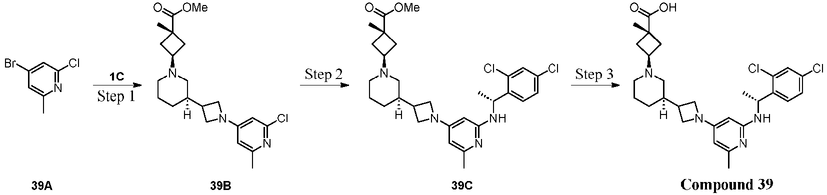

Using compound **39A** (359 mg, 1.74 mmol) and **1C** (300 mg, 0.79 mmol) as the raw materials, the operation method of steps 1 to 3 of example 12 was referenced to obtain **compound 39** (23 mg).

LC-MS (ESI): m/z = 531.2 [M+H]⁺.

¹H NMR (400 MHz, CD₃OD) δ 7.55 (s, 1H), 7.47 (d, J = 8.5 Hz, 1H), 7.38 (d, J = 8.5, 1H), 5.88 (s, 1H), 5.04-5.03 (m, 1H), 5.00 (m, 1H), 4.17 (m, 2H), 3.91 (m, 2H), 3.83-3.64 (m, 2H), 3.51 (m, 1H), 2.88-2.80 (m, 2H), 2.75-2.65 (m, 2H), 2.48 (m, 1H), 2.35 (s, 3H), 2.25-2.20 (m, 2H), 2.12-1.98 (m, 2H), 1.97-1.86 (m, 1H), 1.83-1.72 (m, 1H), 1.59 (d, 3H), 1.44 (s, 3H), 1.18-1.11 (m, 1H).

### Example 40:

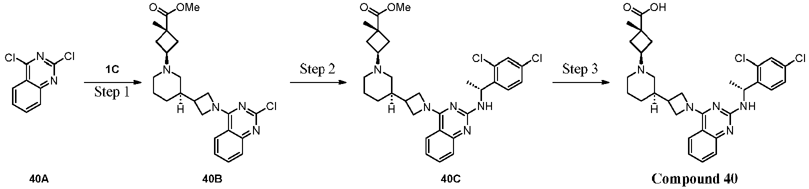

Using compound 1C (275 mg, 0.93 mmol) and compound **40A** (241 mg, 1.21 mmol) as the raw materials, the operation method of steps 1 to 3 of example 12 was referenced to obtain **compound 40** (40 mg).

LC-MS (ESI): m/z = 569.3 [M+H]⁺.

¹H NMR (400 MHz, CD₃OD) δ 7.89 (m, 1H), 7.81 (m, 1H), 7.53 (m, 1H), 7.51-7.46 (m, 2H), 7.42 (m, 1H), 7.35 (m, 1H), 5.64-5.52 (m, 1H), 4.97 (m, 1H), 4.76-4.47 (m, 2H), 4.37-4.14 (m, 1H), 3.79 (m, 1H), 3.53 (m, 1H), 3.41 (m, 1H), 2.83 (m, 2H), 2.72 (m, 2H), 2.52 (m, 1H), 2.31 (m, 2H), 2.12 (m, 2H), 1.81 (m, 2H), 1.60 (m, 3H), 1.44 (s, 3H), 1.21 (m, 1H).

### Example 41:

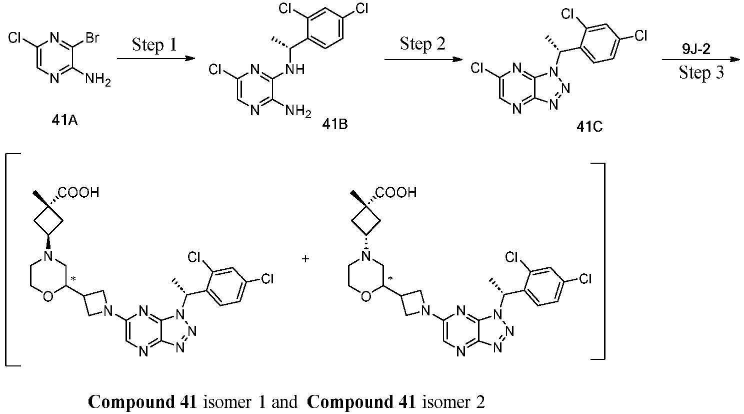

Step 1: Compound **41A** (2.0 g, 9.60 mmol), (R)-1-(2,4-dichlorophenyl)ethan-1-amine (1.83 g, 9.60 mmol) and N, N-diisopropylethylamine (1.86 g, 14.39 mmol) were added to NMP (20 mL), and the temperature was raised to 160°C and the reaction was performed for 48 h. The mixture was cooled to room temperature, and water (100 mL) and ethyl acetate (30 mL) were added for extraction and liquid separation. The aqueous phase was extracted with ethyl acetate (20 mL×3). The combined organic phases were washed with saturated brine, dried over anhydrous sodium sulfate, filtered, and concentrated. After the filtrate was concentrated, the residue was separated and purified by silica gel column chromatography (petroleum ether:ethyl acetate (v/v) = 5:1) to obtain the **compound 41B** of interest (2.2 g, 72.2%).

LC-MS (ESI): m/z =317.0[M+H]⁺.

Step 2: **41B** (2.0 g, 6.30 mmol) was dissolved in acetic acid (18 mL), a solution of sodium nitrite (434 mg, 6.30 mmol) in water (3 mL) was added dropwise, and the obtained system was stirred for 2 h. After the reaction was completed, the solvent was removed by concentration under reduced pressure, saturated sodium bicarbonate solution (30 mL) was added and the mixture was extracted with dichloromethane (20 mL × 3), the combined organic phase was dried over anhydrous sodium sulfate, filtered, and concentrated, and the crude product was separated by silica gel column chromatography (petroleum ether:ethyl acetate (v/v) = 3:1) to obtain **41C** (1.2 g, 58.0%).

LC-MS (ESI): m/z =328.3[M+H]⁺.

Step 3: **41C** (100 mg, 0.30 mmol), **9J-2** (82 mg, 0.30 mmol), and triethylamine (154 mg, 1.52 mmol) were dissolved in acetonitrile (5 mL), the obtained system was stirred at room temperature for 2h. After the reaction was completed, water (30 mL) was added to quench the reaction, and ethyl acetate (15 mL × 3) was used for extraction. The combined organic phase was washed with a saturated saline solution, dried over anhydrous sodium sulfate, and filtered. The filtrate was concentrated and the residue was separated using a silica gel preparation plate (ethyl acetate:methanol (v/v) = 10:1) to obtain compound **41** isomer 1 (70 mg, Rf=0.5, yield: 42.1%) and compound **41** isomer 2 (50 mg, Rf=0.3, yield: 30.1%).

**Compound 41** isomer 1: LC-MS(ESI): m/z =546.2[M+H]⁺.

¹H NMR (400 MHz, DMSO-*d*₆) δ 12.17 (s, 1H), 7.99 (s, 1H), 7.67 (d, 1H), 7.53- 7.42 (m, 2H), 6.33-6.32 (m, 1H), 4.27-3.94 (m, 4H), 3.88-3.76 (m, 1H), 3.65-3.44 (m, 2H), 2.89-2.78 (m, 1H), 2.70-2.55 (m, 3H), 2.47-2.37 (m, 2H), 1.97 (d, 3H), 1.86-1.77 (m, 1H), 1.77-1.68 (m, 2H), 1.54-1.44 (m, 1H), 1.28 (s, 3H).

**Compound 41** isomer 2: LC-MS(ESI): m/z =546.2[M+H]⁺.

¹H NMR (400 MHz, DMSO-*d*₆) δ 12.18 (s, 1H), 7.99 (s, 1H), 7.66 (d, 1H), 7.53- 7.43 (m, 2H), 6.35-6.34 (m, 1H), 4.27-3.91 (m, 4H), 3.88-3.77 (m, 1H), 3.66-3.46 (m, 2H), 2.90-2.80 (m, 1H), 2.80-2.70 (m, 1H), 2.70-2.55 (m, 2H), 2.24-2.10 (m, 2H), 1.97 (d, 3H), 1.94-1.78 (m, 3H), 1.57-1.45 (m, 1H), 1.32 (s, 3H).

### Example 42:

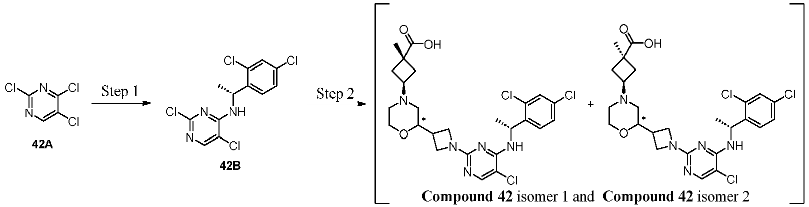

Using **42A** (1 g, 5.46 mmol) and (*R*)-1-(3,5-dichlorophenyl)ethan-1-amine (1.04 g, 5.46 mmol) as the raw materials, the operation method of steps 1 and 2 of example 37 was referenced to obtain **compound 42** isomer 1 (Rf=0.5, (EA:MeOH(v/v)=7:1), 150 mg, 46%) and **compound 42** isomer 2 (Rf=0.4, (EA:MeOH(v/v)=7:1), 20 mg, 6%).

LC-MS (ESI): m/z =554.2 [M+H]⁺.

Compound **42** isomer 1: ¹H NMR (400 MHz, CDCl₃) δ = 7.92 (s, 1H), 7.37 (d, 1H), 7.22 (d, 1H), 7.19-7.18 (m, 1H), 5.54-5.53 (m, 1H), 5.45-5.44 (m, 1H), 4.05-3.87 (m, 3H), 3.84 (s, 2H), 3.64 (d, 2H), 2.83 (s, 1H), 2.69 (s, 2H), 2.64-2.52 (m, 3H), 1.89 (s, 3H), 1.52 (d, 4H), 1.40 (s, 3H).

Compound **42** isomer 2: ¹H NMR (400 MHz, CDCl₃) δ = 7.81 (s, 1H), 7.36 (s, 1H), 7.23 (d, 1H), 7.18 (d, 1H), 5.46 (s, 2H), 3.96 (d, 2H), 3.85 (d, 2H), 3.78 (d, 3H), 3.00-2.99 (m, 2H), 2.87 (s, 1H), 2.63-2.52 (m, 3H), 2.19-2.06 (m, 3H), 1.52-1.51 (m, 3H), 1.42 (s, 3H).

### Example 43:

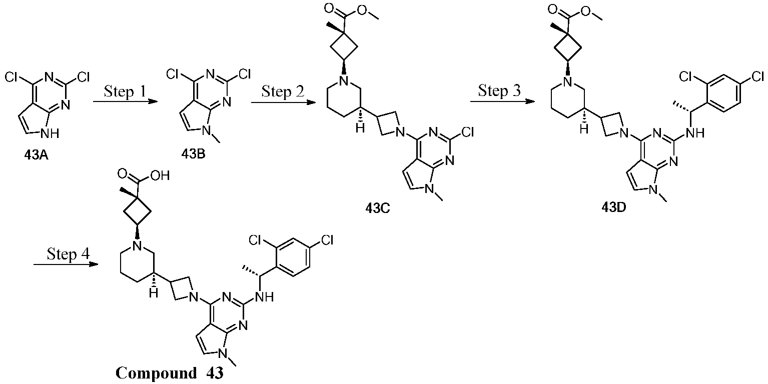

Step 1: **43A** (1.5 g, 8.0 mmol) was dissolved in dry N.N-dimethylformamide (30 mL), the obtained system was cooled to 0°C under nitrogen protection, and sodium hydride (0.40 g, 10 mmol, 60% wt) was added in portions, after the addition was complete, the reaction was performed for 20 min under the same condition. Iodomethane (1.70 g, 12 mmol) was added dropwise and the mixture was reacted at room temperature for 30 min. Water (100 mL) was added to quench the reaction, and the mixture was extracted with ethyl acetate (100 mL×2). The organic phases were combined, washed with saturated brine (100 mL×1), dried over anhydrous sodium sulfate, and filtered, and the filtrate was concentrated under reduced pressure. The residue was separated by silica gel column chromatography (PE:EA (v/v) =5:1) to obtain the compound **43B** of interest (1.43 g, yield: 89%).

LC-MS (ESI): m/z =202.1 [M+H]⁺.

Steps 2 to 4: Using **43B** (0.20 g, 0.99 mmol) and 1C (0.26 g, 1 mmol) as the raw materials, the operation method of steps 1 to 3 of example 12 was referenced to obtain the trifluoroacetate salt of **compound 43** (80 mg).

LC-MS (ESI): m/z =571.6 [M+H]⁺.

¹H NMR (400 MHz, Methanol-*d*₄) δ 7.53-7.42 (m, 2H), 7.31 (d, 1H), 6.90 (d, 1H), 6.46 (d, 1H), 5.52 (q, 1H), 4.65-4.20 (m, 4H), 3.86-3.70 (m, 1H), 3.62 (s, 3H), 3.57- 3.48 (m, 1H), 3.42 (d, 1H), 2.92-2.80 (m, 2H), 2.75 (d, 2H), 2.52 (t, 1H), 2.36-1.90 (m, 5H), 1.81 (d, 1H), 1.56 (d, 3H), 1.44 (s, 3H), 1.35-1.10 (m, 1H).

### Example 44:

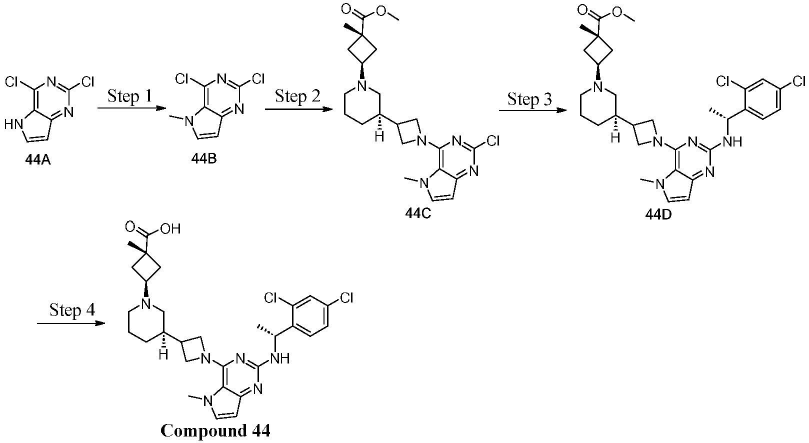

Step 1: **44A** (1.5 g, 8.0 mmol) was dissolved in dry tetrahydrofuran (30 mL), the obtained system was cooled to 0°C under nitrogen protection, and sodium hydride (0.40 g, 10 mmol, 60% wt) was added in portions, after the addition was complete, the reaction was performed for 20 min under the same condition. Iodomethane (1.70 g, 12 mmol) was added dropwise and the mixture was reacted at room temperature for 30 min. Water (100 mL) was added to quench the reaction, and the mixture was extracted with ethyl acetate (100 mL×2). The organic phases were combined, washed with saturated brine (100 mL), dried over anhydrous sodium sulfate, and filtered, and the filtrate was concentrated under reduced pressure. The residue was separated by silica gel column chromatography (PE:EA (v/v) =5:1) to obtain the compound **44B** of interest (1.38 g, yield: 86%).

LC-MS (ESI): m/z =202.1 [M+H]⁺.

Steps 2 to 4: Using **44B** (0.20 g, 0.99 mmol) and 1C (0.26 g, 1 mmol) as the raw materials, the operation method of steps 1 to 3 of example 12 was referenced to obtain the trifluoroacetate salt of **compound 44** (10 mg).

LC-MS (ESI): m/z =571.2 [M+H]⁺.

¹H NMR (400 MHz, Methanol-*d*₄) δ 7.40 (d, 1H), 7.33 (d, 1H), 7.26-7.18 (m, 2H), 6.13 (d, 1H), 5.31 (q, 1H), 4.45-4.27 (m, 2H), 4.24-4.14 (m, 1H), 3.80 (s, 4H), 3.64 (s, 1H), 3.45-3.31 (m, 1H), 2.77-2.67 (m, 2H), 2.60 (s, 1H), 2.46 (s, 1H), 2.35 (s, 1H), 2.21 -2.05 (m, 2H), 2.02-1.80 (m, 3H), 1.80-1.61 (m, 2H), 1.52 (d, 1H), 1.43 (d, 3H), 1.32 (s, 3H).

### Example 45:

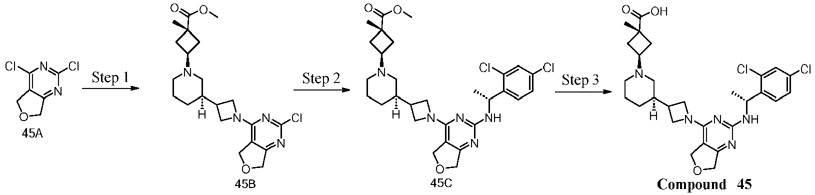

Using **45A** (0.19 g, 1.0 mmol) and 1C (0.26 g, 1.0 mmol) as the raw materials, the operation method of steps 1 to 3 of example 12 was referenced to obtain the trifluoroacetate salt of **compound 45** (100 mg).

LC-MS M/Z (ESI)=560.2 [M+H]⁺.

¹H NMR (400 MHz, Methanol-*d*₄) δ 7.50 (d, 1H), 7.43 (d, 1H), 7.34 (dd, 1H), 5.46 (s, 1H), 5.03 (s, 2H), 4.89 (t, 2H), 4.36 (s, 2H), 4.12 (s, 2H), 3.76 (t, 1H), 3.57-3.45 (m, 1H), 2.84 (t, 2H), 2.72 (t, 1H), 2.62 (d, 1H), 2.47 (t, 1H), 2.26 (s, 2H), 2.07 (d, 2H), 1.98-1.70 (m, 3H), 1.55 (d, 3H), 1.44 (s, 3H), 1.23-1.08 (m, 1H).

### Example 46:

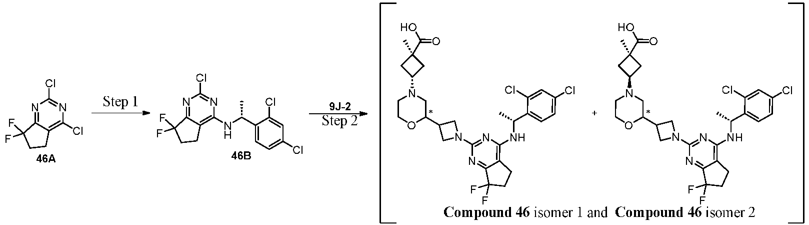

Step 1: Compound **46A** (synthesized by the method described in reference patent WO 2021129737) (1 g, 4.44 mmol), (R)-1-(2,4-dichlorophenyl)ethylamine (0.85 g, 4.44 mmol), triethylamine (0.90 g, 8.88 mmol) and dichloromethane (10 mL) were added in sequence into a 50 mL reaction bottle at room temperature, and the mixture was stirred at room temperature overnight after the addition was completed. After the reaction was completed, dichloromethane (20 mL) was added to the reaction liquid, and then the obtained system was washed with water (10 mL×1) and saturated brine (10 mL×1) in sequence. The organic layer was dried over anhydrous sodium sulfate and filtered. The filtrate was concentrated under reduced pressure. The residue was separated and purified by column chromatography (DCM/MEOH=0%-5%) to obtain **46B** (1.5 g, 89%).

LC-MS (ESI): m/z =378.0 [M+H]⁺.

Step 2: Compound **46B** (150 mg, 0.40 mmol), **9J**-2 (84 mg, 0.33 mmol), sodium bicarbonate (55 mg, 0.66 mmol) and dimethyl sulfoxide (2 mL) were added in sequence into a 25 mL reaction flask at room temperature and the obtained system was stirred at 100°C overnight after the addition was completed. After the reaction was completed, the mixture was cooled to room temperature, ethyl acetate (10 mL) was added to the reaction liquid, and then the obtained system was washed with water (5 mL×1) and saturated brine (5 mL×1) in sequence. The organic layer was dried over anhydrous sodium sulfate, and filtered, and the filtrate was concentrated under reduced pressure. The residue was separated and purified by column chromatography (EA/MEOH=0%-10%) to obtain **compound 46** isomer 1 (70 mg, 36%) and **compound 46** isomer 2 (20 mg, 10%).

**Compound 46** isomer 1 (ethyl acetate:methanol (v/v) = 9: 1, Rf = 0.32): ¹H NMR (400 MHz, CD₃OD) δ 7.36 (d, 1H), 7.24 (d, 1H), 7.18-7.17 (d, 1H), 5.55-5.46 (m, 1H), 5.01 (s, 1H), 4.06-4.00 (m, 1H), 3.92-3.81 (m, 3H), 3.71-3.57 (m, 2H), 2.91-2.67 (m, 4H), 2.66-2.43 (m, 8H), 2.01-1.85 (m, 3H), 1.53 (d, 3H), 1.41 (s, 3H).

LC-MS (ESI): m/z =596.3 [M+H]⁺.

**Compound 46** isomer 2 (ethyl acetate:methanol (v/v) = 9: 1, Rf = 0.27): ¹H NMR (400 MHz, CD₃OD) δ 7.36 (d, 1H), 7.23 (d, 1H), 7.18-7.16 (m, 1H), 5.55-5.46 (m, 1H), 4.85 (d, 1H), 4.04-3.89 (m, 4H), 3.84-3.75 (m, 2H), 3.02 (t, 2H), 2.94-2.85 (m, 2H), 2.64-2.51 (m, 8H), 2.19-2.08 (m, 3H), 1.53 (d, 3H), 1.42 (s, 3H).

LC-MS (ESI): m/z =596.3 [M+H]⁺.

### Example 47:

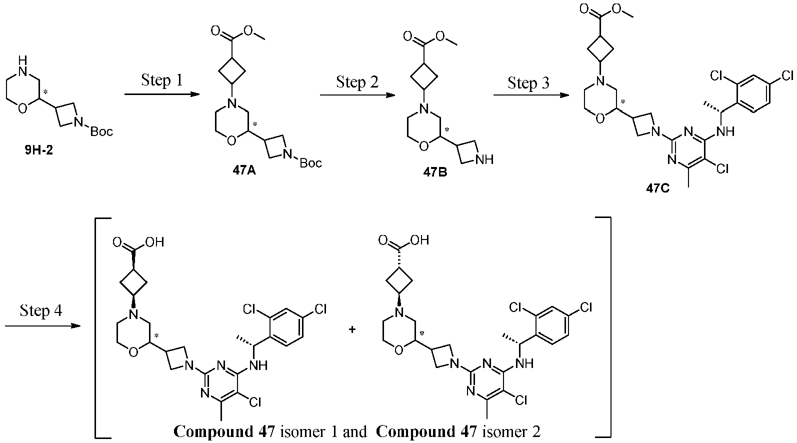

Step 1: Compound **9H**-2 (0.5 g, 2.06 mmol) and methyl 3-carbonyl-cyclobutanecarboxylate (0.32 g, 2.48 mmol) were dissolved in 1,2-dichloroethane (10 mL) in sequence, and glacial acetic acid (0.12 g, 2.06 mmol) was added. Sodium triacetoxyborohydride (1.09 g, 5.15 mmol) was added in portions and the mixture was reacted for 15 h after the addition was completed. After the reaction was completed, water (30 mL) was added to quench the reaction, and dichloromethane (50 mL×2) was used for extraction. The combined organic phase was washed with saturated brine, dried over anhydrous sodium sulfate, and filtered. The filtrate was concentrated and the residue was separated and purified by silica gel column chromatography (dichloromethane:methanol (v/v) = 10:1) to obtain the title compound **47A** (0.61 g, 82.2%).

M/Z (ESI): m/z =299.2[M+H-*^{t}*Bu]⁺.

Step 2: Compound **47A** (0.61 g, 1.72 mmol) was dissolved in dichloromethane (5 mL), trifluoroacetic acid (1 mL) was added, and the reaction was performed at room temperature for 0.5 h. After the reaction was completed, the system was directly concentrated to obtain the trifluoroacetate salt of compound **47B** (0.81 g, 99.1%).

Step 3: Compound **2F** (0.5 g, 1.4 mmol) and compound **47B** (0.81 g, 1.71 mmol) were dissolved in dimethyl sulfoxide (5 mL) in sequence, N,N-diisopropylethylamine (0.55 g, 4.3 mmol) and cesium fluoride (0.43 g, 2.8 mmol) were added, and the obtained system was heated to 100°C for reaction for 5 h. After the reaction was completed, the mixture was cooled to room temperature, and water (50 mL) was added, followed by extraction with ethyl acetate (50 mL ×3). The combined organic phases were washed with saturated brine, dried over anhydrous sodium sulfate, and filtered. The filtrate was concentrated and the residue was separated and purified by silica gel column chromatography (dichloromethane:methanol (v/v) = 10:1) to obtain the title compound **47C** (420.0 mg, 51.8%).

Step 4: Compound **47C** (420.0 mg, 0.74 mmol) was added to a mixed solvent of methanol (5 mL) and water (5 mL) at room temperature. After stirring evenly, lithium hydroxide (151.7 mg, 3.7 mmol) was added and the mixture was reacted at room temperature for 1 h. TLC/LCMS showed that the reaction was complete. 1 M dilute hydrochloric acid was added dropwise to adjust the pH=5. The crude product obtained after concentration under reduced pressure was separated and purified by preparative HPLC to obtain **compound 47** isomer 1 (24.0 mg, 5.9%) and **compound 47** isomer 2 (220.0 mg, 53.7%).

HPLC analytical method: 1. Instruments: Shimadzu LC-2020; 2. Chromatographic column: Phenomenex C18; 3. Mobile phase system: A for 0.1% TFA in H2O; B for ACN; 4. Gradient: B 10-80%; 5. Flow rate: 1.2 mL/min. retention time: **Compound 47** isomer 1: tR = retention time: 3.05 min; **Compound 47** isomer 2: tR = retention time: 3.06 min.

Preparation method: instrument: waters 2767 preparative liquid chromatographic instrument; chromatographic column: SunFire @ Prep C18 (19 mm × 250 mm). The sample was dissolved in methanol and filtered through a 0.22 µm filter head to prepare a sample liquid. Preparative chromatography conditions: composition of mobile phases A and B: mobile phase A: acetonitrile, mobile phase B: water (containing 0.1% ammonia), gradient elution, mobile phase A: 0% to 50%, flow rate: 18 mL/min. Elution time: 30 min.

**Compound 47** isomer 1: ¹H NMR (400 MHz, CDCl₃) δ 7.35 (d, 1H), 7.22 (d, 1H), 7.18-7.16 (m, 1H), 5.54 (d, 1H), 5.46-5.41 (m, 1H), 4.01-3.96 (m, 1H), 3.91-3.57 (m, 6H), 3.04-2.98(m, 2H), 2.74-2.68(m, 2H), 2.59-2.54 (m, 1H), 2.40-2.22 (m, 7H), 1.96-1.94 (m, 1H), 1.60-1.54 (m, 1H), 1.51 (d, 3H).

M/Z (ESI): m/z =554.2[M+H]⁺.

**Compound 47** isomer 2: ¹H NMR (400 MHz, CDCl₃) δ 7.34 (d, 1H), 7.23 (d, 1H), 7.18-7.16 (m, 1H), 5.50 (d, 1H), 5.45-5.39 (m, 1H), 3.96-3.75 (m, 7H), 3.05-2.83 (m, 4H), 2.61-2.48 (m, 5H), 2.29 (s, 3H), 2.12-2.07 (m, 1H), 1.73-1.68 (m, 1H), 1.50 (d, 3H).

M/Z (ESI): m/z =554.2[M+H]⁺.

### Example 48:

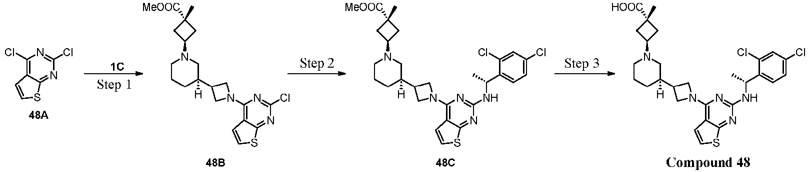

Using **48A** (0.2 g, 0.98 mmol) and 1C (0.26 g, 0.98 mmol) as the raw materials, the operation method of steps 1 to 3 of example 12 was referenced to obtain **compound 48** (100 mg).

¹H NMR (400 MHz, CD₃OD) 7.44-7.40 (m, 2H), 7.24-7.21 (m, 1H), 7.10-7.09 (m, 1H), 6.87-6.86 (m, 1H), 5.48 (s, 1H), 5.41-5.36 (m, 1H), 4.31-4.23 (m, 2H), 4.05-4.01 (m, 1H), 3.15-3.10 (m, 3H), 3.03-3.00 (m, 1H), 2.69-2.64 (m, 2H), 2.55-2.51 (m, 2H), 1.89-1.80 (m, 7H), 1.65-1.62 (m, 1H), 1.46-1.44 (d, 3H), 1.35 (s, 3H), 1.02-0.99 (m, 1H).

LC-MS (ESI): m/z = 574.2 [M+H]⁺.

### Example 49:

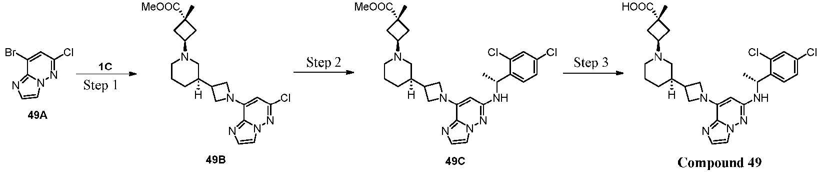

Using **49A** (0.4 g, 1.73 mmol) and 1C (0.46 g, 1.73 mmol) as the raw materials, the operation method of steps 1 to 3 of example 12 was referenced to obtain **compound 49** (10 mg).

¹H NMR (400 MHz, CD₃OD) 7.46-7.42 (m, 3H), 7.26-7.22 (m, 2H), 5.35 (s, 1H), 5.26-5.21 (m, 1H), 4.38-4.30 (m, 2H), 4.05-3.98 (m, 2H), 3.29-3.14 (m, 3H), 2.76-2.72 (m, 2H), 2.61-2.59 (m, 1H), 2.45-2.39 (m, 2H), 2.23-2.16 (m, 2H), 2.00-1.92 (m, 5H), 1.76-1.69 (m, 1H), 1.48-1.47 (m, 3H), 1.39 (s, 3H), 1.17-1.11 (m, 1H).

LC-MS (ESI): m/z = 557.2 [M+H]⁺.

### Example 50:

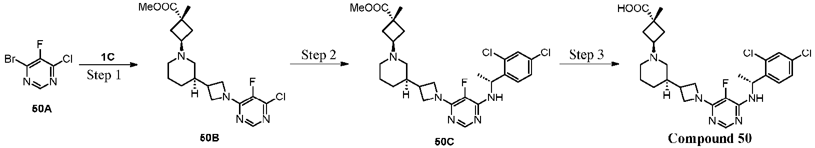

Using **50A** (0.2 g, 1.21 mmol) and 1C (0.33 g, 1.21 mmol) as the raw materials, the operation method of steps 1 to 3 of example 12 was referenced to obtain **compound 50** (100 mg).

¹H NMR (400 MHz, CD₃OD) 7.71-7.70 (m, 1H), 7.41-7.38 (m, 2H), 7.26-7.24 (m, 1H), 5.52-5.46 (m, 1H), 4.25-4.21 (m, 2H), 3.95-3.92 (m, 2H), 3.46-3.39 (m, 2H), 3.21-3.18 (m, 2H), 2.75-2.70 (m, 2H), 2.57-2.47 (m, 2H), 2.28-2.22 (m, 1H), 2.01-1.93 (m, 6H), 1.71-1.68 (m, 1H), 1.50-1.48 (d, 3H), 1.38(s, 3H), 1.16-1.06 (m, 1H).

LC-MS (ESI): m/z = 536.2 [M+H]⁺.

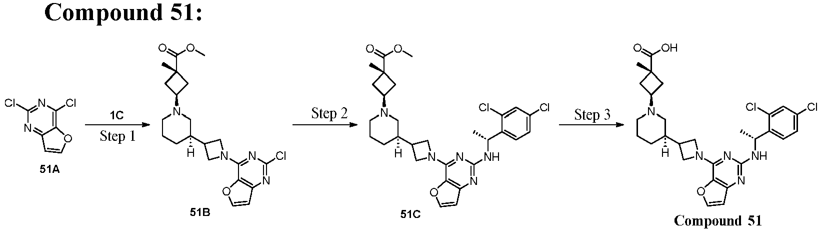

Using compound 1C (300.0 mg, 0.89 mmol) and compound **51A** (168.0 mg, 0.89 mmol) as the raw materials, the operation method of steps 1 to 3 of example 12 was referenced to obtain the title **compound 51** (25 mg).

¹H NMR (400 MHz, Methanol-*d*₄) δ 7.92 (d, 1H), 7.44-7.30 (m, 2H), 7.22 (m, 1H), 6.70 (d, 1H), 5.36 (m, 1H), 4.57-4.09 (m, 3H), 3.66-3.36 (m, 2H), 2.85-1.63 (m, 13H), 1.44 (d, 3H), 1.33 (s, 3H), 1.08 (s, 1H).

LCMS m/z =558.2 [M+H]⁺.

### Example 52:

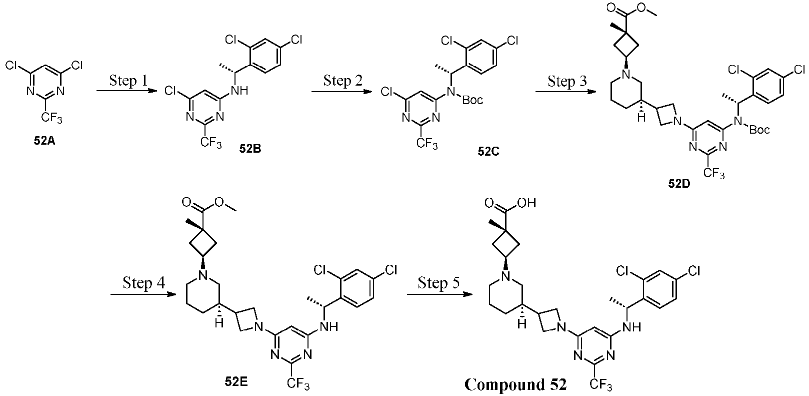

Step 1: Compound **52A** (0.5 g, 2.31 mmol) was dissolved in acetonitrile (15 mL), and (K)-1-(2,4-dichlorophenyl)ethan-1-amine (0.53 g, 2.78 mmol) and triethylamine (0.70 g, 6.93 mmol) were added, the temperature was raised to 70°C and the reaction was performed for 16 h. After the reaction was completed, the mixture was cooled to room temperature and concentrated. The concentrate was dissolved in dichloromethane (30 mL). The organic phase was washed with water (20 mL×3) and brine (20 mL×3) in sequence, dried over anhydrous sodium sulfate, filtered and concentrated. The crude product was separated and purified by silica gel column chromatography (petroleum ether:ethyl acetate (v/v) = 3:1-2:1) to obtain the title compound **52B** (0.51 g, 61%).

LC-MS (ESI): m/z = 370.2 [M+H]⁺.

Step 2: Compound **52B** (0.51 g, 1.38 mmol) was dissolved in tetrahydrofuran (10 mL), di-tert-butyl dicarbonate (0.45 g, 2.07 mmol) and DMAP (16 mg, 0.14 mmol) were added, and the obtained system was reacted under reflux for 12 h. After the reaction was completed, the mixture was cooled to room temperature and concentrated. The residue was dissolved in dichloromethane (30 mL). The organic phase was washed with water (20 mL×3) and brine (20 mL×3) in sequence, dried over anhydrous sodium sulfate, filtered and concentrated. The crude product was separated and purified by silica gel column chromatography (petroleum ether:ethyl acetate (v/v) = 3:1-2:1) to obtain the title compound **52C** (0.45 g, 70%).

LC-MS (ESI): m/z = 470.2 [M+H]⁺.

Step 3: Compound **52C** (0.20 g, 0.43 mmol) was dissolved in dimethyl sulfoxide (5 mL), compound **1C** (0.16 g, 0.47 mmol) and triethylamine (0.13 g, 1.29 mmol) were added, the temperature was raised to 150°C under microwave conditions and the reaction was performed for 1 h. After the reaction was completed, the reaction liquid was cooled to room temperature and dissolved in ethyl acetate (30 mL). The organic phase was washed with water (20 mL×3) and brine (20 mL×3) in sequence, dried over anhydrous sodium sulfate, filtered and concentrated. The crude product was separated and purified by silica gel column chromatography (petroleum ether:ethyl acetate (v/v) = 3:1-2:1) to obtain the title compound **52D** (0.19 g, 65%).

LC-MS (ESI): m/z = 700.2 [M+H]⁺.

Step 4: **52D** (0.19 g, 0.28 mmol) was dissolved in dichloromethane (8 mL), hydrogen chloride dioxane solution (8 mL) was added, and the reaction was performed at room temperature for 1 h after addition was completed. Concentration gave the hydrochloride salt of the title compound **52E** (0.15 g, 90%). The obtained compound could be directly used in the next reaction without further purification.

LC-MS (ESI): m/z =600.2 [M+H]⁺.

Step 5: Compound **52E** (150 mg, 0.25 mmol) was dissolved in tetrahydrofuran (3 mL) and water (3 mL), lithium hydroxide hydrate (53.0 mg, 1.25 mmol) was added, and the obtained system was stirred evenly and reacted at room temperature for 12 h. After the reaction was completed, the mixture was concentrated under reduced pressure and the residue was separated and purified by high performance preparative liquid chromatography to obtain the title **compound 52** (0.09 g, 65%).

Preparation method: 1. Instruments: waters 2767 preparative liquid chromatographic instrument; chromatographic column: SunFire @ Prep C18 (19 mm × 250 mm). 2. The sample was filtered with a 0.45 µm filter head to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min.

¹H NMR (400 MHz, Methanol-*d*₄) δ 7.44 (d, 1H), 7.39 (d, 1H), 7.31-7.25 (m, 1H), 5.21-4.94 (m, 2H), 4.20-3.91 (m, 2H), 3.82-3.65 (m, 2H), 3.56-3.37 (m, 1H), 3.20 (d, 1H), 2.88-2.67 (m, 2H), 2.59-2.42 (m, 2H), 2.25-2.20 (m, 1H), 2.10-1.79 (m, 7H), 1.78-1.61 (m, 1H), 1.47 (d, 3H), 1.37 (s, 3H).

LC-MS (ESI): m/z = 586.2 [M+H]⁺.

### Example 53:

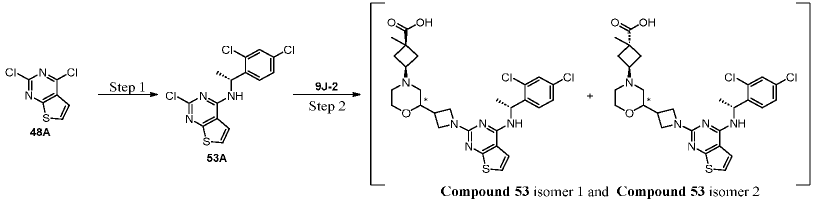

Step 1: Compound **48A** (1.00 g, 4.88 mmol), (*R*)-1-(2,4-dichlorophenyl)ethan-1-amine (1.39 g, 7.32 mmol) and triethylamine (1.48 g, 14.6 mmol) were dissolved in dry acetonitrile (60 mL) and the reaction was performed at room temperature overnight. After the disappearance of the raw materials as detected by TLC, saturated aqueous ammonium chloride solution (50 mL) was added to quench the reaction, and the mixture was extracted with ethyl acetate (20 mL × 5). The organic phases were combined and dried over anhydrous Na₂SO₄, filtered and concentrated. The residue was purified by silica gel column chromatography (petroleum ether:ethyl acetate (v/v) = 80:20) to obtain compound **53A** (920 mg, 52.6%).

LC-MS (ESI): m/z= 358.1 [M+H]⁺.

Step 2: Compounds **9J-2** (300 mg, 0.622 mmol) and **53A** (245 mg, 0.685 mmol) were dissolved in dry dimethyl sulfoxide (6 mL), and triethylamine (378 mg, 3.73 mmol) and cesium fluoride (189 mg, 1.24 mmol) were added, the temperature was raised to 100°C and the reaction was performed for about 4 h. After the disappearance of the raw materials as detected by TLC, the obtained system was cooled to room temperature and then concentrated under reduced pressure. The residue was purified by silica gel column chromatography (ethyl acetate:methanol (v/v)=100:0~75:25) to obtain **compound 53** isomer **1** (54 mg, 15.1%) and **compound 53** isomer **2** (23 mg, 6.42%).

**Compound 53** isomer **1** (ethyl acetate:methanol (v/v)=9:1, Rf = 0.45), ¹H NMR (400 MHz, Methanol-*d*₄) δ 7.46-7.32 (m, 3H), 7.22 (d, 1H), 6.93 (d, 1H), 5.60-5.59 (m, 1H), 4.01-3.80 (m, 4H), 3.78-3.52 (m, 3H), 2.95-2.94 (m, 1H), 2.80 (d, 2H), 2.70-2.55 (m, 3H), 2.09-2.08 (m, 1H), 1. 93-1.82 (m, 2H), 1.71 (t, 1H), 1.53 (d, 3H), 1.37 (s, 3H).

LC-MS (ESI): m/z= 576.2 [M+H]⁺.

**Compound 53** isomer **2** (ethyl acetate:methanol (v/v)=9:1, Rf = 0.42), ¹H NMR (400 MHz, Methanol-*d*₄) *δ* 7.47-7.34 (m, 3H), 7.23 (d, 1H), 6.94 (d, 1H), 5.60-5.59 (m, 1H), 4.02 -3.92 (m, 2H), 3.86 (d, 2H), 3.80-3.59 (m, 3H), 3.08-3.07 (m, 1H), 2.94 (d, 2H), 2.67 (q, 1H), 2.50-2.37 (m, 2H), 2.25-2.24 (m, 1H), 2.17-2.06 (m, 2H), 1.89 (t, 1H), 1.54 (d, 3H), 1.40 (s, 3H).

LC-MS (ESI): m/z= 576.2 [M+H]⁺.

### Example 54:

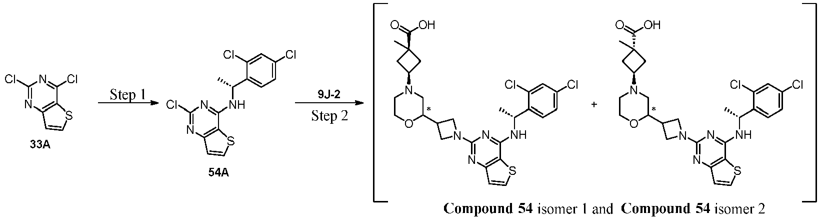

Using compound **33A** (500 mg, 2.44 mmol) and (*R*)-1-(2,4-dichlorophenyl)ethan-1-amine (695 mg, 3.66 mmol) as the raw materials, the operation method of steps 1 to 2 of example 53 was referenced to obtain **compound 54** isomer 1 (33 mg) and **compound 54** isomer 2 (14 mg).

**Compound 54** isomer 1 (ethyl acetate:methanol (v/v)=9:1, Rf = 0.55), ¹H NMR (400 MHz, Methanol-*d*₄) *δ* 7.82 (d, 1H), 7.43 (d, 1H), 7.38 (d, 1H), 7.24-7.23 (m, 1H), 7.07 (d, 1H), 5.64 (q, 1H), 4.00-3.99 (m, 1H), 3.95-3.82 (m, 3H), 3.79-3.71 (m, 1H), 3.69-3.53 (m, 2H), 2.89-2.88 (p, 1H), 2.76 (d, 2H), 2.71-2.63 (m, 1H), 2.65-2.53 (m, 2H), 2.04-2.03 (td, 1H), 1.88-1.78 (m, 2H), 1.66 (t, 1H), 1.54 (d, 3H), 1.37 (s, 3H).

LC-MS (ESI): m/z= 576.2 [M+H]⁺.

**Compound 54** isomer 2 (ethyl acetate:methanol (v/v)=9:1, Rf = 0.50), ¹H NMR (400 MHz, Methanol-*d*₄) *δ* 7.81 (d, 1H), 7.43 (d, 1H), 7.39 (d, 1H), 7.24-7.23 (m, 1H), 7.07 (d, 1H), 5.64-5.63 (q, 1H), 4.04-3.84 (m, 4H), 3.84-3.59 (m, 3H), 3.06-3.05 (p, 1H), 2.94 (d, 2H), 2.76-2.64 (m, 1H), 2.42 (t, 2H), 2.29-2.20 (m, 1H), 2.15-2.04 (m, 2H), 1.88-1.87 (t, 1H), 1.54 (d, 3H), 1.40 (s, 3H).

LC-MS (ESI): m/z= 576.2 [M+H]⁺.

### Example 55:

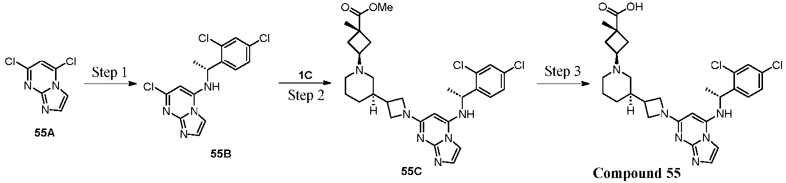

Using compound **55A** (227 mg, 1.18 mmol) and (*R*)-1-(2,4-dichlorophenyl)ethylamine (336 mg, 1.77 mmol) as the raw materials, the operation method of steps 1 to 3 of example 14 was referenced to obtain the trifluoroacetate salt of **compound 55** (75 mg).

¹H NMR (400 MHz, CD₃OD) δ 7.96 (m, 1H), 7.58 (s, 1H), 7.54-7.46 (m, 2H), 7.38 (m, 1H), 5.88 (s, 1H), 5.18-5.17 (m, 1H), 4.97 (s, 1H), 4.21 (m, 1H), 3.96 (m, 2H), 3.75 (m, 1H), 3.51 (m, 1H), 3.38 (m, 1H), 2.83 (m, 2H), 2.71 (m, 1H), 2.61 (m, 1H), 2.47 (m, 1H), 2.25 (m, 2H), 2.04 (m, 2H), 1.94 (m, 1H), 1.81 (m, 1H), 1.69 (d, 3H), 1.44 (s, 3H), 1.33 (m, 1H), 1.17 (m, 1H).

LC-MS (ESI): m/z = 557.2 [M+H]⁺.

### Example 56:

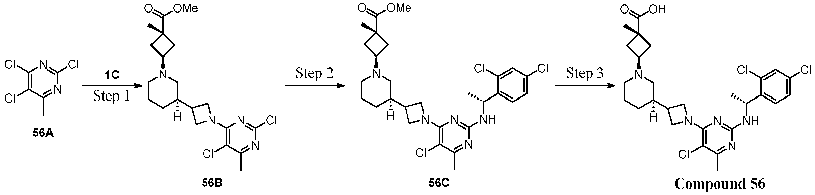

Using compound **56A** (300 mg, 1.5 mmol) and compound **1C** (565 mg, 1.5 mmol) as the raw materials, the operation method of steps 1 to 3 of example 12 was referenced to obtain the trifluoroacetate salt of **compound 56** (191 mg).

LC-MS (ESI): m/z = 566.7 [M+H]⁺.

¹H NMR (400 MHz, CD₃OD) δ 7.48 (s, 1H), 7.42 (d, 1H), 7.32 (d, 1H), 5.39 (m, 1H), 4.41-3.90 (m, 2H), 3.74 (m, 1H), 3.50 (m, 1H), 3.33 (m, 2H), 2.81 (m, 2H), 2.70 (m, 1H), 2.59-2.39 (m, 2H), 2.36 (s, 3H), 2.29-2.20 (m, 2H), 2.10-1.92 (m, 2H), 1.86-1.68 (m, 2H), 1.52 (m, 3H), 1.42 (s, 3H), 1.31 (m, 1H), 1.13 (m, 1H).

### Example 57:

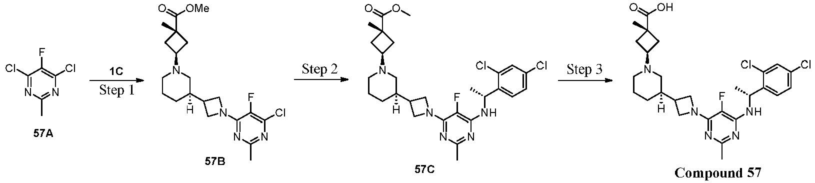

Using compound **57A** (227 mg, 1.08 mmol) and compound **1C** (407 mg, 1.08 mmol) as the raw materials, the operation method of steps 1 to 3 of example 12 was referenced to obtain the trifluoroacetate salt of **compound 57** (144 mg).

¹H NMR (400 MHz, CD₃OD) δ 7.46 (s, 1H), 7.43 (d, J = 8.4 Hz, 1H), 7.32 (d, J = 8.4 Hz, 1H), 5.65-5.64 (m, 1H), 4.46 (m, 2H), 4.23 (m, 2H), 3.75 (m, 1H), 3.51 (m, 1H), 3.38 (m, 1H), 2.82 (m, 2H), 2.72 (m, 2H), 2.47 (m, 1H), 2.37 (s, 3H), 2.30 (m, 2H), 2.18 (m, 1H), 2.05 (m, 1H), 1.94 (m, 1H), 1.83 (m, 1H), 1.56 (d, 3H), 1.43 (s, 3H), 1.18 (m, 1H).

LC-MS (ESI): m/z = 550.2 [M+H]⁺.

### Example 58:

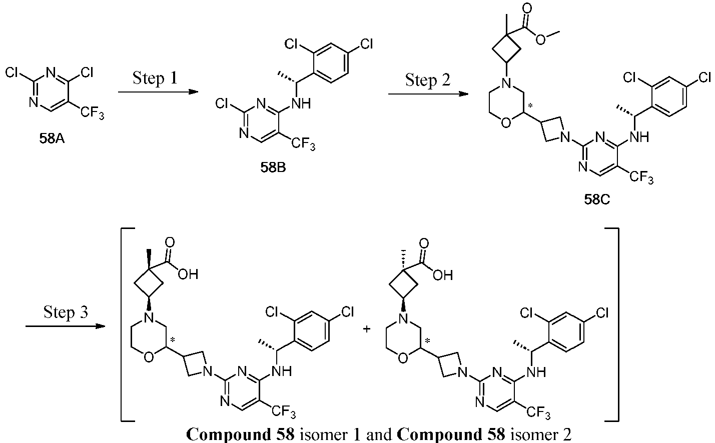

Step 1: Compound **58A** (456 mg, 2.10 mmol) was dissolved in acetonitrile (10 mL), triethylamine (638 mg, 6.30 mmol) and (*R*)-1-(2,4-dichlorophenyl)ethylamine (400 mg, 2.10 mmol) was added in sequence, and the obtained system was stirred at room temperature for 5 h. After the reaction was completed, the mixture was concentrated under reduced pressure and the residue was separated and purified by silica gel column chromatography (petroleum ether:ethyl acetate (v/v) = 10:1) to obtain the compound **58B** of interest (510 mg, 66%).

LC-MS (ESI): m/z = 371.5 [M+H]⁺.

Step 2: Compound **58B** (200 mg, 0.54 mmol) was dissolved in acetonitrile (3 mL), and **29B** (140 mg, 0.54 mmol), N,N-diisopropylethylamine (210 mg, 1.62 mmol), and cesium fluoride (82 mg, 0.54 mmol) were added in sequence, and the mixture was stirred at 70°C overnight. After the reaction was completed, the mixture was cooled to room temperature and concentrated under reduced pressure. The residue was separated and purified by silica gel column chromatography (dichloromethane:methanol (v/v) = 10:1) to obtain the compound **58C** of interest (143 mg, 44%).

LC-MS (ESI): m/z = 603.2 [M+H]⁺.

Step 3: Compound **58C** (143 mg, 0.24 mmol) was dissolved in tetrahydrofuran (1 mL), methanol (1 mL) and water (2 mL) were added, and after stirring evenly, lithium hydroxide monohydrate (29 mg, 1.2 mmol) was added and the obtained system was reacted at room temperature for 2 h. After the reaction was completed, the obtained system was concentrated and the residue was separated by silica gel column chromatography (ethyl acetate:methanol (v/v)=5:1) to obtain **compound 58,** isomer 1 (Rf=0.7, (ethyl acetate:methanol (v/v)=5:1)) (27 mg, 19%) and isomer 2 (Rf=0.6, (EA:MeOH (v/v)=5:1)) (20 mg, 14%).

Compound 58, isomer 1: LC-MS (ESI): m/z =588.6 [M+H]⁺.

¹H NMR (400 MHz, DMSO-*d6*) δ 8.09 (d, 1H), 8.06 (s, 1H), 7.52 (s, 1H), 7.45 (d, 1H), 7.38 (d, 1H), 5.27 (s, 1H), 3.98-3.77 (m, 5H), 3.64 (s, 2H), 3.47 (s, 2H), 2.72-2.63 (m, 2H), 2.59 (d, 2H), 2.43 (d, 1H), 1.76 (d, 3H), 1.36 (d, 3H), 1.31 (s, 3H).

Compound 58, isomer 2: LC-MS (ESI): m/z =588.6 [M+H]⁺.

¹H NMR (400 MHz, DMSO-*d6*) δ 10.22 (s, 1H), 8.37 (d, 1H), 8.14 (s, 1H), 7.59 (s, 1H), 7.47 (d, 1H), 7.41 (d, 1H), 5.33 (d, 1H), 4.21-3.65 (m, 10H), 2.92-2.73 (m, 2H), 2.70-2.54 (m, 3H), 2.27-2.08 (m, 2H), 1.38 (d, 3H), 1.34 (d, 3H).

### Example 59:

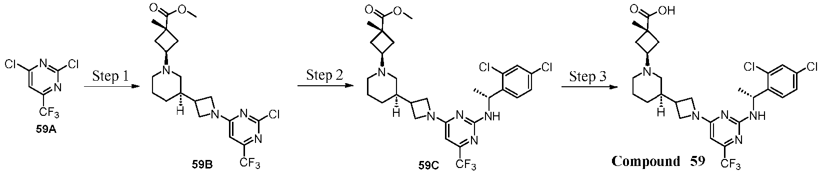

Using 2,4-dichloro-6-(trifluoromethyl)pyrimidine **(59A)** (0.22 g, 1.00 mmol) and compound **1C** (0.26 g, 1.00 mmol) as the raw materials, the operation method of steps 1 to 3 of example 12 was referenced to obtain the trifluoroacetate salt of compound **59** (0.19 g).

¹H NMR (400 MHz, Methanol-*d*₄) δ 7.45-7.38 (m, 2H), 7.25-7.24 (m, 1H), 5.96 (d, 1H), 5.39 (d, 1H), 4.22-3.96 (m, 2H), 3.91-3.66 (m, 3H), 3.53-3.45 (m, 1H), 2.82 (t, 2H), 2.70 (t, 1H), 2.63-2.36 (m, 2H), 2.20 (s, 2H), 2.10-1.67 (m, 4H), 1.54-1.36 (m, 7H), 1.23-1.08 (m, 1H).

LC-MS (ESI): m/z =586.3 [M+H]⁺.

### Example 60:

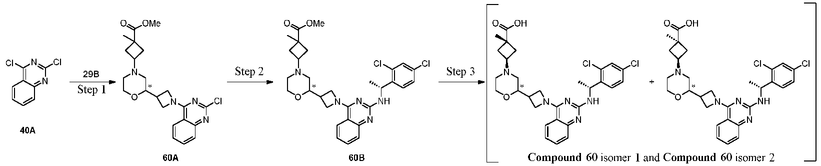

Using compound **40A** (358 mg, 1.8 mmol) and **29B** (409 mg, 1.2 mmol) as the raw materials, the operation method of steps 1 to 3 of example 12 was referenced to obtain **compound 60,** isomer 1 (retention time 0.801 min, 16 mg, yield: 19%), isomer 2 (retention time 1.143 min, 8 mg, yield: 9%).

Analytical method: instrument: SHIMADZU LC-30AD sf, column: Chiralcel C2-3 50×4.6mm I.D., 3µm, mobile phase: A for CO₂, B for MeOH +ACN (0.05% DEA), gradient: B 50%, flow rate: 3mL/min, back pressure: 100 bar, column temperature: 35°C, wavelength: 220 nm. Compound **60,** isomer 1: LC-MS (ESI): m/z = 570.2 [M+H]⁺.

¹H NMR (400 MHz, CD₃OD) δ 7.80 (d, J = 8.4 Hz, 1H), 7.65-7.64 (m, 1H), 7.48 (s, 1H), 7.46-7.45 (m, 1H), 7.38 (d, J = 8.4 Hz, 1H), 7.30-7.29 (m, 1H), 7.24-7.23 (m, 1H), 5.51 (m, 1H), 5.36 (m, 1H), 4.88-4.87 (m, 2H), 4.57-4.56 (m, 2H), 3.91-3.90 (m, 1H), 3.67-3.66 (m, 2H), 2.85-2.84 (m, 1H), 2.76-2.75 (m, 2H), 2.63-2.62 (m, 2H), 2.21-2.20 (m, 1H), 2.04-2.03 (m, 1H), 1.80-1.79 (m, 2H), 1.54-1.53 (m, 3H), 1.38-1.37 (s, 3H), 0.91-0.90 (m, 1H).

Compound **60,** isomer 2: LC-MS (ESI): m/z = 570.1 [M+H]⁺.

¹H NMR (400 MHz, CD₃OD) δ 7.82 (d, J = 8.4 Hz, 1H), 7.69-7.68 (m, 1H), 7.49 (m, 1H), 7.47 (d, J = 8.4 Hz, 1H), 7.40-7.39 (m, 1H), 7.32-7.31 (m, 1H), 7.29-7.28 (m, 1H), 5.52-5.51 (m, 1H), 5.37-5.36 (m, 1H), 4.57-4.55 (m, 4H), 3.92-3.91 (m, 1H), 3.67-3.65 (m, 2H), 3.52-3.51 (m, 1H), 2.87-2.86 (m, 2H), 2.80-2.79 (m, 2H), 2.31-2.30 (m, 1H), 2.26-2.25 (m, 1H), 2.06-2.05 (m, 2H), 1.55-1.51 (m, 3H), 1.43-1.40 (s, 3H), 0.93-0.91 (m, 1H).

### Example 61:

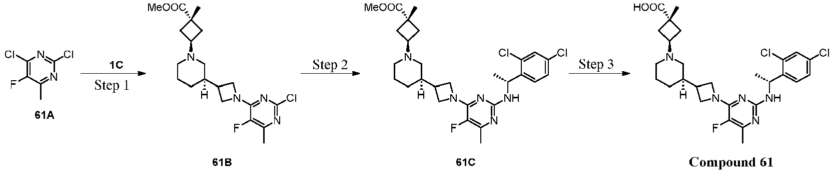

Using **61A** (0.2 g, 1.11 mmol) and **1C** (0.30 g, 1.11 mmol) as the raw materials, the operation method of steps 1 to 3 of example 12 was referenced to obtain **compound 61** (100 mg).

¹H NMR (400 MHz, CD₃OD) 7.42-7.40 (m, 2H), 7.27-7.24 (m, 1H), 5.29-5.24 (m, 1H), 4.19-4.11 (m, 2H), 3.91-3.87 (m, 1H), 3.71-3.69 (m, 1H), 3.46-3.42 (m, 2H), 3.17-3.14 (m, 2H), 2.77-2.72 (m, 2H), 2.54-2.48 (m, 2H), 2.26-2.21 (m, 1H), 2.14-2.13 (m, 3H), 2.03-1.96 (m, 5H), 1.86-1.82 (m, 2H), 1.44-1.42 (d, 3H), 1.40 (s, 3H), 1.14-1.05 (m, 1H).

LC-MS (ESI): m/z = 550.2 [M+H]⁺.

### Example 62:

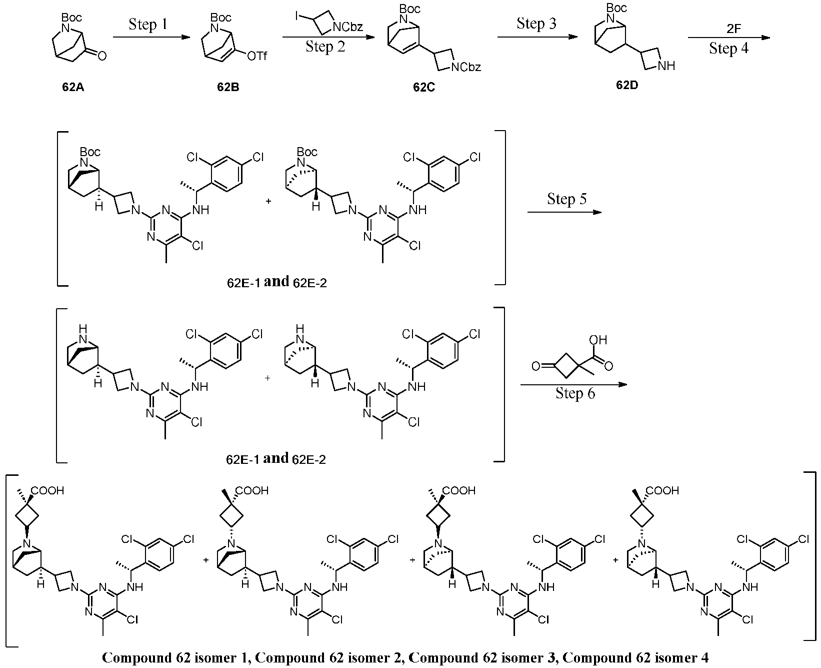

Step 1: The raw material substrate **62A** (6.3 g, 29.85 mmol) was dissolved in THF (100 mL) at room temperature. The obtained system was cooled to 0°C under nitrogen atmosphere. Sodium bis(trimethylsilyl)amide (17.9 mL, 35.82 mmol) was added dropwise. After the dropwise addition was completed, stirring was continued for 1 h. Then, a solution of N-phenylbis(trifluoromethanesulfonyl)imide (21.3 g, 59.7 mmol) in THF (100 mL) was added dropwise. After the dropwise addition was completed, the obtained system was naturally warmed to room temperature and reacted for 2 h. Water (100 mL) was added to quench the reaction, and the mixture was extracted with EA (500 mL×3). The combined organic phase was washed with water (500 mL), washed with saturated brine (500 mL), dried over anhydrous sodium sulfate, filtered, concentrated under reduced pressure, and then subjected to column chromatography (PE:EA=50:1-20:1) to obtain compound **62B** (5.5 g, yield: 61%).

LC-MS (ESI): m/z =344.1 [M+H]⁺.

Step 2: Zinc powder (3.12 g, 48.11 mmol) was added to DMA (20 mL) at room temperature. 1,2-dibromoethane (0.91 g, 4.81 mmol) and trimethylsilyl chloride (0.52 g, 4.81 mmol) were added dropwise to the reaction under nitrogen atmosphere. After the dropwise addition was completed, 1-CBZ-3-iodoacridine (4.52 g, 24.05 mmol) was added, and then stirring was continued at room temperature for 1 h. After filtration, raw material **62B** (5.5 g, 16.03 mmol), CuI (0.46 g, 2.4 mmol), Pd(dppf)₂Cl₂ (1.74 g, 2.4 mmol) were added to the filtrate in sequence, and the obtained system was heated to 85°C and reacted for 16 h. After the reaction was completed, the mixture was cooled to room temperature, water (100 mL) was added, and the mixture was extracted with ethyl acetate (200 mL×3). The combined organic phases were washed with saturated brine (200 mL), dried over anhydrous sodium sulfate, filtered, and the filtrate was concentrated under reduced pressure. The residue was separated by silica gel column chromatography (PE:EA(v/v)=1:1) to obtain the compound **62C** of interest (2.9 g, yield: 48%).

LC-MS (ESI): m/z =385.2 [M+H]⁺.

Step 3: Compound **62C** (2.9 g, 7.55 mmol) was dissolved in methanol (50 mL), and Pd/C (0.3 g, Pd content 10%) was added and the obtained system was reacted under hydrogen atmosphere for 15 h. After the reaction was completed, filtration was performed and the filtrate was concentrated to obtain the crude product of compound **62D** (1.9 g, 100%), which was directly used in the next reaction without further purification.

LC-MS (ESI): m/z =253.2 [M+H]⁺.

Step 4: **62D** (1.9 g, 7.55 mmol), **2F** (2.65 g, 7.55 mmol), triethylamine (1.54 g, 15.1 mmol), and cesium fluoride (1.15 g, 7.55 mmol) were dissolved in dimethyl sulfoxide (100 mL) in sequence at room temperature, and then the obtained system was heated to 100°C and reacted for 4 h. After the reaction was completed, the mixture was cooled to room temperature, water (100 mL) was added, and the mixture was extracted with ethyl acetate (200 mL×3). The combined organic phases were washed with saturated brine (200 mL), dried over anhydrous sodium sulfate, filtered, and the filtrate was concentrated under reduced pressure. The residue was separated by silica gel column chromatography (DCM:MeOH(v/v)=10:1) to obtain 0.97 g of the racemate. The racemate was separated by chiral preparative HPLC to obtain two isomers, compound **62E-1** (0.25 g, retention time 1.63 min, 5.8%) and compound **62E-2** (0.28 g, retention time 1.77 min, 6.6%).

HPLC analytical method: 1. Instruments: SHIMADZU LC-30AD; 2. Chromatographic column: Chiralpak IC-3 50×4.6mm I.D.; 3. Mobile phase system: A for CO2 and B for IPA (0.05%DEA); 4. Gradient: B 30%; 5. Flow rate: 3mL/min.

Preparative chromatography separation conditions: 1. Instruments: Waters 150 SFC; 2. Chromatographic column: Chiralpak IC -Column (250×30mm, I.D 30mm, 10um particle size); 3. Mobile phase system: A for CO2 and B for IPA; 4. Gradient: B 30%; 5. Flow rate: 110 mL/min.

LC-MS (ESI): m/z =566.3 [M+H]⁺.

Step 5: Compound **62E-1** (0.25 g, 0.44 mmol) was dissolved in dichloromethane (10 mL), trifluoroacetic acid (3 mL) was added, and the reaction was performed at room temperature for 30 min. After the reaction was completed, the system was directly concentrated to obtain the trifluoroacetate salt of compound **62F-1** (0.2 g, 98%).

Referring to the above operation, compound **62E-2** (0.86 g, 2.4 mmol) was used as the raw material to obtain the trifluoroacetate salt of the title compound **62F-2** (0.22 g, 98%).

LC-MS (ESI): m/z =466.3 [M+H]⁺.

Step 6: Compound **62F-1** (0.2 g, 0.43 mmol) and 3-carbonyl-1-methyl-cyclobutanecarboxylic acid (55 mg, 0.43 mmol) were dissolved in 1,2-dichloroethane (15 mL) in sequence, and glacial acetic acid (26 mg, 0.43 mmol) was added. Sodium triacetoxyborohydride (0.18 g, 0.86 mmol) was added in portions, and the reaction was carried out for 15 h after the addition was completed. After the reaction was completed, water (30 mL) was added to quench the reaction, and dichloromethane (50 mL×2) was used for extraction. The combined organic phases were washed with saturated brine, dried over anhydrous sodium sulfate, and filtered. The filtrate was concentrated and the residue was separated and purified by silica gel column chromatography (dichloromethane:methanol (v/v) = 10:1) to obtain the title **compound 62** isomer 1 (Rf = 0.4 (dichloromethane:methanol = 10:1), 30 mg, 12%) and the title **compound 62** isomer 2 (Rf = 0.6 (dichloromethane:methanol = 10:1), 20 mg, 8%).

**Compound 62** isomer 1: ¹H NMR (400 MHz, CD₃CN) δ 7.51-7.50 (m, 1H), 7.41-7.39 (m, 1H), 7.34-7.31 (m, 1H), 6.10-6.08 (m, 1H), 5.52-5.45 (m, 1H), 4.17-4.05 (m, 1H), 3.92-3.87 (m, 1H), 3.58-3.55 (m, 1H), 3.24-3.14 (m, 5H), 2.73-2.68 (m, 6H), 2.24 (s, 3H), 1.93-1.85 (m, 5H), 1.73-1.67 (m, 1H), 1.54-1.52 (d, 3H), 1.33 (s, 3H).

M/Z (ESI): m/z =578.2[M+H]⁺.

**Compound 62** isomer 2: ¹H NMR (400 MHz, CD₃CN) δ 7.52-7.51 (m, 1H), 7.41-7.39 (m, 1H), 7.34-7.31 (m, 1H), 6.10-6.08 (m, 1H), 5.49-5.43 (m, 1H), 4.46-4.37 (m, 1H), 3.99-3.89 (m, 2H), 3.57-3.54 (m, 1H), 3.25-3.11 (m, 5H), 2.78-2.67 (m, 6H), 2.24 (s, 3H), 1.89-1.86 (m, 5H), 1.54-1.52 (d, 3H), 1.33 (s, 3H).

M/Z (ESI): m/z =578.2[M+H]⁺.

Referring to the above operation, compound **62F-2** (0.22 g, 0.47 mmol) was used as the raw material to obtain the title **compound 62** isomer 3 (Rf=0.4 (dichloromethane:methanol=10:1), 30 mg, 11%) and the title **compound 62** isomer 4 (Rf=0.6 (dichloromethane:methanol=10:1), 20 mg, 7.5%).

**Compound 62** isomer 3: ¹H NMR (400 MHz, CD₃CN) δ 7.51-7.50 (m, 1H), 7.42-7.39 (m, 1H), 7.33-7.30 (m, 1H), 6.13-6.11 (m, 1H), 5.52-5.45 (m, 1H), 4.17-4.11 (m, 1H), 4.02-3.88 (m, 2H), 3.57-3.49 (m, 1H), 3.29-3.24 (m, 4H), 2.82-2.60 (m, 6H), 2.24 (s, 3H), 1.93-1.75 (m, 5H), 1.61-1.60 (m, 1H), 1.54-1.52 (d, 3H), 1.33 (s, 3H).

M/Z (ESI): m/z =578.2[M+H]⁺.

**Compound 62** isomer 4: ¹H NMR (400 MHz, CD₃CN) δ 7.52-7.51 (m, 1H), 7.41-7.39 (m, 1H), 7.34-7.31 (m, 1H), 6.10-6.08 (m, 1H), 5.49-5.43 (m, 1H), 4.41-4.38 (m, 1H), 3.99-3.89 (m, 2H), 3.57-3.54 (m, 1H), 3.25-3.11 (m, 5H), 2.78-2.67 (m, 6H), 2.24 (s, 3H), 1.89-1.86 (m, 5H), 1.54-1.52 (d, 3H), 1.33 (s, 3H).

M/Z (ESI): m/z =578.2[M+H]⁺.

### Example 63:

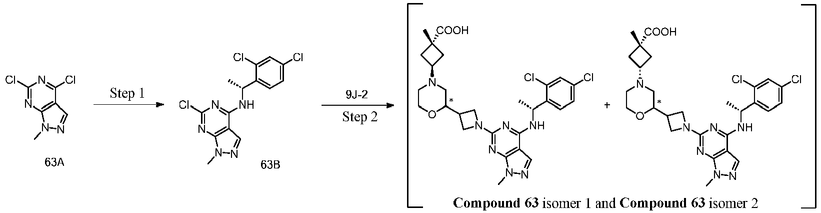

Using compound **63A** (1.0 g, 4.93 mmol) and (*R*)-1-(2,4-dichlorophenyl)ethan-1-amine (936 mg, 4.93 mmol) as the raw materials, the operation method of steps 1 and 2 of example 37 was referenced to obtain **compound 63** isomer 1 (70 mg, Rf=0.5 (ethyl acetate:methanol (v/v)=8:1)) and **compound 63** isomer 2 (30 mg, Rf=0.3 (ethyl acetate:methanol (v/v)=8:1)).

**Compound 63,** isomer 1: LC-MS(ESI): m/z =574.2[M+H]⁺.

¹H NMR (400 MHz, DMSO-*d6*) δ 12.16 (s, 1H), 8.37 (d, 1H), 7.92 (s, 1H), 7.59-7.49 (m, 1H), 7.45-7.30 (m, 2H), 5.52 (s, 1H), 3.90-3.70 (m, 4H), 3.67 (s, 3H), 3.52-3.33 (m, 3H), 2.71-2.53 (m, 4H), 2.47-2.36 (m, 2H), 1.84-1.65 (m, 3H), 1.51 - 1.37 (m, 4H), 1.28 (s, 3H).

**Compound 63,** isomer 2: LC-MS(ESI): m/z =574.2[M+H]⁺.

¹H NMR (400 MHz, DMSO-d6) δ 8.38 (d, 1H), 7.92 (s, 1H), 7.56-7.53 (m, 1H), 7.46-7.32 (m, 2H), 5.59-5.47 (m, 1H), 3.89-3.70 (m, 4H), 3.66 (s, 3H), 3.53-3.39 (m, 3H), 2.80-2.69 (m, 1H), 2.68-2.53 (m, 3H), 2.21-2.09 (m, 2H), 1.95-1.84 (m, 2H), 1.84 -1.74 (m, 1H), 1.52-1.40 (m, 4H), 1.32 (s, 3H).

### Example 64:

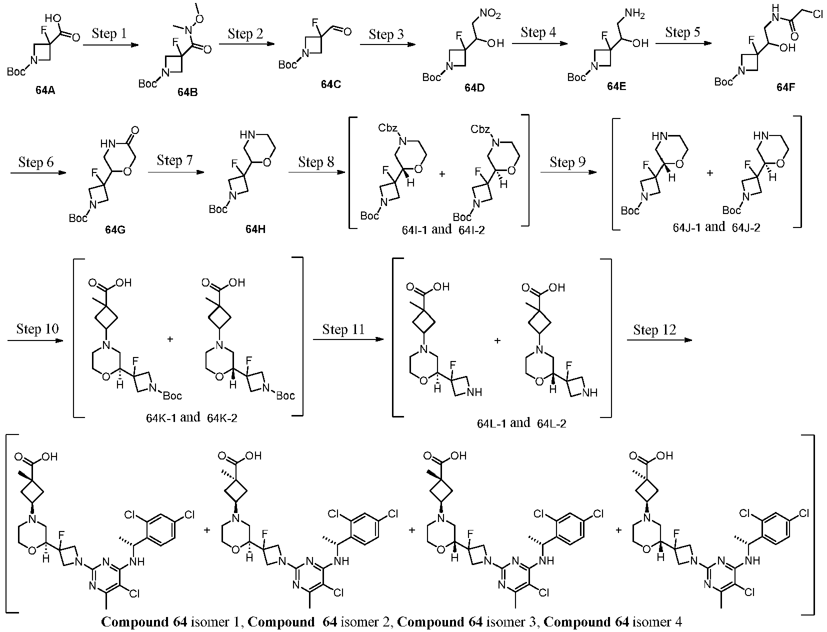

Step 1: Compound **64A** (10.00 g, 45.66 mmol) was dissolved in acetonitrile (50 mL), and dimethylhydroxylamine hydrochloride (5.34 g, 54.79 mmol), N-methylmorpholine (18.45 g, 182.64 mmol), and 1-ethyl-(3-dimethylaminopropyl)carbodiimide hydrochloride (13.13 g, 68.49 mmol) were added in sequence. The mixture was reacted at room temperature for 3 h after the addition was completed. After the reaction was completed, water (50 mL) was added to quench the reaction, and ethyl acetate (100 mL×2) was used for extraction. The combined organic phases were washed with saturated brine, dried over anhydrous sodium sulfate, and filtered, and the filtrate was concentrated to obtain the title compound **64B** (11.72 g, 98.0%), which was directly used in the next step without further purification.

Step 2: Compound **64B** (11.2 g, 42.7 mmol) was dissolved in anhydrous tetrahydrofuran (110 mL) under a nitrogen atmosphere, the obtained system was cooled to -78°C, and diisobutylaluminum hydride (64 mL, 64.05 mmol, 1.0 mol/L solution in toluene) was slowly added dropwise, after the dropwise addition was completed, the reaction was continued at -78°C for 1 h. After the reaction was completed, 10% aqueous sodium potassium tartrate solution (50 mL) was added dropwise to quench the reaction, and ethyl acetate (100 mL×2) was used for extraction. The combined organic phase was washed with saturated brine, dried over anhydrous sodium sulfate, and filtered, and the filtrate was concentrated under reduced pressure to obtain compound **64C** (8.6 g, 99.1%), which was used directly in the next step without further purification.

Step 3: Compound **64C** (8.0 g, 39.37 mmol) was dissolved in methanol (80 mL), triethylamine (7.97 g, 78.74 mmol) and nitromethane (9.61 g, 157.48 mmol) were added in sequence, after the addition was completed, the obtained system was stirred at room temperature for 17 h. After the reaction was completed, the obtained system was concentrated and the residue was separated and purified by silica gel column chromatography (petroleum ether:ethyl acetate (v/v) = 4:1) to obtain the title compound **64D** (9.3 g, 89.39%).

Step 4: Compound **64D** (9.3 g, 35.19 mmol) was dissolved in methanol (100 mL), raney nickel (0.41 g, 7.04 mmol) was added, and the reaction was performed under a hydrogen atmosphere for 15 h. After the reaction was completed, the obtained system was filtered and the filtrate was concentrated to obtain a crude product of compound **64E** (7.6 g, 92.2%), which was used directly in the next step without further purification.

M/Z (ESI): m/z =187.1[M+H-*^{t}*Bu]⁺.

Step 5: Compounds **64E** (7.6 g, 32.44 mmol) was dissolved in a mixed solvent of tetrahydrofuran (70 mL) and water (40 mL), and sodium bicarbonate (8.18 g, 97.32 mmol) was added. The temperature was controlled at 5-10°C, and chloroacetyl chloride (7.33 g, 64.88 mmol) was added dropwise. After the dropwise addition was completed, the temperature was raised to room temperature and the reaction was carried out for 1 h. After the reaction was completed, water (50 mL) was added, and ethyl acetate (50 mL×2) was used for extraction. The combined organic phase was washed with saturated brine, dried over anhydrous sodium sulfate, filtered, and concentrated. The residue was separated and purified by silica gel column chromatography (petroleum ether:ethyl acetate (v/v) = 1:3) to obtain the title compound **64F** (9.2 g, 91.2%).

Step 6: Potassium tert-butoxide (6.65 g, 59.22 mmol) was dissolved in tert-butanol (170 mL), and compound **64F** (9.2 g, 29.61 mmol) was added in portions, after the addition was completed, the temperature was raised to 40°C and the reaction was performed for 1 h. After the reaction was completed, the obtained system was cooled to room temperature, saturated aqueous ammonium chloride solution (100 mL) was added to quench the reaction, and ethyl acetate (100 mL×2) was used for extraction, the combined organic phases were washed with saturated brine, dried over anhydrous sodium sulfate, and filtered, and the filtrate was concentrated and the residue was purified by silica gel column chromatography (petroleum ether:ethyl acetate (v/v) = 1:3) to obtain the title compound **64G** (7.2 g, 88.65%).

Step 7: Under nitrogen atmosphere, the compound **64G** (7.2 g, 26.25 mmol) was dissolved in tetrahydrofuran (75 mL), the obtained system was cooled to 0°C, sodium 2-hydrobis (dimethoxyethoxy) aluminate (22.5 ml, 3.5 mol/L solution in toluene) was added dropwise, and the reaction was continued at 0-5°C for 3 h after the dropwise addition was completed. After the reaction was completed, the temperature was controlled at 0-5°C, and a 10% aqueous sodium hydroxide solution (20 mL) was added dropwise to quench the reaction. After quenching, anhydrous magnesium sulfate was added to dry the mixture, and filtration was performed through celite. After the filtrate was concentrated, the residue was separated and purified by silica gel column chromatography (dichloromethane:methanol (v/v) = 5:1) to obtain the title compound **64H** (5.3 g, 77.5%).

M/Z (ESI): m/z =205.2[M+H-*^{t}*Bu]⁺.

Step 8: Compounds **64H** (5.3 g, 20.36 mmol) was dissolved in a mixed solvent of tetrahydrofuran (30 mL) and water (30 mL), and sodium bicarbonate (5.13 g, 61.08 mmol) was added. The obtained system was cooled to 0-5°C, and benzyl chloroformate (4.17 g, 24.43 mmol) was slowly added dropwise. After the dropwise addition was completed, the reaction was continued at 0-5°C for 1 h. After the reaction was completed, water (50 mL) was added, and ethyl acetate (100 mL×2) was used for extraction. The combined organic phase was washed with saturated brine, dried over anhydrous sodium sulfate, and filtered. The filtrate was concentrated and the residue was separated and purified by silica gel column chromatography (petroleum ether:ethyl acetate (v/v) = 5:1) to obtain 4.8 g of the racemate. The racemate was separated by chiral preparative HPLC to obtain two isomers, compound **64I**-1 (1.6 g, retention time 1.33 min, 19.9%) and compound **641**-2 (2.0 g, retention time 1.48 min, 24.9%).

HPLC analytical method: 1. Instruments: SHIMADZU LC-30AD SFC; 2. Chromatographic column: Chiralcel OX-3 50×4.6mm I.D., 3µm; 3. Mobile phase system: A for CO2, B for 0.05%DEA in IPA; 4. Gradient: B 5%-40%; 5. Flow rate: 3 mL/min.

Preparative chromatography separation conditions: 1. Instruments: Waters 150 SFC; 2. Chromatographic column: Chiralpak OX -Column (250×30mm, I.D 30mm, 10um particle size); 3. Mobile phase system: A for CO₂ and B for IPA; 4. Gradient: B 20%; 5. Flow rate: 100 mL/min; 6. Elution time: 3.3 min.

Step 9: Compounds **64I** -1 (1.6 g, 4.06 mmol) was dissolved in methanol (20 mL), Pd/C (0.4 g, Pd content 10%) was added, and the obtained system was reacted for 3 h under hydrogen atmosphere. After the reaction was completed, filtration was performed, and the filtrate was concentrated to obtain the title compound **64J**-1 (1.00 g, 94.6%).

Referring to the above operation, compound **64I**-2 (2.00 g, 5.31 mmol) was used as the raw material to obtain the title compound **64J**-2 (1.21 g, 94.0%).

Step 10: Compound **64J**-1 (1.00 g, 3.84 mmol) and 3-carbonyl-1-methyl-cyclobutanecarboxylic acid (0.74 g, 5.76mmol) were dissolved in methanol (15 mL) in sequence, and glacial acetic acid (0.46 g, 7.46 mmol) was added. Sodium cyanoborohydride (0.72 g, 11.44 mmol) was added in portions and the mixture was reacted for 1 h after the addition was completed. After the reaction was completed, water (30 mL) was added to quench the reaction, and dichloromethane (50 mL × 2) was used for extraction. The combined organic phases were washed with saturated brine, dried over anhydrous sodium sulfate, and filtered. The filtrate was concentrated and the residue was separated and purified by silica gel column chromatography (dichloromethane:methanol (v/v) = 10:1) to obtain the title compound **64K**-1 (1.20 g, 83.9%).

Referring to the above operation, compound **64J**-2 (1.21 g, 4.1 mmol) was used as the raw material to obtain the title compound **64K**-2 (1.51 g, 87.1%).

M/Z (ESI): m/z =373.3[M+H]⁺.

Step 11: Compound **64K**-1 (1.20 g, 3.22 mmol) was dissolved in dichloromethane (10 mL), trifluoroacetic acid (3 mL) was added, and the reaction was performed at room temperature for 30 min. After the reaction was completed, the obtained system was directly concentrated to obtain the trifluoroacetate salt of compound **64L**-1 (1.6 g, 99.3%).

Referring to the above operation, compound **64K**-2 (1.51 g, 4.26 mmol) was used as the raw material to obtain the trifluoroacetate salt of the title compound **64L**-2 (2.00 g, 97.3%).

Step 12: Compound **2F** (0.15 g, 1.4 mmol) and compound **64L**-1 (0.43 g, 0.86 mmol) were dissolved in dimethyl sulfoxide (3 mL), and N,N-diisopropylethylamine (0.17 g, 1.29 mmol) and cesium fluoride (0.13 g, 0.86 mmol) were added, the temperature was raised to 100°C and the reaction was performed for 3 h. After the reaction was completed, the obtained system was cooled to room temperature, water (20 mL) was added, and ethyl acetate (30 mL ×3) was used for extraction. The combined organic phases were washed with saturated brine, dried over anhydrous sodium sulfate, and filtered. The filtrate was concentrated and the residue was separated and purified by silica gel column chromatography (petroleum ether:ethyl acetate (v/v) = 1:10) to obtain the title **compound 64** isomer 1 (Rf = 0.5 (ethyl acetate:methanol = 20:1), 95.0 mg, 37.6%) and the title **compound 64** isomer 2 (Rf = 0.3 (ethyl acetate:methanol = 20:1), 50.0 mg, 19.8%).

**Compound 64** isomer 1: ¹H NMR (400 MHz, CDCl₃) δ 7.35 (d, 1H), 7.21 (d, 1H), 7.18-7.15 (m, 1H), 5.54 (d, 1H), 5.46-5.40 (m, 1H), 4.23-4.16 (m, 1H), 4.04-3.83 (m, 4H), 3.72-3.63 (m, 2H), 2.84-2.77 (m, 2H), 2.68-3.55 (m, 3H), 2.30 (s, 3H), 1.96-1.71 (m, 4H), 1.51 (d, 3H), 1.39 (s, 3H).

M/Z (ESI): m/z =586.2[M+H]⁺.

**Compound 64** isomer 2: ¹H NMR (400 MHz, CDCl₃) δ 7.36 (d, 1H), 7.21 (d, 1H), 7.18-7.16 (m, 1H), 5.54 (d, 1H), 5.46-5.42 (m, 1H), 4.24-4.16 (m, 1H), 4.04-3.74 (m, 6H), 2.98 (d, 1H), 2.91 (d, 1H), 2.84-2.81 (m, 1H), 2.52-2.47 (m, 2H), 2.30 (s, 3H), 2.10-2.00 (m, 3H), 1.87-1.82 (m, 1H), 1.51 (d, 3H), 1.41 (s, 3H).

M/Z (ESI): m/z =586.2[M+H]⁺.

Referring to the above operation, compound **64L**-2 (0.43 g, 0.86 mmol) was used as the raw material to obtain the title **compound 64** isomer 3 (Rf=0.5 (ethyl acetate:methanol=20:1), 110.0 mg, 13.6%) and the title **compound 64** isomer 4 (Rf=0.3 (ethyl acetate:methanol=20:1), 70.0 mg, 8.7%).

**Compound 64** isomer 3: ¹H NMR (400 MHz, CDCl₃) δ 7.36 (d, 1H), 7.22 (d, 1H), 7.18-7.16 (m, 1H), 5.56 (d, 1H), 5.44-5.41 (m, 1H), 4.19-4.11 (m, 1H), 4.04-3.86 (m, 4H), 3.74-3.65 (m, 2H), 2.91-2.81 (m, 2H), 2.74-3.59 (m, 3H), 2.31 (s, 3H), 2.00-1.74 (m, 4H), 1.51 (d, 3H), 1.41 (s, 3H).

M/Z (ESI): m/z =586.2[M+H]⁺.

**Compound 64** isomer 4: ¹H NMR (400 MHz, CDCl₃) δ 7.36 (d, 1H), 7.23 (d, 1H), 7.19-7.16 (m, 1H), 5.54 (d, 1H), 5.46-5.42 (m, 1H), 4.19-4.12 (m, 1H), 4.04-3.78 (m, 6H), 3.08 (d, 1H), 2.98 (d, 1H), 2.89-2.84 (m, 1H), 2.62-2.57 (m, 2H), 2.30 (s, 3H), 2.13-2.03 (m, 3H), 1.90-1.85 (m, 1H), 1.51 (d, 3H), 1.42 (s, 3H).

M/Z (ESI): m/z =586.2[M+H]⁺.

### Example 65:

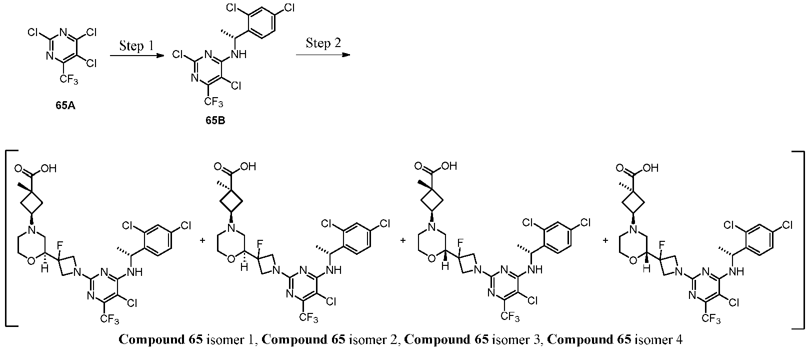

Step 1: Compound **65A** (0.50 g, 1.99 mmol) was dissolved in dichloromethane (10 mL), and (*R*)-1-(2,4-dichlorophenyl)ethanamine (0.45 g, 2.39 mmol) and triethylamine (0.60 g, 5.97 mmol) were added in sequence. The mixture was stirred at room temperature for 1 h after the addition was completed. After the reaction was completed, concentration was performed the residue was purified by silica gel column chromatography (petroleum ether:ethyl acetate (v/v) = 5:1) to obtain the title compound **65B** (0.68 g, 84.5%).

Step 2: Compound **65B** (0.15 g, 0.37 mmol) and compound **64L**-1 (0.37 g, 0.74 mmol) were dissolved in dimethyl sulfoxide (3 mL), and N, N-diisopropylethylamine (0.14 g, 1.11 mmol) and cesium fluoride (0.11 g, 0.74 mmol) were added, the temperature was raised to 100°C and the reaction was performed for 3 h. After the reaction was completed, the mixture was cooled to room temperature, and water (20 mL) was added, followed by extraction with ethyl acetate (30 mL ×3). The combined organic phases were washed with saturated brine, dried over anhydrous sodium sulfate, and filtered. The filtrate was concentrated and the residue was separated and purified by silica gel column chromatography (petroleum ether:ethyl acetate (v/v) = 1:5) to obtain the title **compound 65** isomer 1 (Rf = 0.4 (ethyl acetate:petroleum ether = 10:1), 110.0 mg, 46.4%) and the title **compound 65** isomer 2 (Rf = 0.2 (ethyl acetate:petroleum ether = 10:1), 55.0 mg, 23.2%).

**Compound 65** isomer 1: ¹H NMR (400 MHz, CDCl₃) δ 7.38 (d, 1H), 7.23-7.18 (m, 2H), 5.89 (d, 1H), 5.49-5.43 (m, 1H), 4.29-4.21 (m, 1H), 4.09-3.92 (m, 4H), 3.73-3.65 (m, 2H), 2.89-2.80 (m, 2H), 2.71-3.59 (m, 3H), 1.99-1.87 (m, 3H), 1.78-1.72 (m, 1H), 1.55 (d, 3H), 1.43 (s, 3H).

M/Z (ESI): m/z =640.2[M+H]⁺.

**Compound 65** isomer 2: ¹H NMR (400 MHz, CDCl₃) δ 7.39 (d, 1H), 7.23-7.19 (m, 1H), 5.89 (d, 1H), 5.48-5.45 (m, 1H), 4.30-4.23 (m, 1H), 4.10-3.81 (m, 6H), 3.10 (d, 1H), 3.01 (d, 1H), 2.91-2.88 (m, 1H), 2.61-2.56 (m, 2H), 2.15-2.07 (m, 3H), 1.92-1.87 (m, 1H), 1.55 (d, 3H), 1.43 (s, 3H).

M/Z (ESI): m/z =640.2[M+H]⁺.

Referring to the above operation, compound **65B** (0.15 g, 0.37 mmol) and compound **64L**-2 (0.37 g, 0.74 mmol) were used as the raw materials to obtain the title **compound 65** isomer 3 (Rf=0.4 (ethyl acetate:petroleum ether=10:1), 100.0 mg, 42.1%) and the title **compound 65** isomer 4 (Rf=0.2 (ethyl acetate:petroleum ether=10:1), 50.0 mg, 21.1%).

**Compound 65** isomer 3: ¹H NMR (400 MHz, CDCl₃) δ 7.39 (d, 1H), 7.23-7.18 (m, 2H), 5.88 (d, 1H), 5.47-5.43 (m, 1H), 4.23-4.16 (m, 1H), 4.06-3.91 (m, 4H), 3.71-3.62 (m, 2H), 2.89-2.79 (m, 2H), 2.69-3.60 (m, 3H), 1.96-1.86 (m, 3H), 1.75-1.70 (m, 1H), 1.55 (d, 3H), 1.43 (s, 3H).

M/Z (ESI): m/z =640.2[M+H]⁺.

**Compound 65** isomer 4: ¹H NMR (400 MHz, CDCl₃) δ 7.38 (d, 1H), 7.23-7.19 (m, 1H), 5.89 (d, 1H), 5.48-5.44 (m, 1H), 4.25-4.18 (m, 1H), 4.08-3.80 (m, 6H), 3.09 (d, 1H), 2.99 (d, 1H), 2.90-2.85 (m, 1H), 2.62-2.58 (m, 2H), 2.17-2.04 (m, 3H), 1.89-1.84 (m, 1H), 1.55 (d, 3H), 1.42 (s, 3H).

M/Z (ESI): m/z =640.2[M+H]⁺.

### Example 66:

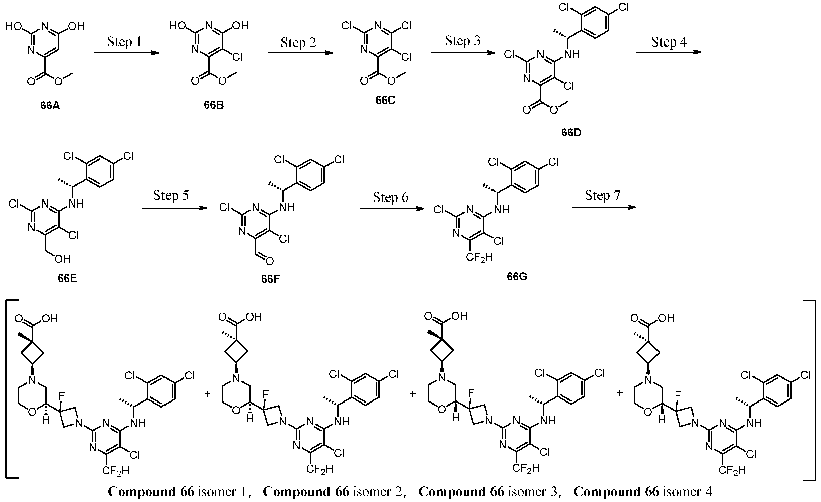

Step 1: Compound **66A** (5.00 g, 29.40 mmol) was dissolved in glacial acetic acid/acetic anhydride (v/v=20/1, 100 mL). A catalytic amount of ferric chloride was added, and the mixture was heated to 95°C and sulfuryl chloride (7.93 g, 58.78 mmol) was added dropwise. After the addition was completed, the mixture was heated to reflux and reacted for 15 h. After the reaction was completed, the solution was cooled to 10°C and filtered. The filter cake was washed with acetic acid and water in sequence, and dried in vacuo to obtain the title compound **66B** (3.6 g, 60%).

Step 2: Compound **66B** (3.0 g, 14.70 mmol) was dissolved in phosphorus oxychloride (30 mL), the obtained system was cooled to 0°C, and N,N-diethylaniline (4.39 g, 29.42 mmol) was added dropwise. The temperature was raised to 100°C after the addition was completed and the mixture was reacted for 15 h. After the reaction was completed, the phosphorus oxychloride was removed by concentration. The residue was separated and purified by silica gel column chromatography (petroleum ether:ethyl acetate (v/v) = 10:1) to obtain the title compound **66C** (2.7 g, 76.1%).

Step 3: Compound **66C** (2.70 g, 11.16 mmol) was dissolved in dichloromethane (30 mL), and (R)-1-(2,4-dichlorophenyl)ethylamine (2.55 g, 13.39 mmol) and triethylamine (3.39 g, 33.48 mmol) were added in sequence. The mixture was stirred at room temperature for 1 h after the addition was completed. After the reaction was completed, concentration was performed the residue was purified by silica gel column chromatography (petroleum ether:ethyl acetate (v/v) = 5:1) to obtain the title compound **66D** (4.20 g, 95.2%).

Step 4: Compound **66D** (4.20 g, 10.63 mmol) was dissolved in ethanol (50 mL), iodine (5.40 g, 21.28 mmol) was added, and sodium borohydride (2.01 g, 53.15 mmol) was added in portions, after the addition was completed, the reaction was performed at room temperature for 3 h. After the reaction was completed, the ethanol was removed by concentration, and water (50 mL) was added to the residue, followed by extraction with ethyl acetate (50 mL×2). The combined organic phases were washed with saturated brine, dried over anhydrous sodium sulfate, filtered, and concentrated. The residue was separated and purified by silica gel column chromatography (petroleum ether:ethyl acetate (v/v) = 3:1) to obtain the title compound **66E** (2.60 g, 66.6%).

Step 5: Compound **66E** (2.60 g, 7.08 mmol) was dissolved in 1,2-dichloroethane (30mL), active manganese dioxide (3.08g, 35.53 mmol) was added, after the addition was completed, the temperature was raised to 80°C and the reaction was performed for 5 h. After the reaction was completed, the mixture was filtered through celite and the filtrate was concentrated to obtain the crude compound **66F** (2.10 g, 81.2%), which was directly used in the next reaction without further purification.

Step 6: Compound **66F** (1.50 g, 4.11 mmol) was dissolved in dichloromethane (20 mL), and diethylaminosulfur trifluoride (1.99 g, 12.33 mmol) was added at room temperature. After the addition was completed, the mixture was stirred at room temperature for 1 h. After the reaction was completed, the residue was concentrated and purified by silica gel column chromatography (petroleum ether:ethyl acetate (v/v) = 5:1) to obtain the title compound **66G** (0.61 g, 38.3%).

Step 7: Compound **66G** (0.15 g, 0.39 mmol) and compound **64L**-1 (0.39 g, 0.78 mmol) were dissolved in dimethyl sulfoxide (3 mL), and N,N-diisopropylethylamine (0.15 g, 1.17 mmol) and cesium fluoride (0.12 g, 0.78 mmol) were added, the temperature was raised to 100°C and the reaction was performed for 3 h. After the reaction was completed, the mixture was cooled to room temperature, water (20 mL) was added, and ethyl acetate (30 mL ×3) was used for extraction. The combined organic phases were washed with saturated brine, dried over anhydrous sodium sulfate, and filtered. The filtrate was concentrated and the residue was separated and purified by silica gel column chromatography (petroleum ether:ethyl acetate (v/v) = 1:5) to obtain the title **compound 66** isomer 1 (Rf = 0.4 (ethyl acetate:petroleum ether = 10:1), 56.0 mg, 23.1%) and the title **compound 66** isomer 2 (Rf = 0.2 (ethyl acetate:petroleum ether = 10:1), 70.0 mg, 28.8%).

**Compound 66** isomer 1: ¹H NMR (400 MHz, CDCl₃) δ 7.37 (d, 1H), 7.23-7.18 (m, 2H), 6.65-6.39 (m, 1H), 5.76 (d, 1H), 5.49-5.42 (m, 1H), 4.26-4.20 (m, 1H), 4.06-3.91 (m, 4H), 3.74-3.65 (m, 2H), 2.89-2.79 (m, 2H), 2.71-2.60 (m, 3H), 1.99-1.87 (m, 3H), 1.78-1.73 (m, 1H), 1.54 (d, 3H), 1.41 (s, 3H).

M/Z (ESI): m/z =622.2[M+H]⁺.

**Compound 66** isomer 2: ¹H NMR (400 MHz, CDCl₃) δ 7.38 (d, 1H), 7.23-7.18 (m, 2H), 6.65-6.39 (m, 1H), 5.77 (d, 1H), 5.48-5.44 (m, 1H), 4.29-4.20 (m, 1H), 4.09-3.81 (m, 6H), 3.09 (d, 1H), 3.00 (d, 1H), 2.91-2.87 (m, 1H), 2.62-2.57 (m, 2H), 2.16-2.06 (m, 3H), 1.92-1.86 (m, 1H), 1.54 (d, 3H), 1.43 (s, 3H).

M/Z (ESI): m/z =622.2[M+H]⁺.

Referring to the above operation, compound **66G** (0.15 g, 0.39 mmol) and compound 64L-2 (0.39 g, 0.78 mmol) were used as the raw materials to obtain the title **compound 66** isomer 3 (Rf=0.4 (ethyl acetate:petroleum ether=10:1), 55.0 mg, 22.6%) and the title **compound 66** isomer 4 (Rf=0.2 (ethyl acetate:petroleum ether=10:1), 65.0 mg, 26.7%).

**Compound 66** isomer 3: ¹H NMR (400 MHz, CDCl₃) δ 7.37 (d, 1H), 7.23-7.18 (m, 2H), 6.65-6.39 (m, 1H), 5.76 (d, 1H), 5.49-5.42 (m, 1H), 4.28-4.20 (m, 1H), 4.06-3.91 (m, 4H), 3.74-3.65 (m, 2H), 2.87-2.79 (m, 2H), 2.71-2.60 (m, 3H), 1.99-1.87 (m, 3H), 1.78-1.73 (m, 1H), 1.54 (d, 3H), 1.41 (s, 3H).

M/Z (ESI): m/z =622.2[M+H]⁺.

**Compound 66** isomer 4: ¹H NMR (400 MHz, CDCl₃) δ 7.38 (d, 1H), 7.24-7.18 (m, 2H), 6.65-6.38 (m, 1H), 5.77 (d, 1H), 5.47-5.44 (m, 1H), 4.24-4.17 (m, 1H), 4.07-3.79 (m, 6H), 3.09 (d, 1H), 3.00 (d, 1H), 2.91-2.87 (m, 1H), 2.61-2.57 (m, 2H), 2.17-2.06 (m, 3H), 1.90-1.84 (m, 1H), 1.54 (d, 3H), 1.42 (s, 3H).

M/Z (ESI): m/z =622.2[M+H]⁺.

### Example 67:

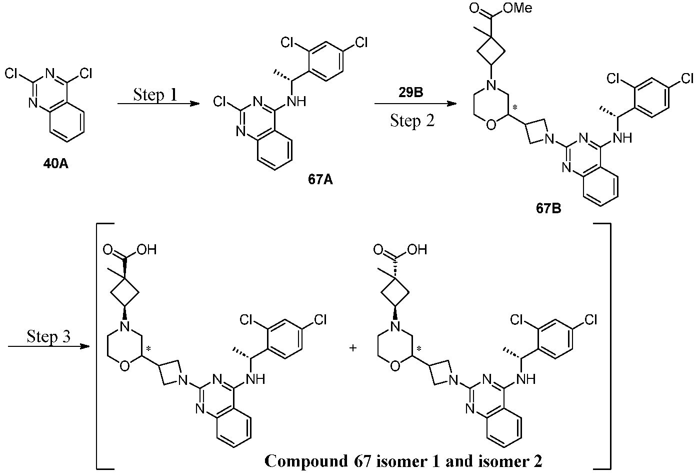

Step 1: Compound **40A** (1.00 g, 5.02 mmol), (*R*)-1-(2,4-dichlorophenyl)ethanamine (1.05 g, 5.53 mmol) and triethylamine (1.53 g, 15.1 mmol) were dissolved in dry acetonitrile (50 mL) and the reaction was performed at room temperature overnight. After the disappearance of the starting materials as detected by TLC, saturated aqueous ammonium chloride solution (50 mL) was added to quench the reaction, and the mixture was extracted with ethyl acetate (20 mL×5). The organic phases were combined and dried over anhydrous sodium sulfate, filtered and concentrated. The residue was purified by silica gel column chromatography (petroleum ether:ethyl acetate (v/v) = 80:20 to 0:100) to obtain compound **67A** (1.48 g, 83.5%).

LC-MS (ESI): m/z= 352.1 [M+H]⁺.

Step 2: The hydrochloride salt of compound **29B** (300 mg, 0.879 mmol) and **67A** (341 mg, 0.967 mmol) were dissolved in dry dimethyl sulfoxide (6 mL), triethylamine (534 mg, 5.27 mmol) and cesium fluoride (267 mg, 1.76 mmol) were added, after the addition was completed, the temperature was raised to 100°C and the reaction was performed for about 5 h. After the disappearance of the starting materials as detected by TLC, the mixture was cooled to room temperature and concentrated under reduced pressure. The residue was purified by silica gel column chromatography (dichloromethane:methanol (v/v)=90:10) to obtain compound **67B** (460 mg, 89.5%).

LC-MS (ESI): m/z= 584.2 [M+H]⁺.

Step 3: Compound **67B** (460 mg, 0.787 mmol) was dissolved in a mixed solvent of tetrahydrofuran (4 mL), methanol (4 mL) and water (4 mL), and lithium hydroxide monohydrate (132 mg, 3.15 mmol) was added at room temperature and the obtained system was reacted for 4 h. After the disappearance of the starting materials as detected by TLC, the pH was adjusted to neutral with dilute hydrochloric acid. After concentration under reduced pressure, the obtained system was directly purified by silica gel column chromatography (ethyl acetate:methanol (v/v)=80:20 to 50:50) to obtain **compound 67,** isomer 1 (172 mg, 38.3%) and **compound 67,** isomer 2 (105 mg, 23.4%).

**Compound 67,** isomer 1 (Rf = 0.25 (ethyl acetate:methanol (v/v) = 5:1): ¹H NMR (400 MHz, CD₃OD) δ 8.28-8.27 (m, 1H), 7.23-7.22 (m, 1H), 7.49-7.41 (m, 2H), 7.42-7.33 (m, 2H), 7.27-7.26 (m, 1H), 5.70-5.69 (m, 1H), 4.21-4.01 (m, 3H), 4.00-3.83 (m, 2H), 3.70-3.58 (m, 2H), 2.87-2.68 (m, 4H), 2.61-2.60 (m, 2H), 1.98-1.97 (m, 1H), 1.80-1.79 (m, 2H), 1.67 (d, 3H), 1.61-1.60 (m, 1H), 1.36 (s, 3H);

LC-MS (ESI): m/z=570.2 [M+H]⁺.

**Compound 67,** isomer 2 (Rf = 0.15 (ethyl acetate:methanol (v/v)=5:1): ¹H NMR (400 MHz, CD₃OD) δ 8.26-8.25 (m, 1H), 7.71-7.70 (m, 1H), 7.50-7.34 (m, 4H), 7.27-7.26 (m, 1H), 5.70-5.69 (m, 1H), 4.17-4.16 (m, 1H), 4.13-4.00 (m, 2H), 3.99-3.87 (m, 2H), 3.67-3.66 (m, 2H), 2.98-2.70 (m, 4H), 2.40-2.27 (m, 2H), 2.17-1.98 (m, 3H), 1.80-1.68 (m, 1H), 1.63-1.62 (m, 3H), 1.40 (s, 3H).

LC-MS (ESI): m/z=570.2 [M+H]⁺.

### Example 68:

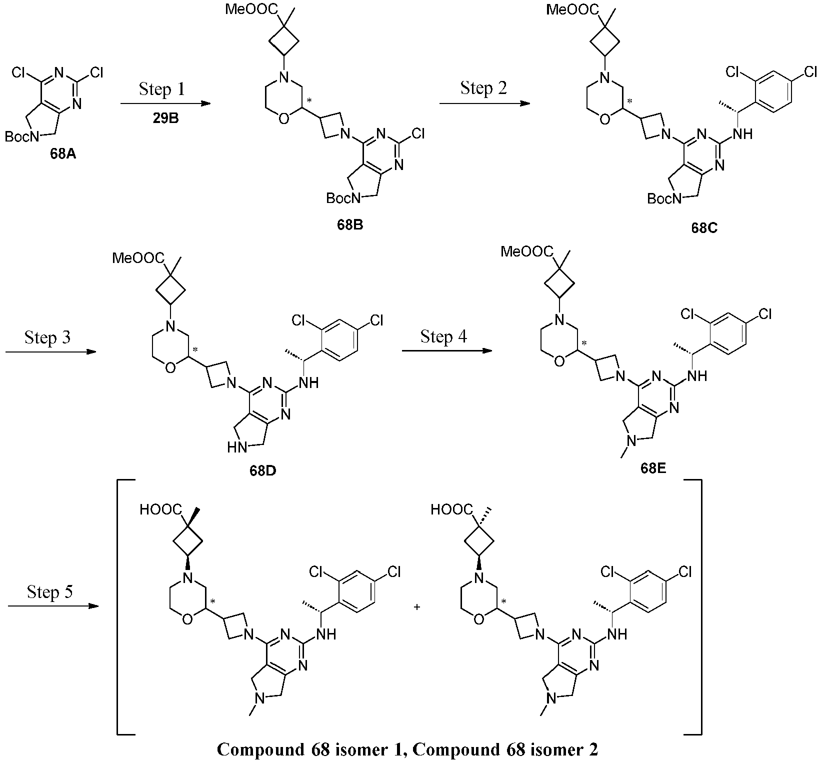

Step 1: **29B** (0.5 g, 1.47 mmol) and **68A** (0.43 g, 1.47 mmol) were dissolved in acetonitrile (20 mL), triethylamine (0.75 g, 7.35 mmol) was added, after the addition was completed, the reaction was performed at room temperature for 16 h. The mixture was concentrated under reduced pressure, and water (20 mL) was added. The mixture was extracted with ethyl acetate (20 mL× 3). The combined organic phases were washed with saturated brine (20 mL), dried over anhydrous sodium sulfate, and filtered, and the filtrate was concentrated under reduced pressure. The residue was separated by silica gel column chromatography (PE:EA (v/v) = 10:1) to obtain compound **68B** (0.6 g, yield: 78%).

LC-MS (ESI): m/z =522.3 [M+H]⁺.

Step 2: **68B** (0.55 g, 1.06 mmol) and (R)-1-(2,4-dichlorophenyl)ethylamine (0.2 g 1.06 mmol), Pd₂(dba)₃ (0.19 g, 0.21 mmol), BINAP (0.26 g, 0.42 mmol) and cesium carbonate(1.03 g, 3.18 mmol) were dissolve in 1,4-dioxane (40 mL) in sequence and the reaction was performed at 100°C for 4 h. After the reaction was completed, the mixture was cooled to room temperature, water (20 mL) was added, and the mixture was extracted with ethyl acetate (20 mL×3). The organic phases were combined, washed with saturated brine (20 mL), dried over anhydrous sodium sulfate, and filtered, and the filtrate was concentrated under reduced pressure. The residue was separated by silica gel column chromatography (DCM:MeOH (v/v) = 10:1) to obtain the compound **68C** of interest (330 mg, yield: 46%).

LC-MS (ESI): m/z =675.3 [M+H]⁺.

Step 3: Compound **68C** (0.33 g, 0.49 mmol) was dissolved in dichloromethane (10 mL), hydrogen chloride dioxane solution (2 mL, 4M) was added, and the reaction was performed at room temperature for 30 min. After the reaction was completed, saturated sodium bicarbonate solution was added to adjust the pH of the reaction liquid to 8, extraction was performed with dichloromethane (20 mL×3), the organic phases was combined and washed with saturated brine (20 mL), dried over anhydrous sodium sulfate, and filtered, and the filtrate was concentrated under reduced pressure to obtain compound **68D** (0.28 g, 100%), which could be directly used in the next reaction without purification.

LC-MS (ESI): m/z =575.3 [M+H]⁺.

Step 4: Compound **68D** (180 mg, 0.31 mmol) and formaldehyde (24 mg, 40% aqueous formaldehyde solution) were dissolved in 1,2-dichloroethane (15 mL), and glacial acetic acid (19 mg, 0.31 mmol) was added. Sodium triacetoxyborohydride (131 mg, 0.62 mmol) was added in portions and the mixture was reacted for 15 h after the addition was completed. After the reaction was completed, water (20 mL) was added to quench the reaction, and dichloromethane (30 mL × 3) was used for extraction. The combined organic phases were washed with saturated brine, dried over anhydrous sodium sulfate, and filtered. The filtrate was concentrated and the residue was separated and purified by silica gel column chromatography (dichloromethane:methanol (v/v) = 10:1) to obtain the title compound **68E** (180 mg, 97%).

LC-MS (ESI): m/z =589.3 [M+H]⁺.

Step 5: Compound **68E** (180 mg, 0.31 mmol) was dissolved in (THF:H₂O (v/v) = 1:1) solution (4 mL), lithium hydroxide (82 mg, 3.1 mmol) was added, and the obtained system was reacted under stirring at room temperature for 4 h. After the reaction was completed, the reaction liquid was concentrated under reduced pressure, and the residue was separated and purified by silica gel column chromatography (dichloromethane:methanol (v/v) = 10:1) to obtain the title **compound 68** isomer 1 (Rf=0.6 (dichloromethane:methanol=10:1), 35 mg, 19.8%) and title **compound 68** isomer 2 (Rf=0.4 (dichloromethane:methanol=10:1), 15 mg, 8.5%).

**Compound 68** isomer 1: ¹H NMR (400 MHz, CD₃OD) δ 7.41-7.39 (m, 2H), 7.25-7.22 (m, 1H), 5.37-5.31 (m, 1H), 4.07-3.90 (m, 6H), 3.78-3.77 (m, 2H), 3.69-3.57 (m, 2H), 2.90-2.74 (m, 4H), 2.66 (s, 3H), 2.61-2.58 (m, 2H), 2.06-1.96 (m, 4H), 1.86-1.81 (m, 2H), 1.66-1.61 (m, 1H), 1.45-1.44 (d, 3H), 1.38 (s, 3H).

M/Z (ESI): m/z =575.2[M+H]⁺.

**Compound 68** isomer 2: ¹H NMR (400 MHz, CD₃OD) δ 7.41-7.39 (m, 2H), 7.25-7.22 (m, 1H), 5.37-5.31 (m, 1H), 4.07-3.90 (m, 6H), 3.71-3.59 (m, 4H), 2.91-2.70 (m, 4H), 2.61 (s, 3H), 2.35-2.30 (m, 2H), 2.13-2.02 (m, 6H), 1.78-1.68 (m, 1H), 1.45-1.43(d, 3H), 1.38 (s, 3H).

M/Z (ESI): m/z =575.2[M+H]⁺.

### Example 69:

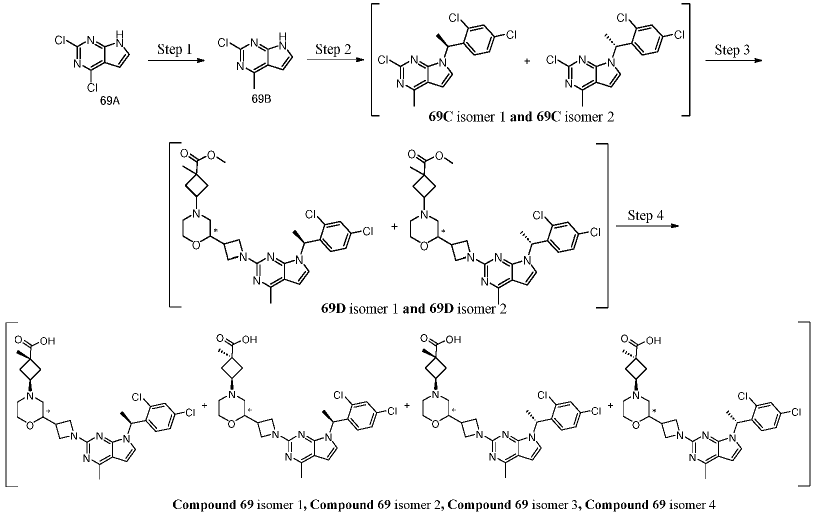

Step 1: Compound **69A** (3.8 g, 20.2 mmol) and iron acetylacetonate (0.71 g, 2.0 mmol) were mixed and dissolved in THF (50 mL), the obtained system was cooled to 0°C, and methylmagnesium chloride (20 mL, 3M solution in THF) was added dropwise, the reaction was contined to performed for 1 h after the dropwise addition was completed. Saturated ammonium chloride (100 mL) was added to quench the reaction, and the mixture was extracted with ethyl acetate (120 mL×2). The combined organic phase was dried over anhydrous sodium sulfate and filtered. The filtrate was concentrated under reduced pressure and separated by silica gel column chromatography (PE:EA (v/v)=3:1) to obtain the compound **69B** of interest (1.9 g, yield: 56%).

LC-MS (ESI): m/z =168.2 [M+H]⁺.

Step 2: Compound **69B** (1.9 g, 11.3 mmol) was dissolved in DMF (30 mL), the obtained system was cooled to 0°C, sodium hydride (0.55 g, 13.6 mmol, 60% wt) was added, and the reaction was continued at room temperature for 20 min, then 1-(1-bromoethyl)-2,4-dichlorobenzene (4.32 g, 17.0 mmol) was added dropwise, after the dropwise addition was completed, the temperature was raised to room temperature and the reaction was performed for 30 min. Saturated aqueous sodium bicarbonate solution (50 mL) was added to quench the reaction, and the mixture was extracted with ethyl acetate (80 mL×2). The combined organic phase was dried over anhydrous sodium sulfate and filtered. The filtrate was concentrated under reduced pressure and separated by silica gel column chromatography (PE:EA (v/v)=4:1) to obtain the compound **69C** of interest (2.5 g, yield: 65%). Further resolution using chiral SFC gave two isomers of compound **69C.**

Resolution method: instrument: Waters 150 SFC, column: Chiralcel OJ Column (250 * 30 mm, I.D 30 mm, 10 um particle size), mobile phase: A for CO₂ and B for IPA, gradient: 15% phase B isocratic elution, flow rate: 100 mL/min, back pressure: 100 bar, column temperature: 25°C, wavelength: 220 nm, cycle time: 2.9 min

Analytical method: instrument: SHIMADZU LC-30AD SFC, column: Chiralcel OJ-3 50 × 4.6 mm I.D., 3 µm, mobile Phase: A for CO₂ B for 0.05% DEA in IPA, gradient: B 5-40%, flow rate: 3 mL/min, back pressure: 100 bar, column temperature: 35°C, wavelength: 220 nm. retention time: Compounds **69C** isomer 1: 1.15 min, compound **69C** isomer 2: 1.40 min.

Compounds **69C** isomer 1: LC-MS M/Z (ESI)=340.2 [M+H]⁺.

Compounds **69C** isomer 2: LC-MS M/Z (ESI)=340.2 [M+H]⁺.

Step 3: Compound **69C** (isomer 1) (0.34 g, 1.0 mmol) was dissolved in DMSO (20 mL), compound **29B** (0.34 g, 1.0 mmol) was added, and the obtained system was stirred evenly, triethylamine (0.40 g, 4.0 mmol) and cesium fluoride (0.30 g, 2.0 mmol) were added in sequence, after the addition was completed, the temperature was raised to 100°C and the reaction was performed for 36 h. The reaction liquid was cooled to room temperature, water (40 mL) was added, extraction was performed with ethyl acetate (40 mL×3), the organic phases were combined and washed with saturated brine (40 mL), dried over anhydrous sodium sulfate, and filterd, and the filtrate was concentrated under reduced pressure and separated and purified by silica gel column chromatography (DCM:MeOH (v/v)=12:1) to obtain the compound **69D** of interest (isomer 1) (0.16 g, yield: 28%).

LC-MS M/Z (ESI)=572.2 [M+H]⁺.

**69C** (isomer 2) (0.34 g, 1.0 mmol) was dissolved in DMSO (20 mL), **29B** (0.34 g, 1.0 mmol) was added and stirred evenly, and then triethylamine (0.40 g, 4.0 mmol) and cesium fluoride (0.30 g, 2.0 mmol) were added, after the addition was completed, the reaction was performed at 100°C for 36 h. The reaction liquid was cooled to room temperature, and water (40 mL) was added, followed by extraction with ethyl acetate (40 mL×3). The organic phases were combined, washed with saturated brine (40 mL), dried over anhydrous sodium sulfate, and filtered. The filtrate was concentrated under reduced pressure and purified by silica gel column chromatography (DCM:MeOH (v/v)=12:1) to obtain the compound **69D** of interest (isomer 2) (0.14 g, yield: 24%).

LC-MS M/Z (ESI)=572.2 [M+H]⁺.

Step 4: Compound **69D** (isomer 1) (0.16 g, 0.28 mmol) was dissolved in tetrahydrofuran (10 mL), methanol (3 mL) and water (3 mL) was added, after the obtained system was stirred evenly, lithium hydroxide monohydrate (0.12 g, 3 mmol) was added, the reaction was performed at room temperature for 3 h, pH was adjusted to 5 with hydrochloric acid (1N aqueous solution), the system was concentrated under reduced pressure and directly separated by silica gel column chromatography (EA: MeOH(v/v)=10:1) to obtain two isomers of the **compound 69** of interest (isomer 1:Rf=0.48 (EA:MeOH(v/v)= 10:1); isomer 2: Rf = 0.32 (EA:MeOH (v/v) =10:1)).

**Compound 69** isomer 1: ¹H NMR (400 MHz, Methanol-*d*₄) δ 7.35 (d, 1H), 7.20-7.11 (m, 2H), 7.03 (d, 1H), 6.36 (d, 1H), 6.06 (q, 1H), 4.04-3.86 (m, 3H), 3.85-3.72 (m, 2H), 3.61-3.50 (m, 2H), 2.88-2.76 (m, 1H), 2.76-2.66 (m, 2H), 2.65-2.54 (m, 1H), 2.53-2.45 (m, 2H), 2.41 (s, 3H), 2.02-1.92 (m, 1H), 1.81-1.70 (m, 5H), 1.65 (t, 1H), 1.26 (s, 3H).

LC-MS (ESI): m/z =558.2 [M+H]⁺.

**Compound 69** isomer 2: ¹H NMR (400 MHz, Methanol-*d*₄) δ 7.35 (d, 1H), 7.21-7.12 (m, 2H), 7.04 (d, 1H), 6.37 (d, 1H), 6.08 (q, 1H), 4.06-3.85 (m, 4H), 3.83-3.76 (m, 1H), 3.69-3.58 (m, 2H), 3.07-2.96 (m, 1H), 2.90 (t, 2H), 2.70-2.58 (m, 1H), 2.42 (s, 3H), 2.38-2.30 (m, 2H), 2.25-2.15 (m, 1H), 2.05-1.97 (m, 2H), 1.89 (t, 1H), 1.73 (d, 3H), 1.30 (s, 3H).

LC-MS (ESI): m/z =558.2 [M+H]⁺.

Compound **69D** (isomer 2) (0.14 g, 0.24 mmol) was dissolved in tetrahydrofuran (10 mL), methanol (3 mL) and water (3 mL) were added, after the obtained system was stirred evenly, lithium hydroxide monohydrate (0.12 g, 3 mmol) was added, the reaction was performed at room temperature for 3 h, pH was adjusted to 5 with hydrochloric acid (1N aqueous solution), the system was concentrated under reduced pressure and directly separated by silica gel column chromatography (EA: MeOH(v/v)=10:1) to obtain the other two isomers of the **compound 69** of interest (isomer 3: Rf=0.49(EA:MeOH(v/v)=10:1); isomer 4: Rf = 0.31 (EA:MeOH(v/v)=10:1)).

**Compound 69** isomer 3: ¹H NMR (400 MHz, Methanol-*d*₄) δ 7.50-7.46 (m, 1H), 7.33-7.23 (m, 2H), 7.16 (d, 1H), 6.49 (d, 1H), 6.19 (q, 1H), 4.12-4.05 (m, 2H), 3.99-3.86 (m, 3H), 3.73-3.63 (m, 2H), 2.99-2.90 (m, 1H), 2.89-2.78 (m, 2H), 2.77-2.68 (m, 1H), 2.66-2.59 (m, 2H), 2.54 (s, 3H), 2.14-2.05 (m, 1H), 1.94-1.82 (m, 5H), 1.77 (t, 1H), 1.39 (s, 3H).

LC-MS (ESI): m/z =558.2 [M+H]⁺.

**Compound 69** isomer 4: ¹H NMR (400 MHz, Methanol-*d*₄) δ 7.48 (d, 1H), 7.33-7.23 (m, 2H), 7.16 (d, 1H), 6.49 (d, 1H), 6.20 (q, 1H), 4.13-4.04 (m, 2H), 4.02-3.89 (m, 3H), 3.80-3.70 (m, 2H), 3.16-3.07 (m, 1H), 3.07-2.96 (m, 2H), 2.80-2.70 (m, 1H), 2.54 (s, 3H), 2.50-2.40 (m, 2H), 2.35-2.25 (m, 1H), 2.17- 2.08 (m, 2H), 2.00 (t, 1H), 1.86 (d, 3H), 1.42 (s, 3H).

LC-MS (ESI): m/z =558.2 [M+H]⁺.

### Example 70:

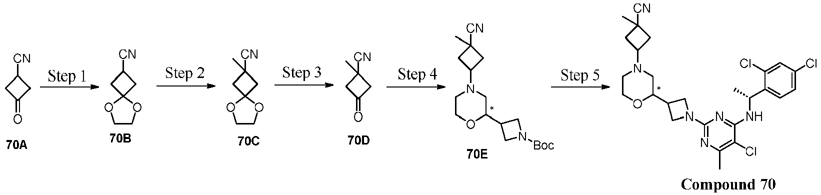

Step 1: 3-oxocyclobutane-1-carbonitrile (3.00 g, 31.49 mmol), p-toluenesulfonic acid monohydrate (0.27 g, 1.57 mmol), and ethylene glycol (2.1 g, 34.64 mmol) were added to toluene (60 mL), and the obtained system was refluxed to remove water and reacted for 16 h. After the reaction was completed, the obtained system was cooled to room temperature and concentrated, and the residue was separated and purified by column chromatography (petroleum ether:ethyl acetate (v/v) = 20:1-2:1)to obtain the product **70B** (3.25 g, 74.17%).

¹H NMR (400 MHz, Chloroform-d) δ 3.91 (s, 4H), 2.93-2.82 (m, 1H), 2.79-2.66 (m, 4H).

Step 2: **70B** (3.25 g, 23.36 mmol) was dissolved in anhydrous tetrahydrofuran (32 mL), the obtained system was cooled to -78°C, and a solution of lithium diisopropylamide in tetrahydrofuran (7.01 mL, 28.03 mmol) was added dropwise, after stirring was performed for 1 h, methyl iodide (4.31 g, 30.31 mmol) was added dropwise, after the dropwise addition was completed, the temperature was raised to room temperature and the reaction was performed for 16 h. Water (120 mL) and ethyl acetate (100 mL) were added, extraction and liquid separation were performed, and the aqueous phase was extracted with ethyl acetate (100 mL × 3), the combined organic phases was washed with saturated brine (300 mL), dried over anhydrous sodium sulfate, filtered, concentrated, separated and purified by column chromatography (petroleum ether:ethyl acetate (v/v) = 20:1-1:1) to obtain the product **70C** (2.33 g, 65.11%).

¹H NMR (400 MHz, Chloroform-d) δ 3.93-3.88 (m, 4H), 2.97-2.93(m, 1H), 2.93-2.89 (m, 1H), 2.44-2.40 (m, 1H), 2.40-2.34 (m, 1H), 1.59 (s, 3H).

Step 3: **70C** (2.33 g, 15.21 mmol) was dissolved in acetone (46 mL), 6N hydrochloric acid (4.6 mL, 27.53mmol) was added, and the obtained system was stirred at room temperature for 16 h. The reaction was stopped and the solvent was removed by concentration to obtain a crude product **70D** (1.72 g).

¹H NMR (400 MHz, Chloroform-d) δ 3.77-3.73 (m, 1H), 3.73-3.68 (m, 1H), 3.20- 3.15 (m, 1H), 3.15-3.09 (m, 1H), 1.73 (s, 3H).

Step 4: **9J-2** (2.25 g, 9.29mmol) and **70D** (1.72 g, 11.06 mmol) were dissolved in DCE (40 mL), acetic acid (0.26 g, 9.29mmol) was added, after stirring was performed at room temperature for 1 h, sodium cyanoborohydride (1.17 g, 18.58 mmol) was added in portions, after the addition was completed, the reaction was performed at room temperature for 16 h. After the reaction was completed, water (50 mL) was added, and extraction and liquid separation was perfomed. The aqueous phase was extracted with dichloromethane (50 mL × 2). The combined organic phases were washed with saturated brine, dried over anhydrous sodium sulfate, filtered, and concentrated. The residue was separated and purified by column chromatography (petroleum ether:ethyl acetate (v/v) = 10:1- 1:1) to obtain **70E** (0.79 g, 25.35%).

LCMS (ESI): m/z =336.3 [M+H]⁺.

Step 5: **70E** (0.10 g, 0.30mmol) was dissloved in dichloromethane (3 mL), trifluoroacetic acid (1 mL) was added dropwise, and the obtained system was stirred at room temperature for 2 h. After the reaction was completed as monitored by LCMS, the mixture was concentrated to remove most of the trifluoroacetic acid. The residue was dissolved in anhydrous DMSO (3 mL), and **2F** (0.13 g, 0.37 mmol), DIEA (0.20 g, 1.51 mmol) and cesium fluoride (91 mg, 0.60 mmol) were added in sequence. The temperature was raised to 102°C and the reaction was carried out for 2 h. After the reaction was completed, the mixture was cooled to room temperature, and water (15 mL) and ethyl acetate (15 mL) were added for extraction and liquid separation. The aqueous phase was extracted with ethyl acetate (15 mL × 2). The combined organic phases were dried over anhydrous sodium sulfate, and filtered, and the filtrate was concentrated. The residue was purified by column chromatography (petroleum ether:ethyl acetate (v/v) = 10:1-1:4) to obtain **compound 70** (61 mg, 36.98%).

¹H NMR (400 MHz, Chloroform-d) δ 7.38-7.32 (m, 1H), 7.25-7.20 (m, 1H), 7.20-7.14 (m, 1H), 5.58-5.46 (m, 1H), 5.46-5.37 (m, 1H), 4.02-3.93 (m, 1H), 3.91-3.81 (m, 2H), 3.81-3.66 (m, 2H), 3.65-3.51 (m, 2H), 2.90-2.80 (m, 1H), 2.64-2.50 (m, 3H), 2.50-2.40 (m, 2H), 2.30 (s, 3H), 2.23-2.15 (m, 2H), 1.98-1.88 (m, 1H), 1.55 (s, 3H), 1.53-1.49 (m, 3H).

LCMS m/z =549.2 [M+H]⁺.

### Example 71:

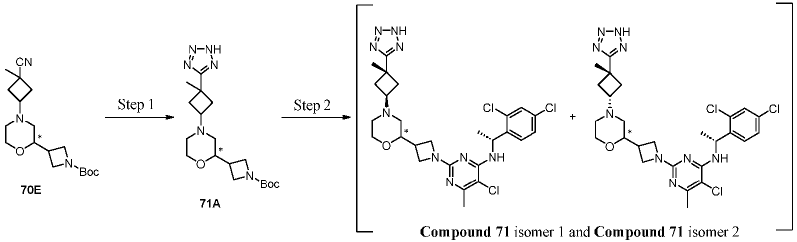

Step 1: **70E** (0.67 g, 2.00 mmol) was dissolved in DMF (12 mL), sodium azide (0.78 g, 12 mmol) and ammonium chloride (0.64 g, 12 mmol) were added, and nitrogen replacement was performed, the temperature was raised to 140°C and the reaction was performed for 5 h. The reaction was stopped, the obtained system was cooled to room temperature, and the crude product was obtained by reverse phase purification. The crude product was concentrated to dryness and subjected to column chromatography (dichloromethane:methanol (v/v) = 100:1-4:1) to obtain **71A** (0.12, 15.85%).

LCMS m/z =579.1 [M+H]⁺.

Step 2: **71A** (0.12 g, 0.32 mmol) was dissolved in dichloromethane (4 mL), trifluoroacetic acid (1 mL) was added, and the obtained system was stirred at room temperature for 2 h. After the reaction was completed as monitored by LCMS, most of the trifluoroacetic acid was removed by concentration, and the residue was dissolved in anhydrous DMSO (3 mL). **2F** (0.13 g, 0.38 mmol), DIEA (0.21 g, 1.60 mmol) and cesium fluoride (97 mg, 0.64 mmol) were added, and the obtained system was heated to 102°C and reacted for 2 h. After the reaction was completed, the mixture was cooled to room temperature, and water (15 mL) and ethyl acetate (15 mL) were added for extraction and liquid separation. The aqueous phase was extracted with ethyl acetate (15 mL × 2). The combined organic phases were dried over anhydrous sodium sulfate, filtered, and concentrated. The residue was separated by column chromatography (dichloromethane :methanol (v/v) = 100:1-1:4) to obtain a crude product. The crude product was further subjected to thin layer chromatography (dichloromethane:methanol (v/v) = 10:1) to obtain **compound 71** isomer 1 (Rf = 0.35 (dichloromethane:methanol = 10:1), 12 mg, 6.32%) and **compound 71 isomer 2** (Rf = 0.40 (dichloromethane:methanol = 10:1), 30 mg, 15.81%).

**Compound 71** isomer 1: ¹H NMR (400 MHz, Chloroform-d) δ 7.38-7.34 (m, 1H), 7.24-7.20 (m, 1H), 7.20-7.15 (m, 1H), 5.66-5.60 (m, 1H), 5.48-5.39 (m, 1H), 4.01-3.93 (m, 1H), 3.93-3.78 (m, 3H), 3.78-3.58 (m, 3H), 3.02-2.93 (m, 1H), 2.83-2.64 (m, 4H), 2.63-2.51 (m, 1H), 2.23 (s, 3H), 2.24-2.12 (m, 2H), 2.05-1.95 (m, 1H), 1.61 (s, 3H), 1.55-1.46 (m, 3H).

LCMS m/z =592.1 [M+H]⁺.

**Compound 71** isomer 2: ¹H NMR (400 MHz, Methanol-d4) δ 7.43-7.39 (m, 1H), 7.37-7.33 (m, 1H), 7.27-7.21 (m, 1H), 5.51-5.42 (m, 1H), 3.94-3.86 (m, 2H), 3.86-3.80 (m, 1H), 3.80-3.74 (m, 1H), 3.73-3.66 (m, 1H), 3.66-3.57 (m, 1H), 3.57-3.51 (m, 1H), 3.18-3.09 (m, 1H), 2.80-2.78 (m, 2H), 2.65-2.57 (m, 1H), 2.50-2.42 (m, 2H), 2.38-2.30 (m, 2H), 2.26 (s, 3H), 2.16-2.08 (m, 1H), 1.78-1.70 (m, 1H), 1.59 (s, 3H), 1.52-1.46 (m, 3H).

LCMS m/z =592.3 [M+H]⁺.

### Example 72:

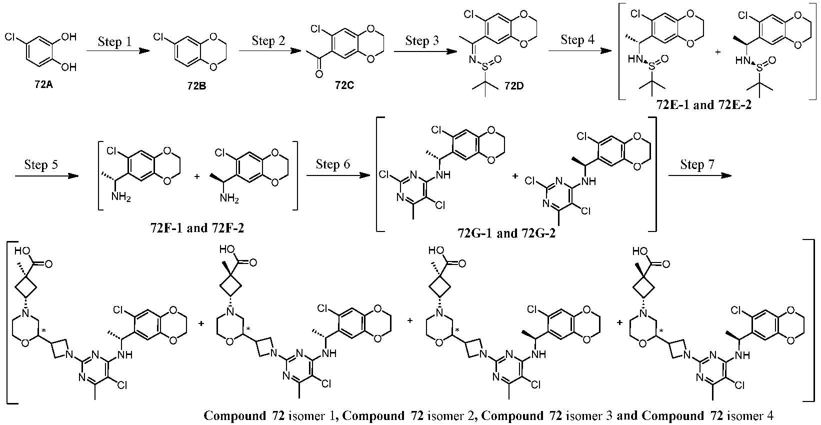

Step 1: Potassium carbonate (14.33 g, 103.71 mmol) and 1,2-dibromoethane (12.99 g, 69.14 mmol) were added to a solution of 72A (5 g, 34.57 mmol) in ethylene glycol (50 mL), and the mixture was heated to 100°C and stirred for 16 h. After the reaction was completed, the mixture was cooled to room temperature, and water (50 mL) and ethyl acetate (60 mL) were added for extraction and liquid separation. The aqueous phase was extracted with ethyl acetate (60 mL). The combined organic phases were washed with saturated brine, dried over anhydrous sodium sulfate, filtered, concentrated, and subjected to column chromatography (petroleum ether:ethyl acetate (v/v) = 5:1) to obtain **72B** (3.47 g, 59%).

LC-MS (ESI): m/z =171.1 [M+H]⁺.

Step 2: Acetyl chloride (3.19 g, 40.68 mmol) was added to a solution of **72B** (3.47 g, 20.34 mmol) in dichloromethane (40 mL), and aluminum trichloride (5.42 g, 40.68 mmol) was added in portions, the reaction was performed at 45°C for 3 h. After the reaction was completed, the reaction liquid was cooled to room temperature and poured into ice water, liquid separation was performed. The aqueous phase was extracted with dichloromethane (50 mL × 2). The combined organic phases were washed with saturated brine, dried over anhydrous sodium sulfate, filtered, concentrated, and subjected to column chromatography (petroleum ether:ethyl acetate (v/v) = 3:1) to obtain **72C** (3.9 g, 90%).

LC-MS (ESI): m/z =213.1 [M+H]⁺.

Steps 3 to 6: Using (R)-(+)-tert-butylsulfenamide (2.67 g, 22.01 mmol) and **72C** (3.9 g, 18.34 mmol) as the raw materials, the operation method of steps 3 to 6 of example 7 was referenced to obtain **compound 72G-1** and compound **72G-2.**

LCMS (ESI): m/z =374.1 [M+H]⁺.

Step 7: **9J-2**(130 mg, 0.53 mmol), DIEA (210 mg, 1.59 mmol) and cesium fluoride (240 mg, 1.59 mmol) were added to a solution of **compound 72G-1** (200 mg, 0.53 mmol) in DMSO (4 mL), the temperature was raised to 100°C and the reaction was performed for 2 h. After the reaction was completed, water (15 mL) and ethyl acetate (15 mL) were added for extraction and liquid separation. The aqueous phase was extracted with ethyl acetate (15 mL × 2). The combined organic phases were washed with saturated brine, dried over anhydrous sodium sulfate, filtered, and concentrated. The residue was purified by silica gel column chromatography (EA:MeOH(v/v)=5:1) to obtain **compound 72** isomer 1 (Rf=0.5, (EA:MeOH(v/v)=3:1), 200 mg, 64%) and **compound 72** isomer 2 (Rf=0.4, (EA:MeOH(v/v)=3:1), 50 mg, 16%).

**Compound 72** isomer 1: ¹H NMR (400 MHz, CDCl₃) δ = 6.85 (s, 1H), 6.78 (s, 1H), 5.56-5.55 (m, 1H), 5.44-5.36 (m, 1H), 4.22 (s, 4H), 4.02-4.01 (m, 1H), 3.91-3.83 (m, 4H), 3.76-3.64 (m, 2H), 2.97-2.88 (m, 1H), 2.80-2.73 (m, 2H), 2.62-2.49 (m, 3H), 2.30 (s, 3H), 2.08-2.04 (m, 3H), 1.91 (m, 1H), 1.48-1.47 (m, 3H), 1.40 (s, 3H).

LCMS (ESI): m/z =592.2 [M+H]⁺.

**Compound 72** isomer 2: ¹H NMR (400 MHz, CDCl₃) δ = 6.84 (s, 1H), 6.78 (s, 1H), 5.48-5.47 (m, 1H), 5.41-5.32 (m, 1H), 4.21 (s, 4H), 4.04-3.85 (m, 5H), 3.84-3.68 (m, 2H), 3.03-2.94 (m, 1H), 2.88-2.82 (m, 2H), 2.60-2.52 (m, 3H), 2.29 (s, 3H), 2.17-2.05 (m, 3H), 1.77-1.72 (m, 1H), 1.48-1.47 (m, 3H), 1.42 (s, 3H).

LCMS (ESI): m/z =592.2 [M+H]⁺.

Referring to the above operation, compound **72G-2** was used as the raw material (0.1 g, 0.27 mmol) to obtain the title **compound 72** isomer 3 (Rf=0.4, (EA:MeOH(v/v)=3:1), 50 mg, 31%) and **compound 72** isomer 4 (Rf=0.3, (EA:MeOH(v/v)=3:1), 20 mg, 13%).

**Compound 72** isomer 3: ¹H NMR (400 MHz, CDCl₃) δ = 6.86 (s, 1H), 6.78 (s, 1H), 5.52-5.51 (m, 1H), 5.41-5.39 (m, 1H), 4.21 (s, 4H), 4.03-4.02 (m, 1H), 3.98-3.78 (m, 4H), 3.69-3.61 (m, 2H), 2.88-2.80 (m, 1H), 2.75-2.71 (m, 2H), 2.60-2.56 (m, 3H), 2.30 (s, 3H), 2.02-1.83 (m, 3H), 1.58-1.57 (m, 1H), 1.48-1.46 (m, 3H), 1.39 (s, 3H).

LCMS (ESI): m/z =592.2 [M+H]⁺.

**Compound 72** isomer 4: ¹H NMR (400 MHz, CDCl₃) δ 6.85 (s, 1H), 6.78 (s, 1H), 5.49-5.48 (m, 1H), 5.41-5.39 (m, 1H), 4.21 (s, 4H), 3.99-3.98 (m, 1H), 3.96-3.76 (m, 6H), 3.01-3.00 (m, 2H), 2.89-2.82 (m, 1H), 2.66-2.56 (m, 3H), 2.28 (s, 3H), 2.13-2.05 (m, 3H), 1.74-1.73 (m, 1H), 1.48-1.47 (m, 3H), 1.42 (s, 3H).

LCMS (ESI): m/z =592.2 [M+H]⁺.

### Example 73:

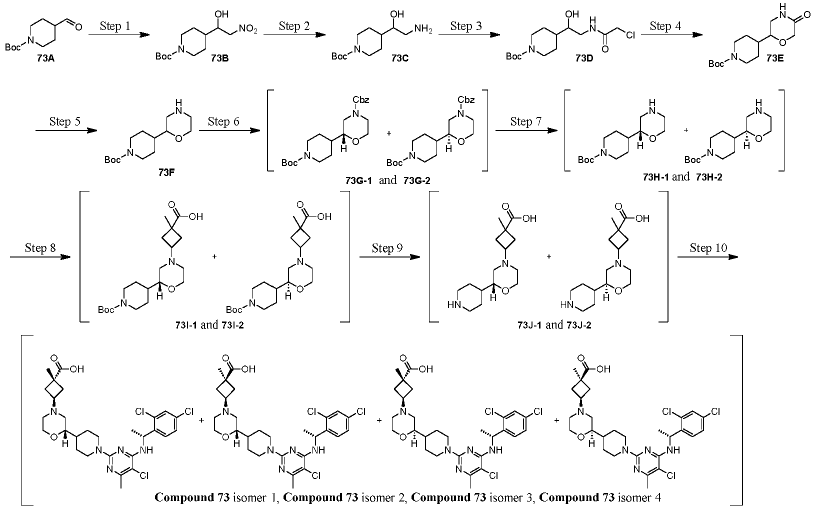

Step 1: Compound **73A** (40.0 g, 187.5 mmol) was dissolved in methanol (400 mL), and nitromethane (45.8 g, 750.2 mmol) and triethylamine (37.9 g, 375.1 mmol) were added in sequence, after the addition was completed, the obtained system was stirred at room temperature for 17 h. The crude product of compound **73B** (55.0 g) was obtained by concentration and used directly in the next reaction without further purification.

LCMS m/z = 219.1 [M+H-*^{t}*Bu]⁺.

Step 2: Compound **73B** (55.0 g, 200.5 mmol) was dissolved in methanol (550 mL), and Pd/C (11.0 g, Pd content 10%) was added. The mixture was reacted under hydrogen atmosphere at room temperature for 24 h. Filtration was performed and the filtrate was concentrated to obtain the crude product of compound **73C** (40.0 g, 82%), which was used directly in the next step without further purification.

LCMS m/z = 189.2 [M+H-*^{t}*Bu]⁺.

Step 3: Compound **73C** (40.0 g, 163.7 mmol) was dissolved in a mixed solvent of ethyl acetate (200 mL) and water (200 mL), and sodium bicarbonate (41.2 g, 491.1 mmol) was added. The obtained system was cooled to 5°C, and chloroacetyl chloride (27.7 g, 245.6 mmol) was added dropwise. The temperature was controlled at 5-10°C, and after the dropwise addition was completed, the obtained system was warmed to room temperature and reacted for 1 h. After the reaction was completed, water (100 mL) was added, and ethyl acetate (300 mL × 2) was used for extraction. The combined organic phase was washed with saturated brine (50 mL), dried over anhydrous sodium sulfate, filtered, and concentrated. The residue was separated and purified by silica gel column chromatography (petroleum ether:ethyl acetate (v/v) = 1:2) to obtain the title compound **73D** (30.0 g, 57%).

LCMS m/z = 221.1 [M+H-Boc]⁺.

Step 4: Potassium tert-butoxide (20.9 g, 187.0 mmol) was added to tert-butanol (200 mL), and a solution of compound **73D** (30.0 g, 93.5 mmol) in tert-butanol (100 mL) was added, the obtained system was controlled at 30-40°C and stirred for 2 h. After the reaction was completed, the mixture was cooled to room temperature, saturated aqueous ammonium chloride solution (200 mL) was added to quench the reaction, and the mixture was extracted with ethyl acetate (300 mL×2). The combined organic phases were washed with saturated brine (50 mL), dried over anhydrous sodium sulfate, and filtered. The filtrate was concentrated and the residue was separated and purified by silica gel column chromatography (petroleum ether:ethyl acetate (v/v) = 1:2) to obtain the title compound **73E** (23.6 g, 89%).

LCMS m/z = 185.2 [M+H-Boc]⁺.

Step 5: Under nitrogen atmosphere, compound **73E** (23.6 g, 83.0 mmol) was dissolved in toluene (230 mL), the obtained system was cooled to -10°C, and sodium 2-hydrobis(dimethoxyethoxy)aluminate (71.1 mL, 3.5 mol/L solution in toluene) was slowly added dropwise. After the dropwise addition was completed, the reaction was continued at -10°C for 3 h. After the reaction was completed, 10% aqueous sodium hydroxide solution (50 mL) was slowly added dropwise to quench the reaction. After quenching, ethyl acetate (100 mL) was added to the reaction liquid for extraction. The organic phase was washed with water (50 mL), washed with saturated brine (50 mL), dried over anhydrous sodium sulfate, and filtered, and the filtrate was concentrated under reduced pressure to obtain **73F** (20.3 g, 90%).

LCMS m/z = 215.2 [M+H-*^{t}*Bu]⁺.

Step 6: Compound **73F** (8.0 g, 29.6 mmol) was dissolved in a mixed solvent of tetrahydrofuran (50 mL) and water (50 mL), and sodium bicarbonate (7.4 g, 88.8 mmol) was added. The obtained system was cooled to 0-5°C, and benzyl chloroformate (6.0 g, 35.6 mmol) was slowly added dropwise. After the dropwise addition was completed, the reaction was continued at 0-5°C for 1 h. After the reaction was completed, water (100 mL) was added and ethyl acetate (150 mL × 2) was used for extraction. The combined organic phases were washed with saturated brine (50 mL), dried over anhydrous sodium sulfate, and filtered, and the filtrate was concentrated. The residue was separated and purified by silica gel column chromatography (petroleum ether:ethyl acetate (v/v) = 3:1) to obtain 10.0 g of the racemate. The racemate was separated by chiral preparative HPLC to obtain two isomers, compound **73G**-1 (3.8 g, 32%) and compound **73G**-2 (3.5 g, 29%).

LCMS m/z = 305.2 [M+H-Boc]⁺.

Preparative chromatography separation conditions: 1. Instruments: Waters 150 SFC; 2. Chromatographic column: Chiralcel OX Column (250×30mm, I.D 30mm, 10um particle size); 3. Mobile phase system: A for CO₂ and B for IPA (0.1% NH₃•H₂O); 4. Gradient: B 25%; 5. Flow rate: 100 mL/min.

HPLC analytical method: 1. Instruments: SHIMADZU LC-30AD; 2. Chromatographic column: Whelk- O1 column; 3. Mobile phase system: A for CO2; B for 0.05% DEA in MEOH; 4. Gradient: B 5%-40%; 5. Flow rate: 3mL/min. Compound **73G**-1 (retention time 1.46 min) and compound **73G**-2 (retention time 1.55 min).

Step 7: Compound **73G**-1 (3.8 g, 9.4 mmol) was dissolved in ethyl acetate (40 mL), and Pd/C (0.8 g, Pd content 10%) was added, and the mixture was reacted at room temperature under a hydrogen atmosphere for 3 h. Filtration was performed and the filtrate was concentrated to obtain the title compound **73H**-1 (2.3 g, 91%).

Referring to the above operation, compound **73G**-2 (3.5 g, 9.0 mmol) was used as the raw material to obtain the title compound **73H**-2 (2.1 g, 90%).

LCMS m/z = 215.3 [M+H-*^{t}*Bu]⁺.

Step 8: Compound **73H**-1 (200 mg, 0.74 mmol) and 3-carbonyl-1-methyl-cyclobutanecarboxylic acid (113.7 mg, 0.89 mmol) were dissolved in methanol (5 mL) in turn, glacial acetic acid (44.4 mg, 0.74 mmol) was added, and finally sodium cyanoborohydride (93.2 mg, 1.48 mmol) was added. After the addition was completed, the mixture was reacted at room temperature for 30 min. After the reaction was complete, water (2 mL) was added to quench the reaction, and dichloromethane (5 mL × 2) was used for extraction. The combined organic phases were washed with saturated brine (3 mL), dried over anhydrous sodium sulfate, and filtered. The filtrate was concentrated and the residue was separated and purified by silica gel column chromatography (dichloromethane:methanol (v/v) = 10:1) to obtain the title compound **731**-1 (200 mg, 71%).

Referring to the above operation, compound **73H**-2 (200 mg, 0.74 mmol) was used as the raw material to obtain the title compound **73I**-2 (200 mg, 71%).

LCMS m/z = 383.6 [M+H]⁺.

Step 9: Compound **73I**-1 (200 mg, 0.52 mmol) was dissolved in dichloromethane (4 mL), trifluoroacetic acid (1 mL) was added, and the reaction was performed at room temperature for 2 h. After the reaction was completed, the mixture was directly concentrated to obtain the trifluoroacetate salt of compound **73J**-1 (146 mg, 99%).

Referring to the above operation, compound **73I**-2 (200 mg, 0.52 mmol) was used as the raw material to obtain the trifluoroacetate salt of the title compound **73J**-2 (142 mg, 96%).

LCMS m/z = 283.4 [M+H]⁺.

Step 10: Compound **2F** (90.5 mg, 0.26 mmol) and compound **73J**-1 (146 mg, 0.52 mmol) were dissolved in dimethyl sulfoxide (2 mL), and N,N-diisopropylethylamine (199.7 mg, 1.56 mmol) and cesium fluoride (160.2 mg, 1.04 mmol) were added, the temperature was raised to 100°C and the reaction was performed for 5 h. After the reaction was completed, the mixture was cooled to room temperature, water (2 mL) was added, and ethyl acetate (5 mL ×3) was used for extraction. The combined organic phases were washed with saturated brine (2 mL), dried over anhydrous sodium sulfate, and filtered. The filtrate was concentrated and the residue was separated and purified by silica gel column chromatography (ethyl acetate:methanol (v/v) = 9:1) to obtain the title **compound 73** isomer 1 (Rf = 0.3 (ethyl acetate:methanol (v/v) = 9:1), 30.0 mg, 10%) and the title **compound 73** isomer 2 (Rf = 0.2 (ethyl acetate:methanol (v/v) = 9:1), 10.0 mg, 3%).

**Compound 73** isomer 1: ¹H NMR (400 MHz, CDCl₃) δ 7.35-7.32 (m, 1H), 7.24-7.21 (m, 1H), 7.17-7.13 (m, 1H), 5.43-5.34 (m, 2H), 4.58-4.47 (m, 2H), 3.94-3.87 (m, 1H), 3.72-3.62 (m, 1H), 3.35-3.28 (m, 1H), 2.98-2.89 (m, 2H), 2.85-2.78 (m, 1H), 2.63-2.38 (m, 7H), 2.27 (s, 3H), 2.06- 1.98 (m, 4H), 1.76-1.70 (m, 1H), 1.51-1.48 (m, 3H), 1.37 (s, 3H).

LCMS m/z = 596.2 [M+H]⁺.

**Compound 73** isomer 2: ¹H NMR (400 MHz, CDCl₃) δ 7.35-7.32 (m, 1H), 7.24-7.21 (m, 1H), 7.17-7.13 (m, 1H), 5.43-5.34 (m, 2H), 4.58-4.47 (m, 2H), 3.94-3.88 (m, 1H), 3.71-3.62 (m, 1H), 3.35-3.21 (m, 1H), 2.98-2.88 (m, 2H), 2.85-2.78 (m, 1H), 2.63-2.38 (m, 7H), 2.27 (s, 3H), 2.06- 1.98 (m, 4H), 1.76-1.71 (m, 1H), 1.51-1.48 (m, 3H), 1.37 (s, 3H).

LCMS m/z = 596.2 [M+H]⁺.

Referring to the above operation, compound **73J**-2 (142 mg, 0.50 mmol) was used as the raw material to obtain the title **compound 73** isomer 3 (Rf=0.3 (ethyl acetate:methanol (v/v)= 9:1), 50.0 mg, 17%) and the title **compound 73** isomer 4 (Rf=0.2 (ethyl acetate:methanol (v/v)= 9:1), 8.0 mg, 3%). **Compound 73** isomer 3: ¹H NMR (400 MHz, CDCl₃) δ 7.35-7.33 (m, 1H), 7.23-7.20 (m, 1H), 7.16-7.13 (m, 1H), 5.44-5.36 (m, 2H), 4.58-4.46 (m, 2H), 3.90-3.83 (m, 1H), 3.64-3.57 (m, 1H), 3.23-3.16 (m, 1H), 2.87-2.49 (m, 8H), 2.27 (s, 3H), 1.98-1.84 (m, 3H), 1.77-1.63 (m, 2H), 1.58- 1.51 (m, 2H), 1.50-1.48 (m, 3H), 1.40 (s, 3H).

LCMS m/z = 596.2 [M+H]⁺.

**Compound 73** isomer 4: ¹H NMR (400 MHz, CDCl₃) δ 7.36-7.33 (m, 1H), 7.23-7.20 (m, 1H), 7.17-7.14 (m, 1H), 5.46-5.35 (m, 2H), 4.60-4.45 (m, 2H), 3.97-3.90 (m, 1H), 3.80-3.69 (m, 1H), 3.40-3.31 (m, 1H), 3.10-3.01 (m, 2H), 2.63-2.50 (m, 6H), 2.28 (s, 3H), 2.19-2.08 (m, 4H), 1.90- 1.83 (m, 1H), 1.70-1.59 (m, 2H), 1.52-1.49 (m, 3H), 1.40 (s, 3H).

LCMS m/z = 596.2 [M+H]⁺.

### Example 74:

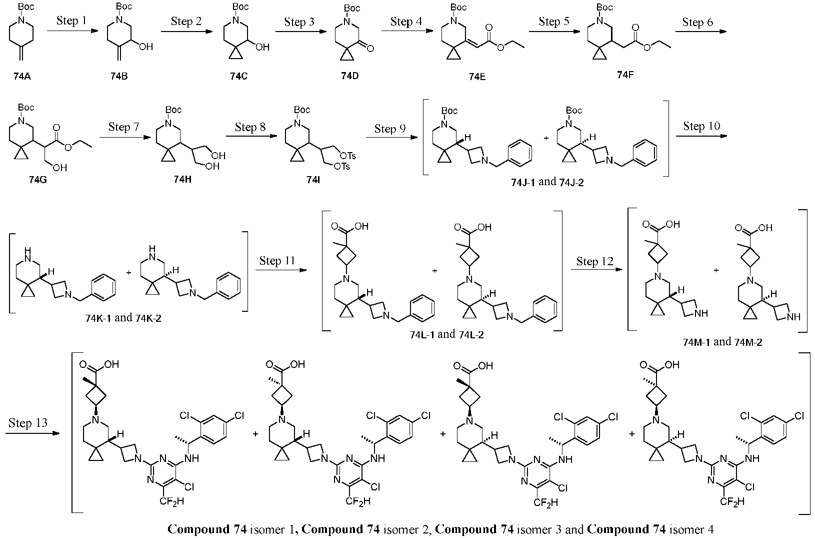

Step 1: Compound **74A** (50.0 g, 253.46 mmol) was dissolved in dichloromethane (1000 mL), and selenium oxide (14.06 g, 126.73 mmol) was added. After the addition was completed, the obtained system was cooled to 0-5°C. Tert-butyl hydroperoxide (81.58 g, 633.65 mmol, 70% aqueous solution) was slowly added dropwise. After the addition was completed, the temperature was controlled at 0-5°C and the reaction was continued for 15 h. After the reaction was completed, saturated aqueous sodium bisulfite solution (300 mL) was slowly added dropwise. After quenching, liquid separation was performed. The aqueous phase was extracted with dichloromethane (150 mL×2). The combined organic phases were washed with saturated brine, dried over anhydrous sodium sulfate, and filtered. The filtrate was concentrated and the residue was separated by silica gel column chromatography (petroleum ether:ethyl acetate (v/v) = 2:1) to obtain compound **74B** (25.0 g, 46.2%).

Step 2: Under nitrogen atmosphere, compound **74B** (25.0 g, 117.24 mmol) was dissolved in toluene (250 mL), the temperature was controlled at 25-30°C, and diethylzinc (235 mL, 235.0 mmol, 1.0 mol/L solution in n-hexane) was added dropwise, after the dropwise addition was completed, the obtained system was stirred for 0.5 h, diiodomethane (94.20 g, 351.72 mmol) was slowly added dropwise, after the dropwise addition was completed, the reaction was continued at 25-30°C for 1 h After the reaction was completed, the obtained system was cooled to 0-5°C, and a saturated aqueous ammonium chloride solution (100 mL) was slowly added dropwise to quench the reaction. After quenching, the mixture was filtered and the filtrate was extracted with ethyl acetate (100 mL×2). The combined organic phases were washed with saturated brine, dried over anhydrous sodium sulfate, and filtered, and the filtrate was concentrated under reduced pressure. The residue was separated by silica gel column chromatography (petroleum ether:ethyl acetate (v/v) = 2:1) to obtain compound **74C** (23.0 g, 86.3%).

Step 3: Compound **74C** (23.00 g, 101.19 mmol) was dissolved in dichloromethane (250 mL), the obtained system was cooled to 0°C, and Dess-Martin periodinane (85.84 g, 202.38 mmol) was added in portions. After the addition was completed, the obtained system was warmed to room temperature and reacted for 2 h. After the reaction was completed, saturated aqueous sodium bicarbonate solution (200 mL) was added to quench the reaction. After quenching, the filtration was performed through diatomaceous earth, and the filtrate was subjected to extractioni and liquid separation. The aqueous phase was extracted with dichloromethane (100 mL×2). The combined organic phases were washed with saturated brine, dried over anhydrous sodium sulfate, and filtered, and the filtrate was concentrated to obtain compound **74D** (22.00 g, 96.5%).

Step 4: Under nitrogen atmosphere, triethyl phosphoryl acetate (43.79 g, 195.32 mmol) was dissolved in tetrahydrofuran (200 mL), the temperature was controlled at 0-5°C, and sodium hydride (5.86 g, 146.5 mmol, 60% content) was added in portions, after the addition was completed, stirring was continued for 0.5 h, a solution of compound **74D** (22.00 g, 97.66 mmol) in tetrahydrofuran (100 mL) was added dropwise, and after the dropwise addition was completed, the temperature was naturally raised to room and the reaction was performed for 1 h. After the reaction was completed, saturated aqueous ammonium chloride solution (100 mL) was added to quench the reaction. After quenching, the obtained system was extracted with ethyl acetate (150 mL × 2). The combined organic phase was washed with saturated brine, dried over anhydrous sodium sulfate, filtered, and the filtrate was concentrated. The residue was separated by silica gel column chromatography (petroleum ether:ethyl acetate (v/v) = 4:1) to obtain compound **74E** (25.0 g, 86.7%).

Step 5: Compound **74E** (25.0 g, 84.64 mmol) was dissolved in methanol (250 mL), and Lindlar catalyst (9.01 g, Pd content 5%) was added, and the reaction was carried out under hydrogen atmosphere for 3 h. After the reaction was completed, the mixture was filtered and the filtrate was concentrated to obtain a crude product of compound **74F** (23.0 g, 91.3%), which was used directly in the next step without further purification.

Step 6: Under nitrogen atmosphere, compound **74F** (23.0 g, 77.34 mmol) was dissolved in tetrahydrofuran (250 mL), the obtained system was cooled to -78°C, and lithium diisopropylamide (135 mL, 270.68 mmol, 2 mol/L) was slowly added dropwise. After the dropwise addition was completed, the temperature was controlled below -70°C and the reaction was continued for 0.5 hour. Paraformaldehyde (23.22 g, 773.33 mmol) was added in portions. After the addition was completed, the temperature was slowly raised to room temperature and the reaction was continued for 15 h. After the reaction was completed, saturated aqueous ammonium chloride solution (300 mL) was added to quench the reaction, and ethyl acetate (150 mL×2) was used for extraction. The combined organic phase was washed with saturated brine, dried over anhydrous sodium sulfate, and filtered. The filtrate was concentrated and the residue was separated by silica gel column chromatography (petroleum ether:ethyl acetate (v/v) = 2:1) to obtain compound **74G** (17.0 g, 67.1%).

Step 7: Under nitrogen atmosphere, compound **74G** (17.0 g, 51.92 mmol) was dissolved in tetrahydrofuran (200 mL), the obtained system was cooled to 0-5°C, lithium aluminum hydride (3.94 g, 103.84 mmol) was added in portions, after the addition was completed, the temperature was controlled at 0-5°C and the reaction was performed for 2 h. After the reaction was completed, the temperature was controlled at 0°C, and water (4.0 mL) was slowly added dropwise. After the dropwise addition was completed, the mixture was stirred for 10 mins. A 15% aqueous sodium hydroxide solution (4.0 mL) was added dropwise. After the addition was completed and the mixture was stirred for 10 min, water (12.0 mL) was added dropwise again. After the dropwise addition was completed, anhydrous magnesium sulfate was added and stirred for 1 h. The mixture was filtered, and the filtrate was concentrated. The residue was separated by silica gel column chromatography (petroleum ether:ethyl acetate (v/v) = 1:1) to obtain compound **74H** (11.0 g, 74.2%).

Step 8: Compound **74H** (11.0 g, 38.55 mmol) was dissolved in dichloromethane (200 mL), 4-dimethylaminopyridine (18.84 g, 154.2 mmol) was added, the obtained system was cooled to 0-5°C, p-toluenesulfonyl chloride (22.05 g, 115.66 mol) was added in portions, and the obtained system was warmed to room temperature and reacted for 1 h after the addition was completed. After the reaction was completed, water was added to quench the reaction, and then the obtained system was extracted with ethyl acetate (100 mL×2). The combined organic phase was washed with saturated brine, dried over anhydrous sodium sulfate, and filtered, and the filtrate was concentrated under reduced pressure. The residue was separated by silica gel column chromatography (petroleum ether:ethyl acetate (v/v) = 10:1) to obtain compound **74I** (19.1 g, 83.5%).

Step 9: Compound **74I** (19.1 g, 32.17 mmol) was dissolved in acetonitrile (200 mL), benzylamine (10.34 g, 96.51 mmol) was added, and then the temperature was raised to 80°C and the reaction was performed for 15 h. After the reaction was completed, the obtained system was concentrated and the residue was separated by silica gel column chromatography (dichloromethane:methanol (v/v) = 20:1) to obtain the racemate of compound **74J.** The racemate was separated by chiral preparative HPLC to obtain two isomers, compound **74J**-1 (1.0 g, retention time 1.70 min, 8.72%) and compound **74J**-2 (1.1 g, retention time 1.85 min, 9.6%).

HPLC analytical method: 1. Instruments: SHIMADZU LC-30AD SFC; 2. Chromatographic column: Chiralcel OX-3 50×4.6mm I.D., 3µm; 3. Mobile phase system: A for CO2, B for 0.05%DEA in MeOH; 4. Gradient: B 5%-40%; 5. Flow rate: 3 mL/min.

Preparative chromatography separation conditions: 1. Instruments: Waters 150 SFC; 2. Chromatographic column: Chiralpak OX -Column (250×30mm, I.D 30mm, 10um particle size); 3. Mobile phase system: A for CO₂ and B for B for MeOH +ACN (0.1%NH₃•H₂O); 4. Gradient: B 20%; 5. Flow rate: 100 mL/min; 6. Elution time: 5.2 min.

Step 10: Compound **74J**-1 (1.0g, 2.81mmol) was dissolved in a mixed solvent of dichloromethane (10 mL) and trifluoroacetic acid (3 mL), and the obtained system was stirred at room temperature for 1 h. After the reaction was completed, the obtained system was concentrated and the residue dissolved in dichloromethane (50 mL). Saturated aqueous sodium bicarbonate solution was added to adjust the pH to 7-8, and extraction and separation was performed. The aqueous phase was extracted with dichloromethane (30 mL×2). The combined organic phases were dried over anhydrous sodium sulfate, and filtered, and the filtrate was concentrated to obtain the crude compound **74K**-1 (0.68 g, 94.3%), which was directly used in the next step without further purification.

Referring to the above operation, compound **74J**-2 (1.0 g, 3.09 mmol) was used as the raw material to obtain the title compound **74K**-2 (0.73 g, 92.1%).

Step 11: Compound **74K**-1 (0.68 g, 2.65 mmol) and 3-carbonyl-1-methyl-cyclobutanecarboxylic acid (0.41 g, 3.21mmol) were dissolved in methanol (10 mL) in sequence, and glacial acetic acid (0.32 g, 5.33 mmol) was added. Sodium cyanoborohydride (0.33 g, 5.25 mmol) was added in portions and the mixture was reacted for 1 h after the addition was completed. After the reaction was completed, the mixture was concentrated and the residue was separated and purified by a reverse phase column (0.2% aqueous trifluoroacetic acid solution:acetonitrile (v/v) = 5:95-95:5) to obtain the trifluoroacetate salt of the title compound **74L**-1 (1.0 g, 63.26%).

Referring to the above operation, compound **74K**-2 (0.73 g, 2.85 mmol) was used as the raw material to obtain the trifluoroacetate salt of the title compound **74L**-2 (1.1 g, 64.7%).

M/Z (ESI): m/z =369.2[M+H]⁺.

Step 12: Compound **74L**-1 (1.0 g, 1.68 mmol) was dissolved in methanol (10 mL), Pd/C (0.18 g, Pd content 10%) was added, and the reaction was carried out under hydrogen atmosphere for 15 h. After filtration, the filtrate was concentrated to obtain the crude product of compound **74M**-1 (0.80 g, 94.0%), which was directly used in the next step without further purification.

Referring to the above operation, compound **74L**-2 (11 g, 1.84 mmol) was used as the raw material to obtain the trifluoroacetate salt of the title compound **74M**-2 (0.85 g, 91.2%).

M/Z (ESI): m/z =279.2[M+H]⁺.

Step 13: Compound **66G** (90.0 mg 0.23 mmol) and compound **74M**-1 (233.0 mg, 0.46 mmol) were dissolved in dimethyl sulfoxide (5 mL), and N,N-diisopropylethylamine (0.12 g, 0.93 mmol) and cesium fluoride (70 mg, 0.46 mmol) were added, after the addition was completed, the temperature was raised to 100°C and the reaction was performed for 5 h. After the reaction was completed, the mixture was cooled to room temperature, water (20 mL) was added, and ethyl acetate (20 mL×3) was used for extraction. The combined organic phases were washed with saturated brine, dried over anhydrous sodium sulfate, and filtered, and the filtrate was concentrated. The residue was separated and purified by silica gel column chromatography (ethyl acetate:methanol (v/v) = 10:1) to obtain the title **compound 74** isomer 1 (Rf = 0.5 (ethyl acetate:methanol = 10:1), 20.0 mg, 13.8%) and the title **compound 74** isomer 2 (Rf = 0.3 (dichloromethane:methanol = 10:1), 10.0 mg, 6.9%).

**Compound 74** isomer 1:¹H NMR (400 MHz, CD₃OD) δ 7.32 (d, 1H), 7.28 (d, 1H), 7.18-7.15 (m, 1H), 6.71-6.44 (m, 1H), 5.41-5.39 (m, 1H), 3.97-3.93 (m, 1H), 3.85-3.78 (m, 1H), 3.65-3.62 (m, 1H), 3.42-3.34 (m, 1H), 2.75-2.45 (m, 7H), 1.97-1.92 (m, 1H), 1.87-1.82 (m, 1H), 1.55-1.46 (m, 2H), 1.42 (d, 3H), 1.30 (s, 3H), 1.23-1.19 (m, 2H), 0.49-0.47 (m, 1H), 0.28-0.23 (m, 3H).

M/Z (ESI): m/z =630.0[M+H]⁺.

**Compound 74** isomer 2:¹H NMR (400 MHz, CD₃OD) δ 7.32 (d, 1H), 7.27 (d, 1H), 7.17-7.14 (m, 1H), 6.68-6.41 (m, 1H), 5.43-5.37 (m, 1H), 4.01-3.97 (m, 1H), 3.84-3.80 (m, 1H), 3.50-3.46 (m, 1H), 3.32-3.25 (m, 1H),3.03-2.59 (m, 5H), 2.48-2.40 (m, 2H), 2.16-2.09 (m, 2H), 1.84-1.50 (m, 2H), 1.40 (d, 3H), 1.27 (s, 3H), 1.23-1.17(m, 2H), 0.56-0.54 (m, 1H), 0.32-0.28 (m, 3H).

M/Z (ESI): m/z =630.0[M+H]⁺.

Referring to the above operation, compound **66G** (90.0 mg 0.23 mmol) and compound **74M**-2 (233.0 mg, 0.46 mmol) were used as the raw materials to obtain the title **compound 74** isomer 3 (Rf=0.5 (ethyl acetate:methanol=10:1), 11.0 mg, 7.6%) and the title **compound 74** isomer 4 (Rf=0.3 (ethyl acetate:methanol=10:1), 8.0 mg, 5.5%).

**Compound 74** isomer 3:¹H NMR (400 MHz, CD₃OD) δ 7.32 (d, 1H), 7.27 (d, 1H), 7.18-7.15 (m, 1H), 6.71-6.44 (m, 1H), 5.39-5.35 (m, 1H), 3.95-3.90 (m, 1H), 3.88-3.80 (m, 1H), 3.61-3.57 (m, 1H), 3.38-3.30 (m, 1H), 2.78-2.59 (m, 7H), 1.93-1.88 (m, 1H), 1.81-1.76 (m, 1H), 1.58-1.48 (m, 2H), 1.42 (d, 3H), 1.29 (s, 3H), 1.23-1.19 (m, 2H), 0.47-0.45 (m, 1H), 0.30-0.25 (m, 3H).

M/Z (ESI): m/z =630.0[M+H]⁺.

**Compound 74** isomer 4:¹H NMR (400 MHz, CD₃OD) δ 7.32 (d, 1H), 7.25 (d, 1H), 7.16-7.14 (m, 1H), 6.69-6.42 (m, 1H), 5.40-5.38 (m, 1H), 3.96-3.92 (m, 2H), 3.68-3.63 (m, 1H), 3.38-3.29 (m, 2H), 3.03-2.59 (m, 5H), 2.46-2.40 (m, 2H), 2.13-2.11 (m, 2H), 1.84-1.51 (m, 2H), 1.41 (d, 3H), 1.27 (s, 3H), 1.24-1.17 (m, 2H), 0.53-0.51 (m, 1H), 0.31-0.27 (m, 3H).

M/Z (ESI): m/z =630.0[M+H]⁺.

### Example 75:

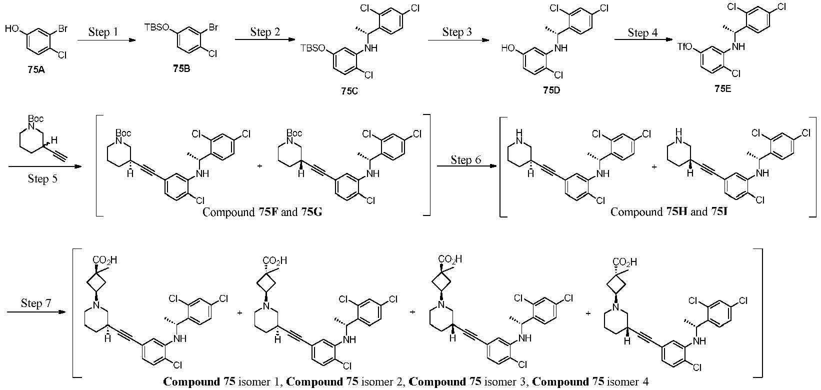

Step 1: Compound **75A** (10.0 g, 48.2 mmol) was dissolved in dichloromethane (200 mL), and imidazole (4.59 g, 67.5 mmol) and tert-butyldimethylchlorosilane (9.45 g, 62.7 mmol) were added at 0°C in sequence, after the addition was completed, stirring was continued at 0°C for 30 min, and then the temperature was raised to room temperature and the reaction was performed under stirring overnight. After the disappearance of the raw materials as monitored by TLC, water (200 mL) was added to quench the reaction. The aqueous phase was extracted with dichloromethane (50 mL × 3). The organic phases were combined and dried over anhydrous sodium sulfate. After filtration and concentration, the crude product was purified by silica gel column chromatography (petroleum ether:ethyl acetate (v/v) = 100:0 to 90:10) to obtain compound **75B** (15.1 g, 97.4%).

Step 2: Under nitrogen protection, compound **75B** (8.00 g, 24.9 mmol), (R)-1-(2,4-dichlorophenyl)ethan-1-amine (5.20 g, 27.4 mmol), palladium acetate (558 mg, 2.49 mmol), 1,1'-binaphthyl-2,2'-bisdiphenylphosphine (2.32 g, 3.73 mmol), and sodium tert-butoxide (3.58 g, 37.3 mmol) were added to a dry toluene (150 mL), and the temperature was raised to 110°C and the reaction was performed for 3 h. After the disappearance of the raw materials as monitored by TLC, the mixture was cooled to room temperature, filtered through celite and washed with ethyl acetate (100 mL × 5). The filtrate was concentrated and purified by silica gel column chromatography (petroleum ether:ethyl acetate (v/v) = 85:15) to obtain compound **75C** (8.48 g, 79.1%).

LC-MS (ESI): m/z =430.1 [M+H]⁺.

Step 3: Compound **75C** (8.48 g, 19.7 mmol) was dissolved in dry tetrahydrofuran (100 mL), and tetrabutylammonium fluoride (29.5 mL, 29.5 mmol, 1 moL in THF) was added at 0°C, after the addition was completed, the temperature was raised to room temperature and stirring was continued for 30 min. After the disappearance of the raw materials as monitored by TLC, saturated aqueous ammonium chloride solution (100 mL) was added to quench the reaction, and the aqueous phase was extracted with ethyl acetate (50 mL × 4). The organic phases were combined and dried over anhydrous sodium sulfate, filtered and concentrated, and the crude product was purified by silica gel column chromatography (petroleum ether:ethyl acetate (v/v) = 80:20) to obtain compound **75D** (6.10 g, 97.9%).

LC-MS (ESI): m/z =316.1 [M+H]⁺.

Step 4: Compound **75D** (3.50 g, 11.1 mmol) was dissolved in dry dichloromethane (100 mL), and triethylamine (1.79 g, 17.7 mmol) and trifluoromethanesulfonic anhydride (3.43 g, 12.2 mmol) were added in sequence at 0°C, after the addition was completed, stirring was continued at 0°C for 2 h. After the disappearance of the raw materials as monitored by TLC, water (200 mL) was added to quench the reaction, and the aqueous phase was extracted with dichloromethane (50 mL × 3). The organic phases were combined and dried over anhydrous sodium sulfate, filtered and concentrated, and the crude product was purified by silica gel column chromatography (petroleum ether:ethyl acetate (v/v) = 90:10) to obtain compound **75E** (4.27 g, 86.1%).

LC-MS (ESI): m/z =448.0 [M+H]⁺.

Step 5: Under nitrogen protection, compound **75E** (1.50 g, 3.34 mmol), tert-butyl 3-ethynylpiperidine-1-carboxylate (1.06 g, 5.01 mmol), bis(triphenylphosphine)palladium(II) chloride (235 mg, 0.334 mmol), copper iodide (318 mg, 1.67 mmol), and triphenylphosphine (175 mg, 0.669 mmol) were added to a mixed solvent of dry *N,N-*dimethylformamide (20 mL) and triethylamine (8 mL) in sequence, and then the temperature was raised to 100°C and the reaction was performed overnight. After the disappearance of the starting material as monitored by TLC, the mixture was cooled to room temperature, and concentrated under reduced pressure. The residue was separated and purified by silica gel column chromatography (petroleum ether:ethyl acetate (v/v) = 90:10) to obtain a mixture of **75F** and **75G** (1.55 g, 91.2%). The above mixture was separated by chiral HPLC to obtain compound **75F** (624 mg, 36.7%) and compound **75G** (694 mg, 40.8%).

HPLC analysis conditions: 1. Instruments: SHIMADZU LC-30AD SFC; 2. Chromatographic column: Chiralcel OX-3 50×4.6mm I.D., 3µm; 3. Mobile phase system: A for CO2, B for 0.05%DEA in MeOH; 4. Gradient: B 5%-40%; 5. Flow rate: 3 mL/min. Compounds **75F,** retention time 2.11 min; Compounds **75G,** retention time 2.24 min;

Chiral preparation method: instrument: Waters 150 MGM; chromatographic column: Chiralpak Column; mobile phase: A: carbon dioxide, and B: methanol (0.1% aqueous ammonia); isocratic elution: 35% mobile phase B; flow rate: 100 mL /min; back pressure: 100 bar; column temperature: 25°C; wavelength: 220 nm.

LC-MS (ESI): m/z =507.1 [M+H]⁺;

Step 6: Compound **75F** (200 mg, 0.394 mmol) was dissolved in dichloromethane (9 mL), and trifluoroacetic acid (3 mL) was added at room temperature. The reaction was continued for 1 h after the addition was completed. After the reaction was completed as monitored by TLC, the mixture was concentrated to obtain the crude trifluoroacetate salt of **75H,** which was directly used in the next reaction without purification.

LC-MS (ESI): m/z = 407.1 [M+H]⁺;

Using **75G** as the raw material, compound **75I** was obtained by referring to the above synthesis method.

Step 7: The crude trifluoroacetate salt of compound **75H** obtained in the previous step was dissolved in methanol (30 mL), and compound **2B** (101 mg, 0.788 mmol) and acetic acid (23.7 mg, 0.394 mmol) were added, after the obtained system was stirred at room temperature for 1 h, sodium cyanoborohydride (74.2 mg, 1.18 mmol) was added, and stirring was continued for 1 h. After the reaction was completed as monitored by TLC, water (1 mL) was added to quench the reaction, and the obtained system was directly concentrated under reduced pressure. The residue was purified by preparative silica gel plate (ethyl acetate:methanol (v/v)=90:10) to obtain **compound 75** isomer 1 (70 mg, two-step yield: 34.2%), and **compound 75** isomer 2 (88 mg, two-step yield: 43.0%).

**Compound 75** isomer 1: R_{f} = 0.60 (ethyl acetate: methanol (v/v)=90:10), ¹H NMR (400 MHz, DMSO-*d*₆) δ 12.15 (s, 1H), 7.62-7.61 (m, 1H), 7.50-7.49 (m, 1H), 7.43-7.38 (m, 1H), 7.22-7.21 (m, 1H), 6.61-6.53 (m, 1H), 6.21-6.20 (d, 1H), 5.88-5.87 (d, 1H), 4.88-4.76 (m, 1H), 2.76-2.52 (m, 4H), 2.47-2.37 (m, 2H), 1.89-1.73 (m, 3H), 1.72-1.55 (m, 3H), 1.50-1.49 (d, 3H), 1.46-1.35 (m, 1H), 1.35-1.20 (m, 4H).

LC-MS (ESI): m/z =519.1 [M+H]⁺;

**Compound 75** isomer 2: R_{f} = 0.40 (ethyl acetate: methanol (v/v)=90:10), ¹H NMR (400 MHz, DMSO-*d*₆) δ 7.63-7.62 (d, 1H), 7.50-7.49 (d, 1H), 7.43-7.37 (m, 1H), 7.22-7.21 (d, 1H), 6.60-6.55 (m, 1H), 6.22-6.21 (d, 1H), 5.87-5.86 (d, - 1H), 4.88-4.77 (m, 1H), 2.79-2.68 (m, 2H), 2.65-2.53 (m, 2H), 2.18-2.05 (m, 2H), 1.94-1.72 (m, 5H), 1.66-1.55 (m, 1H), 1.51-1.50 (d, 3H), 1.47-1.36 (m, 1H), 1.34 -1.20 (m, 4H).

LC-MS (ESI): m/z =519.1 [M+H]⁺;

Using **75I** as the raw material, **compound 75** isomer 3 (75 mg, two-step yield: 36.6%) and **compound 75** isomer 4 (85 mg, two-step yield: 41.5%) were obtained by referring to the above synthesis method.

**Compound 75** isomer 3: R_{f} = 0.65 (ethyl acetate: methanol (v/v)=90:10), ¹H NMR (400 MHz, DMSO-*d*₆) δ 12.14 (s, 1H), 7.61-7.60 (d, 1H), 7.51-7.50 (d, 1H), 7.44-7.37 (m, 1H), 7.23-7.22 (d, 1H), 6.61-6.54 (m, 1H), 6.21-6.20 (d, 1H), 5.88-5.87 (d, 1H), 4.87-4.77 (m, 1H), 2.76-2.52 (m, 4H), 2.47-2.39 (m, 2H), 1.88-1.55 (m, 6H), 1.51-1.50 (d, 3H), 1.47-1.35 (m, 1H), 1.34-1.21 (m, 4H).

LC-MS (ESI): m/z =519.1 [M+H]⁺;

**Compound 75** isomer 4: R_{f} = 0.50 (ethyl acetate: methanol (v/v)=90:10), ¹H NMR (400 MHz, DMSO-*d*₆) δ 7.62-7.61 (d, 1H), 7.50-7.49 (d, 1H), 7.43-7.37 (m, 1H), 7.23-7.22 (d, 1H), 6.636.51 (m, 1H), 6.23-6.22 (d, 1H), 5.87-5.86 (d, 1H), 4.89-4.76 (m, 1H), 2.82-2.68 (m, 2H), 2.66-2.54 (m, 2H), 2.19-2.06 (m, 2H), 1.96-1.73 (m, 5H), 1.68-1.56 (m, 1H), 1.50-1.49 (d, 3H), 1.46-1.35 (m, 1H), 1.34 -1.25 (m, 4H).

LC-MS (ESI): m/z =519.1 [M+H]⁺.

### Example 76:

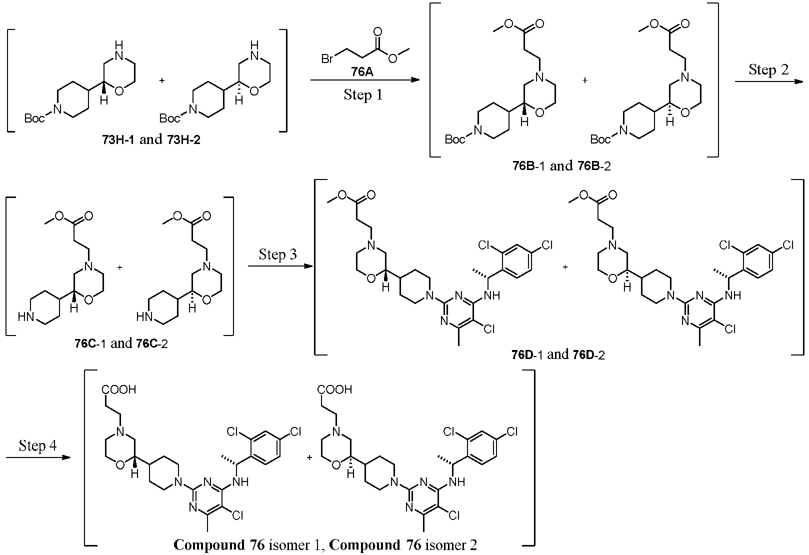

Step 1: Compound **76A**(135.9 mg, 0.81 mmol) and compound **73H**-1 (200 mg, 0.74mmol) were dissolved in acetonitrile (3.0 mL) at room temperature, and then triethylamine (149.7 mg, 1.48 mmol) was added, after the addition was completed, the obtained system was stirred at room temperature for 12 h. After the reaction was completed, the obtained system was directly concentrated and the residue was separated and purified by silica gel column chromatography (petroleum ether:ethyl acetate (v/v) = 2:1) to obtain the title compound **76B**-1 (180 mg, 68%).

Referring to the above operation, compound **76A** (135.9 mg, 0.81 mmol) and compound **73H**-2 (200 mg, 0.74 mmol) were used as the raw materials to obtain the title compound **76B**-2 (175 mg, 66%).

LCMS m/z = 357.3 [M+H]⁺.

Step 2: Compound **76B**-1 (180 mg, 0.50 mmol) was dissolved in dichloromethane (4.0 mL), trifluoroacetic acid (1.0 mL) was added, and the reaction was performed at room temperature for 2 h. After the reaction was completed, the mixture was directly concentrated to obtain the trifluoroacetate salt of compound **76C**-1 (120 mg, 93%).

Referring to the above operation, compound **76B**-2 (175 mg, 0.49 mmol) was used as the raw material to obtain the trifluoroacetate salt of the title compound **76C**-2 (115 mg, 91%).

LCMS m/z = 257.3 [M+H]⁺.

Step 3: Compound **2F** (82.2 mg, 0.23 mmol) and compound **76C**-1 (120 mg, 0.47 mmol) were dissolved in dimethyl sulfoxide (2.0 mL), and N,N-diisopropylethylamine (180.5 mg, 1.41 mmol) and cesium fluoride (144.8 mg, 0.94 mmol) were added, the temperature was raised to 100°C and the reaction was performed for 5 h. After the reaction was completed, the mixture was cooled to room temperature, and water (2.0 mL) was added, followed by extraction with ethyl acetate (5.0 mL ×3). The combined organic phases were washed with saturated brine (2.0 mL), dried over anhydrous sodium sulfate, and filtered. The filtrate was concentrated and the residue was separated and purified by silica gel column chromatography (petroleum ether:ethyl acetate (v/v) = 1:1) to obtain the title compound **76D**-1 (150 mg, 56%).

Referring to the above operation, compound **76C**-2 (115 mg, 0.45 mmol) was used as the raw material to obtain the title compound **76D**-2 (136 mg, 53%).

LCMS m/z = 570.0 [M+H]⁺.

Step 4: Compound **76D**-1 (150 mg, 0.26 mmol) was dissolved in methanol (4 mL) and water (1 mL) at room temperature, lithium hydroxide (32.8 mg, 0.78 mmol) was added, after the addition was completed, the obtained system was stirred at room temperature for 4 h. After the reaction was completed, the solvent was dried by spinning, water (2.0 mL) was added, 1M HCl was added dropwise to adjust the pH to 6-7, and the mixture was extracted with dichloromethane (5.0 mL×3). The combined organic phases were washed with saturated brine (2.0 mL), dried over anhydrous sodium sulfate, and filtered, and the filtrate was concentrated. The residue was separated and purified by silica gel column chromatography (dichloromethane:methanol (v/v) = 9:1) to obtain the title compound **76** isomer 1 (Rf = 0.3 (dichloromethane:methanol (v/v) = 9:1), 100 mg, 68%).

Referring to the above operation, compound **76D**-2 (136 mg, 0.24 mmol) was used as the raw material to obtain the title **compound 76** isomer 2 (Rf=0.3 (dichloromethane:methanol (v/v)= 9:1), 90 mg, 68%).

**Compound 76** isomer 1: ¹H NMR (400 MHz, Methanol-*d*₄) δ 7.43-7.40 (m, 1H), 7.35-7.32 (m, 1H), 7.25-7.22 (m, 1H), 5.43-5.37 (m, 1H), 4.54-4.45 (m, 2H), 4.03-3.98 (m, 1H), 3.72-3.64 (m, 1H), 3.37-3.32 (m, 1H), 3.26-3.20 (m, 2H), 3.14-3.07 (m, 2H), 2.75-2.66 (m, 1H), 2.65-2.59 (m, 1H), 2.59-2.49 (m, 4H), 2.25 (s, 3H), 1.74-1.62 (m, 2H), 1.52-1.48 (m, 3H), 1.44-1.37 (m, 1H), 1.19-1.08 (m, 1H), 0.93-0.81 (m, 1H).

LCMS m/z = 556.4 [M+H]⁺.

**Compound 76** isomer 2: ¹H NMR (400 MHz, Methanol-*d*₄) δ 7.43-7.40 (m, 1H), 7.35-7.32 (m, 1H), 7.25-7.22 (m, 1H), 5.45-5.39 (m, 1H), 4.57-4.48 (m, 2H), 4.02-3.97 (m, 1H), 3.71-3.64 (m, 1H), 3.31-3.26 (m, 1H), 3.20-3.12 (m, 2H), 3.07-3.00 (m, 2H), 2.66-2.58 (m, 3H), 2.55-2.51 (m, 2H), 2.46-2.38 (m, 1H), 2.27 (s, 3H), 1.69-1.60 (m, 2H), 1.57-1.54 (m, 1H), 1.53- 1.50 (m, 3H), 1.24-1.14 (m, 1H), 0.87-0.74 (m, 1H).

LCMS m/z = 556.4 [M+H]⁺.

### Example 77:

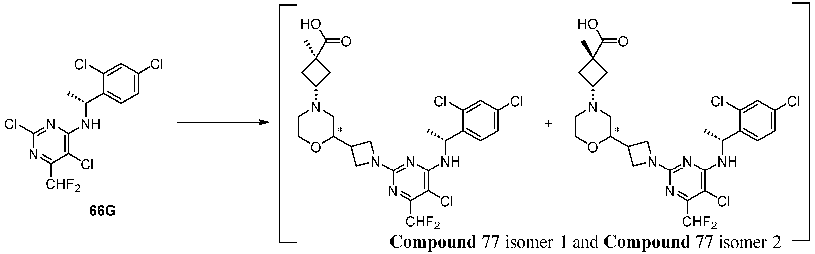

Compound **9J-2** (61 mg, 0.24 mmol), DIEA (210 mg, 1.59 mmol) and cesium fluoride (72 mg, 0.48 mmol) were added to a solution of compound **66G** (100 mg, 0.24 mmol) in DMSO (4 mL) in sequence, the temperature was raised to 100°C and reaction was performed for 2 h. After the reaction was completed, water (15 mL) and ethyl acetate (15 mL) were added, extraction and separation were performed. The aqueous phase was extracted with ethyl acetate (15 mL ×2). The combined organic phases were washed with saturated brine (15 mL), dried over anhydrous sodium sulfate, filtered, and concentrated. The residue was purified by silica gel column chromatography (EA:MeOH (v/v) = 5:1) to obtain **compound 77** isomer 1 (Rf = 0.5, (EA:MeOH (v/v) = 3:1), 56 mg, 39%) and **compound 77** isomer 2 (Rf = 0.4, (EA:MeOH (v/v) = 3:1), 23 mg, 16%).

**Compound 77** isomer 1: ¹H NMR (400 MHz, CDCl₃) δ 7.36 (d, 1H), 7.22 (d, 1H), 7.21-7.18 (m, 1H), 6.50 (t, 1H), 5.71 (d, 1H), 5.48-5.41 (m, 1H), 3.98 (t, 1H), 3.87-3.81 (m, 3H), 3.78-3.73 (m, 3H), 3.04-2.95 (m, 2H), 2.90-2.84 (m, 1H), 2.64-2.55 (m, 3H), 2.19-2.03 (m, 3H), 1.53 (d, 3H), 1.42 (s, 3H).

LCMS (ESI): m/z =604.9 [M+H]⁺.

**Compound 77** isomer 2: ¹H NMR (400 MHz, CDCl₃)δ 7.37 (d, 1H), 7.22 (d, 1H), 7.20-7.17 (m, 1H), 6.51 (t, 1H), 5.71 (d, 1H), 5.48-5.41 (m, 1H), 4.00 (t, 1H), 3.94-3.77 (m, 4H), 3.67-3.58 (m, 2H), 2.89-2.79 (m, 1H), 2.69-2.67 (m, 2H), 2.63-2.56 (m, 3H), 2.01 -1.82 (m, 3H), 1.53 (d, 3H), 1.42 (s, 3H).

LCMS (ESI): m/z =604.9 [M+H]⁺.

### Example 78:

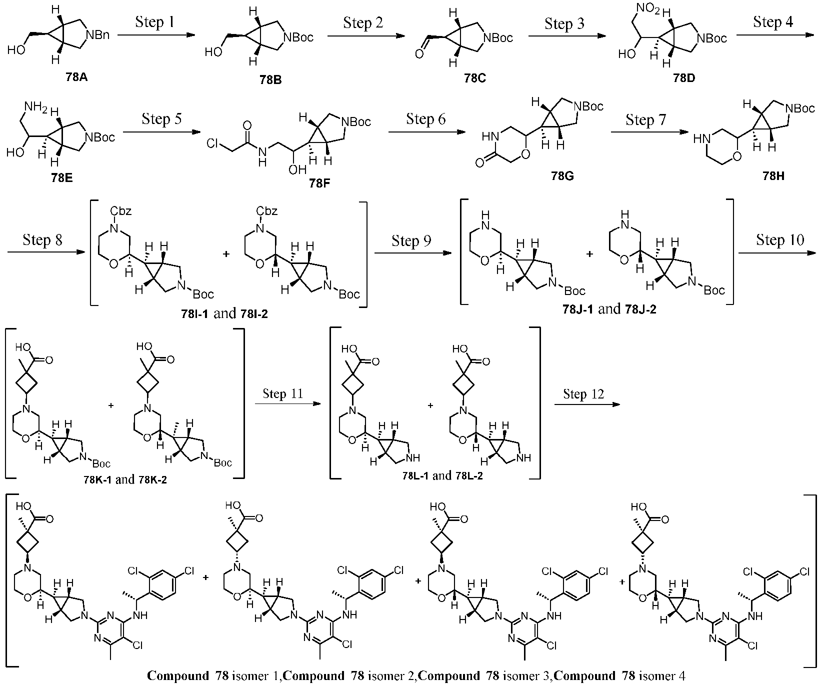

Step 1: Compound **78A** (30.0 g, 147.6 mmol) was dissolved in methanol (300 mL), and di-tert-butyl dicarbonate (38.6 g, 177.1 mmol) and Pd/C (3.0 g, Pd content 10%) were added in sequence. After the addition was completed, the obtained system was stirred at room temperature for 16 h under a hydrogen atmosphere. After the reaction was completed, the mixture was filtered, the filtrate was concentrated, and the residue was separated and purified by silica gel column chromatography (petroleum ether:ethyl acetate (v/v) = 2:1) to obtain the title compound **78B** (15.0 g 48%).

LCMS m/z=158.2[M+H-tBu]⁺.

Step 2: Compound **78B** (15.0 g, 70.3 mmol) was dissolved in ethyl acetate (200 mL), and 2-iodoacylbenzoic acid (41.3 g, 147.6 mmol) was added. After the addition was completed, the obtained system was stirred at 80°C for 12 h. After the reaction was completed, the mixture was cooled to room temperature and filtered. The filtrate was concentrated under reduced pressure to obtain the crude product of the title compound **78C** (13.0 g, 87%).

LCMS m/z=156.1[M+H-tBu]⁺.

Step 3: Compound **78C** (13.0 g, 61.5 mmol) was dissolved in a mixed solvent of methanol (130 mL) and nitromethane (130 mL), and triethylamine (12.4 g, 123 mmol) was added. The mixture was stirred at room temperature for 17 h after the addition was completed. After the reaction was completed, the mixture was concentrated directly and the residue was separated and purified by silica gel column chromatography (petroleum ether:ethyl acetate (v/v) = 1:1) to obtain the title compound **78D** (7.0 g, 42%).

LCMS m/z=217.1[M+H-tBu]⁺.

Step 4: Compound **78D** (7.0 g, 25.7 mmol) was dissolved in a mixed solvent of methanol (70 mL) and water (10 mL), and ammonium chloride (16.3 g, 308.4 mmol) was added. Then, zinc powder (16.7 g, 257 mmol) was slowly added at 0°C. After the addition was completed, the mixture was heated to room temperature and reacted for 2 h. After the reaction was completed, the mixture was filtered and the filtrate was concentrated to obtain the crude product of compound **78E** (6.0 g, 96%), which was directly used in the next reaction without further purification.

LCMS m/z=187.2[M+H-tBu]⁺.

Step 5: Compound **78E** (6.0 g, 24.8 mmol) was dissolved in a mixed solvent of ethyl acetate (60 mL) and water (60 mL), and sodium bicarbonate (6.2 g, 74.4 mmol) was added. The obtained system was cooled to 5°C, and chloroacetyl chloride (4.2 g, 37.2 mmol) was added dropwise, and the temperature was controlled at 5-10°C. After the dropwise addition was completed, the obtained system was warmed to room temperature and reacted for 1 h. After the reaction was completed, water (30 mL) was added, and ethyl acetate (30 mL×2) was used for extraction. The combined organic phase was washed with saturated brine (30 mL), dried over anhydrous sodium sulfate, filtered, and concentrated. The residue was separated and purified by silica gel column chromatography (petroleum ether:ethyl acetate (v/v) = 1:2) to obtain the title compound **78F** (5.5 g, 70%).

LCMS m/z =245.2[M+H-Boc-H₂O]⁺.

Step 6: Potassium tert-butoxide (3.5 g, 31.4 mmol) was dissolved in tert-butanol (50 mL), a solution of compound **78F** (5.5 g, 15.7 mmol) in tert-butanol (50 mL) was added, after the addition was completed, the temperature was controlled at 30-40°C and the reaction was performed for 2 h. After the reaction was completed, the mixture was cooled to room temperature, saturated aqueous ammonium chloride solution (30 mL) was added to quench the reaction, and the mixture was extracted with ethyl acetate (30 mL×2). The combined organic phases were washed with saturated brine (30 mL), dried over anhydrous sodium sulfate, and filtered. The filtrate was concentrated and the residue was separated and purified by silica gel column chromatography (petroleum ether:ethyl acetate (v/v) = 1:2) to obtain the title compound **78G** (4.5 g, 92%).

LCMS m/z =227.2[M+H-Boc]⁺.

Step 7: Compound **78G** (4.5 g, 15.9 mmol) was dissolved in toluene (45 mL) under nitrogen atmosphere, the obtained system was cooled to -10°C, and sodium 2-hydrobis(dimethoxyethoxy)aluminate (13.6 mL, 3.5 mol/L solution in toluene) was slowly added dropwise. After the dropwise addition was completed, the reaction was continued at -10°C for 3 h. After the reaction was completed, 10% aqueous sodium hydroxide solution (10 mL) was slowly added dropwise to quench the reaction. After quenching, ethyl acetate (30 mL) was added to the reaction liquid for extraction. The organic phase was washed with water (20 mL), washed with saturated brine (20 mL), dried over anhydrous sodium sulfate, and filtered, and the filtrate was concentrated under reduced pressure to obtain **78H** (3.8 g, 89%).

LCMS m/z =213.4[M+H-tBu]⁺.

Step 8: Compound **78H** (3.8 g, 14.2 mmol) was dissolved in a mixed solvent of tetrahydrofuran (30 mL) and water (30 mL), and sodium bicarbonate (3.6 g, 42.6 mmol) was added. The obtained sytem was cooled to 0-5°C, and benzyl chloroformate (2.9 g, 17.0 mmol) was slowly added dropwise. After the dropwise addition was completed, the reaction was continued at 0-5°C for 1 h. After the reaction was completed, water (10 mL) was added and ethyl acetate (30 mL×2) was used for extraction. The combined organic phase was washed with saturated brine (20 mL), dried over anhydrous sodium sulfate, and filtered, and the filtrate was concentrated. The residue was separated and purified by silica gel column chromatography (petroleum ether:ethyl acetate (v/v) = 3:1) to obtain 4.2 g of the racemate. The racemate was separated by chiral preparative HPLC to obtain two isomers, compound **78I-1** (1.9 g, retention time 4.02 min, 33%) and compound **781-2** (1.9 g, retention time 4.53 min, 33%).

HPLC analytical method: 1. Instruments: Waters UPC2 analytical SFC (SFC-H); 2. Chromatographic column: ChiralPak IC, 100×4.6mm I.D., 3µm; 3. Mobile phase system A for CO2 and B for Ethanol (0.05% DEA); 4. Gradient: B 40%; 5. Flow rate: 3 mL/min.

LCMS m/z =303.2 [M+H-Boc]⁺.

Preparative chromatography separation conditions: 1. Instruments: Waters 150 SFC; 2. Chromatographic column: ChiralPak IC, 250×30 mm I.D., 10 µm.; 3. Mobile phase system: A for CO₂ and B for Ethanol; 4. Gradient: B 25%; 5. Flow rate: 80 mL/min.

Step 9: Compound **78I-1** (1.0 g, 2.48 mmol) was dissolved in ethyl acetate (10 mL), and Pd/C (0.2 g, Pd content 10%) was added. The reaction was carried out under a hydrogen atmosphere for 3 h. After the reaction was completed, the obtained system was filtered and the filtrate was concentrated to obtain the title compound **78J-1** (0.63 g, 94.7%).

Referring to the above operation, compound **78I-2** (1 g, 2.48 mmol) was used as the raw material to obtain the title compound **78J-2** (0.61 g, 91.7%).

LC-MS (ESI): m/z =213.2 [M+H]⁺.

Step 10: Compound **78J-1** (0.63 g, 2.35 mmol) and 3-carbonyl-1-methyl-cyclobutanecarboxylic acid (0.45 g, 3.50 mmol) were dissolved in methanol (15 mL) in sequence, and glacial acetic acid (0.14 g, 2.35 mmol) was added, and sodium cyanoborohydride (0.73 g, 11.69 mmol) was added in portions, after the addition was completed, the reaction was performed for 1 h. After the reaction was completed, water (30 mL) was added to quench the reaction, and dichloromethane (50 mL×2) was used for extraction. The combined organic phase was washed with saturated brine, dried over anhydrous sodium sulfate, and filtered. The filtrate was concentrated and the residue was separated and purified by silica gel column chromatography (dichloromethane:methanol (v/v) = 10:1) to obtain the title compound **78K-1** (0.75 g, 83.9%).

Referring to the above operation, compound **78J-2** (0.61 g, 2.27 mmol) was used as the raw material to obtain the title compound **78K-2** (0.73 g, 84.5%).

LC-MS (ESI): m/z =325.2 [M+H]⁺.

Step 11: Compound **78K-1** (0.3 g, 0.79 mmol) was dissolved in dichloromethane (3 mL), trifluoroacetic acid (1 mL) was added, and the reaction was performed at room temperature for 30 min. After the reaction was completed, the obtained system was directly concentrated to obtain the trifluoroacetate salt of compound **78L-1** (0.3 g, 100%).

Referring to the above operation, compound **78K-2** (0.3 g, 0.79 mmol) was used as the raw material to obtain the trifluoroacetate salt of the title compound **78L-2** (0.31 g, 100%).

LC-MS (ESI): m/z =281.2 [M+H]⁺.

Step 12: Compound 2F (0.27 g, 0.79 mmol), DIEA (0.3 g, 2.23 mmol) and cesium fluoride (0.25 g, 0.79 mmol) were added to a solution of compound **78L-1** (0.3 g, 0.79 mmol) in DMSO (4 mL) in sequence, the temperature was raised to 100°C and the reaction was performed for 2 h. After the reaction was completed, water (15 mL) and ethyl acetate (15 mL) were added, extraction and separation were performed. The aqueous phase was extracted with ethyl acetate (15 mL×2). The combined organic phases were washed with saturated brine, dried over anhydrous sodium sulfate, filtered, and concentrated. The residue was purified by silica gel column chromatography (EA:MeOH(v/v)=5:1) to obtain **compound 78** isomer 1 (Rf=0.5, (EA:MeOH(v/v)=3:1), 102 mg, 22.12%) and **compound 78** isomer 2 (Rf=0.4, (EA:MeOH(v/v)=3:1), 50 mg, 11.06%).

**Compound 78** isomer 1: ¹H NMR (400 MHz, CDCl₃) δ 7.36 (d, 1H), 7.22 (d, 1H), 7.17-7.15 (m, 1H), 5.47-5.41 (m, 2H), 3.87 (d, 1H), 3.72 (d, 1H), 3.59 (t, 2H), 3.37-3.34 (m, 1H), 2.99 (t, 1H), 2.86-2.81 (m, 2H), 2.70-2.55 (m, 4H), 2.27 (s, 3H), 2.04- 1.84 (m, 4H), 1.78 (t, 1H), 1.65-1.61 (m, 1H), 1.49 (d, 3H), 1.41 (s, 3H).

LCMS (ESI): m/z =594.2 [M+H]⁺.

**Compound 78** isomer 2: ¹H NMR (400 MHz, CDCl₃) δ = 7.37 (d, 1H), 7.22 (d, 1H), 7.18-7.15 (m, 1H), 5.47-5.41 (m, 2H), 3.99-3.90 (m, 1H), 3.73 (d, 2H), 3.37-3.34 (m, 1H), 3.16-3.11 (m, 3H), 3.00 (d, 1H), 2.91-2.87 (m, 1H), 2.52-2.50 (m, 2H), 2.28 (s, 3H), 2.21-2.09 (m, 4H), 1.91 (t, 2H), 1.69-1.64 (m, 2H), 1.57-1.53 (m, 1H), 1.50 (d, 3H), 1.40 (s, 3H).

LCMS (ESI): m/z =594.2 [M+H]⁺.

Referring to the above operation, compound **78L-2** was used as the raw material (0.1 g, 0.27 mmol) to obtain the title **compound 78** isomer 3 (Rf=0.4, (EA:MeOH(v/v)=3:1), 98 mg, 21.67%) and **compound 78** isomer 4 (Rf=0.3, (EA:MeOH(v/v)=3:1), 33 mg, 7.3%).

**Compound 78** isomer 3:¹H NMR (400 MHz, CDCl₃) δ 7.35 (d, 1H), 7.21 (d, 1H), 7.16-7.14 (m, 1H), 5.43-5.41 (m, 2H), 3.87 (d, 1H), 3.79 (d, 1H), 3.59 (t, 2H), 3.34-3.30 (m, 1H), 2.97 (t, 1H), 2.86-2.77 (m, 2H), 2.65-2.58 (m, 4H), 2.27 (s, 3H), 2.02-1.86 (m, 4H), 1.77 (t, 1H), 1.64-1.60 (m, 1H), 1.49 (d, 3H), 1.42 (s, 3H).

LCMS (ESI): m/z =594.2 [M+H]⁺.

**Compound 78** isomer 4: ¹H NMR (400 MHz, CDCl₃) δ 7.35 (d, 1H), 7.21 (d, 1H), 7.17-7.15 (m, 1H), 5.44-5.40 (m, 2H), 3.95 (d, 1H), 3.80 (d, 1H), 3.72 (t, 1H), 3.36-3.32 (m, 1H), 3.16-3.10 (m, 3H), 3.02 (d, 1H), 2.93-2.88 (m, 1H), 2.55-2.50 (m, 2H), 2.29 (s, 3H), 2.20-2.11 (m, 4H), 1.92-1.91 (t, 2H), 1.68-1.64 (m, 1H), 1.56-1.53 (m, 2H), 1.50 (d, 3H), 1.41 (s, 3H).

LCMS (ESI): m/z =594.2 [M+H]⁺.

### Example 79:

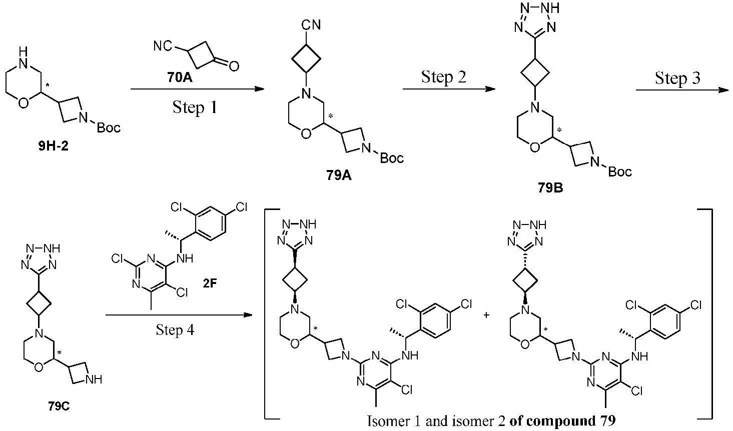

Step 1: Compound **9H-2** (1.50 g, 6.19 mmol) was dissolved in dry methanol (40 mL), and 3-oxocyclobutanenitrile (706 mg, 7.43 mmol) and acetic acid (372 mg, 6.19 mmol) were added in sequence, after the obtained system was stirred at room temperature for 1 h, sodium cyanoborohydride (778 mg, 12.4 mmol) was added, and stirring was continued for 3 h. After the reaction was completed as monitored by TLC, water (100 mL) was added to quench the reaction, and the mixture was extracted with ethyl acetate (50 mL × 8). The organic phases were combined and dried over anhydrous sodium sulfate. After filtration and concentration, the crude product was purified by silica gel column chromatography (dichloromethane:methanol (v/v) = 75:25) to obtain compound **79A** (1.38 g, 69.3%).

LC-MS (ESI): m/z =322.2 [M+H]⁺.

Step 2: Compound **79A** (1.38 g, 4.29 mmol), ammonium chloride (1.15 g, 21.5 mmol), and sodium azide (1.40 g, 21.5 mmol) were added to dry N, N-dimethylformamide (30 mL) in sequence, the temperature was raised to 140°C and the reaction was performed for about 5 h. After the disappearance of the raw materials as monitored by TLC, the mixture was cooled to room temperature and directly concentrated under reduced pressure. The residue was purified by silica gel column chromatography (dichloromethane:methanol (v/v) = 80:20 to 60:40) to obtain a crude product of **79B** (1.35 g).

LC-MS (ESI): m/z =365.2 [M+H]⁺.

Step 3: **79B** (1.35 g) was dissolved in dry dichloromethane (30 mL), trifluoroacetic acid (10 mL) was added at room temperature, and the reaction was continued for 1 h after addition was completed. After the reaction was completed as monitored by LCMS, the product was concentrated to obtain the crude trifluoroacetate salt of **79C** (1.45 g), which was used in the next step without purification.

LC-MS (ESI): m/z = 265.2 [M+H]⁺.

Step 4: The crude trifluoroacetate salt of **79C** (1.45 g), compound **2F** (350 mg, 0.997 mmol), N,N-diisopropylethylamine (772 mg 5.98 mmol) and cesium fluoride (303 mg, 1.99 mmol) were added to a dry dimethyl sulfoxide (20 mL), and the temperature was raised to 100°C and the reaction was performed for about 2 h. After the disappearance of the raw materials as monitored by TLC, the mixture was cooled to room temperature and concentrated under reduced pressure. The residue was separated and purified by silica gel column chromatography (dichloromethane:methanol (v/v)=90:10) to obtain a mixture of isomers 1 and 2 of **compound 79** (310 mg, three-step yield: 12.4%).

After further separation and purification by preparative HPLC, **compound 79** isomer 1 (26 mg, three-step yield: 1.05%) and **compound 79** isomer 2 (23 mg, three-step yield: 0.924%) were obtained.

Preparative HPLC separation and purification method: 1. Instruments: waters 2767 preparative liquid chromatographic instrument; chromatographic column: SunFire @ Prep C18 (19 mm × 250 mm). 2. The sample was filtered with a 0.45 µm filter head to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 5%-50%; c. flow rate: 12 mL/min; d. elution time: 30 min.

HPLC analysis conditions: 1. Instruments: Shimadzu LC-20AT; 2. Chromatographic column: Xtimate C18 4.6*50mm, 3µm; 3. Mobile phase system: A for 0.05% TFA in H2O; B for ACN; 4. Gradient: B 5-95%; 5. Flow rate: 1.0 mL/min, run time: 10 min. retention time: **Compound 79** isomer 1: tR = retention time: 3.226 min; **Compound 79** isomer 2: tR = retention time: 3.201 min.

**Compound 79** isomer 1: ¹H NMR (400 MHz, Methanol-d₄) δ 7.43-7.42 (d, 1H), 7.36-7.35 (d, 1H), 7.27-7.26 (d, 1H), 5.50-5.49 (t, 1H), 4.60 (s, 1H), 4.06-3.79 (m, 4H), 3.79-3.58 (m, 4H), 3.44-3.43 (d, 1H), 2.98-2.97 (t, 2H), 2.74-2.56 (m, 3H), 2.57-2.42 (m, 2H), 2.37 -2.43 (m, 4H), 1.96-1.95 (t, 1H), 1.51-1.49 (d, 3H);

LC-MS (ESI): m/z =578.2 [M+H]⁺;

**Compound 79** isomer 2: ¹H NMR (400 MHz, Methanol-d₄) δ 7.42-7.41 (d, 1H), 7.36-7.35 (d, 1H), 7.25-7.24 (d, 1H), 5.49-5.48 (q, 1H), 4.07-3.79 (m, 4H), 3.80-3.49 (m, 4H), 3.26-3.25 (d, 1H), 3.09-2.97 (m, 2H), 2.78-2.65 (m, 3H), 2.48-2.22 (m, 6H), 2.05-2.04 (t, 1H), 1.51-1.50 (d, 3H).

LC-MS (ESI): m/z =578.2[M+H]⁺.

### Example 80:

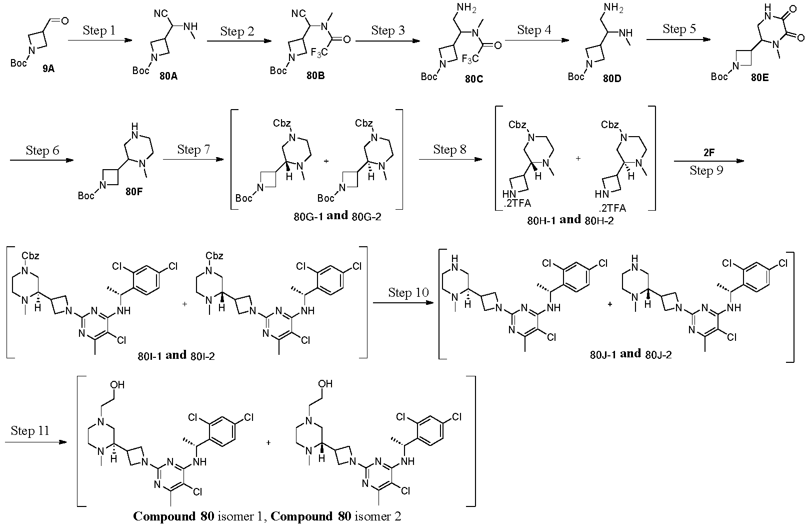

Step 1: Compound **9A** (65 g, 350.92 mmol) was dissolved in methylamine ethanol solution (30%, 300 mL), the obtained system was stirred at room temperature for 0.5 h, and cooled to 0°C, and trimethylsilyl cyanide (34.8 g, 350.92 mmol) was added dropwise, after the dropwise addition was completed the reaction was performed at room temperature for 16 h After the reaction was completed, the mixture was concentrated, water (300 mL) was added, and the mixture was extracted with dichloromethane (200 mL × 2). The combined organic phases were washed with saturated brine, dried over anhydrous sodium sulfate, filtered, and concentrated to obtain a crude compound **80A** (80 g, 100%).

LCMS (ESI): m/z =170.2 [M+H-tBu]⁺.

Step 2: Compound **80A** (80 g, 350.93 mmol) was dissolved in dichloromethane (400 mL), triethylamine (106.5 g, 1052.76 mmol) was added, and trifluoroacetic anhydride (73.7 g, 350.93 mmol) was added dropwise at room temperature, and the reaction was continued for 1 h after the dropwise addition was completed. Water (300 mL) was added and extraction was performed. The aqueous phase was extracted with dichloromethane (200 mL×2). The combined organic phases were washed with saturated brine, dried over anhydrous sodium sulfate, filtered, and concentrated to obtain crude compound **80B** (100 g, 88.6%).

LCMS (ESI): m/z =266.1 [M+H-tBu]⁺.

Step 3: Compound **80B** (100g, 311.24 mmol) was dissolved in methanol (1000 mL), Raney nickel (20 g) was added, and the reaction was performed at room temperature under a hydrogen atmosphere for 16 h. After the reaction was completed, the crude product **80C** (90 g, 88.9%) was obtained by filtration and concentration.

LCMS (ESI): m/z =270.4 [M+H-tBu]⁺.

Step 4: Compound **80C** (90 g, 276.64 mmol) was dissolved in a mixed solvent of methanol (500 mL) and water (500 mL), sodium hydroxide (33.2 g, 830.17 mmol) was added, and the reaction was performed at room temperature for 2 h. After the reaction was completed, the mixture was extracted with dichloromethane (500 mL×3). The combined organic phases were dried over anhydrous sodium sulfate, filtered, and concentrated to obtain a crude compound **80D** (60 g, 94.5%).

LCMS (ESI): m/z =230.3 [M+H]⁺.

Step 5: Compound **80D** (60 g, 261.64 mmol) was dissolved in ethanol (500 mL), dimethyl oxalate (30.9 g, 261.64 mmol) was added, and after the addition was completed, the temperature was raised to reflux and the reaction was performed for 16 h. After the reaction was completed, the mixture was cooled to room temperature, and concentrated, and water (500 mL) was added. The mixture was extracted with dichloromethane (500 mL×3). The combined organic phases were dried over anhydrous sodium sulfate, filtered, and concentrated to obtain the crude compound **80E** (40 g, 53.9%).

LCMS (ESI): m/z =228.2 [M+H-tBu]⁺.

Step 6: Compound **80E** (40 g, 141.18 mmol) was dissolved in tetrahydrofuran (800 mL), lithium aluminum hydride (13.4 g, 352.98 mmol) was added in portions at 0°C, after the addition was completed, the reaction was continued at 0°C for 1 h. After the reaction was completed, water (13.4 mL), 10% aqueous sodium hydroxide solution (13.4 mL), and water (40.2 mL) were added dropwise in sequence. After the dropwise addition was completed, anhydrous magnesium sulfate was added and stirred for 0.5 h. After quenching, the mixture was filtered and the filtrate was concentrated to obtain the crude compound **80F** (32 g, 88.7%).

LCMS (ESI): m/z =256.3 [M+H]⁺.

Step 7: Compound **80F** (32 g, 125.31 mmol) was dissolved in tetrahydrofuran (300 mL), water (13.4 mL) and sodium bicarbonate (31.6 g, 375.93 mmol) were added, and benzyl chloroformate (21.38 g, 125.31 mmol) was added dropwise at 0°C, after the dropwise addition was completed, the reaction was performed at room temperature for 1 h. Water (500 mL) was added, and the mixture was extracted with dichloromethane (500 mL×3). The combined organic phase was dried over anhydrous sodium sulfate and filtered. The filtrate was concentrated and subjected to silica gel column chromatography (dichloromethane:methanol (v/v) = 10:1) to obtain 4 g of the **80F** racemate. The racemate was separated by chiral preparative HPLC to obtain two isomers, compound **80G-1** (1.1 g, retention time 0.753 min, 4.5%) and compound **80G-2** (0.9 g, retention time 0.942 min, 3.6%).

HPLC analytical method: 1. Instruments: SHIMADZU LC-30AD SFC; 2. Chromatographic column: Chiralcel OX-3 50×4.6mm I.D., 3µm; 3. Mobile phase system: A for CO2, B for 0.05%DEA in MeOH; 4. Gradient: B 5%-40%; 5. Flow rate: 3 mL/min.

Preparative chromatography separation conditions: 1. Instruments: Waters 150 SFC; 2. Chromatographic column: Chiralpak IG Column (250 * 30 mm, I.D 30 mm, 10 um particle size) 3. Mobile phase system: A for CO₂ and B for MeOH +CAN (0.1%NH₃•H₂O); 4. Gradient: B 45%; 5. Flow rate: 180 mL/min.

LCMS (ESI): m/z =334.4 [M+H-tBu]⁺.

Step 8: Compound **80G-1** (0.4 g, 1.03 mmol) was dissolved in dichloromethane (5 mL), trifluoroacetic acid (1 mL) was added, and the reaction was performed at room temperature for 1 h. The title compound **80H-1** (320 mg, 100%) was obtained by concentration.

Referring to the above operation, compound **80G-2** (0.4 g, 1.03 mmol) was used as the raw material to obtain the title compound **80H-2** (330 mg, 100%).

LCMS (ESI): m/z =290.4 [M+H]⁺.

Step 9: Compound **80H-1** (320 mg, 1.03 mmol) was dissolved in dimethyl sulfoxide (5 mL), compound **2F** (358 mg, 1.03 mmol), triethylamine (312 mg, 3.09 mmol) and cesium fluoride (313 mg, 2.06 mmol) were added in sequence, the temperature was raised to 100°C and the reaction was performed for 6 h. After the reaction was completed, the mixture was cooled to room temperature, water (10 mL) was added, and the mixture was extracted with ethyl acetate (10 mL×3). The combined organic phases were dried over anhydrous sodium sulfate and filtered. The filtrate was concentrated and separated by silica gel column chromatography (dichloromethane:methanol (v/v) = 10:1) to obtain the title compound **80I-1** (400 mg, 64.5%).

Referring to the above operation, compound **80H-2** (0.4 g, 1.03 mmol) was used as the raw material to obtain the title compound **80I-2** (410 mg, 64.5%).

LCMS (ESI): m/z =603.3 [M+H]⁺.

Step 10: Compound **80I-1** (400 mg, 0.66 mmol) was dissolved in methanol (10 mL), Pd/C (0.8 g, Pd content 10%) was added, and the reaction was carried out under a hydrogen atmosphere for 16 h. Filtration was performed and the filtrate was concentrated to obtain the title compound **80J-1** (300 mg, 97.4%).

Referring to the above operation, compound **80I-2** (410 mg, 0.68 mmol) was used as the raw material to obtain the title compound **80J-2** (320 mg, 100%).

LCMS (ESI): m/z =469.4 [M+H]⁺.

Step 11: Compound **80J-1** (300 mg, 0.64 mmol) was dissolved in acetonitrile (10 mL), and 2-bromoethane-1-ol (158 mg, 1.28 mmol) and triethylamine (194 mg, 1.92 mmol) were added, the reaction was performed at 50°C for 16 h. After concentration, the obtained system was directly separated and purified using a liquid phase preparative column (liquid phase preparation conditions: C18 reverse phase preparative column, mobile phase was deionized water containing 0.1% aqueous ammonia (A), acetonitrile (B), gradient elution, B content = 5%-50%, elution time 15min, flow rate 12 mL/min, column temperature: 30°C) to obtain the title **compound 80** isomer 1 (50 mg, yield: 15.2%).

**Compound 80** isomer 1: ¹H NMR (400 MHz, DMSO-d₆) δ 7.50-7.41 (m, 2H), 7.40-7.30 (m, 2H), 5.47-5.23 (m, 1H), 4.44-3.91 (m, 2H), 3.78-3.53 (m, 2H), 3.52-3.39 (m, 2H), 2.87-2.51 (m, 5H), 2.45-2.28 (m, 2H), 2.27-1.89 (m, 9H), 1.86-1.60 (m, 1H), 1.46 (d, 3H).

LC-MS (ESI): m/z =513.3 [M+H]⁺.

Referring to the above operation, compound **80J-2** (320 mg, 0.68 mmol) was used as the raw material to obtain the title **compound 80** isomer 2 (55 mg, yield: 15.8%).

**Compound 80** isomer 2: ¹H NMR (400 MHz, DMSO-*d*₆) δ 7.58-7.43 (m, 2H), 7.42 -7.21 (m, 2H), 5.61-5.24 (m, 1H), 4.55-4.19 (m, 1H), 3.94-3.75 (m, 2H), 3.76-3.61 (m, 1H), 3.55-3.44 (m, 2H), 2.75-2.51 (m, 5H), 2.42-2.28 (m, 2H), 2.27-1.89 (m, 9H), 1.77 -1.65 (m, 1H), 1.44 (d, 3H).

LC-MS (ESI): m/z =513.3 [M+H]⁺.

### Example 81:

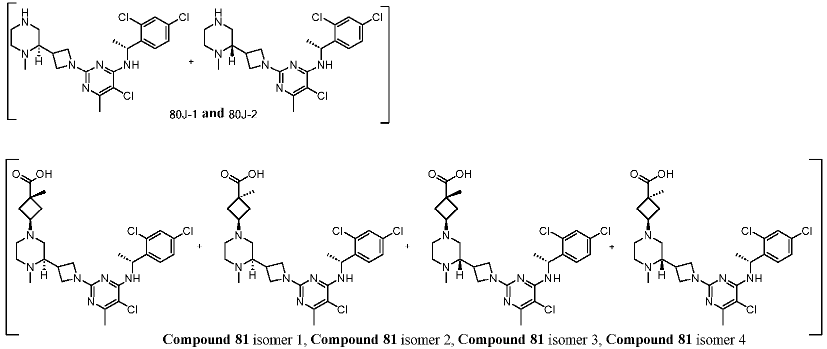

Compound **80J-1** (300 mg, 0.64 mmol) was dissolved in ethanol (10 mL), 1-methyl-3-oxocyclobutane-1-carboxylic acid (82 mg, 0.64 mmol) and glacial acetic acid (6 drops) were added, and after stirring for 0.5 hour, sodium cyanoborohydride (80 mg, 1.28 mmol) was added and the obtained system was reacted at room temperature for 1 h. After concentration, the title **compound 81** isomer 1 (60 mg, 16.2%, retention time: 2.325 min) and the title **compound 81** isomer 2 (55 mg, 14.8%, retention time: 2.629 min) were obtained by chiral preparation.

HPLC analytical method: 1. Instruments: SHIMADZU LC-30AD SFC; 2. Chromatographic column: Chiralcel OX-3 50×4.6mm I.D., 3µm; 3. Mobile phase system: A for CO2, B for 0.05%DEA in MeOH; 4. Gradient: B 5%-40%; 5. Flow rate: 3 mL/min.

Chiral preparation method: instrument: Waters 150 MGM; chromatographic column: Chiralpak Column; mobile phase: A: carbon dioxide, and B: Isopropanol (0.1% aqueous ammonia); isocratic elution: 30% mobile phase B; flow rate: 100 mL /min; back pressure: 100 bar; column temperature: 25°C; wavelength: 220 nm; elution time: 20 min.

**Compound 81** isomer 1: ¹H NMR (400 MHz, DMSO-*d*₆) δ 7.57-7.40 (m, 2H), 7.39-7.27 (m, 2H), 5.47- 5.26 (m, 1H), 4.01-3.88 (m, 1H), 3.77-3.45 (m, 3H), 2.72-2.52 (m, 3H), 2.48-2.30 (m, 4H), 2.23-1.83 (m, 9H), 1.62-1.35 (m, 6H), 1.21 (s, 3H).

LC-MS (ESI): m/z =581.2 [M+H]⁺.

**Compound 81** isomer 2:¹H NMR (400 MHz, DMSO-*d*₆) δ 7.52-7.42 (m, 2H), 7.41-7.26 (m, 2H), 5.59- 5.14 (m, 1H), 4.01-3.86 (m, 1H), 3.80-3.46 (m, 3H), 2.77-2.58 (m, 3H), 2.46-2.37 (m, 1H), 2.33-1.78 (m, 14H), 1.70-1.36 (m, 4H), 1.30 (s, 3H).

LC-MS (ESI): m/z =581.2 [M+H]⁺.

Referring to the above operation, compound **80J-2** (320 mg, 0.68 mmol) was used as the raw material to obtain the title **compound 81** isomer 3 (70 mg, yield: 17.8%, retention time 2.030 min) and the title **compound 81** isomer 4 (50 mg, 12.7%, retention time: 4.050 min).

HPLC analytical method: 1. Instruments: SHIMADZU LC-30AD SFC; 2. Chromatographic column: Chiralcel OX-3 50×4.6mm I.D., 3µm; 3. Mobile phase system: A for CO2, B for 0.05%DEA in MeOH; 4. Gradient: B 5%-40%; 5. Flow rate: 3 mL/min.

**Compound 81** isomer 3: ¹H NMR (400 MHz, DMSO-*d*₆) δ 7.59-7.30 (m, 4H), 5.46 -5.27 (m, 1H), 3.95-3.62 (m, 3H), 3.53-3.33 (m, 1H), 2.68-2.54 (m, 3H), 2.50-2.34 (m, 4H), 2.33-1.72 (m, 9H), 1.71-1.34 (m, 6H), 1.24 (s, 3H).

LC-MS (ESI): m/z =581.2 [M+H]⁺.

**Compound 81** isomer 4: ¹H NMR (400 MHz, DMSO-*d*₆) δ 7.52-7.27 (m, 4H), 5.46-5.34 (m, 1H), 3.99-3.63 (m, 3H), 3.46-3.34 (m, 1H), 3.31-2.81 (m, 2H), 2.73-2.55 (m, 3H), 2.46-2.31 (m, 1H), 2.21-2.02 (m, 9H), 2.02-1.74 (m, 3H), 1.60-1.38 (m, 4H), 1.27 (s, 3H).

LC-MS (ESI): m/z =581.2 [M+H]⁺.

### Example 82:

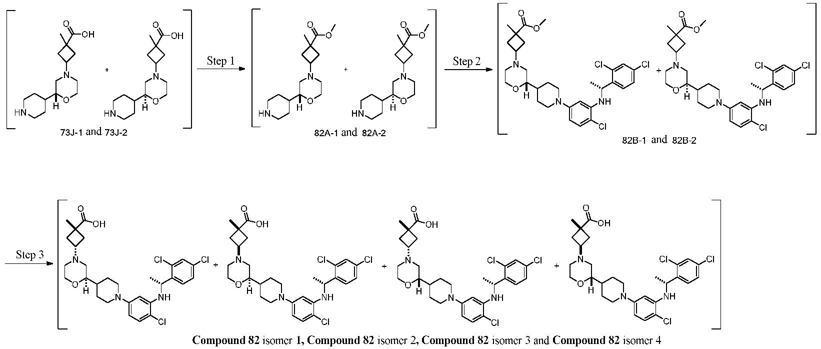

Step 1: Compound **73J**-1 (0.8 g, 2.84 mmol) was dissolved in anhydrous methanol (10 mL), the obtained system was cooled to 0°C, and thionyl chloride (676 mg, 5.68 mmol) was slowly added dropwise. After the dropwise addition was completed, the obtained system was naturally warmed to room temperature and reacted for 15 h. After the reaction was completed, the mixture was concentrated under reduced pressure to obtain the hydrochloride salt of compound **82A**-1 (1.04 g, 100%).

Referring to the above operation, compound **73J**-2 (0.8 g, 2.84 mmol) was used as the raw material to obtain the hydrochloride salt of the title compound **82A**-2 (1.04 g, 100%).

Step 2: Under a nitrogen atmosphere, compound **82A-1** (0.50 g, 1.35 mmol), compound **75E** (0.91 g, 2.03 mmol), tris(dibenzylideneacetone)palladium (250 mg, 0.270 mmol), 2-di-tert-butylphosphino-2-(N,N-dimethylamino)biphenyl (180 mg, 0.53 mmol), and cesium carbonate (1.32 g, 4.05 mmol) were added to a dry toluene (10 mL) in sequence, and then the temperature was raised to 100°C and the reaction was performed for 15 h. After the reaction was completed, the mixture was cooled to room temperature and concentrated under reduced pressure. The residue was separated and purified by silica gel column chromatography (petroleum ether:ethyl acetate (v/v) = 1:1) to obtain compound **82B**-1 (0.26 g, 32.4%).

Referring to the above operation, compound **82A**-2 (0.5 g, 1.35 mmol) was used as the raw material to obtain the title compound **82B**-2 (0.23 g, 28.6%).

Step 3: Compound **82B**-1 (260.0 mg, 0.44 mmol) was added to a mixed solvent of methanol (5 mL) and water (5 mL) at room temperature. After stirring, lithium hydroxide (180.0 mg, 4.29 mmol) was added and the mixture was reacted at room temperature for 3 h. After TLC/LCMS showed that the reaction was completed, 1 M dilute hydrochloric acid was added dropwise to adjust the pH to 7. The obtained system was concentrated under reduced pressure and the residue was purified by silica gel column chromatography (dichloromethane:methanol (v/v) = 10:1) to obtain the title **compound 82** isomer 1 (Rf = 0.4 (dichloromethane:methanol = 10: 1), 60.0 mg, 23.5%) and the title **compound 82** isomer 2 (Rf = 0.2 (dichloromethane:methanol = 10: 1), 30.0 mg, 11.7%).

**Compound 82** isomer 1:¹H NMR (400 MHz, CD₃OD) δ 7.48 (d, 1H), 7.42 (d, 1H), 7.28-7.25 (m, 1H), 7.04 (d, 1H), 6.24-6.21 (m, 1H), 5.84 (d, 1H), 4.97-4.92 (m, 1H), 3.92-3.88 (m, 1H), 3.64-3.61 (m, 1H), 3.48-3.41 (m, 2H), 3.27-3.23 (m, 1H), 2.91-2.78 (m, 3H), 2.62-2.46 (m, 4H), 2.11-2.04 (m, 1H), 1.86-1.81 (m, 4H), 1.56-1.45 (m, 5H), 1.38 (s, 3H), 1.35-1.28(m, 2H),

M/Z (ESI): m/z =580.2[M+H]⁺.

**Compound 82** isomer 2:¹H NMR (400 MHz, CD₃OD) δ 7.48 (d, 1H), 7.42 (d, 1H), 7.29-7.26 (m, 1H), 7.04 (d, 1H), 6.24-6.21 (m, 1H), 5.84 (d, 1H), 4.97-4.92 (m, 1H), 4.00-3.97 (m, 1H), 3.77-3.61 (m, 1H), 3.49-3.43 (m, 3H), 3.24-3.09 (m, 3H), 2.99-2.39 (m, 5H), 2.25-2.14 (m, 3H), 1.84-1.80 (m, 1H), 1.59-1.50 (m, 5H), 1.41-1.34(m, 5H),

M/Z (ESI): m/z =580.2[M+H]⁺.

Referring to the above operation, compound **82B**-2 (230.0 mg, 0.39 mmol) was used as the raw material to obtain the title **compound 82** isomer 3 (Rf=0.4 (dichloromethane:methanol=10:1), 50.0 mg, 22.0%) and the title **compound 82** isomer 4 (Rf=0.2 (dichloromethane:methanol=10:1), 20.0 mg, 8.8%).

**Compound 82** isomer 3:¹H NMR (400 MHz, CD₃OD) δ 7.48 (d, 1H), 7.42 (d, 1H), 7.28-7.25 (m, 1H), 7.04 (d, 1H), 6.24-6.21 (m, 1H), 5.84 (d, 1H), 4.97-4.92 (m, 1H), 3.92-3.88 (m, 1H), 3.64-3.58 (m, 1H), 3.48-3.42 (m, 2H), 3.25-3.21 (m, 1H), 2.93-2.78 (m, 3H), 2.62-2.46 (m, 4H), 2.09-2.02 (m, 1H), 1.82-1.80 (m, 4H), 1.56-1.42 (m, 5H), 1.37 (s, 3H), 1.34-1.24(m, 2H),

M/Z (ESI): m/z =580.2[M+H]⁺.

**Compound 82** isomer 4:¹H NMR (400 MHz, CD₃OD) δ 7.48 (d, 1H), 7.42 (d, 1H), 7.28-7.26 (m, 1H), 7.04 (d, 1H), 6.24-6.21 (m, 1H), 5.84 (d, 1H), 4.97-4.92 (m, 1H), 3.96-3.93 (m, 1H), 3.74-3.66 (m, 1H), 3.46-3.43 (m, 3H), 3.08-2.98 (m, 3H), 2.57-2.29 (m, 5H), 2.12-2.07 (m, 3H), 1.82-1.79 (m, 1H), 1.60-1.47 (m, 5H), 1.40-1.31(m, 5H),

M/Z (ESI): m/z =580.2[M+H]⁺.

### Example 83:

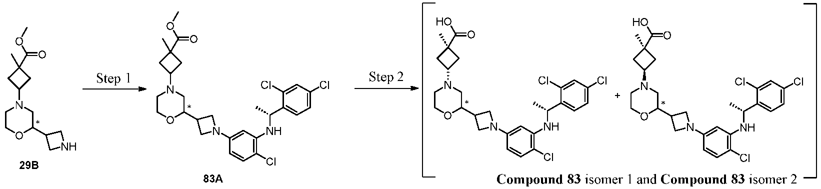

Step 1: Under a nitrogen atmosphere, compound **29B** (0.50 g, 1.86 mmol), compound **75E** (1.2 g, 2.80 mmol), tris(dibenzylideneacetone)palladium (176 mg, 0.19 mmol), 2-di-tert-butylphosphino-2-(N,N-dimethylamino)biphenyl (129 mg, 0.38 mmol) and cesium carbonate (1.82 g, 5.58 mmol) were added to a dry toluene (10 mL), and then the temperature was raised to 100°C and the reaction was performed for 15 h. After the reaction was completed, the mixture was cooled to room temperature and concentrated under reduced pressure. The residue was separated and purified by silica gel column chromatography (petroleum ether:ethyl acetate (v/v) = 1:1) to obtain compound **83A** (0.23 g, 22%).

Step 2: Compound **83A** (100 mg, 0.18 mmol) was dissolved in methanol (1 mL), tetrahydrofuran (1 mL) and water (0.5 mL), lithium hydroxide (13 mg, 0.54 mmol) was added, and the reaction was performed at room temperature for 2 h. After the reaction was completed, water (15 mL) and ethyl acetate (15 mL) were added for extraction and liquid separation. The aqueous phase was extracted with ethyl acetate (15 mL×2). The combined organic phases were washed with saturated brine, dried over anhydrous sodium sulfate, filtered, and concentrated. The residue was purified by silica gel column chromatography (EA:MeOH(v/v)=5:1) to obtain **compound 83** isomer 1 (Rf=0.5, (EA:MeOH(v/v)=3:1), 47 mg, 48%) and **compound 83** isomer 2 (Rf=0.4, (EA:MeOH(v/v)=3:1), 18 mg, 18%).

**Compound 83** isomer 1: ¹H NMR (400 MHz, CDCl₃) δ 7.37 (d, 1H), 7.33 (d, 1H), 7.19-7.16 (m, 1H), 7.02 (d, 1H), 5.71-5.68 (m, 1H), 5.30 (d, 1H), 4.88-4.87 (q, 1H), 4.65 (s, 1H), 3.89 (d, 1H), 3.74-3.73 (t, 1H), 3.67-3.57 (m, 3H), 3.56-3.51 (m, 2H), 2.83 (s, 1H), 2.71-2.56 (m, 6H), 1.97-1.68 (m, 3H), 1.51 (d, 3H), 1.42 (s, 3H).

LCMS (ESI): m/z =552.5 [M+H]⁺.

**Compound 83** isomer 2: ¹H NMR (400 MHz, CDCl₃) NMR δ 7.37 (d, 1H), 7.33 (d, 1H), 7.19-7.16 (m, 1H), 7.02 (d, 1H), 5.71-5.68 (m, 1H), 5.30 (d, 1H), 4.88 (d, 1H), 4.65 (s, 1H), 3.96 (d, 1H), 3.79-3.78 (t, 2H), 3.75-3.69 (m, 1H), 3.64-3.63 (t, 1H), 3.60-3.55 (m, 2H), 3.03-3.02 (t, 2H), 2.91-2.85 (m, 1H), 2.68-2.63 (m, 2H), 2.56 (d, 2H), 2.19 - 2.08 (m, 3H), 1.73-1.72 (t, 1H), 1.52 (d, 3H), 1.41 (s, 3H).

LCMS (ESI): m/z =552.5 [M+H]⁺.

### Example 84:

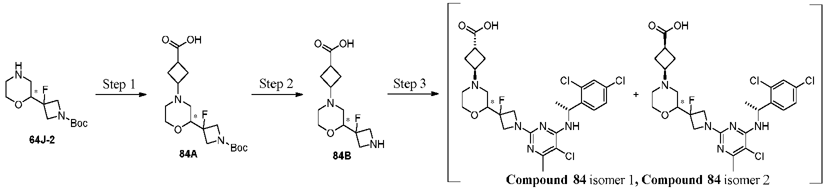

Step 1: Compound **64J-2** (200 mg, 0.77 mmol) and 3-oxocyclobutane-1-carboxylic acid (106 mg, 0.93 mmol) were dissolved in methanol (5 mL) in sequence, and glacial acetic acid (56 mg, 0.93 mmol) was added, the obtained system was stirred evenly, sodium cyanoborohydride (243 mg, 3.85 mmol) was added in portions, and the reaction was performed for 1 h after addition was completed. After the reaction was completed, water (30 mL) was added to quench the reaction, and dichloromethane (50 mL×2) was used for extraction. The combined organic phase was washed with saturated brine, dried over anhydrous sodium sulfate, and filtered. The filtrate was concentrated and the residue was separated and purified by silica gel column chromatography (dichloromethane:methanol (v/v) = 10:1) to obtain the title compound **84A** (200 mg, 72%).

LCMS m/z =303.2 [M+H]⁺.

Step 2: Compound **84A** (240 mg, 0.56 mmol) was dissolved in dichloromethane (3 mL), trifluoroacetic acid (1 mL) was added, and the reaction was performed at room temperature for 30 min. After the reaction was completed, the obtained system was concentrated directly to obtain the trifluoroacetate salt of compound **84B** (144 mg).

LCMS m/z =259.2 [M+H]⁺.

Step 3: Compound **2F** (196 mg, 0.56 mmol), DIEA (216 mg, 1.68 mmol) and cesium fluoride (85 mg, 0.56 mmol) were added to a solution of compound **84B** (144 mg 0.56 mmol) in DMSO (4 mL) in sequence, the obtained system was heated to 100°C and reacted for 2 h. After the reaction was completed, the mixture was cooled to room temperature, and water (15 mL) and ethyl acetate (15 mL) were added for extraction and liquid separation. The aqueous phase was extracted with ethyl acetate (15 mL×2). The combined organic phases were washed with saturated brine, dried over anhydrous sodium sulfate, filtered, and concentrated. The residue was purified by silica gel column chromatography (EA:MeOH(v/v) = 5:1) to obtain **compound 84** isomer 1 (Rf=0.5, (EA:MeOH(v/v)=3:1), 24 mg, 7%) and **compound 84** isomer 2 (Rf=0.4, (EA:MeOH(v/v) = 3:1), 50 mg, 16%).

**Compound 84** isomer 1: ¹H NMR (400 MHz, CDCl₃) δ = 7.37 (d, 1H), 7.22 (d, 1H), 7.19-7.17 (m, 1H), 5.58 (d, 1H), 5.46-5.39 (m, 1H), 4.20-4.12 (m, 1H), 4.05-4.02 (d, 1H), 4.00-3.92 (m, 2H), 3.84-3.66 (m, 2H), 3.18-3.02 (m, 2H), 2.94-2.83 (m, 1H), 2.82 -2.72 (m, 1H), 2.44-2.37 (m, 3H), 2.32 (s, 3H), 2.07-1.99 (m, 3H), 1.52 (d, 3H).

LCMS m/z =572.1 [M+H]⁺.

**Compound 84** isomer 2: ¹H NMR (400 MHz, CDCl₃) δ = 7.37 (d, 1H), 7.23-7.21 (m, 1H), 7.20-7.18 (m, 1H), 5.67 (s, 1H), 5.48-5.39 (m, 1H), 4.25-4.18 (m, 1H), 4.08 (d, 1H), 3.99 (d, 2H), 3.95-3.79 (m, 3H), 3.12 (d, 1H), 3.05-2.90 (m, 2H), 2.68-2.60 (m, 2H), 2.43-2.45 (m, 1H), 2.36 (s, 3H), 2.24-2.12 (m, 2H), 2.03-1.93 (m, 1H), 1.53 (d, 3H).

LCMS m/z =572.1 [M+H]⁺.

### Example 85:

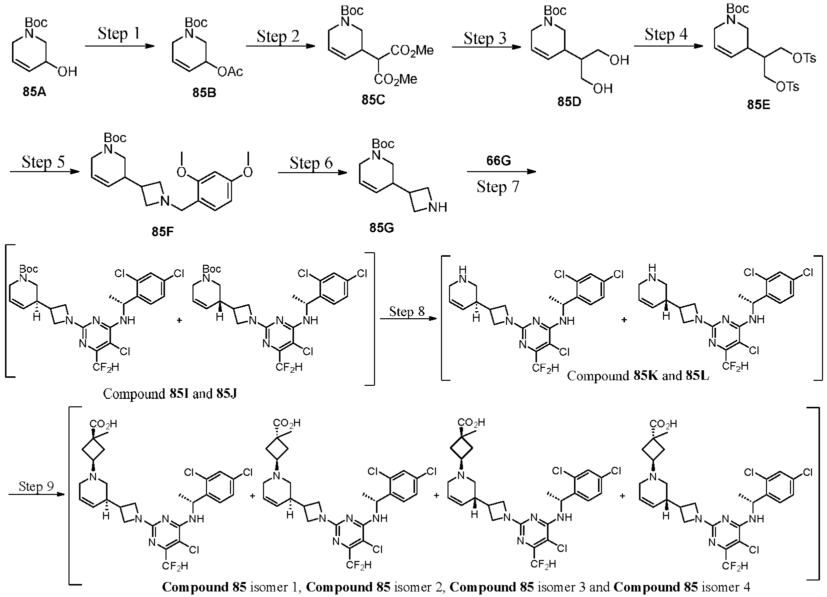

Step 1: Compound **85A** (10.0 g, 50.2 mmol) was dissolved in dichloromethane (200 mL), and pyridine (19.8 g, 251 mmol), 4-dimethylaminopyridine (1.23 g, 10.0 mmol) and acetyl chloride (5.91 g, 75.3 mmol) were added at 0°C in sequence, after the addition was completed, the temperature was raised to room temperature and stirring was continued for 2 h. After the disappearance of the raw materials as monitored by TLC, water (200 mL) was added to quench the reaction, and extraction and liquid separation were performed. The aqueous phase was extracted with dichloromethane (50 mL×5). The combined organic phases were dried over anhydrous sodium sulfate, filtered and concentrated. The crude product was purified by silica gel column chromatography (petroleum ether:ethyl acetate (v/v) = 70:30) to obtain compound **85B** (11.5 g, 95.0%).

Step 2: Dimethyl malonate (18.1 g, 137 mmol) was dissolved in tetrahydrofuran (100 mL), the obtained system was cooled to 0°C, sodium hydride (5.11 g, 128 mmol, 60% in oil) was added, then the temperature was raised to room temperature and the reaction was performed for 30 min, compound **85B** (11.0 g, 45.6 mmol) and tetrakis triphenylphosphine palladium (5.27 g, 4.56 mmol) were added in sequence, and the reaction was continued at room temperature overnight. After the disappearance of the starting material was monitored by TLC, saturated aqueous ammonium chloride solution (100 mL) was added to quench the reaction, the organic and aqueous phases were separated, the aqueous phase was extracted with ethyl acetate (50 mL × 5), the organic phases were combined and dried over anhydrous sodium sulfate, filtered and concentrated, and the crude product was purified by silica gel column chromatography (petroleum ether: ethyl acetate (v/v) = 70:30) to obtain the compound **85C** (9.25 g, 64.8%).

LC-MS (ESI): m/z =314.2 [M+H]⁺.

Step 3: Compound **85C** (9.25 g, 29.5 mmol) was dissolved in dry tetrahydrofuran (100 mL), the obtained system was cooled to -50°C, lithium aluminum hydride (3.36 g, 88.5 mmol) was added, and after the addition was completed, the temperature was raised to -10°C and stirring was continued for about 2 h. After the disappearance of the raw materials as monitored by TLC, saturated aqueous sodium sulfate solution was added to quench the reaction, the mixture was filtered through celite, and washed with ethyl acetate (100 mL×5). The organic phase was dried over anhydrous sodium sulfate, filtered and concentrated. The crude product was purified by silica gel column chromatography (petroleum ether:ethyl acetate (v/v) = 50:50-0:100) to obtain compound **85D** (5.43 g, 71.4%).

LC-MS (ESI): m/z =258.2 [M+H]⁺.

Step 4: Compound **85D** (5.43 g, 21.1 mmol) was dissolved in dry 1,2-dichloroethane (100 mL), and 4-dimethylaminopyridine (12.9 g, 106 mmol) and p-toluenesulfonyl chloride (12.1 g, 63.4 mmol) were added in sequence, after the addition was completed, stirring was continued at room temperature for 2 h. After the disappearance of the raw materials as monitored by TLC, water (200 mL) was added to quench the reaction, and the organic phase and the aqueous phase were separated. The aqueous phase was extracted with dichloromethane (50 mL×3), and the organic phases were combined and dried over anhydrous sodium sulfate, filtered, and concentrated. The crude product was purified by silica gel column chromatography (petroleum ether:ethyl acetate (v/v) = 65:35) to obtain compound **85E** (8.90 g, 74.5%).

Step 5: Compound **85E** (1.93 g, 3.41 mmol) and 2,4-dimethoxybenzylamine (2.85 g, 17.1 mmol) were dissolved in acetonitrile (50 mL) in sequence, the temperature was raised to 70°C and the reaction was performed overnight. The reaction was stopped after the complete disappearance of the raw materials as monitored by TLC. After cooling to room temperature, the obtained system was directly concentrated under reduced pressure. The residue was separated by silica gel column chromatography (dichloromethane:methanol (v/v)=80:20) to obtain **85F** (1.98 g).

LC-MS (ESI): m/z =389.3 [M+H]⁺.

Step 6: **85F** (1.98 g) was dissolved in a mixed solvent of acetonitrile (30 mL) and water (6 mL), ceric ammonium nitrate (5.60 g, 10.2 mmol) was added, and the reaction was performed at room temperature overnight. After the disappearance of the raw materials as monitored by LCMS, solid sodium thiosulfate was added to quench the reaction. After filtration, the filtrate was concentrated under reduced pressure. The residue was separated by C18 silica gel column chromatography [water (containing 0.5% trifluoroacetic acid):acetonitrile (v/v) = 95:5-55:45)] to obtain the trifluoroacetate salt of compound **85G** (310 mg, two-step yield: 25.8%).

LC-MS (ESI): m/z =239.2 [M+H]⁺.

Step 7: The trifluoroacetate salt of compound **85G** (310 mg, 0.878 mmol), compound **66G** (408 mg, 1.05 mmol), N,N-diisopropylethylamine (566 mg, 4.39 mmol), and cesium fluoride (267 mg, 1.76 mmol) were dissolved in dimethyl sulfoxide (6 mL) in sequence, and the temperature was raised to 100°C after dissolution and the reaction was performed for about 2 h. After the disappearance of the raw materials as monitored by TLC, the mixture was cooled to room temperature and concentrated under reduced pressure. The residue was purified by silica gel column chromatography (petroleum ether:ethyl acetate (v/v) = 85:15) to obtain a mixture of compounds **85I** and **85J** (270 mg, 52.2%).

The above mixture was separated by chiral HPLC to prepare compound **85I** (85.4 mg, 16.5%) and compound **85J** (94.2 mg, 18.2%). Chiral preparation method: instrument: Waters 150 MGM; chromatographic column: Chiralpak Column; mobile phase: A: carbon dioxide, and B: methanol (0.1% aqueous ammonia); isocratic elution: 35% mobile phase B; flow rate: 100 mL/min; back pressure: 100 bar; column temperature: 25°C; wavelength: 220 nm.

Chromatographic analytical method: 1. Instruments: SHIMADZU LC-30AD SFC; 2. Chromatographic column: Chiralcel OX-3 50×4.6mm I.D., 3µm; 3. Mobile phase system: A for CO2, B for 0.05%DEA in MeOH; 4. Gradient: B 5%-40%; 5. Flow rate: 3 mL/min. Compounds **85I,** retention time 1.85 min; Compounds **85J,** retention time 2.07 min.

LC-MS (ESI): m/z =588.1 [M+H]⁺;

Step 8: Compounds **85I** (85.4 mg, 0.145 mmol) was dissolved in dichloromethane (6 mL), trifluoroacetic acid (2 mL) was added at room temperature, and the reaction was continued for 1 h after the addition was completed. After the reaction was completed as monitored by TLC, the obtained system was concentrated to obtain the crude trifluoroacetate salt of **85K** (138 mg), which was directly used in the next step without purification.

LC-MS (ESI): m/z = 488.1 [M+H]⁺;

Using **85J** as the raw material, the crude trifluoroacetate salt of compound **85L** (166 mg) was obtained by referring to the above synthesis method.

Step 9: The crude trifluoroacetate salt of compound **85K** obtained in the previous step was dissolved in methanol (5 mL), and compound **2B** (44.7 mg, 0.349 mmol) and acetic acid (14.0 mg, 0.233 mmol) were added in sequence and stirred at room temperature for 1 h, then sodium cyanoborohydride (29.2 mg, 0.465 mmol) was added, and stirring was continued for 2 h. After the reaction was completed as monitored by LCMS, water (1 mL) was added to quench the reaction, and the residue was separated and purified by preparative HPLC to obtain **compound 85** isomer 1 (37 mg, two-step yield: 42.5%) and **compound 85** isomer 2 (32 mg, two-step yield: 36.7%).

Preparative HPLC separation and purification method: 1. Instruments: waters 2767 preparative liquid chromatographic instrument; chromatographic column: SunFire @ Prep C18 (19 mm × 250 mm). 2. The sample was filtered with a 0.45 µm filter head to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 5%-50%; c. flow rate: 12 mL/min; d. elution time: 30 min.

HPLC analysis conditions: 1. Instruments: Shimadzu LC-20AT; 2. Chromatographic column: Xtimate C18 4.6*50mm, 3µm; 3. Mobile phase system: A for 0.05% TFA in H2O; B for ACN; 4. Gradient: B 5-95%; 5. Flow rate: 1.0 mL/min, run time: 10 min. retention time: Compound 85 isomer 1 retention time: : tR=4.112 min; Compound 85 isomer 2: tR = retention time: 4.144 min.

**Compound 85** isomer 1: ¹H NMR (400 MHz, Methanol-d4) *δ* 7.43 (d, 1H), 7.37 (d, 1H), 7.26 (dd, 1H), 6.67 (s, 1H), 5.82 (ddt, 1H), 5.74 (d, 1H), 5.50 (q, 1H), 4.03 (t, 1H), 3.98-3.87( m, 1H), 3.80 (dd, 1H), 3.66-3.51(m, 1H), 3.43-3.34 (m, 1H), 3.30-3.21( d, 1H), 3.13-2.95 (m, 2H), 2.75-2.49 (m, 4H), 2.31-2.18 (m, 1H), 1.97 -1.87 (m, 2H), 1.53 (d, 3H), 1.37 (s, 3H).

LC-MS (ESI): m/z =600.1 [M+H]⁺;

**Compound 85** isomer 2: ¹H NMR (400 MHz, Methanol-d4) *δ* 7.43 (d, 1H), 7.37 (d, 1H), 7.26 (dd, 1H), 6.67 (s, 1H), 5.88-5.78 (m, 2H), 5.52 (q, 1H), 4.05 (t, 1H), 3.99-3.89 (m, 1H), 3.82 (dd, 1H), 3.69-3.53 (m, 2H), 3.44 (q, 1H), 3.24 (dd, 2H), 2.85-2.74 (m, 1H), 2.64-2.51 (m, 3H), 2.45 (t, 1H), 2.26-2.12 (m, 2H), 1.53 (d, 3H), 1.38 (s, 3H).

LC-MS (ESI): m/z =600.1 [M+H]⁺;

Using **85L** as the raw material, **compound 85** isomer 3 (28 mg, two-step yield: 32.1%) and **compound 85** isomer 4 (33 mg, two-step yield: 37.9%) were obtained by referring to the above synthesis method.

HPLC analysis conditions: 1. Instruments: Shimadzu LC-20AT; 2. Chromatographic column: Xtimate C18 4.6*50mm, 3µm; 3. Mobile phase system: A for 0.05% TFA in H2O; B for ACN; 4. Gradient: B 5-95%; 5. Flow rate: 1.0 mL/min, run time: 10 min. retention time: Compound 85 isomer 3 retention time: 4.114 min; Compound 85 isomer 4 retention time: 4.149 min.

**Compound 85** isomer 3: ¹H NMR (400 MHz, Methanol-d4) *δ* 7.42 (d, 1H), 7.38 (d, 1H), 7.27 (dd, 1H), 6.67 (s, 1H), 5.90-5.78 (m, 1H), 5.73 (d, 1H), 5.52 (q, 1H), 4.05 (t, 1H), 3.99-3.87 (m, 1H), 3.78 (dd, 1H), 3.68-3.54 (m, 1H), 3.41- 3.33 (m, 1H), 3.25 (q, 1H), 3.10-2.95 (m, 2H), 2.76-2.49 (m, 4H), 2.21 (dd, 1H), 1.93 (ddd, 2H), 1.53 (d, J = 7.1 Hz, 3H), 1.38 (s, 3H).

LC-MS (ESI): m/z =600.1 [M+H]⁺;

**Compound 85** isomer 4: ¹H NMR (400 MHz, Methanol-d4) *δ* 7.43 (d, 1H), 7.38 (d, 1H), 7.27 (dd, 1H), 6.67 (s, 1H), 5.92-5.74 (m, 2H), 5.53 (q, 1H), 4.07 (t, 1H), 3.99-3.87 (m, 1H), 3.80 (dd, 1H), 3.71-3.60 (m, 1H), 3.55 (d, 1H), 3.41 (q, 1H), 3.21 (d, 2H), 2.82-2.70 (m, 1H), 2.68-2.56 (m, 1H), 2.56-2.39 (m, 3H), 2.21 (qd, 2H), 1.53 (d, 3H), 1.38 (s, 3H).

LC-MS (ESI): m/z =600.1 [M+H]⁺.

### Example 86:

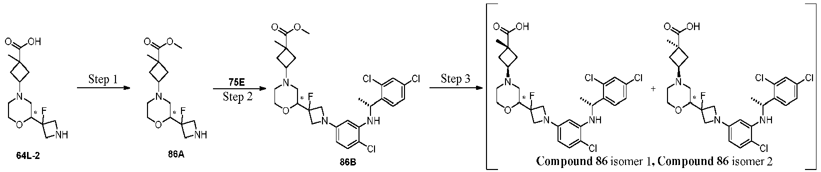

Step 1: Compound **64L-2** (300 mg, 1.10 mmol) was dissolved in methanol (5 mL) at room temperature, dichlorosulfoxide (261.7 mg, 2.20 mmol) was added, and the obtained system was reacted under stirring for 3 h at room temperature. After the reaction was completed, the mixture was concentrated under reduced pressure to obtain a crude compound **86A** (150 mg, 47%).

LCMS m/z =287.2 [M+H]⁺.

Step 2: Compound **86A** (150 mg, 0.52 mmol), compound **75E** (282.0 mg, 0.62 mmol), tris(dibenzylideneacetone)palladium (42 mg, 0.052 mmol), 2-di-tert-butylphosphino-2-(N,N-dimethylamino)biphenyl (35.5 mg, 0.10 mmol) and cesium carbonate (508.3 mg, 1.56 mmol) were added to a dry 1,4-dioxane (5.0 mL), then the temperature was raised to 100°C and the reaction was performed for 15 h. After the reaction was completed, the mixture was cooled to room temperature and concentrated under reduced pressure. The residue was separated and purified by silica gel column chromatography (petroleum ether:ethyl acetate (v/v) = 1:1) to obtain compound **86B** (150 mg, 49%).

LCMS m/z =584.6 [M+H]⁺.

Step 3: Compound **86B** (150 mg, 0.26 mmol) was dissolved in methanol (1 mL), tetrahydrofuran (1 mL) and water (0.5 mL) at room temperature, and lithium hydroxide (18.7 mg, 0.78 mmol) was added, the reaction was performed at room temperature for 2 h. After the reaction was completed, water (10 mL) and ethyl acetate (15 mL) were added for extraction and liquid separation. The aqueous phase was extracted with ethyl acetate (15 mL×2). The combined organic phases were washed with saturated brine, dried over anhydrous sodium sulfate, filtered, and concentrated. The residue was purified by silica gel column chromatography (EA:MeOH(v/v)=9:1) to obtain **compound 86** isomer 1 (Rf=0.3, (EA:MeOH(v/v)=9:1), 45 mg, 31%) and **compound 86** isomer 2 (Rf=0.25, (EA:MeOH(v/v)=9:1), 32 mg, 22%).

**Compound 86** isomer 1: ¹H NMR (400 MHz, CDCl₃) δ 7.39 (d, 1H), 7.33 (d, 1H), 7.18 (dd, 1H), 7.04 (d, 1H), 5.73 (dd, 1H), 5.34 (d, 1H), 4.91-4.85 (m, 1H), 4.68 (s, 1H), 3.97-3.85 (m, 3H), 3.79-3.62 (m, 4H), 2.91-2.80 (m, 2H), 2.69 (d, 1H), 2.65-2.58 (m, 2H), 2.02-1.86 (m, 3H), 1.79 (t, 1H), 1.52 (d, 3H), 1.42 (s, 3H).

LCMS m/z =570.6 [M+H]⁺.

**Compound 86** isomer 2: ¹H NMR (400 MHz, CDCl₃) δ 7.39 (d, 1H), 7.33 (d, 1H), 7.18 (dd, 1H), 7.05 (d, 1H), 5.73 (dd, 1H), 5.34 (d, 1H), 4.92-4.86 (m, 1H), 4.69 (s, 1H), 4.02-3.90 (m, 3H), 3.89-3.61 (m, 4H), 3.11 (d, 1H), 3.00 (d, 1H), 2.93-2.86 (m, 1H), 2.58 (dd, 2H), 2.18-2.06 (m, 3H), 1.94 (t, 1H), 1.52 (d, 3H), 1.42 (s, 3H).

LCMS m/z =570.6 [M+H]⁺.

### Example 87:

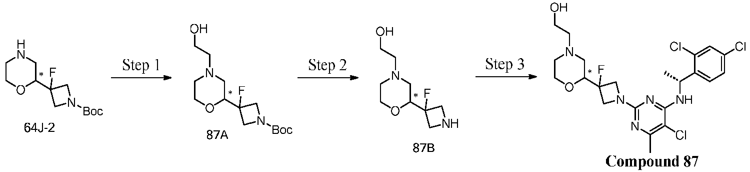

Step 1: **64J-2** (100.0 mg, 0.38 mmol), bromoethanol (94.97 mg, 0.76 mmol), and N,N-diisopropylethylamine (49 mg, 0.38 mmol) were dissolved in acetonitrile (2 mL) in sequence, and the obtained system was stirred at room temperature for 2 h. After the reaction was completed, water was added and the mixture was extracted with ethyl acetate three times. The combined organic phases were washed with saturated brine, dried over anhydrous sodium sulfate, filtered, concentrated under reduced pressure, and purified by flash column chromatography (MeOH:DCM=10%) to obtain the title compound **87A** (97 mg, 84%).

LCMS m/z =305.3 [M+H]⁺.

Step 2: **87A** (97.4 mg, 0.32 mmol) was dissolved in dichloromethane (2 mL), trifluoroacetic acid (1 mL) was added, and the obtained system was stirred at room temperature for 0.5 h. After the reaction was completed, the mixture was concentrated under reduced pressure to obtain the title compound **87B** (65 mg, 100%) which was directly used for the next step.

LCMS m/z =205.3 [M+H]⁺.

Step 3: **87B** (65 mg, 0.32 mmol), **2F** (134.8 mg, 0.38 mmol), cesium fluoride (145.8 mg, 0.96 mmol), and N,N-diisopropylethylamine (124.0 mg, 0.96 mmol) were dissolved in dimethyl sulfoxide (3 mL) in sequence, the obtained system was stirred evenly, heated to 100°C and stirred for 2 h. After the reaction was completed, the mixture was cooled to room temperature and the reaction solution was separated by preparative HPLC to obtain (separation method: 1. Instruments: waters 2767 preparative liquid chromatographic instrument; chromatographic column: SunFire@ Prep C18 (19 mm × 250 mm). 2. The sample was filtered with a 0.45 µm filter head to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min; retention time 7.0 min) the title **compound 87** (50 mg, 30%).

LCMS m/z =519.8[M+H]⁺.

¹H NMR (400 MHz, DMSO) δ 7.54 (d, 1H), 7.49 (d, 1H), 7.44 (d, 1H), 7.38 (dd, 1H), 5.47-5.35 (m, 1H), 4.08 (dd, 1H), 3.85 (d, 1H), 3.82-3.74 (m, 2H), 3.67-3.57 (m, 2H), 3.51 (t, 3H), 2.83 (d, 1H), 2.70 (d, 1H), 2.41 (t, 2H), 2.21 (s, 3H), 2.10-1.99 (m, 1H), 1.90 (s, 1H), 1.84 (t, 1H), 1.45 (d, 3H).

### Example 88:

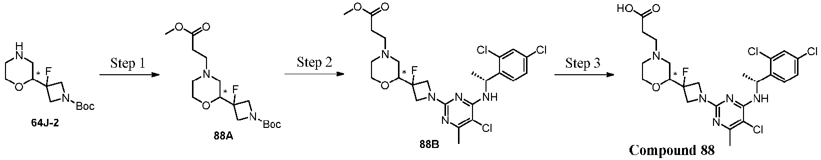

Step 1: **64J-2** (0.10 g, 0.38 mmol) was dissolved in acetonitrile (3 mL), triethylamine (77 mL, 0.76 mmol) and methyl 3-bromopropionate (76 mg, 0.46 mmol) were added, after the addition was completed, the reaction was performed at room temperature for 16 h. Water (10 mL) and ethyl acetate (10 mL) were added for extraction and liquid separation. The aqueous phase was extracted with ethyl acetate (10 mL × 3). The combined organic phases were washed with saturated brine (20 mL), dried over anhydrous sodium sulfate, filtered, and concentrated to obtain the product **88A** (124 mg, 94.20%).

LCMS (ESI): m/z =347.7 [M+H]⁺.

Step 2: **88A**(124 mg, 0.36 mmol) was added to dichloromethane (3 mL), and trifluoroacetic acid (1 mL) was added dropwise, and the obtained system was stirred at room temperature for 2 h after the dropwise addition was completed. After the reaction was completed as monitored by LCMS, most of the trifluoroacetic acid was removed by concentration, and the residue was dissolved in DMSO (3 mL). **2F** (0.14 g, 0.40 mmol), DIEA (0.23 g, 1.80 mmol) and cesium fluoride (0.11 g, 0.72 mmol) were added in sequence. After stirring evenly, the mixture was heated to 100°C and reacted for 2 h. After the reaction was completed, the mixture was cooled to room temperature, and water (15 mL) and ethyl acetate (15 mL) were added. The mixture was stirred and subjected to liquid separation. The aqueous phase was extracted with ethyl acetate (15 mL×2). The combined organic phases were dried over anhydrous sodium sulfate, filtered, concentrated, and separated by column chromatography (dichloromethane:methanol (v/v) = 100:1-10:1) to obtain compound **88B** (125 mg, 61.91%).

LCMS m/z =560.7 [M+H]⁺.

Step 3: Lithium hydroxide monohydrate (27 mg, 0.65 mmol) was added to a solution of **88B** (125 mg, 0.22mmol) in tetrahydrofuran (3 mL) and water (1 mL), and the reaction was performed at room temperature for 16 h. After the reaction was completed, tetrahydrofuran was removed by concentration, water (3 mL) was added, the pH was adjusted to 3-4 with 1N dilute hydrochloric acid, ethyl acetate (10×2 mL) was added for extraction, and the combined organic phases were dried over anhydrous sodium sulfate, filtered, concentrated, and separated by column chromatography (dichloromethane:methanol (v/v) = 100:1-10:1) to obtain **compound 88** (100 mg, 83.12%).

¹H NMR (400 MHz, Chloroform-d) δ 7.40-7.33 (m, 1H), 7.25-7.20 (m, 1H), 7.25-7.15 (m, 1H), 5.62-5.48 (m, 1H), 5.46-5.37 (m, 1H), 4.19-4.08 (m, 1H), 4.08-3.96 (m, 3H), 3.96-3.86 (m, 1H), 3.81-3.64 (m, 2H), 3.06-2.97 (m, 1H), 2.97-2.86 (m, 1H), 2.85-2.74 (m, 2H), 2.61-2.51 (m, 2H), 2.43-2.34 (m, 1H), 2.31 (s, 3H), 2.23-2.13 (m, 1H), 1.58-1.45 (m, 3H).

LCMS m/z =546.2 [M+H]⁺.

### Example 89:

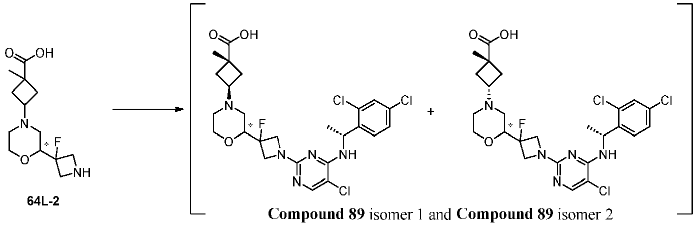

**64L-2** (0.17 g, 0.46 mmol) was dissolved in dichloromethane (4 mL), trifluoroacetic acid (1 mL) was added, and the obtained system was stirred at room temperature for 2 h. After the reaction was completed as monitored by LCMS, most of the trifluoroacetic acid was removed by concentration and the obtained system was dissolved in DMSO (3 mL). **42B** (0.17 g, 0.51 mmol), DIEA (0.21 g, 0.93 mmol) and cesium fluoride (0.35 g, 2.30 mmol) were added in sequence. After stirring evenly, the temperature was raised to 100°C and the reaction was performed for 2 h. After the reaction was completed, the mixture was cooled to room temperature, and water (15 mL) and ethyl acetate (15 mL) were added. The mixture was stirred and subjected to liquid separation. The aqueous phase was extracted with ethyl acetate (15 mL×2). The combined organic phases were dried over anhydrous sodium sulfate, filtered, concentrated, and subjected to column chromatography (dichloromethane:methanol (v/v) = 100:1-10:1) to obtain **compound 89** isomer 1 (Rf = 0.50 (dichloromethane:methanol = 10:1), 71 mg, 26.94%) and **compound 89** isomer 2 (Rf = 0.45 (dichloromethane:methanol = 10:1), 17 mg, 6.45%).

**Compound 89** isomer 1: ¹H NMR (400 MHz, Chloroform-d) δ7.93 (s, 1H), 7.42-7.35 (m, 1H), 7.24-7.15 (m, 2H), 5.61-5.31 (m, 1H), 5.50-5.41 (m, 1H), 4.24-3.83 (m, 5H), 3.77-3.61 (m, 2H), 2.92-2.75 (m, 2H), 2.75-2.65 (m, 1H), 2.65-2.54 (m, 2H), 2.01-1.79 (m, 3H), 1.75-1.61 (m, 1H), 1.58-1.48 (m, 3H), 1.41 (s, 3H).

LCMS m/z =572.1 [M+H]⁺.

**Compound 89** isomer 2: ¹H NMR (400 MHz, Chloroform-d) δ7.84 (s, 1H), 7.41-7.34 (m, 1H), 7.25-7.21 (m, 1H), 7.21-7.16 (m, 1H), 5.58-5.50 (m, 1H), 5.50-5.41 (m, 1H), 4.23-4.12 (m, 1H), 4.09-3.76 (m, 6H), 3.17-3.07 (m, 1H), 3.07-2.97 (m, 1H), 2.60-2.46 (m, 2H), 2.23-2.04 (m, 3H), 1.96-1.82 (m, 1H), 1.57-1.49 (m, 3H), 1.42 (s, 3H).

LCMS m/z =572.1 [M+H]⁺.

### Example 90:

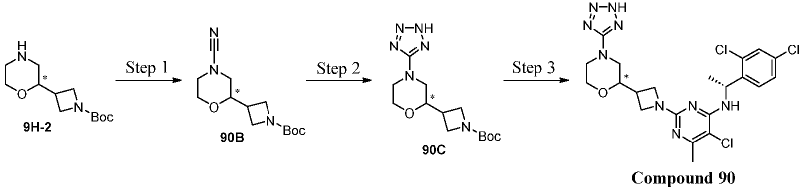

Step 1: **9H-2** (0.10 g, 0.41 mmol) was dissolved in dichloromethane (3 mL), and cyanogen bromide (52 mg, 0.49 mmol), sodium bicarbonate (69 mg, 0.82 mmol) and water (0.3 mL) were added in sequence, and the reaction was performed at room temperature for 16 h. After the reaction was completed, water (5 mL) and dichloromethane (5 mL) were added for extraction and liquid separation. The organic phase was washed with saturated brine (8 mL), dried over anhydrous sodium sulfate, filtered, and concentrated. The residue was separated and purified by column chromatography (petroleum ether:ethyl acetate (v/v) = 100:1-1:1) to obtain the product **90B** (99 mg, 90.33%).

LCMS (ESI): m/z =168.10 [M-Boc+H]⁺.

Step 2: **90B** (99 mg, 0.37 mmol) was dissolved in anhydrous DMF (3 mL), ammonium chloride (0.19 g, 2.21 mmol) and sodium azide (0.23 g, 2.21 mmol) were add and stirred evenly, the temperature was raised to 140°C and the reaction was performed for 5 h. After the reaction was completed, the mixture was cooled to room temperature and concentrated to remove DMF. The residue was separated and purified by column chromatography (dichloromethane:methanol (v/v) = 100:1-10:1) to obtain the product **90C** (107 mg, 93.18%).

LCMS (ESI): m/z =301.2 [M+H]⁺.

Step 3: **90C** (107 mg, 0.46 mmol) was dissolved in dichloromethane (4 mL), trifluoroacetic acid (1 mL) was added, and the obtained system was stirred at room temperature for 2 h. After the reaction was completed as monitored by LCMS, concentration was performed to remove trifluoroacetic acid, the obtained system was dissolved in anhydrous DMSO (3 mL), and **2F** (0.14 g, 0.40 mmol), DIEA (0.22 g, 1.70 mmol) and cesium fluoride (0.10 g, 0.68 mmol) were added, the obtained system was stirred evenly, heated to 100°C and reacted for 2 h. After the reaction was completed, the mixture was cooled to room temperature, and water (15 mL) and ethyl acetate (15 mL) were added. The mixture was stirred and subjected to liquid separation. The aqueous phase was extracted with ethyl acetate (15 mL × 2). The combined organic phases were dried over anhydrous sodium sulfate, filtered, concentrated, and separated by column chromatography (dichloromethane:methanol (v/v) = 100:1-10:1) to obtain **compound 90** (20 mg, 11.21%).

1H NMR (400 MHz, Methanol-d4) δ 7.46-7.43 (m, 1H), 7.41-7.36 (m, 1H), 7.31-7.25 (m, 1H), 5.56-5.49 (m, 1H), 4.05-3.89 (m, 4H), 3.81-3.62 (m, 5H), 3.21-3.13 (m, 1H), 2.81-2.71 (m, 2H), 2.32 (s, 3H), 1.58-1.49 (m, 3H).

LCMS m/z =524.1 [M+H]⁺.

### Example 91:

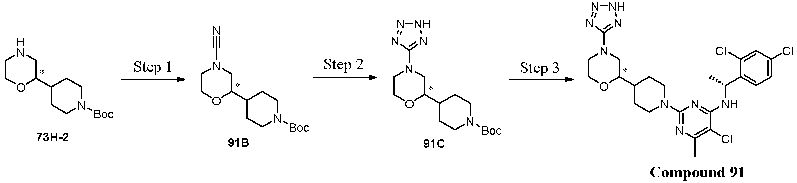

Step 1: **73H-2** (0.15 g, 0.55 mmol) was dissolved in dichloromethane (3 mL), cyanogen bromide (70 mg, 0.66 mmol), sodium bicarbonate (92 mg, 1.10 mmol) and water (0.3 mL) were added in sequence, the reaction was performed at room temperature for 16 h. After the reaction was completed, water (5 mL) and dichloromethane (5 mL) were added for extraction and liquid separation, and the organic phase was washed with saturated brine (8 mL), dried over anhydrous sodium sulfate, filtered, and concentrated. The residue was separated and purified by column chromatography (petroleum ether:ethyl acetate (v/v) = 100:1-1:1) to obtain the product **91B** (0.16 g, 98.49%).

LCMS (ESI): m/z =296.2 [M+H]⁺.

Step 2: **91B** (0.16 g, 0.54 mmol) was dissolved in anhydrous DMF (3 mL), ammonium chloride (0.17 g, 3.18 mmol) and sodium azide (0.21 g, 3.23 mmol) were added, and the obtained system was stirred evenly, heated to 140°C and reacted for 5 h. After the reaction was completed, the mixture was cooled to room temperature and concentrated to remove DMF. The residue was separated and purified by column chromatography (dichloromethane:methanol (v/v) = 100:1~10:1) to obtain the product **91C** (0.15 g, 82.08%).

LCMS (ESI): m/z =339.4 [M+H]⁺.

Step 3: **91C** (0.15 g, 0.44 mmol) was dissolved in dichloromethane (4 mL), trifluoroacetic acid (1 mL) was added, and the obtained system was stirred at room temperature for 2 h. After the reaction was completed as monitored by LCMS, the trifluoroacetic acid was removed by concentration and the obtained system was dissolved in anhydrous DMSO (3 mL). **2F** (0.19 g, 0.54 mmol), DIEA (0.28 g, 2.16 mmol) and cesium fluoride (0.13 g, 0.86 mmol) were added in sequence. After stirring evenly, the mixture was heated to 100°C and reacted for 2 h. After the reaction was completed, the mixture was cooled to room temperature, and water (15 mL) and ethyl acetate (15 mL) were added. The mixture was stirred and subjected to liquid separation. The aqueous phase was extracted with ethyl acetate (15 mL×2). The combined organic phases were dried over anhydrous sodium sulfate, filtered, concentrated, and separated by column chromatography (dichloromethane:methanol (v/v) = 100:1-10:1) to obtain **compound 91** (90 mg, 37.0%).

1H NMR (400 MHz, DMSO-*d6*) δ 15.07 (s, 1H), 7.57-7.50 (m, 1H), 7.49-7.41 (m, 1H), 7.41-7.34 (m, 1H), 7.31-7.15 (m, 1H), 5.44-5.33 (m, 1H), 4.54-4.37 (m, 2H), 3.97 -3.87 (m, 1H), 3.69-3.49 (m, 3H), 3.22-3.14 (m, 1H), 3.10-2.97 (m, 1H), 2.88-2.75 (m, 1H), 2.69-2.52 (m, 2H), 2.20 (s, 3H), 1.78-1.49 (m, 3H), 1.49-1.38 (m, 3H), 1.18-1.01 (m, 1H), 0.91-0.68 (m, 1H).

LCMS m/z =552.2 [M+H]⁺.

### Example 92:

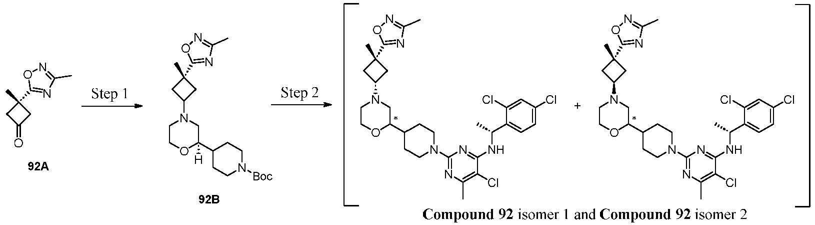

Step 1: **92A** (0.15 g, 0.90 mmol) was dissolved in ethanol (3 mL), **73H-2** (0.29 g, 1.08 mmol) was added, the obtained system was stirred at room temperature for 16 h, and sodium cyanoborohydride (0.11 g, 1.80 mmol) was added in portions, then the reaction was performed at room temperature for 16 h. After the reaction was completed, saturated aqueous ammonium chloride solution (10 mL) and ethyl acetate (10 mL) were added for extraction and liquid separation. The aqueous phase was extracted with ethyl acetate (10 mL×2). The combined organic phases were washed with saturated brine, dried over anhydrous sodium sulfate, filtered, concentrated, and subjected to column chromatography (petroleum ether :ethyl acetate (v/v) = 100:1-3:1) to obtain **92B** (0.12 g, 31.70%).

LCMS m/z =421.3 [M+H]⁺.

Step 2: **92B** (0.12 g, 0.29 mmol) was dissolved in dichloromethane (4 mL), trifluoroacetic acid (1 mL) was added, and the obtained system was stirred at room temperature for 2 h. After the reaction was completed as monitored by LCMS, most of the trifluoroacetic acid was removed by concentration and the obtained system was dissolved in DMSO (3 mL). **2F (0.12** g, 0.34 mmol), DIEA (0.19 g, 1.47 mmol) and cesium fluoride (88 mg, 0.58 mmol) were added in sequence. The obtained system was stirred evenly, heated to 100°C and reacted for 2 h. After the reaction was completed, the mixture was cooled to room temperature, and water (15 mL) and ethyl acetate (15 mL) were added. The mixture was stirred and subjected to liquid separation. The aqueous phase was extracted with ethyl acetate (15 mL×2). The combined organic phases were dried over anhydrous sodium sulfate, filtered, concentrated, and subjected to column chromatography (dichloromethane:methanol (v/v) = 100:1-10:1) to obtain a crude product of **compound 92.** The crude product was prepared by SFC (instrument: SFC Prep 150 AP; chromatographic column: Daicel IC-H (19mm*250mm), the sample was dissolved in methanol, and the chromatographic conditions were: a. composition of mobile phase A, B: mobile phase A: CO₂, mobile phase B: methanol, b. isocratic elution, mobile phase B content 20%, c. flow rate 40 mL/min) to obtain **compound 92** isomer 1 (8 mg, 4.34%) and **compound 92** isomer 2 (10 mg, 5.43%).

HPLC analysis conditions: 1. Instruments: Shimadzu LC-20AT; 2. Chromatographic column: Xtimate C18 4.6*50mm, 3µm; 3. Mobile phase system: A for 0.05% TFA in H2O; B for ACN; 4. Gradient: B 5-95%; 5. Flow rate: 1.0 mL/min, run time: 10 min. retention time: **Compound 92** isomer 1 retention time: 3.112 min; **Compound 92** isomer 2 retention time: 3.244 min.

**Compound 92** isomer 1: ¹H NMR (400 MHz, Chloroform-d) δ 7.36-7.32 (m, 1H), 7.24-7.19 (m, 1H), 7.18-7.12 (m, 1H), 5.46-5.34 (m, 2H), 4.59-4.44 (m, 2H), 3.94-3.82 (m, 1H), 3.65-3.53 (m, 1H), 3.24-3.11 (m, 1H), 2.96-2.83 (m, 1H), 2.78-2.49 (m, 6H), 2.40 (s, 3H), 2.28 (s, 3H), 2.16-2.00 (m, 2H), 1.98-1.87 (m, 1H), 1.86 -1.61 (m, 3H), 1.60 (s, 3H), 1.52-1.48 (m, 3H), 1.22-1.08 (m, 1H), 0.95-0.81 (m, 1H).

LCMS m/z =634.2 [M+H]⁺.

**Compound 92** isomer 2: ¹H NMR (400 MHz, Chloroform-d) δ 7.39-7.31 (m, 1H), 7.24-7.20 (m, 1H), 7.19-7.12 (m, 1H), 5.46-5.32 (m, 2H), 4.60-4.44 (m, 2H), 4.07-3.82 (m, 2H), 3.65-3.52 (m, 1H), 3.48-3.10 (m, 3H), 2.99-2.87 (m, 1H), 2.81-2.43 (m, 6H), 2.38 (s, 3H), 2.28 (s, 3H), 2.04-1.90 (m, 1H), 1.82-1.66 (m, 2H), 1.55 (s, 3H), 1.52-1.43 (m, 3H), 1.30-1.24 (m, 1H), 0.93-0.82 (m, 1H).

LCMS m/z =634.2[M+H]⁺.

### Example 93:

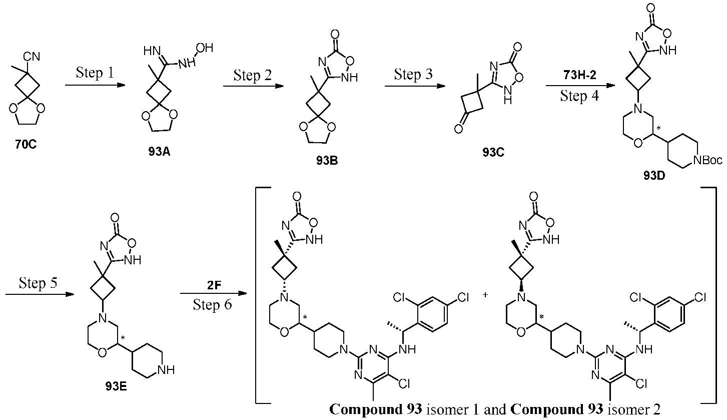

Step 1: Compound **70C** (1.5 g, 9.8 mmol) was dissolved in ethanol (15 mL), hydroxylamine hydrochloride (676 mg, 9.8 mmol) and triethylamine (1.98 g, 19.6mmol) were added, and the reaction was performed at 70°C for 16 h. After the reaction was cooled to room temperature, the obtained system was concentrated and water (30 mL) was added. The mixture was extracted with dichloromethane (20 mL × 2). The combined organic phases were washed with saturated brine, dried over anhydrous sodium sulfate, filtered, and concentrated to obtain the crude compound **93A** (1.4 g, 77%).

LCMS (ESI): m/z =187.1 [M+H]⁺.

Step 2: Compound **93A** (1.4 g, 7.5 mmol) was dissolved in dioxane (40 mL), N,N'-carbonyldiimidazole (1.34 g, 8.25 mmol) and 1,8-diazabicyclo[5.4.0]undec-7-ene (1.25 g, 8.25 mmol) were added, the obtained system was stirred evenly and reacted at 80°C for 16 h. After the reaction was completed, the mixture was cooled to room temperature, water (30 mL) was added, and ethyl acetate (30 mL) was used to extract the mixture. The pH of the aqueous phase was adjusted to 1-2 with dilute hydrochloric acid (1N), and the mixture was extracted with ethyl acetate (30 mL×3). The organic phases were combined, washed with saturated brine, dried over anhydrous sodium sulfate, filtered, and concentrated to obtain the crude compound **93B** (0.6 g, 37.7%).

LCMS (ESI): m/z =213.2 [M+H]⁺.

Step 3: Compound **93B** (0.6 g, 2.82 mmol) was dissolved in trifluoroacetic acid (5 mL) and the reaction was performed at room temperature for 16 h. After the reaction was completed, the obtained system was concentrated and the residue was purified by silica gel column chromatography (dichloromethane:methanol (v/v) = 10:1) to obtain compound **93C** (350 mg, 73.9%).

LCMS (ESI): m/z =169.1 [M+H]⁺.

Step 4: Compound **93C** (350 mg, 2.08 mmol) and compound **73H-2** (562 mg, 2.08 mmol) were dissolved in ethanol (500 mL), a drop of glacial acetic acid was added, and the reaction was performed at 50°C for 16 h, then sodium cyanoborohydride (262 mg, 4.16 mmol) was added at room temperature, and the reaction was performed at room temperature for 1 h. After the reaction was completed, the obtained system was concentrated and the residue was separated by silica gel column chromatography (dichloromethane:methanol (v/v) = 10:1) to obtain compound **93D** (250 mg, 28.5%).

LCMS (ESI): m/z =423.2 [M+H]⁺.

Step 5: Compound **93D** (250 mg, 0.59 mmol) was dissolved in dichloromethane (3 mL), trifluoroacetic acid (1 mL) was added, and the reaction was performed at room temperature for 1 h. After the reaction was completed, the crude product **93E** (260 mg, 100%) was obtained after concentration.

LCMS (ESI): m/z =323.2 [M+H]⁺.

Step 6: Compound **93E** (260 mg, 0.59 mmol) was dissolved in dimethyl sulfoxide (3 mL), compound **2F** (205 mg, 0.59 mmol), triethylamine (298 mg, 2.95 mmol) and cesium fluoride (179 mg, 1.18 mmol) was added in sequence, the obtained system was stirred evenly and reacted at 100°C for 6 h. After the reaction was completed, the mixture was cooled to room temperature, water (30 mL) was added, and the mixture was extracted with ethyl acetate (10 mL×3). The combined organic phases were washed with water (10 mL), washed with saturated brine (10 mL), dried over anhydrous sodium sulfate, filtered, concentrated, and separated by silica gel column chromatography (dichloromethane:methanol (v/v) = 10:1) to obtain the title **compound 93** isomer 1 (Rf=0.6 (dichloromethane:methanol=10:1), 30.0 mg, 8%) and the title **compound 93** isomer 2 (Rf=0.3 (dichloromethane:methanol=10:1), 25 mg, 6%).

**Compound 93 isomer 1:** LC-MS (ESI): m/z =636.2 [M+H]⁺.

¹H NMR (400 MHz, DMSO-d₆) δ 7.54-7.51 (m, 1H), 7.46-7.41 (m, 1H), 7.38- 7.34 (m, 1H), 7.27-7.21 (m, 1H), 5.51-5.24 (m, 1H), 4.50-4.38 (m, 2H), 3.79-3.72 (m, 1H), 3.43-3.35 (m, 1H), 3.07-3.01 (m, 1H), 2.95-2.88 (m, 1H), 2.62-2.51 (m, 4H), 2.25 -2.17 (m, 5H), 2.09-1.99 (m, 2H), 1.87-1.77 (m, 1H), 1.63-1.41 (m, 10H), 1.32-1.21 (m, 1H), 1.11-0.95 (m, 1H), 0.71 (s, 1H).

**Compound 93 isomer 2:** LC-MS (ESI): m/z =636.2 [M+H]⁺.

¹H NMR (400 MHz, DMSO-*d*₆) δ 7.55-7.51 (m, 1H), 7.45-7.41 (m, 1H), 7.39- 7.34 (m, 1H), 7.26-7.22 (m, 1H), 5.42-5.30 (m, 1H), 4.55-4.35 (m, 2H), 3.82-3.69 (m, 1H), 3.46-3.33 (m, 3H), 3.08-2.95 (m, 1H), 2.73-2.52 (m, 5H), 2.19 (s, 3H), 1.90-1.74 (m, 3H), 1.58-1.38 (m, 10H), 1.27-1.22 (m, 1H), 1.10-0.97 (m, 1H), 0.77 (s, 1H).

### Example 94:

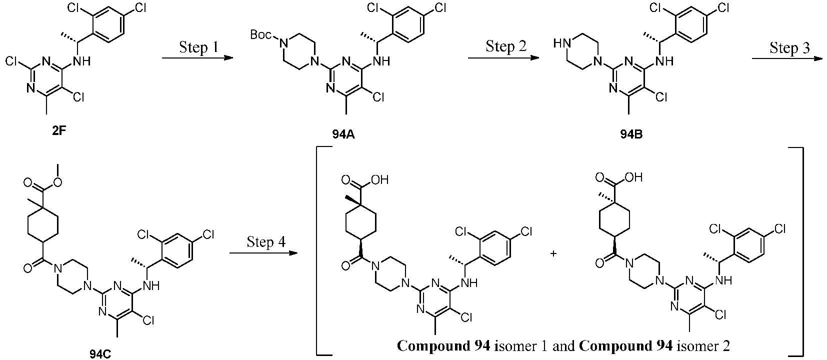

Step 1: Compound **2F** (200.0 mg, 0.57 mmol), N-Boc-piperazine (64.0 mg, 0.75 mmol) and triethylamine (172.7 mg, 1.71 mmol) were dissolved in dimethyl sulfoxide (14 mL), the obtained system was stirred evenly and reacted at 150°C for 0.5 h under microwave. After the reaction was completed as detected by TLC, the obtained system was cooled to room temperature, water (30 mL) was added, and ethyl acetate (10 mL ×3) was used for extraction. The combined organic phase was washed with saturated saline solution, dried over anhydrous sodium sulfate, filtered, and concentrated. The crude product was separated by silica gel column chromatography (dichloromethane:methanol (v/v) = 10:1-5:1) to obtain the title compound **94A** (242.0 mg, 85%).

LC-MS (ESI): m/z = 500.2 [M+H]⁺.

Step 2: Compound **94A** (242 mg, 0.48 mmol) was dissolved in dichloromethane (8 mL), hydrogen chloride dioxane solution (8 mL) was added, after the addition was completed, the reaction was performed at room temperature for 1 h. Concentration gave the title compound **94B** as a hydrochloride salt. The obtained compound could be directly used in the next reaction without further purification.

LC-MS (ESI): m/z = 400.2 [M+H]⁺.

Step 3: 4-(methoxycarbonyl)-4-methylcyclohexanecarboxylic acid (96 mg, 0.48 mmol) was dissolved in N,N-dimethylformamide (8 mL), and DIPEA (186 mg, 1.44 mmol) and HATU (201 mg, 0.53 mmol) were added in sequence under an ice bath, and stirred for 15 min, then the crude product **94B** from the previous step was added, and then the obtained system was warmed to room temperature and stirred for 1 h. 50 mL of water was added to the reaction liquid, and the mixture was extracted with ethyl acetate (20 mL×3). The organic layers were combined, washed with saturated brine (40 mL), and dried over anhydrous sodium sulfate. The residue was concentrated and purified using a medium-pressure preparation instrument Biotage Isolera One (12 g silica gel column, eluent: 0-10% MeOH/DCM) to obtain the compound **94C** of interest (181 mg, two-step yield: 65%).

LC-MS (ESI): m/z = 582.2 [M+H]⁺.

Step 4: Compound **94C** (181 mg, 0.31 mmol) was dissolved in N,N-dimethylformamide (5 mL), lithium hydroxide monohydrate (66 mg, 1.56 mmol) was added, the obtained system was heated to 60°C and stirred overnight. After the reaction was completed, the mixture was cooled to room temperature, 6N dilute hydrochloric acid was added dropwise to adjust the pH to 6-7, and the mixture was concentrated to obtain a crude product. The crude product was purified by preparative high performance liquid phase chromatography to obtain **compound 94** isomer 1 (35 mg, retention time 3.89 min, yield: 19.58%) and **compound 94** isomer 2 (37 mg, retention time 4.04 min, yield: 21.09%).

HPLC analytical method: 1. Instrument: Shimadzu LC-20AT; 2. Chromatographic column: Xtimate C18 4.6*50mm, 3µm; 3. Mobile phase A: 0.05% TFA solution, mobile phase B: acetonitrile; 4. Gradient: A 95-5% B 5-95%; 5. Flow rate: 1 mL/min, column temperature: 35°C, wavelength: 210 nm/254 nm, acquisition time: 10 min.

High performance liquid phase preparation method: 1. Instruments: waters 2767 preparative liquid chromatographic instrument; chromatographic column: SunFire @ Prep C18 (19 mm x 250 mm). 2. The sample was filtered with a 0.45 µm filter head to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min;
Compound **94,** isomer 1: LC-MS (ESI): m/z =568.2 [M+H]⁺.

¹H NMR (400 MHz, Methanol-*d*₄) δ 7.44-7.41 (m, 1H), 7.36-7.31 (m, 1H), 7.25-7.21 (m, 1H), 5.44-5.41 (m, 1H), 3.83-3.36 (m, 8H), 2.64-2.61 (m, 1H), 2.27 (s, 3H), 1.95-1.57 (m, 5H), 1.51-1.48 (m, 3H), 1.36-1.26 (m, 3H), 1.24 (s, 3H).

Compound **94,** isomer 2: LC-MS (ESI): m/z =568.2 [M+H]⁺.

¹H NMR (400 MHz, Methanol-*d*₄) δ 7.44-7.41 (m, 1H), 7.36-7.31 (m, 1H), 7.25-7.21 (m, 1H), 5.44-5.41 (m, 1H), 3.80-3.33 (m, 8H), 2.62-2.58 (m, 1H), 2.27 (s, 3H), 1.63-1.61 (m, 3H), 1.51-1.48 (m, 3H), 1.41-1.21 (m, 5H), 1.18 (s, 3H).

### Example 95:

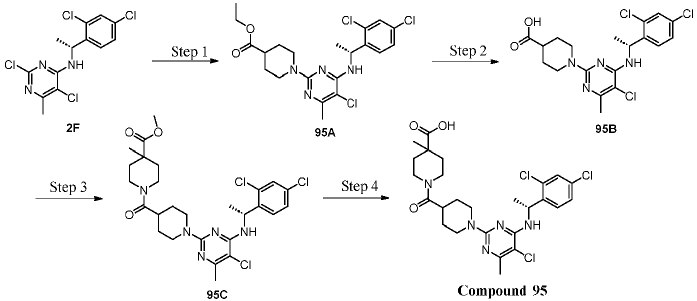

Step 1: Compound **2F** (300.0 mg, 0.86 mmol), ethyl 4-piperidinecarboxylate (168.0 mg, 0.89 mmol), and triethylamine (270.0 mg, 2.67 mmol) were dissolved in dimethyl sulfoxide (14 mL) in sequence, the obtained system was stirred evenly, heated to 150°C and reacted for 0.5 h under microwave. After the reaction was completed as detected by TLC, the mixture was cooled to room temperature, water (30 mL) was added, and ethyl acetate (10 mL×3) was used for extraction. The combined organic phases were washed with saturated saline solution, dried over anhydrous sodium sulfate, filtered, and concentrated. The crude product was separated by silica gel column chromatography (dichloromethane:methanol (v/v) = 10:1-5:1) to obtain the title compound **95A** (343.0 mg, 85%).

LC-MS (ESI): m/z = 471.2 [M+H]⁺.

Step 2: Compound **95A** (343 mg, 0.73 mmol) was dissolved in tetrahydrofuran (3 mL) and water (3 mL), lithium hydroxide hydrate (153.0 mg, 3.65 mmol) was added, and the obtained system was stirred evenly and reacted at room temperature for 12 h. After the reaction was completed, the pH was adjusted to 4-5 with dilute hydrochloric acid, the obtained system was extracted with dichloromethane (5 mL×3), and the organic phases were combined and concentrated under reduced pressure to obtain the crude title compound **95B** (340 mg).

LC-MS (ESI): m/z = 443.2 [M+H]⁺.

Step 3: Compound **95B** (170 mg, 0.38 mmol) was dissolved in N,N-dimethylformamide (8 mL), DIPEA (149 mg, 1.15 mmol) and HATU (159 mg, 0.42 mmol) were added in sequence under an ice bath, and stirred for 15 min, methyl 4-methylpiperidine-4-carboxylate (67 mg, 0.42 mmol) was added, and then the obtained system was warmed to room temperature and stirred for 1 h. 50 mL of water was added to the reaction liquid, and the mixture was extracted with ethyl acetate (20 mL×3). The organic layers were combined, washed with saturated brine (40 mL), and dried over anhydrous sodium sulfate. After concentration, the residue was purified using a medium-pressure preparation instrument Biotage Isolera One (12 g silica gel column, eluent: 0-10% MeOH/DCM) to obtain the compound **95C** of interest (128 mg, 58%).

LC-MS (ESI): m/z = 582.2 [M+H]⁺.

Step 4: Compound **95C** (128 mg, 0.22 mmol) was dissolved in tetrahydrofuran (3 mL) and water (3 mL), lithium hydroxide hydrate (46.0 mg, 1.1 mmol) was added, and the mixture was stirred evenly and reacted at room temperature for 12 h. After the reaction was completed, the mixture was concentrated under reduced pressure and the residue was separated and purified by preparative high performance liquid chromatography to obtain the title **compound 95** (68 mg, 55%).

Preparation method: 1. Instruments: waters 2767 preparative liquid chromatographic instrument; chromatographic column: SunFire @ Prep C18 (19 mm x 250 mm). 2. The sample was filtered with a 0.45 µm filter head to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min;
¹H NMR (400 MHz, Methanol-*d*₄) δ 7.40-7.36 (m, 1H), 7.34-7.30 (m, 1H), 7.23-7.20 (m, 1H), 5.42-5.38 (m, 1H), 4.64-4.42 (m, 3H), 4.19-4.16 (m, 1H), 3.99-3.96 (m, 1H), 3.69-3.65 (m, 1H), 3.33-3.21 (m, 4H), 2.98-2.64 (m, 2H), 2.48-2.44 (m, 1H), 2.26 (s, 3H), 1.70-1.39 (m, 10H).

LC-MS (ESI): m/z = 568.2 [M+H]⁺.

### Example 96:

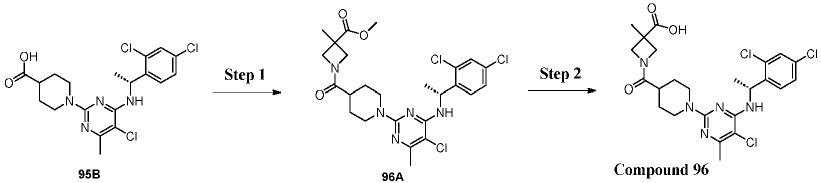

Step 1: Compound **95B** (170 mg, 0.38 mmol) was dissolved in N,N-dimethylformamide (8 mL), DIPEA (149 mg, 1.15 mmol) and HATU (159 mg, 0.42 mmol) was added in sequence under an ice bath, and stirred for 15 min, then methyl 3-methylazetidine-3-carboxylate (54 mg, 0.42 mmol) was added, and then the obtained system was warmed to room temperature and stirred for 1 h. 50 mL of water was added to the reaction liquid, and the mixture was extracted with ethyl acetate (20 mL×3). The organic layers were combined, washed with saturated brine (40 mL), and dried over anhydrous sodium sulfate. After concentration, the residue was purified using a medium-pressure preparation instrument Biotage Isolera One (12 g silica gel column, eluent: 0-10% MeOH/DCM) to obtain the compound **96A** of interest (109 mg, 52%).

LC-MS (ESI): m/z = 554.2 [M+H]⁺.

Step 2: Compound **96A** (109 mg, 0.20 mmol) was dissolved in tetrahydrofuran (3 mL) and water (3 mL), lithium hydroxide hydrate (42.0 mg, 0.99 mmol) was added, and the obtained system was stirred evenly and reacted at room temperature for 12 h. After the reaction was completed, the mixture was concentrated under reduced pressure and the residue was separated and purified by preparative high performance liquid chromatography to obtain the title **compound 96** (45 mg, 45%).

Preparation method: 1. Instruments: waters 2767 preparative liquid chromatographic instrument; chromatographic column: SunFire @ Prep C18 (19 mm x 250 mm). 2. The sample was filtered with a 0.45 µm filter head to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min;
¹H NMR (400 MHz, Methanol-*d*₄) δ 7.39-7.36 (m, 1H), 7.34-7.31 (m, 1H), 7.27 - 7.19-7.16 (m, 1H), 5.42-5.36 (m, 1H), 4.51-4.46 (m, 2H), 4.18-4.03 (m, 1H), 3.85-3.81 (m, 1H), 3.08-2.65 (m, 4H), 2.26 (s, 3H), 2.17-2.14 (m, 1H), 1.65-1.35 (m, 7H), 1.24-1.21 (m, 3H).

LC-MS (ESI): m/z = 540.2 [M+H]⁺.

### Example 97:

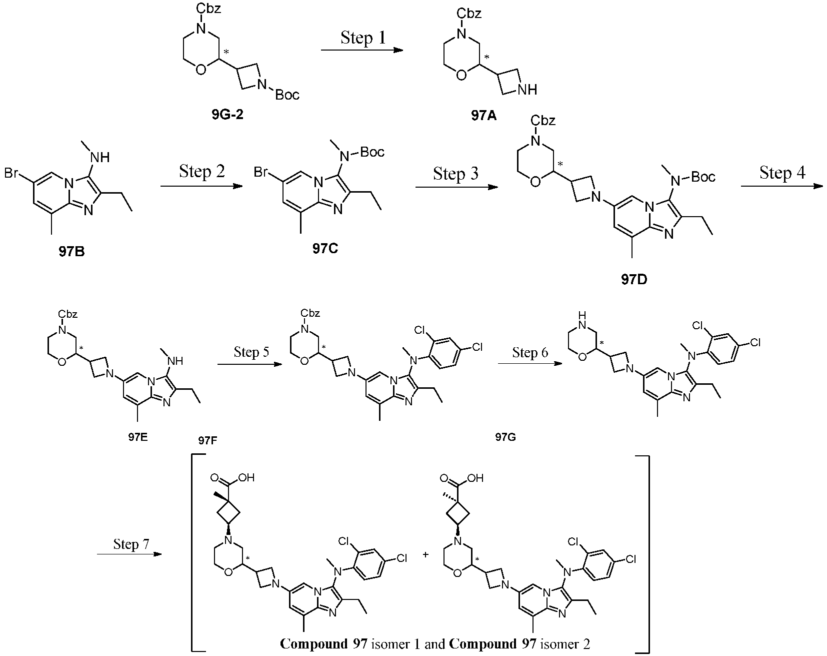

Step 1: Compound **9G-2** (752 mg, 2.00 mmol) was dissolved in dichloromethane (8 mL), trifluoroacetic acid (4 mL) was added, and the obtained system was stirred at room temperature for 2 h. The reaction liquid was concentrated, dichloromethane (4 mL) was added, and the pH was adjusted to 6-7 with triethylamine. The system was concentrated to obtain the crude compound **97A** of interest. The obtained compound could be directly used in the next reaction without further purification.

LC-MS (ESI): m/z = 277.2 [M+H]⁺.

Step 2: Compound **97B** (510 mg, 1.91 mmol) was dissolved in di-tert-butyl dicarbonate (10 mL), DMAP (35 mg, 0.28 mmol) was added, and the obtained system was reacted under reflux for 12 h. After the reaction was completed, the mixture was cooled to room temperature and most of the di-tert-butyl dicarbonate was concentrated. The crude product after the concertration was separated and purified by silica gel column chromatography (petroleum ether:ethyl acetate (v/v) = 3:1-2:1) to obtain the title compound **97C** (547 mg, 78%).

LC-MS (ESI): m/z = 368.2 [M+H]⁺.

Step 3: Compound **97C** (547 mg, 1.49 mmol), compound **97A** (619 mg, 2.24 mmol), Pd₂(dba)₃ (205 mg, 0.22 mmol), JohnPhos (131 mg, 0.44 mmol) and sodium tert-butoxide (572 mg, 5.96 mmol) were added to toluene (15 mL) in sequence and the reaction was performed at 100°C for 4 h. After the reaction was completed, the mixture was cooled to room temperature, water (20 mL) was added, and the mixture was extracted with ethyl acetate (20 mL×3). The combined organic phases were washed with saturated brine (20 mL), dried over anhydrous sodium sulfate, and filtered, and the filtrate was concentrated under reduced pressure. The residue was separated by silica gel column chromatography (DCM:MeOH (v/v) = 10:1) to obtain the compound **97D** of interest (462 mg, yield: 55%).

LC-MS (ESI): m/z = 564.2 [M+H]⁺.

Step 4: Compound **97D** (462 mg, 0.82 mmol) was dissolved in dichloromethane (8 mL), trifluoroacetic acid (4 mL) was added, and the obtained system was stirred at room temperature for 2 h. The reaction liquid was concentrated, dichloromethane (4 mL) was added, and the pH was adjusted to 6-7 with triethylamine. The system was concentrated to obtain the crude compound **97E** of interest. The obtained compound could be directly used in the next reaction without further purification.

LC-MS (ESI): m/z =464.2 [M+H]⁺.

Step 5: The crude compound **97E** from the previous step, 2,4-dichloro-1-iodobenzene (289 mg, 1.06 mmol), Pd₂(dba)₃ (0.11 g, 0.12 mmol), Xphos (114 mg, 0.24 mmol), and cesium carbonate (802 mg, 2.46 mmol) were added to toluene (15 mL) in sequence and the reaction was performed at 100°C for 4 h. After the reaction was completed, the mixture was cooled to room temperature, water (20 mL) was added, and the mixture was extracted with ethyl acetate (20 mL×3). The combined organic phases were washed with saturated brine (20 mL), dried over anhydrous sodium sulfate, and filtered, and the filtrate was concentrated under reduced pressure. The residue was separated by silica gel column chromatography (DCM:MeOH (v/v) = 10:1) to obtain the compound **97F** of interest (200 mg, two-step yield: 40%).

LC-MS (ESI): m/z = 608.2 [M+H]⁺.

Step 6: Compound **97F** (200 mg, 0.33 mmol) was dissolved in dichloromethane (50 mL), trifluoromethanesulfonic acid (1 mL) was added under an ice bath, and the obtained system was stirred for 2 h. After the reaction was completed, saturated sodium bicarbonate aqueous solution was added to adjust the pH to 7-8, saturated brine (40 mL) was added, and extraction was performed. The organic phase was dried over anhydrous sodium sulfate, filtered, and concentrated to obtain the crude compound **97G** of interest. The obtained compound could be directly used in the next reaction without further purification.

LC-MS (ESI): m/z =474.2 [M+H]⁺.

Step 7: The crude compound **97G** from the previous step and 3-carbonyl-1-methyl-cyclobutanecarboxylic acid (63 mg, 0.49 mmol) were dissolved in methanol (10 mL), two drops of acetic acid were added dropwise, and the obtained system was stirred at room temperature for 6 h, then sodium cyanoborohydride (42 mg, 0.66 mmol) was added under an ice bath, the obtained system was warmed to room temperature naturally and stirred for 1h. After the reaction was completed as detected by TLC, the reaction liquid was concentrated. Water (20 mL) was added and the obtained system was extracted with dichloromethane (20 mL×2). The organic phase was washed with water (20 mL), washed with brine (20 mL), dried over anhydrous sodium sulfate, filtered and concentrated to obtain a crude product. The crude product was purified by preparative high performance liquid phase chromatography to obtain **compound 97** isomer 1 (35 mg, retention time 3.33 min, two-step yield: 17.58%) and **compound 97** isomer 2 (29 mg, retention time 3.34 min, two-step yield: 15.09%).

HPLC analytical method: 1. Instrument: Shimadzu LC-20AT; 2. Chromatographic column: Xtimate C18 4.6*50mm, 3µm; 3. Mobile phase A: 0.05% TFA solution, mobile phase B: acetonitrile; 4. Gradient: A 95-5% B 5-95%; 5. Flow rate: 1 mL/min, column temperature: 35°C, wavelength: 210 nm/254 nm, acquisition time: 10 min.

Preparation method: 1. Instruments: waters 2767 preparative liquid chromatographic instrument; chromatographic column: SunFire @ Prep C18 (19 mm x 250 mm). 2. The sample was filtered with a 0.45 µm filter head to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 10%-55%; c. flow rate: 12 mL/min;

Compound **97,** isomer 1: LC-MS (ESI): m/z =586.2 [M+H]⁺.

¹H NMR (400 MHz, Methanol-*d*₄) δ 7.33-7.18 (m, 3H), 6.97-6.93 (m, 1H), 6.58-6.54 (m, 1H), 3.89-3.50 (m, 7H), 3.13 (s, 3H), 2.88-2.60 (m, 4H), 2.55-2.37 (m, 5H), 2.24-2.21 (m, 2H), 1.97-1.49 (m, 4H), 1.22-1.19 (m, 6H).

Compound **97,** isomer 2: LC-MS (ESI): m/z =586.2 [M+H]⁺.

¹H NMR (400 MHz, Methanol-*d*₄) δ 7.35-7.20 (m, 3H), 7.02-6.98 (m, 1H), 6.66-6.63 (m, 1H), 3.93-3.50 (m, 7H), 3.14 (s, 3H), 2.99-2.62 (m, 4H), 2.41 (s, 3H), 2.27-2.21 (m, 4H), 2.03-1.98 (m, 4H), 1.24-1.12 (m, 6H).

### Example 98:

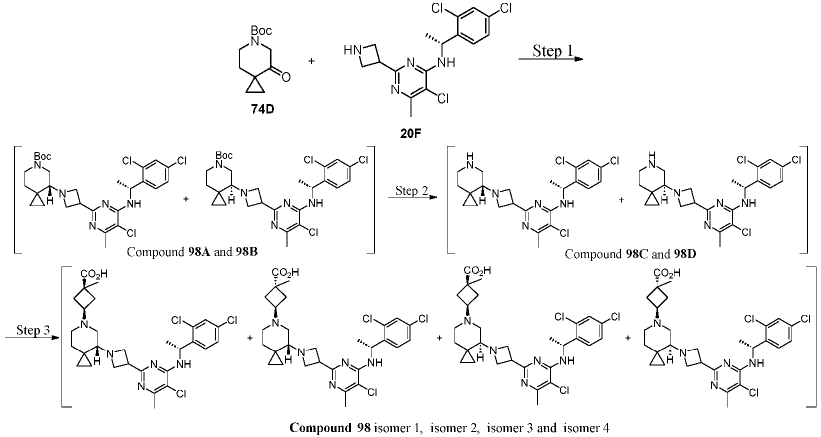

Step 1: The crude trifluoroacetate salt of compound **20F** (1.40 g, 2.33 mmol) was dissolved in dry 1,2-dichloroethane (100 mL), and 74**D** (789 mg, 3.50 mmol) and acetic acid (140 mg, 2.33 mmol) were added in sequence, and stirring was continued at 50°C for 1 h, then sodium triacetoxyborohydride (989 mg, 4.67 mmol) was added, and the reaction was continued at 50°C. After the disappearance of the raw materials as monitored by TLC, water (2 mL) was added to quench the reaction and the mixture was concentrated. The resulting crude product was purified by silica gel column chromatography (dichloromethane:methanol (v/v) = 90:10) to obtain a mixture of compounds **98A** and **98B** (450 mg, 33.2%).

The above mixture was separated by chiral HPLC to obtain compound **98A** (147 mg, 10.8%) and compound **98B** (104 mg, 7.68%). Chiral preparation method: instrument: Waters 150 MGM; chromatographic column: Chiralpak Column; mobile phase: A: carbon dioxide, and B: methanol (0.1% aqueous ammonia); isocratic elution: 35% mobile phase B; flow rate: 100 mL/min; back pressure: 100 bar; column temperature: 25°C; wavelength: 220 nm.

HPCL analytical method: 1. Instruments: SHIMADZU LC-30AD SFC; 2. Chromatographic column: Chiralcel OJ-3 50×4.6mm I.D., 3µm; 3. Mobile phase system: A for CO2, B for 0.05%DEA in MeOH; 4. Gradient: B 5%-40%; 5. Flow rate: 3 mL/min. Compound **98A,** retention time 1.07 min; Compound **98B,** retention time 1.19 min.

LC-MS (ESI): m/z =580.2 [M+H]⁺;

Step 2: Compound **98A** (140 mg, 0.241 mmol) was dissolved in dichloromethane (6 mL), and trifluoroacetic acid (2 mL) was added at room temperature. The reaction was continued for 1 h after the addition was completed. After the reaction was completed as monitored by LCMS, the obtained system was concentrated to obtain the crude trifluoroacetate salt of **98C** (270 mg), which was used directly in the next step without purification.

LC-MS (ESI): m/z = 480.1 [M+H]⁺;

Using **98B** (100 mg, 17.2 mmol) as the raw material, the crude trifluoroacetate salt of compound **98D** (220 mg) was obtained by referring to the above synthesis method.

Step 3: The crude trifluoroacetate salt (270 mg) of compound **98C** obtained in the previous step was dissolved in methanol (5 mL), and compound **2B** (73.2 mg, 0.571 mmol) and acetic acid (22.9 mg, 0.381 mmol) were added in sequence, and stirred at room temperature for 1 h, then sodium cyanoborohydride (47.9 mg, 0.762 mmol) was added, and stirring was continued for 2 h. After the reaction was completed as monitored by LCMS, water (1 mL) was added to quench the reaction. The reaction liquid was separated and purified by preparative HPLC to obtain **compound 98** isomer 1 (31 mg, two-step yield: 21.9%) and **compound 98** isomer 2 (47 mg, two-step yield: 32.9%).

Preparative HPLC separation and purification method: 1. Instruments: waters 2767 preparative liquid chromatographic instrument; chromatographic column: SunFire @ Prep C18 (19 mm x 250 mm). 2. The sample was filtered with a 0.45

µm filter head to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 5%-50%; c. flow rate: 12 mL/min; d. elution time: 30 min.

HPCL analysis conditions: 1. Instruments: Shimadzu LC-20AT; 2. Chromatographic column: Xtimate C18 4.6*50mm, 3µm; 3. Mobile phase system: A for 0.05% TFA in H₂O; B for ACN; 4. Gradient: B 5-95%; 5. Flow rate: 1.0 mL/min, run time: 10 min. retention time: **Compound 98** isomer 1 retention time: tR = 3.599 min; **Compound 98** isomer 2 retention time: tR = 3.669 min.

**Compound 98** isomer 1: ¹H NMR (400 MHz, Methanol-*d*₄) *δ* 7.45 (d, 1H), 7.36 (d, 1H), 7.27 (dd, 1H), 5.53 (q, 1H), 3.83-3.64 (m, 3H), 3.57 (t, 1H), 3.39 (q, 1H), 3.24-3.07 (m, 3H), 2.81-2.50 (m, 4H), 2.42 (s, 3 H), 2.30 (td, 1H), 2.03 (dd, 1H), 1.86 (s, 1H), 1.77 (dd, 1H), 1.56 (d, 3H), 1.37 (s, 3H), 0.87 (dd, 1H), 0.67-0.32 (m, 4H).

LC-MS (ESI): m/z =592.2 [M+H]⁺;

**Compound 98** isomer 2: ¹H NMR (400 MHz, Methanol-*d*₄) *δ* 7.44 (d, 1H), 7.36 (d, 1H), 7.27 (dd, 1H), 5.51 (q, 1H), 3.82 (td, 1H), 3.79-3.66 (m, 2H), 3.56 (t, 1H), 3.39 (p, 1H), 3.29-3.23 (m, 1H), 3.22-3.03 (m, 2H), 2.69 (dd, 1H), 2.61- 2.47 (m, 2H), 2.41 (s, 3H), 2.38-2.27 (m, 2H), 2.23-2.06 (m, 2H), 1.83 (s, 1H), 1.56 (d, 3H), 1.36 (s, 3H), 0.86 (dd, 1H), 0.65-0.48 (m, 2H), 0.40 (ddt, 2H).

LC-MS (ESI): m/z =592.2 [M+H]⁺;

Using the crude trifluoroacetate salt of **98D** (220 mg) as the raw material, **compound 98** isomer 3 (22 mg, two-step yield: 21.5%) and **compound 98** isomer 4 (46 mg, two-step yield: 45.1%) were obtained by referring to the above synthesis method.

HPCL analysis conditions: 1. Instruments: Shimadzu LC-20AT; 2. Chromatographic column: Xtimate C18 4.6*50mm, 3µm; 3. Mobile phase system: A for 0.05% TFA in H₂O; B for ACN; 4. Gradient: B 5-95%; 5. Flow rate: 1.0 mL/min, run time: 10 min. retention time: **Compound 98** isomer 3 retention time: 3.628 min; **Compound 98** isomer 4 retention time: 3.715 min.

**Compound 98** isomer 3: ¹H NMR (400 MHz, Methanol-*d*₄) *δ* 7.46 (d, 1H), 7.33 (d, 1H), 7.26 (dd, 1H), 5.47 (q, 1H), 3.85 (td, 1H), 3.76-3.65 (m, 2H), 3.52 (t, 1H), 3.40-3.30 (m, 1H), 3.25-3.14 (m, 1H), 3.07-2.90 (m, 2H), 2.78-2.50 (m, 4H), 2.46-2.30 (m, 4H), 2.02 (dd, 1H), 1.90 (s, 1H), 1.72 (dd, 1H), 1.58 (d, 3H), 1.36 (s, 3H), 0.93-0.78 (m, 2H), 0.58 (dt, 1H), 0.50-0.36 (m, 2H).

LC-MS (ESI): m/z =592.2 [M+H]⁺;

**Compound 98** isomer 4: ¹H NMR (400 MHz, Methanol-*d*₄) *δ* 7.45 (d, 1H), 7.33 (d, 1H), 7.26 (dd, 1H), 5.46 (q, 1H), 3.85 (td, 1H), 3.80-3.66 (m, 2H), 3.49 (t, 1H), 3.39-3.31 (m, 1H), 3.30-3.23 (m, 1H), 3.00 (dt, 2H), 2.64 (dd, 1H), 2.57- 2.48 (m, 2H), 2.40 (s, 4H), 2.26 (dd, 1H), 2.20-2.06 (m, 2H), 1.88 (s, 1H), 1.57 (d, 3H), 1.35 (s, 3H), 0.94-0.78 (m, 2H), 0.59 (dt, 1H), 0.42 (ddt, 2H).

LC-MS (ESI): m/z =592.2 [M+H]⁺.

### Example 99:

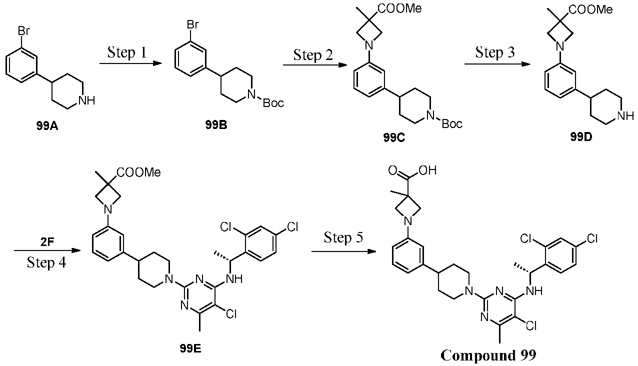

Step 1: **99A** (500 mg, 2.07 mmol) and triethylamine (314 mg, 3.10 mmol) were dissolved in tetrahydrofuran (10 mL), then di-tert-butyl dicarbonate (490 mg, 2.26 mmol) was added, the reaction was performed at room temperature overnight. After the reaction was completed, 40 mL of water was added to the reaction liquid, and the mixture was extracted with ethyl acetate (25 mL×3). The organic layers were combined, washed with saturated brine (30 mL), dried over anhydrous sodium sulfate, filtered, and concentrated. The residue was separated and purified by silica gel column chromatography (petroleum ether:ethyl acetate (v/v) = 10:1) to obtain **99B** (688 mg, 97%).

LC-MS (ESI): m/z =284.2 [M+H-56]⁺.

Step 2: **99B** (688 mg, 2.03 mmol) and methyl 3-methylazetidine-3-carboxylate (262 mg, 2.03 mmol) were dissolved in 1,4-dioxane (20 mL), then Pd₂(dba)₃ (186 mg, 0.20 mmol), 4,5-bis(diphenylphosphino)-9,9-dimethylxanthene (235 mg, 0.41 mmol) and cesium carbonate (992 mg, 3.04 mmol) were added, the reaction was performed overnight at 80°C under a nitrogen atmosphere. After cooling to room temperature, 50 mL of water was added to the reaction liquid, and the mixture was extracted with ethyl acetate (25 mL×3). The organic layers were combined, washed with saturated brine (30 mL), dried over anhydrous sodium sulfate, filtered, and concentrated. The residue was separated and purified by silica gel column chromatography (petroleum ether:ethyl acetate (v/v) = 5:1) to obtain **99C** (260 mg, 33%).

LC-MS (ESI): m/z = 389.3 [M+H]⁺.

Step 3: **99C** (260 mg, 0.67 mmol) was dissolved in dichloromethane (6 mL), a solution of 4M hydrogen chloride in 1,4-dioxane (2 mL) was added dropwise under an ice bath, and the reaction was performed at room temperature for1 h. The reaction liquid was concentrated, and the residue was separated and purified by silica gel column chromatography (dichloromethane:methanol (v/v) = 40:1) to obtain **99D** (178 mg, 92%).

LC-MS (ESI): m/z = 289.2 [M+H]⁺.

Step 4: **99D** (178 mg, 0.62 mmol) and **2F** (217 mg, 0.62 mmol) were dissolved in DMSO (10 mL), then cesium fluoride (103 mg, 0.68 mmol) and N,N-diisopropylethylamine (400 mg, 3.10 mmol) were added, the reaction was perform under nitrogen atmosphere at 100°C overnight. After cooling to room temperature, 40 mL of water was added to the reaction liquid, and the mixture was extracted with ethyl acetate (25 mL×3). The organic layers were combined, washed with saturated brine (30 mL), dried over anhydrous sodium sulfate, filtered, and concentrated. The residue was separated and purified by silica gel column chromatography (petroleum ether:ethyl acetate (v/v) = 10:1) to obtain **99E** (315 mg, 84%).

LC-MS (ESI): m/z = 602.2 [M+H]⁺.

Step 5: **99E** (315 mg, 0.52 mmol) was dissolved in a mixed solvent of tetrahydrofuran (4 mL) and water (4 mL), then lithium hydroxide monohydrate (109 mg, 2.60 mmol) was added, and reaction was performed at room temperature for 3 h. 20 mL of water was added to the reaction liquid, and the mixture was extracted with ethyl acetate (10 mL×4). The organic layers were combined, dried over anhydrous sodium sulfate, filtered, and concentrated. The residue was separated and purified by silica gel column chromatography (dichloromethane:methanol (v/v) = 20:1) to obtain **compound 99** (142 mg, 46%).

¹H NMR (400 MHz, CD₃Cl) δ 7.32-7.31 (m, 1H), 7.26-7.25 (m, 1H), 7.19-7.11 (m, 2H), 6.58-6.55 (m, 1H), 6.32-6.29 (m, 1H), 6.25 (s, 1H), 5.48-5.38 (m, 2H), 4.67-4.63 (m, 2H), 4.18-4.15 (m, 2H), 3.70-3.67 (m, 2H), 2.78-2.55 (m, 3H), 2.31 (s, 3H), 1.80-1.69 (m, 2H), 1.64 (s, 3H), 1.58-1.49 (m, 4H).

LC-MS (ESI): m/z = 588.2 [M+H]⁺.

### Example 100:

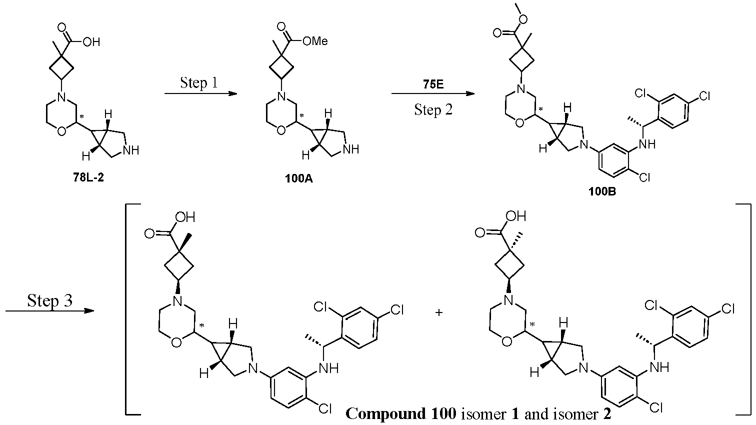

Step 1: The trifluoroacetate salt of compound **78L-2** (500 mg, 0.983 mmol) was dissolved in methanol (10 mL), thionyl chloride (468 mg, 3.93 mmol) was added at 0°C, after the addition was completed, stirring was continued at room temperature. After the complete disappearance of the raw materials as monitored by LCMS, the obtained system was directly concentrated and the crude product was purified by silica gel column chromatography (dichloromethane:methanol:aqueous ammonia (v/v/v) = 80:20:0.5) to obtain compound **100A** (277 mg, 95.7%).

LC-MS (ESI): m/z =295.2 [M+H]⁺.

Step 2: Using **100A** (150 mg, 0.510 mmol) and **75E** (343 mg, 0.764 mmol) as the raw materials, the operation method of step 2 of example 82 was referenced to obtain compound **100B** (202 mg, 66.9%).

LC-MS (ESI): m/z =592.2 [M+H]⁺.

Step 3: Using **100B** (202 mg, 0.341 mmol) as the raw material, the operation method of step 3 of example 82 was referenced to obtain compound **100** isomer 1 (41 mg, 20.8%) and compound **100** isomer 2 (10 mg, 5.07%).

HPLC analysis conditions: 1. Instruments: Shimadzu LC-20AT; 2. Chromatographic column: Xtimate C18 4.6*50mm, 3µm; 3. Mobile phase system: A for 0.05% TFA in H₂O; B for ACN; 4. Gradient: B 5-95%; 5. Flow rate: 1.0 mL/min, run time: 10 min. retention time: **Compound 100** isomer 1 retention time: tR = 4.404 min; **Compound 100** isomer 2 retention time: tR = 4.439 min.

**Compound 100** isomer 1: ¹H NMR (400 MHz, Methanol-d4) *δ* 7.47 (d, 1H), 7.40 (d, 1H), 7.25 (dd, 1H), 6.94 (d, 1H), 5.82 (dd, 1H), 5.42 (d, 1H), 4.92 (d, 1H), 3.97 - 3.86 (m, 1H), 3.59 (td, 1H), 3.35 (d, 1H), 3.25 - 3.17 (m, 1H), 3.10 - 2.95 (m, 4H), 2.92 - 2.80 (m, 2H), 2.64 (dd, 2H), 2.24 - 2.14 (m, 1H), 2.04 (t, 1H), 1.96 - 1.84 (m, 2H), 1.71 (dt, 1H), 1.64 (dt, 1H), 1.52 (d, 3H), 1.38 (s, 3H), 0.74 (dt, 1H).

LC-MS (ESI): m/z =578.2 [M+H]⁺;

**Compound 100** isomer 2: ¹H NMR (400 MHz, Methanol-*d*₄) δ 7.47 (d, 1H), 7.40 (d, 1H), 7.25 (dd, 1H), 6.95 (d, 1H), 5.83 (dd, 1H), 5.42 (d, 1H), 4.92 (d, 1H), 3.94 (dd, 1H), 3.66 (td, 1H), 3.35 (d, 1H), 3.22 (d, 1H), 3.17 - 3.03 (m, 4H), 2.98 (d, 1H), 2.87 (dd, 1H), 2.50 - 2.39 (m, 2H), 2.35 - 2.24 (m, 1H), 2.21 - 2.04 (m, 3H), 1.69 (tp, 2H), 1.52 (d, 3H), 1.39 (s, 3H), 0.75 (dt, 1H).

LC-MS (ESI): m/z =578.2 [M+H]⁺.

### Example 101:

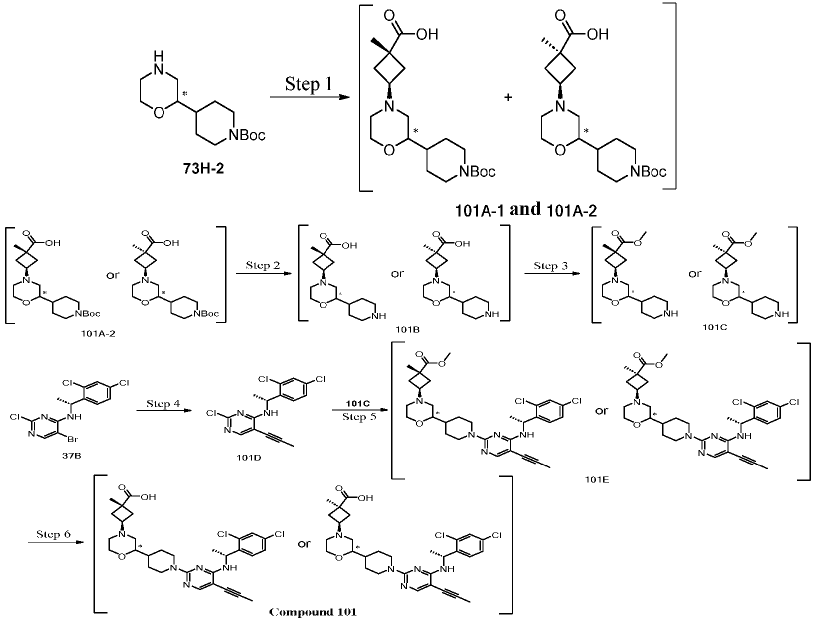

Step 1: **73H-2** (1.0 g, 3.70 mmol) and 3-carbonyl-1-methyl-cyclobutanecarboxylic acid (0.5 g, 3.90 mmol) were dissolved in 1,2-dichloroethane (10 mL) in sequence, glacial acetic acid (0.3 g, 5.00 mmol) was added, the obtained system was cooled to 0°C, and stirred evenly, then sodium cyanoborohydride (0.6 g, 9.55 mmol) was added in portions, and after the addition was completed, stirring was continued for 1 h. After the reaction was completed, water (30 mL) was added to quench the reaction, and the obained system was extracted with dichloromethane (50 mL×2). The combined organic phases were washed with saturated brine, dried over anhydrous sodium sulfate, and filtered. The filtrate was concentrated and the residue was separated and purified by silica gel column chromatography (dichloromethane:methanol (v/v) = 10:1) to obtain isomers **101A-1** (Rf=0.4 (dichloromethane:methanol (v/v)= 10:1), 700 mg, 49%) and **101A-2** (Rf=0.2 (dichloromethane:methanol (v/v)= 10:1), 240 mg, 17%).

Step 2: **101A-2** (700 mg, 1.77 mmol) was dissolved in dichloromethane (6 mL), and trifluoroacetic acid (2 mL) was added dropwise at room temperature. After the dropwise addition was completed, the mixture was stirred for 30 min. After the reaction was completed, the obtained system was concentrated under reduced pressure to obtain **101B** trifluoroacetate (730 mg, 100%).

Step 3: **101B** (730 mg, 1.84 mmol) was dissolved in methanol (5 mL) and dichlorosulfoxide (2.2 g, 18.4 mmol) was added slowly dropwise at 0°C, after the dropwise addition was completed, the obtained system was warmed to room temperature and stirred for 1 h. After the reaction was completed, water (10 mL) was added, and ethyl acetate (15 mL ×3) was used for extraction. The combined organic phase was washed with saturated brine (10 mL), dried over anhydrous sodium sulfate, and filtered. The filtrate was concentrated and the residue was separated and purified by silica gel column chromatography (ethyl acetate: petroleum ether (v/v) = 1:1) to obtain **101C** (470 mg, 86%).

Step 4: Compound **37B** (2.0 g, 5.2 mmol), propyne (0.31 g, 7.76 mmol), copper iodide (0.1 g, 0.53 mmol) and bis(triphenylphosphine)palladium(II) chloride (0.37 g, 0.53 mmol) were dissolved in triethylamine (20 mL), and the obtained system was stirred at 80°C for 12 h under nitrogen atmosphere. After the reaction was completed, the mixture was cooled to room temperature and concentrated under reduced pressure. The residue was purified by column chromatography (petroleum ether:ethyl acetate (v/v) = 3:1) to obtain compound **101D** (1.3 g, 71%).

LC-MS (ESI): m/z =340.0 [M+H]⁺.

Step 2: Compounds **101D** (116 mg, 0.34 mmol), **101C** (100 mg, 0.34 mmol), cesium fluoride (206.6 mg, 1.36 mmol), and triethylamine (103.2 mg, 1.02 mmol) were dissolved in N,N-dimethylformamide (2 mL) and the obtained system was stirred at 100°C for 3 h. After the reaction was completed, the mixture was cooled to room temperature, and water (10 mL) and ethyl acetate (15 mL) were added respectively and extraction was performed. The organic phase was separated, and the aqueous phase was extracted with ethyl acetate (10 mL×2). The combined organic phases were washed with saturated brine, dried over anhydrous sodium sulfate, filtered, and concentrated. The residue was purified by column chromatography (dichloromethane:methanol (v/v) = 10:1) to obtain compound **101E** (61 mg, 30%).

LC-MS (ESI): m/z =600.2 [M+H]⁺.

Step 3: Compound **101E** (61.0 mg, 0.10 mmol) was dissolved in a mixed solution of tetrahydrofuran (1 mL), methanol (1 mL) and water (2 mL). After stirring evenly, lithium hydroxide (12.5 mg, 0.50 mmol) was added, the reaction was performed at room temperature for 2 h. After the reaction was completed, purification were performed using preparative high performance liquid phase chromatography to obtain **Compound 101** (41 mg, 70%).

Preparation method: instrument: waters 2767 preparative liquid chromatographic instrument; chromatographic column: SunFire @ Prep C18 (19 mm × 250 mm). The sample was dissolved in methanol and filtered through a 0.22 µm filter head to prepare a sample liquid. Preparative chromatography conditions: composition of mobile phases A and B: mobile phase A: acetonitrile, mobile phase B: water (containing 1% TFA), gradient elution, mobile phase A: 10% to 55%, flow rate: 15 mL/min. elution time: 20 min.

¹H NMR (400 MHz, DMSO-d6) δ 10.36 (s, 1H), 7.92 (s, 1H), 7.71 (s, 1H), 7.59 (d, 1H), 7.50 (d, 1H), 7.42 (dd, 1H), 5.45 (p, 1H), 4.39 (d, 2H), 4.03 (d, 1H), 3.76 (s, 1H), 3.61 (t, 1H), 3.31 (dd, 3H), 2.90-2.55 (m, 6H), 2.27-2.17 (m, 2H), 2.13 (s, 3H), 1.63 (dd, 3H), 1.50 (d, 3H), 1.32 (s, 3H), 1.18 (d, 1H), 0.93 (s, 1H).

LC-MS (ESI): m/z =586.1 [M+H]⁺.

### Example 102:

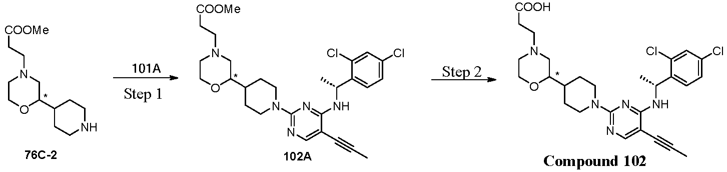

Step 1: Using compound **76C-2** (170 mg, 0.66 mmol) and **101A** (271 mg, 0.80 mmol) as the raw materials, the operation method of step 3 of example 76 was referenced to obtain compound **102A** (140 mg, 37.7%).

LCMS m/z = 560.2 [M+H]⁺.

Step 2: Using compound **102A** (140 mg, 0.25 mmol) as the raw material, the operation method of step 4 of example 76 was referenced to obtain **compound 102** (25 mg, 18.3%).

LCMS m/z = 546.6 [M+H]⁺.

¹H NMR (400 MHz, DMSO-*d*₆) δ 7.84 (s, 1H), 7.54 (d, 1H), 7.48-7.42 (m, 1H), 7.40-7.34 (m, 1H), 6.90 (d, 1H), 5.44-5.34 (m, 1H), 4.53-4.37 (m, 2H), 3.79-3.68 (m, 1H), 3.43-3.35 (m, 3H), 3.10-3.00 (m, 1H), 2.75-2.51 (m, 6H), 2.36-2.28 (m, 2H), 2.11 (s, 3H), 2.03-1.90 (m, 1H), 1.83-1.69 (m, 1H), 1.65-1.41 (m, 6H).

### Example 103:

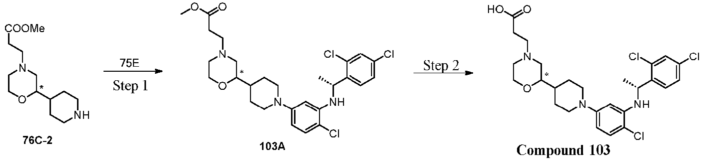

Step 1: Using **76C-2** (230 mg, 0.90 mmol) and **75E** (0.61 g, 1.36 mmol) as the raw materials, the operation method of step 3 of example 76 was referenced to obtain compound **103A** (0.17 g, 34.04%).

LCMS (ESI): m/z =554.1 [M+H]⁺.

Step 2: Using **103A** (170 mg, 0.31 mmol) as the raw material, the operation method of step 4 of example 76 was referenced to obtain compound **103** (108 mg, 64.41%).

¹H NMR (400 MHz, Methanol-d4) δ 7.49-7.44 (m, 1H), 7.43-7.38 (m, 1H), 7.29-7.24(m, 1H), 7.22-7.15 (m, 1H), 6.51-6.40 (m, 1H), 6.13-6.01 (m, 1H), 4.99-4.91 (m, 1H), 4.16 - 4.08 (m, 1H), 3.83 - 3.73 (m, 1H), 3.59 - 3.43 (m, 7H), 3.16-3.06 (m, 1H), 3.02-2.93 (m, 1H), 2.93-2.79 (m, 4H), 1.98 - 1.88 (m, 1H), 1.78-1.59 (m, 4H), 1.58-1.54 (m, 3H).

LCMS m/z =540.4 [M+H]⁺.

### Example 104: (1S,3R)-3-((S)-2-(1-(5-chloro-4-(((R)-1-(2,4-dichlorophenyl) ethyl)amino)pyrimidin-2-yl)-3-fluoroazetidin-3-yl)morpholino)-1-methylcyclobutane-1-carboxylic acid (Compound 104)

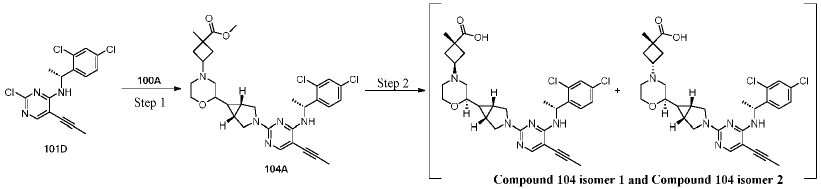

Step 1: Using **100A** (0.13 g, 0.45 mmol) and **101D** (190 mg, 0.56 mmol) as the raw materials, the operation method of step 5 of example 101 was referenced to obtain **104A** (154 mg, 57.58%).

LCMS m/z =598.7 [M+H]⁺.

Step 2: Using **104A** (154 mg, 0.26 mmol) as the raw material, the operation method of step 6 of example 101 was referenced to obtain compound **104** isomer 1 (21 mg, 13.82%) and **isomer 2** (53 mg, 34.87%).

HPLC analysis conditions: 1. Instruments: Shimadzu LC-20AT; 2. Chromatographic column: Xtimate C18 4.6*50mm, 3µm; 3. Mobile phase system: A for 0.05% TFA in H2O; B for ACN; 4. Gradient: B 5-95%; 5. Flow rate: 1.0 mL/min, run time: 10 min. retention time: **Compound 104** isomer 1 retention time: tR = 3.404 min; **Compound 1040** isomer 2 retention time: tR = 3.439 min.

Compound 104 isomer 1: 1H NMR (400 MHz, Chloroform-d) δ 8.10 (s, 1H), 7.39-7.31 (m, 1H), 7.23-7.13 (m, 2H), 5.84-5.70 (m, 1H), 5.46-5.37 (m, 1H), 4.07-3.93 (m, 1H), 3.91-3.75 (m, 2H), 3.69-3.54 (m, 1H), 3.47-3.35 (m, 2H), 3.32-2.93 (m, 4H), 2.81-2.60 (m, 2H), 2.11 (s, 7H), 1.76-1.63 (m, 2H), 1.56-1.46 (m, 3H), 1.42 (s, 3H).

LCMS m/z =584.7 [M+H]⁺.

Compound 104 isomer 2: 1H NMR (400 MHz, Chloroform-d) δ 7.92 (s, 1H), 7.40-7.32 (m, 1H), 7.25-7.20 (m, 1H), 7.20-7.13 (m, 1H), 5.64 (s, 1H), 5.48-5.39 (m, 1H), 3.98-3.89 (m, 1H), 3.87-3.61 (m, 3H), 3.53-3.28 (m, 2H), 3.20-3.09 (m, 2H), 3.04-2.96 (m, 1H), 2.93-2.85 (m, 1H), 2.62-2.51 (m, 2H), 2.26-2.02 (m, 6H), 1.96-1.87 (m, 1H), 1.73-1.64 (m, 1H), 1.64-1.55 (m, 1H), 1.55-1.46 (m, 3H), 1.41 (s, 3H).

LCMS m/z =584.7 [M+H]⁺.

### Example 105:

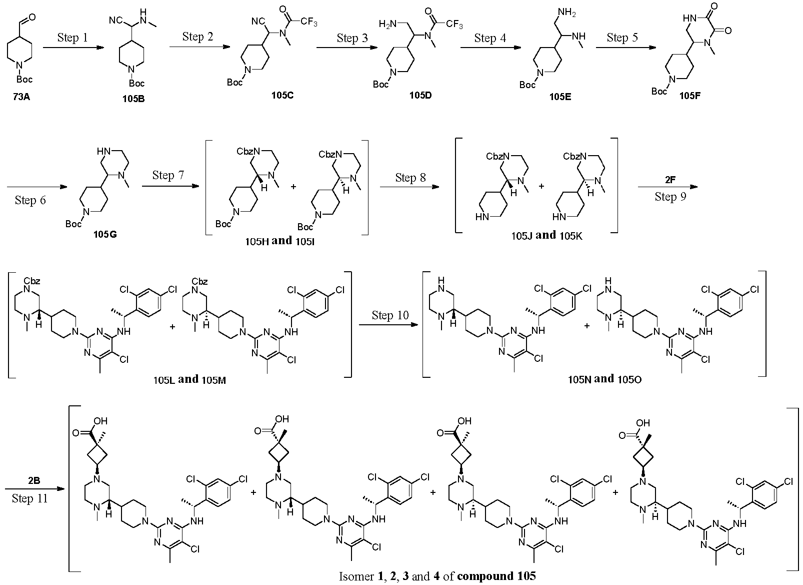

Step 1: Using compound **73A** (50.0 g, 0.234 mol) as the raw material, the operation method of step 1 of example 80 was referenced to obtain crude compound **105B** (42.1 g).

LCMS (ESI): m/z =254.2 [M+H]⁺.

Step 2: Using compound **105B** (42.1 g, 0.166 mol) as the raw material, the operation method of step 2 of example 80 was referenced to obtain crude compound **105C** (53.0 g).

LCMS (ESI): m/z =350.2 [M+H]⁺.

Step 3: Using compound **105C** (53.0 g) as the raw material, the operation method of step 3 of example 80 was referenced to obtain crude compound **105D** (49.2 g), which was directly used for the next step reaction.

LCMS (ESI): m/z =354.2 [M+H]⁺.

Step 4: Using compound **105D** (49.2 g) as the raw material, the operation method of step 4 of example 80 was referenced to obtain crude compound **105E** (31.0 g).

LCMS (ESI): m/z =258.2 [M+H]⁺.

Step 5: Using compound **105E** (31.0 g) as the raw material, the operation method of step 5 of example 80 was referenced to obtain compound **105F** (24.7 g, five-step yield: 33.8%).

LCMS (ESI): m/z =312.2 [M+H]⁺.

Step 6: Using compound **105E** (24.0 g, 77.1 mmol) as the raw material, the operation method of step 6 of example 80 was referenced to obtain crude compound **105G** (19.0 g).

LCMS (ESI): m/z =284.2 [M+H]⁺.

Step 7: Using compound **105G** (19.0 g) as the raw material, the operation method of step 7 of example 80 was referenced to obtain compound **105H** (1.39 g, two-step yield: 4.20%) and compound **105I** (1.55 g, two-step yield: 4.68%).

HPLC analytical method: 1. Instruments: SHIMADZU LC-30AD SFC; 2. Chromatographic column: Chiralcel OJ-3 50×4.6mm I.D., 3µm; 3. Mobile phase system: A for CO₂, B for 0.05%DEA in MeOH; 4. Gradient: B 5%-40%; 5. Flow rate: 3 mL/min. retention time: Compound **105H,** 0.695 min; Compound **105I**, 0.824 min.

LCMS (ESI): m/z =418.3 [M+H]⁺.

Step 8: Using compound **105H** (500 mg, 1.20 mmol) as the raw material, the operation method of step 8 of example 80 was referenced to obtain the crude trifluoroacetate salt of compound **105J** (1.10 g), which was directly used in the next step reaction without purification.

LCMS (ESI): m/z =318.3 [M+H]⁺.

Using compound **105I** (500 mg, 1.20 mmol) as a raw material, the operation method of step 8 of example 80 was referenced to obtain the crude trifluoroacetate salt of compound **105K** (1.10 g).

LCMS (ESI): m/z =318.3 [M+H]⁺.

Step 9: Using the crude trifluoroacetate salt of **105J** (1.10 g) obtained in the previous step and compound **2F** (472 mg, 1.35 mmol) as the raw materials, the operation method of step 9 of example 80 was referenced to obtain compound **105L** (570 mg, two-step yield: 75.3%).

LCMS (ESI): m/z =631.2 [M+H]⁺.

Using the crude trifluoroacetate salt of compound **105K** (1.10 g) and compound **2F** (472 mg, 1.35 mmol) as the raw materials, the operation method of step 9 of example 80 was referenced to obtain compound **105M** (660 mg, two-step yield: 87.2%).

LCMS (ESI): m/z =631.2 [M+H]⁺.

Step 10: Using compound **105L** (570 mg, 0.902 mmol) as the raw material, the operation method of step 10 of example 80 was referenced to obtain the crude product of compound **105N** (544 mg), which was directly used in the next reaction without purification.

LCMS (ESI): m/z =497.2 [M+H]⁺.

Using compound **105M** (660 mg, 1.04 mmol) as the raw material, the operation method of step 10 of example 80 was referenced to obtain the crude product of compound **1050** (454 mg).

LCMS (ESI): m/z =497.2 [M+H]⁺

Step 11: The crude compound **105N** obtained in the previous step (544 mg) was dissolved in methanol (15 mL), and compound **2B** (210 mg, 1.64 mmol) and acetic acid (65.6 mg, 1.09 mmol) were added in sequence and the obtained system was stirred at room temperature for 1 h, then sodium cyanoborohydride (137 mg, 2.19 mmol) was added, and the stirring was continued for 3 h. After the reaction was completed as monitored by LCMS, water (1 mL) was added to quench the reaction and the product was directly separated and purified by preparative HPLC to obtain compound **105** isomer 1 (90 mg, two-step yield: 16.4%) and compound **105** isomer 2 (96 mg, two-step yield: 17.5%).

Preparative HPLC separation and purification method: 1. Instruments: waters 2767 preparative liquid chromatographic instrument; chromatographic column: SunFire @ Prep C18 (19 mm x 250 mm). 2. The sample was filtered with a 0.45 µm filter head to prepare a sample liquid. 3. Preparative chromatography conditions: a. composition of mobile phases A and B: mobile phase A: acetonitrile; mobile phase B: water (containing 0.1% ammonium acetate); b. gradient elution, mobile phase A: 5%-50%; c. flow rate: 12 mL/min; d. elution time: 30 min.

HPLC analysis conditions: 1. Instruments: Shimadzu LC-20AT; 2. Chromatographic column: Xtimate C18 4.6*50mm, 3µm; 3. Mobile phase system: A for 0.05% TFA in H2O; B for ACN; 4. Gradient: B 5-95%; 5. Flow rate: 1.0 mL/min, run time: 10 min. retention time: **Compound 105** isomer 1 retention time: tR = 3.243 min; **Compound 105** isomer 2 retention time: tR = 3.262 min.

Compounds **105** isomer 1: ¹H NMR (400 MHz, Methanol-d4) *δ* 7.41 (d, 1H), 7.32 (d, 1H), 7.23 (dd, 1H), 5.41 (q, 1H), 4.69-4.50 (m, 2H), 3.19-2.95 (m, 3H), 2.84 (d, 1H), 2.73-2.53 (m, 5H), 2.45 (s, 3H), 2.43-2.31 (m, 2H), 2.24 (s, 3H), 2.15-1.98 (m, 2H), 1.95-1.79 (m, 2H), 1.63-1.43 (m, 5H), 1.34 (s, 3H), 1.20 (qd, 1H), 0.77 (q, 1H).

LC-MS (ESI): m/z =609.2 [M+H]⁺;

Compounds **105** isomer 2: ¹H NMR (400 MHz, Methanol-d4) *δ* 7.42 (d, 1H), 7.35 (d, 1H), 7.25 (dd, 1H), 5.43 (q, 1H), 4.68-4.55 (m, 2H), 3.30-3.19 (m, 2H), 3.11-2.96 (m, 2H), 2.73-2.48 (m, 6H), 2.43 (dd, 1H), 2.37 (s, 3H), 2.31-2.18 (m, 4H), 2.17-2.01 (m, 3H), 1.61-1.45 (m, 5H), 1.35 (s, 3H), 1.20 (qd, 1H), 0.88-0.72 (m, 1H).

LC-MS (ESI): m/z =609.2 [M+H]⁺;

Using **105O** as the raw material, compound **105** isomer 3 (40 mg, two-step yield: 6.28%) and compound **105** isomer 4 (31 mg, two-step yield: 4.87%) were obtained referring to the above synthesis method.

HPLC analysis conditions: 1. Instruments: Shimadzu LC-20AT; 2. Chromatographic column: Xtimate C18 4.6*50mm, 3µm; 3. Mobile phase system: A for 0.05% TFA in H2O; B for ACN; 4. Gradient: B 5-95%; 5. Flow rate: 1.0 mL/min, run time: 10 min. retention time: **Compound 105** isomer 3 retention time: 3.271 min; **Compound 105** isomer 4 retention time: 3.233 min.

**Compound 105** isomer 3: ¹H NMR (400 MHz, Methanol-d4) *δ* 7.40 (d, 1H), 7.35 (d, 1H), 7.24 (dd, 1H), 5.39 (q, 1H), 4.66-4.51 (m, 2H), 3.15-2.92 (m, 3H), 2.76 (d, 1H), 2.70- 2.51 (m, 5H), 2.47 (s, 3H), 2.43-2.35 (m, 1H), 2.31 (td, 1H), 2.25 (s, 3H), 2.13-2.00 (m, 2H), 1.93- 1.82 (m, 2H), 1.62 (d, 1H), 1.49 (d, 3H), 1.41-1.32 (m, 4H), 1.11 (qd, 1H), 0.89 (d, 1H).

LC-MS (ESI): m/z =609.2 [M+H]⁺;

**Compound 105** isomer 4: ¹H NMR (400 MHz, Methanol-d4) *δ* 7.40 (d, 1H), 7.35 (d, 1H), 7.24 (dd, 1H), 5.43 (q, 1H), 4.60 (d, 2H), 3.28-3.17 (m, 2H), 3.08-2.95 (m, 2H), 2.66 (tt, 2H), 2.60-2.49 (m, 4H), 2.44-2.34 (m, 4H), 2.30 (t, 1H), 2.25 (s, 3H), 2.17-2.00 (m, 3H), 1.65 (d, 1H), 1.50 (d, 3H), 1.46 (d, 1H), 1.36 (s, 3H), 1.12 (qd, 1H), 1.06-0.92 (m, 1H).

LC-MS (ESI): m/z =609.2 [M+H]⁺.

### Example 106:

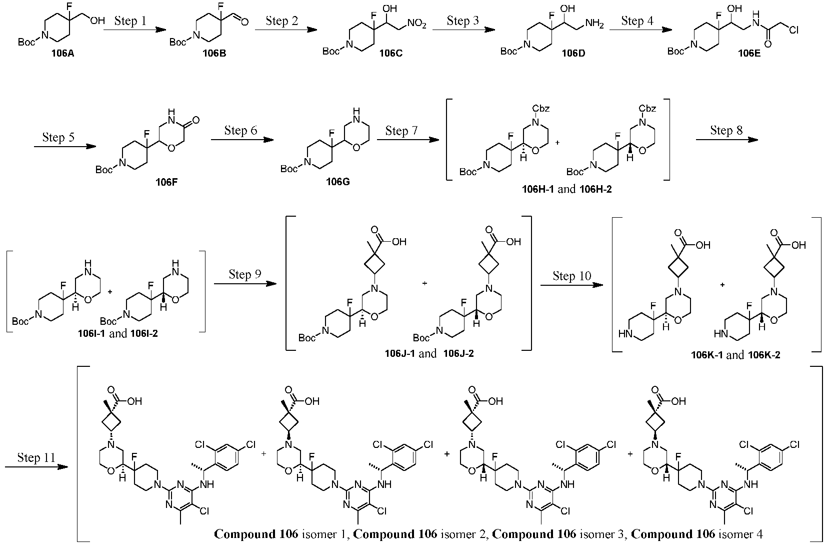

Step 1: Compound **106A** (18.0 g, 80.6 mmol) was dissolved in ethyl acetate (200 mL), IBX (45.1 g, 161.2 mmol) was added, and the obtained system was stirred under reflux for 17 h. After the reaction was completed, the mixture was cooled to room temperature, filtered and concentrated to obtain a crude product of compound **106B** (18.0 g), which was directly used in the next reaction without further purification.

LCMS m/z = 176.1 [M+H-*^{t}*Bu]⁺.

Step 2: Using compound **106B** (18.0 g, 77.8 mmol) as the raw material, the operation method of step 1 of example 73 was referenced to obtain compound **106C** (21.0 g, 92.3%).

LCMS m/z = 237.1 [M+H-*^{t}*Bu]⁺.

Step 3: Using compound **106C** (21.0 g, 71.8 mmol) as the raw material, the operation method of step 2 of example 73 was referenced to obtain the crude compound **106D** (18.4 g, 97.6%), which was directly used in the next reaction without further purification.

LCMS m/z = 207.2 [M+H-*^{t}*Bu]⁺.

Step 4: Using compound **106D** (17.3 g, 65.9 mmol) as the raw material, the operation method of step 3 of example 73 was referenced to obtain compound **106E** (17.0 g, 76.1%).

LCMS m/z = 239.1 [M+H-Boc]⁺.

Step 5: Using compound **106E** (17 g, 50.2 mmol) as the raw material, the operation method of step 4 of example 73 was referenced to obtain compound **106F** (15.0 g, 98.7%).

LCMS m/z = 203.2 [M+H-Boc]⁺.

Step 6: Using compound **106F** (16 g, 52.9 mmol) as the raw material, the operation method of step 5 of example 73 was referenced to obtain **106G** (13 g, 90.9%).

LCMS m/z = 233.2 [M+H-*^{t}*Bu]⁺.

Step 7: Using compound **106G** (13 g, 45.1 mmol) as the raw material, the operation method of step 6 of example 73 was referenced to obtain two isomers, compound **106H-1** (4.89 g, retention time 1.525 min, 25.7%) and compound **106H-2** (5.34 g, retention time 1.656 min, 28.1%). HPLC analytical method: 1. Instruments: SHIMADZU LC-30AD SFC; 2. Chromatographic column: Chiralcel OJ-3 50×4.6mm I.D., 3µm; 3. Mobile phase system: A for CO₂, B for 0.05%DEA in MeOH; 4. Gradient: B 5%-40%; 5. Flow rate: 3 mL/min.

LCMS m/z = 323.2 [M+H-Boc]⁺.

Step 8: Using compound **106H-1** (1.0 g, 2.37 mmol) as the raw material, the operation method of step 7 of example 73 was referenced to obtain compound **106I-1** (650 mg, 95.2%).

Using compound **106H-2** (500 mg, 1.18 mmol) as the raw material, the operation method of step 7 of example 73 was referenced to obtain compound **1061-2** (320 mg, 93.8%).

LCMS m/z = 233.2 [M+H-*^{t}*Bu]⁺.

Step 9: Using compound **1061-1** (650 mg, 2.25 mmol) and 3-carbonyl-1-methyl-cyclobutanecarboxylic acid (349 mg, 2.70 mmol) as the raw materials, the operation method of step 8 of example 73 was referenced to obtain compound **106J-1** (600 mg, 66.5%).

Using compound **1061-2** (320 mg, 1.11 mmol) as the raw material, the operation method of step 8 of example 73 was referenced to obtain compound **106J-2** (300 mg, 67.5%).

LCMS m/z = 401.3 [M+H]⁺.

Step 10: Using compound **106J-1** (600 mg, 1.50 mmol) as the raw material, the operation method of step 9 of example 73 was referenced to obtain the trifluoroacetate salt of compound **106K-1** (600 mg, 96.6%).

Using compound **106J-2** (300 mg, 0.75 mmol) as the raw material, the operation method of step 9 of example 73 was referenced to obtain the trifluoroacetate salt of compound **106K-2** (300 mg, 96.6%).

LCMS m/z = 301.2 [M+H]⁺.

Step 11: Using compound **2F** (200 mg, 0.57 mmol) and compound **106K-1** (472 mg, 1.14 mmol) as the raw materials, the operation method of step 10 of example 73 was referenced to obtain the title **compound 106** isomer 1 (Rf=0.3 (ethyl acetate:methanol (v/v)= 9:1), 100.0 mg, 28.5%) and the title **compound 106** isomer 2 (Rf=0.2 (ethyl acetate:methanol (v/v)= 9:1), 80.0 mg, 22.8%).

**Compound 106** isomer 1: ¹H NMR (400 MHz, DMSO-*d*₆) δ 12.16 (s, 1H), 7.55 (d, 1H), 7.44-7.40 (m, 1H), 7.38-7.33 (m, 1H), 7.28 (d, 1H), 5.38-5.29 (m, 1H), 4.36-4.19 (m, 2H), 3.85-3.75 (m, 1H), 3.49-3.38 (m, 1H), 3.30-3.20 (m, 1H), 2.96-2.77 (m, 2H), 2.71-2.62 (m, 2H), 2.61-2.54 (m, 1H), 2.47-2.37 (m, 2H), 2.20 (s, 3H), 1.81-1.55 (m, 6H), 1.52-1.41 (m, 5H), 1.27 (s, 3H).

LCMS m/z = 614.2 [M+H]⁺.

**Compound 106** isomer 2: ¹H NMR (400 MHz, DMSO-*d*₆) δ 7.54 (d, 1H), 7.44-7.40 (m, 1H), 7.38-7.33 (m, 1H), 7.28 (d, 1H), 5.37-5.29 (m, 1H), 4.37-4.18 (m, 2H), 3.85- 3.74 (m, 1H), 3.49-3.38 (m, 1H), 3.29-3.20 (m, 2H), 2.97-2.70 (m, 3H), 2.69-2.60 (m, 1H), 2.59- 2.53 (m, 2H), 2.20 (s, 3H), 2.18-2.09 (m, 2H), 1.94-1.83 (m, 2H), 1.82-1.58 (m, 3H), 1.50-1.42 (m, 4H), 1.31 (s, 3H).

LCMS m/z = 614.2 [M+H]⁺.

Using compound **106K-2** (236 mg, 0.57 mmol) and compound **2F** (100 mg, 0.28 mmol) as the raw materials, the operation method of step 10 of example 73 was referenced to obtain **compound 106** isomer 3 (Rf=0.3 (ethyl acetate:methanol (v/v)= 9:1), 50.0 mg, 28.5%) and **compound 106** isomer 4 (Rf=0.2 (ethyl acetate:methanol (v/v)= 9:1), 60.0 mg, 34.3%).

**Compound 106** isomer 3: ¹H NMR (400 MHz, DMSO-*d*₆) δ 12.19 (s, 1H), 7.56 (d, 1H), 7.46-7.41 (m, 1H), 7.38-7.33 (m, 1H), 7.29 (d, 1H), 5.40-5.29 (m, 1H), 4.37-4.21 (m, 2H), 3.82-3.74 (m, 1H), 3.47-3.36 (m, 1H), 3.29-3.21 (m, 1H), 2.92-2.79 (m, 2H), 2.74-2.61 (m, 2H), 2.60-2.53 (m, 1H), 2.48-2.38 (m, 2H), 2.20 (s, 3H), 1.82-1.52 (m, 7H), 1.48-1.42 (m, 4H), 1.27 (s, 3H).

LCMS m/z =614.2 [M+H]⁺.

**Compound 106** isomer 4: ¹H NMR (400 MHz, DMSO-*d*₆) δ 7.55 (d, 1H), 7.46-7.42 (m, 1H), 7.38-7.34 (m, 1H), 7.29 (d, 1H), 5.39-5.30 (m, 1H), 4.38-4.20 (m, 2H), 3.84- 3.72 (m, 1H), 3.47-3.36 (m, 1H), 3.31-3.20 (m, 1H), 2.92-2.81 (m, 2H), 2.81-2.70 (m, 1H), 2.69- 2.60 (m, 1H), 2.59-2.53 (m, 1H), 2.20 (s, 3H), 2.18-2.09 (m, 2H), 1.93-1.76 (m, 3H), 1.71-1.54 (m, 4H), 1.48-1.42 (m, 4H), 1.31 (s, 3H).

LCMS m/z = 614.2 [M+H]⁺.

### Example 107:

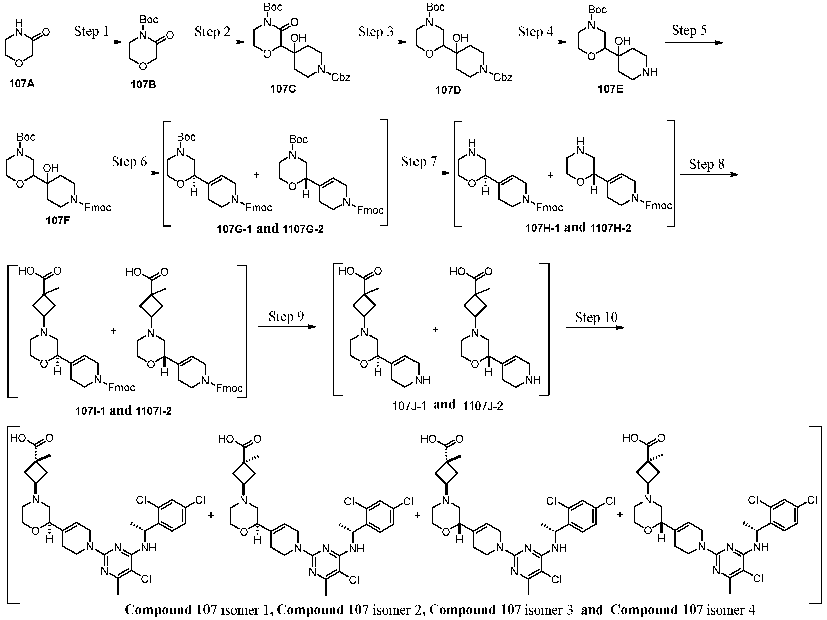

Step 1: Compound **107A** (30.0 g, 296.78 mmol) was dissolved in dichloromethane (300 ml), and triethylamine (90.1 g, 890.37 mmol) and 4-dimethylaminopyridine (3.63 g, 29.68 mmol) were added in sequence, and after the addition was completed, di-tert-butyl dicarbonate (129.54 g, 593.56 mmol) was slowly added dropwise at a controlled temperature of 0-10°C, and after the addition was completed, the temperature was raised to room temperature and reaction was performed for 2 h. After the reaction was completed, the obtained system was concentrated and the residue was separated by silica gel column chromatography (petroleum ether:ethyl acetate (v/v) = 10:1) to obtain compound **107B** (56.0 g, 93.7%).

Step 2: Under nitrogen atmosphere, compound **107B** (50.0 g, 248.48 mmol) was dissolved in anhydrous tetrahydrofuran (500 ml), the temperature was lowered to -78°C, and then lithium bis(trimethylsilyl)amide (261 ml, 261 mmol, 1.0 mol/L) was slowly added dropwise, after the dropwise addition was completed, stirring was performed at -78°C for 0.5 h, a solution of 1-benzyloxycarbonyl-4-piperidone (63.75 g, 273.31 mmol) in tetrahydrofuran (100 mL) was slowly added dropwise at a controlled temperature of -78°C, after the dropwise addition was completed, the reaction was performed at -78°C for 1 h, then the temperature was slowly raised to room temperature and the reaction was performed for 2 h. After the reaction was completed, saturated aqueous ammonium chloride solution (300 mL) was added to quench the reaction. After quenching, the obtained system was extracted with ethyl acetate (150 mL×2). The combined organic phase was washed with saturated brine, dried over anhydrous sodium sulfate, filtered, and the filtrate was concentrated. The residue was separated by silica gel column chromatography (petroleum ether:ethyl acetate (v/v) = 4:1) to obtain compound **107C** (35.2 g, 32.6%).

Step 3: Compound **107C** (35.2 g, 81.02 mmol) was dissolved in tetrahydrofuran (350 mL), borane dimethyl sulfide (202.5 ml, 405.1 mmol, 2.0 mol/L) was slowly added dropwise at a controlled temperature of 0-10°C, and after the dropwise addition was completed, the temperature was raised to 70°C for reaction for 15h. After the reaction was completed, the temperature was controlled at 0-10°C, and methanol was slowly added dropwise to quench the reaction. After quenching, the obtained system was concentrated, and the residue was separated by silica gel column chromatography (petroleum ether:ethyl acetate (v/v)=4:1) to obtain compound **107D** (17.3 g, 50.7%).

Step 4: Compound **107D** (17.3 g, 41.14 mmol) was dissolved in methanol (170 mL), palladium on carbon (2.23 g, Pd content 10%) was added, and the reaction was performed for 3 h under a hydrogen atmosphere. After the reaction was completed, the obtained system was filtered and the filtrate was concentrated to obtain the crude product of compound **107E** (11.1 g, 94.2%), which was directly used in the next reaction without further purification.

Step 5: Compound **107E** (11.1 g, 38.76 mmol) was dissolved in a mixed solvent of tetrahydrofuran (50 mL) and water (50 mL), solid sodium bicarbonate (9.77 g, 116.21 mmol) was added, and then the temperature was lowered to 0-5°C, 9-fluorenylmethyl-N-succinimidyl carbonate (14.38 g, 42.64 mmol) was added in portions, and then the reaction was continued at 0-5°C for 1 h. After the reaction was completed, water was added and the obtained system was extracted with ethyl acetate (150 mL×2). The combined organic phase was washed with saturated brine, dried over anhydrous sodium sulfate, filtered, and the filtrate was concentrated. The residue was separated and purified by silica gel column chromatography (petroleum ether:ethyl acetate (v/v) = 4:1) to obtain compound **107F** (13.3 g, 67.4%).

Step 6: Compound **107F** (13.3 g, 26.15 mmol) was dissolved in toluene (150 mL), Burgess reagent (12.46 g, 52.3 mmol) was added, after the addition was completed, the temperature was raised to 100°C and the reaction was performed for 3 h; after the reaction was completed, concentration was performed,the residue after the concentration was separated and purified by silica gel column chromatography (petroleum ether:ethyl acetate (v/v) = 5:1) to obtain **107G** racemate (4.8 g, 37.4%), which was subjected to chiral resolution to obtain compound **107G-1** (1.9 g, retention time 1.97 min, 14.8%) and compound **107G-2** (2.1 g, retention time 2.25 min, 16.3 %).

HPLC analytical method: 1. Instruments: SHIMADZU LC-30AD SFC; 2. Chromatographic column: Chiralcel OX-3 50×4.6mm I.D., 3µm; 3. Mobile phase system: A for CO2, B for 0.05%DEA in MeOH; 4. Gradient: B 5%-40%; 5. Flow rate: 3 mL/min.

Preparative chromatography separation conditions: 1. Instruments: Waters 150 SFC; 2. Chromatographic column: Chiralpak OX -Column (250×30mm, I.D 30mm, 10um particle size); 3. Mobile phase system: A for CO₂ and B for B for MeOH +ACN (0.1%NH₃•H₂O); 4. Gradient: B 20%; 5. Flow rate: 100 mL/min; 6. Elution time: 5.2min.

Step 7: Compound **107G-1** (1.0 g, 2.04 mmol) was dissolved in dichloromethane (10 mL), trifluoroacetic acid (3.0 mL) was added, after the addition was completed, the obtained system was stirred at room temperature for 3 h After the reaction was completed, the obtained system was concentrated, and the residue was adjusted to pH 7-8 with saturated aqueous sodium bicarbonate solution. The obtained system was extracted with dichloromethane (50 mL×2), and the combined organic phases were washed with saturated brine, dried over anhydrous sodium sulfate, filtered, and the filtrate was concentrated to obtain compound **107H-1** (0.78 g, 97.9%).

Referring to the above operation, compound **107G-2** (1.0 g, 2.04 mmol) was used as the raw material to obtain the title compound **107H-2** (0.75 g, 94.1%).

Step 8: Using compound **107H-1** (0.78 g, 2.00 mmol) and 3-carbonyl-1-methyl-cyclobutanecarboxylic acid (0.28 g, 2.18 mmol) as the raw materials, the operation method of step 8 of example 73 was referenced to obtain compound **107I-1** (0.85 g, 84.5%).

Using compound **107H-2** (0.75 g, 1.92 mmol) as the raw material, the operation method of step 8 of example 73 was referenced to obtain the title compound **107I-2** (0.82 g, 84.9%).

M/Z (ESI): m/z =503.5[M+H]⁺.

Step 9: Compound **107I-1** (0.85 g, 1.69 mmol) was dissolved in methanol, and a solution of lithium hydroxide (0.35 g, 8.53 mmol) in water (1 mL) was added, and then the obtained system was stirred at room temperature for 5 h. After the reaction was completed, the pH value was adjusted to 7-8 with 1N hydrochloric acid, and the mixture was concentrated under reduced pressure. Water (10 mL) was added to the residue, and the mixture was extracted with ethyl acetate (20 mL×2). The aqueous phase was collected, and after the aqueous phase was concentrated, the residue was dissolved with dichloromethane, and filtered, and the filtrate was concentrated to obtain compound **107J-1** (0.41 g, 86.5%).

Referring to the above operation, compound **107I-2** (0.82 g, 1.63 mmol) was used as the raw material to obtain the title compound **107J-2** (0.4 g, 87.5%).

M/Z (ESI): m/z =281.3[M+H]⁺.

Step 10: Using compound **2F** (0.3 g, 0.85 mmol) and compound **107J-1** (0.31 g, 1.10 mmol) as the raw materials, the operation method of step 10 of example 73 was referenced to obtain **compound 107** isomer 1 (Rf=0.4 (ethyl acetate:methanol=10:1), 110.0 mg, 21.7%) and **compound 107** isomer 2 (Rf=0.2 (ethyl acetate:methanol=10:1), 100.0 mg, 19.7%).

**Compound 107** isomer 1: ¹H NMR (400 MHz, CDCl₃) δ 7.34 (d, 1H), 7.22 (d, 1H), 7.16-7.14 (m, 1H), 5.70 (s, 1H), 5.46-5.39 (m, 2H), 4.12-4.08 (m, 1H), 3.95-3.86 (m, 3H), 3.75-3.62 (m,3H), 2.87-2.72 (m,3H), 2.61-2.58 (m,2H), 2.29 (s, 3H), 1.99-1.94 (m, 5H), 1.81-1.75 (m, 1H), 1.50(d, 3H), 1.41(s, 3H).

MS M/Z (ESI): m/z =594.6[M+1]⁺.

**Compound 107** isomer 2: ¹H NMR (400 MHz, CDCl₃) δ 7.36 (d, 1H), 7.23 (d, 1H), 7.17-7.15 (m, 1H), 5.74 (s, 1H), 5.46-5.40 (m, 2H), 4.12-3.82 (m, 5H), 3.66-3.64 (m, 2H), 3.08-3.03 (m, 2H), 2.88-2.85 (m,1H), 2.61-2.59 (m,2H), 2.29 (s, 3H), 2.17-2.02 (m, 5H), 1.92-1.86 (m, 1H), 1.50 (d, 3H), 1.42 (s, 3H).

MS M/Z (ESI): m/z =594.6[M+1]⁺.

Referring to the above operation, compound **107J-2** (0.31 g, 1.10 mmol) was used as the raw material to obtain the title **compound 107** isomer 3 (Rf=0.4 (ethyl acetate:methanol=10:1), 130.0 mg, 25.7%) and the title **compound 107** isomer 4 (Rf=0.2 (ethyl acetate:methanol=10:1), 110.0 mg, 21.75%).

**Compound 107** isomer 3: ¹H NMR (400 MHz, CD₃OD) δ 7.42 (d, 1H), 7.33 (d, 1H), 7.24-7.21 (m, 1H), 5.69 (s, 1H), 5.45-5.40 (m, 1H), 4.21-4.16 (m, 1H), 3.95-3.75 (m, 4H), 3.69-3.63 (m, 1H), 3.43-3.38 (m, 1H), 2.98-2.92 (m, 1H), 2.82-2.76 (m, 2H), 2.62-2.57 (m, 2H), 2.26 (s, 3H), 2.14-2.08 (m, 1H), 2.02-1.97 (m, 1H), 1.91-1.83 (m, 3H),1.76-1.72 (m, 1H), 1.50 (d, 3H), 1.36(s, 3H).

MS M/Z (ESI): m/z =594.6[M+1]⁺.

**Compound 107** isomer 4: ¹H NMR (400 MHz, CD₃OD) δ 7.43 (d, 1H), 7.34 (d, 1H), 7.25-7.22 (m, 1H), 5.73 (s, 1H), 5.47-5.41 (m, 1H), 4.19-4.14 (m, 1H), 4.07-3.98 (m, 2H), 3.83-3.77 (m, 3H), 3.50-3.44 (m, 1H), 3.22-3.13 (m, 1H), 3.09-3.03 (m, 2H), 2.57-2.48 (m, 2H), 2.42-2.38 (m, 1H), 2.26 (s, 3H), 2.21-2.01 (m, 4H), 1.82-1.78 (m, 1H), 1.50 (d, 3H), 1.39(s, 3H).

MS M/Z (ESI): m/z =594.6[M+1]⁺.

### Example 108:

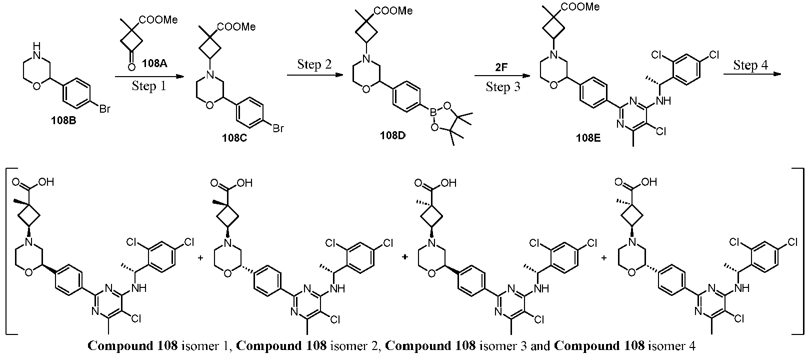

Step 1: Using **108A** (400 mg, 2.81 mmol) and **108B** (680 mg, 2.81 mmol) as the raw materials, the operation method of step 8 of example 9 was referenced to obtain **108C** (460 mg, 44%).

LC-MS (ESI): m/z = 368.4 [M+H]⁺.

Step 2: **108C** (460 mg, 1.25 mmol), pinacol diborate (413 mg, 1.63 mmol), dichloro[1,1'-bis(diphenylphosphino)ferrocene]palladium (91 mg, 0.13 mmol) and potassium acetate (245 mg, 2.50 mmol) were dissolved in DMSO (10 mL), and the reaction was performed overnight at 90°C under nitrogen atmosphere. After the reaction was completed, the mixture was cooled to room temperature, 30 mL of water was added, and the mixture was extracted with ethyl acetate (15 mL × 3). The organic layers were combined, washed with saturated brine (20 mL), dried over anhydrous sodium sulfate, filtered, and concentrated. The residue was separated and purified by silica gel column chromatography (dichloromethane:methanol (v/v) = 40:1) to obtain **108D** (415 mg, 80%).

LC-MS (ESI): m/z = 416.3 [M+H]⁺.

Step 3: **108D** (415 mg, 1.0 mmol), **2F** (351 mg, 1.0 mmol), dichloro[1,1'-bis(diphenylphosphino)ferrocene]palladium (73 mg, 0.10 mmol) and potassium carbonate (276 mg, 2.0 mmol) were dissolved in a mixed solvent of 1,4-dioxane (10 mL) and water (1 mL), and the reaction was performed at 90°C under nitrogen atmosphere overnight. After the reaction was completed, the mixture was cooled to room temperature, 30 mL of water was added to the reaction liquid, and the mixture was extracted with ethyl acetate (15 mL×3). The organic layers were combined, washed with saturated brine (20 mL), dried over anhydrous sodium sulfate, filtered, and concentrated. The residue was separated and purified by silica gel column chromatography (petroleum ether:ethyl acetate (v/v) = 4:1) to obtain **108E** (240 mg, 40%).

LC-MS (ESI): m/z = 603.2 [M+H]⁺.

Step 4: Using **108E** (240 mg, 0.40 mmol) as the raw material, the operation method of step 3 of example 1 was referenced to obtain **compound 108** isomer 1 (8 mg, 3.42%), **compound 108** isomer 2 (9 mg, 3.85%), **compound 108** isomer 3 (11 mg, 4.70%) and **compound 108** isomer 4 (9 mg, 3.85%).

HPLC analytical method: 1. Instruments: Shimadzu LC-2020; 2. Chromatographic column: Phenomenex C18; 3. Mobile phase system: A for 0.1% TFA in H₂O; B for ACN; 4. Gradient: B 10-80%; 5. Flow rate: 1.2 mL/min. retention time: Compound 108 isomer 1 retention time: tR = 1.794 min; Compound 108 isomer 2 retention time: tR = 1.908 min; Compound 108 isomer 3 retention time: tR = 2.074 min; Compound 108 isomer 4 retention time: tR = 2.247 min.

**Compound 108** isomer 1: ¹H NMR (400 MHz, CDCl₃) δ 8.14-8.11 (m, 2H), 7.34-7.33 (m, 1H), 7.30-7.28 (m, 2H), 7.22-7.19 (m, 1H), 7.11-7.07 (m, 1H), 5.62-5.58 (m, 2H), 4.54-4.50 (m, 1H), 4.01-3.97 (m, 1H), 3.80-3.75 (m, 1H), 2.85-2.81 (m, 2H), 2.72-2.68 (m, 1H), 2.61-2.49 (m, 2H), 2.44 (s, 3H), 2.06-2.02 (m, 1H), 1.92-1.79 (m, 3H), 1.52 (d, *J =* 6.0 Hz, 3H), 1.37 (s, 3H).

LC-MS (ESI): m/z = 589.2 [M+H]⁺.

**Compound 108** isomer 2: ¹H NMR (400 MHz, CDCl₃) δ 8.13-8.10 (m, 2H), 7.34-7.33 (m, 1H), 7.31-7.28 (m, 2H), 7.22-7.19 (m, 1H), 7.11-7.07 (m, 1H), 5.62-5.58 (m, 2H), 4.54-4.51 (m, 1H), 4.01-3.97 (m, 1H), 3.80-3.74 (m, 1H), 2.86-2.82 (m, 2H), 2.73-2.70 (m, 1H), 2.61-2.48 (m, 2H), 2.44 (s, 3H), 2.07-2.01 (m, 1H), 1.91-1.80 (m, 3H), 1.52 (d, *J =* 6.0 Hz, 3H), 1.36 (s, 3H).

LC-MS (ESI): m/z = 589.2 [M+H]⁺.

**Compound 108** isomer 3: ¹H NMR (400 MHz, CDCl₃) δ 8.16-8.13 (m, 2H), 7.36-7.31 (m, 3H), 7.23-7.20 (m, 1H), 7.12-7.08 (m, 1H), 5.64-5.58 (m, 2H), 4.69-4.66 (m, 1H), 4.09-4.04 (m, 1H), 3.95-3.88 (m, 1H), 3.17-3.14 (m, 1H), 3.08-3.04 (m, 1H), 2.86-2.82 (m, 1H), 2.58-2.55 (m, 2H), 2.45 (s, 3H), 2.20-2.06 (m, 3H), 1.96-1.90 (m, 1H), 1.52 (d, *J =* 6.0 Hz, 3H), 1.37 (s, 3H).

LC-MS (ESI): m/z = 589.2 [M+H]⁺.

**Compound 108** isomer 4: ¹H NMR (400 MHz, CDCl₃) δ 8.15-8.12 (m, 2H), 7.35-7.31 (m, 3H), 7.23-7.20 (m, 1H), 7.12-7.08 (m, 1H), 5.64-5.57 (m, 2H), 4.70-4.66 (m, 1H), 4.09-4.04 (m, 1H), 3.95-3.88 (m, 1H), 3.18-3.14 (m, 1H), 3.08-3.04 (m, 1H), 2.88-2.81 (m, 1H), 2.60-2.54 (m, 2H), 2.45 (s, 3H), 2.21-2.05 (m, 3H), 1.97-1.90 (m, 1H), 1.52 (d, *J =* 6.0 Hz, 3H), 1.37 (s, 3H).

LC-MS (ESI): m/z = 589.2 [M+H]⁺.

### Example 109:

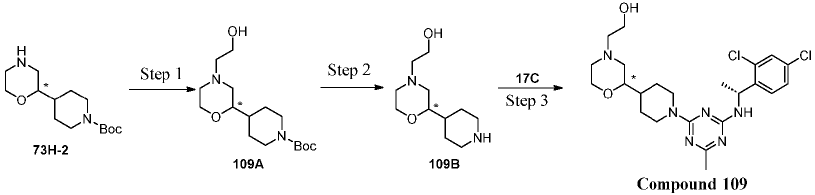

Step 1: Using **73H-2** (1.1 g, 4.07 mmol) as the raw material, the operation method of step 1 of example 87 was referenced to obtain compound **109A** (1.13 g, 88.3%).

LCMS m/z =315.2 [M+H]⁺.

Step 2: Using **109A** (200 mg, 0.64 mmol) as the raw material, the operation method of step 2 of example 87 was referenced to obtain compound **109B** (198 mg, 99.7%).

LCMS m/z =215.2 [M+H]⁺.

Step 3: Using **109B** (67 mg, 0.31 mmol) and **17C** (100 mg, 0.31 mmol) as the raw materials, the operation method of step 3 of example 87 was referenced to obtain **compound 109** (60 mg, 38.5%).

LCMS m/z =495.2[M+H]⁺.

¹H NMR (400 MHz, Chloroform-*d*) δ 7.34 (d, 1H), 7.30-7.26 (m, 1H), 7.20-7.15 (m, 1H), 5.56 -5.29 (m, 2H), 4.86-4.47 (m, 2H), 3.94-3.81 (m, 1H), 3.67-3.56 (m, 3H), 3.31-3.14 (m, 1H), 2.84- 2.45 (m, 7H), 2.27-2.18 (m, 4H), 2.04-1.69 (m, 3H), 1.67-1.53 (m, 1H), 1.45 (d, 3H), 1.35-1.05 (m, 2H).

### Example 110:

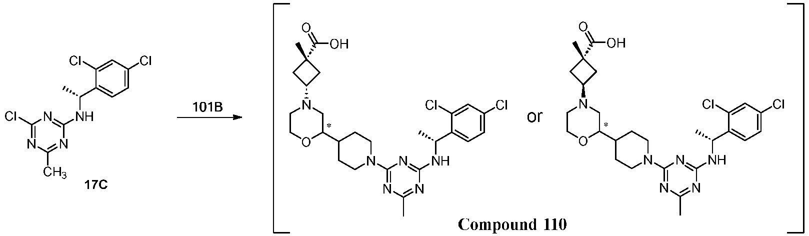

Using compound **101B** (178 mg, 0.52 mmol) and compound **17C** (200 mg, 0.63 mmol) as the raw materials, the operation method of step 10 of example 9 was referenced to obtain the title **compound 110** (50 mg, 28.2%).

¹H NMR (400 MHz, Chloroform-*d*) δ 7.98-7.87 (m, 1H), 7.35-7.29 (m, 2H), 7.19-7.15 (m, 1H), 5.47-5.25 (m, 1H), 4.87-4.44 (m, 2H), 3.94-3.81 (m, 1H), 3.75-3.56 (m, 1H), 3.40-3.20 (m, 1H), 2.93-2.45 (m, 7H), 2.22 (s, 3H), 2.01-1.52 (m, 7H), 1.49-1.39 (m, 6H), 1.32-1.04 (m, 2H).

LCMS m/z = 563.2 [M+H]⁺.

### Example 111:

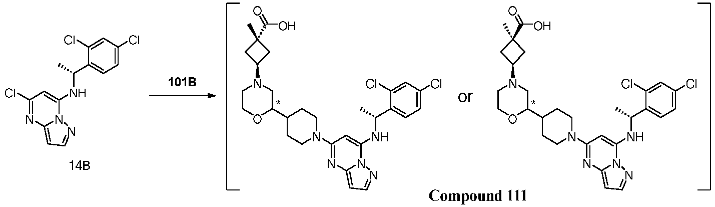

Using **14B** (61.5 mg, 0.18 mmol) and **101B** (50.0 mg, 0.18 mmol) as the raw materials, the operation method of step 10 of example 9 was referenced to obtain **compound 111** (25 mg, 24%).

¹H NMR (400 MHz, DMSO) δ 11.65 (s, 1H), 7.94 (d, 1H), 7.82 (d, 1H), 7.65 (dd, 2H), 7.43 (dd, 1H), 5.90 (d, 1H), 5.16 (s, 1H), 5.10-5.04 (m, 1H), 4.16 (d, 2H), 3.75 (d, 1H), 3.43-3.36 (m, 1H), 3.12-3.06 (m, 1H), 2.78-2.53 (m, 5H), 2.41 (dd, 2H), 1.80-1.65 (m, 5H), 1.57 (dd, 7H), 1.26 (s, 3H).

LC-MS (ESI): m/z =587.3 [M+H]⁺.

### Example 112:

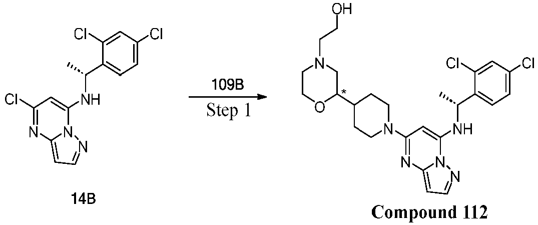

Using **109B** (67 mg, 0.31 mmol) and **17B** (100 mg, 0.31 mmol) as the raw materials, the operation method of step 3 of example 87 was referenced to obtain **compound 112** (27 mg, 20%).

¹H NMR (400 MHz, DMSO-*d6*) δ 7.92 (d, 1H), 7.82 (d, 1H), 7.64 (dd, 2H), 7.43 (dd, 1H), 5.90 (d, 1H), 5.16 (s, 1H), 5.07 (p, 1H), 4.36 (s, 1H), 4.16 (d, 2H), 3.72 (d,1H), 3.49 (t, 1H), 3.45- 3.41 (m, 1H), 3.14-3.08 (m, 1H), 2.71 (dt, 4H), 2.36 (td, 2H), 2.01 (td, 1H), 1.85-1.70 (m, 3H), 1.57 (dd, 5H), 1.17 (d, 1H), 1.01 (dd, 1H).

LC-MS (ESI): m/z =519.7 [M+H]⁺.

### Example 113:

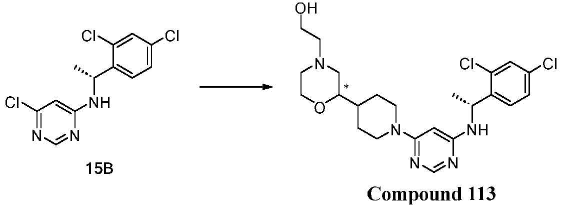

Using **109B** (67 mg, 0.31 mmol) and **15B** (100 mg, 0.31 mmol) as the raw materials, the operation method of step 3 of example 87 was referenced to obtain **compound 113** (80 mg, 35.43%).

¹H NMR (400 MHz, Chloroform-d) δ 8.14-8.04 (m, 1H), 7.39-7.37(m, 1H), 7.367-7.32(m, 1H), 7.24-7.19 (m, 1H), 5.70 (s, 1H), 5.12 (s, 1H), 5.03-4.91 (m, 1H), 4.30-4.10 (m, 2H), 3.93-3.82 (m, 1H), 3.69-3.56 (m, 3H), 3.29-3.20 (m, 1H), 2.82-2.66 (m, 4H), 2.59-2.52 (m, 2H), 2.27-2.19 (m, 1H), 2.04-1.95 (m, 1H), 1.93-1.84 (m, 1H), 1.69-1.55 (m, 2H), 1.54-1.46 (m, 3H), 1.33-1.08 (m, 2H).

LCMS (ESI): m/z =480.5 [M+H]⁺.

### Example 114:

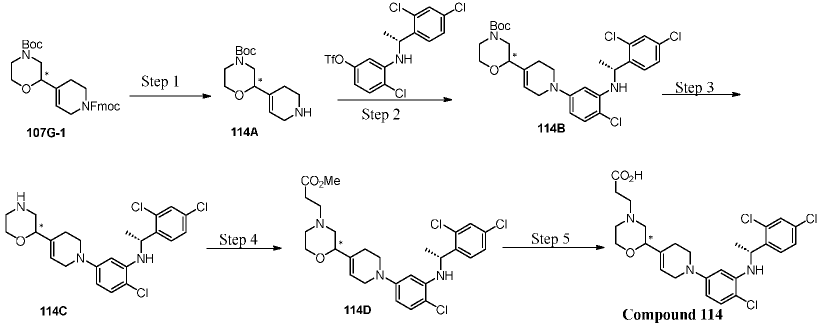

Step 1: Compound **107G-1** (350 mg, 0.713 mmol) was dissolved in a mixed solvent of tetrahydrofuran (6 mL) and water (2 mL), and lithium hydroxide monohydrate (89.8 mg, 2.14 mmol) was added at room temperature, after the addition was completed, the reaction was performed at room temperature for about 4 h. After disappearance of the raw materials as monitored by TLC, water (5 mL) was added, and the mixture was extracted with dichloromethane (5 mL×8). The organic phases were combined, dried over anhydrous sodium sulfate, filtered and concentrated. The crude product was purified by silica gel column chromatography (dichloromethane:methanol (v/v) = 100:0 to 80:20) to obtain compound **114A** (144 mg, 75.2%).

LCMS (ESI): m/z =269.2 [M+H]⁺.

Step 2: Using compounds **114A** (135 mg, 0.503 mmol) and **75E** (293 mg, 0.654 mmol) as the raw materials, the operation method of step 2 of example 82 was referenced to obtain compound **114B** (179 mg, 62.8%).

LCMS (ESI): m/z =566.2 [M+H]⁺.

Step 3: Compound **114B** (179 mg, 0.306 mmol) was dissolved in dry dichloromethane (6 mL), trifluoroacetic acid (2 mL) was added at room temperature, after the addition was completed, the reaction was continued for 1 h. After the reaction was completed as monitored by LCMS, the obtained system was concentrated to obtain the crude trifluoroacetate salt of **114C** (350 mg), which was used in the next step without purification.

LCMS (ESI): m/z =466.1 [M+H]⁺.

Step 4: Using the trifluoroacetate salt of compound **114C** (200 mg) and methyl 3-bromopropionate (84.6 mg, 0.507 mmol) as the raw materials, the operation method of step 1 of example 88 was referenced to obtain compound **114D** (62 mg, two-step yield: 62.2%).

LCMS (ESI): m/z =552.2 [M+H]⁺.

Step 5: Using compound **114D** (62 mg, 0.112 mmol) as the raw material, the operation method of step 4 of example 76 was referenced to obtain **compound 114** (36 mg, 60.4%).

¹H NMR (400 MHz, Methanol-*d*₄) *δ* 7.47 (d, 1H), 7.41 (d, 1H), 7.26 (dd, 1H), 7.03 (d, 1H), 6.20 (dd, 1H), 5.85 - 5.75 (m, 2H), 4.96 (t, 1H), 4.01 (ddd, 2H), 3.80 - 3.71 (m, 1H), 3.57 - 3.39 (m, 2H), 3.23 - 3.06 (m, 4H), 2.98 (t, 2H), 2.64 - 2.40 (m, 4H), 2.16 (tt, 2H), 1.54 (d, 3H).

LC-MS (ESI): m/z =538.1 [M+H]⁺.

### Example 115:

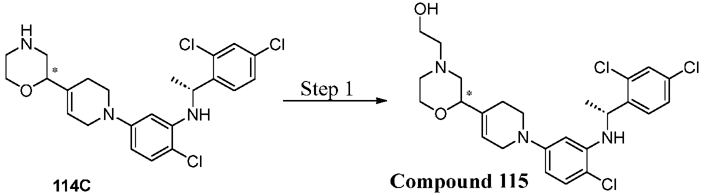

Using the crude trifluoroacetate salt of compound **114C** (150 mg) as the raw material, step 1 of example 87 was referenced to obtain compound **115** (21 mg, two-step yield: 30.4%).

¹H NMR (400 MHz, Methanol-*d4*) *δ* 7.46 (d, 1H), 7.40 (d, 1H), 7.24 (dd, 1H), 7.03 (d, 1H), 6.19 (dd, 1H), 5.81 (d, 1H), 5.73 (dq, 1H), 4.94 (q, 1H), 3.98-3.84 (m, 2H), 3.76-3.65 (m, 3H), 3.55 -3.36 (m, 2H), 3.20-3.04 (m, 2H), 2.88 (ddt, 2H), 2.59 (t, 2H), 2.27 (td, 1H), 2.18-2.07 (m, 3H), 1.53 (d, 3H).

LCMS (ESI): m/z =510.1 [M+H]⁺.

### Example 116:

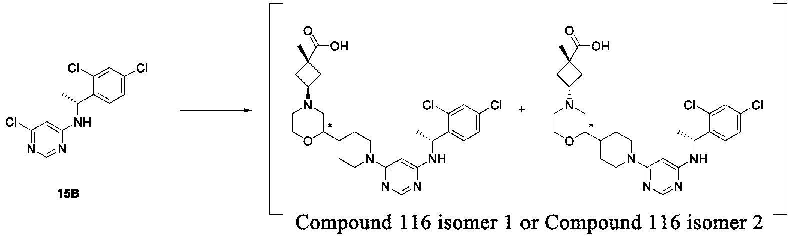

Using **73J-2** (280 mg, 0.99 mmol) and **15B** as the raw materials, the operation method of step 10 of example 73 was referenced to obtain **compound 116** isomer 1 (Rf=0.3 (ethyl acetate:methanol (v/v)= 9:1), 60 mg, 11.05%) and **compound 116** isomer 2 (Rf=0.2 (ethyl acetate: methanol (v/v)= 9:1), 40 mg, 7.37%).

**Compound 116** isomer 1: LCMS m/z =548.3 [M+H]⁺.

¹H NMR (400 MHz, Chloroform-d) δ 10.29-10.12 (m, 1H), 7.99 (s, 1H), 7.49-7.41 (m, 1H), 7.40-7.35 (m, 1H), 7.31-7.26 (m, 1H), 5.14 (s, 1H), 4.97-4.87 (m, 1H), 4.07-3.98 (m, 1H), 3.98-3.86 (m, 1H), 3.73-3.62 (m, 1H), 3.55-3.32 (m, 3H), 3.05-2.73 (m, 4H), 2.66-2.51 (m, 1H), 2.40-2.27 (m, 1H), 2.26-2.07 (m, 2H), 1.93-1.82 (m, 1H), 1.82-1.66 (m, 2H), 1.65-1.53 (m, 3H), 1.45 (s, 3H).

**Compound 116** isomer 2: LCMS m/z =548.3 [M+H]⁺.

¹H NMR (400 MHz, Methanol-d4) δ 8.11 (s, 1H), 7.54-7.49 (m, 1H), 7.47-7.41 (m, 1H), 7.39-7.33 (m, 1H), 5.48 (s, 1H), 5.23-5.11 (m, 1H), 4.38-4.18 (m, 2H), 4.14-4.06 (m, 1H), 3.83-3.67 (m, 2H), 3.42-3.34 (m, 1H), 3.42-3.34 (m, 2H), 3.07-2.95 (m, 2H), 2.93-2.82 (m, 1H), 2.80-2.71 (m, 1H), 2.71-2.62 (m, 2H), 2.36-2.24 (m, 2H), 1.95-1.80 (m, 2H), 1.78-1.69 (m, 1H), 1.62-1.53 (m, 3H), 1.51-1.37 (s, 4H), 1.35-1.20 (m, 1H).

### Example 117:

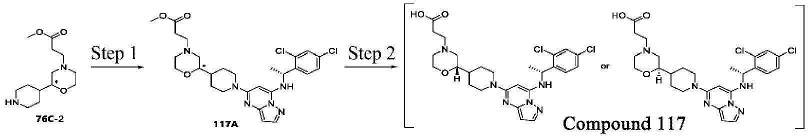

Using **14B** (95.2 mg, 0.31 mmol) and **76C-2** (80.0 mg, 0.31 mmol) as the raw materials, the operation method of steps 3 and 4 of example 76 was referenced to obtain **compound 117** (14 mg, 18.3%).

¹H NMR (400 MHz, DMSO) δ 7.96 (d, 1H), 7.82 (d, 1H), 7.66 (d, 1H), 7.64 (d, 1H), 7.43 (dd, 1H), 5.90 (d, 1H), 5.16 (s, 1H), 5.11-5.03 (m, 1H), 4.17 (d, 2H), 3.74 (d, 1H), 3.44-3.35 (m, 1H), 3.12-3.04 (m, 1H), 2.74 (d, 3H), 2.64 (d, 1H), 2.53 (d, 1H), 2.34 (t, 2H), 1.98 (dd, 1H), 1.89 (s, 2H), 1.77 (dd, 2H), 1.59 (d, 4H), 1.55 (s, 1H), 1.25 (s, 1H).

LC-MS (ESI): m/z =547.2 [M+H]⁺.

### Example 118:

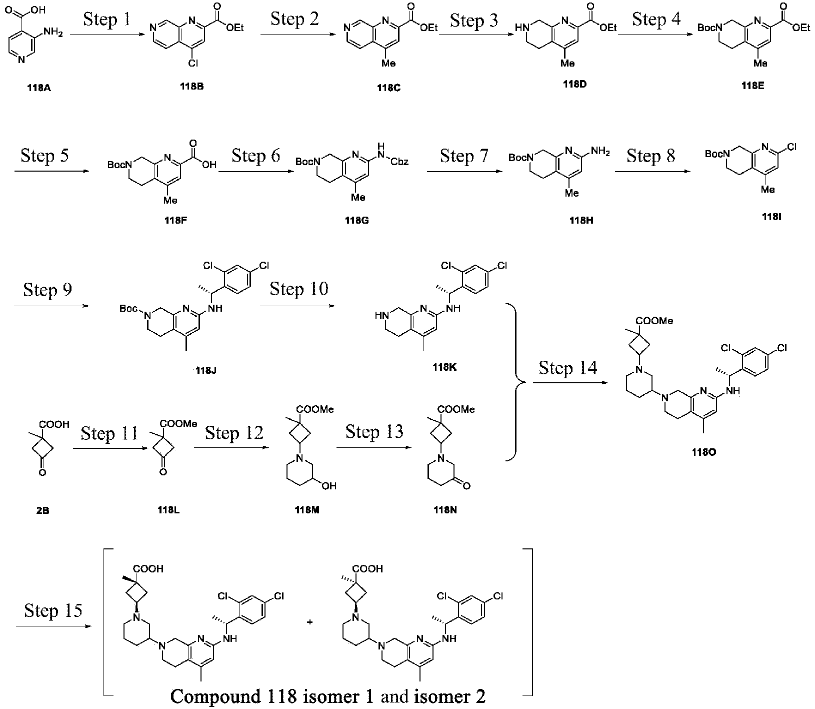

Step 1: **118A** (35.0 g, 0.25 mol) was dissolved in ethyl pyruvate (105 mL) and stirred at room temperature for 30 min, then phosphorus oxychloride (175 mL) was added, after the addition was completed, the temperature was raised to 100°C and the reaction was performed for 3 h. After the reaction was complete, the mixture was cooled to room temperature and concentrated to remove most of the phosphorus oxychloride. Ethyl acetate (500 mL) was added to the residue, and then the obtained system was poured into 500 g of ice cubes. The pH was adjusted to 7-8 with saturated aqueous sodium bicarbonate solution, the obtained system was filtered through diatomaceous earth, and the filtrate was extracted to separate the organic phase. The aqueous phase was extracted with ethyl acetate (500 mL × 2). The combined organic phases were washed with saturated brine, dried over anhydrous sodium sulfate, filtered, and concentrated to obtain a crude product. The crude product was separated and purified by column chromatography (petroleum ether:ethyl acetate (v/v) = 100:0-1:1) to obtain compound **118B** (12.1 g, 20.23%).

LC-MS (ESI): m/z =237.1[M+H]⁺.

Step 2: Compound **118B** (13.0 g, 54.3 mmol) was dissolved in 1,4-dioxane (150 mL), and then methylboronic acid (6.55 g, 0.11mol), Pd(PPh₃)₄ (6.35 g, 5.5 mmol), and potassium carbonate (30.4 g, 0.22mol) were added in sequence, after the addition was completed, nitrogen replacement was performed, and then the temperature was raised to 100°C and the reaction was performed overnight. After the reaction was completed, the mixture was cooled to room temperature and filtered. The filter cake was washed with ethyl acetate (20 mL×3). The filtrate was concentrated to obtain a crude product, which was separated and purified by column chromatography (petroleum ether:ethyl acetate (v/v) = 100:0-1:1) to obtain the product **118C** (11.2 g, 94.29%).

LC-MS (ESI): m/z =217.3[M+H]⁺.

Step 3: Compound **118C** (11.2 g, 51.79 mmol) was dissolved in acetic acid (100 mL), and sodium cyanoborohydride (13.4 g, 0.21 mol) was added in portions at room temperature, after the addition was completed, the reaction was performed at room temperature overnight. After the reaction was completed, the mixture was concentrated to obtain a crude compound **118D,** which was directly used in the next reaction.

LC-MS (ESI): m/z =221.2[M+H]⁺.

Step 4: The crude compound **118D** obtained in the previous step was dissolved in tetrahydrofuran (200 mL), water (300 mL) was added and stirred evenly, then sodium bicarbonate solid was added in portions to adjust the pH to 8-9, and then di-tert-butyl dicarbonate (16.9 g, 77.63 mmol) was added, after the addition was completed, the reaction was performed at room temperature overnight. Ethyl acetate (200 mL×2) was added for extraction. The combined organic phases were washed with saturated brine, dried over anhydrous sodium sulfate, filtered, and concentrated to obtain a crude product. The crude product was separated and purified by column chromatography (petroleum ether:ethyl acetate (v/v) = 100:0-1:1) to obtain compound **118E** (11.9 g, 77.47%).

LC-MS (ESI): m/z =321.2[M+H]⁺.

Step 5: Compound **118E** (11.9 g, 37.1 mmol) was dissolved in tetrahydrofuran (100 mL), water (50 mL) and lithium hydroxide monohydrate (2.3 g, 55.71 mmol) were added in sequence, and the obtained system was stirred at room temperature overnight. After the reaction was completed, the obtained system was cooled to 0-5°C, 0.5M hydrochloric acid was added dropwise to adjust the pH to 4-5, and then ethyl acetate (200 mL×2) was added for extraction. The combined organic phases were washed with saturated brine, dried over anhydrous sodium sulfate, filtered, and concentrated to remove most of the solvent. Petroleum ether (50 mL) was added and stirred to precipitate a solid, filtration was performed, and the filter cake was dried to obtain compound **118F** (10.0g, 92.11%).

LC-MS (ESI): m/z =293.2 [M+H]⁺.

Step 6: Compound **118F** (8.0 g, 27.4 mmol) was dissolved in 1,4-dioxane (100 mL), and triethylamine (13.8 g, 0.14 mmol) was added. DPPA (15.0 g, 54.7 mmol) was added dropwise at 0°C. After the dropwise addition was completed, the mixture was stirred at room temperature for 30 min. Benzyl alcohol (8.8 g, 82.2 mmol) was then added. The obtained system was then heated to 100°C and reacted for 5 h. After the reaction was completed, the obtained system was cooled to room temperature, and concentrated, most of the solvent was removed, ethyl acetate (100 mL) was added for dilution, the organic phase was washed with saturated brine, dried over anhydrous sodium sulfate, filtered, and concentrated to obtain a crude product, which was separated and purified by column chromatography (petroleum ether:ethyl acetate (v/v) = 100:0-2:1) to obtain the product **118G** (1.9 g, 17.47%).

LC-MS (ESI): m/z =398.5 [M+H]⁺.

Step 7: Compound **118G** (1.9 g, 4.7 mmol) was dissolved in methanol (30 mL), 10% Pd/C (0.2 g) was added, and the mixture was stirred at room temperature overnight under a hydrogen atmosphere. After the reaction was completed, the obtained system was filtered and the filtrate was concentrated to obtain a crude product, which was separated and purified by column chromatography (DCM: MeOH (v/v) = 100:0-10:1) to obtain compound **118H** (0.8 g, 63.55%).

LC-MS (ESI): m/z =264.2 [M+H]⁺.

Step 8: Compound **118H** (0.4 g, 1.5 mmol) was dissolved in dry dichloromethane (10 mL), anhydrous copper chloride (0.40 g, 3.1 mmol) was added, and then isoamyl nitrite (0.87 g, 7.6 mmol) was added dropwise, after the dropwise addition was completed, the obtained system was stirred at room temperature for 40 h. Filtration was performed and the filtrate concentrated to obtain a crude product, which was separated and purified by column chromatography (petroleum ether:ethyl acetate (v/v) = 100:0-5:1) to obtain compound **1181** (0.21 g, 48.86%).

LC-MS (ESI): m/z =283.1 [M+H]⁺.

Step 9: Compound **1181** (0.21 g, 0.74 mmol) was dissolved in dry 1,4-dioxane (5 mL), and then (R)-1-(2,4-dichlorophenyl)ethan-1-amine (0.28 g, 1.47 mmol), Pd₂ (dba)₃ (0.14 g, 0.15mmol), XantPhos (0.17 g, 0.30 mmol), and potassium carbonate (0.51 g, 3.7 mmol) were added in sequence. After the addition was completed, the obtained system was heated to 100°C under nitrogen atmosphere and reacted overnight. The mixture was cooled to room temperature, diluted with ethyl acetate (10 mL), and filtered, and the filtrate was concentrated to obtain a crude product, which was separated and purified by column chromatography (petroleum ether:ethyl acetate (v/v) = 100:0-1:1) to obtain compound **118J** (0.20 g, 61.94%).

LC-MS (ESI): m/z =436.6 [M+H]⁺.

Step 10: Compound **118J** (0.20g, 0.46 mmol) was dissolved in 1,4-dioxane (2 mL), then hydrogen chloride 1,4-dioxane solution (2.0 mL, 4M) was added, after the addition was completed, the obtained system was stirred at room temperature until the raw materials disappeared. Saturated aqueous potassium carbonate solution was added to adjust the pH to 9-10, and ethyl acetate (10 mL ×3) was used for extraction. The combined organic phase was washed with saturated brine, dried over anhydrous sodium sulfate, and concentrated to obtain the product compound **118K** (0.14 g, 93.3%).

LC-MS (ESI): m/z =336.1 [M+H]⁺.

Step 11: Compound **2B** (1.5 g, 11.7 mmol) was dissolved in dry DMF (20 mL), potassium carbonate powder (2.4 g, 17.5 mmol) was added, and stirred for 5 min, then methyl iodide (3.3 g, 23.4 mmol) was added dropwise, after the dropwise addition was completed, the obtained system was stirred at room temperature for 4 h After the reaction was completed, water (60 mL) was added and the obtained system was extracted with methyl tert-butyl ether (20 mL ×3), and the combined organic phases were washed with saturated brine, dried over anhydrous sodium sulfate, and concentrated to obtain the crude compound **118L** which was directly used in the next reaction.

LC-MS (ESI): m/z =143.1 [M+H]⁺.

Step 12: The crude compound **118L** was dissolved in 1,2-dichloroethane (40 mL), 3-hydroxypiperidine (2.4 g, 23.4 mmol) was added, and then acetic acid (0.7 g, 11.7 mmol) was added dropwise. After the addition was completed, the mixture was stirred for 30 min. The obtained system was cooled to 0°C, sodium triacetoxyborohydride (4.9 g, 23.4 mmol) was added, and the obtained system was allowed to warm to room temperature naturally overnight. Silica gel (5.0 g) was added and the obtained system was concentrated. The residue was separated and purified by column chromatography (DCM: MeOH (v/v) = 100:0-5:1) to obtain compound **118M** (0.92 g, 34.65%).

LC-MS (ESI): m/z =228.2 [M+H]⁺.

Step 13: Oxalyl chloride (1.34 g, 10.5 mmol) was dissolved in dry dichloromethane (20 mL), the temperature was lowered to -70°C under nitrogen atmosphere, dimethyl sulfoxide (1.64 g, 21.1 mmol) was added dropwise, and stirred for 30 min, a solution of compound **118M** (0.8 g, 3.5 mmol) in dichloromethane (5 mL) was added dropwise, after the dropwise addition was completed, the reaction was continued for 30 min. DIPEA (4.1 g, 31.7 mmol) was added dropwise, and after the dropwise addition was completed, the obtained system was naturally warmed to room temperature and reacted for 3 h. The reaction liquid was poured into an aqueous ammonium chloride solution (20 mL), extraction was performed, the aqueous phases were extracted with dichloromethane (50 mL), the combined organic phases were washed with saturated brine, dried over anhydrous sodium sulfate, filtered, and concentrated, the obtained crude product was separated and purified by column chromatography (ethyl acetate:petroleum ether (v/v) = 100:0-3:1) to obtain compound **118N** (0.55 g, 69.36%).

LC-MS (ESI): m/z =226.3 [M+H]⁺.

Step 14: Compound **118K** (0.14 g, 0.42 mmol) was dissolved in 1,2-dichloroethane (5 mL), compound **118N** (0.14 g, 0.62 mmol) was added, and 3 drops of acetic acid were added dropwise, after the addition was completed, the obtained system was stirred for 10 min, then the temperature was cooled to 0°C, sodium triacetoxyborohydride (0.17 g, 0.84 mmol) was added, and the reaction was performed at room temperature overnight. Saturated aqueous sodium bicarbonate solution (10 mL) was added to the reaction liquid, and the mixture was stirred for 30 min for extraction and liquid separation. The aqueous phase was extracted with dichloromethane (20 mL×3). The combined organic phases were dried over anhydrous sodium sulfate, filtered, and concentrated to obtain the crude compound **118O,** which was directly used in the next reaction.

LC-MS (ESI): m/z =545.8 [M+H]⁺.

Step 15: The crude compound **118O** was dissolved in tetrahydrofuran (5 mL), 10% aqueous lithium hydroxide solution (5 mL) was added, and stirred at room temperature overnight, 5% HCl was added dropwise to adjust the pH to 5-6, the obtained system was concentrated, the obtained crude product was purified by preparative high performance liquid phase chromatography to obtain compound **118** isomer 1 (26.0 mg, yield: 11.65%), and compound **118** isomer 2 (23.0 mg, yield: 10.30%).

Preparation method: instrument: waters 2767 preparative liquid chromatographic instrument; chromatographic column: SunFire @ Prep C18 (19 mm × 250 mm). The sample was dissolved in methanol and filtered through a 0.22 µm filter head to prepare a sample liquid. Preparative chromatography conditions: composition of mobile phases A and B: mobile phase A: acetonitrile, mobile phase B: water (containing 1% TFA), gradient elution, mobile phase A: 10% to 70%, flow rate: 10 mL/min. Elution time: 25 min.

Analytical method: instrument: Shimadzu LC-20AT, column: chromatographic column model: Xtimate C18 4.6*50mm, 3µm, mobile phase A: 0.05% TFA solution, mobile phase B: acetonitrile, gradient: A 95-5% B 5-95%, flow rate: 1mL/min, column temperature: 35°C, wavelength: 210 nm/254 nm, acquisition time: 10 min.

**Compound 118** isomer 1: retention time: 3.312min, LC-MS (ESI): m/z =531.3 [M+H]⁺.

¹H NMR (400 MHz, CD3OD) δ7.40-7.37 (m, 2H), 7.21-7.18 (m, 1H), 6.09 (s, 1H), 5.18-5.13(m, 1H), 3.66-3.52 (m, 2H), 3.24-3.09 (m, 3H), 2.93-2.79 (m, 3H), 2.60 -2.58 (m, 2H), 2.46-2.29 (m, 4H), 2.15-1.99 (m, 6H), 1.70-1.51 (m, 2H), 1.45 (d, 3H), 1.37-1.27 (m, 4H).

**Compound 118** isomer 2: retention time: 3.333 min, LC-MS (ESI): m/z =531.5 [M+H]⁺.

¹H NMR (400 MHz, CD3OD) δ7.44-7.40 (m, 2H), 7.25-7.22 (m, 1H), 6.26 (s, 1H), 5.22-5.17(m, 1H), 3.86-3.72 (m, 2H), 3.63-3.59 (m, 1H), 3.21-3.04 (m, 4H), 2.83-2.74 (m, 3H), 2.67-2.64 (m, 3H), 2.21-2.14 (m, 5H), 2.04-2.02 (m, 2H), 1.76-1.74 (m, 2H), 1.50 (d, 3H),1.39 (s, 3H), 1.33-1.28 (m, 4H).

### Example 119:

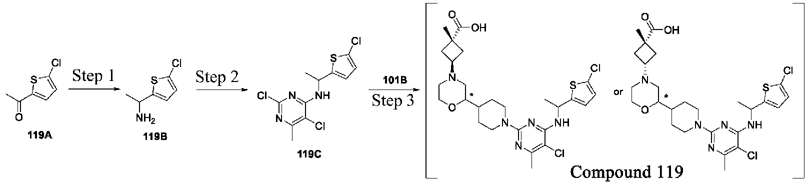

Step 1: Compound **119A** (2 g, 12.45 mmol) was dissolved in a solution ammonia in methanol (20 mL, 7M), tetraisopropyl titanate (7.08 g, 24.90 mmol) was added dropwise, and stirred for 2-3h, then the temperature was lowered to 0°C, sodium borohydride (568 mg, 14.94 mmol) was added in portions, and after the addition was completed, stirring was continued at room temperature for 3h. After the reaction was completed as monitored by LCMS, the reaction liquid was poured into 2M aqueous ammonia (40 mL), the obtained system was stirred for 5 min and filtered, and the filtrate was concentrated to remove most of the methanol. The pH was adjusted to acidic with dilute hydrochloric acid, the obtained system was extracted with ethyl acetate, the aqueous phase was retained, the pH was adjusted to alkaline with aqueous sodium hydroxide solution, the obtained system was extracted again with ethyl acetate (30 mL×3), the organic phases were combined and concentrated to obtain the compound **119B** of interest (1 g, 49.7%).

LCMS m/z=162.1&164.1 [M+H]⁺.

Step 2: Compound **119B** (500 mg, 3.09 mmol) and 2,4,5-trichloro-6-methylpyrimidine (611 mg, 3.09 mmol) were dissolved in acetonitrile (10 mL), and triethylamine (939 mg, 9.28 mmol) was added, the reaction was performed under stirring at 80°C for 5 h. After the reaction was completed as monitored by LCMS, the mixture was cooled to room temperature, water (30 mL) was added, and the mixture was extracted with ethyl acetate (15 mL×3). The organic phases were combined, concentrated, and separated by a silica gel column (petroleum ether:ethyl acetate (v/v) = 10:1) to obtain the compound **119C** of interest (850 mg, 85.2%).

LCMS m/z= 322.0&324.0 [M+H]⁺.

Step 3: Using compound **119C** (200 mg, 0.62 mmol) and compound **101B** (175 mg, 0.62 mmol) as the raw materials, the operation method of step 10 of example 9 was referenced to obtain **compound 119** (150 mg, 42.6%).

LCMS m/z = 568.2&570.2 [M+H]⁺.

¹H NMR (400 MHz, Chloroform-d) δ 6.97-6.93 (m, 1H), 6.85-6.82 (m, 1H), 5.49 -5.39 (m, 1H), 5.32-5.27 (m, 1H), 4.70 (d, 2H), 3.96-3.86 (m, 1H), 3.79-3.66 (m, 1H), 3.47-3.28 (m, 1H), 3.13-2.81 (m, 2H), 2.78-2.66 (m, 2H), 2.66-2.57 (m, 2H), 2.30 (s, 3H), 2.18-1.96 (m, 3H), 1.93-1.76 (m, 2H), 1.68-1.53 (m, 5H), 1.40 (s, 3H), 1.33-1.14 (m, 2H).

### Example 120:

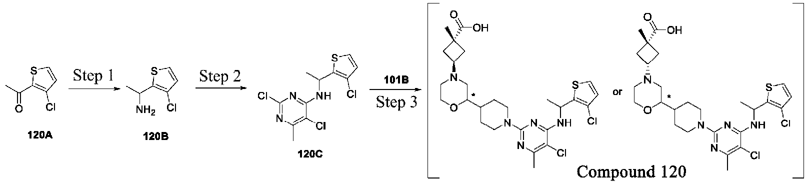

Using **120A** (2 g, 12.45 mmol) as the raw material, the operation method of steps 1 to 3 of example 119 was referenced to obtain **compound 120** (60 mg, 34.0%).

LCMS m/z = 568.2&570.2 [M+H]⁺.

¹H NMR (400 MHz, Chloroform-*d*) δ 7.12-7.07 (m, 1H), 6.85-6.81 (m, 1H), 5.54 -5.45 (m, 1H), 5.42-5.36 (m, 1H), 4.71-4.61 (m, 2H), 3.93-3.83 (m, 1H), 3.67-3.57 (m, 1H), 3.29-3.19 (m, 1H), 2.90-2.54 (m, 6H), 2.28 (s, 3H), 2.02-1.79 (m, 4H), 1.74-1.48 (m, 6H), 1.42 (d, 3H), 1.22-1.05 (m, 2H).

### Example 121:

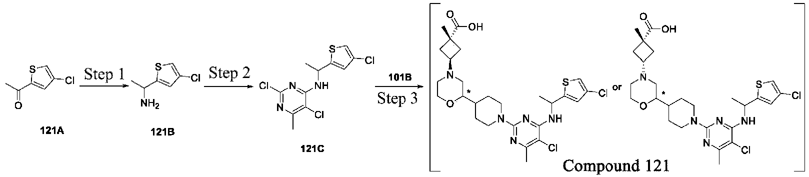

Using **121A** (2 g, 12.45 mmol) as the raw material, the operation method of steps 1 to 3 of example 119 was referenced to obtain **compound 121** (50 mg, 28.4%).

LCMS m/z = 568.2&570.2 [M+H]⁺.

¹H NMR (400 MHz, Chloroform-*d*) δ 6.75-6.70 (m, 2H), 5.46-5.35 (m, 1H), 5.29 -5.22 (m, 1H), 4.77-4.67 (m, 2H), 3.94-3.83 (m, 1H), 3.70-3.59 (m, 1H), 3.34-3.22 (m, 1H), 2.92-2.55 (m, 6H), 2.29 (s, 3H), 2.02-1.83 (m, 4H), 1.77-1.67 (m, 1H), 1.65-1.57 (m, 5H), 1.41 (s, 3H), 1.31-1.17 (m, 2H).

### Example 122:

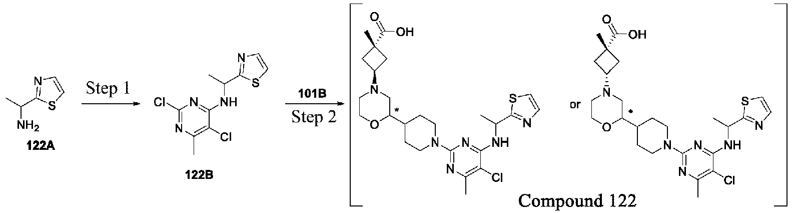

Using compound 2,4,5-trichloro-6-methylpyrimidine (500 mg, 2.53 mmol) and 1-(2-thiazolyl)ethylamine (649 mg, 5.06 mmol) as the raw materials, the operation method of steps 2 and 3 of example 119 was referenced to obtain compound **122** (130 mg, 41.3%).

¹H NMR (400 MHz, Methanol-*d*₄) δ 7.68 (d, 1H), 7.43 (dd, 1H), 5.50 (q, 1H), 4.61-4.48 (m, 2H), 3.88 (dd, 1H), 3.59 (td, 1H), 3.29-3.18 (m, 1H), 2.97-2.77 (m, 3H), 2.72-2.55 (m, 4H), 2.28 (s, 3H), 2.15-2.03 (m, 1H), 1.91-1.72 (m, 4H), 1.69 (d, 3H), 1.65-1.46 (m, 2H), 1.35 (d, 3H), 1.28-0.98 (m, 2H).

LC-MS (ESI): m/z =535.2 [M+H]⁺.

### Example 123:

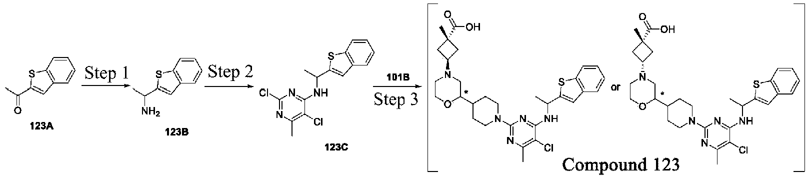

Using compound **123A** (5 g, 28.4 mmol) as the raw material, the operation method of step 1 to step 3 of example 119 was referenced to obtain **compound 123** (116 mg, 33.8%).

¹H NMR (400 MHz, Methanol-*d*₄) δ 7.79-7.72 (m, 1H), 7.71-7.64 (m, 1H), 7.29 (tt, 1H), 7.25 (ddt, 1H), 7.21 (s, 1H), 5.61-5.47 (m, 1H), 4.63 (d, 2H), 3.88 (dd, 1H), 3.55 (ddd, 1H), 3.22-3.12 (m, 1H), 2.98 (p, 1H), 2.81 (q, 2H), 2.75-2.67 (m, 2H), 2.64 -2.54 (m, 2H), 2.28 (s, 3H), 2.11 (t, 1H), 1.92-1.81 (m, 3H), 1.78-1.66 (m, 4H), 1.65- 1.40 (m, 2H), 1.37 (d, *J =* 2.4 Hz, 3H), 1.24-0.95 (m, 2H).

LC-MS (ESI): m/z =584.2 [M+H]⁺.

### Example 124:

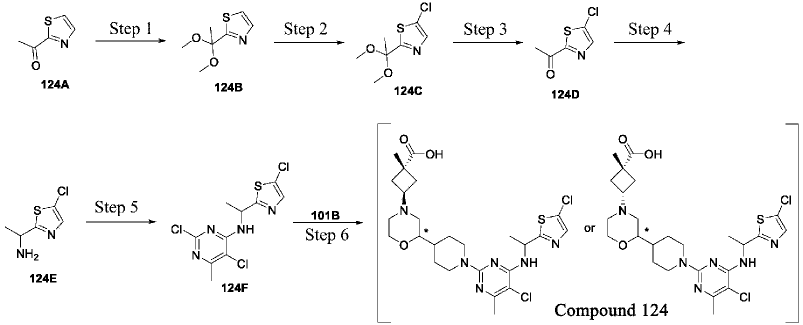

Step 1: Compound **124A** (7 g, 55.1 mmol) was dissolved in methanol (100 mL), trimethyl orthoformate (34 g, 320.8 mmol) and p-toluenesulfonic acid (10 g, 58.1 mmol) were added, the obtained system was stirred at 50°C for 12h. After the reaction was completed, the obtained system was cooled to room temperature and concentrated, saturated aqueous sodium bicarbonate solution (150 mL) was added to the residue, and extraction was performed with ethyl acetate (50 mL×3). The organic phases were combined and concentrated, and then separated and purified using a silica gel column (ethyl acetate:petroleum ether (v/v) = 10:90) to obtain the compound **124B** of interest (6.9 g, 72.3%).

LCMS m/z=174.1[M+H]⁺.

Step 2: Compound **124B** (6.5 g, 37.5 mmol) was dissolved in tetrahydrofuran (100 mL), n-butyllithium solution (15 mL, 2.5M solution in toluene) was slowly added dropwise at -78°C, and stirred for 30 min, then a solution of carbon tetrachloride (28.8 g, 187.6 mmol) in tetrahydrofuran (50 mL) was added dropwise to the reaction system, after the dropwise addition was completed, stirring was continued for 30 min. After the reaction was completed as monitored by TLC, the obtained system was warmed to 0°C and quenched by the addition of saturated aqueous ammonium chloride (150 mL). The obtained system was extracted with ethyl acetate (50 mL×3). The organic phases were combined, concentrated, and separated and purified using a silica gel chromatography column (ethyl acetate: petroleum ether (v/v) = 10:90) to obtain the compound **124C** of interest (4.4 g, 56.4%).

LCMS m/z=208.1&210.1[M+H]⁺.

Step 3: Compound **124C** (2 g, 9.6 mmol) was dissolved in dichloromethane (12 mL), trifluoroacetic acid (17 mL) and water (0.6 mL) were added, and the obtained system was stirred at room temperature for 16 h, and concentrated to obtain an oily substance; saturated aqueous sodium bicarbonate solution (100 mL) was added, extraction was performed with ethyl acetate (50 mL×3), the organic phases were combined and concentrated, the residue was separated and purified by silica gel chromatography column (ethyl acetate:petroleum ether (v/v) =10:90) to obtain the compound **124D** of interest (1.5 g, 96.3%).

LCMS m/z=162.1&164.1[M+H]⁺.

Step 4: Using **124D** (1.5 g, 9.3 mmol) as the raw material, the operation method of step 1 of example 119 was referenced to obtain **compound 124E** (1.1 g, 72.9%).

LCMS m/z=163.1&165.1 [M+H]⁺.

Step 5: Using **124E** (1.1 g, 6.8 mmol) as the raw material, the operation method of step 2 of example 119 was referenced to obtain **compound 124F** (1.9 g, 86.8%).

LCMS m/z=323.1&325.1[M+H]⁺.

Step 6: Using **124F** (100 mg, 0.31 mmol) and **101B** (87.5 mg, 0.31 mmol) as the raw materials, the operation method of step 3 of example 119 was referenced to obtain **compound 124** (55 mg, 31.3%).

LCMS m/z = 569.2&571.2 [M+H]⁺.

¹H NMR (400 MHz, DMSO-*d*₆) δ 12.19 (s, 1H), 7.68 (s, 1H), 7.34 (dd, 1H), 5.38-5.27 (m, 1H), 4.56-4.38 (m, 2H), 3.81-3.69 (m, 1H), 3.48-3.36 (m, 1H), 3.15-3.03 (m, 1H), 2.71-2.52 (m, 5H), 2.47-2.37 (m, 2H), 2.23 (s, 3H), 1.81-1.65 (m, 4H), 1.61 (d, 3H), 1.59-1.42 (m, 3H), 1.27 (s, 3H), 1.14-0.89 (m, 2H).

### Example 125:

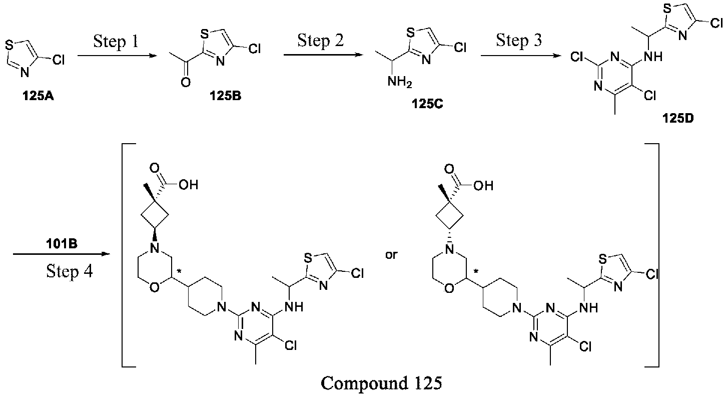

Step 1: Compound **125A** (2 g, 16.7 mmol) was dissolved in tetrahydrofuran (20 mL), the temperature was lowered to -70°C, n-butyllithium solution (7.3 mL, 2.5M solution in toluene) was slowly added dropwise under nitrogen protection, and stirred for 30 min, then N-methoxy-N-methylacetamide (1.7 g, 20.1 mmol) was added. Stirring was continued for 1 h. The reaction was completed as monitored by TLC. Saturated aqueous sodium bicarbonate solution (50 mL) was added to quench the reaction, and ethyl acetate was used for extraction (20 mL×3). The organic phases were combined and concentrated, and then separated and purified using a silica gel chromatography column (ethyl acetate:petroleum ether (v/v) = 10:90) to obtain the compound **125B** of interest (1.7 g, 37.0%).

LCMS m/z=162.1&164.1 [M+H]⁺.

Step 2: Using **125B** (1.6 g, 9.9 mmol) as the raw material, the operation method of step 1 of example 119 was referenced to obtain compound **125C** (1.5 g, 93.2%).

LCMS m/z=163.1&165.1 [M+H]⁺.

Step 3: Using **125C** (500 mg, 3.07 mmol) and 2,4,5-trichloro-6-methylpyrimidine (607 mg, 3.07 mmol) as the raw materials, the operation method of step 2 of example 119 was referenced to obtain compound **125D** (560 mg, 56.3%).

LCMS m/z=323.1&325.1[M+H]⁺.

Step 4: Using **125D** (200 mg, 0.62 mmol) and **101B** (175 mg, 0.62 mmol) as the raw materials, the operation method of step 3 of example 119 was referenced to obtain compound **125** (120 mg, 34.1%).

LCMS m/z = 569.2&571.2 [M+H]⁺.

¹H NMR (400 MHz, DMSO-*d*₆) δ 12.21 (s, 1H), 7.53 (d, 1H), 7.39 (d, 1H), 5.43-5.29 (m, 1H), 4.54-4.38 (m, 2H), 3.81-3.70 (m, 1H), 3.49-3.35 (m, 2H), 3.14-3.02 (m, 1H), 2.70-2.52 (m, 4H), 2.47-2.37 (m, 2H), 2.22 (s, 3H), 1.81-1.65 (m, 4H), 1.65-1.59 (m, 3H), 1.59-1.42 (m, 3H), 1.27 (s, 3H), 1.12-0.86 (m, 2H).

### Example 126:

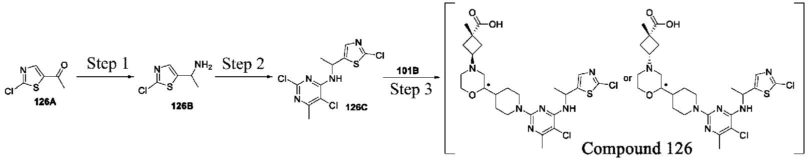

Step 1: Using compound **126A** (1.0 g, 6.19 mmol) as the raw material, the operation method of step 1 of example 119 was referenced to obtain **compound 126B** (600 mg, 60%).

LCMS m/z=163.1&165.1 [M+H]⁺.

Step 2: Using compound **126B** (300 mg, 1.84 mmol) as the raw material, the operation method of step 2 of example 119 was referenced to obtain **compound 126C** (400 mg, 67%).

LCMS m/z=323.1&325.1[M+H]⁺.

Step 3: Using compound **126C** (100 mg, 0.31 mmol) and **101B** (87.3 mg, 0.31 mmol) as the raw materials, the operation method of step 3 of example 119 was referenced to obtain **compound 126** (60 mg, 34%).

¹H NMR (400 MHz, Chloroform-*d*) δ 7.41 (d, 1H), 5.46-5.38 (m, 1H), 5.24 (d, 1H), 4.70 (d, 2H), 3.89 (d, 1H), 3.72-3.58 (m, 1H), 3.30 (s, 1H), 2.95-2.81 (s, 2H), 2.79-2.69 (m, 3H), 2.63-2.56 (m, 2H), 2.29 (s, 3H), 2.04-1.85 (m, 4H), 1.81-1.71 (m, 1H), 1.68 (d, 3H), 1.65-1.58 (m, 2H), 1.41 (s, 3H), 1.28-1.21 (m, 2H).

LCMS m/z = 569.2&571.2 [M+H]⁺.

### Example 127:

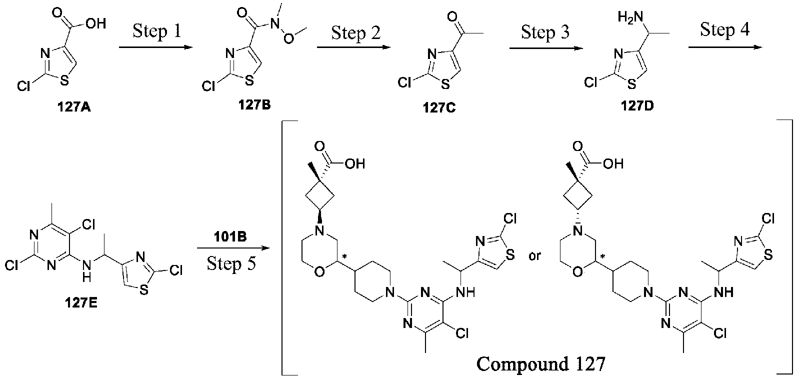

Step 1: Compound **127A** (5.0 g, 30.57 mmol), dimethylhydroxylamine hydrochloride (2.98 g, 30.57 mmol), N,N,N',N'-tetramethyl-O-(7-azabenzotriazol-1-yl)uronium hexafluorophosphate (13.94 g, 36.68 mmol) were dissolved in DMF (60 mL) at room temperature, and then N,N-diisopropylethylamine (11.74 g, 91.71 mmol) was added, the reaction was performed at room temperature for 12 h. After the reaction was completed, water (20 mL) was added, and the mixture was extracted with ethyl acetate (20 mL×3). The combined organic phases were washed with saturated brine (10 mL), dried over anhydrous sodium sulfate, filtered, and concentrated. The residue was quickly separated and purified by column chromatography (eluent EA/PE (v/v) = 0~30%) to obtain the compound **127B** of interest (3.5 g, 55%).

LCMS m/z=207.2 [M+H]⁺.

Step 2: Compound **127B** (3.0 g, 14.52 mmol) was dissolved in anhydrous tetrahydrofuran (50 mL) at room temperature. A solution of 1 N methylmagnesium bromide in tetrahydrofuran (24.7 mL, 24.68 mmol) was added dropwise at 0°C under nitrogen atmosphere. After the dropwise addition was completed, the mixture was stirred under an ice bath for 1 h. After the reaction was completed, the reaction was quenched with water (10 mL) and the obtained system was extracted with ethyl acetate (20 mL×3). The combined organic phases were washed with saturated brine (10 mL), dried over anhydrous sodium sulfate, filtered, and concentrated. The residue was quickly separated and purified by column chromatography (eluent EA/PE (v/v) = 0-10%) to obtain the compound **127C** of interest (1.2 g, 51%).

¹H NMR (400 MHz, Chloroform-d) δ 8.02 (s, 1H), 2.62 (s, 3H).

Step 3: Using compound **127C** (1.2 g, 7.43 mmol) as the raw material, the operation method of step 1 of example **119** was referenced to obtain compound **127D** (1.0 g, 83%).

LCMS m/z=163.1&165.1 [M+H]⁺.

Step 4: Using compound **127D** (300 mg, 1.84 mmol) as the raw material, the operation method of step 2 of example **119** was referenced to obtain compound **127E** (400 mg, 67%).

LCMS m/z=323.1&325.1[M+H]⁺.

Step 3: Using compound **127E** (100 mg, 0.31 mmol) and **101B** (87.3 mg, 0.31 mmol) as the raw materials, the operation method of step 3 of example **119** was referenced to obtain **compound 127** (60 mg, 34%).

¹H NMR (400 MHz, Chloroform-d) δ 6.95 (d, 1H), 5.47 (d, 1H), 5.35 - 5.28 (m, 1H), 4.66 (d, 2H), 3.89 (d, 1H), 3.70 - 3.55 (m, 1H), 3.27 (s, 1H), 2.91 - 2.76 (m, 2H), 2.74 - 2.67 (m, 3H), 2.63 - 2.60 (m, 2H), 2.29 (s, 3H), 2.05 - 1.80 (m, 5H), 1.58 (d, 3H), 1.58 - 1.51 (m, 2H), 1.43 (s, 3H), 1.24 - 1.12 (m, 2H) .

LCMS m/z = 569.2&571.2 [M+H]⁺.

### Biological test

### 1. Calcium flow detected by FLIPR

CCR4/HEK293 cells were added to a 384-well cell culture plate (Greiner #781946) at a rate of 20 µL/well (20,000 cells/well) and incubated overnight in a 37°C, 5% CO₂ incubator (Incubator, Thermo). On the second day, the 384-well cell culture plate was taken out of the incubator and the culture medium was removed; Then, 20 µL of detection buffer and 20 µL of 2X Fluo-4 DirectTM (Invitrogen, Cat# F10471) were added. Then, compounds were added at 10 µL/well (compounds were subjected to 4-fold serial dilution, and the highest detection concentration was 10 µM), and the cells were incubated in a 37°C, 5% CO₂ incubator for 50 min, and then incubated at room temperature for 10 min. The 384-well culture plate was placed in FLIPR (Vi-cell XR Cell Viability Analyzer, Beckman Coulter), 10 µL of 6X agonist CCL17 was added by using an automatic loader, and the fluorescence signal was read immediately. The mean and standard error (Mean ± SEM) were statistically analyzed using Graphpad Prism software, and the IC₅₀ value was calculated.

The experimental results are shown in Table 3, where A represents IC₅₀ < 100 nm; B represents 100 nM ≤ IC₅₀ < 500 nM, and C represents 500 nM ≤ IC₅₀.

**Table 3**

| Compounds | IC₅₀ (µM) | Compounds | IC₅₀ (µM) |
|---|---|---|---|
| **Compound 4** | A | **Compound 40** | A |
| **Compound 7** isomer 2 | B | **Compound 42 isomer 1** | A |
| **Compound 8** isomer 1 | B | **Compound 43** | B |
| **Compound 9** isomer 3 | A | **Compound 44** | B |
| **Compound 10** isomer 1 | B | **Compound 45** | B |
| **Compound 11** isomer 1 | A | **Compound 51** | A |
| **Compound 12** | A | **Compound 52** | B |
| **Compound 13** | B | **Compound 54 isomer 1** | B |
| **Compound 14** | A | **Compound 55** | A |
| **Compound 15** | A | **Compound 56** | B |
| **Compound 16** isomer 1 | B | **Compound 60 isomer 1** | B |
| **Compound 16** isomer 2 | A | **Compound 64 isomer 3** | A |
| **Compound 17** | A | **Compound 65 isomer 3** | B |
| **Compound 18** isomer 2 | A | **Compound 66 isomer 3** | B |
| **Compound 19** | A | **Compound 67 isomer 1** | A |
| **Compound 20** isomer 3 | A | **Compound 68 isomer 1** | B |
| **Compound 21** | A | **Compound 70** | B |
| **Compound 22** | A | **Compound 71 isomer 1** | B |
| **Compound 23** | A | **Compound 73 isomer 3** | A |
| **Compound 24** | A | **Compound 74 isomer 3** | B |
| **Compound 25** | A | **Compound 75 isomer 2** | B |
| **Compound 26** | A | **Compound 76 isomer 2** | A |
| **Compound 27** | A | **Compound 77 isomer 2** | A |
| **Compound 28** isomer 1 | A | **Compound 78 isomer 3** | A |
| **Compound 29** isomer 1 | B | **Compound 79 isomer** 1 | B |
| **Compound 30** isomer 1 | A | **Compound 79 isomer** 2 | B |
| **Compound 31** | A | **Compound 80 isomer 1** | A |
| **Compound 32** isomer 2 | A | **Compound 81 isomer 1** | B |
| **Compound 33** | A | **Compound 82 isomer 3** | A |
| **Compound 34** | B | **Compound 83 isomer 1** | A |
| **Compound 35** | A | **Compound 84 isomer 1** | B |
| **Compound 36** | B | **Compound 85 isomer 1** | A |
| **Compound 37** isomer 1 | A | **Compound 86 isomer 1** | A |
| **Compound 37** isomer 2 | B | **Compound 88** | A |
| **Compound 38** | B | **Compound 89 isomer 1** | A |
| **Compound 39** | A | **Compound 106 isomer 3** | A |
| **Compound 105 isomer 3** | A | **Compound 107 isomer 1** | A |
| **Compound 111** | **A** | **Compound 112** | A |

### 2. Pharmacokinetic test in rats

**1.1 Experimental animals:** male SD rats, about 220 g, 6-8 weeks old, 6 rats/compound. Purchased from Chengdu Ddossy Experimental Animals Co., Ltd.

**1.2 Experimental design:** on the day of the experiment, 36 SD rats were randomly grouped according to their body weights. The animals were fasted with water available for 12 to 14 h one day before the administration and were fed 4 h after the administration.

**Table 4. Administration information**

| Group | Number | Administration information | | | | | |
|---|---|---|---|---|---|---|---|
| | Male | Test compound | Administrati on dosage (mg/kg) | Administration concentration (mg/mL) | Administrati on volume (mL/kg) | Collected samples | Mode of administration |
| G1 | 3 | **Compound 9** isomer 3 | 2.5 | 0.5 | 5 | Plasma | Intravenous administration |
| G2 | 3 | | 10 | 1 | 10 | Plasma | Intragastric administration |
| G3 | 3 | **Compound 64** isomer 3 | 2.5 | 0.5 | 5 | Plasma | Intravenous administration |
| G4 | 3 | | 10 | 1 | 10 | Plasma | Intragastric administration |
| G5 | 3 | **Compound 73** isomer 3 | 2.5 | 0.5 | 5 | Plasma | Intravenous administration |
| G6 | 3 | | 10 | 1 | 10 | Plasma | Intragastric administration |
| G7 | 3 | **Compound 78** isomer 3 | 2.5 | 0.5 | 5 | Plasma | Intravenous administration |
| G8 | 3 | | 10 | 1 | 10 | Plasma | Intragastric administration |
| G9 | 3 | **Compound 83** isomer 1 | 2.5 | 0.5 | 5 | Plasma | Intravenous administration |
| G10 | 3 | | 10 | 1 | 10 | Plasma | Intragastric administration |
| G11 | 3 | Compound DW-01 | 2.5 | 0.5 | 5 | Plasma | Intravenous administration |
| G12 | 3 | | 10 | 1 | 10 | Plasma | Intragastric administration |

| | | | | | | | |
|---|---|---|---|---|---|---|---|
| Note: solvent for intravenous administration: 5% DMA+5% Solutol+90% Saline; vehicle for intragastric administration: 5% DMSO+95% (20% SBE-β-CD in saline) | | | | | | | |

Before and after the administration, 0.15 ml of blood was taken from the orbit of the animals under isoflurane anesthesia and placed in an EDTAK2 centrifuge tube. The blood was centrifuged at 5000 rpm and 4°C for 10 min to collect plasma. The blood collection time points for the intravenous administration group and intragastric administration group were: 0, 5 min, 15 min, 30 min, 1 h, 2 h, 4 h, 6 h, 8 h and 24 h. Before analysis and detection, all samples were stored at -80°C, and a quantitative analysis of samples was performed using LC-MS/MS.

**Table 5. Pharmacokinetic parameters of test compound in plasma of rats**

| Test compound | Mode of administration | CL (mL/min/kg) | Vdₛₛ (Like) | AUC₀₋ₜ (hr*ng/mL) | F (%) |
|---|---|---|---|---|---|
| **Compound 9** isomer 3 | i.v. (2.5 mg/kg) | 6.61±0.42 | 1.49±0.13 | 6283±402 | - |
| | i.g. (10 mg/kg) | - | - | 24805±4809 | 98.7±19 |
| **Compound 64** isomer 3 | i.v. (2.5 mg/kg) | 6.85±1.2 | 1.38±0.25 | 6022±1390 | - |
| | i.g. (10 mg/kg) | - | - | 26117±5148 | 108±21 |
| **Compound 73** isomer 3 | i.v. (2.5 mg/kg) | 9.19±2.1 | 1.55±0.091 | 4629±1027 | - |
| | i.g. (10 mg/kg) | - | - | 14862±6375 | 80.3±34 |
| **Compound** | i.v. (2.5 mg/kg) | 9.22±1.2 | 2.07±0.28 | 4561±638 | - |
| **78** isomer 3 | i.g. (10 mg/kg) | - | - | 28377±7069 | >98% |
| **Compound 83** isomer 1 | i.v. (2.5 mg/kg) | 5.53±1.0 | 2.25±0.27 | 7324±1276 | - |
| | i.g. (10 mg/kg) | - | - | 36976±2821 | >98% |
| Compound DW-01 | i.v. (2.5 mg/kg) | 15.9±1.5 | 4.41±0.23 | 2590±238 | - |
| | i.g. (10 mg/kg) | - | - | 7346±1323 | 70.9±13 |

| | | | | | |
|---|---|---|---|---|---|
| -: not applicable. | | | | | |

Compound DW-01 was synthesized according to the synthesis method of EXAMPLE38 in patent WO 2019147862.

Conclusion: The compounds, such as the compounds in the examples, have good pharmacokinetic profiles in rats.

### 3. Pharmacokinetic test in mice

**1.1 Experimental animals:** Male C57 mice, 20-25 g, 6 mice/compound. Purchased from Chengdu Ddossy Experimental Animals Co., Ltd.

**1.2 Experimental design:** On the day of the experiment, 24 C57 mice were randomly grouped according to their body weight. The animals were fasted with water available for 12 to 14 h one day before the administration and were fed 4 h after the administration.

**Table 6. Administration information**

| Group | Number | Administration information | | | | | |
|---|---|---|---|---|---|---|---|
| | Male | Test compound | Administration dosage (mg/kg) | Administrati on concentration (mg/mL) | Administrati on volume (mL/kg) | Collected samples | Mode of administration |
| G1 | 3 | **Compound 64** isomer 3 | 2.5 | 0.5 | 5 | Plasma | Intravenous administration |
| G2 | 3 | | 10 | 1 | 10 | Plasma | Intragastric administration |
| G3 | 3 | **Compound 73** isomer 3 | 2.5 | 0.5 | 5 | Plasma | Intravenous administration |
| G4 | 3 | | 10 | 1 | 10 | Plasma | Intragastric administration |
| G5 | 3 | **Compound 76** isomer 2 | 2.5 | 0.5 | 5 | Plasma | Intravenous administration |
| G6 | 3 | | 10 | 1 | 10 | Plasma | Intragastric administration |
| G7 | 3 | **Compound 83** isomer 1 | 2.5 | 0.5 | 5 | Plasma | Intravenous administration |
| G8 | 3 | | 10 | 1 | 10 | Plasma | Intragastric administration |

| | | | | | | | |
|---|---|---|---|---|---|---|---|
| Note: solvent for intravenous administration: 5% DMA+5% Solutol+90% Saline; vehicle for intragastric administration: 5% DMSO+95% (20% SBE-β-CD in saline) | | | | | | | |

Before and after the administration, 0.06 mL of blood was taken from the orbit of the animals under isoflurane anesthesia and placed in an EDTAK2 centrifuge tube. The blood was centrifuged at 5000 rpm and 4°C for 10 min to collect plasma. The blood collection time points for the intravenous group and intragastric administration group were: 0 min, 5 min, 15 min, 30 min, 1 h, 2 h, 4 h, 7 h and 24 h. Before analysis and detection, all samples were stored at -80°C, and a quantitative analysis of samples was performed using LC-MS/MS.

**Table 7. Pharmacokinetic parameters of test compounds in plasma of mice**

| Test compound | Mode of administration | CL (mL/min/kg) | Vdₛₛ (L/kg) | AUC₀₋ₜ (hr*ng/mL) | F (%) |
|---|---|---|---|---|---|
| **Compound 64** isomer 3 | i.v. (2.5 mg/kg) | 9.26±0.73 | 1.94±0.053 | 4489±361 | - |
| | i.g. (10 mg/kg) | - | - | 21207±815 | 118±4.5 |
| **Compound 73** isomer 3 | i.v. (2.5 mg/kg) | 5.62±0.29 | 1.04±0.055 | 7404±385 | - |
| | i.g. (10 mg/kg) | - | - | 26775±4114 | 90.4±14 |
| **Compound 76** isomer 2 | i.v. (2.5 mg/kg) | 23.1±1.7 | 1.44±0.12 | 1794±125 | - |
| | i.g. (10 mg/kg) | - | - | 8839±1014 | 123±14 |
| **Compound 83** isomer 1 | i.v. (2.5 mg/kg) | 5.69±0.60 | 1.77±0.069 | 7251±726 | - |
| | i.g. (10 mg/kg) | - | - | 26575±3519 | 91.6±12 |

| | | | | | |
|---|---|---|---|---|---|
| -: not applicable. | | | | | |

Conclusion: The compounds, such as the compounds in the examples, have good pharmacokinetic profiles in mice.

### 4. Pharmacokinetic test in monkeys

**Experimental animals:** Male cynomolgus monkeys, 3-5 kg, 3-6 years old, 4 monkeys/compound. purchased from Suzhou Xishan Biotechnology Inc.

**Experimental method:** On the day of the experiment, 8 monkeys were randomly grouped according to their body weights. The animals were fasted with water available for 14 to 18 h one day before the administration, and were fed 4 h after the administration.

**Table 8. Administration information**

| Group | Number | Administration information | | | | | |
|---|---|---|---|---|---|---|---|
| | Male | Test compound | Administrati on dosage (mg/kg) | Administration concentration (mg/mL) | Administration volume (mL/kg) | Collected samples | Mode of administration |
| G1 | 2 | **Compound 9** isomer 3 | 1 | 1 | 1 | Plasma | Intravenous administration |
| G2 | 2 | | 5 | 1 | 5 | Plasma | Intragastric administration |
| G3 | 2 | **Compound 83** isomer 1 | 1 | 1 | 1 | Plasma | Intravenous administration |
| G4 | 2 | | 5 | 1 | 5 | Plasma | Intragastric administration |

| | | | | | | | |
|---|---|---|---|---|---|---|---|
| Note: vehicle for intravenous administration: 5% DMA+5% Solutol+90% Saline; vehicle for intragastric administration: 5% DMSO+95% (20% SBE-β-CD in saline) | | | | | | | |

Before and after the administration, 1.0 mL of blood samples were drawn from the limb veins and placed in an EDTAK2 centrifuge tube. Centrifugation was carried out at 5000 rpm at 4°C for 10 min, and the plasma was collected. The blood collection time points for the intravenous group and intragastric administration group were: 0 min, 5 min, 15 min, 30 min, 1 h, 2 h, 4 h, 6 h, 8 h, 10 h, 12 h, 24 h, and 48 h. Before analysis and detection, all samples were stored at -80°C, and a quantitative analysis of samples was performed using LC-MS/MS.

**Table 9. Pharmacokinetic parameters of test compound in plasma of monkeys**

| Test compound | Mode of administration | CL (mL/min/kg) | Vdₛₛ (L/kg) | AUC₀₋ₜ (hr*ng/mL) | F (%) |
|---|---|---|---|---|---|
| **Compound** 9 isomer 3 | i.v. (1 mg/kg) | 2.44±0.51 | 0.995±0.088 | 6930±1435 | - |
| | i.g. (5 mg/kg) | - | - | 25243±19865 | 72.8±57 |
| **Compound 83** isomer 1 | i.v. (1 mg/kg) | 1.02±0.14 | 0.754±0.15 | 15821±1740 | - |
| | i.g. (5 mg/kg) | - | - | 60729±21554 | 76.8±27 |

Conclusion: The compounds, such as the compounds in the examples, have good pharmacokinetic profiles in monkeys.

### 5. Pharmacokinetic test in beagle dogs

Experimental animals: male beagle dogs, about 8-11 kg, 6 beagle dogs/compound, purchased from Beijing Marshall Biotechnology Co., Ltd.

Experimental method: On the day of the experiment, 18 beagle dogs were randomly grouped according to their body weights. The animals were fasted with water available for 12 to 14 h one day before the administration and were fed 4 h after the administration.

**Table 10. Administration information**

| Group | Number | Administration information | | | | | |
|---|---|---|---|---|---|---|---|
| | Male | Test compound | Administrati on dosage (mg/kg) | Administration concentration (mg/mL) | Administrati on volume (mL/kg) | Collected samples | Mode of administration |
| G1 | 3 | **Com pound 73** isomer 3 | 1 | 1 | 1 | Plasma | Intravenous administration |
| G2 | 3 | | 3 | 1 | 3 | Plasma | Intragastric administration |
| G3 | 3 | **Com pound 82** isomer 3 | 1 | 1 | 1 | Plasma | Intravenous administration |
| G4 | 3 | | 3 | 1 | 3 | Plasma | Intragastric administration |
| G5 | 3 | Compound DW-01 | 1 | 1 | 1 | Plasma | Intravenous administration |
| G6 | 3 | | 3 | 1 | 3 | Plasma | Intragastric administration |

| | | | | | | | |
|---|---|---|---|---|---|---|---|
| Note: vehicle for intravenous administration: 5% DMA+5% Solutol+90% Saline; vehicle for intragastric administration: 5% DMSO+95% (20% SBE-β-CD in saline) | | | | | | | |

Before and after the administration, 1 ml of blood was taken from the jugular veins or limb veins, and placed in an EDTAK2 centrifuge tube. Centrifugation was performed at 5000 rpm at 4°C for 10 min, and plasma was collected. The blood collection time points for the intravenous group and intragastric administration group were: 0 min, 5 min, 15 min, 30 min, 1h, 2 h, 4 h, 6 h, 8 h, 10 h, 12 h, 24 h, 48 h, and 72 h. Before analysis and detection, all samples were stored at -80°C. The samples were analyzed quantitatively by LC-MS/MS.

**Table 11. Pharmacokinetic parameters of test compound in plasma of dogs**

| Test compound | Mode of administration | CL (mL/min/kg) | Vdₛₛ (L/kg) | AUC₀₋ₜ (hr*ng/mL) | F (%) |
|---|---|---|---|---|---|
| **Compound 73** isomer 3 | i.v. (1 mg/kg) | 1.38±0.40 | 1.20±0.16 | 12077±3172 | - |
| | i.g. (3 mg/kg) | - | - | 37685±10094 | >98% |
| **Compound 82** isomer 3 | i.v. (1 mg/kg) | 0.672±0.14 | 1.04±0.20 | 21070±3835 | - |
| | i.g. (3 mg/kg) | - | - | 68403±7194 | >98% |
| Compound DW-01 | i.v. (1 mg/kg) | 1.39±0.29 | 1.00±0.25 | 11955±2500 | |
| | i.g. (3 mg/kg) | - | - | 26598±12124 | 74.2±34 |

Conclusion: The compounds, such as the compounds in the examples, have good pharmacokinetic profiles in dogs.

## Claims

1. A compound as shown in formula (I), a stereoisomer, a deuterated compound, a solvate, or a pharmaceutically acceptable salt or a co-crystal thereof, 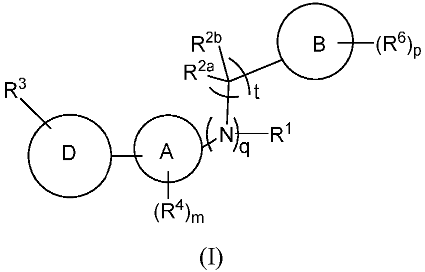 **characterized in that**
ring A is selected from 6-membered heteroaryl, 9- or 10-membered bicyclic heteroaryl, 6- to 10-membered aryl, 9- or 10-membered bicyclic heterocycloalkyl;
ring B is selected from phenyl, 8- to 10-membered aryl, 5- or 6-membered heteroaryl, or 8- to 10-membered heteroaryl;
ring D is selected from -Cy2-Cy3-#, -L₁-Cy2-Cy3-#, -Cy2-L₁-#, -L₁-Cy2-#, - L₁-Cy3-#, Cy4, or -L₁-Cy4-#, wherein # represents the site where the ring D is attached to the ring A;
Cy2 is selected from 4- to 7-membered monoheterocycloalkyl, 7- to 10-membered bridged heterocycloalkyl, 7- to 10-membered spiroheterocycloalkyl, 8-to 10-membered fused heterocycloalkyl, 6- or 7-membered cycloalkyl, or phenyl, and the Cy2 is optionally substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy, haloC₁₋₄ alkoxy, deuterated C₁₋₄ alkoxy and NH₂;
Cy3 is selected from 4- to 7-membered monoheterocycloalkyl, 7- to 10-membered spiroheterocycloalkyl, 5- or 6-membered heteroaryl, 6- to 10-membered fused heterocycloalkyl, or phenyl, and the Cy3 is optionally substituted with 1 to 3 groups selected from =O, halogen, deuterium, CN, OH, C₁₋₄ alkyl and NH₂;
Cy4 is selected from 7- to 10-membered bridged heterocycloalkyl, 7- to 10-membered spiroheterocycloalkyl, 8- to 10-membered fused heterocycloalkyl;
L₁ is selected from a bond, C₁₋₆ alkylene, C₂₋₆ alkenylene, C₂₋₆ alkynylene, or - C(=O)-;
each of m, p, q, and t is independently selected from 0, 1, 2, 3, 4, or 5;
each of R¹, R^{2a}, and R^{2b} is independently selected from H, deuterium, C₁₋₄ alkyl, haloC₁₋₄ alkyl, or deuterated C₁₋₄ alkyl;
R³ is selected from -Cyl-R^{3a} or R^{3a};
Cy1 is selected from 3- to 10-membered cycloalkyl or 4- to 10-membered heterocycloalkyl, and the cycloalkyl and heterocycloalkyl are optionally further substituted with 1 to 3 groups selected from C₁₋₆ alkyl, halogen, deuterium, cyano, nitro, OH, haloC₁₋₆ alkyl, or deuterated C₁₋₆ alkyl;
R^{3a} is selected from -COOH, COOC₁₋₆ alkyl, -P(O)(OH)OH, -P(O)(OH)H, hydroxyC₁₋₆ alkyl, -C₁₋₆ alkyl-COOH, 5- or 6-membered heteroaryl, or 5- or 6-membered heterocycloalkyl, and the heteroaryl and heterocycloalkyl are optionally further substituted with 1 to 3 groups selected from =O, C₁₋₆ alkyl, halogen, deuterium, cyano, nitro, OH, haloC₁₋₆ alkyl, or deuterated C₁₋₆ alkyl;
R⁴ is selected from deuterium, halogen, cyano, nitro, OH, amino, SF₅, N₃, C₁₋₆ alkyl, haloC₁₋₆ alkyl, deuterated C₁₋₆ alkyl, C₂₋₆ alkenyl, C₂₋₆ alkynyl, C₁₋₆ alkoxy, haloC₁₋₆ alkoxy, deuterated C₁₋₆ alkoxy, or -C(=O)R^{4a};
R^{4a} is selected from deuterium, halogen, OH, amino, or C₁₋₆ alkyl;
each of R⁶ and R^{6a} is independently selected from deuterium, halogen, cyano, nitro, OH, amino, SF₅, N₃, C₁₋₆ alkyl, haloC₁₋₆ alkyl, deuterated C₁₋₆ alkyl, C₂₋₆ alkenyl, C₂₋₆ alkynyl, C₁₋₆ alkoxy, haloC₁₋₆ alkoxy, or deuterated C₁₋₆ alkoxy;
alternatively, two R⁶ together with the atoms to which they are attached form 5- or 6-membered cycloalkenyl; the cycloalkenyl is optionally substituted with 1 to 5 selected from R^{6a};
alternatively, R⁴ and R¹ together with the atoms to which they are attached form 5- or 6-membered heteroaryl or 5- or 6-membered heterocycloalkyl;
alternatively, R¹ and R⁶ together with the atoms to which they are attached form 5- or 6-membered heterocycloalkyl;
alternatively, R^{2a} and R⁶ together with the atoms to which they are attached form 5- or 6-membered cycloalkyl;
alternatively, R¹ and R^{2a} together with the atoms to which they are attached form 4- to 6-membered heterocycloalkyl;
alternatively, R^{2a} and R^{2b} on the same carbon atom or different carbon atoms together with the atoms to which they are attached form 3-membered cycloalkyl, or 4- to 6-membered cycloalkyl;
alternatively, two R⁴ on adjacent ring atoms together with the atoms to which they are attached form C₄₋₆ cycloalkyl or 4- to 7-membered heterocycloalkyl, and the cycloalkyl and heterocycloalkyl are optionally further substituted with 1 to 3 groups selected from deuterium, halogen, C₁₋₆ alkyl, cyano, OH, amino, SF₅, N₃, haloC₁₋₆ alkyl, deuterated C₁₋₆ alkyl, C₂₋₆ alkenyl, C₂₋₆ alkynyl, C₁₋₆ alkoxy, haloC₁₋₆ alkoxy, deuterated C₁₋₆ alkoxy and COC₁₋₆ alkyl.

2. The compound of formula (I), or the stereoisomer, the deuterated compound, the solvate, or the pharmaceutically acceptable salt or the co-crystal thereof according to claim 1, **characterized in that** the compound has a structure as shown in formula (I-1), (I-1a), (I-1b), (I-1c), (I-1d), (I-1e), (I-1f), or (I-1g): 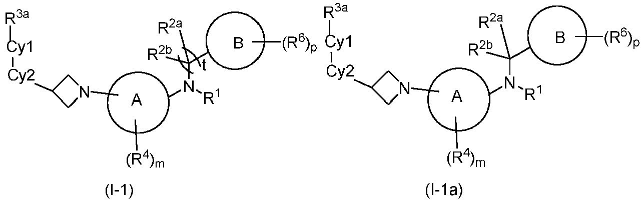 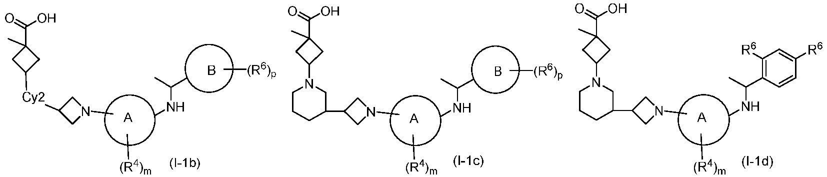 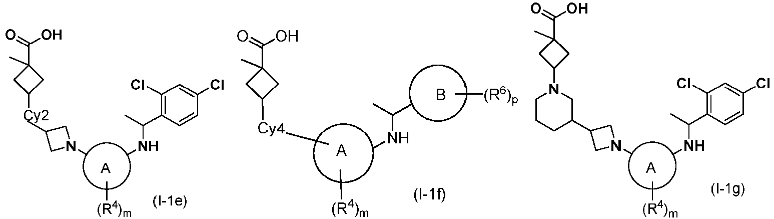 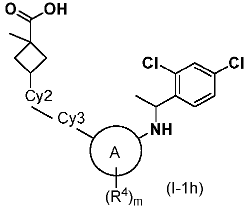 provided that:
(1) ring 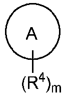 is not 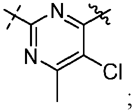
(2) in formula (I-1h), ring 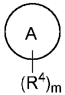 is not 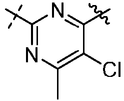 and Cy2 is not 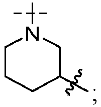
(3) in formula (I-1d), ring 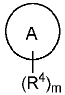 is selected from 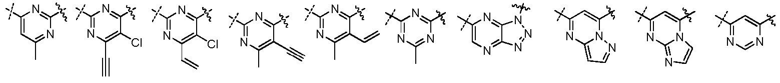 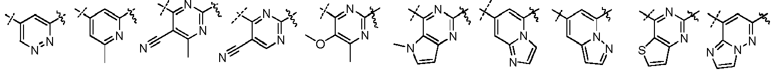 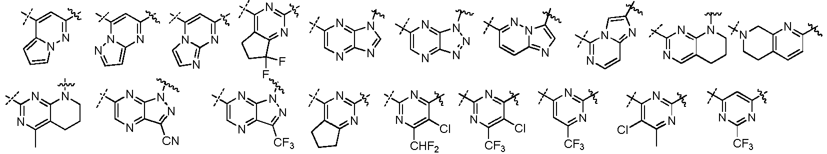 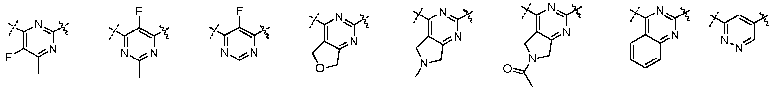 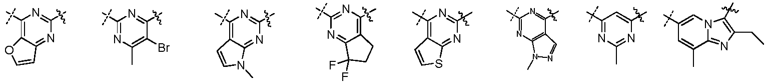 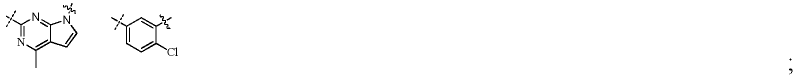 where 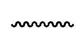 represents an attachment to the right side, andrepresents an attachment to the left side.

3. The compound of formula (I), or the stereoisomer, the deuterated compound, the solvate, or the pharmaceutically acceptable salt or the co-crystal thereof according to claim 1, **characterized in that** the compound has a structure as shown in formula (I-2a), (I-2b), (I-2c), (I-2d), or (I-2): 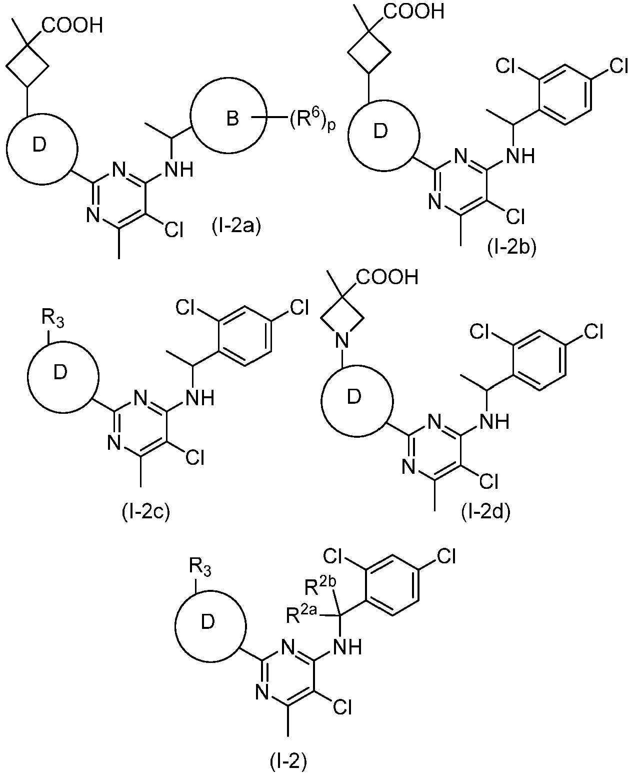 provided that, the ring D is not selected from 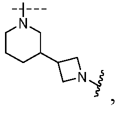 where 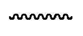 represents an attachment to the right side and ------- represents an attachment to the left side.

4. The compound of formula (I), or the stereoisomer, the deuterated compound, the solvate, or the pharmaceutically acceptable salt or the co-crystal thereof according to claim 1, **characterized in that** the compound has a structure as shown in formula (I-3) or (I-3a): 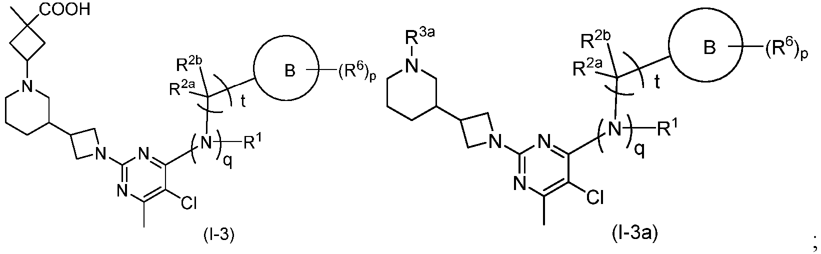 provided that, 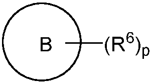 is not selected from

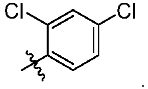

5. The compound of formula (I), or the stereoisomer, the deuterated compound, the solvate, or the pharmaceutically acceptable salt or the co-crystal thereof according to claim 1, **characterized in that** the compound has a structure as shown in formula (I-4):

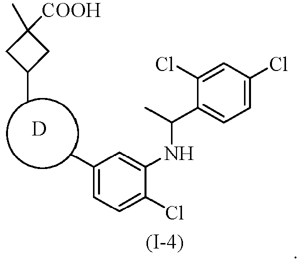

6. The compound of formula (I), or the stereoisomer, the deuterated compound, the solvate, or the pharmaceutically acceptable salt or the co-crystal thereof according to claim 1, **characterized in that**
ring 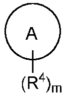
is selected from 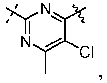 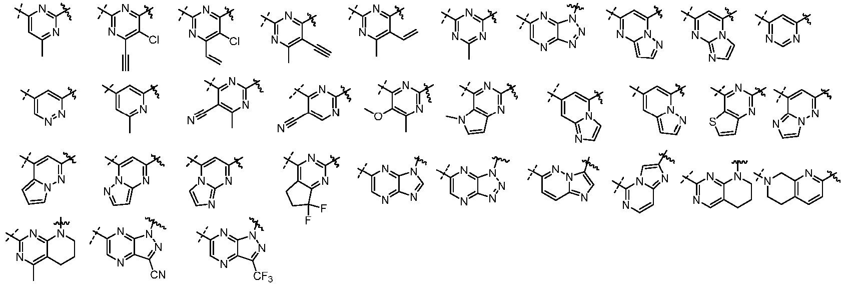 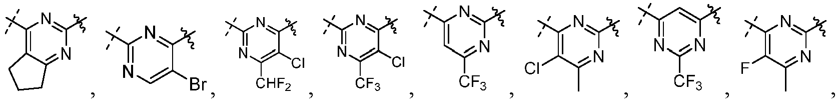 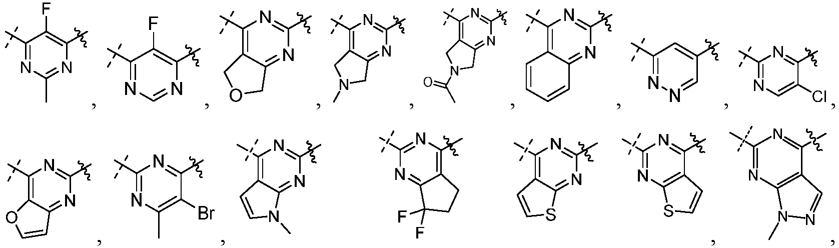 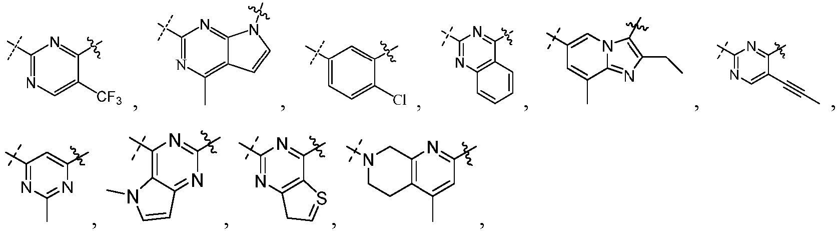
where 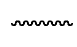 represents an attachment to the right side, andrepresents an attachment to the left side;
alternatively, R⁴, R¹ and the atoms to which they are attached together with the ring A form

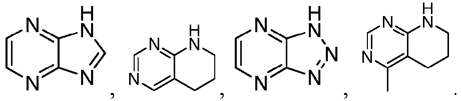

7. The compound of formula (I), or the stereoisomer, the deuterated compound, the solvate, or the pharmaceutically acceptable salt or the co-crystal thereof according to claim 1, **characterized in that**
ring 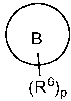
is selected from 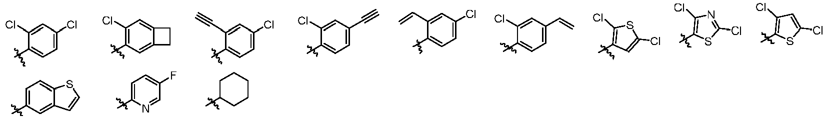 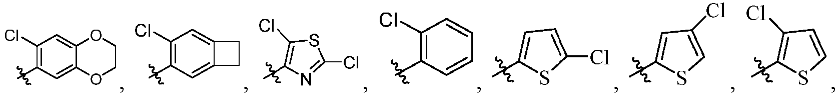 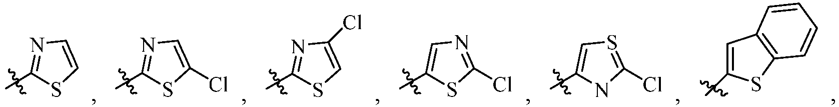 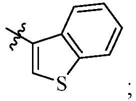
alternatively, R¹, R⁶ and the atoms to which they are attached together with the ring B form 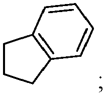
alternatively, R^{2a}, R⁶ and the atoms to which they are attached together with the ring B form

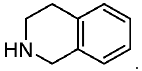

8. The compound of formula (I), or the stereoisomer, the deuterated compound, the solvate, or the pharmaceutically acceptable salt or the co-crystal thereof according to claim 1, **characterized in that**
the ring D is selected from -Cy2-Cy3-#, -Cy2-L₁-#, -L₁-Cy2-#, or Cy4, wherein # represents the site where the ring D is attached to the ring A;
Cy2 is selected from 4- to 7-membered monoheterocycloalkyl, 7- to 10-membered bridged heterocycloalkyl, 7- to 10-membered spiroheterocycloalkyl, 8-to 10-membered fused heterocycloalkyl, 6- or 7-membered cycloalkyl, or phenyl, and the Cy2 is optionally substituted with 1 to 3 groups selected from =O, halogen, deuterium, CN, OH, C₁₋₄ alkyl, haloC₁₋₄ alkyl, deuterated C₁₋₄ alkyl, C₁₋₄ alkoxy and NH₂;
Cy3 is selected from 4- to 7-membered monoheterocycloalkyl, 5- or 6-membered heteroaryl, 6- to 10-membered fused heterocycloalkyl, or phenyl, and the Cy3 is optionally substituted with 1 to 3 groups selected from =O, halogen, deuterium, CN, OH, C₁₋₂ alkyl and NH₂;
L₁ is selected from methylene, ethylene, vinylene, ethynylene, or -C(=O)-.

9. The compound of formula (I), or the stereoisomer, the deuterated compound, the solvate, or the pharmaceutically acceptable salt or the co-crystal thereof according to claim 1, **characterized in that**
ring Cy2 is selected from: 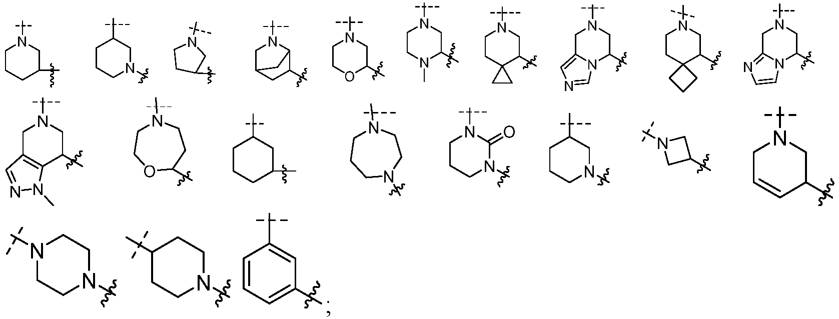
ring Cy3 is selected from 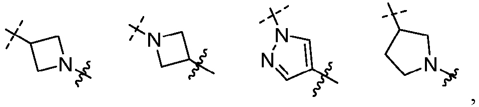 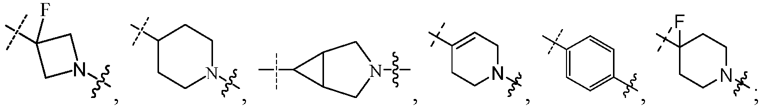
ring Cy4 is selected from 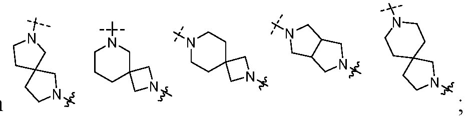
L₁ is selected from methylene, alkynylene, or -C(=O)-;
where 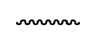 represents an attachment to the right side, andrepresents an attachment to the left side.

10. The compound of formula (I), or the stereoisomer, the deuterated compound, the solvate, or the pharmaceutically acceptable salt or the co-crystal thereof according to claim 1, **characterized in that**
the ring D is selected from: 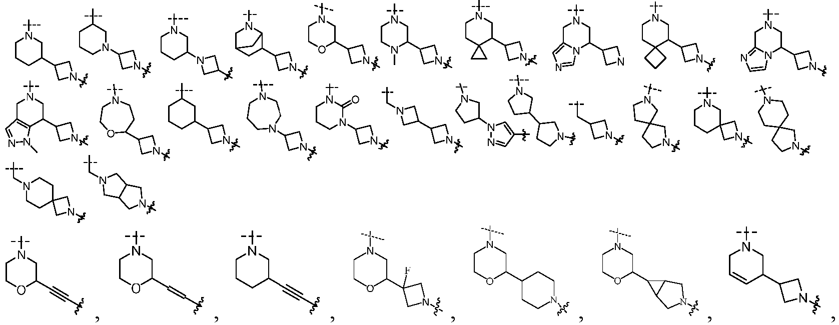 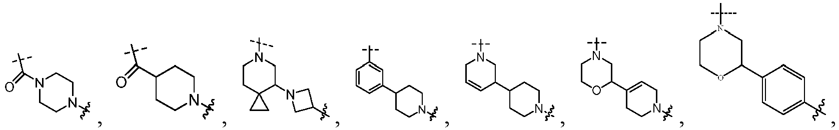 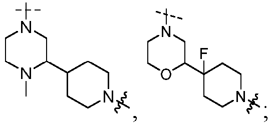
where 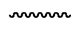 represents an attachment to the right side, andrepresents an attachment to the left side.

11. The compound of formula (I), or the stereoisomer, the deuterated compound, the solvate, or the pharmaceutically acceptable salt or the co-crystal thereof according to claim 1, **characterized in that**
Cy1 is selected from 3- to 6-membered monocyclic cycloalkyl, 7- to 10-membered bicyclic cycloalkyl, 4- to 6-membered monoheterocycloalkyl, or 7- to 10-membered bicyclic heterocycloalkyl, and the cycloalkyl and heterocycloalkyl are optionally further substituted with 1 to 3 groups selected from C₁₋₄ alkyl, halogen, deuterium, cyano, nitro, OH, haloC₁₋₄ alkyl, and deuterated C₁₋₄ alkyl;
R³ is selected from

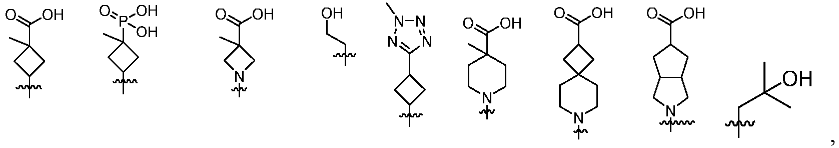

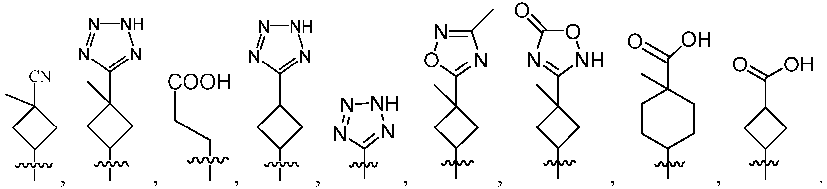

12. The compound, or the stereoisomer, the deuterated compound, the solvate, or the pharmaceutically acceptable salt or the co-crystal thereof according to claim 1, **characterized in that** the compound is selected from one of the structures in Table 1.

13. The compound, or the stereoisomer, the deuterated compound, the solvate, or the pharmaceutically acceptable salt or the co-crystal thereof according to claim 1, **characterized in that** the compound is selected from one of the structures in Table 2.

14. A pharmaceutical composition or pharmaceutical preparation, **characterized in that** the pharmaceutical composition or pharmaceutical preparation comprises the compound, or the stereoisomer, the deuterated compound, the solvate, or the pharmaceutically acceptable salt or the co-crystal thereof according to any one of claims 1 to 13, and a pharmaceutically acceptable carrier and/or excipient.

15. The pharmaceutical composition or the pharmaceutical preparation according to claim 1, **characterized in that** the pharmaceutical composition or pharmaceutical preparation comprises 1-1500 mg of the compound, or the stereoisomer, the deuterated compound, solvate, the pharmaceutically acceptable salt or the co-crystal thereof according to any one of claims 1-13, and a carrier and/or an excipient.

16. The compound, or the stereoisomer, the deuterated compound, the solvate, or the pharmaceutically acceptable salt or the co-crystal thereof according to any one of claims 1 to 13, or the composition according to claim 14 or 15 for use in the preparation of a drug for treating/preventing a CCR4-mediated disease.

17. The use according to claim 16, **characterized in that** the CCR4-mediated disease is selected from a tumor or inflammation.

18. A method for treating a disease in a mammal or human, **characterized in that** the method comprisess administering to a subject a therapeutically effective amount of the compound or the stereoisomer, the deuterated compound, the solvate, the pharmaceutically acceptable salt or the co-crystal thereof according to any one of claims 1-13, wherein the therapeutically effective amount is preferably 1-1500 mg, and the disease is preferably a tumor or inflammation.
